(19) Europäisches Patentamt
European Patent Office
Office européen des brevets

(11) EP 1 927 594 A1

(12) **EUROPEAN PATENT APPLICATION**

(43) Date of publication:
**04.06.2008 Bulletin 2008/23**

(21) Application number: **07019362.8**

(22) Date of filing: **14.01.2004**

(51) Int Cl.:
***C07D 401/14*** (2006.01) ***C07D 401/04*** (2006.01)
***C07D 413/14*** (2006.01) ***C07D 403/14*** (2006.01)
***C07D 405/14*** (2006.01) ***C07D 417/14*** (2006.01)
***A61K 31/506*** (2006.01) ***A61K 31/513*** (2006.01)
***A61P 3/10*** (2006.01)

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **14.01.2003 US 440394 P**
**24.02.2003 US 449829 P**
**06.03.2003 US 453390 P**
**14.05.2003 US 470875 P**

(62) Document number(s) of the earlier application(s) in accordance with Art. 76 EPC:
**04702248.8 / 1 599 468**

(71) Applicant: **Arena Pharmaceuticals, Inc.**
**San Diego, CA 92121-3223 (US)**

(72) Inventors:
- **Jones, Robert M**
  **San Diego**
  **CA 92130 (US)**
- **Semple, Graeme**
  **San Diego**
  **CA 92127 (US)**
- **Fioravanti, Beatriz**
  **Tucson**
  **Arizona 85704 (US)**
- **Pereira, Guilherme**
  **Minas Gerais (MG)**
  **Brazil cep 30220-090 (BR)**
- **Calderon, Imelda**
  **San Diego**
  **CA 92131 (US)**
- **Uy, Jane**
  **Monrovia**
  **California 91016 (US)**
- **Duvvuri, Kameshwari**
  **Lexington**
  **Kentucky 40502 (US)**
- **Choi, Jin Sun Karoline**
  **San Diego**
  **CA 92131 (US)**
- **Xiong, Yifeng**
  **San Diego**
  **CA 92130 (US)**
- **Dave, Vibha**
  **San Diego**
  **CA 92121 (US)**
- **Azimioara, Mihai D.**
  **La Jolla**
  **CA 92037 (US)**

(74) Representative: **Wytenburg, Wilhelmus Johannes et al**
**Mewburn Ellis LLP**
**York House**
**23 Kingsway**
**London WC2B 6HP (GB)**

Remarks:
This application was filed on 02-10-2007 as a divisional application to the application mentioned under INID code 62.

(54) **1,2,3-Trisubstituted aryl and heteroaryl derivatives as modulators of metabolism and the prophylaxis and treatment of disorders related thereto such as diabetes and hyperglycemia**

(57) The present invention relates to certain 1,2,3-trisubstituted aryl and heteroaryl derivatives of Fomula (1a) that are modulators of metabolism. Accordingly, compounds of the present invention are useful in the prophylaxis or treatment of metabolic disorders and complications thereof, such as, diabetes and obesity.

EP 1 927 594 A1

## Description

### FIELD OF THE INVENTION

The present invention relates to certain 1,2,3-trisubstituted aryl and heteroaryl derivatives that are modulators of glucose metabolism. Accordingly, compounds of the present invention are useful in the prophylaxis or treatment of metabolic disorders and complications thereof, such as, diabetes and obesity.

### BACKGROUND OF THE INVENTION

Diabetes mellitus is a serious disease afflicting over 100 million people worldwide. In the United States, there are more than 12 million diabetics, with 600,000 new cases diagnosed each year.

Diabetes mellitus is a diagnostic term for a group of disorders characterized by abnormal glucose homeostasis resulting in elevated blood sugar. There are many types of diabetes, but the two most common are Type I (also referred to as insulin-dependent diabetes mellitus or IDDM) and Type II (also referred to as non-insulin-dependent diabetes mellitus or NIDDM).

The etiology of the different types of diabetes is not the same; however, everyone with diabetes has two things in common: overproduction of glucose by the liver and little or no ability to move glucose out of the blood into the cells where it becomes the body's primary fuel.

People who do not have diabetes rely on insulin, a hormone made in the pancreas, to move glucose from the blood into the cells of the body. However, people who have diabetes either don't produce insulin or can't efficiently use the insulin they produce; therefore, they can't move glucose into their cells. Glucose accumulates in the blood creating a condition called hyperglycemia, and over time, can cause serious health problems.

Diabetes is a syndrome with interrelated metabolic, vascular, and neuropathic components. The metabolic syndrome, generally characterized by hyperglycemia, comprises alterations in carbohydrate, fat and protein metabolism caused by absent or markedly reduced insulin secretion and/or ineffective insulin action. The vascular syndrome consists of abnormalities in the blood vessels leading to cardiovascular, retinal and renal complications. Abnormalities in the peripheral and autonomic nervous systems are also part of the diabetic syndrome.

People with IDDM, which accounts for about 5% to 10% of those who have diabetes, don't produce insulin and therefore must inject insulin to keep their blood glucose levels normal. IDDM is characterized by low or undetectable levels of endogenous insulin production caused by destruction of the insulin-producing β cells of the pancreas, the characteristic that most readily distinguishes IDDM from NIDDM. IDDM, once termed juvenile-onset diabetes, strikes young and older adults alike.

Approximately 90 to 95% of people with diabetes have Type II (or NIDDM). NIDDM subjects produce insulin, but the cells in their bodies are insulin resistant: the cells don't respond properly to the hormone, so glucose accumulates in their blood. NIDDM is characterized by a relative disparity between endogenous insulin production and insulin requirements, leading to elevated blood glucose levels. In contrast to IDDM, there is always some endogenous insulin production in NIDDM; many NIDDM patients have normal or even elevated blood insulin levels, while other NIDDM patients have inadequate insulin production (Rotwein, R. et al. N. Engl. J. Med. 308, 65-71 (1983)). Most people diagnosed with NIDDM are age 30 or older, and half of all new cases are age 55 and older. Compared with whites and Asians, NIDDM is more common among Native Americans, African-Americans, Latinos, and Hispanics. In addition, the onset can be insidious or even clinically inapparent, making diagnosis difficult.

The primary pathogenic lesion on NIDDM has remained elusive. Many have suggested that primary insulin resistance of the peripheral tissues is the initial event. Genetic epidemiological studies have supported this view. Similarly, insulin secretion abnormalities have been argued as the primary defect in NIDDM. It is likely that both phenomena are important contributors to the disease process (Rimoin, D. L., et. al. Emery and Rimoin's Principles and Practice of Medical Genetics 3rd Ed. 1:1401-1402 (1996)).

Many people with NIDDM have sedentery lifestyles and are obese; they weigh approximately 20% more than the recommended weight for their height and build. Furthermore, obesity is characterized by hyperinsulinemia and insulin resistance, a feature shared with NIDDM, hypertension and atherosclerosis.

Obesity and diabetes are among the most common human health problems in industrialized societies. In industrialized countries a third of the population is at least 20% overweight. In the United States, the percentage of obese people has increased from 25% at the end of the 1970s, to 33% at the beginning the 1990s. Obesity is one of the most important risk factors for NIDDM. Definitions of obesity differ, but in general, a subject weighing at least 20% more than the recommended weight for his/her height and build is considered obese. The risk of developing NIDDM is tripled in subjects 30% overweight, and three-quarters with NIDDM are overweight.

Obesity, which is the result of an imbalance between caloric intake and energy expenditure, is highly correlated with insulin resistance and diabetes in experimental animals and human. However, the molecular mechanisms that are

involved in obesity-diabetes syndromes. are not clear. During early development of obesity, increase insulin secretion balances insulin resistance and protects patients from hyperglycemia (Le Stunff, et al. Diabetes 43, 696-702 (1989)). However, after several decades, β cell function deteriorates and non-insulin-dependent diabetes develops in about 20% of the obese population (Pederson, P. Diab. Metab. Rev. 5, 505-509 (1989)) and (Brancati, F. L., et al., Arch. Intern. Med. 159, 957-963 (1999)). Given its high prevalence in modem societies, obesity has thus become the leading risk factor for NIDDM (Hill, J. O., et al., Science 280,1371-1374 (1998)). However, the factors which predispose a fraction of patients to alteration of insulin secretion in response to fat accumulation remain unknown.

Whether someone is classified as overweight or obese is generally determined on the basis of their body mass index (BMI) which is calculated by dividing body weight (kg) by height squared ($m^2$). Thus, the units of BMI are $kg/m^2$ and it is possible to calculate the BMI range associated with minimum mortality in each decade of life. Overweight is defined as a BMI in the range 25-30 $kg/m^2$, and obesity as a BMI greater than 30 $kg/m^2$ (see TABLE below). There are problems with this definition in that it does not take into account the proportion of body mass that is muscle in relation to fat (adipose tissue). To account for this, obesity can also be defined on the basis of body fat content: greater than 25% and 30% in males and females, respectively.

| CLASSIFICATION OF WEIGHT BY BODY MASS INDEX (BMI) | |
|---|---|
| BMI | CLASSIFICATION |
| < 18.5 | Underweight |
| 18.5-24.9 | Normal |
| 25.0-29.9 | Overweight |
| 30.0-34.9 | Obesity (Class I) |
| 35.0-39.9 | Obesity (Class II) |
| >40 | Extreme Obesity (Class III) |

As the BMI increases there is an increased risk of death from a variety of causes that is independent of other risk factors. The most common diseases with obesity are cardiovascular disease (particularly hypertension), diabetes (obesity aggravates the development of diabetes), gall bladder disease (particularly cancer) and diseases of reproduction. Research has shown that even a modest reduction in body weight can correspond to a significant reduction in the risk of developing coronary heart disease.

Compounds marketed as anti-obesity agents include Orlistat (XENICAL™) and Sibutramine. Orlistat (a lipase inhibitor) inhibits fat absorption directly and tends to produce a high incidence of unpleasant (though relatively harmless) side-effects such as diarrhea. Sibutramine (a mixed 5-HT/noradrenaline reuptake inhibitor) can increase blood pressure and heart rate in some patients. The serotonin releaser/reuptake inhibitors fenfluramine (Pondimin™) and dexfenfluramine (Redux™) have been reported to decrease food intake and body weight over a prolonged period (greater than 6 months). However, both products were withdrawn after reports of preliminary evidence of heart valve abnormalities associated with their use. Accordingly, there is a need for the development of a safer anti-obesity agent.

Obesity considerably increases the risk of developing cardiovascular diseases as well. Coronary insufficiency, atheromatous disease, and cardiac insufficiency are at the forefront of the cardiovascular complication induced by obesity. It is estimated that if the entire population had an ideal weight, the risk of coronary insufficiency would decrease by 25% and the risk of cardiac insufficiency and of cerebral vascular accidents by 35%. The incidence of coronary diseases is doubled in subjects less than 50 years of age who are 30% overweight. The diabetes patient faces a 30% reduced lifespan. After age 45, people with diabetes are about three times more likely than people without diabetes to have significant heart disease and up to five times more likely to have a stroke. These findings emphasize the inter-relations between risks factors for NIDDM and coronary heart disease and the potential value of an integrated approach to the prevention of these conditions based on the prevention of these conditions based on the prevention of obesity (Perry, I. J., et al., BMJ310, 560-564 (1995)).

Diabetes has also been implicated in the development of kidney disease, eye diseases and nervous-system problems. Kidney disease, also called nephropathy, occurs when the kidney's "filter mechanism" is damaged and protein leaks into urine in excessive amounts and eventually the kidney fails. Diabetes is also a leading cause of damage to the retina at the back of the eye and increases risk of cataracts and glaucoma. Finally, diabetes is associated with nerve damage, especially in the legs and feet, which interferes with the ability to sense pain and contributes to serious infections. Taken together, diabetes complications are one of the nation's leading causes of death.

## SUMMARY OF THE INVENTION

The present invention is drawn to compounds, which bind to and modulate the activity of a GPCR referred to herein as **RUP3,** and uses thereof. The term **RUP3,** as used herein, includes the human sequences found in GeneBank accession number XM_066873, naturally-occurring allelic variants, mammalian orthologs, and recombinant mutants thereof. A preferred human **RUP3** for use in screening and testing of the compounds of the invention is provided in the nucleotide sequence of Seq. ID.No:1 and the corresponding amino acid sequence in Seq. ID.No:2.

One aspect of the present invention encompasses 1,2,3-trisubstituted aryl and heteroaryl derivatives as shown in Formula (**Ia**):

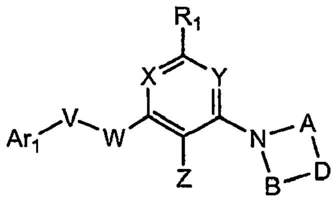

**(Ia)**

or a pharmaceutically acceptable salt, hydrate or solvate thereof,
wherein:

A and B are independently $C_{1-3}$ alkylene optionally substituted with 1 to 4 methyl groups;
D is O, S, S(O), $S(O)_2$, $CR_2R_3$ or N-$R_2$;

V is selected from the group consisting of $C_{1-3}$ alkylene, ethynylene and $C_{1-2}$ heteroalkylene wherein each are optionally substituted with 1 to 4 substituents selected from the group consisting of $C_{1-3}$ alkyl, $C_{1-4}$ alkoxy, carboxy, cyano, $C_{1-3}$ haloalkyl and halogen; or V is absent;
W is $NR_4$, O, S, S(O) or $S(O)_2$; or W is absent;

X is N or $CR_5$;
Y is N or $CR_6$;
Z is selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, $C_{1-2}$ alkylamino, $C_{2-4}$ dialkylamino, carbo-$C_{1-6}$-alkoxy, carboxamide, carboxy, cyano, $C_{4-8}$ diacylamino, $C_{2-6}$ dialkylcarboxamide, $C_{1-4}$ dialkylthiocarboxamide, $C_{2-6}$ dialkylsulfonamide, $C_{1-4}$ dialkylsulfonylamino, formyl, $C_{1-4}$ haloalkoxy, $C_{1-4}$ haloalkyl, $C_{1-4}$ haloalkylcarboxamide, $C_{1-4}$ haloalkylsulfinyl, $C_{1-4}$ haloalkylsulfonyl, $C_{1-4}$ haloalkylthio, halogen, aryl, heterocyclic, heteroaryl, hydroxyl, hydroxylamino, nitro and tetrazolyl, wherein $C_{1-8}$ alkyl and $C_{1-5}$ acyl are each optionally substituted with 1, 2, 3 or 4 groups selected from the group consisting of $C_{1-5}$ acyl, $C_{1-5}$ acyloxy, $C_{1-4}$ alkoxy, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, $C_{1-2}$ alkylamino, $C_{2-4}$ dialkylamino, carbo-$C_{1-6}$-alkoxy, carboxamide, carboxy, cyano, formyl, $C_{1-4}$ haloalkoxy, $C_{1-4}$ haloalkylsulfinyl, $C_{1-4}$ haloalkylsulfonyl, $C_{1-4}$ haloalkylthio, halogen, hydroxyl, hydroxylamino and nitro; or
Z is a group of Formula (A):

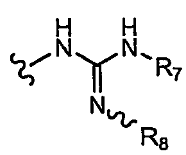

**(A)**

wherein:

$R_7$ is H, $C_{1-8}$ alkyl or $C_{3-6}$ cycloalkyl; and $R_8$ is H, nitro or nitrile;

$Ar_1$ is aryl or heteroaryl wherein each are optionally substituted with $R_9$-$R_{13}$;

$R_1$ is selected from the group consisting of H, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, $C_{1-4}$ alkylamino, $C_{2-8}$ dialkylamino, carboxamide, cyano, $C_{3-6}$ cycloalkyl, $C_{2-6}$ dialkylcarboxamide, $C_{2-6}$ dialkylsulfonamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio and hydroxyl;

$R_2$ is selected from the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, heteroaryl, hydroxyl and phenyl; and wherein $C_{1-8}$ alkyl, heteroaryl and phenyl are each optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl, $C_{3-6}$-cycloallcyl-$C_{1-3}$-alkylene, $C_{3-6}$-cycloalkyl-$C_{1-3}$-heteroalkylene, $C_{2-8}$ dialkylamino, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heterocyclic, hydroxyl, hydroxylamino and nitro; or

$R_2$ is -$Ar_2$-$Ar_3$ wherein $Ar_2$ and $Ar_3$ are independently aryl or heteroaryl each optionally substituted with 1 to 5 substituents selected from the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, hydroxyl and nitro; or

$R_2$ is a group of Formula (B):

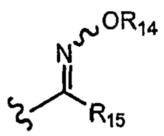

(B)

wherein:

$R_{14}$ is $C_{1-8}$ alkyl or $C_{3-6}$ cycloalkyl; and $R_{15}$ is F, Cl, Br or CN; or

$R_2$ is a group of Formula (**C**):

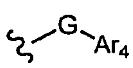

. (C)

wherein:

G is C=O, $CR_{16}R_{17}$, O, S, S(O), $S(O)_2$; where $R_{16}$ and $R_{17}$ are independently H or $C_{1-8}$ alkyl; and

$Ar_4$ is phenyl or heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro;
$R_3$ is H, $C_{1-8}$ alkyl, $C_{1-4}$ alkoxy, halogen or hydroxyl;

$R_4$ is H or $C_{1-8}$ alkyl;

$R_5$ and $R_6$ are independently H, $C_{1-8}$ alkyl or halogen;

$R_9$ is selected from the group consisting of $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylamino, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, arylsulfonyl, carbo-$C_{1-6}$-alkoxy, carboxamide, carboxy, cyano, $C_{3-6}$ cycloalkyl, $C_{2-6}$ dialkylamino, $C_{2-6}$ dialkylcarboxamide, $C_{2-6}$ dialkylsulfonamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, heterocyclic, heterocyclicsulfonyl, heteroaryl, hydroxyl, nitro, $C_{4-7}$ oxo-cycloalkyl, phenoxy, phenyl, sulfonamide and sulfonic acid, and wherein $C_{1-5}$ acyl, $C_{1-4}$ alkoxy, $C_{1-8}$ alkyl, $C_{1-4}$ alkylsulfonamide, alkylsulfonyl, arylsulfonyl, heteroaryl, phenoxy and phenyl are each optionally substituted with 1 to 5 substituents selected independently from the group consisting of $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, heteroaryl, heterocyclic, hydroxyl, nitro and phenyl; or

$R_9$ is a group of Formula (D):

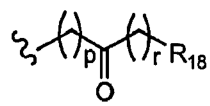

(D)

wherein:

"p" and "r" are independently 0,1,2 or 3; and

$R_{18}$ is H, $C_{1-5}$ acyl, $C_{2-6}$ alkenyl, $C_{1-8}$ alkyl, $C_{1-4}$ alkylcarboxamide, $C_{2-6}$ alkynyl, $C_{1-4}$ alkylsulfonamide, carbo-$C_{1-6}$-alkoxy, carboxamide, carboxy, cyano, $C_{3-6}$ cycloalkyl, $C_{2-6}$ dialkylcarboxamide, halogen, heteroaryl or phenyl, and wherein the heteroaryl and phenyl are each optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl; and

$R_{10}$-$R_{13}$ are independently selected form the group consisting of $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, hydroxyl and nitro; or two adjacent $R_{10}$-$R_{11}$ groups together with $Ar_1$ form a 5, 6 or 7 membered cycloalkyl, cycloalkenyl or heterocyclic group wherein the 5, 6 or 7 membered group is optionally substituted with halogen.

Some embodiments of the present invention include pharmaceutical compositions comprising at least one compound of the present invention and a pharmaceutically acceptable carrier.

Some embodiments of the present invention include methods for prophylaxis or treatment of a metabolic disorder in an individual comprising administering to the individual a therapeutically effective amount of a compound of the present invention or a pharmaceutical composition thereof.

Some embodiments of the present invention include methods of controlling or decreasing weight gain of an individual comprising administering to the individual a therapeutically effective amount of a compound of the present invention or pharmaceutical composition thereof.

Some embodiments of the present invention include methods of modulating a **RUP3** receptor comprising contacting the receptor with a compound of the present invention.

Some embodiments of the present invention include methods of modulating a **RUP3** receptor in an individual comprising contacting the receptor with a compound of the present invention. In some embodiments, the compound is an agonist. In some embodiments, the compound is an inverse agonist.

Some embodiments of the present invention include methods of modulating a **RUP3** receptor in an individual comprising contacting the receptor with a compound of the present invention wherein the modulation of the **RUP3** receptor is prophylaxis or treatment of a metabolic disorder.

Some embodiments of the present invention include methods of modulating a **RUP3** receptor in an individual comprising contacting the receptor with a compound of the present invention wherein the modulation of the **RUP3** receptor controls or reduces weight gain of the individual.

Some embodiments of the present invention include the use of compounds of the present invention for production of a medicament for use in prophylaxis or treatment of a metabolic disorder.

Some embodiments of the present invention include the use of compounds of the present invention for production of a medicament for use in controlling or decreasing weight gain in an individual.

One aspect of the present invention pertains to compounds of the present invention, as described herein, for use in methods of treatment of the human or animal body by therapy.

One aspect of the present invention pertains to compounds of the present invention, as described herein, for use in methods of prophylaxis or treatment of a metabolic disorder of the human or animal body by therapy.

In some embodiments, the metabolic disorder is type I, type II diabetes, inadequate glucose tolerance, insulin resistance, hyperglycemia, hyperlipidemia, hypertriglyceridemia, hypercholesterolemia, dyslipidemia, syndrome X or metabolic syndrome.

One aspect of the present invention pertains to methods of producing a pharmaceutical compositions comprising admixing at least one compound of the present invention and a pharmaceutically acceptable carrier.

## BRIEF DESCRIPTION OF THE DRAWINGS

**Figure 1A** shows RT-PCR analysis of **RUP3** expression in human tissues. A total of twenty-two (22) human tissues were analyzed.

**Figure 1B** shows the cDNA Dot-Blot analysis of **RUP3** expression in human tissues.

**Figure 1C** shows analysis of **RUP3** by RT-PCR with isolated huyman pancreatic islets of Langerhans.

**Figure 1D** shows analysis of **RUP3** expression with cDNAs of rat origin by RT-PCR.

**Figure 2A** shows a polyclonal anti-**RUP3** antibody prepared in Rabbits.

**Figure 2B** shows the expression of **RUP3** in insulin-producing β cells of pancreatic islets.

**Figure 3** shows functional activities **of RUP3** *In vitro.*

**Figure 4A** shows a **RUP3** RNA blot.

**Figure 4B** shows **RUP3** agonist, Compound **B84,** stimulates cAMP production in HIT cells, at a level comparable to that seen with the adenyl cyclase activator forskolin.

**Figure 4C** shows **RUP3** agonist, Compound **B84**, stimulates insulin secretion in HIT cells exposed to 15 mM glucose, at a level comparable to that seen with the adenylcyclase activator forskolin.

**Figure 4D** shows two **RUP3** compounds, Compounds **A48** and **A51** (at 10 μM concentration), and the enhanced glucose-dependent insulin release compared to control.

**Figure 5A** shows the *In vivo* effects of a **RUP3** agonist (Compound **B70)** on glucose homeostasis in mice and specifically the effect by a **RUP3** agonist in a dose-dependent manner on the lowering of blood glucose after glucose challenge.

**Figure 5B** shows the acute response of db mice to a **RUP 3** agonist and Ex-4. Figure 5B shows Compound **B70** and Ex-4 significantly reduces glucose levels compared to vehicle control.

**Figure 6** shows a representative scheme for the syntheses of compounds of the present invention.

## DEFINITIONS

The scientific literature that has evolved around receptors has adopted a number of terms to refer to ligands having various effects on receptors. For clarity and consistency, the following definitions will be used throughout this patent document.

**AGONISTS** shall mean moieties that activate the intracellular response when they bind to the receptor, or enhance GTP binding to membranes.

**AMINO ACID ABBREVIATIONS** used herein are set out in Table 1:

| TABLE 1 | | |
|---|---|---|
| ALANINE | ALA | A |
| ARGININE | ARG | R |
| ASPARAGINE | ASN | N |
| ASPARTIC ACID | ASP | D |
| CYSTEINE | CYS | C |

(continued)

| TABLE 1 | | |
|---|---|---|
| GLUTAMIC ACID | GLU | E |
| GLUTAMINE | GLN | Q |
| GLYCINE | GLY | G |
| HISTIDINE | HIS | H |
| ISOLEUCINE | ILE | I |
| LEUCINE | LEU | L |
| LYSINE | LYS | K |
| MMTIEONINE | MET | M |
| PHENYLALANINE | PHE | F |
| PROLINE | PRO | P |
| SERINE | SER | S |
| THREONINE | THR | T |
| TRYPTOPHAN | TRP | W |
| TYROSINE | TYR | Y |
| VALINE | VAL | V |

**CHEMICAL GROUP, MOIETY OR RADICAL:**

The term **"$C_{1-5}$ acyl"** denotes an alkyl radical attached to a carbonyl wherein the definition of alkyl has the same definition as described herein; some examples include formyl, acetyl, propionyl, butanoyl, *iso*-butanoyl, pentanoyl, hexanoyl, heptanoyl, and the like.

The term **"$C_{1-5}$ acyloxy"** denotes an acyl radical attached to an oxygen atom wherein acyl has the same definition has described herein; some examples include acetyloxy, propionyloxy, butanoyloxy, iso-butanoyloxy and the like.

The term **"$C_{2-6}$ alkenyl"** denotes a radical containing 2 to 6 carbons wherein at least one carbon-carbon double bond is present, some embodiments are 2 to 4 carbons, some embodiments are 2 to 3 carbons, and some embodiments have 2 carbons. Both E and Z isomers are embraced by the term **"alkenyl."** Furthermore, the term **"alkenyl"** includes di- and tri-alkenyls. Accordingly, if more than one double bond is present then the bonds may be all *E* or *Z* or a mixtures of E and Z. Examples of an alkenyl include vinyl, allyl, 2-butenyl, 3-butenyl, 2-pentenyl, 3-pentenyl, 4-pentenyl, 2-hexenyl, 3-hexenyl, 4-hexenyl, 5-hexanyl, 2,4-hexadienyl and the like.

The term **"$C_{1-4}$ alkoxy"** as used herein denotes a radical alkyl, as defined herein, attached directly to an oxygen atom. Example include methoxy, ethoxy, n-propoxy, iso-propoxy, n-butoxy, t-butoxy, iso-butoxy and the like.

The term **"$C_{1-8}$ alkyl"** denotes a straight or branched carbon radical containing 1 to 8 carbons, some embodiments are 1 to 6 carbons, some embodiments are 1 to 3 carbons, and some embodiments are 1 or 2 carbons. Examples of an alkyl include methyl, ethyl, n-propyl, iso-propyl, n-butyl, sec-butyl, t-butyl, amyl, t-amyl, n-pentyl and the like.

The term "**$C_{1-4}$ alkylcarboxamido"** denotes a single alkyl group attached to an amide, wherein alkyl has the same definition as found herein. The $C_{1-5}$ alkylcarboxamido may be represented by the following:

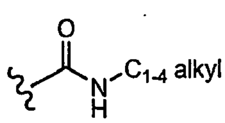

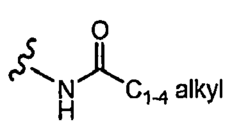

.

Examples include *N*-methylcarboxamide, *N*-ethylcarboxamide, *N*-(*iso*-propyl)carboxamide and the like.

The term **"$C_1$-$C_3$ alkylene"** refers to a divalent straight carbon group, such as, $-CH_2-$, $-CH_2CH_2-$, $-CH_2CH_2CH_2-$.

The term **"$C_{1-4}$ alkylsulfinyl"** denotes an alkyl radical attached to a sulfoxide radical of the formula: -S(O)- wherein the alkyl radical has the same definition as described herein. Examples include methylsulfinyl, ethylsulfinyl and

the like.

The term **"$C_{1-4}$ alkylsulfonamide"** refers to the groups

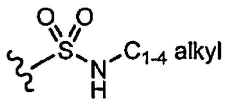
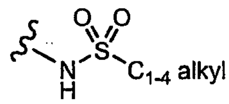

.

The term **"$C_{1-4}$ alkylsulfonyl"** denotes an alkyl radical attached to a sulfone radical of the formula: $-S(O)_2-$ wherein the alkyl radical has the same definition as described herein. Examples include methylsulfonyl, ethylsulfonyl and the like.

The term **"$C_{1-4}$ alkylthio"** denotes an alkyl radical attached to a sulfide of the formula: -S- wherein the alkyl radical has the same definition as described herein.

Examples include methylsulfanyl (i.e., $CH_3S$-), ethylsulfanyl, isopropylsulfanyl and the like.

The term **"$C_{1-4}$ alkylthiocarboxamide"** denotes a thioamide of the following formulae:

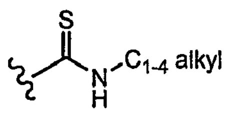
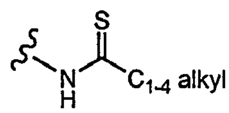

.

The term **"$C_{1-4}$ alkylthioureyl"** denotes the group of the formula: -NC(S)N- wherein one are both of the nitrogens are substituted with the same or different alkyl group and alkyl has the same definition as described herein. Examples of an alkylthioureyl include, $CH_3NHC(O)NH$-, $NH_2C(O)NCH_3$-, $(CH_3)_2N(O)NH$-, $(CH_3)_2N(O)NH$-, $(CH_3)_2N(O)NCH_3$-, $CH_3CH_2NHC(O)NH$-, $CH_3CH_2NHC(O)NCH_3$-, and the like.

The term **"$C_{1-4}$ alkylureyl"** denotes the group of the formula: -NC(O)N-wherein one are both of the nitrogens are substituted with the same or different alkyl group wherein alkyl has the same definition as described herein. Examples of an alkylureyl include, $CH_3NHC(O)NH$-, $NH_2C(O)NCH_3$-, $(CH_3)_2N(O)NH$-, $(CH_3)_2N(O)NH$-, $(CH_3)_2N(O)NCH_3$-, $CH_3CH_2NHC(O)NH$-, $CH_3CH_2NHC(O)NCH_3$-, and the like.

The term **"$C_{2-6}$ alkynyl"** denotes a radical containing 2 to 6 carbons and at least one carbon-carbon triple bond, some embodiments are 2 to 4 carbons, some embodiments are 2 to 3 carbons, and some embodiments have 2 carbons. Examples of an alkynyl include ethynyl, ethynyl, 1-propynyl, 2-propynyl, 1-butynyl, 2-butynyl, 3-butynyl, 1-pentynyl, 2-pentynyl, 3-pentynyl, 4-pentynyl,1-hexynyl, 2-hexynyl, 3-hexynyl, 4-hexynyl, 5-hexynyl and the like. The term **"alkynyl"** includes di- and tri-ynes.

The term **"amino"** denotes the group $-NH_2$.

The term "$C_{1-4}$ **alkylamino"** denotes one alkyl radical attached to an amino radical wherein the alkyl radical has the same meaning as described herein. Some examples include methylamino, ethylamino, propylamino and the like.

The term **"aryl"** denotes an aromatic ring radical containing 6 to 10 ring carbons. Examples include phenyl and naphthyl.

The term **"arylalkyl"** defines a $C_1$-$C_4$ alkylene, such as $-CH_2-$, $-CH_2CH_2-$ and the like, which is further substituted with an aryl group. Examples of an "arylalkyl" include benzyl, phenethylene and the like.

The term **"arylcarboxamido"** denotes a single aryl group attached to the amine of an amide, wherein aryl has the same definition as found herein. The example is *N*-phenylcarboxamide.

The term **"arylureyl"** denotes the group -NC(O)N- where one of the nitrogens are substituted with an aryl.

The term **"benzyl"** denotes the group $-CH_2C_6H_5$.

The term **"carbo-$C_{1-6}$-alkoxy"** refers to an alkyl ester of a carboxylic acid, wherein the alkyl group is $C_{1-6}$. Examples include carbomethoxy, carboethoxy, carboisopropoxy and the like.

The term **"carboxamide"** refers to the group $-CONH_2$.

The term **"carboxy"** or "**carboxyl**" denotes the group $-CO_2H$; also referred to as a carboxylic acid.

The term "**cyano**" denotes the group -CN.

The term "**$C_{3-7}$ cycloalkenyl**" denotes a non-aromatic ring radical containing 3 to 6 ring carbons and at least one double bond; some embodiments contain 3 to 5 carbons; some embodiments contain 3 to 4 carbons. Examples include cyclopropenyl, cyclobutenyl, cyclopentenyl, cyclopentenyl, cyclohexenyl, and the like.

The term **"$C_{3-7}$ cycloalkyl"** denotes a saturated ring radical containing 3 to 6 carbons; some embodiments contain 3 to 5 carbons; some embodiments contain 3 to 4 carbons. Examples include cyclopropyl, cyclobutyl, cyclopentyl,

cyclohexyl, and the like.

The term **"$C_{3-6}$ cycloalkyl-$C_{1-3}$-alkylene"** denotes a saturated ring radical containing 3 to 6 carbons bonded to a $C_{1-3}$-alkylene as described herein; some embodiments contain 3 to 5 carbons; some embodiments contain 3 to 4 carbons.
Examples include cyclopropyl-$CH_2$-, cyclopropyl-$CH_2CH_2$-, cyclobutyl-$CH_2$-, cyclopentyl-$CH_2$-, cyclohexyl-$CH_2$-, and the like.

The term **"$C_{4-8}$ diacylamino"** denotes an amino group bonded with two acyl groups defined herein wherein the acyl groups may be the same or different, such as:

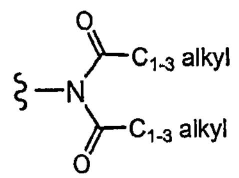

Represented dialkylamino groups include diacetylamino, dipropionylamino, acetylpropionylamino and the like.

The term **"$C_{2-6}$ dialkylamino"** denotes an amino substituted with two of the same or different alkyl radicals wherein alkyl radical has the same definition as described herein. Some embodiments are $C_{2-4}$ dialkylamino groups. Some examples include dimethylamino, methylethylamino, diethylamino and the like.

The term **"$C_{1-4}$ dialkylcarboxamido"** or **"$C_{1-4}$ dialkylcarboxamide"denotes** two alkyl radicals, that are the same or different, attached to an amide group, wherein alkyl has the same definition as described herein. A $C_{1-4}$ dialkylcarboxamido may be represented by the following groups:

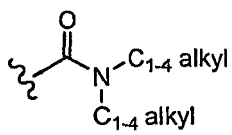 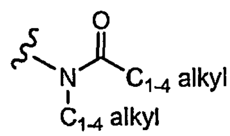 .

Examples of a dialkylcarboxamide include *N,N*-dimethylcarboxamide, N-methyl-N-ethylcarboxamide and the like.

The term **"$C_{2-6}$ dialkylsulfonamide"** refers to one of the following groups shown below:

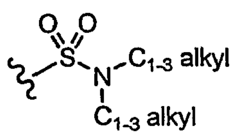 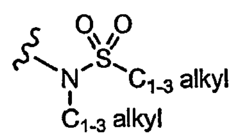 .

The term **"$C_{1-4}$ dialkylthiocarboxamido"** or **"$C_{1-4}$ dialkylthiocarboxamide"** denotes two alkyl radicals, that are the same or different, attached to a thioamide group, wherein alkyl has the same definition as described herein. A $C_{1-4}$ dialkylthiocarboxamido may be represented by the following groups:

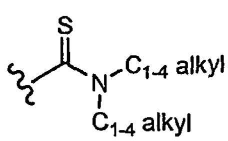 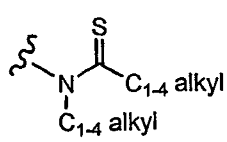 .

Examples of a dialkylthiocarboxamide include *N,N*-dimethylthiocarboxamide, *N*-methyl-*N*-ethylthiocarboxamide and the like.

The term **"$C_{1-4}$dialkylsulfonylamino"** refers to an amino group bonded with two $C_{1-4}$ alkylsulfonyl groups as defined herein.

The term "ethynylene" refers to the carbon-carbon triple bond group as represented below:

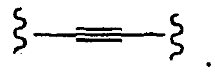

The term **"formyl"** refers to the group -CHO.

The term **"$C_{1-4}$ haloalkoxy"** denotes a haloalkyl, as defined herein, that is directly attached to an oxygen to form a difluoromethoxy, trifluoromethoxy, 2,2,2-trifluoroethoxy, pentafluoroethoxy and the like.

The term **"$C_{1-4}$ haloalkyl"** denotes an alkyl group, defined herein, wherein the alkyl is substituted with one halogen up to fully substituted represented by the formula $C_nF_{2n+1}$; when more than one halogen is present they may be the same or different and selected from F, Cl, Br or I. Some embodiments are 1 to 3 carbons. Examples include fluoromethyl, difluoromethyl, trifluoromethyl, chlorodifluoromethyl, 2,2,2-trifluoroethyl, pentafluoroethyl and the like.

The term "$C_{1-4}$ **haloalkylcarboxamide"** denotes an alkylcarboxamide group, defined herein, wherein the alkyl is substituted with one halogen up to fully substituted represented by the formula $C_nF_{2n+1}$ and "n" is 1, 2, 3 or 4. When more than one halogen is present they may be the same or different and selected from F, Cl, Br or I. Examples include 2-fluoroacetyl, 2,2-difluoroacetyl, 2,2,2-trifluoroacetyl, 2-chloro-2,2-difluoroacetyl, 3,3,3-trifluoropropionyl, 2,2,3,3,3-pentafluoropropionyl and the like.

The term **"$C_{1-4}$ haloalkylsulfinyl"** denotes a haloalkyl radical attached to a sulfoxide of the formula: -S(O)- wherein the alkyl radical has the same definition as described herein. Examples include trifluoromethylsulfinyl, 2,2,2-trifluoroethylsulfinyl, 2,2-difluoroethylsulfinyl and the like.

The term **"$C_{1-4}$ haloalkylsulfonyl"** denotes a haloalkyl attached to a sulfone of the formula: $-S(O)_2-$ wherein haloalkyl has the same definition as described herein.
Examples include trifluoromethylsulfonyl, 2,2,2-trifluoroethylsulfonyl, 2,2-difluoroethylsulfonyl and the like.

The term **"$C_{1-4}$ haloalkylthio"** denotes an alkylthio radical substituted with one or more halogens. Examples include trifluoromethylthio, 1,1-difluoroethylthio, 2,2,2-trifluoroethylthio and the like.

The term **"halogen"** or **"halo"** denotes to a fluoro, chloro, bromo or iodo group.

The term **"$C_{1-2}$ heteroalkylene"** refers to a $C_{1-2}$ alkylene bonded to a heteroatom selected from O, S, S(O), $S(O)_2$ and NH. Some represented examples include the groups of the following formulae:

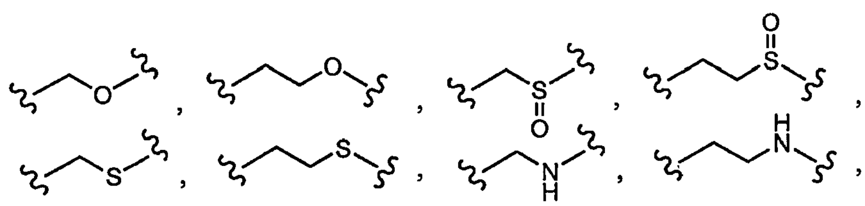

and the like

The term **"heteroaryl"** denotes an aromatic ring system that may be a single ring, two fused rings or three fused rings containing carbons and at least one ring heteroatom selected from O, S and N. Examples of heteroaryl groups include, but not limited to, pyridyl, benzofuranyl, pyrazinyl, pyridazinyl, pyrimidinyl, triazinyl, quinoline, benzoxazole, benzothiazole, 1*H*-benzimidazole, isoquinoline, quinazoline, quinoxaline, 1H-imidazolyl, [1,2,4]triazolyl, [1,2,3] triazolyl, [1,2,4]triazolyl, pyrrolyl, pyrazolyl, 1H-pyrazolyl, imidazolyl, oxazolyl, oxazolyl, [1,3,4]oxadiazolyl, [1,3,4]thiadiazolyl, [1,2,4]oxadiazolyl; [1,2,4]thiadiazolyl, tetrazolyl, 1,3-dioxo-1,3-dihydro-isoindolyl, [1,2,3]thiadiazol-4-yl and the like.

The term **"heterocyclic"** denotes a non-aromatic carbon ring (i.e., cycloalkyl or cycloalkenyl as defined herein) wherein one, two or three ring carbons are replaced a heteroatom, such as, O, S, N, wherein the N can be optionally substituted with H, $C_{1-4}$ acyl or $C_{1-4}$ alkyl, and ring carbon atoms optionally substituted with oxo or a thiooxo thus forming a carbonyl or thiocarbonyl group. The heterocyclic group is a 3-, 4-, 5-, 6- or 7-membered containing ring. Examples of a heterocyclic group include but not limited to aziridin-1-yl, aziridin-2-yl, azetidin-1-yl, azetidin-2-yl, azetidin-3-yl, piperidin-1-yl, piperidin-4-yl, morpholin-4-yl, piperzin-1-yl, piperzin-4-yl, pyrrolidin-1-yl, pyrrolidin-3-yl, [1,3]-dioxolan-2-yl and the like. Additional examples of heterocyclic groups are shown in Tables 2B, 2C, 2D, 2E, 2F and 2G, *infra.*

The term **"heterocycliccarboxamido"** denotes a heterocyclic group with a ring nitrogen where the ring nitrogen is bonded directly to the carbonyl forming an amide. Examples include:

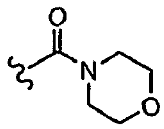, 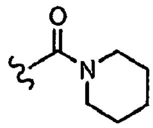, 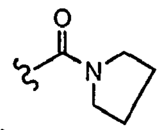

and the like.

The term **"heterocyclicsulfonyl"** denotes a heterocyclic group with a ring nitrogen where the ring nitrogen is bonded directly to an $SO_2$ group forming an sulfonamide, Examples include:

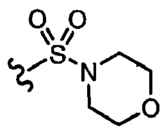, 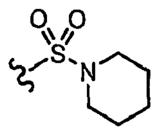, 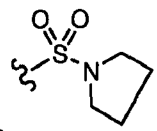

and the like.

The term **"hydroxyl"** refers to the group -OH.

The term **"hydroxylamino"** refers to the group -NHOH.

The term **"nitro"** refers to the group $-NO_2$.

The term **"$C_{4-7}$ oxo-cycloalkyl"** refers to a $C_{4-7}$ cycloalkyl, as defined herein,: wherein one of the ring carbons is replaced with a carbonyl. Examples of $C_{4-7}$ oxo-cycloalkyl include but are not limited to: 2-oxo-cyclobutyl, 3-oxo-cyclobutyl, 3-oxo-cyclopentyl, 4-oxo-cyclohexyl, and the like and represented the following structures respectively:

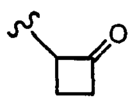, 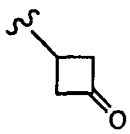, 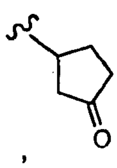, 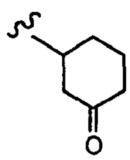, 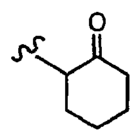 and 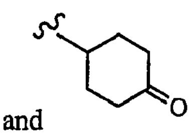

The term **"perfluoroalkyl"** denotes the group of the formula $-C_nF_{2n+1}$; stated differently, a perfluoroalkyl is an alkyl as defined herein herein wherein the alkyl is fully substituted with fluorine atoms and is therefore considered a subset of haloalkyl.

Examples of perfluoroalkyls include $CF_3$, $CF_2CF_3$, $CF_2CF_2CF_3$, $CF(CF_3)_2$, $CF_2CF_2CF_2CF_3$, $CF_2CF(CF_3)_2$, $CF(CF_3)CF_2CF_3$ and the like.

The term **"phenoxy"** refers to the group $C_6H_5O$-.

The term **"phenyl"** refers to the group $C_6H_5$-.

The term **"sulfonic acid"** refers to the group $-SO_3H$.

The term "**tetrazolyl**" refers to the five membered heteroaryl of the following formulae:

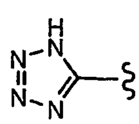, 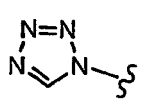.

In some embodiments, the tetrazolyl group is further substituted at either the 1 or 5 position resepectively.

The term **"thiol"** denotes the group -SH.

**CODON** shall mean a grouping of three nucleotides (or equivalents to nucleotides) which generally comprise a nucleoside (adenosine (A), guanosine (G), cytidine (C), uridine (U) and thymidine (T)) coupled to a phosphate group and which, when translated, encodes an amino acid.

**COMPOSITION** shall mean a material comprising at least two compounds or two components; for example, and not limitation, a Pharmaceutical Composition is a Composition.

**COMPOUND EFFICACY** shall mean a measurement of the ability of a compound to inhibit or stimulate receptor

functionality, as opposed to receptor binding affinity.

**CONSTITUTIVELY ACTIVATED RECEPTOR** shall mean a receptor subject to constitutive receptor activation.

**CONSTITUTIVE RECEPTOR ACTIVATION** shall mean stabilization of a receptor in the active state by means other than binding of the receptor with its endogenous ligand or a chemical equivalent thereof.

**CONTACT** or **CONTACTING** shall mean bringing at least two moieties together, whether in an in vitro system or an in vivo system.

**ENDOGENOUS** shall mean a material that a mammal naturally produces. ENDOGENOUS in reference to, for example and not limitation, the term "receptor" shall mean that which is naturally produced by a mammal (for example, and not limitation, a human) or a virus.

In contrast, the term **NON-ENDOGENOUS** in this context shall mean that which is not naturally produced by a mammal (for example, and not limitation, a human) or a virus. For example, and not limitation, a receptor which is not constitutively active in its endogenous form, but when manipulated becomes constitutively active, is most preferably referred to herein as a "non-endogenous, constitutively activated receptor." Both terms can be utilized to describe both "in vivo" and "in vitro" systems. For example, and not a limitation, in a screening approach, the endogenous or non-endogenous receptor may be in reference to an in vitro screening system. As a further example and not limitation, where the genome of a mammal has been manipulated to include a non-endogenous constitutively activated receptor, screening of a candidate compound by means of an in vivo system is viable.

**INDIVIDUAL** as used herein refers to any animal, including mammals, preferably mice, rats, other rodents, rabbits, dogs, cats, swine, cattle, sheep, horses, or primates, and most preferably humans.

**INHIBIT** or **INHIBITING,** in relationship to the term "response" shall mean that a response is decreased or prevented in the presence of a compound as opposed to in the absence of the compound.

**INVERSE AGONISTS** shall mean moieties that bind the endogenous form of the receptor or to the constitutively activated form of the receptor, and which inhibit the baseline intracellular response initiated by the active form of the receptor below the normal base level of activity which is observed in the absence of agonists or partial agonists, or decrease GTP binding to membranes. Preferably, the baseline intracellular response is inhibited in the presence of the inverse agonist by at least 30%, more preferably by at least 50%, and most preferably by at least 75%, as compared with the baseline response in the absence of the inverse agonist.

**LIGAND** shall mean an endogenous, naturally occurring molecule specific for an endogenous, naturally occurring receptor.

As used herein, the terms **MODULATE** or **MODULATING** shall mean to refer to an increase or decrease in the amount, quality, response or effect of a particular activity, function or molecule.

**PHARMACEUTICAL COMPOSITION** shall mean a composition comprising at least one active ingredient, whereby the composition is amenable to investigation for a specified, efficacious outcome in a mammal (for example, and not limitation, a human). Those of ordinary skill in the art will understand and appreciate the techniques appropriate for determining whether an active ingredient has a desired efficacious outcome based upon the needs of the artisan.

## DETAILED DESCRIPTION

### Compound Of The Present Invention

One aspect of the present invention pertains to certain 1,2,3-trisubstituted aryl and heteroaryl derivatives as shown in Formula **(Ia):**

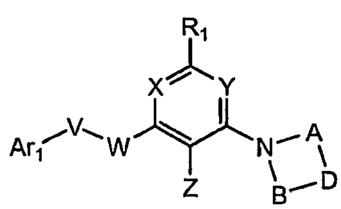

**(Ia)**

or a pharmaceutically acceptable salt, hydrate or solvate thereof, wherein A, B, D, V, W, X, Y, Z $Ar_1$, and $R_1$ are as described herein, *supra* and *infra.*

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable

subcombination.

One aspect of the present invention encompasses N-oxides of 1,2,3-trisubstituted aryl and heteroaryl derivatives of Formula **(Ia).**

One aspect of the present invention encompasses 1,2,3-trisubstituted aryl and heteroaryl derivatives as shown in Formula **(Ia)** wherein W is $NR_4$ and compounds may be represented by Formula **(Ib)** as shown below:

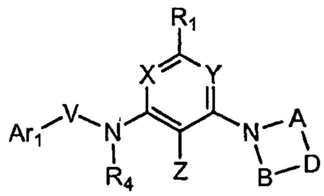

**(Ib)**

wherein each variable in Formula **(Ib)** has the same meaning as described herein. In some embodiments, $R_4$ is H. In some embodiments, $R_4$ is $CH_3$ or $CH_2CH_3$.

One aspect of the present invention encompasses 1,2,3-trisubstituted aryl and heteroaryl derivatives as shown in Formula **(Ia)** wherein W is O, (i.e., an oxygen atom) and compounds may be represented by Formula **(Ic)** as shown below:

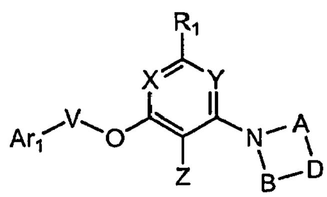

**(Ic)**

wherein each variable in Formula (**Ic**) has the same meaning as described herein.

One aspect of the present invention encompasses 1,2,3-trisubstituted aryl and heteroaryl derivatives as shown in Formula (**Ia**) wherein W is S, S(O) or $S(O)_2$ and compounds may be represented by Formulae (**Id**), (**Ie**) and (**If**) respectively as shown below:

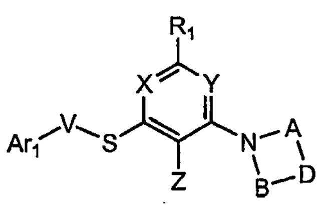

**(Id)**

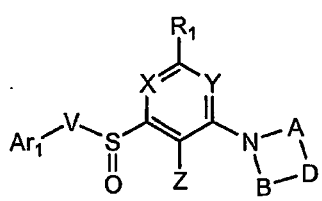

**(Ie)**

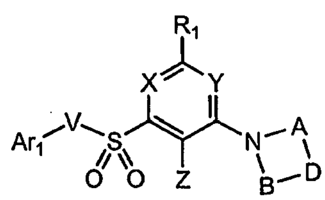

**(If)**

wherein each variable in Formulae (**Id**), (**Ie**) and (**If**) has the same meaning as described herein.

One aspect of the present invention encompasses 1,2,3-trisubstituted aryl and heteroaryl derivatives as shown in Formula (**Ia**) wherein W is absent and compounds may be represented by Formula (**Ig**) as shown below:

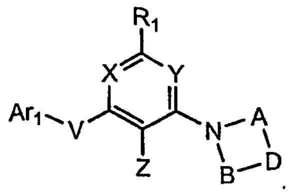

(Ig)

wherein each variable in Formula **(Ig)** has the same meaning as described herein. In some embodiments, compounds of the present invention are of Formula **(Ig)** wherein V is absent and accordingly these compounds may be represented by Formula **(Ih)** as shown below:

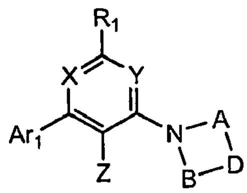

(Ih)

wherein each variable in Formula (**Ih**) has the same meaning as described herein.

One aspect of the present invention encompasses 1,2,3-trisubstituted aryl and heteroaryl derivatives as shown in Formula **(Ia)** wherein W is absent and V is ethynylene. Compounds may be represented by Formula **(Ii)** as shown below:

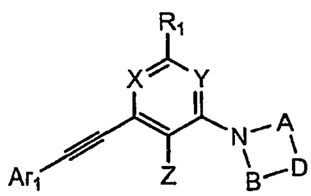

(Ii)

wherein each variable in Formula **(Ii)** has the same meaning as described herein.

In some embodiments, V is $C_{1-3}$ alkylene optionally substituted with 1 to 4 substituents selected from the group consisting of $C_{1-3}$ alkyl, $C_{1-4}$ alkoxy and halogen. In some embodiments, V is a methylene group (i.e., $-CH_2-$). In some embodiments, V is an ethylene group (i.e., $-CH_2CH_2-$). In some embodiments, V is a methylene and W is an oxygen atom. In some embodiments, V is methylene and W is a $NR_4$ group. In some embodiments, V is methylene and W is a NH group. In some embodiments, V is ethylene and W is an oxygen atom. In some embodiments, V is ethylene and W is a $NR_4$ group. In some embodiments, V is ethylene and W is a NH group.

In some embodiments, V is $C_{1-2}$ heteroalkylene optionally substituted with 1 to 4 substituents selected from the group consisting of $C_{1-3}$alkyl, $C_{1-4}$ alkoxy and halogen. In some embodiments, V is $-OCH_2CH_2-$. In some embodiments, V is $-OCH_2CH_2-$ and W is an oxygen atom and may be represented by the formula: $-OCH_2CH_2O-$. In some embodiments, V is $-OCH_2CH_2-$ and W is a NH group and may be represented by the formula: $-OCH_2CH_2NH-$.

In some embodiments, V is absent and may be represented by Formula **(Ij)** as shown below:

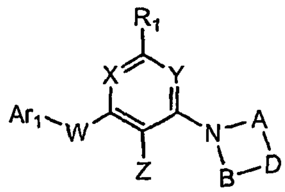

(Ij)

wherein each variable in Formula (**Ij**) has the same meaning as described herein.

In some embodiments, A and B are both methylene wherein A and B are optionally substituted with 1 to 2 methyl groups and therefore form a four-membered nitrogen containing ring. In some embodiments, compounds of the invention may be represented by Formula **(Ik)** as shown below:

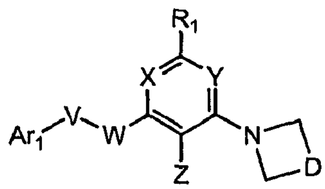

(Ik)

wherein each variable in Formula **(Ik)** has the same meaning as described herein. In some embodiments, D is $-CHR_2-$.

In some embodiments, A is ethylene and B is methylene wherein A is optionally substituted with 1 to 4 methyl groups and B is optionally substituted with 1 to 2 methyl groups. In some embodiments, compounds of the invention may be represented by Formula **(Im)** as shown below:

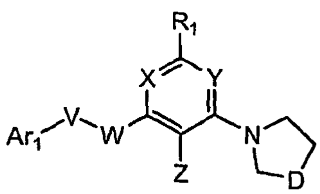

(Im)

wherein each variable in Formula (**Im**) has the same meaning as described herein. In some embodiments, D is $-CHR_2-$. In some embodiments, $R_2$ is $C_{1-4}$ alkylsulfonyl.

In some embodiments, A is propylene and B is methylene wherein A is optionally substituted with 1 to 4 methyl groups and B is optionally substituted with 1 to 2 methyl groups. In some embodiments, compounds of the invention may be represented by Formula (In) as shown below:

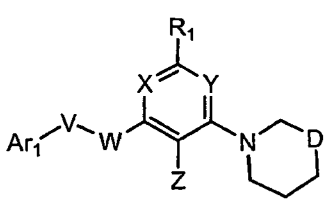

(In)

wherein each variable in Formula **(In)** has the same meaning as described herein. In some embodiments, D is $-CHR_2-$.

In some embodiments, A and B are both ethylene wherein A and B are optionally substituted with 1 to 4 methyl

groups. In some embodiments, compounds of the invention may be represented by Formula **(Io)** as shown below:

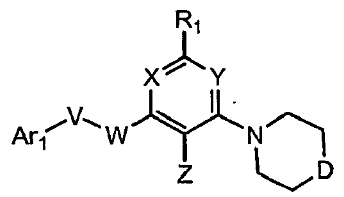

**(Io)**

wherein each variable in Formula (**Io**) has the same meaning as described herein. In some embodiments, D is $-CHR_2-$.

In some embodiments, A is propylene and B is ethylene wherein A and B are optionally substituted with 1 to 4 methyl groups. In some embodiments, compounds of the invention may be represented by Formula **(Ip)** as shown below:

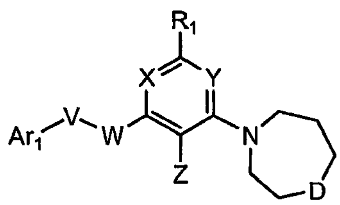

**(Ip)**

wherein each variable in Formula **(Ip)** has the same meaning as described herein. In some embodiments, D is $-CHR_2-$.

In some embodiments, A and B are both propylene wherein A and B are optionally substituted with 1 to 4 methyl groups. In some embodiments, compounds of the invention may be represented by Formula **(Iq)** as shown below:

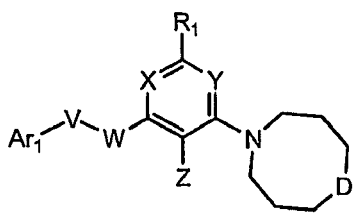

**(Iq)**

wherein each variable in Formula (**Iq**) has the same meaning as described herein. In some embodiments, D is $-CHR_2-$.

In some embodiments, D is O, S, S(O) or $S(O)_2$. In some embodiments, D is S, S(O) or $S(O)_2$; and A and B are independently optionally substituted with 1 or 2 methyl groups. In some embodiments, A and B are ethylene groups. In some embodiments, A and B are ethylene groups substituted with 2 methyl groups and D is an oxygen atom (i.e., forming a 2,6-dimethyl-morpholin-4-yl group).

In some embodiments, D is $CR_2R_3$.

In some embodiments, $R_2$ is selected from the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxyl, $C_{3-6}$ cycloalkyl, $C_{1-4}$ haloalkoxy, $C_{1-4}$ haloalkyl, halogen and hydroxyl.

In some embodiments, $R_2$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $OC(O)CH_3$, $OC(O)CH_2CH_3$, $OC(O)CH_2CH_2CH_3$, $OCH_3$, $OCH_2CH_3$, $OCH_2CH_2CH_3$, $OCH(CH_3)_2$, $OCH_2(CH_2)_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, $CH_2(CH_2)_3CH_3$, $C(O)NHCH_3$, $C(O)NHCH_2CH_3$, $C(O)NHCH_2CH_2CH_3$, $C(O)NHCH(CH_3)_2$, $C(O)NHCH_2(CH_2)_2CH_3$, $CO_2CH_3$, $CO_2CH_2CH_3$, $CO_2CH_2CH_2CH_3$, $CO_2CH(CH_3)_2$ and $CO_2CH_2(CH_2)_2CH_3$.

In some embodiments, $R_2$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $OC(O)CH_3$, $OC(O)CH_2CH_3$, $OC(O)CH_2CH_2CH_3$, $OCH_3$, $OCH_2CH_3$, $OCH_2CH_2CH_3$, $OCH(CH_3)_2$, $OCH_2(CH_2)_2CH_3$, $CH_3$, $CH_2CH_3$, $CH_2CH_2CH_3$, $CH(CH_3)_2$, $CH(CH_3)(CH_2CH_3)$, $CH_2$

$(CH_2)_2CH_3$, $CH_2(CH_2)_3CH_3$, $C(O)NH_2$, $CO_2CH_3$, $CO_2CH_2CH_3$, $CO_2CH_2CH_2CH_3$, $CO_2CH(CH_3)_2$, $CO_2CH_2(CH_2)_2CH_3$, and $CO_2H$.

In some embodiments, $R_2$ is selected from the group consisting of $SCH_3$, $SCH_2CH_3$, $SCH_2CH_2CH_3$, $SCH(CH_3)_2$, $SCH_2(CH_2)_2CH_3$, $S(O)CH_3$, $S(O)CH_2CH_3$, $S(O)CH_2CH_2CH_3$, $S(O)CH(CH_3)_2$, $S(O)CH_2(CH_2)_2CH_3$, $S(O)_2CH_3$, $S(O)_2CH_2CH_3$, $S(O)_2CH_2CH_2CH_3$, $S(O)_2CH(CH_3)_2$, $S(O)_2CH_2(CH_2)_2CH_3$, cyclopropyl, cyclobutyl, cyclopentyl, cyclohexyl, $OCF_3$, $OCHF_2$, $CF_3$, $CHF_2$ and F.

In some embodiments, $R_2$ is selected from the group consisting of $S(O)_2CH_3$, $S(O)_2CH_2CH_3$, $S(O)_2CH_2CH_2CH_3$, $S(O)_2CH(CH_3)_2$, $S(O)_2CH_2(CH_2)_2CH_3$, cyclopropyl, cyclobutyl, cyclopentyl, cyclohexyl, hydroxyl, and F.

In some embodiments, $R_2$ is $C_{1-8}$ alkyl, or heteroaryl each optionally substituted with 1 to 5 substituents selected from the group consisting of $C_{1-5}$ acyloxy, $C_{1-4}$ alkoxy, $C_{1-8}$ alkyl, $C_{1-4}$ alkylsulfonyl, carbo-$C_{1-6}$-alkoxy, carboxamide, carboxy, $C_{3-6}$-cycloalkyl, $C_{3-6}$-cycloalkyl-$C_{1-3}$-alkylene, $C_{3-6}$-cyloalkyl-$C_{1-3}$-heteroalkylene, and hydroxyl.

In some embodiments, $R_2$ is selected from the group consisting of $CH_2OCH_3$, $CH_2CH_2OCH_3$, $CH_2OCH_2CH_3$, $CH_2OCH_2CH_2CH_3$, $CH_2CH_2OCH_2CH_3$, $CH_2CH_2OCH_2CH_2CH_3$, $CH_2OCH(CH_3)_2$, $CH_2OCH_2CH(CH_3)_2$, $CH_2CO_2H$, $CH_2CH_2CO_2H$, $CH_2OH$, $CH_2CH_2OH$ and $CH_2CH_2CH_2OH$.

In some embodiments, $R_2$ is $C_{1-8}$ alkyl, heteroaryl or phenyl each optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-8}$ dialkylamino, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heterocyclic, hydroxyl, hydroxylamino and nitro.

In some embodiments, $R_2$ is selected from the group consisting of $CH_2OCH_3$, $CH_2CH_2OCH_3$, $CH_2OCH_2CH_3$, $CH_2OCH_2CH_2CH_3$, $CH_2CH_2OCH_2CH_3$, $CH_2CH_2OCH_2CH_2CH_3$, $CH_2OCH(CH_3)_2$, $CH_2OCH_2CH(CH_3)_2$, $CH_2CO_2H$, $CH_2CH_2CO_2H$, $CH_2OH$, $CH_2CH_2OH$ and $CH_2CH_2CH_2OH$.

In some embodiments, $R_2$ is selected from the group consisting of $CH_2SCH_3$, $CH_2SCH_2CH_3$, $CH_2SCH_2CH_2CH_3$, $CH_2SCH(CH_3)_2$, $CH_2SCH_2(CH_2)_2CH_3$, $CH_2CH_2SCH_3$, $CH_2CH_2SCH_2CH_3$, $CH_2CH_2SCH_2CH_2CH_3$, $CH_2CH_2SCH(CH_3)_2$, $CH_2CH_2SCH_2(CH_2)_2CH_3$, $CH_2S(O)CH_3$, $CH_2S(O)CH_2CH_3$, $CH_2S(O)CH_2CH_2CH_3$, $CH_2S(O)CH(CH_3)_2$, $CH_2S(O)CH_2(CH_2)_2CH_3$, $CH_2CH_2S(O)CH_3$, $CH_2CH_2S(O)CH_2CH_3$, $CH_2CH_2S(O)CH_2CH_2CH_3$, $CH_2CH_2S(O)CH(CH_3)_2$, $CH_2CH_2S(O)CH_2(CH_2)_2CH_3$, $CH_2S(O)_2CH_3$, $CH_2S(O)_2CH_2CH_3$, $CH_2S(O)_2CH_2CH_2CH_3$, $CH_2S(O)_2CH(CH_3)_2$, $CH_2S(O)_2CH_2(CH_2)_2CH_3$, $CH_2CH_2S(O)_2CH_3$, $CH_2CH_2S(O)_2CH_2CH_3$, $CH_2CH_2S(O)_2CH_2CH_2CH_3$, $CH_2CH_2S(O)_2CH(CH_3)_2$ and $CH_2CH_2S(O)_2CH_2(CH_2)_2CH_3$.

In some embodiments, $R_2$ is selected from the group consisting of $CH_2OCH_2$-cyclopropyl, $CH_2OCH_2$-cyclobutyl, $CH_2OCH_2$-cyclopentyl, $CH_2OCH_2$-cyclohexyl, $CH_2OCH_2CH_2$-cyclopropyl, $CH_2OCH_2CH_2$-cyclobutyl, $CH_2OCH_2CH_2$-cyclopentyl, $CH_2OCH_2CH_2$-cyclohexyl, $CH_2CH_2OCH_2$-cyclopropyl, $CH_2CH_2OCH_2$-cyclobutyl, $CH_2CH_2OCH_2$-cyclopentyl, $CH_2CH_2OCH_2$-cyclohexyl, $CH_2CH_2OCH_2CH_2$-cyclopropyl, $CH_2CH_2OCH_2CH_2$-cyclobutyl, $CH_2CH_2OCH_2CH_2$-cyclopentyl and $CH_2CH_2OCH_2CH_2$-cyclohexyl.

In some embodiments, $R_2$ is selected from the group consisting of 1,2,4-oxadiazol-3-yl, 1,2,4-oxadiazol-5-yl, 1,3,4-oxadiazol-2-yl, 1,2,4-triazol-5-yl and 1,2,4-triazol-1-yl, 3-methyl-1,2,4-oxadiazol-5-yl, 3-methyl-1,2,4-oxadiazol-5-yl, 3-ethyl-1,2,4-oxadiazol-5-yl, 5-ethyl-1,2,4-oxadiazol-3-yl, 5-methyl-1,3,4-oxadiazol-2-yl, 5-ethyl-1,3,4-oxadiazol-2-yl, 3-methyl-1,2,4-triazol-5-yl, 3-ethyl-1,2,4-triazol-5-yl, 3-methyl-1,2,4-triazol-1-yl, 3-ethyl-1,2,4-triazol-1-yl, 5-methyl-1,2,4-triazol-1-yl and 5-ethyl-1,2,4-triazol-1-yl.

In some embodiments, $R_2$ is selected from the group consisting of 1,2,4-oxadiazol-3-yl, 1,2,4-oxadiazol-5-yl, 1,3,4-oxadiazol-2-yl, 3-methyl-1,2,4-oxadiazol-5-yl, 3-ethyl-1,2,4-oxadiazol-5-yl, 3-isopropyl-1,2,4-oxadiazol-5-yl, 3-propyl-1,2,4-oxadiazol-5-yl, 3-t-butyl-1,2,4-oxadiazol-5-yl, and 3-cyclopropyl-1,2,4-oxadiazol-5-yl.

In some embodiments, $R_2$ is selected from the group consisting of 3-methyl-1,2,4-oxadiazol-5-yl, 3-ethyl-1,2,4-oxadiazol-5-yl, 3-propyl-1,2,4-oxadiazol-5-yl, 3-isopropyl-1,2,4-oxadiazol-5-yl, 3 butyl-1,2,4-oxadiazol-5-yl, and 3-(t-butyl)-1,2,4-oxadiazol-5-yl.

In some embodiments, $R_2$ is a heteroaryl comprising 5-atoms in the aromatic ring and are represented by the following formulae:

**TABLE 2A**

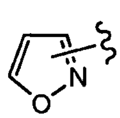 , 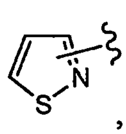 , 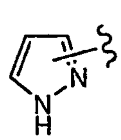 , 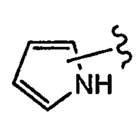 , 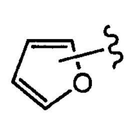 ,

(continued)

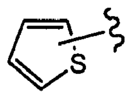, 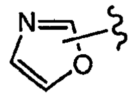, 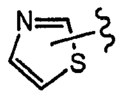, 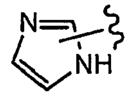, 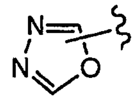,

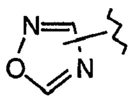, 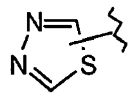, 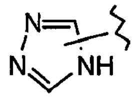, 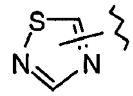,

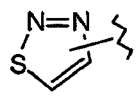 and 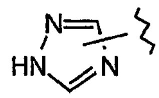

wherein the 5-membered heteroaryl is bonded at any available position of the ring, for example, a imidazolyl ring can be bonded at one of the ring nitrogens (i.e., imidazol-1-yl group) or at one of the ring carbons (i.e., imidazol-2-yl, imidazol-4-yl or imiadazol-5-yl group).

In some embodiments, $R_2$ is a 5-membered heteroaryl optionally substituted with 1 to 4 substituents selected from the group consisting of $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-8}$ dialkylamino, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heterocyclic, hydroxyl, hydroxylamino and nitro.

In some embodiments, compounds of the present invention are of the following formula:

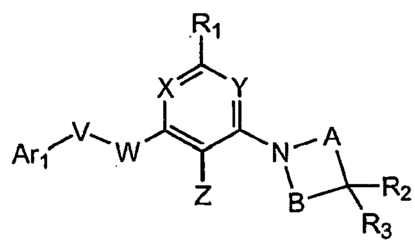

wherein $R_2$ is a 5-membered heteroaryl optionally substituted with 1 to 4 substituents selected from the group consisting of $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-8}$ dialkylamino, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heterocyclic, hydroxyl, hydroxylamino and nitro; and $R_3$ is hydrogen or $C_{1-4}$ alkyl.

In some embodiments, $R_2$ is a 5-membered heteroaryl optionally substituted with 1 or 2 substituents selected from the group consisting of $C_{1-8}$ alkyl, $C_{1-4}$ haloalkyl and halogen; and $R_3$ is hydrogen.

In some embodiments, $R_2$ is a 5-membered heteroaryl optionally substituted with lor 2 $C_{1-8}$ alkyl substituents; and $R_3$ is hydrogen.

In some embodiments, $R_2$ is a 5-membered heteroaryl optionally substituted with $CH_3$, $CH_2CH_3$, $CH(CH_3)_2$, $CH_2CH_2CH_3$, $C(CH_3)_3$; and $R_3$ is hydrogen.

In some embodiments, $R_2$ is a fused heteroaryl group containing to aromatic rings wherein at least one is a heteroaryl ring, such as, benzofuranyl, benzimidazole, benzoxazole, benzothiazole, indole, benzothiophenyl. In some embodiments, $R_2$ is a benzofuran-2-yl group.

In some embodiments, $R_2$ is a heterocyclic represented, for example, by the formulae in TABLE 2B.

**TABLE 2B**

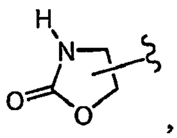, 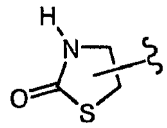, 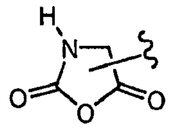, 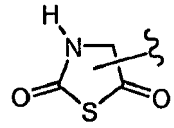,

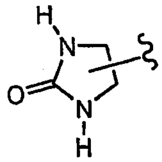, 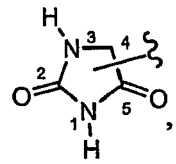,

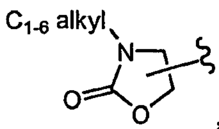, 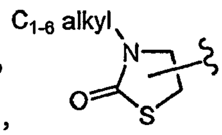, 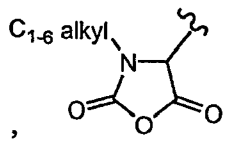, 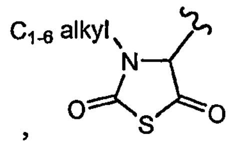,

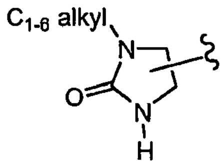 and 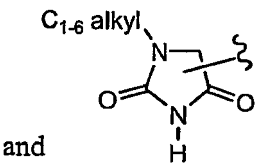.

It is understood that any one of the heterocyclic groups shown in TABLES 2B to 2E may be bonded at any available ring carbon or ring nitrogen as allowed by the respective formula. For example, a 2,5-dioxo-imidazolidinyl group may be bonded at the ring carbon or at either of the two ring nitrogens to give the following formulae respectively:

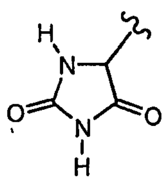, 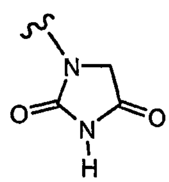, 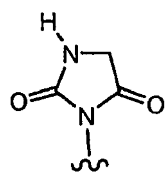.

In some embodiments, $R_2$ is a heterocyclic represented, for example, by the formulae in TABLE 2C.

**TABLE2C**

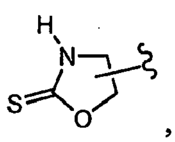, 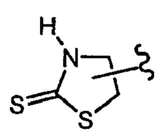, 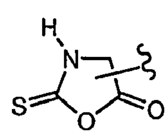, 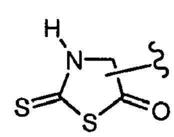,

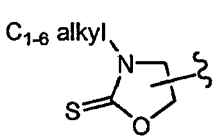, 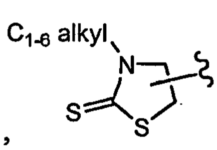, 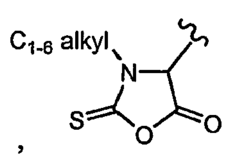 and 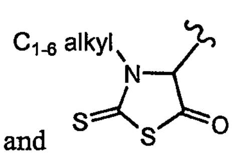.

In some embodiments, $R_2$ is a heterocyclic represented, for example, by the formulae in TABLE 2D.

**TABLE 2D**

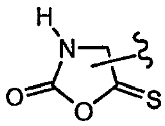 , 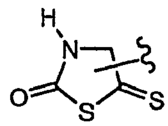 , 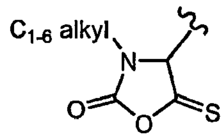 and 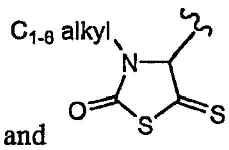 .

In some embodiments, $R_2$ is a heterocyclic represented, for example, by the formulae in TABLE 2E.

**TABLE 2E**

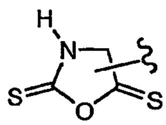 , 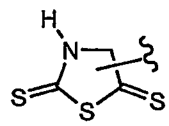 , 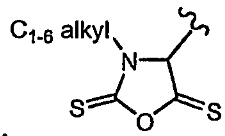 and 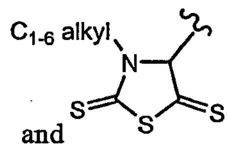 .

In some embodiments, $R_2$ is a heterocyclic represented, for example, by the formulae in TABLE 2F wherein the $C_{1-6}$ alkyl group on the respective ring nitrogen atoms may be the same or different.

**TABLE 2F**

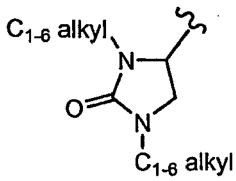 and 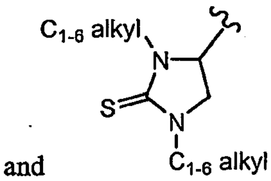 .

In some embodiments, $R_2$ is a heterocyclic represented, for example, by the formulae in TABLE 2G wherein the $C_{1-6}$ alkyl group on the respective ring nitrogen atoms may be the same or different.

**TABLE 2G**

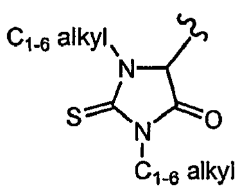 , 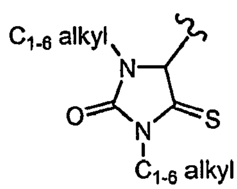 and 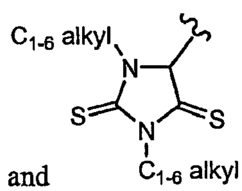 .

In some embodiments, D is $CR_2R_3$ and $R_2$ is $-Ar_2-Ar_3$ wherein $Ar_2$ and $Ar_3$ are independently aryl or heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, hydroxyl and nitro. In some embodiments, $Ar_2$ is a heteroaryl and $Ar_3$ is phenyl. In some embodiments, compounds of the present invention are represented by Formula **(Ir)** as shown below:

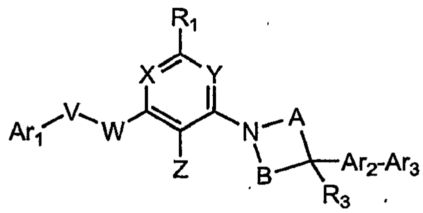

**(Ir)**

wherein each variable in Formula (**Ir**) has the same meaning as described herein. In some embodiments, compounds of the present invention are of Formula (**Ir**) wherein $R_3$ is H.

In some embodiments, $Ar_2$ is a heteroaryl comprising 5-atoms in the aromatic ring and are represented by the following formulae:

**TABLE 3**

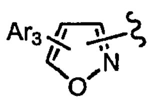 , 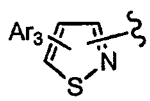 , 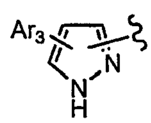 , 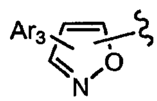 , 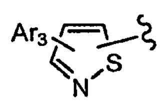 ,

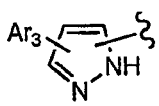 , 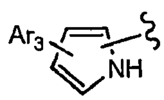 , 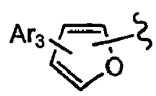 , 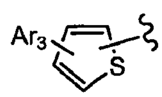 , 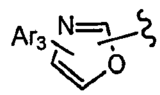 ,

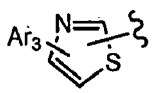 , 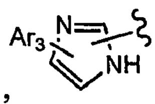 , 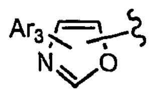 , 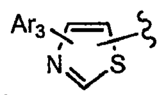 , 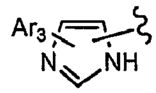 ,

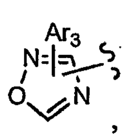 , 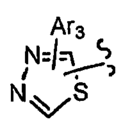 , 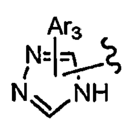 , 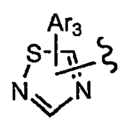 ,

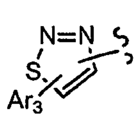 and 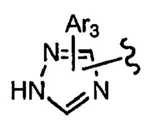

wherein the 5-membered heteroaryl is bonded at any available position of the ring, for example, a imidazolyl ring can be bonded at one of the ring nitrogens (i.e., imidazol-1-yl group) or at one of the ring carbons (i.e., imidazol-2-yl, imidazol-4-yl or imiadazol-5-yl group) and $Ar_3$ is bonded to any remaining available ring atom.

In some embodiments, $Ar_2$ is a heteroaryl and $Ar_3$ is phenyl. In some embodiments, the heteroaryl and phenyl are optionally substituted with 1 to 5 substituents selected from the group consisting of H, $C_{1-4}$ alkoxy, $C_{1-8}$ alkyl, $C_{1-4}$ alkylcarboxamide, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, halogen, hydroxyl and nitro.

In some embodiments, D is $CR_2R_3$ and $R_2$ is Formula (B):

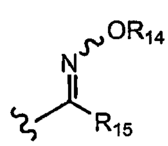

**(B)**

wherein:

$R_{14}$ is $C_{1-8}$ alkyl or $C_{3-6}$ cycloalkyl; and $R_{15}$ is F, Cl, Br or CN. In some embodiments, $R_{14}$ is $C_{1-8}$ alkyl and $R_{15}$ is F, Cl or CN.

In some embodiments, D is $CR_2R_3$ and $R_2$ is Formula (C):

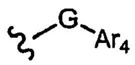

(C)

wherein:

G is C=O, $CR_{16}R_{17}$, O, S, S(O), $S(O)_2$; where $R_{16}$ and $R_{17}$ are independently H or $C_{1-8}$ alkyl; and $Ar_4$ is phenyl or heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro.

In some embodiments, $R_2$ is Formula (C) wherein G is C=O, $CR_{16}R_{17}$, O, S, S(O), $S(O)_2$; wherein $R_{16}$ and $R_{17}$ are independently H or $C_{1-2}$ alkyl; and $Ar_4$ is phenyl or heteroaryl optionally substituted with 1 to 5 substituents selected from the group consisting of $C_{1-4}$ alkoxy, $C_{1-8}$ alkyl, $C_{1-4}$ haloalkoxy, $C_{1-4}$ haloalkyl, and halogen.

In some embodiments, G is C=O, $CH_2$ or O. In some embodiments, G is S, S(O) or $S(O)_2$.

In some embodiments, $Ar_4$ is selected from the group consisting of pyridinyl, pyridazinyl, pyrimidinyl and pyrazinyl. In some embodiments, $Ar_4$ is 2-pyridyl.

In some embodiments, compounds of the present invention are represented by Formula **(Is)** as shown below:

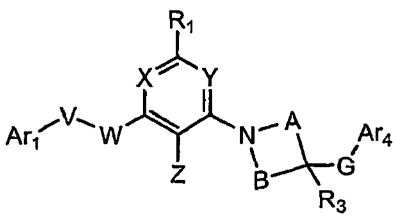

(Is)

wherein each variable in Formula **(Is)** has the same meaning as described herein.

In some embodiments, D is $CR_2R_3$, $R_2$ is Formula **(C)** and G is C=O, $CR_{16}R_{17}$ or O. In some embodiments, $Ar_4$ is phenyl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro.

In some embodiments, $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, halogen and hydroxyl.

In some embodiments, $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, $C_{1-4}$ haloalkyl, halogen and hydroxyl.

In some embodiments, $Ar_4$ is heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$

alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro.

In some embodiments, $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, halogen and hydroxyl.

In some embodiments, $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, $C_{1-4}$ haloalkyl, halogen and hydroxyl.

In some embodiments, $Ar_4$ is a 5-membered heteroaryl, for example, as shown in TABLE 2A *supra.* In some embodiments, $Ar_4$ is a 6-membered heteroaryl, for example, the 6-membered heteroaryls as shown in TABLE 4:

**TABLE 4**

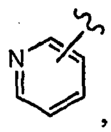, 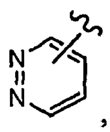, 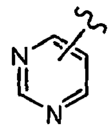, 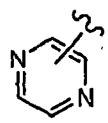, 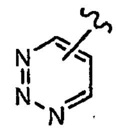,

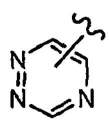, 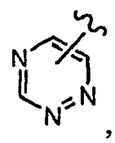, 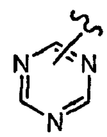 and 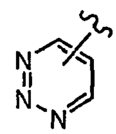

wherein the heteroaryl group is bonded at any ring carbon. In some embodiments, $Ar_4$ is selected from the group consisting of pyridinyl, pyridazinyl, pyrimidinyl and pyrazinyl. In some embodiments, $Ar_4$ is 2-pyridyl. In some embodiments, D is $CR_2R_3$, $R_2$ is Formula (C), G is $CR_{16}R_{17}$ and $R_{16}$ and $R_{17}$ are independently H or $C_{1-2}$ alkyl.

In some embodiments, D is $CR_2R_3$, $R_2$ is Formula (C) and G is S, S(O) or $S(O)_2$.

In some embodiments, $Ar_4$ is phenyl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalk-ylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro.

In some embodiments, $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, halogen and hydroxyl.

In some embodiments, $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, $C_{1-4}$ haloalkyl, halogen and hydroxyl.

In some embodiments, $Ar_4$ is heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro.

In some embodiments, $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, halogen and hydroxyl.

In some embodiments, $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, $C_{1-4}$ haloalkyl, halogen and hydroxyl. In some embodiments, $Ar_4$ is a 5-membered heteroaryl, for example, as shown in TABLE 2A, *supra.*

In some embodiments, $Ar_4$ is a 6-membered heteroaryl, for example, as shown in TABLE 4, *supra.*

In some embodiments, $Ar_4$ is selected from the group consisting of pyridinyl, pyridazinyl, pyrimidinyl and pyrazinyl. In some embodiments, $Ar_4$ is 2-pyridyl.

In some embodiments, $R_3$ is H.

In some embodiments, D is N-$R_2$. In some embodiments, $R_2$ is selected from the group consisting of H, $C_{1-5}$ acyl, $C_{1-8}$ alkyl, $C_{1-4}$ alkylcarboxamide, $C_{1-4}$ alkylsulfonyl, carbo-$C_{1-6}$-alkoxy, carboxamide, $C_{3-6}$-cycloalkyl and $C_{1-4}$ haloalkyl. In some embodiments, $R_2$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, $CH_2(CH_2)_3CH_3$, $C(O)NHCH_3$, $C(O)NHCH_2CH_3$, $C(O)NHCH_2CH_2CH_3$, $C(O)NHCH(CH_3)_2$, $C(O)NHCH_2(CH_2)_2CH_3$, $CO_2CH_3$, $CO_2CH_2CH_3$, $CO_2CH_2CH_2CH_3$, $CO_2CH(CH_3)_2$ and $CO_2CH_2(CH_2)_2CH_3$. In some embodiments, $R_2$ is selected from the group consisting of $S(O)_2CH_3$, $S(O)_2CH_2CH_3$, $S(O)_2CH_2CH_2CH_3$, $S(O)_2CH(CH_3)_2$, $S(O)_2CH_2(CH_2)_2CH_3$, cyclopropyl, cyclobutyl, cyclopentyl, cyclohexyl, $CH_2CF_3$, $CF_3$ and $CHF_2$.

In some embodiments, D is N-$R_2$ and $R_2$ is H, or carbo-$C_{1-6}$-alkoxy. In some embodiments, $R_2$ is selected from the group consisting of $CO_2CH_3$, $CO_2CH_2CH_3$, $CO_2CH_2CH_2CH_3$, $CO_2CH(CH_3)_2$ and $CO_2CH_2(CH_2)_2CH_3$. In some embodiments, $R_2$ is $C_{1-8}$ alkyl optionally substituted with 1 to 5 substituents selected from the group consisting of $C_{1-4}$ alkylsulfonyl, carbo-$C_{1-6}$-alkoxy, and carboxy. In some embodiments, $R_2$ is $CH_2CO_2Et$, or $CH_2CH_2CO_2H$. In some embodiments, $R_2$ is selected from the group consisting of $CH_2CH_2S(O)_2CH_3$, $CH_2CH_2S(O)_2CH_2CH_3$, $CH_2CH_2S(O)_2CH_2CH_2CH_3$, $CH_2CH_2S(0)_2CH(CH_3)_2$ and $CH_2CH_2S(O)_2CH_2(CH_2)_2CH_3$.

In some embodiments, D is N-$R_2$ wherein $R_2$ is $C_{1-8}$ alkyl, heteroaryl or phenyl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-8}$ dialkylamino, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heterocyclic, hydroxyl, hydroxylamino and nitro. In some embodiments, $R_2$ is selected from the group consisting of $CH_2CH_2OCH_3$, $CH_2CH_2OCH_2CH_3$, $CH_2CH_2OCH_2CH_2CH_3$, $CH_2CO_2H$, $CH_2CH_2CO_2H$, $CH_2CH_2OH$ and $CH_2CH_2CH_2OH$. In some embodiments, $R_2$ is selected from the group consisting of $CH_2CH_2SCH_3$, $CH_2CH_2SCH_2CH_3$, $CH_2CH_2SCH_2CH_2CH_3$, $CH_2CH_2SCH(CH_3)_2$, $CH_2CH_2SCH_2(CH_2)_2CH_3$, $CH_2CH_2S(O)CH_3$, $CH_2CH_2S(O)CH_2CH_3$, $CH_2CH_2S(O)CH_2CH_2CH_3$, $CH_2CH_2S(O)CH(CH_3)_2$, $CH_2CH_2S(O)CH_2(CH_2)_2CH_3$, $CH_2CH_2S(O)_2CH_3$, $CH_2CH_2S(O)_2CH_2CH_3$, $CH_2CH_2S(O)_2CH_2CH_2CH_3$, $CH_2CH_2S(O)_2CH(CH_3)_2$ and $CH_2CH_2S(O)_2CH_2(CH_2)_2CH_3$. In some embodiments, $R_2$ is selected from the group consisting of 1,2,4-oxadiazol-3-yl, 1,2,4-oxadiazol-5-yl, 1,3,4-oxadiazol-2-yl, 1,2,4-triazol-5-yl and 1,2,4-triazol-1-yl, 3-methyl-1,2,4-oxadiazol-5-yl, 3-methyl-1,2,4-oxadiazol-5-yl, 3-ethyl-1,2,4-oxadiazol-5-yl, 3-ethyl-1,2,4-oxadiazol-5-yl, 5-methyl-1,3,4-oxadiazol-2-yl, 5-ethyl-1,3,4-oxadiazol-2-yl, 3-methyl-1,2,4-triazol-5-yl, 3-ethyl-1,2,4-triazol-5-yl, 3-methyl-1,2,4-triazol-1-yl, 3-ethyl-1,2,4-triazol-1-yl, 5-methyl-1,2,4-triazol-1-yl and 5-ethyl-1,2,4-triazol-1-yl.

In some embodiments, D is N-$R_2$ and $R_2$ is -$Ar_2$-$Ar_3$ wherein $Ar_2$ and $Ar_3$ are independently aryl or heteroaryl optionally substituted with 1 to 5 substituents selected from the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, hydroxyl and nitro.. In some embodiments, compounds of the present invention are represented by Formula (It) as shown below:

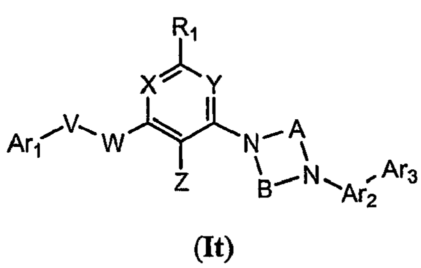

**(It)**

wherein each variable in Formula (It) has the same meaning as described herein. In some embodiments, $Ar_2$ is a heteroaryl and $Ar_3$ is phenyl. In some embodiments, the heteroaryl and phenyl are optionally substituted with 1 to 5 substituents selected from the group consisting of H, $C_{1-4}$ alkoxy, $C_{1-8}$ alkyl, $C_{1-4}$ alkylcarboxamide, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, halogen, hydroxyl and nitro.

In some embodiments, D is N-$R_2$ wherein $R_2$ is Formula (C):

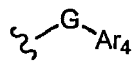

(C)

wherein:

G is C=O or $CR_{16}R_{17}$; where $R_{16}$ and $R_{17}$ are independently H or $C_{1-8}$ alkyl; and $Ar_4$ is phenyl or heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro. In some embodiments, compounds of the present invention are represented by Formula **(Iu)** as shown below:

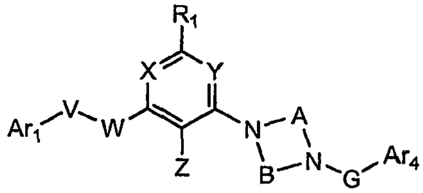

(Iu)

wherein each variable in Formula **(Iu)** has the same meaning as described herein.

In some embodiments, D is N-$R_2$, $R_2$ is Formula (C) and G is C=O. In some embodiments, $Ar_4$ is phenyl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro. In some embodiments, $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, halogen and hydroxyl. In some embodiments, $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, $C_{1-4}$ haloalkyl, halogen and hydroxyl. In some embodiments, $Ar_4$ is heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro. In some embodiments, $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, halogen and hydroxyl. In some embodiments, $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, $C_{1-4}$ haloalkyl, halogen and hydroxyl. In some embodiments, $Ar_4$ is a 5-membered heteroaryl, for example, as shown in TABLE 2A, *supra.* In some embodiments, $Ar_4$ is a 6-membered heteroaryl, for example, as shown in TABLE 4, *supra.* In some embodiments, $Ar_4$ is selected from the group consisting of pyridinyl, pyridazinyl, pyrimidinyl and pyrazinyl. In some embodiments, $Ar_4$ is 2-pyridyl.

In some embodiments, D is N-$R_2$ wherein $R_2$ is Formula (C) and G is $CR_{16}R_{17}$. In some embodiments, $Ar_4$ is phenyl optionally substituted with 1 to 5 substituents selected from the group consisting of $C_{1-5}$ acyl, $C_{1-5}$ acyloxy, $C_{1-4}$

alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro. In some embodiments, $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, halogen and hydroxyl. In some embodiments, $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, $C_{1-4}$ haloalkyl, halogen and hydroxyl. In some embodiments, $Ar_4$ is heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro. In some embodiments, $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, halogen and hydroxyl. In some embodiments, $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, $C_{1-4}$ haloalkyl, halogen and hydroxyl. In some embodiments, $Ar_4$ is a 5-membered heteroaryl, for example, as shown in TABLE 2A, *supra.* In some embodiments, $Ar_4$ is a 6-membered heteroaryl, for example, as shown in TABLE 4, *supra.* In some embodiments, $Ar_4$ is selected from the group consisting of pyridinyl, pyridazinyl, pyrimidinyl and pyrazinyl. In some embodiments, $Ar_4$ is 2-pyridyl. In some embodiments, D is N-$R_2$ wherein $R_2$ is Formula (C), G is $CR_{16}R_{17}$ and $R_{16}$ and $R_{17}$ are independently H or $C_{1-2}$ alkyl.

In some embodiments, Z is selected from the group consisting of $C_{1-5}$ acyl, $C_{1-8}$ alkyl, $C_{1-4}$ alkylcarboxamide, amino, cyano, $C_{4-8}$ diacylamino, $C_{2-6}$ dialkylsulfonamide, formyl, halogen, heterocyclic, and nitro wherein $C_{1-8}$ alkyl and $C_{1-5}$ acyl are each optionally substituted with 1, or 2 groups selected from the group consisting of $C_{2-4}$ dialkylmino, hydroxy, and halogen.

In some embodiments, Z is selected from the group consisting of nitro, amino, formyl, $NHC(O)CF_3$, Br, $NHC(O)CH_3$, $N(C(O)CH_3)_2$, $N(S(O)_2CH_3)_2$, $CH_3$, [1,3]dioxolan-2-yl, $CH_2OH$, $CH_2N(CH_3)_2$, and $C(O)CH_3$.

In some embodiments, Z is selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, carboxamide, carboxy, cyano, aryl, $C_{1-4}$ haloalkyl, $C_{1-4}$ haloalkylcarboxamide, heteroaryl, hydroxyl, hydroxylamino, nitro and tetrazolyl, wherein $C_{1-8}$ alkyl is optionally substituted with 1, 2, 3 or 4 groups selected from the group consisting of $C_{1-5}$ acyl, $C_{1-5}$ acyloxy, $C_{1-4}$ alkoxy, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, carbo-$C_{1-6}$-alkoxy, carboxamide, carboxy, cyano, formyl, $C_{1-4}$ haloalkoxy, $C_{1-4}$ haloalkylsulfinyl, $C_{1-4}$ haloalkylsulfonyl, $C_{1-4}$ haloalkylthio, halogen, hydroxyl, hydroxylamino and nitro.

In some embodiments, Z is selected from the group consisting of $C_{1-5}$ acyl, $C_{1-4}$ alkoxy, $C_{1-8}$ alkyl, $C_{1-4}$ alkylcarboxamide, $C_{1-4}$ alkylthiocarboxamide, $C_{1-4}$ alkylthioureyl, $C_{1-4}$ alkylureyl, carboxamide, carboxy, cyano, aryl, $C_{1-4}$ haloalkyl, $C_{1-4}$ haloalkylcarboxamide, heteroaryl, hydroxyl, hydroxylamino, nitro and tetrazolyl.

In some embodiments, Z is a heterocyclic group. In some embodiments, Z is a 5-membered heterocyclic group containing two oxygen atoms.

In some embodiments, Z is an alkyl group optionally substituted $C_{2-4}$-dialkylamino or hydroxy.

In some embodiments, Z is selected from the group consisting of formyl, $NHC(O)CH_3$, $NHC(O)CH_2CH_3$, $NHC(O)CH(CH_3)_2$, $CH_3$, $CH_2CH_3$, $CH(CH_3)_2$, $CH_2CH_2CH_2CH_3$, $NHC(O)CF_3$, carboxy, $CF_3$, $CF_2CF_3$, nitro and 1*H*-tetrazol-5-yl.

In some embodiments, Z is selected from the group consisting of carboxy, $CF_3$, nitro and 1*H*-tetrazol-5-yl.

In some embodiments, Z is [1,3]-dioxolan-2-yl.

In some embodiments, Z is a formyl group.

In some embodiments, Z is a carboxy group.

In some embodiments, Z is a $-CH_2OH$ group.

In some embodiments, Z is a $-CH_2N(CH_3)_2$ group.

In some embodiments, Z is Formula (A):

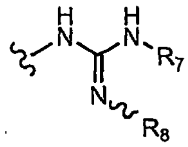

(A)

wherein:

$R_7$ is H, $C_{1-8}$ alkyl or $C_{3-6}$ cycloalkyl; and $R_8$ is H, nitro or nitrile. In some embodiments, $R_7$ is H or $C_{1-8}$ alkyl.

In some embodiments, $R_1$ is selected from the group consisting of H, $C_{1-4}$ alkoxy, $C_{1-8}$ alkyl, $C_{2-6}$ alkynyl, amino, $C_{3-6}$ cycloalkyl and $C_{1-4}$ haloalkyl. In some embodiments, $R_1$ is H or amino.

In some embodiments, $R_1$ is selected from the group consisting of H, $C_{1-8}$ alkyl, and amino.

In some embodiments, $Ar_1$ is aryl optionally substituted with $R_9$-$R_{13}$. In some embodiments, $Ar_1$ is phenyl.

In some embodiments, $Ar_1$ is heteroaryl. In some embodiments, $Ar_1$ is heteroaryl optionally substituted with $R_9$-$R_{13}$. In some embodiments, $Ar_1$ is a heteroaryl selected from TABLE 2A. In some embodiments, $Ar_1$ is a heteroaryl selected from TABLE 4 or an N-oxide thereof. In some embodiments, compounds of the invention are of Formula **(Iv):**

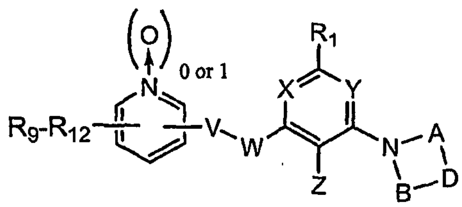

(Iv)

wherein A, B, D, V, W, X, Y, Z, $R_1$, $R_9$, $R_{10}$, $R_{11}$ and $R_{12}$ have the same meaning as described herein, *supra* and *infra.*

In some embodiments, $Ar_1$ is heteroaryl and $R_9$ is selected from the group consisting of H, $C_{1-5}$ acyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, carboxamide and sulfonamide.

In some embodiments, $R_9$ is selected from the group consisting of $C_{1-5}$ acyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{2-6}$ dialkylsulfonamide, $C_{1-4}$ alkylsulfinyl, $C_{1-4}$ alkylsulfonyl, $C_{1-4}$ alkylthio, amino, arylsulfonyl, $C_{2-6}$ dialkylamino, $C_{2-6}$ dialkylsulfonamide, and carboxamide.

In some embodiments, $R_9$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $OCH_3$, $OCH_2CH_3$, $OCH_2CH_2CH_3$, $OCH(CH_3)_2$, $OCH_2CH_2CH_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, $CH_2(CH_2)_3CH_3$, $CH_2CH_2)_4CH_3$, $CH_2(CH_2)_5CH_3$, $C(O)NHCH_3$, $C(O)NHCH_2CH_3$, $C(O)NHCH_2CH_2CH_3$, $C(O)NHCH(CH_3)_2$, C≡CH, $S(O)_2NHCH_3$, $S(O)_2NHCH_2CH_3$, $S(O)_2NHCH_2CH_2CH_3$, $S(O)_2NHCH(CH_3)_2$, $S(O)_2NHCH_2(CH_2)_2CH_3$, $S(O)_2NHCH(CH_3)CH_2CH_3$, $S(O)_2N(CH_3)_2$, $S(O)_2N(Et)(CH_3)$, $S(O)_2CH_3$, $S(O)_2CH_2CH_3$, $S(O)_2CH_2CH_2CH_3$, $S(O)_2CH(CH_3)_2$, $S(O)_2CH_2(CH_2)_2CH_3$, $S(O)_2CH(CH_3)CH_2CH_3$, $SCH_3$, $SCH_2CH_3$, $SCH_2CH_2CH_3$, $SCH(CH_3)_2$, $SCH_2(CH_2)_2CH_3$, amino, $S(O)_2Ph$, $N(CH_3)_2$, $N(CH_3)(Et)$, $N(Et)_2$ and $C(O)NH_2$.

In some embodiments, $R_9$ is selected from the group consisting of cyano, $C_{3-6}$ cycloalkyl, halogen, $C_{1-4}$ haloalkoxy, $C_{1-4}$ haloalkyl, $C_{1-4}$ haloalkylsulfonyl, and $C_{1-4}$ haloalkylthio.

In some embodiments, $R_9$ is selected from the group consisting of cyclopropyl, cyclobutyl, cyclopentyl, cyclohexyl, Cl, F, Br, $OCF_3$, $OCHF_2$, $OCH_2CF_3$, $CF_3$, $CHF_2$, $CH_2CF_3$, $SCF_3$, $SCHF_2$ and $SCH_2CF_3$.

In some embodiments, $R_9$ is selected from the group consisting of CN, $CO_2Me$, $CO_2Et$, $S(O)_2CH_3$, $S(O)_2CF_3$, $N(CH_3)_2$, $N(Et)_2$, $C(O)NHCH_3$, $C(O)NHEt$, $C(O)N(CH_3)_2$, OH, $OCH_3$, and OEt.

In some embodiments, $R_9$ is selected from the group consisting of heterocyclic, heterocyclicsulfonyl, heteroaryl, hydroxy, $C_{4-7}$ oxo-cycloalkyl, phenoxy and phenyl.

In some embodiments, $R_9$ is selected from the group consisting of $C_{1-5}$ acyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, carboxamide, halogen and sulfonamide.

In some embodiments, $R_9$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $OCH_3$, $OCH_2CH_3$, $OCH_2CH_2CH_3$, $OCH(CH_3)_2$, $OCH_2CH_2CH_2CH_3$, $CH_3$,

$CH_2CH_3$, $CH_2CH_2CH_3$, $CH(CH_3)_2$, $CH(CH_3)(CH_{2CH3})$, $CH_2(CH_2)_2CH_3$, $CH_2(CH_2)_3CH_3$, $CH_2(CH_2)_4CH_3$, $CH_2(CH_2)_5CH_3$, $C(O)NHCH_3$, $C(O)NHCH_2CH_3$, $C(O)NHCH_2CH_2CH_3$, $C(O)NHCH(CH_3)_2$, $C(O)NHCH_2(CH_2)_2CH_3$, CCH, $S(O)_2NHCH_3$, $S(O)_2NHCH_2CH_3$, $S(O)_2NHCH_2CH_2CH_3$, $S(O)_2NHCH(CH_3)_2$, $S(O)_2NHCH_2(CH_2)_2CH_3$, $S(O)_2NHCH(CH_3)CH_2CH_3$, $S(O)CH_3$, $S(O)CH_2CH_3$, $S(O)CH_2CH_2CH_3$, $S(O)CH(CH_3)_2$, $S(O)CH_2(CH_2)_2CH_3$, $S(O)CH(CH_3)CH_2CH_3$, $S(O)_2CH_3$, $S(O)_2CH_2CH_3$, $S(O)_2CH_2CH_2CH_3$, $S(O)_2CH(CH_3)_2$, $S(O)_2CH_2(CH_2)_2CH_3$, $S(O)_2CH(CH_3)CH_2CH_3$, $SCH_3$, $SCH_2CH_3$, $SCH_2CH_2CH_3$, $SCH(CH_3)_2$ and $SCH_2(CH_2)_2CH_3$.

In some embodiments, $R_9$ is selected from the group consisting of amino, arylsulfonyl, carboxy, cyano, $C_{3-6}$ cycloalkyl, halogen, $C_{1-4}$ haloalkoxy, $C_{1-4}$ haloalkyl and $C_{1-4}$ haloalkylthio.

In some embodiments, $R_9$ is selected from the group consisting of phenylsulfonyl, cyclopropyl, cyclobutyl, cyclopentyl, cyclohexyl, Cl, F, Br, $OCF_3$, $OCHF_2$, $OCH_2CF_3$, $CF_3$, $CHF_2$, $CH_2CF_3$, $SCF_3$, $SCHF_2$ and $SCH_2CF_3$.

In some embodiments, $R_9$ is selected from the group consisting of heterocyclic, heteroaryl, $C_{4-7}$ oxo-cycloalkyl, phenoxy and phenyl. In some embodiments, $R_9$ is selected from the group consisting of morpholin-4-yl, thiomorpholin-4-yl, 1-oxo-1$\lambda^4$-thiomorpholin-4-yl, 1,1-Dioxo-1$\lambda^6$-thiomorpholin4-yl, piperazin-1-yl, 4-methyl-piperazin-1-yl, 4-ethyl-piperazin-1-yl, 4-propyl-piperazin-1-yl, piperidin-1-yl, pyrrolidin-1-yl, 2,5-dioxo-imidazolidin-4-yl, 2,4-dioxo-thiazolidin-5-yl, 4-oxo-2-thioxo-thiazolidin-5-yl, 3-methyl-2,5-dioxo-imidazolidin-4-yl, 3-methyl-2,4-dioxo-thiazolidin-5-yl, 3-methyl-4-oxo-2-thioxo-thiazolidin-5-yl, 3-ethyl-2,5-dioxo-imidazolidin-4-yl, 3-ethyl-2,4-dioxo-thiazolidin-5-yl, and 3-ethyl-4-oxo-2-thioxo-thiazolidin-5-yl.

In some embodiments, $R_9$ is selected from the group consisting of 1H-imidazol-4-yl, [1,2,4]triazol-1-yl, [1,2,3]triazol-1-yl, [1,2,4]triazol-4-yl, pyrrol-1-yl, pyrazol-1-yl, 1H-pyrazol-3-yl, imidazol-1-yl, oxazol-5-yl, oxazol-2-yl, [1,3,4]oxadiazol-2-yl, [1,3,4]thiadiazol-2-yl, [1,2,4]oxadiazol-3-yl, [1,2,4]thiadiazol-3-yl, tetrazol-1-yl, pyrimidin-5-yl, pyrimidin-2-yl, pyrimidin-4-yl, pyridazin-3-yl, pyridazin-4-yl, pyrazin-2-yl, 1,3-dioxo-1,3-dihydro-isoindol-2-yl and [1,2,3]thiadiazol-4-yl.

In some embodiments, $R_9$ is $C_{1-8}$ alkyl or $C_{1-4}$ alkoxy optionally substituted with 1 to 5 substituents selected independently from the group consisting of $C_{1-4}$ alkoxy, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carbo-$C_{1-6}$-alkoxy; carboxamide, carboxy; cyano, heterocyclic, hydroxyl and phenyl.

In some embodiments, $R_9$ is $C_{1-4}$ alkylsulfonyl optionally substituted with 1 to 5 substituents selected independently from the group consisting of $C_{1-4}$ alkoxy, carboxamide, heteroaryl, heterocyclic and phenyl.

In some embodiments, $R_9$ is $C_{1-4}$ alkylsulfonyl substituted with the heteroaryl group. In some embodiments, the heteroaryl is selected from the group consisting of 1H-imidazol-4-yl, [1,2,4]triazol-1-yl, [1,2,3]triazol-1-yl, [1,2,4]triazol-4-yl, pyrrol-1-yl, pyrazol-1-yl, 1H-pyrazol-3-yl, imidazol-1-yl, oxazol-5-yl, oxazol-2-yl, [1,3,4]oxadiazol-2-yl,[1,3,4]thiadiazol-2-yl, [1,2,4]oxadiazol-3-yl, [1,2,4]thiadiazol-3-yl, tetrazol-1-yl, pyrimidin-5-yl, pyrimidin-2-yl, pyrimidin-4-yl, pyridazin-3-yl, pyridazin-4-yl, pyrazin-2-yl, 1,3-dioxo-1,3-dihydro-isoindol-2-yl and [1,2,3]thiadiazol-4-yl.

In some embodiments, $R_9$ is arylsulfonyl, heteroaryl, phenoxy or phenyl optionally substituted with 1 to 5 substituents selected independently from the group consisting of $C_{1-5}$ acyl, $C_{1-4}$ alkoxy, $C_{1-8}$ alkyl, $C_{1-4}$ alkylsulfonamide, $C_{1-4}$ alkylsulfinyl, $C_{1-4}$ alkylsulfonyl, $C_{1-4}$ alkylthio, carboxamide, carboxy, cyano, halogen, $C_{1-4}$ haloalkoxy, $C_{1-4}$ haloalkyl, $C_{1-4}$ haloalkylthio and hydroxyl.

In some embodiments, $R_9$ is arylsulfonyl, heteroaryl, phenoxy or phenyl each optionally substituted with 1 to 5 substituents selected independently from the group consisting of $C_{1-4}$ alkoxy, $C_{1-8}$ alkyl, cyano, halogen, $C_{1-4}$ haloalkoxy, $C_{1-4}$ haloalkyl and hydroxyl.

In some embodiments, $R_9$ is a heterocyclic group as described herein.

In some embodiments, $R_9$ is a heterocyclic group represented by the formulae shown in Table 2B, *supra.* In some embodiments, $R_9$ is a heterocyclic group represented by the formulae shown in Table 2C, *supra.* In some embodiments, $R_9$ is a heterocyclic group represented by the formulae shown in Table 2D, *supra.* In some embodiments, $R_9$ is a heterocyclic group represented by the formulae shown in Table 2E, *supra.* In some embodiments, $R_9$ is a heterocyclic group represented by the formulae shown in Table 2F, *supra.* In some embodiments, $R_9$ is a heterocyclic group represented by the formulae shown in Table 2G, *supra.*

In some embodiments, $R_9$ is of Formula (D):

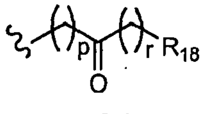

**(D)**

wherein:

"p" and "r" are independently 0, 1, 2 or 3; and $R_{18}$ is H, $C_{1-5}$ acyl, $C_{2-6}$ alkenyl, $C_{1-8}$ alkyl, $C_{1-4}$ alkylcarboxamide,

$C_{2-6}$ alkynyl, $C_{1-4}$ alkylsulfonamide, carbo-$C_{1-6}$-alkoxy, carboxamide, carboxy, cyano, $C_{3-6}$ cycloalkyl, $C_{2-6}$ dialkylcarboxamide, halogen, heteroaryl or phenyl, and wherein the heteroaryl or phenyl may be optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl.

In some embodiments, $R_9$ is of Formula (D) wherein "p" and "r" are independently 0, or 1; and $R_{18}$ is H, carbo-$C_{1-6}$-alkoxy, heteroaryl or phenyl, and wherein the heteroaryl and phenyl are each optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl.

In some embodiments, p = 0 and r = 0.

In some embodiments, $R_{18}$ is phenyl optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl.

In some embodiments, p = 0 and r = 1.

In some embodiments, $R_{18}$ is carbo-$C_{1-6}$-alkoxy or carboxy.

In some embodiments, p = 0 and r = 0.

In some embodiments, $R_{18}$ is heteroaryl or phenyl optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl. In some embodiments, the heteroaryl is selected from the group consisting of 1H-imidazol-4-yl, [1,2,4]triazol-1-yl, [1,2,3]ttiazol-1-yl, [1,2,4]triazol-4-yl, pyrrol-1-yl, pyrazol-1-yl, 1H-pyrazol-3-yl, imidazol-1-yl, oxazol-5-yl, oxazol-2-yl, [1,3,4]oxadiazol-2-yl, [1,3,4]thiadiazol-2-yl, [1,2,4]oxadiazol-3-yl, [1,2,4]thiadiazol-3-yl, tetrazol-1-yl, pyrimidin-5-yl, pyrimidin-2-yl, pyrimidin-4-yl, pyridazin-3-yl, pyridazin-4-yl, pyrazin-2-yl, 1,3-dioxo-1,3-dihydro-isoindol-2-yl and [1,2,3]thiadiazol-4-yl.

In some embodiments, p = 2 and r =1. In some embodiments, $R_{18}$ is H, $C_{1-5}$ acyl or $C_{1-8}$ alkyl.

In some embodiments, $R_{10}$-$R_{13}$ are independently $C_{1-5}$ acyl, $C_{1-4}$ alkoxy, $C_{1-8}$ alkyl, $C_{1-4}$ alkylcarboxamide, $C_{1-4}$ alkylureyl, carbo-$C_{1-6}$-alkoxy, carboxamide, carboxy, cyano, $C_{3-6}$ cycloalkyl, halogen, $C_{1-4}$ haloalkoxy and $C_{1-4}$ haloalkyl.

In some embodiments, one or two $R_{10}$-$R_{13}$ groups are independently halogen. In some embodiments, one $R_{10}$-$R_{13}$ group is a halogen.

In some embodiments, $Ar_1$ is phenyl and $R_9$ is substituted at the para position on the phenyl.

In some embodiments, $Ar_1$ is phenyl optionally substituted with $R_9$, $R_{10}$ and $R_{11}$. In some embodiments, the compounds of the invention are of Formula (**Iw**):

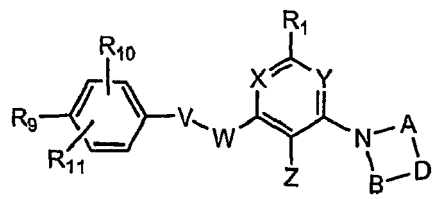

(Iw)

wherein A, B, D, V, W, X, Y, Z, $R_1$ have the same meaning as described herein, *supra* and *infra,* and $R_9$ is cyano, carbo-$C_{1-6}$-alkoxy, carboxy, $C_{2-6}$ dialkylamino, $C_{1-4}$ alkylcarboxamide, $C_{2-6}$ dialkylsulfonamide, $C_{1-4}$ alkylsulfonyl, hydroxyl, $C_{1-4}$ alkoxy, 5-membered heteroaryl, 6-membered heteroaryl, or heterocyclic, wherein the 6-membered heteoaryl is optionally an N-oxide; and $R_{10}$ and $R_{11}$ are independently H or halogen. In some embodiments, $R_9$ is cyano, carbomethoxy, carboethoxy, carboisopropoxy, carboxy, dimethylamino, diethylamino, methylethylamino, $C(O)NHCH_3$, $C(O)NHCH_2CH_3$, $C(O)NH(CH_3)_2$, $S(O)_2CH_3$, $S(O)_2CH_2CH_3$, hydroxyl, $OCH_3$, [1,2,4]triazol-4-yl, thiazol-2-yl, 3H-imidazol-4-yl, 1H-imidazol-2-yl, 1H-imidazol-4-yl, pyridin-2-yl, pyridin-3-yl, pyridin-4-yl, 1-oxy-pyridin-2-yl, 1-oxy-pyridin-3-yl, 1-oxy-pyridin-4-yl, or 2-oxo-oxazolidin-4-yl. In some embodiments, $R_{10}$ is H and $R_{11}$ is F.

In some embodiments, $Ar_1$ is phenyl and two adjacent $R_{10}$-$R_{11}$ groups form a 5, 6 or 7 membered cycloalkyl, cycloalkenyl or heterocyclic group with the phenyl group wherein the 5, 6 or 7 membered group is optionally substituted with halogen.

In some embodiments, $Ar_1$ is phenyl and two adjacent $R_{10}$-$R_{11}$ groups form a 5, 6 or 7 membered cycloalkyl group with the phenyl group and is of the formulae shown below:

**TABLE 5**

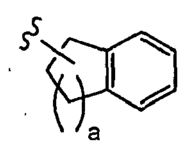 , 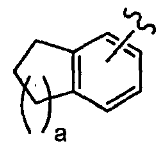

wherein "a" is 1, 2 or 3 to give a 5, 6 or 7 membered cycloalkyl fused together with the phenyl group where two of the ring carbons are shared between the cycloalkyl and phenyl group. In some embodiments, the cycloalkyl is optionally substituted with halogen. In some embodiments, the halogen is fluorine.

In some embodiments, $Ar_1$ is phenyl and two adjacent $R_{10}$-$R_{11}$ groups form a 5, 6 or 7 membered cycloalkenyl group with the phenyl group and is of the formulae shown in TABLE 5 and has at least one carbon-carbon ring double bond present that is not part of the phenyl group (i.e., cycloalkenyl), for example, 1*H*-Indenyl and dihydro-naphthyl. In some embodiments, the cycloalkenyl is optionally substituted with halogen. In some embodiments, the halogen is fluorine.

In some embodiments, $Ar_1$ is phenyl and two adjacent $R_{10}$-$R_{11}$ groups form a 5, 6 or 7 heterocyclic group with the phenyl group and is of the formulae in TABLE 5 wherein one or more ring cycloalkyl carbons are replaced by a O, S, S(O), $S(O)_2$, NH or N-$C_{1-4}$-alkyl group. In some embodiments, the heterocyclic group is optionally substituted with halogen. In some embodiments, the halogen is fluorine.

In some embodiments, $Ar_1$ is phenyl and two adjacent $R_{10}$-$R_{11}$ groups form a 5 membered heterocyclic group with the phenyl group. In some embodiments, the 5 membered heterocyclic group with the phenyl group together form a 2,3-dihydro-benzofuran-5-yl or benzo[1,3]dioxol-5-yl group. In some embodiments, the two adjacent groups form a 6 membered heterocyclic group with the phenyl group. In some embodiments, the 6 membered heterocyclic group with the phenyl group together form a 2,3-dihydro-benzo[1,4]dioxin-6-yl or 2,3-dihydro-benzo[1,4]dioxin-2-yl group. In some embodiments, the two adjacent groups form a 7 membered heterocyclic group with the phenyl group. In some embodiments, the 7 membered heterocyclic group with the phenyl group together form a 3,4-dihydro-2H-benzo[b][1,4]dioxepin-7-yl group.

In some embodiments, $Ar_1$ is heteroaryl and two adjacent $R_{10}$-$R_{11}$ groups form a 5, 6 or 7 membered cycloalkyl, cycloalkenyl or heterocyclic group with the heteroaryl group wherein the 5, 6 or 7 membered group is optionally substituted with halogen. In some embodiments, $Ar_1$ is a heteroaryl selected from TABLE 2A. In some embodiments, $Ar_1$ is a heteroaryl selected from TABLE 4. In some embodiments, the two adjacent groups form a 5 membered heterocyclic group with the heteroaryl group. In some embodiments, the two adjacent groups form a 6 membered heterocyclic group with the heteroaryl group. In some embodiments, the two adjacent groups form a 7 membered heterocyclic group with the heteroaryl group.

In some embodiments, $R_5$ is H, $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, arylsulfonyl, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, heterocyclic, hydroxyl, nitro, $C_{4-7}$ oxo-cycloalkyl, sulfonamide and nitro.

In some embodiments, $R_5$ and $R_6$ are independently H or F.

In some embodiments, X is N and Y is CH.

In some embodiments, X is N and Y is CF.

In some embodiments, X is CH and Y is N.

In some embodiments, X and Y are N.

In some embodiments, X and Y are CH.

In some embodiments, X is CH and Y is CF.

Some embodiments of the present invention include compounds illustrated in TABLES A, B, C, D and E; these TABLES are shown below.

**TABLE A**

| Cmpd# | Structure | Chemical Name |
|---|---|---|
| A1 | 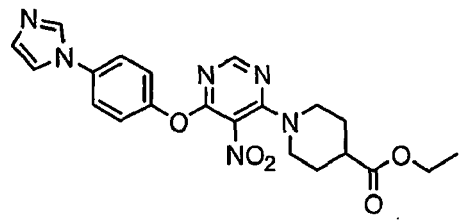 | 1-[6-(4-Imidazol-1-yl-phenoxy)-5-nitro-pyrimidin-4-yl]-piperidine-4-carboxylic acid ethyl ester |
| A2 | 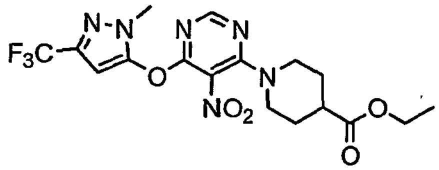 | 1-[6-(2-Methyl-5-trifluoromethyl-2H-pyrazol-3-yloxy)-5-nitro-pyrimidin-4-yl]-piperidine-4-carboxylic acid ethyl ester |
| A3 | 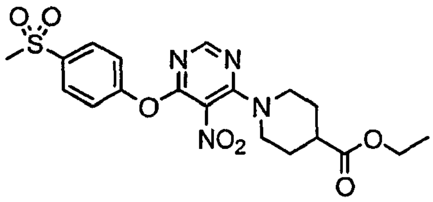 | 1-[6-(4-Methanesulfonyl-phenoxy)-5-nitro-pyrimidin-4-yl]-piperidine-4-carboxylic acid ethyl ester |
| A4 | 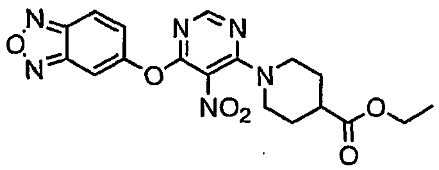 | 1-[6-(Benzo[1,2,5]oxadiazol-5-yloxy)-5-nitro-pyrimidin-4-yl]-piperidine-4-carboxylic acid ethyl ester |
| A5 | 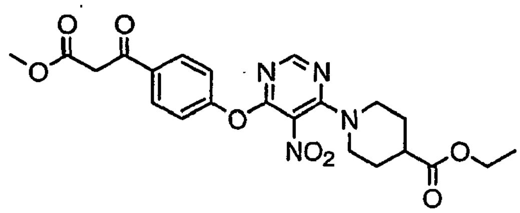 | 1-{6-[4-(2-Methoxycarbonyl-acetyl)-phenoxy]-5-nito-pyrimidin-4-yl}-piperidine-4-carboxylic acid ethyl ester |
| A6 | 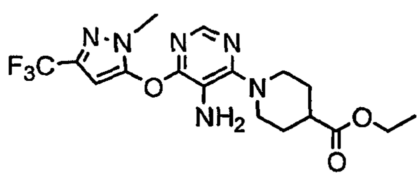 | 1-[5-Amino-6-(2-methyl-5-trifluoromethyl-2H-pyrazol-3-yloxy)-pyrimidin-4-yl]-piperidine-4-carboxylic acid ethyl ester |
| A7 | 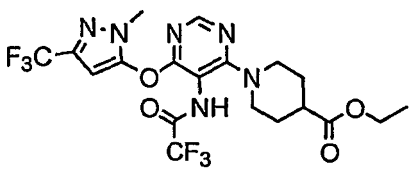 | 1-[6-(2-Methyl-5-trifluoromethyl-2H-pyrazol-3-yloxy)-5-(2,2,2-trifluoro-acetylamino)-pyrimidin-4-yl]-piperidine-4-carboxylic acid ethyl ester |

(continued)

| Cmpd# | Structure | Chemical Name |
|---|---|---|
| A8 | 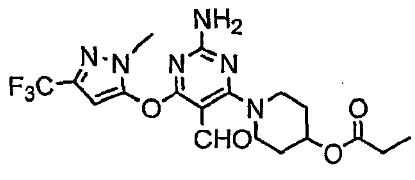 | Propionic acid 1-[2-amino-5-formyl-6-(2-methyl-5-trifluoromethyl-2H-pyrazol-3-yloxy)-pyrimidin-4-yl]-piperidin-4-yl ester |
| A9 | 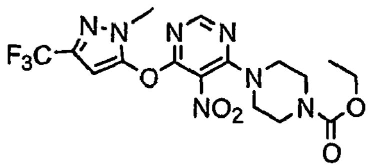 | 4-[6-(2-Methyl-5-trifluoromethyl-2H-pyrazol-3-yloxy)-5-nitro-pyrimidin-4-yl]-piperazine-1-carboxylic acid ethyl ester |
| A10 | 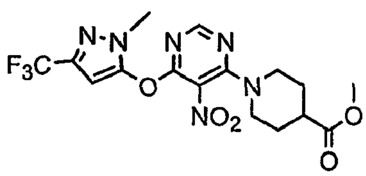 | 1-[6-(2-Methyl-5-trifluoromethyl-2H-pyrazol-3-yloxy)-5-nitro-pyrimidin-4-yl]-piperidine-4-carboxylic acid methyl ester |
| A11 | 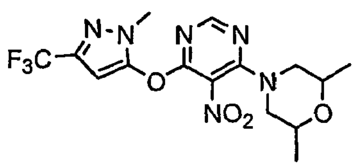 | 2,6-Dimethyl-4-[6-(2-methyl-5-trifluoromethyl-2H-pyrazol-3-yloxy)-5-nitro-pyrimidin-4-yl]-morpholine |
| A12 | 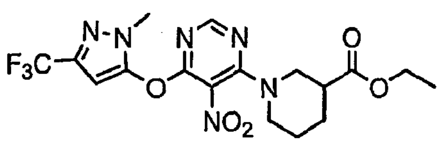 | 1-[6-(2-Methyl-5-trifluoromethyl-2H-pyrazol-3-yloxy)-5-nitro-pyrimidin-4-yl]-piperidine-3-carboxylic acid ethyl ester |
| A13 | 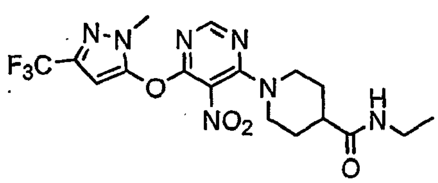 | 1-[6-(2-Methyl-5-trifluoromethyl-2H-pyrazol-3-yloxy)-5-nitro-pyrimidin-4-yl]-piperidine-4-carboxylic acid ethylamide |
| A14 | 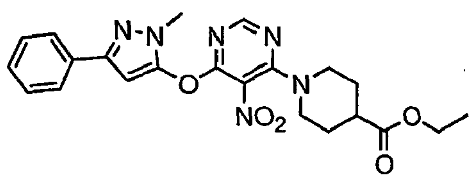 | 1-[6-(2-Methyl-5-phenyl-2H-pyrazol-3-yloxy)-5-nitro-pyrimidin-4-yl]-piperidine-4-carboxylic acid ethyl ester |
| A15 | 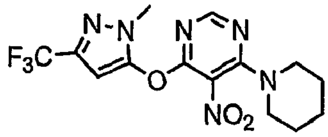 | 4-(2-Methyl-5-trifluoromethyl-2H-pyrazol-3-yloxy)-5-nitro-6-piperidin-1-yl-pyrimidine |

(continued)

| Cmpd# | Structure | Chemical Name |
|---|---|---|
| A16 | 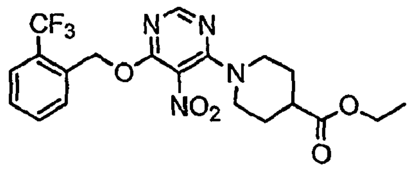 | 1-[5-Nitro-6-(2-trifluoromethyl-benzyloxy)-pyrimidin-4-yl]-piperidine-4-carboxylic acid ethyl ester |
| A17 | 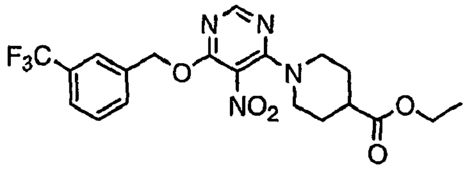 | 1-[5-Nitro-6-(3-trifluoromethyl-benzyloxy)-pyrimidin-4-yl]-piperidine-4-carboxylic acid ethyl ester |
| A18 | 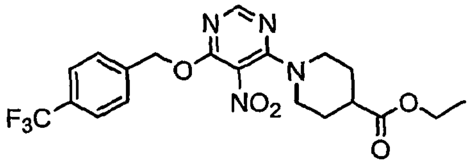 | 1-[5-Nitro-6-(4-trifluoromethyl-benzyloxy)-pyrimidin-4-yl]-piperidine-4-carboxylic acid ethyl ester |
| A19 | 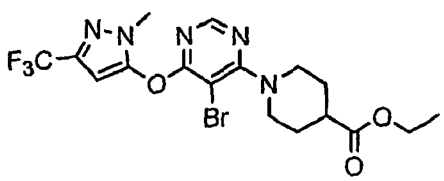 | 1-[5-Bromo-6-(2-methyl-5-trifluoromethyl-2H-pyrazol-3-yloxy)-pyrimidin-4-yl]-piperidine-4-carboxylic acid ethyl ester |
| A20 | 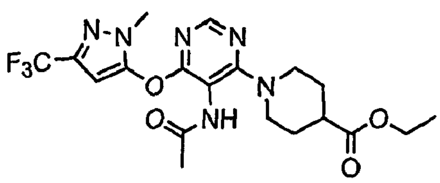 | 1-[5-Acetylamino-6-(2-methyl-5-trifluoromethyl-2H-pyrazol-3-yloxy)-pyrimidin-4-yl]-piperidine-4-carboxylic acid ethyl ester |
| A21 | 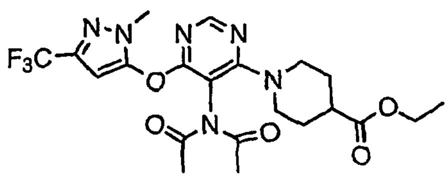 | 1-[5-Diacetylamino-6-(2-methyl-5-trifluoromethyl-2H-pyirazol-3-yloxy)-pyrimidin-4-yl]-piperidine-4-carboxylic acid ethyl ester |
| A22 | 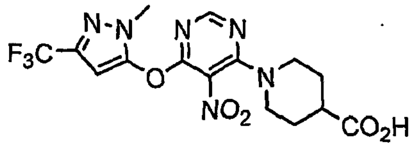 | 1-[6-(2-Methyl-5-trifluoromethyl-2H-pyrazol-3-yloxy)-5-nitro-pyrimidin-4-yl]-piperidine-4-carboxylic acid |
| A23 | 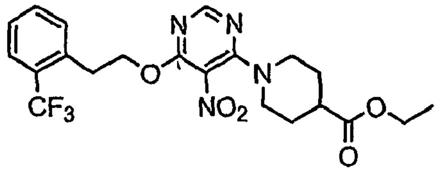 | 1-{5-Nitro-6-[2-(2-trifluoromethyl-phenyl)-ethoxy]-pyrimidin-4-yl}-piperidine-4-carboxylic acid ethyl ester |

(continued)

| Cmpd# | Structure | Chemical Name |
|---|---|---|
| A24 | 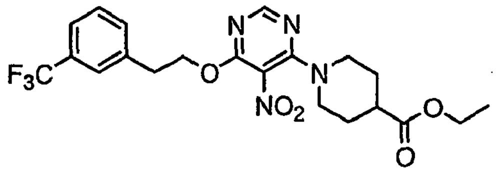 | 1-{5-Nitro-6-[2-(3-trifluoromethyl-phenyl)-ethoxy]-pyrimidin-4-yl}-piperidine-4-carboxylic acid ethyl ester |
| A25 | 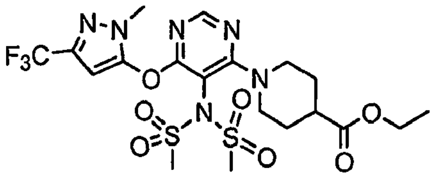 | 1-[5-Di-(methanesulfonyl)amino-6-(2-methyl-5-trifluoromethyl-2H-pyrazol-3-yloxy)-pyrimidin-4-yl]-piperidine-4-carboxylic acid ethyl ester |
| A26 | 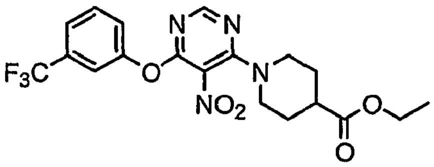 | 1-[5-Nitro-6-(3-trifluoromethyl-phenoxy)-pyrimidin-4-yl]-piperidine-4-carboxylic acid ethyl ester |
| A27 | 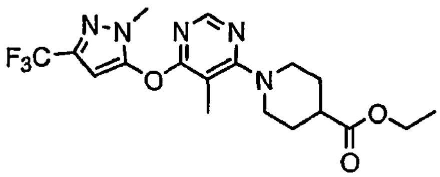 | 1-[5-Methyl-6-(2-methyl-5-trifluoromethyl-2H-pyrazol-3-yloxy)-pyrimidin-4-yl]-piperidine-4-carboxylic acid ethyl ester |
| A28 | 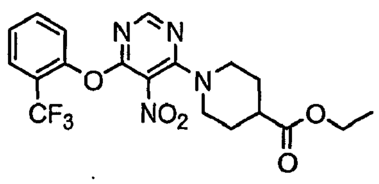 | 1-[5-Nitro-6-(2-trifluoromethyl-phenoxy)-pyrimidin-4-yl]-piperidine-4-carboxylic acid ethyl ester |
| A29 | 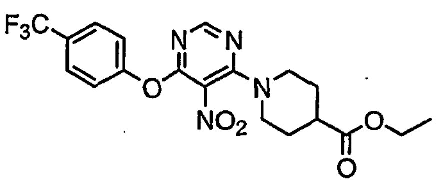 | 1-[5-Nitro-6-(4-trifluoromethyl-phenoxy)-pyrimidin-4-yl]-piperidine-4-carboxylic acid ethyl ester |
| A30 | 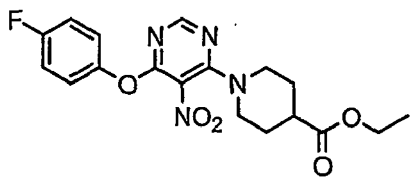 | 1-[6-(4-Fluoro-phenoxy)-5-nitro-pyrimidin-4-yl]-piperidine-4-carboxylic acid ethyl ester |
| A31 | 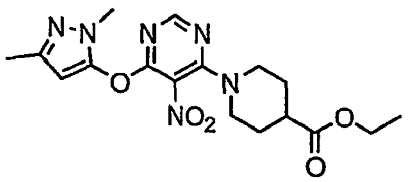 | 1-[6-(2,5-Dimethyl-2H-pyrazol-3-yloxy)-5-nitro-pyrimidin-4-yl]-piperidine-4-carboxylic acid ethyl ester |

(continued)

| Cmpd# | Structure | Chemical Name |
|---|---|---|
| A32 | 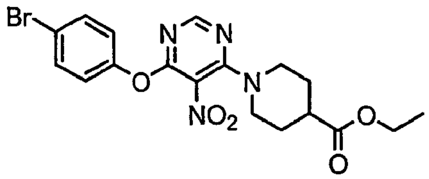 | 1-[6-(4-Bromo-phenoxy)-5-nitro-pyrimidin-4-yl]-piperidine-4-carboxylic acid ethyl ester |
| A33 | 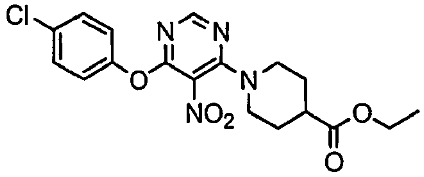 | 1-[6-(4-Chloro-phenoxy)-5-nitro-pyrimidin-4-yl]-piperidine-4-carboxylic acid ethyl ester |
| A34 | 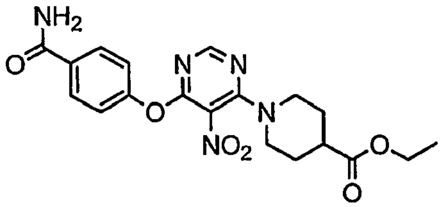 | 1-[6-(4-Carbamoyl-phenoxy)-5-nitro-pyrimidin-4-yl]-piperidine-4-carboxylic acid ethyl ester |
| A35 | 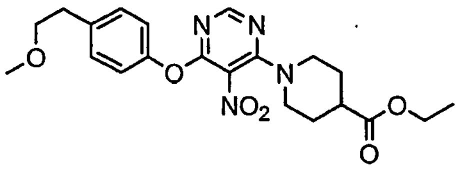 | 1-{6-[4-(2-Methoxy-ethyl)-phenoxy]-5-nito-pyrimidin-4-yl}-piperidine-4-carboxylic acid ethyl ester |
| A36 | 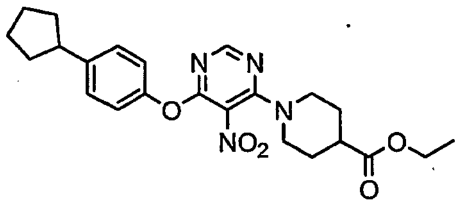 | 1-[6-(4-Cyclopentyl-phenoxy)-5-nitro-pyrimidin-4-yl]-piperidine-4-carboxylic acid ethyl ester |
| A37 | 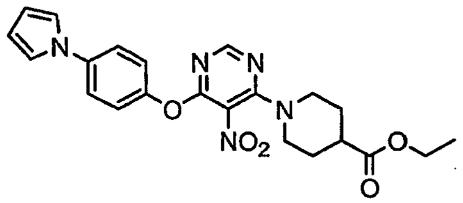 | 1-[5-Nitro-6-(4-pyrrol-1-yl-phenoxy)-pyrimidin-4-yl]-piperidine-4-carboxylic acid ethyl ester |
| A38 | 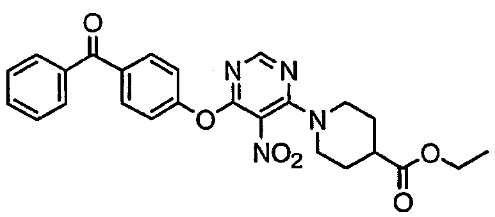 | 1-[6-(4-Benzoyl-phenoxy)-5-nitro-pyrimidin-4-yl]-piperidine-4-carboxylic acid ethyl ester |

(continued)

| Cmpd# | Structure | Chemical Name |
|---|---|---|
| A39 | 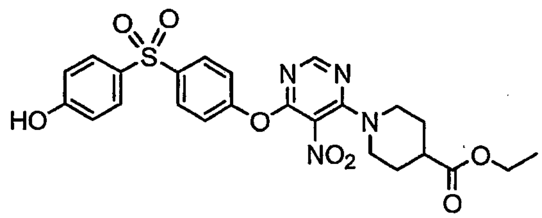 | 1-{6-[4-(4-Hydroxy-benzenesulfonyl)-phenoxy]-5-nitro-pyrimidin-4-yl}-piperidine-4-carboxylic acid ethyl ester |
| A40 | 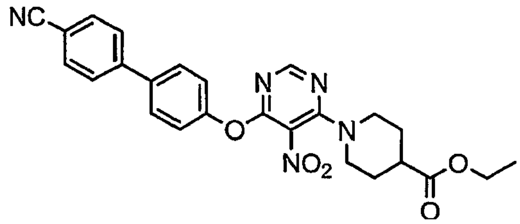 | 1-[6-(4'-Cyano-biphenyl-4-yloxy)-5-nitro-pyrimidin-4-yl]-piperidine-4-carboxylic acid ethyl ester |
| A41 | 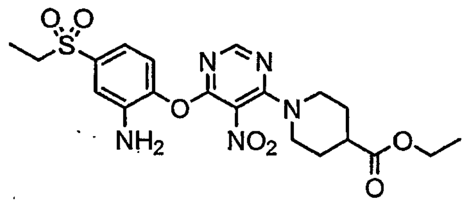 | 1-[6-(2-Amino-4-ethanesulfonyl-phenoxy)-5-nitro-pyrimidin-4-yl]-piperidine-4-carboxylic acid ethyl ester |
| A42 | 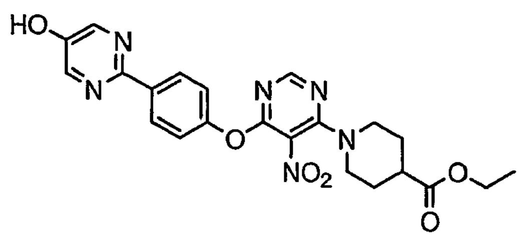 | 1-{6-[4-(5-Hydroxy-pyrimidin-2-yl)-phenoxy]-5-nitro-pyrimidin-4-yl}-piperidine-4-carboxylic acid ethyl ester |
| A43 | 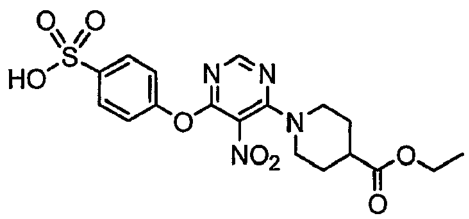 | 1-[5-Nitro-6-(4-sulfo-phenoxy)-pyrimidin-4-yl]-piperidine-4-carboxylic acid ethyl ester |
| A44 | 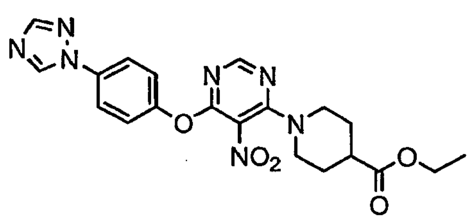 | 1-[5-Nitro-6-(4-[1,2,4]triazol-1-yl-phenoxy)-pyrimidin-4-yl]-piperidine-4-carboxylic acid ethyl ester |
| A45 | 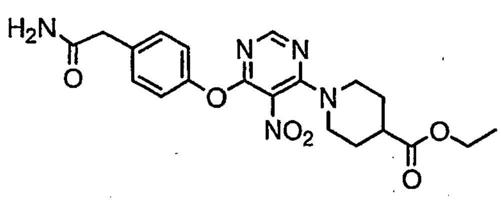 | 1-[6-(4-Carbamoylmethyl-phenoxy)-5-nitro-pyrimidin-4-yl]-piperidine-4-carboxylic acid ethyl ester |

(continued)

| Cmpd# | Structure | Chemical Name |
|---|---|---|
| A46 | 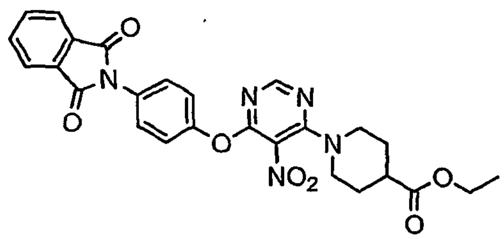 | 1-{6-[4-(1,3-Dioxo-1,3-dihydro-isoindol-2-yl)-phenoxy]-5-nitro-pyrimidin-4-yl} -piperidine-4-carboxylic acid ethyl ester |
| A47 | 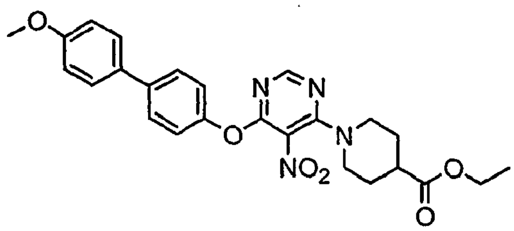 | 1-[6-(4'-Methoxy-biphenyl-4-yloxy)-5-nitro-pyrimidin-4-yl]-piperidine-4-carboxylic acid ethyl ester |
| A48 | 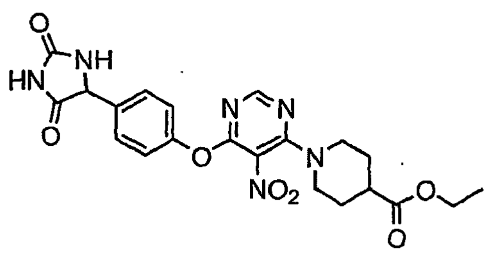 | 1-{6-[4-(2,5-Dioxo-imidazolidin-4-yl)-phenoxy]-5-nitro-pyrimidin-4-yl} -piperidine-4-carboxylic acid ethyl ester |
| A49 | 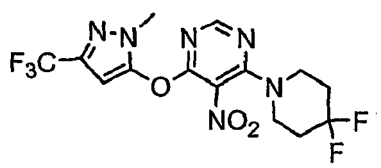 | 4-(4,4-Difluoro-piperidin-1-yl)-6-(2-methyl-5-trifluoromethyl-2H-pyrazol-3-yloxy)-5-nitro-pyrimidine |
| A50 | 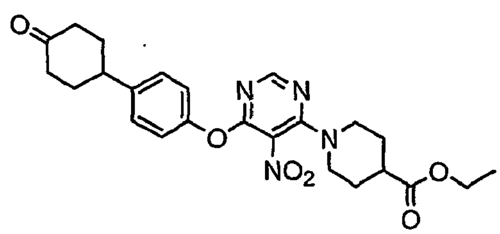 | 1-{5-Nitro-6-[4-(4-oxo-cyclohexyl)-phenoxy]-pyrimidin-4-yl}-piperidine-4-carboxylic acid ethyl ester |
| A51 | 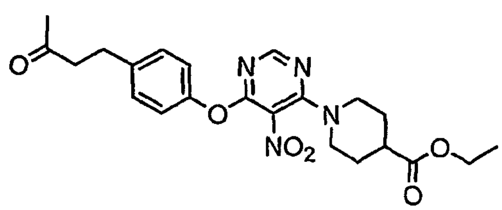 | 1-{5-Nitro-6-[4-(3-oxo-butyl)-phenoxy]-pyrimidin-4-yl}-piperidine-4-carboxylic acid ethyl ester |

(continued)

| Cmpd# | Structure | Chemical Name |
|---|---|---|
| A52 | 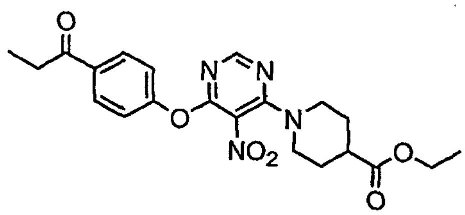 | 1-[5-Nitro-6-(4-propionyl-phenoxy)-pyrimidin-4-yl]-piperidine-4-carboxylic acid ethyl ester |
| A53 | 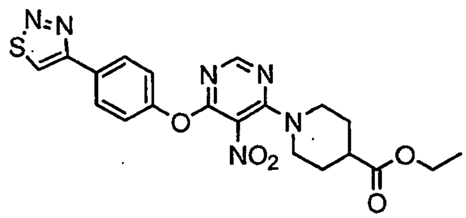 | 1-[5-Nitro-6-(4-[1,2,3]thiadiazol-4-yl-phenoxy)-pyrimidin-4-yl]-piperidine-4-carboxylic acid ethyl ester |
| A54 | 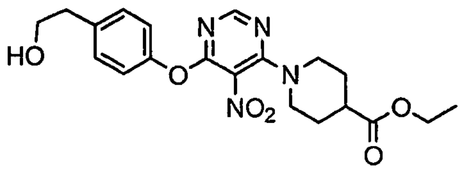 | 1-{6-[4-(2-Hydroxy-ethyl)-phenoxy]-5-nitro-pyrimidin-4-yl}-piperidine-4-carboxylic acid ethyl ester |
| A55 | 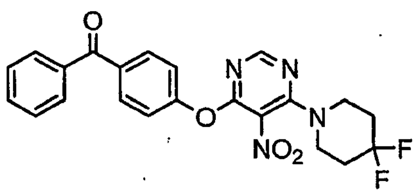 | {4-[6-(4,4-Difluoro-piperidin-1-yl)-5-nitro-pyrimidin-4-yloxy]-phenyl}-phenyl-methanone |
| A56 | 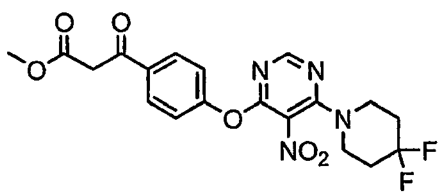 | 3-{4-[6-(4,4-Difluoro-piperidin-1-yl)-5-nitro-pyrimidein-4-yloxy]-phenyl}-3-oxo-propionic acid methyl ester |
| A57 | 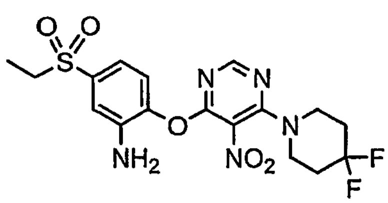 | 2-[6-(4,4-Difluoro-piperidin-1-yl)-5-nitro-pyrimidin-4-yloxy]-5-ethanesulfonyl-phenylamine |
| A58 | 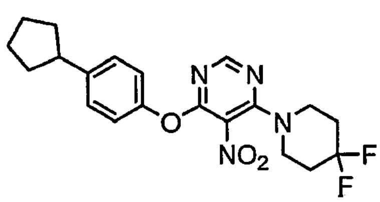 | 4-(4-Cyclopentyl-phenoxy)-6-(4,4-difluoro-piperidin-1-yl)-5-nitro-pyrimidine |

(continued)

| Cmpd# | Structure | Chemical Name |
|---|---|---|
| A59 | 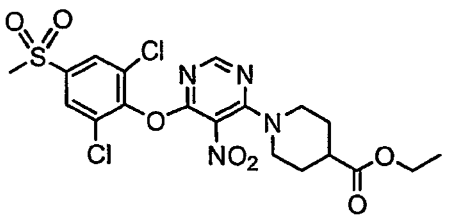 | 1-[6-(2,6-Dichloro-4-methanesulfonyl-phenoxy)-5-nitro-pyrimidin-4-yl]-piperidine-4-carboxylic acid ethyl ester |
| A60 | 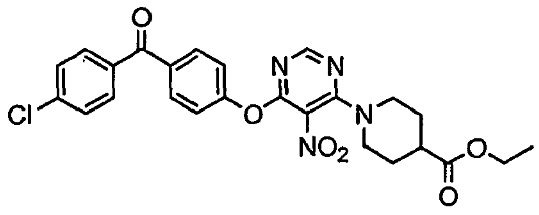 | 1-{6-[4-(4-Chloro-benzoyl)-phenoxy]-5-nitro-pyrimidin-4-yl}-piperidine-4-carboxylic acid ethyl ester |
| A61 | 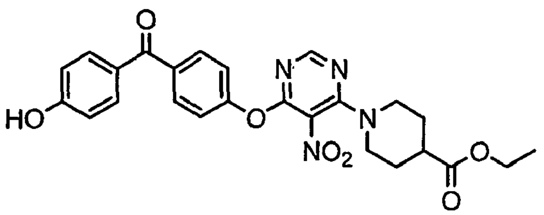 | 1-{6-[4-(4-Hydroxy-benzoyl)-phenoxy]-5-nitro-pyrimidin-4-yl}-piperidine-4-carboxylic acid ethyl ester |
| A62 | 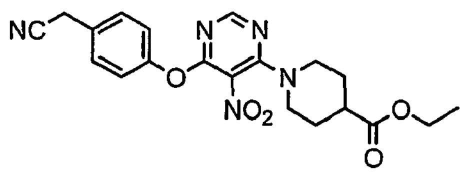 | 1-[6-(4-Cyanomethyl-phenoxy)-5-nitro-pyrimidin-4-yl]-piperidine-4-carboxylic acid ethyl ester |
| A63 | 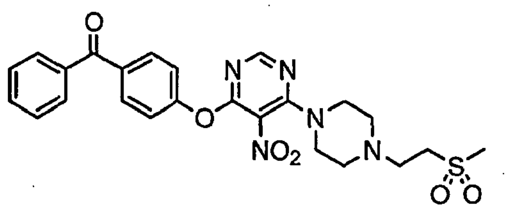 | (4-{6-[4-(2-Methanesulfonyl-ethyl)-piperazin-1-yl]-5-nitro-pyrimidin-4-yloxy}-phenyl)-phenyl-methanone |
| A64 | 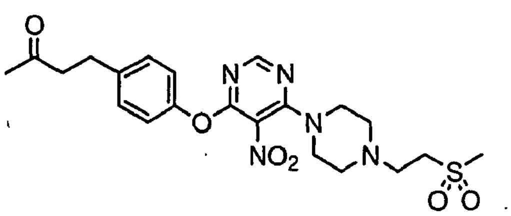 | 4-(4-{6-[4-(2-Methanesulfonyl-ethyl)-piperazin-1-yl]-5-nitro-pyrimidin-4-yloxy}-phenyl)-butan-2-one |
| A65 | 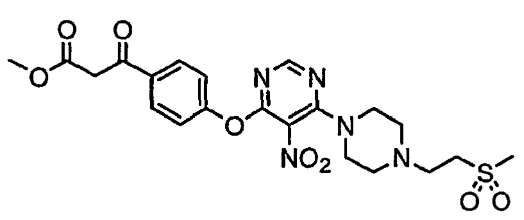 | 3-(4-{6-[4-(2-Methanesulfonyl-ethyl)-piperazin-1-yl]-5-nitro-pyrimidin-4-yloxy}-phenyl)-3-oxo-propionic acid methyl ester |

(continued)

| Cmpd# | Structure | Chemical Name |
|---|---|---|
| A66 | 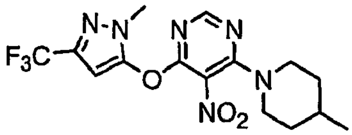 | 4-(4-Methyl-piperidin-1-yl)-6-(2-methyl-5-trifluoromethyl-2H-pyrazol-3-yloxy)-5-nitro-pyrimidine |
| A67 | 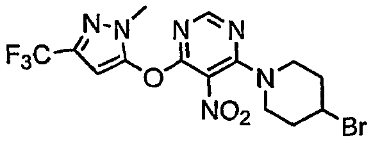 | 4-(4-Bromo-piperidin-1-yl)-6-(2-methyl-5-trifluoromethyl-2H-pyrazol-3-yloxy)-5-nitro-pyrimidine |
| A68 | 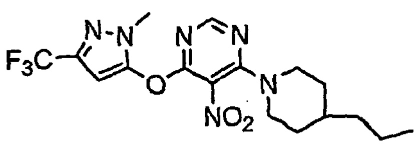 | 4-(2-Methyl-5-tnifluoromethyl-2H-pyrazol-3-yloxy)-5-nitro-6-(4-propyl-piperidin-1-yl)-pyrimidine |
| A69 | 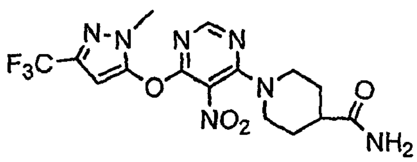 | 1-[6-(2-Methyl-5-trifluoromethyl-2H-pyrazol-3-yloxy)-5-nitro-pyrimidin-4-yl]-piperidine-4-carboxylic acid amide |
| A70 | 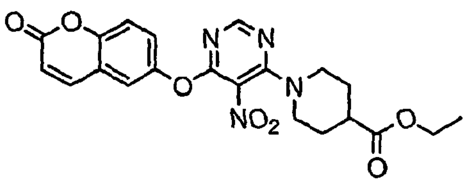 | 1-[5-Nitro-6-(2-oxo-2H-chromen-6-yloxy)-pyrimidin-4-yl]-piperidine-4-carboxylic acid ethyl ester |
| A71 | 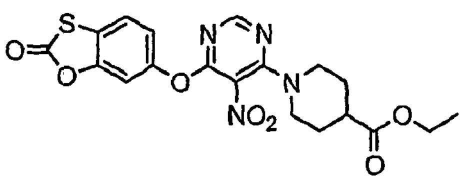 | 1-[5-Nitro-6-(2-oxo-benzo[1,3]oxathiol-6-yloxy)-pyrimidin-4-yl]-piperidine-4-carboxylic acid ethyl ester |
| A72 | 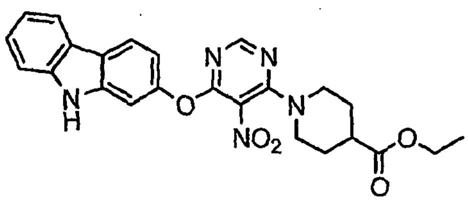 | 1-[6-(9H-Carbazol-2-yloxy)-5-nitro-pyrimidin-4-yl]-piperidine-4-carboxylic acid ethyl ester |
| A73 | 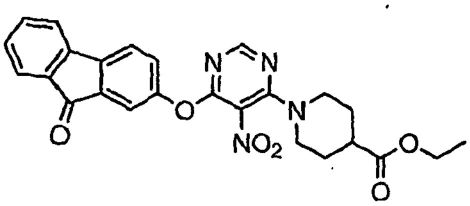 | 1-[5-Nitro-6-(9-oxo-9H-fluoren-2-yloxy)-pyrimidin-4-yl]-piperidine-4-carboxylic acid ethyl ester |

(continued)

| Cmpd# | Structure | Chemical Name |
|---|---|---|
| A74 | 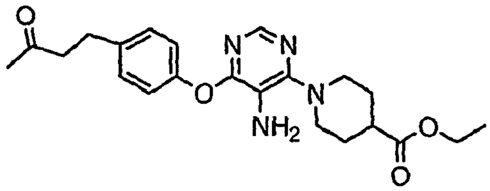 | 1-{5-Amino-6-[4-(3-oxo-butyl)-phenoxy]-pyrimidin-4-yl}-piperidine-4-carboxylic acid ethyl ester |
| A75 | 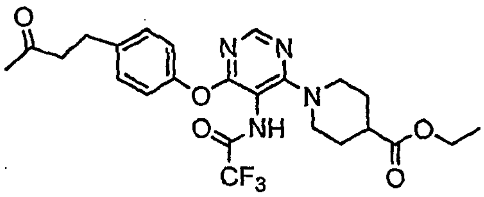 | 1-[6-[4-(3-Oxo-butyl)-phenoxy]-5-(2,2,2-trifluoro-acetylamino)-pyrimidin-4-yl]-piperidine-4-carboxylic acid ethyl ester |
| A76 | 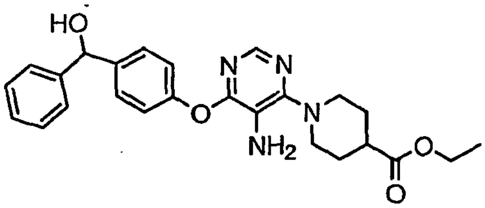 | 1-{5-Amino-6-[4-(hydroxy-phenyl-methyl)-phenoxy]-pyrimidm-4-yl} -piperidine-4-carboxylic acid ethyl ester |
| A77 | 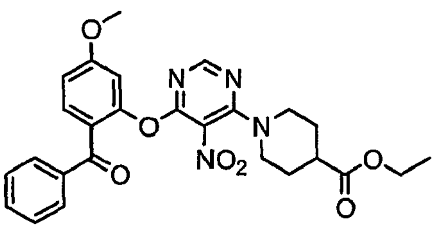 | 1-[6-(2-Benzoyl-5-methoxy-phenoxy)-5-nitro-pyrimidin-4-yl]-piperidine-4-carboxylic acid ethyl ester |
| A78 | 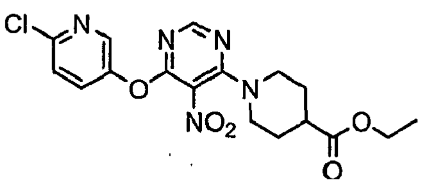 | 1-[6-(6-Chloro-pyridin-3-yloxy)-5-nitro-pyrimidin-4-yl]-piperidine-4-carboxylic acid ethyl ester |
| A79 | 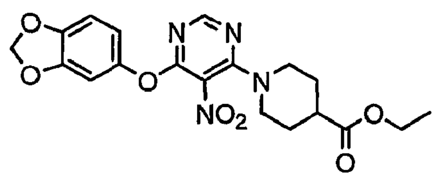 | 1-[O-(Benzo[1,3]dioxol-5-yloxy)-5-nitro-pyrimidin-4-yl]-piperidine-4-carboxylic acid ethyl ester |
| A80 | 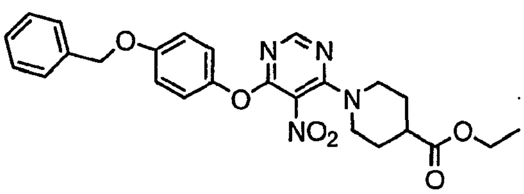 | 1-[6-(4-Benzyloxy-phenoxy)-5-nitro-pyrimidin-4-yl]-piperidine-4-carboxylic acid ethyl ester |

(continued)

| Cmpd# | Structure | Chemical Name |
|---|---|---|
| A81 | 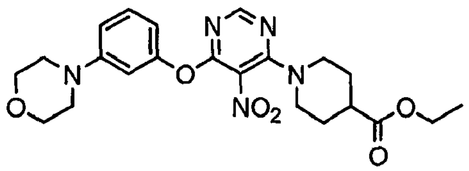 | 1-[6-(3-Morpholin-4-yl-phenoxy)-5-nitro-pyrimidin-4-yl]-piperidine-4-carboxylic acid ethyl ester |
| A82 | 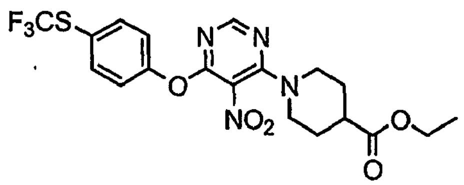 | 1-[5-Nitro-6-(4-trifluoromethylsulfanyl-phenoxy)-pyrimidin-4-yl]-piperidine-4-carboxylic acid ethyl ester |
| A83 | 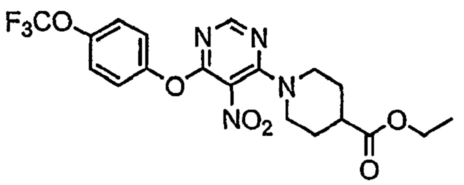 | 1-[5-Nitro-6-(4-trifluoromethoxy-phenoxy)-pyrimidin-4-yl]-piperidine-4-carboxylic acid ethyl ester |
| A84 | 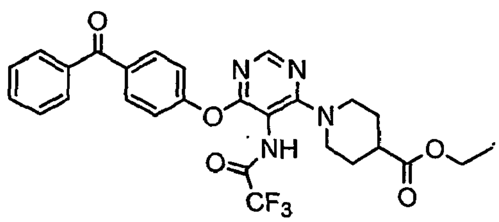 | 1-[6-(4-Benzoyl-phenoxy)-5-(2,2,2-trifluoro-acetylamino)-pyrimidin-4-yl]-piperidine-4-carboxylic acid ethyl ester |
| A85 | 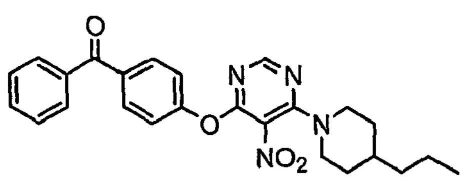 | {4-[5-Nitro-6-(4-propyl-piperidin-1-yl)-pyrimidin-4-yloxy]-phenyl}-phenyl-methanone |
| A86 | 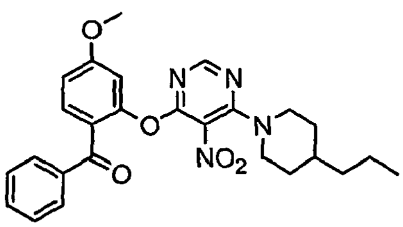 | {4-Methoxy-2-[5-nitro-6-(4-propyl-piperidin-1-yl)-pyrimidin-4-yloxy]-phenyl}-phenyl-methanone |
| A87 | 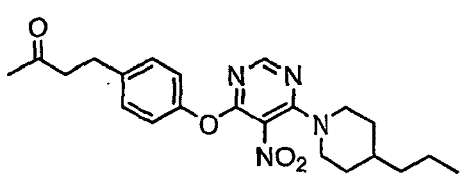 | 4-{4-[5-Nitro-6-(4-propyl-piperidin-1-yl)-pyrimidin-4-yloxy]-phenyl} -butan-2-one |

(continued)

| Cmpd# | Structure | Chemical Name |
|---|---|---|
| A88 | 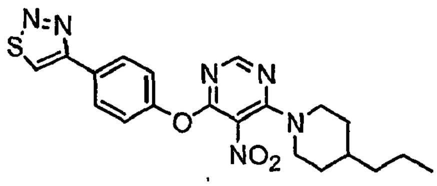 | 5-Nitro-4-(4-propyl-piperidin-1-yl)-6-(4-[1,2,3] thiadiazol-4-yl-phenoxy)-pyrimidine |
| A89 | 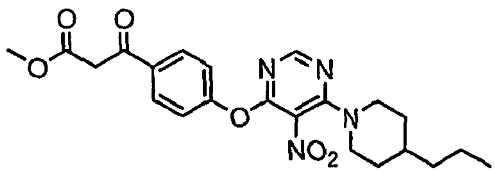 | 3- {4-[5-Nitro-6-(4-propyl-piperidin-1-yl)-pyrimidin-4-yloxy]-phenyl}-3-oxo-propionic acid methyl ester |
| A90 | 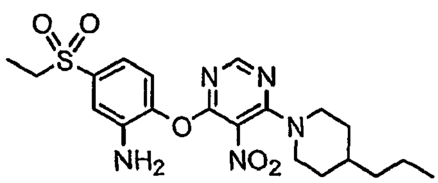 | 5-Ethanesulfonyl-2-[5-nitro-6-(4-propyl-piperidin-1-yl)-pyrimidin-4-yloxy]-phenylamine |
| A91 | 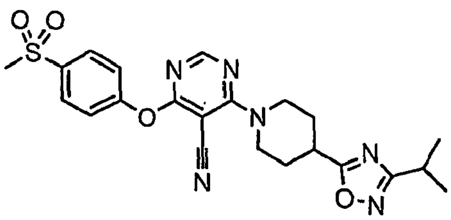 | 4-[4-(3-Isopropyl-[1,2,4]oxadiazol-5-yl)-piperidin-1-yl]-6-(4-methanesulfonyl-phenoxy)-pyrimidine-5-carbonitrile |
| A92 | 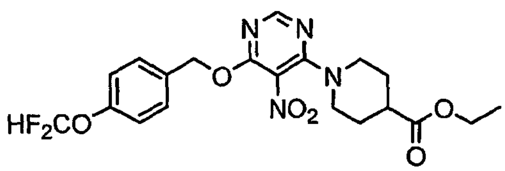 | 1-[6-(4-Difluoromethoxy-benzyloxy)-5-nitro-pyrimidin-4-yl]-piperidine-4-carboxylic acid ethyl ester |
| A93 | 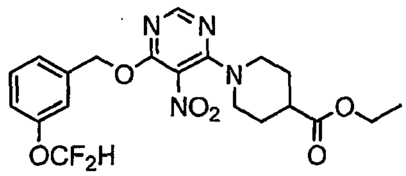 | 1-[6-(3-Difluoromethoxy-benzyloxy)-5-nitro-pyrimidin-4-yl]-piperidine-4-carboxylic acid ethyl ester |
| A94 | 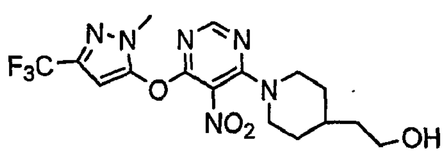 | 2-{1-[6-(2-Methyl-5-trifluoromethyl-2H-pyrazol-3-yloxy)-5-nitro-pyrimidin-4-yl]-piperidin-4-yl} -ethanol |
| A95 | 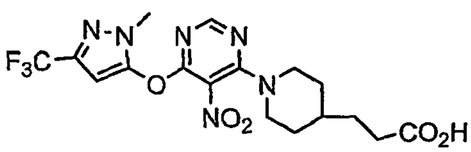 | 3-{1-[6-(2-Methyl-5-trifluoromethyl-2H-pyrazol-3-yloxy)-5-nitro-pyrimidin-4-yl]-piperidin-4-yl}-propionic acid |

(continued)

| Cmpd# | Structure | Chemical Name |
|---|---|---|
| A96 | 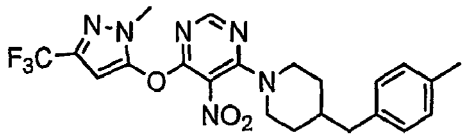 | 4-[4-(4-Methyl-benzyl)-piperidin-1-yl]-6-(2-methyl-5-trifluoromethyl-2H-pyrazol-3-yloxy)-5-nitro-pyrimidine |
| A97 | 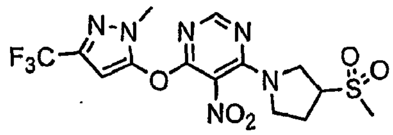 | 4-(3-Methanesulfonyl-pyrrolidin-1-yl)-6-(2-methyl-5-trifluoromethyl-2H-pyrazol-3-yloxy)-5-nitro-pyrimidine |
| A98 | 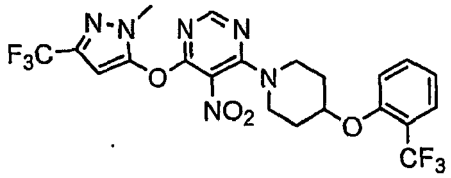 | 4-(2-Methyl-5-trifluoromethyl-2H-pyrazol-3-yloxy)-5-nitro-6-[4-(2-trifluoromethyl-phenoxy)-piperidin-1-yl]-pyrimidine |
| A99 | 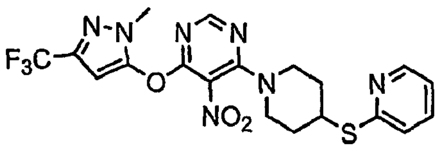 | 4-(2-Methyl-5-trifluoromethyl-2H-pyrazol-3-yloxy)-5-nitro-6-[4-(pyridin-2-ylsulfanyl)-piperidin-1-yl]-pyrimidine |
| A100 | 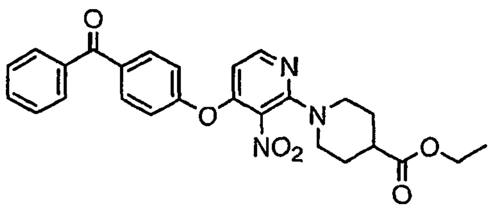 | 4'-(4-Benzoyl-phenoxy)-3'-nitro-3,4,5,6-tetrahydro-2H-[1,2']bipyridinyl-4-carboxylic acid ethyl ester |
| A101 | 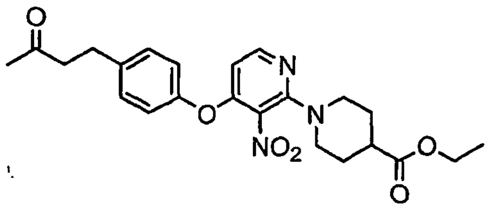 | 3'-Nitro-4'-[4-(3-oxo-butyl)-phenoxy]-3,4,5,6-tetrahydro-2H-[1,2']bipyridinyl-4-carboxylic acid ethyl ester |
| A102 | 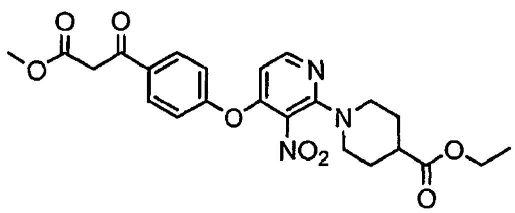 | 4'-[4-(2-Methoxycarbonyl-acetyl)-phenoxy]-3'-nitro-3,4,5,6-tetrahydro-2H-[1,2']bipyridinyl-4-carboxylic acid ethyl ester |
| A103 | 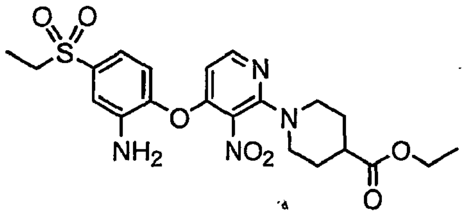 | 4'-(2-Amino-4-ethanesulfonyl-phenoxy)-3'-nitro-3,4,5,6-tetrahydro-2H-[1,2']bipyridinyl-4-carboxylic acid ethyl ester |

(continued)

| Cmpd# | Structure | Chemical Name |
|---|---|---|
| A104 | 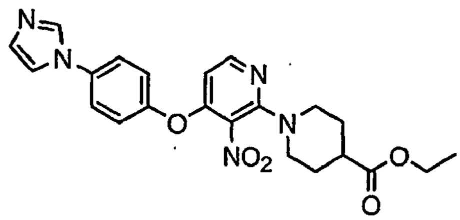 | 4'-(4-Imidazol-1-yl-phenoxy)-3'-nitro-3,4,5,6-tetrahydro-2H-[1,2']bipyridinyl-4-carboxylic acid ethyl ester |
| A105 | 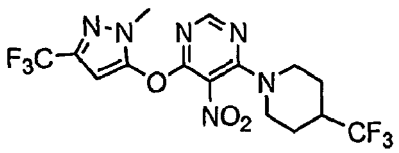 | 4-(2-Methyl-5-trifluoromethyl-2H-pyrazol-3-yloxy)-5-nitro-6-(4-trifluoromethyl-piperidin-1-yl)-pyrimidine |
| A106 | 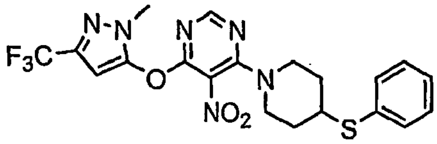 | 4-(2-Methyl-5-trifluoromethyl-2H-pyrazol-3-yloxy)-5-nitro-6-(4-phenylsulfanyl-piperidin-1-yl)-pyrimidine |
| A107 | 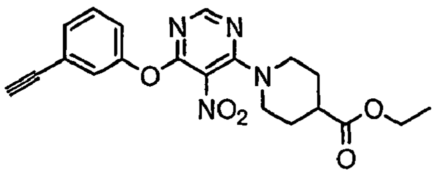 | 1-[6-(3-Ethynyl-phenoxy)-5-nitro-pyrimidin-4-yl]-piperidine-4-carboxylic acid ethyl ester |
| A108 | 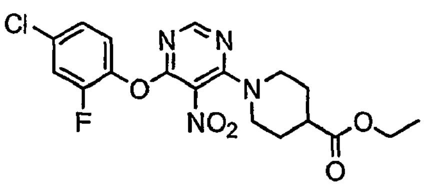 | 1-[6-(4-Chloro-2-fluoro-phenoxy)-5-nitro-pyrimidin-4-yl]-piperidine-4-carboxylic acid ethyl ester |
| A109 | 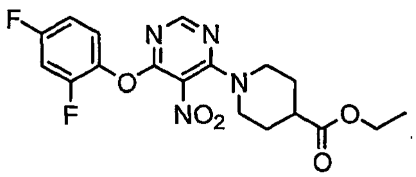 | 1-[6-(2,4-Difluoro-phenoxy)-5-nitro-pyrimidin-4-yl]-piperidine-4-carboxylic acid ethyl ester |
| A110 | 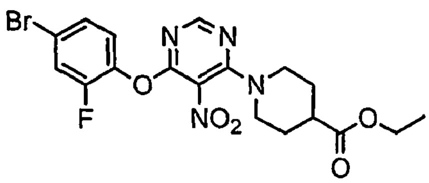 | 1-[6-(4-Bromo-2-fluoro-phenoxy)-5-nitro-pyrimidin-4-yl]-piperidine-4-carboxylic acid ethyl ester |
| A111 | 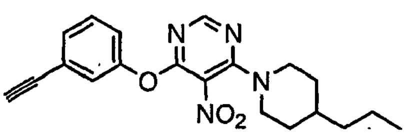 | 4-(3-Ethynyl-phenoxy)-5-nitro-6-(4-propyl-piperidin-1-yl)-pyrimidine |

(continued)

| Cmpd# | Structure | Chemical Name |
|---|---|---|
| A112 | 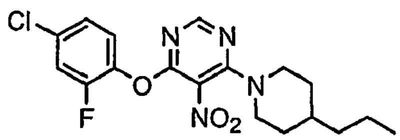 | 4-(4-Chloro-2-fluoro-phenoxy)-5-nitro-6-(4-propyl-piperidin-1-yl)-pyrimidine |
| A113 | 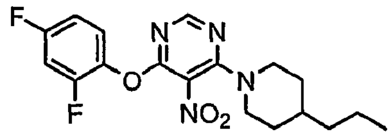 | 4-(2,4-Difluoro-phenoxy)-5-nitro-6-(4-propyl-piperidin-1-yl)-pyrimidine |
| A114 | 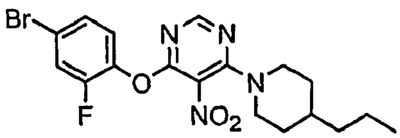 | 4-(4-Bromo-2-fluoro-phenoxy)-5-nitro-6-(4-propyl-piperidin-1-yl)-pyrimidine |
| A115 | 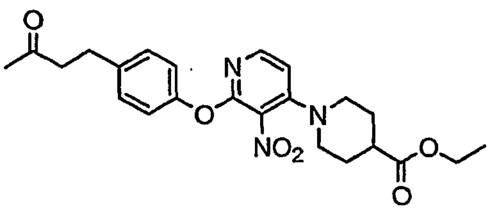 | 3'-Nitro-2'-[4-(3-oxo-butyl)-phenoxy]-3,4,5,6-tetrahydro-2H-[1,4']bipyridinyl-4-carboxylic acid ethyl ester |
| A116 | 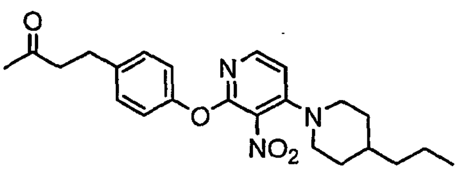 | 4-[4-(3'-Nitro-4-propyl-3,4,5,6-tetrahydro-2H-[1,4']bipyridinyl-2'-yloxy)-phenyl]-butan-2-one |
| A117 | 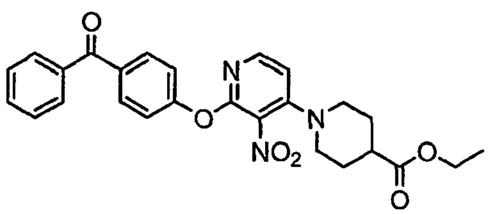 | 2'-(4-Benzoyl-phenoxy)-3'-nitro-3,4,5,6-tetrahydro-2H-[1,4']bipyridinyl-4-carboxylic acid ethyl ester |
| A118 | 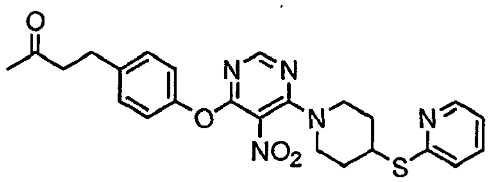 | 4-(4-{5-Nitro-6-[4-(pyridin-2-ylsulfanyl)-piperidin-1-yl]-pyrimidin-4-yloxy}-phenyl)-butan-2-one |
| A119 | 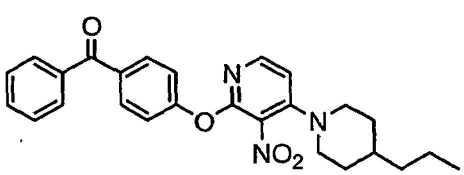 | [4-(3'-Nitro-4-propyl-3,4,5,6-tetrahydro-2H-[1 , 4']bipyridinyl-2'-yloxy)-phenyl]-phenyl-methanone |

(continued)

| Cmpd# | Structure | Chemical Name |
|---|---|---|
| A120 | 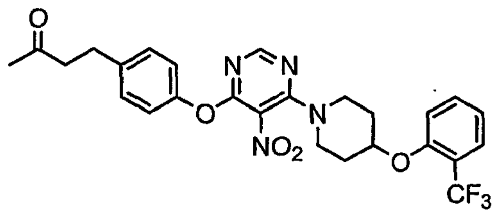 | 4-(4- {5-Nitro-6-[4-(2-trifluoromethyl-phenoxy)-piperidin-1-yl]-pyrimidin-4-yloxy}-phenyl)-butan-2-one |
| A121 | 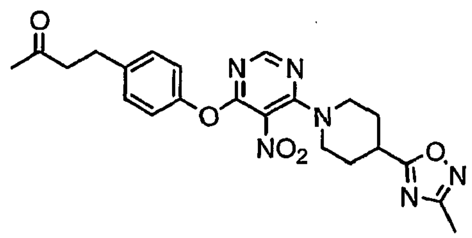 | 4-(4-{6-[4-(3-Methyl-[1,2,4]oxadiazol-5-yl)-piperidin-1-yl]-5-nitro-pyrimidin-4-yloxy}-phenyl)-butan-2-one |
| A122 | 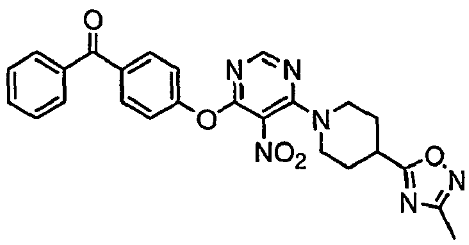 | (4-{6-[4-(3-Methyl-[1,2,4]oxadiazol-5-yl)-piperidin-1-yl]-5-nitro-pyrimidin-4-yloxy}-phenyl)-phenyl-methanone |
| A123 | 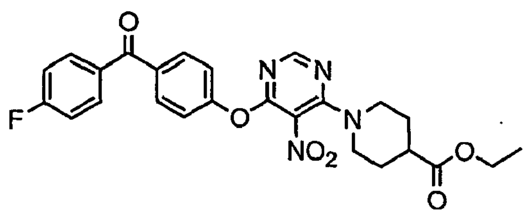 | 1-{6-[4-(4-Fluoro-benzoyl)-phenoxy]-5-nitro-pyrimidin-4-yl}-piperidine-4-carboxylic acid ethyl ester |
| A124 | 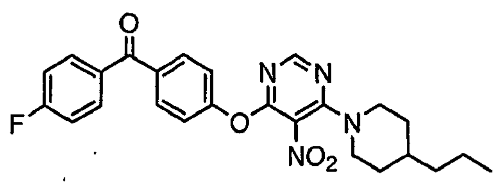 | (4-Fluoro-phenyl)-{4-[5-nito-6-(4-propyl-piperidin-1-yl)-pyrimidin-4-yloxy]-phenyl}-methanone |
| A125 | 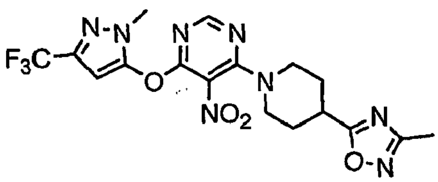 | 4-[4-(3-Methyl-[1,2,4]oxadiazol-5-yl)-piperidin-1-yl]-6-(2-methyl-5 -trifluoromethyl-2H-pyrazol-3-yloxy)-5-nitro-pyrimidine |
| A126 | 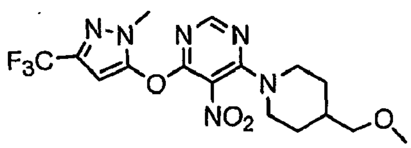 | 4-(4-Methoxymethyl-piperidin-1-yl)-6-(2-methyl-5-trifluoromethyl-2H-pyrazol-3-yloxy)-5-nitro-pyrimidine |

(continued)

| Cmpd# | Structure | Chemical Name |
|---|---|---|
| A127 | 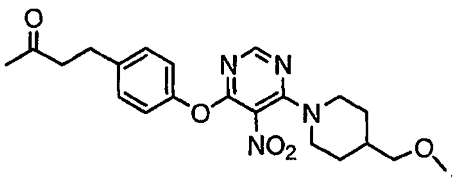 | 4-{4-[6-(4-Methoxymethyl-piperidin-1-yl)-5-nitro-pyrimidin-4-yloxy]-phenyl}-butan-2-one |
| A128 | 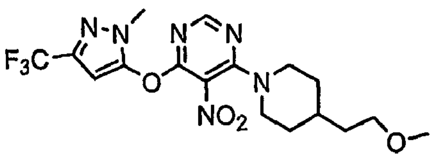 | 4-[4-(2-Methoxy-ethyl)-piperidin-1-yl]-6-(2-methyl-5-trifluoromethyl-2H-pyrazol-3-yloxy)-5-nitro-pyrimidine |
| A129 | 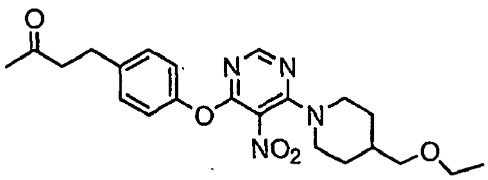 | 4-{4-[6-(4-Ethoxymethyl-piperidin-1-yl)-5-nitro-pyrimidin-4-yloxy]-phenyl}-butan-2-one |
| A130 | 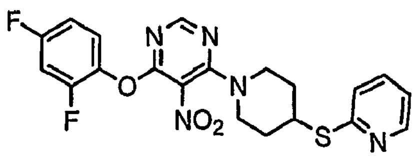 | 4-(2,4-Difluoro-phenoxy)-5-nitro-6-[4-(pyridin-2-ylsulfanyl)-piperidin-1 -yl]-pynmidme |
| A131 | 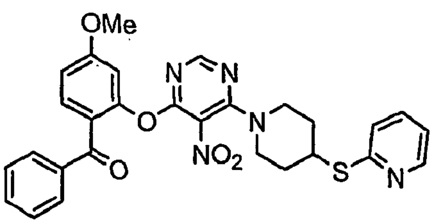 | (4-Methoxy-2-{5-nitro-6-[4-(pyridin-2-ylsulfanyl)-piperidin-1-yl]-pyrimidin-4-yloxy}-phenyl)-phenyl-methanone |
| A132 | 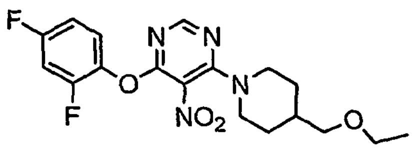 | 4-(2,4-Difluoro-phenoxy)-6-(4-ethoxymethyl-piperidin-1-yl)-5-nitro-pyrimidine |
| A133 | 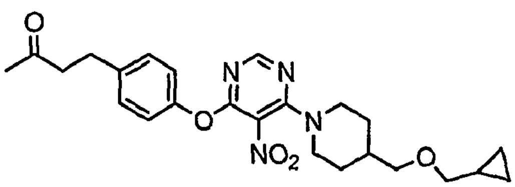 | 4-{4-[6-(4-Cyclopropylmethoxymethyl-piperidin-1-yl)-5-nitro-pyrimidin-4-yloxy]-phenyl}-butan-2-one |
| A134 | 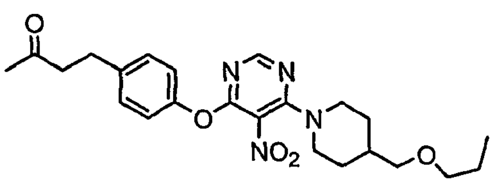 | 4-{4-[5-Nitro-6-(4-propoxymethyl-piperidin-1-yl)-pyrimidin-4-yloxy]-phenyl}-butan-2-one |

(continued)

| Cmpd# | Structure | Chemical Name |
|---|---|---|
| A135 | 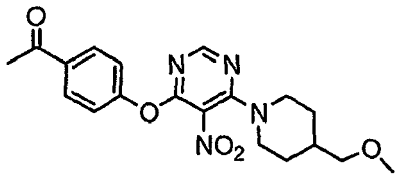 | 1-{4-[6-(4-Methoxymethyl-piperidin-1-yl)-5-nito-pyrimidin-4-yloxy]-phenyl}-ethanone |
| A136 | 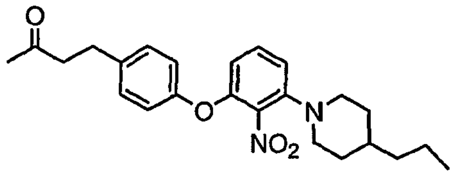 | 4-{4-[2-Nitro-3-(4-propylpiperidin-1-yl)-phenoxy]-phenyl}-butan-2-one |
| A137 | 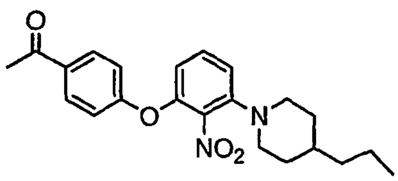 | 1-{4-[2-Nitro-3-(4-propyl-piperidin-1-yl)-phenoxy]-phenyl}-ethanone |
| A138 | 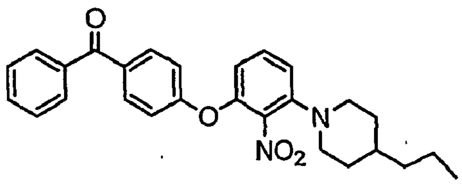 | {4-[2-Nitro-3-(4-propyl-piperidin-1-yl)-phenoxy]-phenyl}-phenyl-methanone |
| A139 | 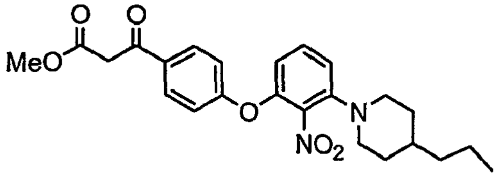 | 3-{4-[2-Nitro-3-(4-propyl-piperidin-1-yl)-phenoxy]-phenyl}-3-oxo-propionic acid methyl ester |
| A140 | 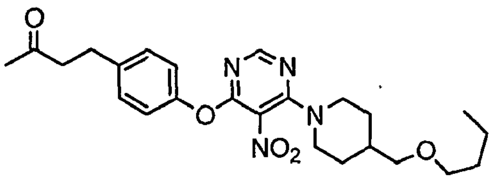 | 4-{4-[6-(4-Butoxymethyl-piperidin-l-yl)-5-nitro-pyrimidin-4-yloxy]-phenyl}-butan-2-one |
| A141 | 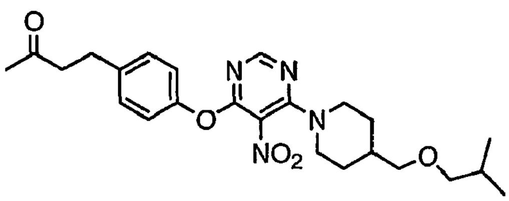 | 4-{4-[6-(4-Isobutoxymethyl-piperidin-1-yl)-5-nitro-pyrimidin-4-yloxy]-phenyl}-butan-2-one |

(continued)

| Cmpd# | Structure | Chemical Name |
|---|---|---|
| A142 | 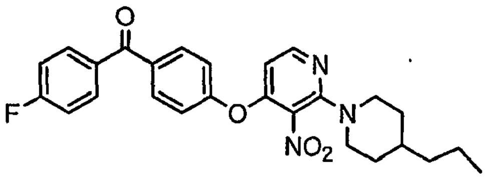 | (4-Fluoro-phenyl)-[4-(3'-nitro-4.-propyl-3,4,5,6-tetrahydro-2H-[1,2']bipyridinyl-4'-yloxy)-phenyl]-methanone |
| A143 | 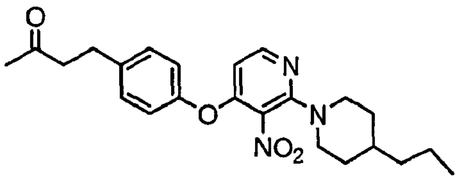 | 4-[4-(3'-Nitro-4-propyl-3,4,5,6-tetrahydro-2H-[1,2']bipyridinyl-4'-yloxy)-phenyl]-butan-2-one |
| A144 | 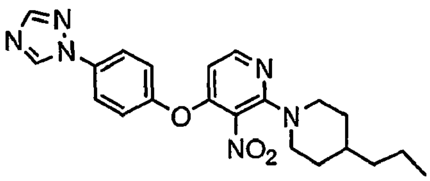 | 3'-Nitro-4-propyl-4'-(4-[1,2,4]triazol-1-yl-phenoxy)-3,4,5,6-tetrahydro-2H-[1,2'] bipyridinyl |
| A145 | 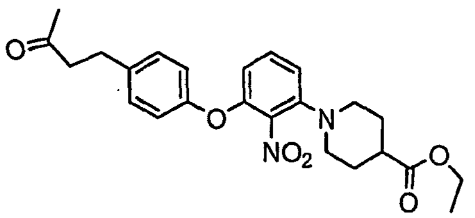 | 1-{2-Nitro-3-[4-(3-oxo-butyl)-phenoxy]-phenyl}-piperidine-4-carboxylic acid ethyl ester |
| A146 | 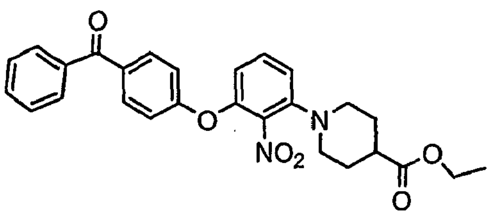 | 1-[3-(4-Benzoyl-phenoxy)-2-nitro-phenyl]-piperidine-4-carboxylic acid ethyl ester |
| A147 | 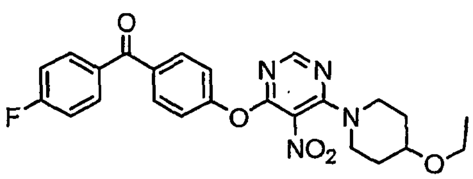 | {4-[6-(4-Ethoxy-piperidin-1-yl)-5-nitro-pyrimidin-4-yloxy]-phenyl}-(4-fluoro-phenyl)-methanone |
| A148 | 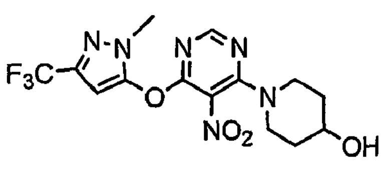 | 1-[6-(2-Methyl-5-trifluoromethyl-2H-pyrazol-3-yloxy)-5-nitro-pyrimidin-4-yl]-piperidin-4-ol |

(continued)

| Cmpd# | Structure | Chemical Name |
|---|---|---|
| A149 | 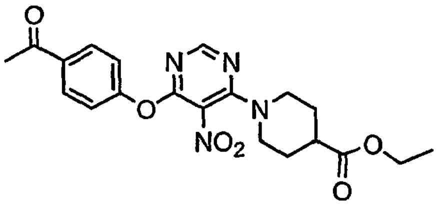 | 1-[6-(4-Ac6tyl-phenoxy)-5-nitro-pyrimidin-4-yl]-piperidine-4-carboxylic acid ethyl ester |
| A150 | 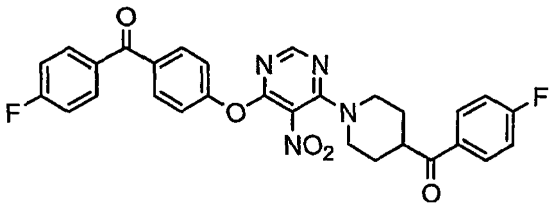 | (1-{6-[4-(4-Fluoro-benzoyl)-phenoxy]-5-nitro-pyrimidin-4-yl}-piperidin-4-yl)-(4-fluoro-phenyl)-methanone |
| A151 | 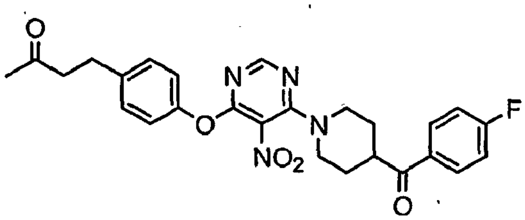 | 4-(4-{6-[4-(4-Fluoro-benzoyl)-piperidin-1-yl]-5-nitro-pyrimidin-4-yloxy}-phenyl)-butan-2-one |
| A152 | 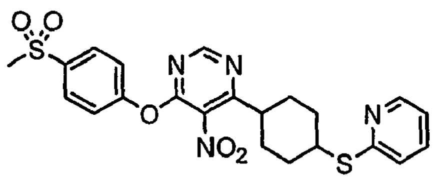 | 4-(4-Methanesulfonyl-phenoxy)-5-nitro-6-[4-(pyridin-2-ylsulfanyl)-cyclohexyl]-pyrimidine |
| A153 | 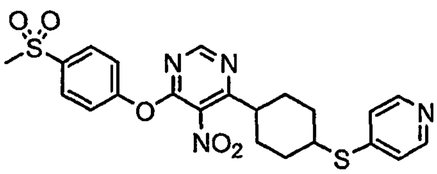 | 4-(4-Methanesulfonyl-phenoxy)-5-nitro-6-[4-(pyridin-4-ylsulfanyl)-cyclohexyl]-pyrimidine |
| A154 | 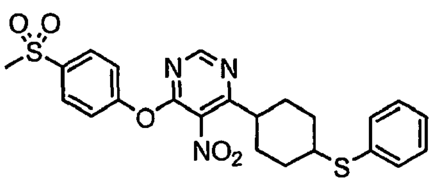 | 4-(4-Methanesulfonyl-phenoxy)-5-nitro-6-(4-phenylsulfanyl-cyclohexyl)-pyrimidine |
| A155 | 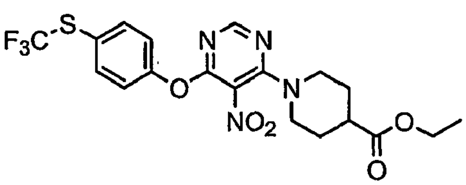 | 1-[5-Nitro-6-(4-trifluoromethylsulfanyl-phenoxy)-pyrimidin-4-yl]-piperidine-4-carboxylic acid ethyl ester |

(continued)

| Cmpd# | Structure | Chemical Name |
|---|---|---|
| A156 | 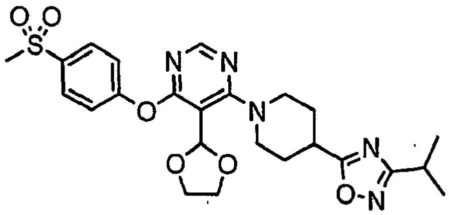 | 5-[1,3]Dioxolan-2-yl-4-[4-(3-isopropyl-[1,2,4] oxadiazol-5-yl)-piperidin-1-yl]-6-(4-methanesulfonyl-phenoxy)-pyrimidine |
| A157 | 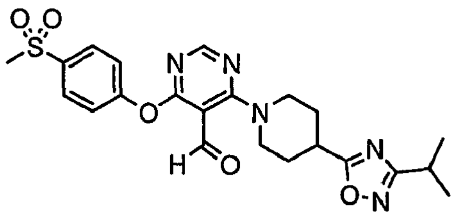 | 4-[4-(3-Isopropyl-[1,2,4]oxadiazol-5-yl)-piperidin-1-yl]-6-(4-methanesulfonyl-phenoxy)-pyrimidine-5-carbaldehyde |
| A158 | 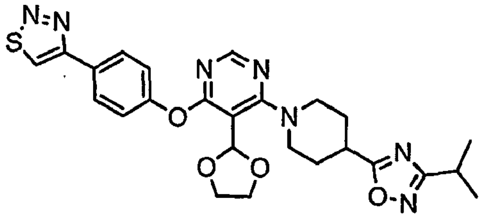 | 5-[1,3]Dioxolan-2-yl-4-[4-(3-isopropyl-[1,2,4] oxadiazol-5-yl)-piperidin-1-yl]-6-(4-[1,2,3] thiadiazol-4-yl-phenoxy)-pyrimidine |
| A159 | 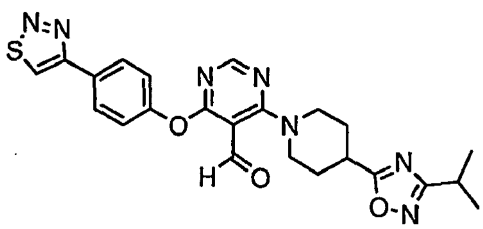 | 4-[4-(3-Isopropyl-[1,2,4]oxadiazol-5-yl)-piperidin-1-yl]-6-(4-[1,2,3]thiadiazol-4-yl-phenoxy)-pyrimidine-5-carbaldehyde |
| A160 | 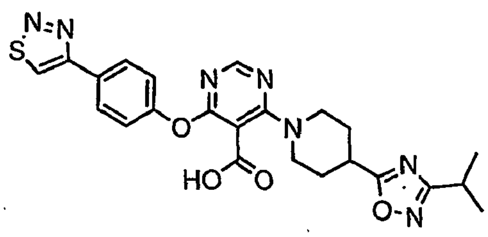 | 4-[4-(3-Isopropyl-[1,2,4]oxadiazol-5-yl)-piperidin-1-yl]-6-(4-[1,2,3]thiadiazol-4-yl-phenoxy)-pyrimidine-5-carboxylic acid |
| A161 | 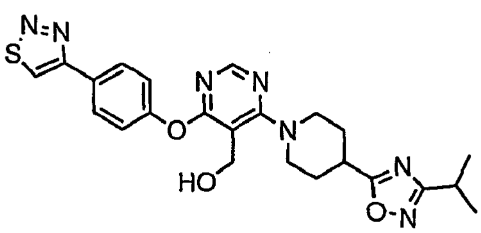 | [4-[4-(3-Isopropyl-[1,2,4]oxadiazol-5-yl)-piperidin-1-yl]-6-(4-[1,2,3]thiadiazol-4-yl-phenoxy)-pyrimidin-5-yl]-methanol |

(continued)

| Cmpd# | Structure | Chemical Name |
|---|---|---|
| A162 | 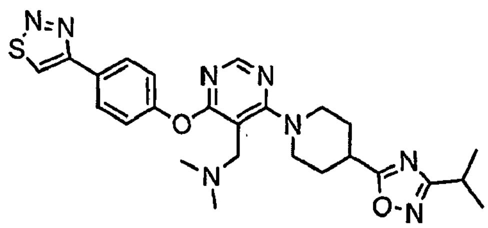 | [4-[4-(3-Isopropyl-[1,2,4]oxadiazol-5-yl)-piperidin-1-yl]-6-(4-[1,2,3]tliiadiazol-4-yl-phenoxy)-pyrimidin-5-ylTnethyl]-dimethyl-amine |
| A163 | 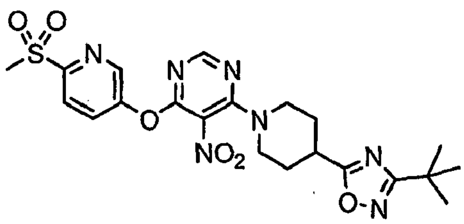 | 4-[4-(3-tert-Butyl-[1,2,4]oxadiazol-5-yl)-piperidin-1-yl]-6-(6-methanesulfonyl-pyridin-3 -yloxy)-5-nitro-pyrimidine |
| A164 | 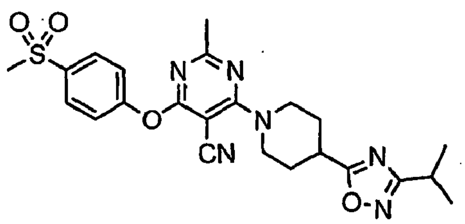 | 4-[4-(3-Isopropyl-[1,2,4]oxadiazol-5 yl)-piperidin-1-yl]-6-(4-methanesulfonyl-phenoxy)-2-methyl-pyrimidine-5-carbonitrile |
| A165 | 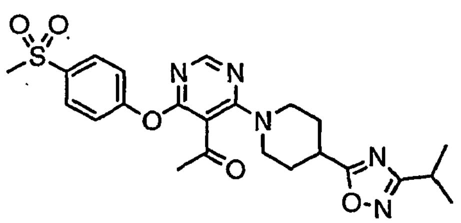 | 1-[4-[4-(3-Isopropyl-[1,2,4]oxadiazol-5-yl)-piperidin-1-yl]-6-(4-methanesulfonyl-phenoxy)-pyrimidin-5-yl]-ethanone |

**TABLE B**

| Cmpd# | Structure | Chemical Name |
|---|---|---|
| B1 | 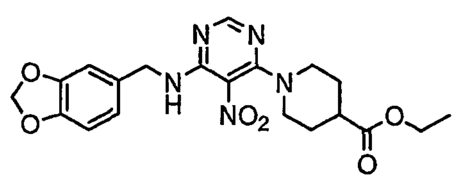 | 1-{6-[(Benzo[1,3]dioxol-5-ylmethyl)-amino]-5-nitro-pyrimidin-4-yl}-piperidine-4-carboxylic acid ethyl ester |
| B2 | 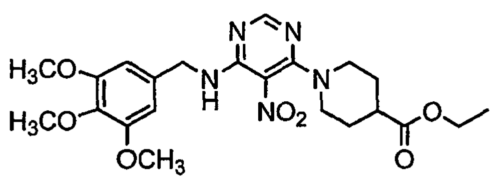 | 1-[5-Nitro-6-(3,4,5-trimethoxy-benzylamino)-pyrimidin-4-yl]-piperidine-4-carboxylic acid ethyl ester |
| B3 | 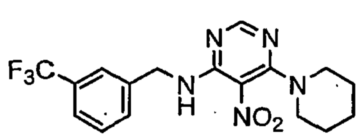 | (5-Nitro-6-piperidin-1-yl-pyrimidin-4-yl)-(3-trifluoromethyl-benzyl)-amine |

(continued)

| Cmpd# | Structure | Chemical Name |
|---|---|---|
| B4 | 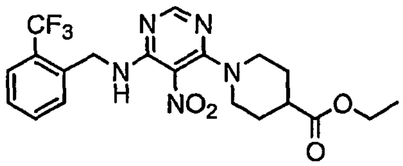 | 1-[5-Nitro-6-(2-trifluoromethyl-benzylamino)-pyrimidin-4-yl]-piperidine-4-carboxylic acid ethyl ester |
| B5 | 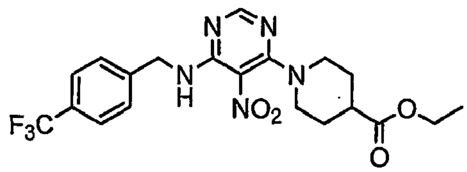 | 1-[5-Nitro-6-(4-trifluoromethyl-benzylamino)-pyrimidin-4-yl]-piperidine-4-carboxylic acid ethyl ester |
| B6 | 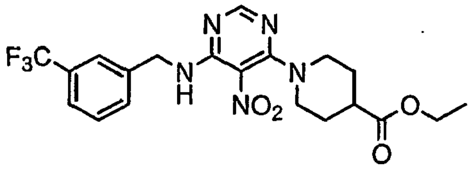 | 1-[5-Nitro-6-(3-trifluoromethyl-benzylamino)-pyrimidin-4-yl]-piperidine-4-carboxylic acid ethyl ester |
| B7 | 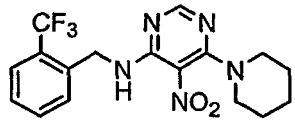 | (5-Nitro-6-piperidin-1-yl-pyrimidin-4-yl)-(2-trifluoromethyl-benzyl)-amine |
| B8 | 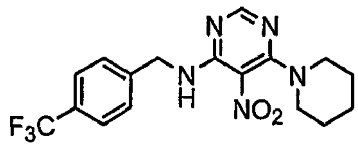 | (5-Nitro-6-piperidin-1-yl-pyrimidin-4-yl)-(4-trifluoromethyl-benzyl)-amine |
| B9 | 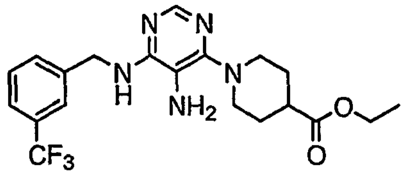 | 1-[5-Arnino-6-(3-trifluoromethyl-benzylamino)-pyrimidin-4-yl]-piperidine-4-carboxylic acid ethyl ester |
| B10 | 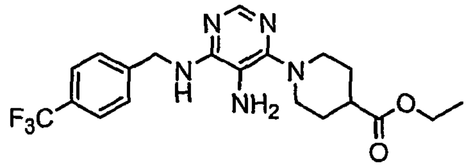 | 1-[5-Amino-6-(4-trifluoromethyl-benzylamino)-pyrimidin-4-yl]-piperidine-4-carboxylic acid ethyl ester |
| B11 | 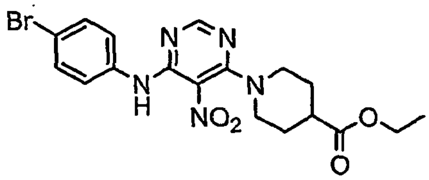 | 1-[6-(4-Bromo-phenylamino)-5-nitro-pyrimidin-4-yl]-piperidine-4-carboxylic acid ethyl ester |

(continued)

| Cmpd# | Structure | Chemical Name |
|---|---|---|
| B12 | 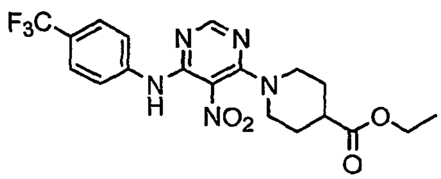 | 1-[5-Nitro-6-(4-trifluoromethyl-phenylamino)-pyrimidin-4-yl]-piperidine-4-carboxylic acid ethyl ester |
| B13 | 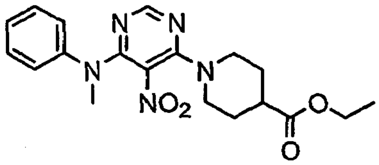 | 1-[6-(Methyl-phenyl-amino)-5-nitro-pyrimidin-4-yl]-piperidine-4-carboxylic acid ethyl ester |
| B14 | 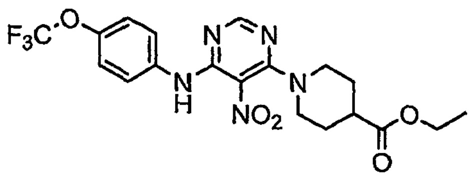 | 1-[5-Nitro-6-(4-trifluoromethoxy-phenylamino)-pyrimidin-4-yl]-piperidine-4-carboxylic acid ethyl ester |
| B15 | 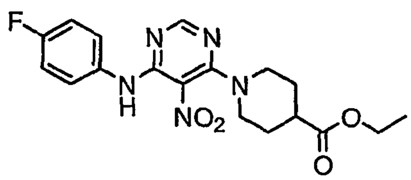 | 1-[6-(4-Fluoro-phenylamino)-5-nitro-pyrimidin-4-yl]-piperidine-4-carboxylic acid ethyl ester |
| B16 | 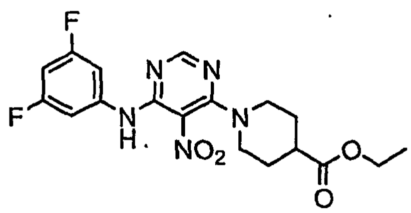 | 1-[6-(3,5-Difluoro-phenylamino)-5-nitro-pyrimidin-4-yl]-piperidine-4-carboxylic acid ethyl ester |
| B17 | 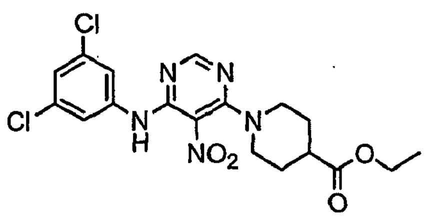 | 1-[6-(3,5-Dichloro-phenylamino)-5-nitro-pyrinfdin-4-yl]-piperidine-4-carboxylic acid ethyl ester |
| B18 | 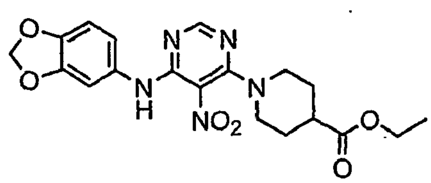 | 1-[6-(Benzo[1,3]dioxol-5-ylamino)-5-nitro-pyrimidin-4-yl]-piperidine-4-carboxylic acid ethyl ester |
| B19 | 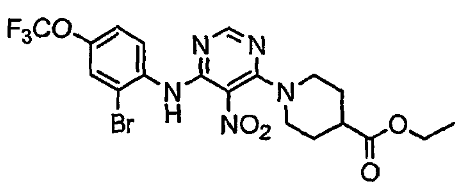 | 1-[6-(2-Bromo-4-trifluoromethoxy-phenylamino)-5-nitro-pyrimidin-4-yl]-piperidine-4-carboxylic acid ethyl ester |

(continued)

| Cmpd# | Structure | Chemical Name |
|---|---|---|
| B20 | 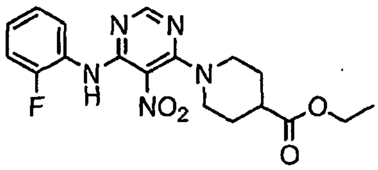 | 1-[6-(2-Fluoro-phenylamino)-5-nitro-pyrimidin-4-yl]-piperidine-4-carboxylic acid ethyl ester |
| B21 | 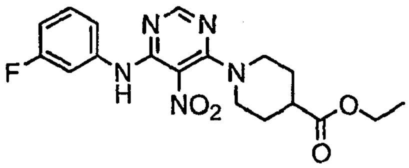 | 1-[6-(3-Fluoro-phenylamino)-5-nitro-pyrimidin-4-yl]-piperidine-4-carboxylic acid ethyl ester |
| B22 | 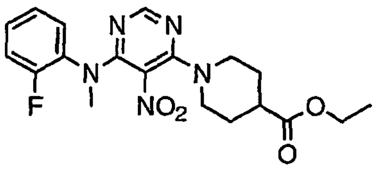 | 1-{6-[(2-Fluoro-phenyl)-methyl-amino]-5-nitro-pyrimidin-4-yl}-piperidine-4-carboxylic acid ethyl ester |
| B23 | 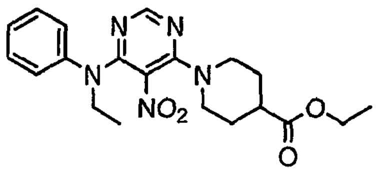 | 1-[6-(Ethyl-phenyl-amino)-5-nitro-pyrimidin-4-yl]-piperidine-4-carboxylic acid ethyl ester |
| B24 | 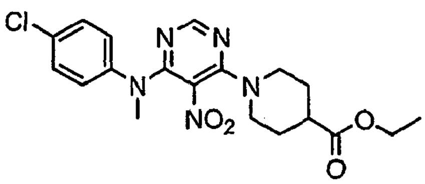 | 1-{6-[(4-Chloro-phenyl)-methyl-amino]-5-nitro-pyrimidin-4-yl}-piperidine-4-carboxylic acid ethyl ester |
| B25 | 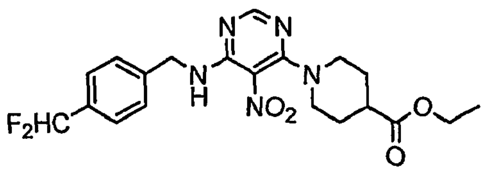 | 1-[6-(4-Difluoromethyl-benzylamino)-5-nitro-pyrimidin-4yl]-piperidine-4-carboxylic acid ethyl ester |
| B26 | 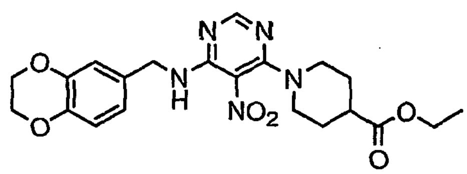 | 1-{6-[(2,3-Dihydro-benzo[1,4]dioxin-6-ylmethyl)-amino]-5-nitro-pyrimidin-4-yl}-piperidine-4-carboxylic acid ethyl ester |
| B27 | 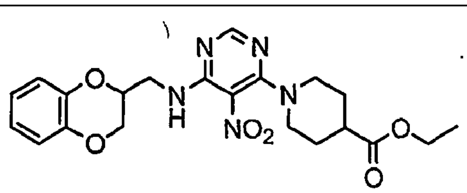 | 1-{6-[(2,3-Dihydro-benzo[1,4]dioxin-2-ylmethyl)-amino]-5-nitro-pynmidin-4-yl}-piperidine-4-carboxylic acid ethyl ester |

(continued)

| Cmpd# | Structure | Chemical Name |
|---|---|---|
| B28 | 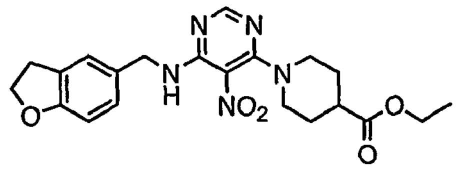 | 1-{6-[(2,3-Dihydro-benzofuran-5-ylmethyl)-amino]-5-nitro-pyrimidin-4-yl}-piperidine-4-carboxylic acid ethyl ester |
| B29 | 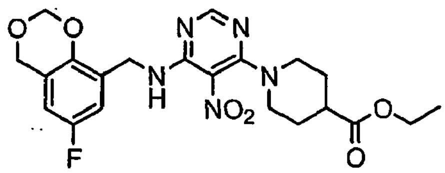 | 1-{6-[(6-Fluoro-4H-benzo[1,3]dioxin-8-ylmethyl)-amino]-5-nitro-pyrimidin-4-yl}-piperidine-4-carboxylic acid ethyl ester |
| B30 | 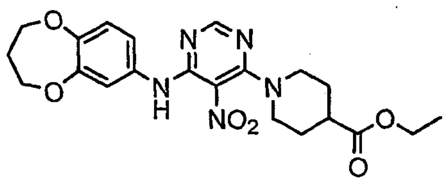 | 1-[6-(3,4-Dihydro-2H-benzo[b][1,4]dioxepin-7-ylamino)-5-nitro-pyrimidin-4-yl]-piperidine-4-carboxylic acid ethyl ester |
| B31 | 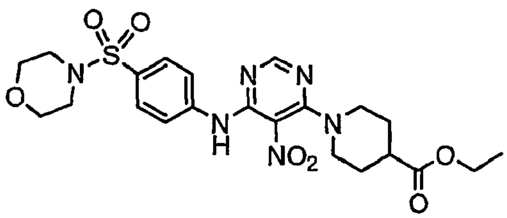 | 1-{6-[4-(Morpholine-4-sulfonyl)-phenylamino]-5-nitro-pyhmidin4-yl}-piperidine-4-carboxylic acid ethyl ester |
| B32 | 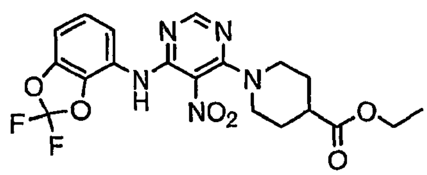 | 1-[6-(2,2-Difluoro-benzo[1,3]dioxol-4-ylamino)-5-nitro-pyrimidin-4-yl]-piperidine-4-carboxylic acid ethyl ester |
| B33 | 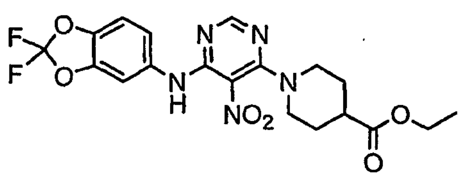 | 1-[6-(2,2-Difluoro-benzo[1,3]dioxol-5-ylamino)-5-nitro-pyrimidin-4-yl]-piperidine-4-carboxylic acid ethyl ester |
| B34 | 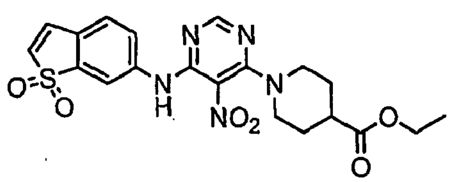 | 1-[6-(1,1-Dioxo-1H-1$\lambda^6$-benzo[b]thiophen-6-ylamino)-5-nitro-pyrimidin4-yl]-piperidine-4-carboxylic acid ethyl ester |
| B35 | 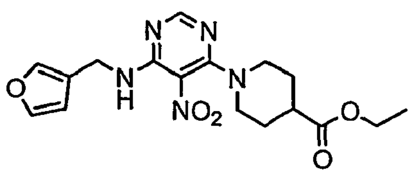 | 1-{6-[(Furan-3-ylmethyl)-amino]-5-nitro-pyrimidin-4-yl} -piperidine-4-carboxylic acid ethyl ester |

(continued)

| Cmpd# | Structure | Chemical Name |
|---|---|---|
| B36 | 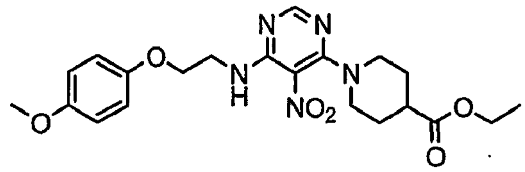 | 1-{6-[2-(4-Methoxy phenoxy)-ethylamino]-5-nitro-pyrimidin-4-yl}-piperidine-4-carboxylic acid ethyl ester |
| B37 | 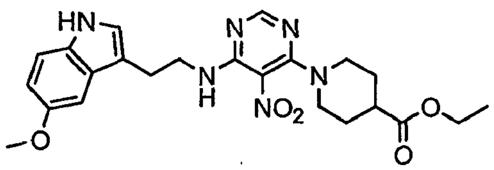 | 1-{6-[2-(5-Methoxy-1H-indol-3-yl)-ethylamino]-5-nitro-pyrimidin-4-yl}-piperidine-4-carboxylic acid ethyl ester |
| B38 | 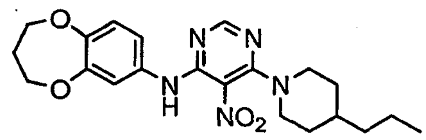 | (3,4-Dihydro-2H-benzo[b][1,4]dioxepin-7-yl)-[5-nitro-6-(4-propyl-piperidin-1-yl)-pyrimidin-4-yl]-amine |
| B39 | 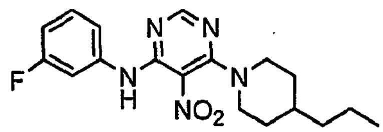 | (3-Fluoro-phenyl)-[5-nitro-6-(4-propyl-piperidin-1-yl)-pyrimidin-4-yl]-amine |
| B40 | 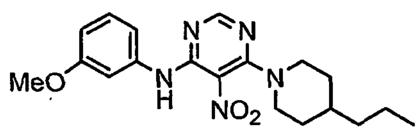 | (3-Methoxy-phenyl)-[5-nitro-6-(4-propyl-piperidin-1-yl)-pyrimidin-4-yl]-amine |
| B41 | 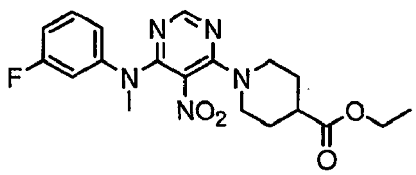 | 1-{6-[(3-Fluoro-phenyl)-methyl-amino]-5-nitro-pyrimidin-4-yl}-piperidine-4-carboxylic acid ethyl ester |
| B42 | 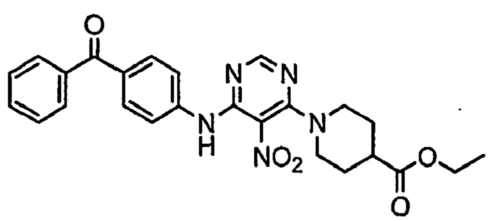 | 1-[6-(4-Benzoyl-phenylamino)-5-nito-pyrimidin-4-yl] -piperidine-4-carboxylic acid ethyl ester |
| B43 | 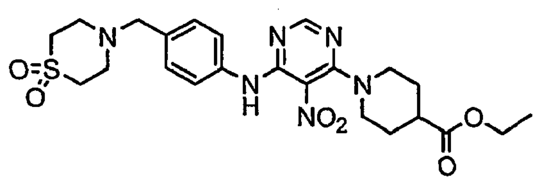 | 1-{6-[4-(1,1-Dioxo-1$\lambda^6$-thiomorpholin-4-ylmethyl)-phenylamino]-5-nitro-pyrimidin-4-yl}-piperidine-4-carboxylic acid ethyl ester |

(continued)

| Cmpd# | Structure | Chemical Name |
|---|---|---|
| B44 | 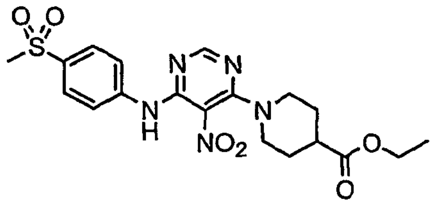 | 1-[6-(4-Methanesulfonyl-phenylamino)-5-nitro-pyrimidin-4yl]-piperidine-4-carboxylic acid ethyl ester |
| B45 | 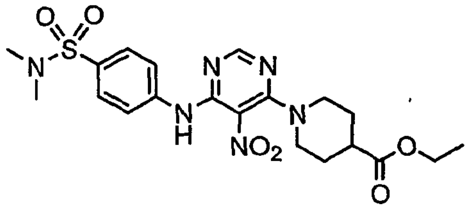 | 1-[6-(4-Dimethylsulfamoyl-phenylamino)-5-nitro-pyrimidin-4-yl]-piperidine-4-carboxylic acid ethyl ester |
| B46 | 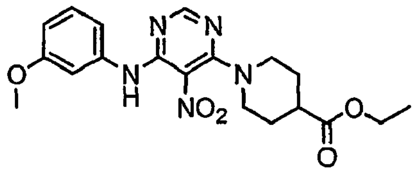 | 1-[6-(3-Methoxy-phenylamino)-5-nitro-pyrimidin-4-yl]-piperidine-4-carboxylic acid ethyl ester |
| B47 | 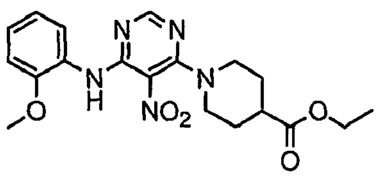 | 1-[6-(2-Methoxy-phenylamino)-5-nitro-pyrimidin-4-yl]-piperidine-4-carboxylic acid ethyl ester |
| B48 | 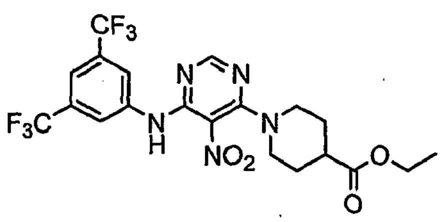 | 1-[6-(3,5-Bis-trifluoromethyl-phenylamino)-5-nitro-pyrimidin-4-yl]-piperidine-4-carboxylic acid ethyl ester |
| B49 | 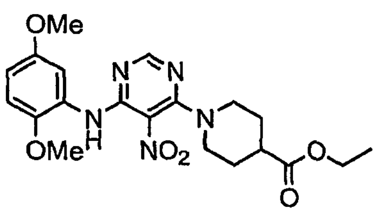 | 1-[6-(2,5-Dimethoxy-phenylamino)-5-nitro-pyrimidin-4-yl]-piperidine-4-carboxylic acid ethyl ester |
| B50 | 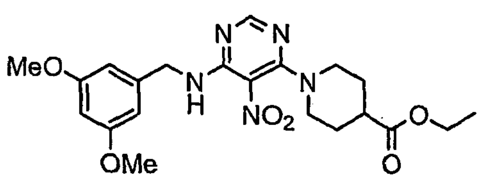 | 1-[6-(3,5-Dimethoxy-benzylamino)5-nitro-pyrimidin-4-yl]-piperidine-4-carboxylic acid ethyl ester |

(continued)

| Cmpd# | Structure | Chemical Name |
|---|---|---|
| B51 | 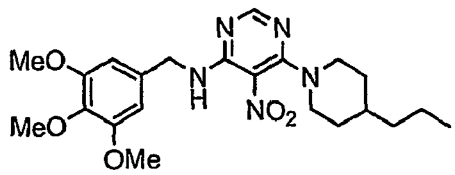 | [5-Nitro-6-(4-propyl-piperidin-1-yl)-pyrirnidin-4-yl]-(3,4,5-trimethoxy-benzyl)-amine |
| B52 | 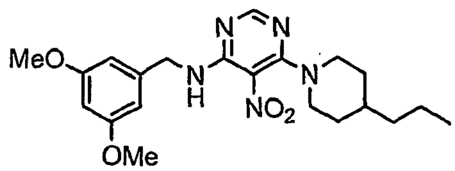 | (3,5-Dimethoxy-benzyl)-[5-nitro-6-(4-propyl-piperidin-1-yl)-pyrimidin-4-yl]-amine |
| B53 | 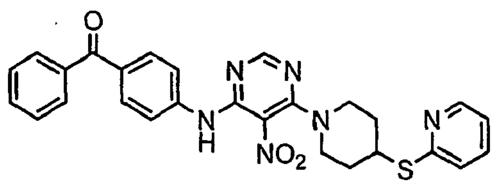 | (4-(5-Nitro-6-[4-(pyridin-2-ylsulfanyl)-piperidin-1-yl]-pyrimidin-4-ylamino}-phenyl)-phenyl-methanone |
| B54 | 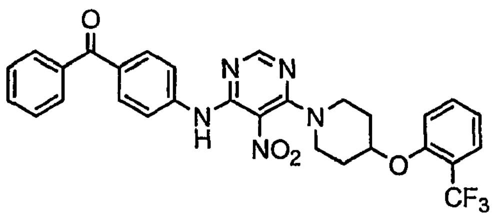 | (4-{5-Nitro-6-[4-(2-trifluoromethyl-phenoxy)-piperidin-1-yl]-pyrimidin-4-ylamino}-phenyl)-phenyl-methanone |
| B55 | 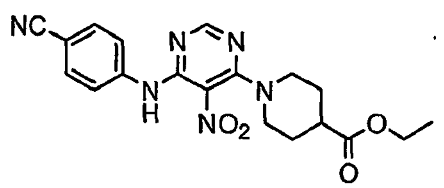 | 1-[6-(4-Cyano-phenylamino)-5-nitro-pyrimidin-4-yl]-piperidine-4-carboxylic acid ethyl ester |
| B56 | 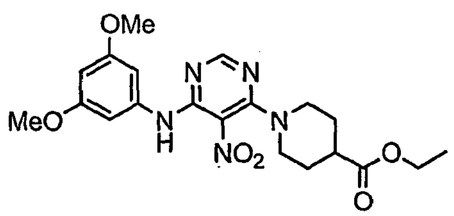 | 1-[6-(3,5-Dimethoxy-phenylamino)-5-nitro-pyrimidin-4-yl]-piperidine-4-carboxylic acid ethyl ester |
| B57 | 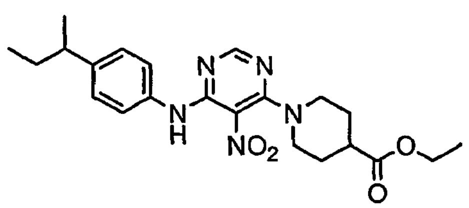 | 1-[6-(4-sec-Butyl-phenylamino)-5-nitro-pyrimidin-4-yl]-piperidine-4-carboxylic acid ethyl ester |

(continued)

| Cmpd# | Structure | Chemical Name |
|---|---|---|
| B58 | 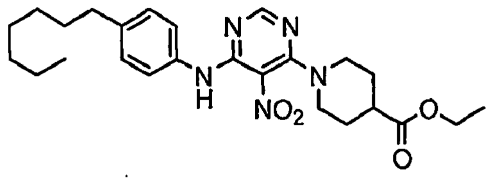 | 1-[6-(4-Heptyl-phenylamino)-5-nitro-pyrimidin-4-yl]-piperidine-4-carboxylic acid ethyl ester |
| B59 | 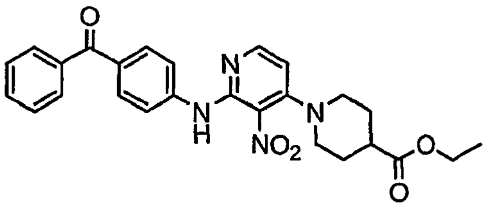 | 2'-(4-Benzoyl-phenylamino)-3'-nitro-3,4,5,6-tetrahydro-2H-[1,4']bipyridinyl-4-carboxylic acid ethyl ester |
| B60 | 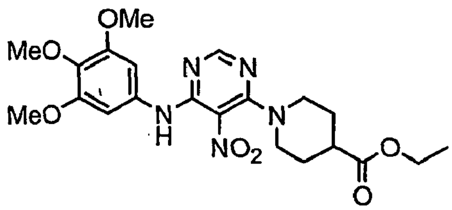 | 1-[5-Nitro-6-(3,4,5-ttimethoxy-phenylamino)-pyrimidin-4-yl]-piperidine-4-carboxylic acid ethyl ester |
| B61 | 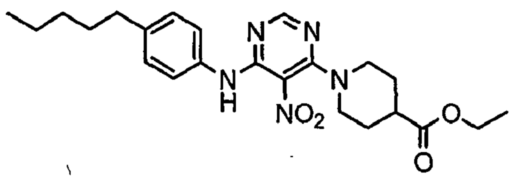 | 1-[5-Nitro-6-(4-pentyl-phenylamino)-pyrimidin-4-yl]-piperidine-4-carboxylic acid ethyl ester |
| B62 | 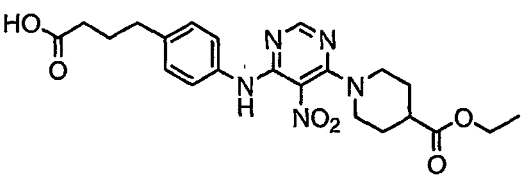 | 1-{6-[4-(3-Carboxy-propyl)-phenylamino]-5-nitro-pyrimidin-4-yl}-piperidine-4-carboxylic acid ethyl ester |
| B63 | 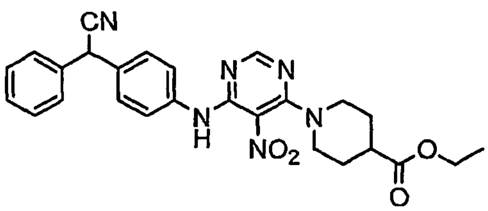 | 1-{6-[4-(Cyano-phenyl-methyl)-phenylamino]-5-nitro-pyrimidin-4-yl}-piperidine-4-carboxylic acid ethyl ester |
| B64 | 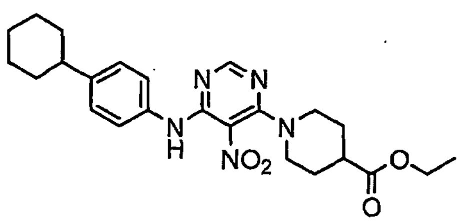 | 1-[6-(4-Cyclohexyl-phenylamino)-5-nitro-pyrimidin-4-yl]-piperidine-4-carboxylic acid ethyl ester |

(continued)

| Cmpd# | Structure | Chemical Name |
|---|---|---|
| B65 | 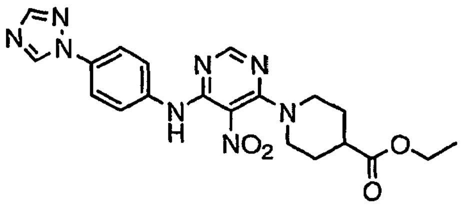 | 1-[5-Nitro-6-(4-[1,2,4]triazol-1-yl-phenylamino)-pyrimidin-4-yl]-piperidine-4-carboxylic acid ethyl ester |
| B66 | 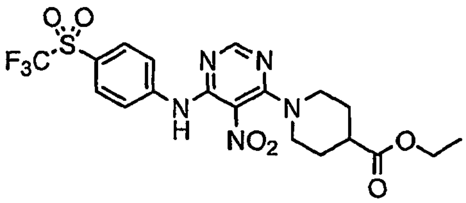 | 1-[5-Nitro-6-(4-trifluoromethanesulfonyl-phenylamino)-pyrimidin-4-yl]-piperidine-4-carboxylic acid ethyl ester |
| B67 | 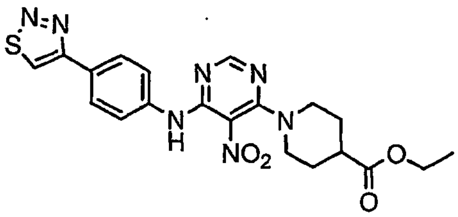 | 1-[5-Nitro-6-(4-[1,2,3]thiadiazol-4-yl-phenylamino)-pyrimidin-4-yl]-piperidine-4-carboxylic acid ethyl ester |
| B68 | 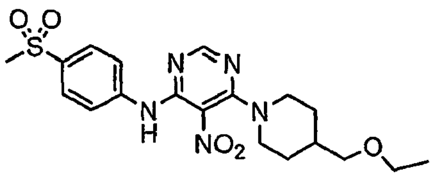 | [6-(4-Ethoxymethyl-pipehdm-1-yl)-5-nito-pyrimidin-4-yl]-(4-methanesulfonyl-phenyl)-amine |
| B69 | 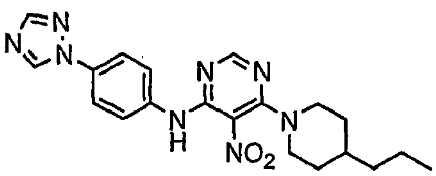 | [5-Nitro-6-(4-propyl-piperidin-1-yl)-pyrimidin-4-yl]-(4-[1,2,4]triazol-1-yl-phenyl)-amine |
| B70 | 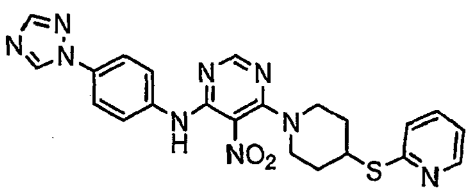 | {5-Nitro-6-[4-(pyridin-2-ylsulfanyl)-piperidin-1-yl]-pyrimidin-4-yl}-(4-[1,2,4]triazol-1-yl-phenyl)-amine |
| B71 | 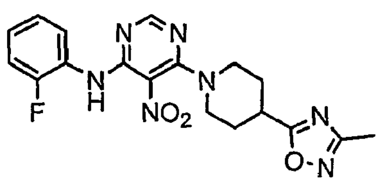 | (2-Fluoro-phenyl)-{6-[4-(3-methyl-[1,2,4] oxadiazol-5-yl)-piperidin-1-yl]-5nitro-pyrimidin-4-yl}-amine |

(continued)

| Cmpd# | Structure | Chemical Name |
|---|---|---|
| B72 | 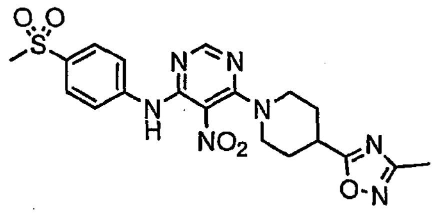 | (4-Methanesulfonyl-phenyl)-{6-[4-(3-methyl-[1,2,4]oxadiazol-5-yl)-piperidin-1-yl]-5-nitro-pyrimidin-4-yl}-amine |
| B73 | 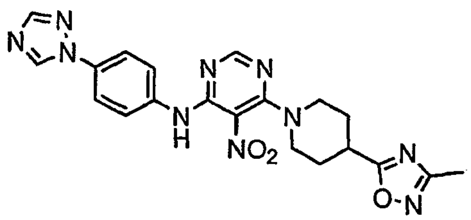 | {6-[4-(3-Methyl-[1,2,4]oxadiazol-5-yl)-piperidin-1-yl]-5-nitro-pyrimidin-4-yl}-(4-[1,2,4]triazol-1-yl-phenyl)-amine |
| B74 | 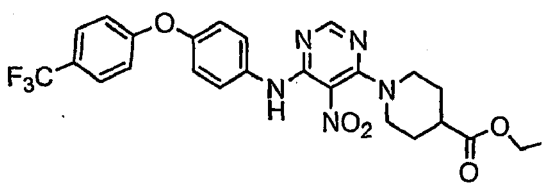 | 1-{5-Nitro-6-[4-(4-trifluoromethyl-phenoxy)-phenylamino]-pyrimidin-4-yl}-piperidine-4-carboxylic acid ethyl ester |
| B75 | 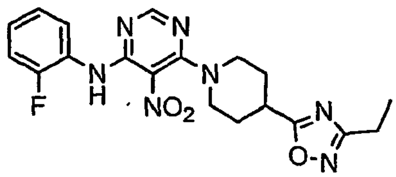 | {6-[4-(3-Ethyl-[1,2,4]oxadiazol-5-yl)-piperidin-1-yl]-5-nitro-pyrimidin-4-yl}-(2-fluoro-phenyl)-amine |
| B76 | 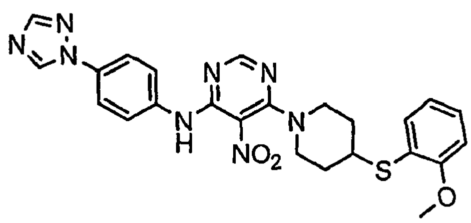 | {6-[4-(2-Methoxy-phenylsulfanyl)-piperidin-1-yl]-5-nito-pyrimidin-4-yl}-(4-[1,2,4]triazol-1-yl-phenyl)-amine |
| B77 | 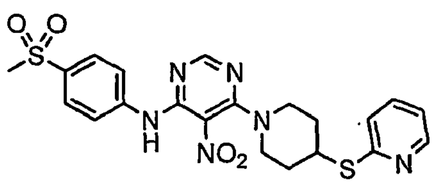 | (4-Methanesulfonyl-phenyl)-{5-nitro-6-[4-(pyridin-2-ylsulfanyl)-piperidin-1-yl]-pyrimidin-4-yl} -amine |
| B78 | 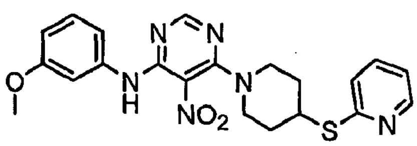 | (3-Methoxy-phenyl)-{5-nitro-6-[4-(pyridin-2-ylsulfanyl)-piperidin-1-yl]-pyrimidin-4-yl}-amine |
| B79 | 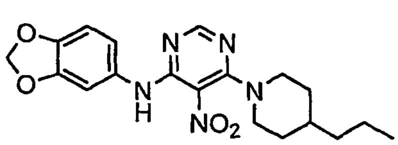 | Benzo[1,3]dioxol-5-yl-[5-nitro-6-(4-propyl-piperidin-1-yl)-pyrimidin-4-yl]-amine |

(continued)

| Cmpd# | Structure | Chemical Name |
|---|---|---|
| B80 | 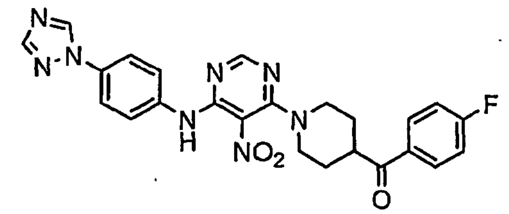 | (4-Fluoro-phenyl)-{1-[5-nitro-6-(4-[1,2,4]triazol-1-yl-phenylamino)-pyrimidin-4-yl]-piperidin-4-yl}-methanone |
| B81 | 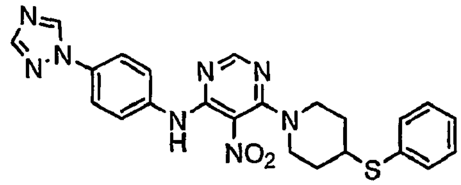 | [5-Nitro-6-(4-phenylsulfanyl-piperidin-1-yl)-pyrimidin-4-yl]-(4-[1,2,4]triazol-l-yl-phenyl)-amine |
| B82 | 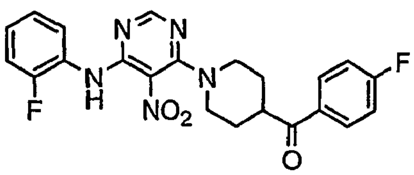 | (4-Fluoro-phenyl)-{1-[6-(2-fluoro-phenylarnino)-5-nitro-pyrimidin-4-yl]-piperidin-4-yl}-methanone |
| B83 | 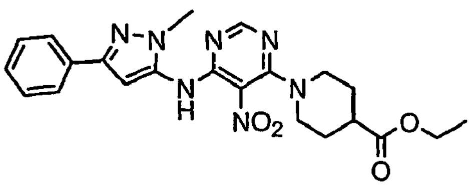 | 1-[6-(2-Methyl-5-phenyl-2H-pyrazol-3-ylamino)-5-nitro-pyrimidin-4-yl] piperidine-4-carboxylic acid ethyl ester |
| B84 | 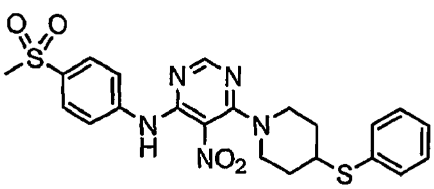 | (4-Methanesulfonyl-phenyl)-[5-nitro-6-(4-phenylsulfanyl-piperidin-1-yl)-pyrimidin-4-yl]-amine |
| B85 | 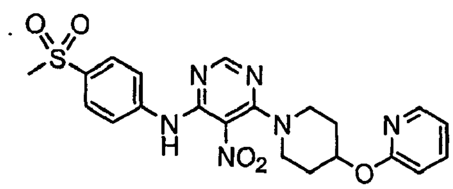 | (4-Methanesulfonyl-phenyl)-{5-nitro-6-[4-(pyridin-2-yloxy)-piperidin-1-yl]-pyrimidin-4-yl}-amine |
| B86 | 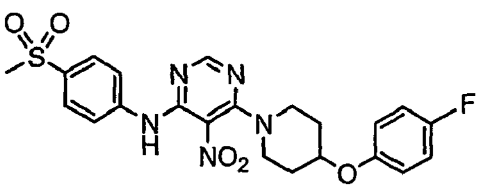 | {6-[4-(4-Fluoro-phenoxy)-piperidin-1-yl]-5-nitro-pyrimidin-4-yl}-(4-methanesulfonyl-phenyl)-amine |

(continued)

| Cmpd# | Structure | Chemical Name |
|---|---|---|
| B87 | 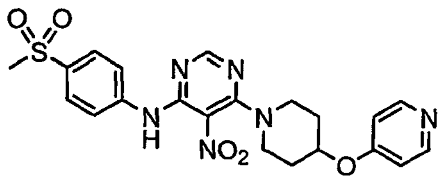 | (4-Methanesulfonyl-phenyl)-{5-nitro-6-[4-(pyridin-4-yloxy)-piperidin-1-yl]-pyrimidin-4-yl}-amine |
| B88 | 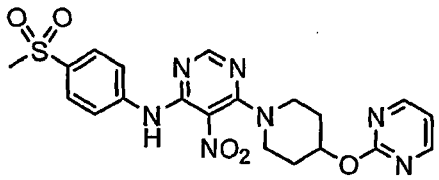 | (4-Methanesulfonyl-phenyl)-{5-nitro-6-[4-(pyrimidin-2-yloxy)-piperidin-1-yl]-pyrimidin-4-yl}-amine |
| B89 | 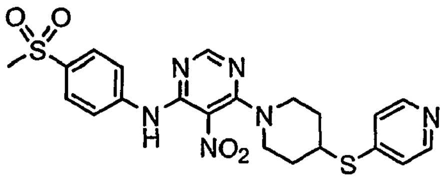 | (4-Methanesulfonyl-phenyl)-{5-nitro-6-[4-(pyridin-4-ylsulfanyl)-piperidin-1-yl]-pyrimidin-4-yl}-amine |
| B90 | 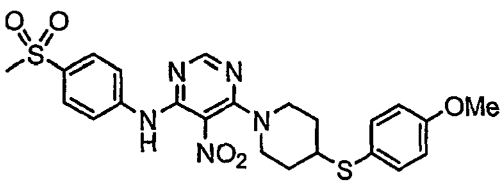 | (4-Methanesulfonyl-phenyl)-{6-[4-(4-methoxy-phenylsulfanyl)-piperidin-1-yl]-5-nitro-pyrimidin-4-yl}-amine |
| B91 | 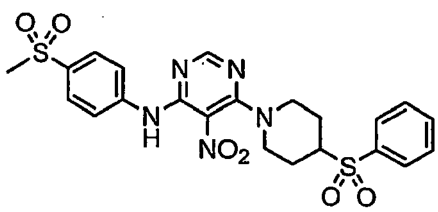 | [6-(4-Benzenesulfonyl-piperidin-1-yl)-5-nitro-pyrimidin-4-yl]-(4-methanesulfonyl-phenyl)-amine |
| B92 | 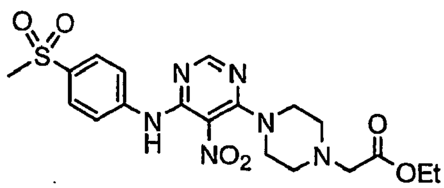 | {4-[6-(4-Methanesulfonyl-phenylamino)-5-nitro-pyrimidin-4-yl]-piperazin-1-yl}-acetic acid ethyl ester |
| B93 | 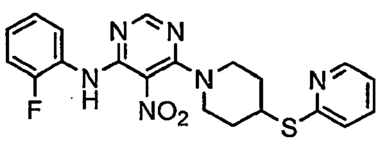 | (2-Fluoro-phenyl)-{5-nitro-6-[4-(pyridin-2-ylsulfanyl)-piperidin-1-yl]-pyrimidin-4-yl}-amine |
| B94 | 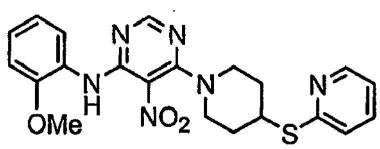 | 2-Methoxy-phenyl)-{5-nitro-6-[4-(pyridin-2-ylsulfanyl)-piperidin-1-yl]-pyrimidin-4-yl} -amine |

(continued)

| Cmpd# | Structure | Chemical Name |
|---|---|---|
| B95 | 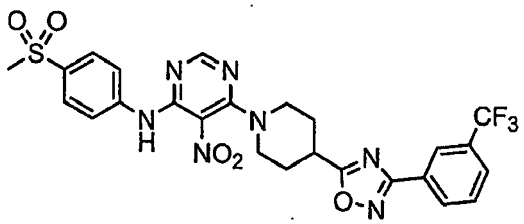 | (4-Methanesulfonyl-phenyl)-(5-nitro-6-{4-[3-(3-trifluoromethyl-phenyl)-[1,2,4]oxadiazol-5-yl]-piperidin-1-yl} pyrimidin-4-yl)-amine |
| B96 | 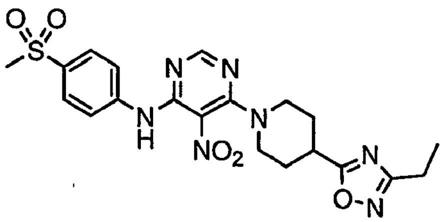 | {6-[4-(3-Ethyl-[1,2,4]oxadiazol-5-yl)-piperidin-1-yl]-5-nitro-pyrimidin-4-yl}-(4-methanesulfonyl-phenyl)-amine |
| B97 | 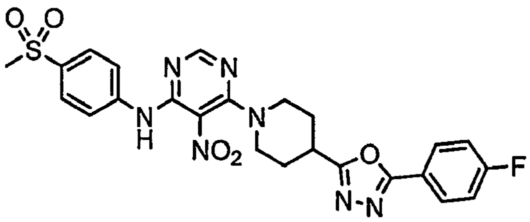 | (6-{4-[5-(4-Fluoro-phenyl)-[1,3,4]oxadiazol-2-yl]-piperidin-1-yl}-5-nitro-pyrimidin-4-yl)-(4-methanesulfonyl-phenyl)-amine |
| B98 | 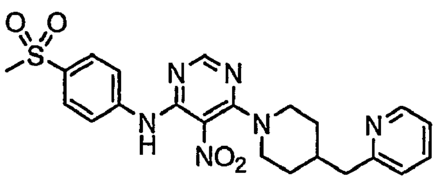 | (4-Methanesulfonyl-phenyl)-[5-nitro-6-(4-pyridin-2-ylmethyl-piperidin-1-yl)-pyrimidin-4-yl]-amine |
| B99 | 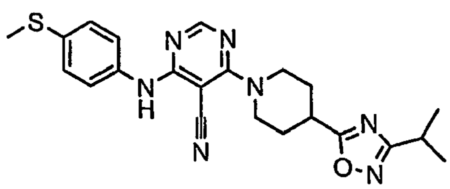 | 4-[4-(3-Isopropyl-[1,2,4]oxadiazol-5-yl)-piperidin-1-yl]-6-(4-methylsulfanyl-phenylamino)-pyrimidine-5-carbonitrile |
| B100 | 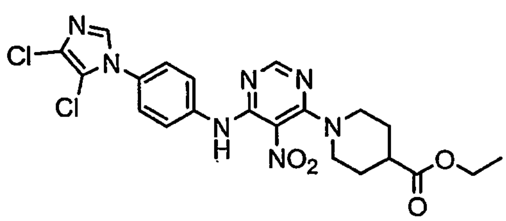 | 1-{6-[4-(4,5-Dichloro-imidazol-1-yl)-phenylamino]-5-nitro-pyrimidin-4-yl}-piperidine-4-carboxylic acid ethyl ester |
| B101 | 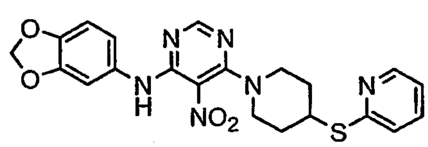 | Benzo[1,3]dioxol-5-yl-{5-nitro-6-[4-(pyridin-2-ylsulfanyl)-piperidin-1-yl]-pyrimidin-4-yl}-amine |

(continued)

| Cmpd# | Structure | Chemical Name |
|---|---|---|
| B102 | 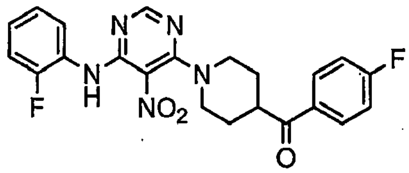 | (4-Fluoro-phenyl)-{1-[6-(2-fluoro-phenylamino)-5-nitro-pyrimidin-4-yl]-piperidi-4-yl} -methanone |
| B103 | 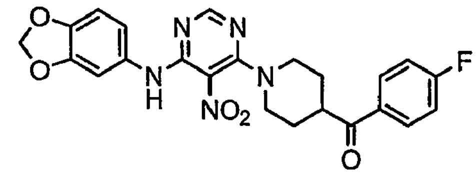 | {1-[6-(Benzo[1,3]dioxol-5-ylamino)-5-nitro-pyrinudin-4-yl]-piperidin-4-yl} -(4-fluoro-phenyl)-methanone |
| B104 | 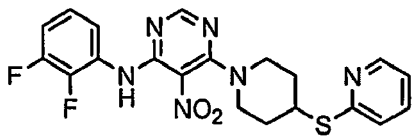 | (2,3-Difluoro-phenyl)-{5-nitro-6-[4-(pyridin-2-ylsulfanyl)-piperidin-1-yl]-pyrimidin4-yl}-amine |
| B105 | 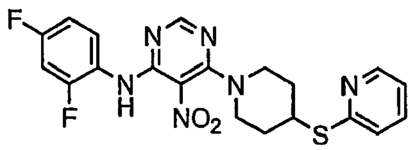 | (2,4-Difluoro-phenyl)-{5-nitro-6-[4-(pyridin-2-ylsulfanyl)-piperidin-1-yl]-pyrimidin-4-yl}-amine |
| B106 | 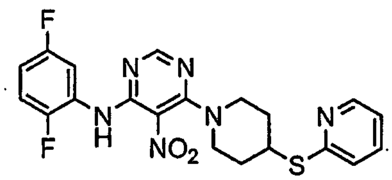 | (2,5-Difluoro-phenyl)-{5-nito-6-[4-(pyridin-2-ylsulfanyl)-piperidin-1-yl]-pyrimidin-4-yl}-amine |
| B107 | 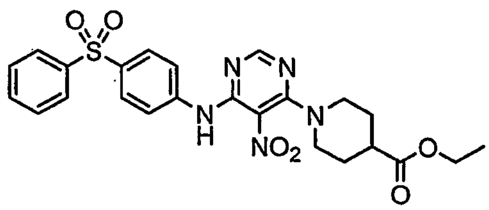 | 1-[6-(4-Benzenesulfonyl-phenylamino)-5-nitro-pyrimidin-4-yl]-piperidine-4-carboxylic acid ethyl ester |
| B108 | 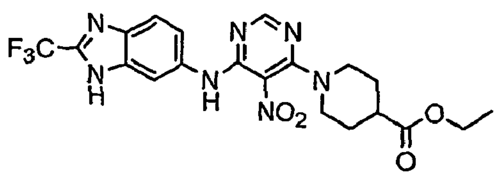 | 1-[5-Nitro-6-(2-trifluoromethyl-3H-benzoimidazol-5-ylamino)-pyrimidin-4-yl]-piperidine-4-carboxylic acid ethyl ester |
| B109 | 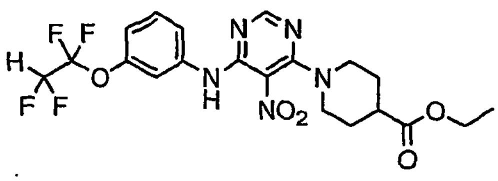 | 1-{5-Nitro-6-[3-(1,1,2,2-tetrafluoro-ethoxy)-phenylamino]-pyrimidin-4-yl}-piperidine-4-carboxylic acid ethyl ester |

(continued)

| Cmpd# | Structure | Chemical Name |
|---|---|---|
| B110 | 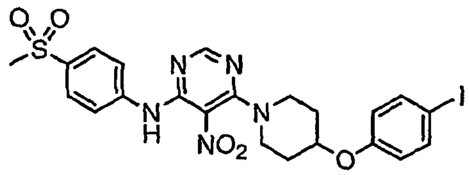 | {6-[4-(4-Iodo-phenoxy)-piperidin-1-yl]-5-nitro-pyrimidin-4-yl}-(4-methanesulfonyl-phenyl)-amine |
| B111 | 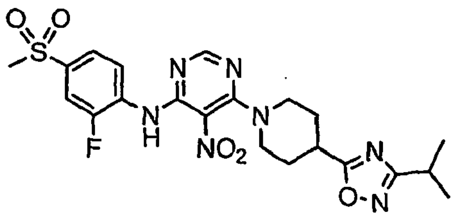 | (2-Fluoro-4-methanesulfonyl-phenyl)-{6-[4-(3-isopropyl-[1,2,4]oxadiazol-5-yl)-piperidin-1-yl]-5-nitro-pyrimidin-4-yl}-amine |
| B112 | 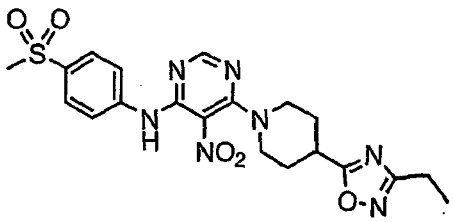 | {6-[4-(3-Ethyl-[1,2,4]oxadiazol-5-yl)-piperidin-1-yl]-5-nitro-pyrimidin-4-yl}-(2-fluoro-4-methanesulfonyl-phenyl)-amine |
| B113 | 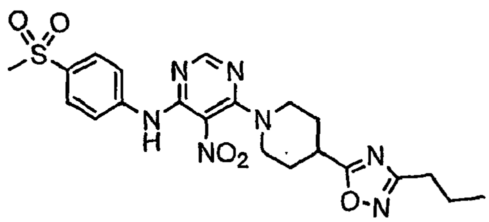 | (4-Methanesulfonyl-pheriyl)-{5-nitro-6-[4-(3-propyl-[1,2,4]oxadiazol-5-yl)-piperidin-1-yl]-pyrimidin-4-yl}-amine |
| B114 | 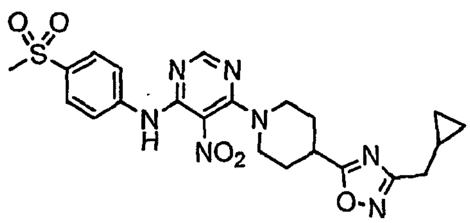 | {6-[4-(3-Cyclopropylmethyl-[1,2,4]oxadiazol-5-yl)-piperidin-1-yl]-5-nitro-pyrimidin-4-yl}-(4-methanesulfonyl-phenyl)-amine |
| B115 | 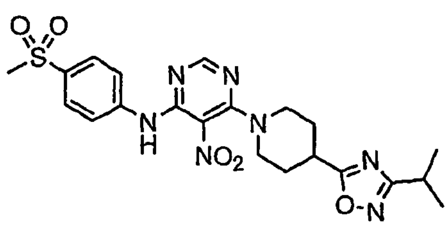 | {6-[4-(3-Isopropyl-[1,2,4]oxadiazol-5-yl)-piperidin-1-yl]-5-nitro-pyrimidin-4-yl}-(4-methanesulfonyl-phenyl)-amine |
| B116 | 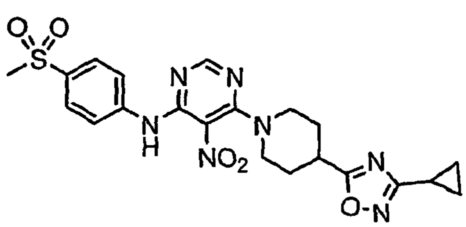 | {6-[4-(3-Cyclopropyl-[1,2,4]oxadiazol-5-yl)-piperidin-1-yl]-5-nitro pyrimidin-4-yl}-(4-methanesulfonyl-phenyl)-amine |

(continued)

| Cmpd# | Structure | Chemical Name |
|---|---|---|
| B117 | 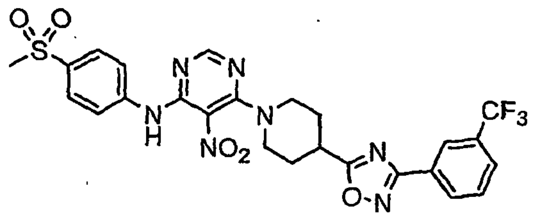 | (4-Methanesulfonyl-phenyl)-(5-nitro-6-{4-[3-(3-trifluoromethyl-phenyl)-[1,2,4]oxadiazol-5-yl]-pipendin-1-yl} pyrimidin-4-yl)-amine |
| B118 | 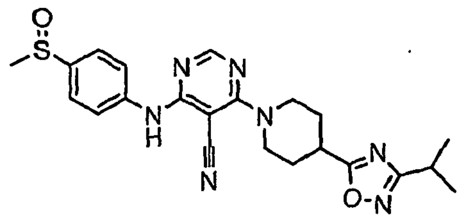 | 4-[4-(3-Isopropyl-[1,2,4]oxadiazol-5-yl)-piperidin-1-yl]-6-(4-methanesulfinyl-phenylamino)-pyrimidine-5-carbonitrile |
| B119 | 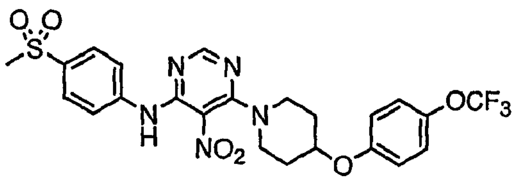 | (4-Methanesulfonyl-phenyl)-{5-nitro-6-[4-(4-trifluoromethoxy-phenoxy)-piperidin-1-yl]-pyrimidin-4-yl}-amine |
| B120 | 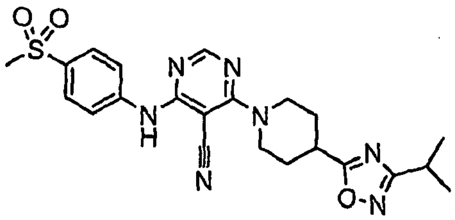 | 4-[4-(3-Isopropyl-[1,2,4]oxadiazol-5-yl)-piperidin-1-yl]-6-(4-methanesulfonyl-phenylamino)-pyrimidine-5-carbonitrile |
| B121 | 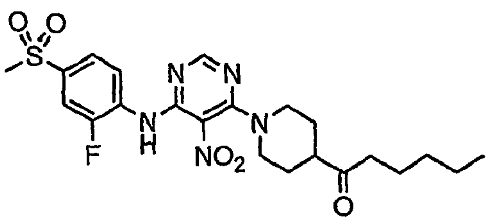 | 1-{1-[6-(2-Fluoro-4-methanesulfonyl-phenylamino)-5-nitro-pyrimidin-4-yl]-piperidin-4-yl}-hexan-1-one |
| B122 | 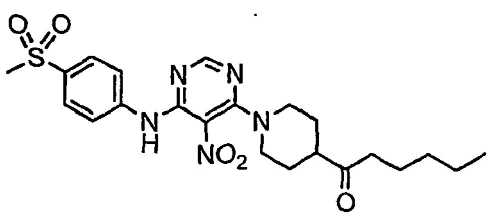 | 1-{1-[6-(4-Methanesulfonyl-phenylamino)-5-nitro-pyrimidin-4-yl]-piperidin-4-yl}-hexan-1-one |
| B123 | 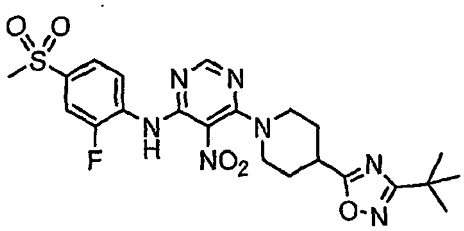 | {6-[4-(3-tert-Butyl-[1,2,4]oxadiazol-5-yl)-piperidin-1-yl]-5-nitro-pyrimidin-4-yl}-(2-fluoro-4-methanesulfonyl-phenyl)-amine |

(continued)

| Cmpd# | Structure | Chemical Name |
|---|---|---|
| B124 | 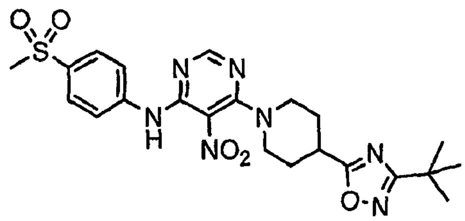 | {6-[4-(3-tert-Butyl-[1,2,4]oxadiazol-5-yl)-piperidin-1-yl]-5-nitro-pyrimidin-4-yl}-(4-methanesulfonyl-phenyl)-amine |
| B125 | 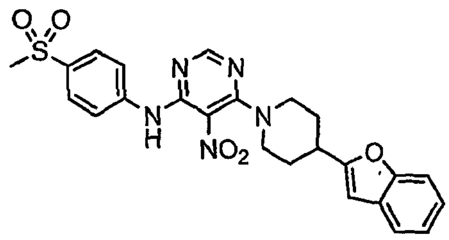 | [6-(4-Benzofuran-2-yl-piperidin-1-yl)-5-nitro-pyrimidin-4-yl]-(4-methanesulfonyl-phenyl)-amine |
| B126 | 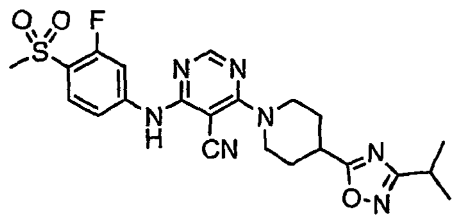 | 4-(3-Fluoro-4-methanesulfonyl-phenylamino)-6-[4-(3-isopropyl-[1,2,4]oxadiazol-5-yl)-piperidin-1-yl]-pyrimidine-5-carbonitrile |
| B127 | 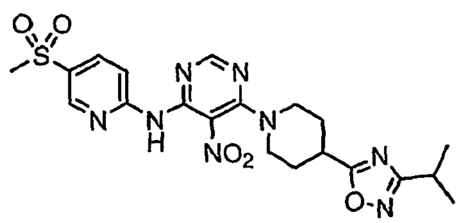 | {6-[4-(3-Isopropyl-[1,2,4]oxadiazol-5-yl)-piperidin-1-yl]-5-nitro-pyrimidin-4-yl}-(5-methanesulfonyl-pyridin-2-yl)-amine |
| B128 | 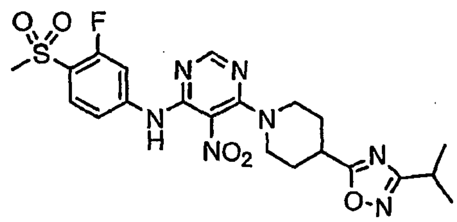 | (3-Fluoro-4-methanesulfonyl-phenyl)-{6-[4-(3-isopropyl-[1,2,4]oxadiazol-5-yl)-piperidin-1-yl]-5-nitro-pyrimidin-4-yl}-amine |
| B129 | 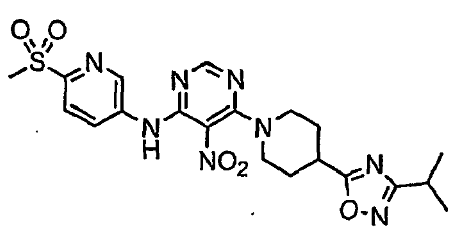 | {6-[4-(3-Isopropyl-[1,2,4]oxadia2ol-5-yl)-piperidin-1-yl]-5-nitro-pyrimidin-4-yl}-(6-methanesulfonyl-pyridin-3-yl)-amine |

(continued)

| Cmpd# | Structure | Chemical Name |
|---|---|---|
| B130 | 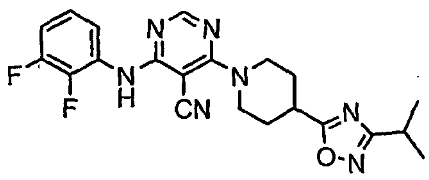 | 4-(2,3-Difluoro-phenylamino)-6-[4-(3-isopropyl-[1,2,4]oxadiazol-5-yl)-piperidin-1-yl]-pyrimidine-5-carbonitrile |
| B131 | 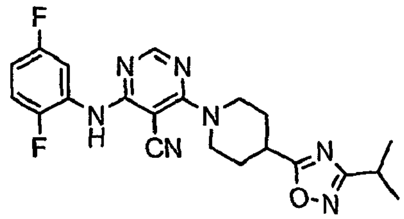 | 4-(2,5-Difluoro-phenylamino)-6-[4-(3-isopropyl-[1,2,4]oxadiazol-5-yl)-piperidin-1-yl]-pyrimidine-5-carbonitrile |
| B132 | 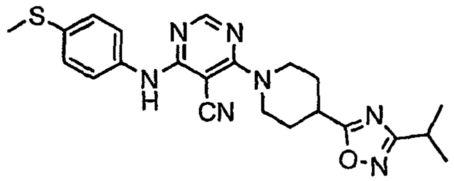 | 4-[4-(3-Isopropyl-[1,2,4]oxadiazol-5-yl)-piperidin-1-yl]-6-(4-methylsulfanyl-phenylamino)-pyrimidine-5-carbonitrile |
| B133 | 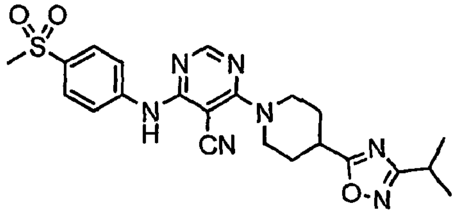 | 4-[4-(3-Isopropyl-[1,2,4]oxadiazol-5-yl)-piperidin-1-yl]-6-(4-methanesulfonyl-phenylamino)-pyrimidine-5-carbonitrile |
| B134 | 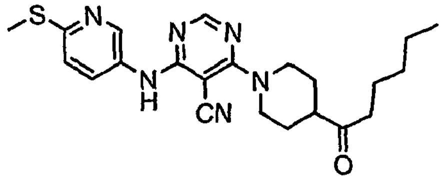 | 4-(4-Hexanoyl-piperidin-1-yl)-6-(6-methylsulfanyl-pyridin-3-ylamino)-pyrimidine-5-carbonitrile |
| B135 | 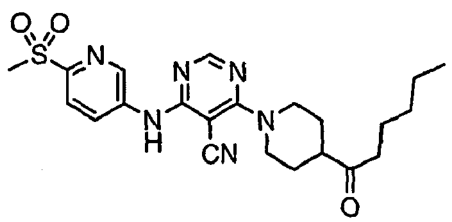 | 4-(4-Hexanoyl-piperidin-1-yl)-6-(6-methanesulfonyl-pyridin-3-ylamino)-pyrimidine-5-carbonitrile |
| B136 | 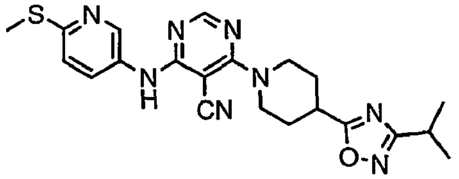 | 4-[4-(3-Isopropyl-[1,2,4]oxadiazol-5-yl)-piperidin-1-yl]-6-(6-methylsulfanyl-pyridin-3-ylamino)-pyrimidine-5-carbonitrile |

(continued)

| Cmpd# | Structure | Chemical Name |
|---|---|---|
| B137 | 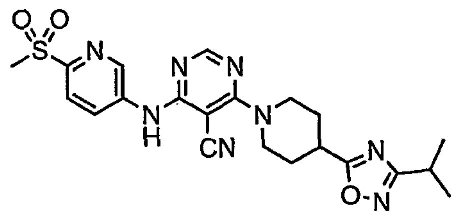 | 4-[4-(3-Isopropyl-[1,2,4]oxadiazol-5-yl)-piperidin-1-yl]-6-(6-methanesulfonyl-pyridin-3-ylamino)-pyrimidine-5-carbonitrile |
| B138 | 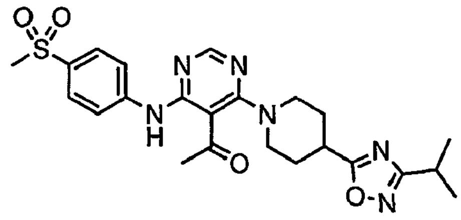 | 1-[4-[4-(3-Isopropyl-[1,2,4]oxadiazol-5-yl)-piperidin-1-yl]-6-(4-methanesulfonyl-phenylamino)-pyrimidin-5-yl]-ethanone |
| B139 | 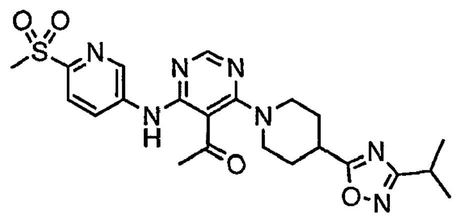 | 1-[4-[4-(3-Isopropyl-[1,2,4]oxadiazol-5-yl)-piperidin-1-yl]-6-(6-methanesulfonyl-pyridin-3-ylamino)-pyrimidin-5-yl]-ethanone |

**TABLE C**

| Cmpd# | Structure | Chemical Name |
|---|---|---|
| C1 | 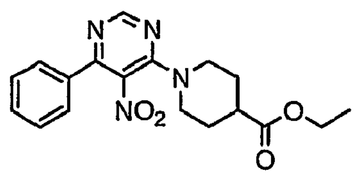 | 1-(5-Nitro-6-phenyl-pyrimidin-4-yl)-piperidine-4-carboxylic acid ethyl ester |
| C2 | 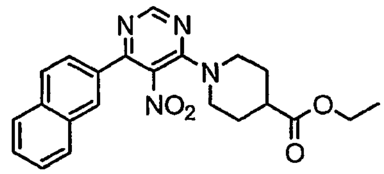 | 1-(6-Naphthalen-2-yl-5-nitro-pyrimidin-4-yl)-piperidine-4-carboxylic acid ethyl ester |
| C3 | 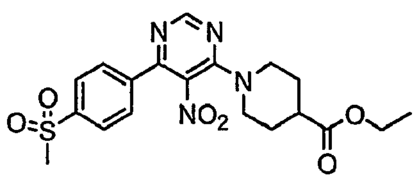 | 1-[6-(4-Methanesulfonyl-phenyl)-5-nitro-pyrimidin-4-yl]-piperidine-4-carboxylic acid ethyl ester |

(continued)

| Cmpd# | Structure | Chemical Name |
|---|---|---|
| C4 | 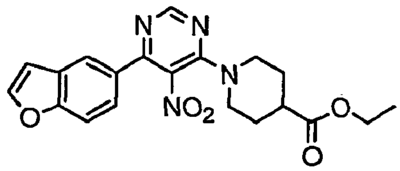 | 1-(6-Benzofuran-5-yl-5-nitro-pyrimidin-4-yl)-piperidine-4-carboxylic acid ethyl ester |
| C5 | 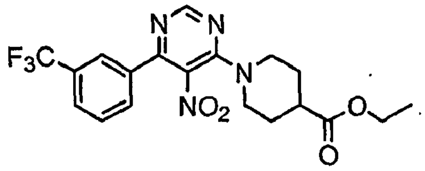 | 1-[5-Nitro-6-(3-tnifluoromethyl-phenyl)-pyrimidin-4-yl]-piperidine-4-carboxylic acid ethyl ester |
| C6 | 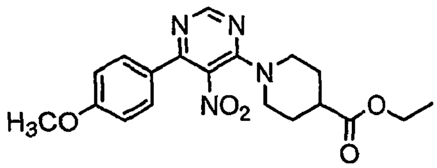 | 1-[6-(4-Methoxy-phenyl)-5-nitro-pyrimidin-4-yl]-piperidine-4-carboxylic acid ethyl ester |
| C7 | 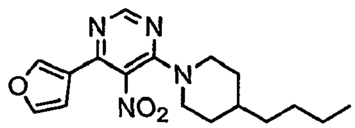 | 4-(4-Butyl-piperidin-1-yl)-6-furan-3-yl-5-nitro-pyrimidine |
| C8 | 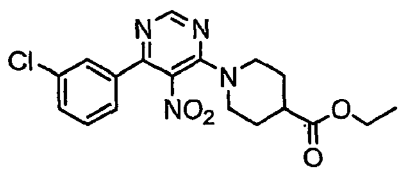 | 1-[6-(3-Chloro-phenyl)-5-nitro-pyrimidin-4-yl]-piperidine-4-carboxylic acid ethyl ester |
| C9 | 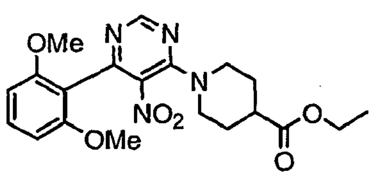 | 1-[6-(2,6-Dimethoxy-phenyl)-5-nitro-pyrimidin-4-yl]-piperidine-4-carboxylic acid ethyl ester |
| C10 | 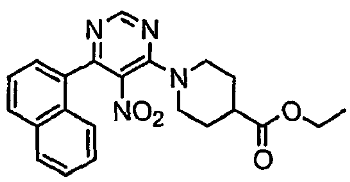 | 1-(6-Naphthalen-1-yl-5-nitro-pyrimidin-4-yl)-piperidine-4-carboxylic acid ethyl ester |
| C11 | 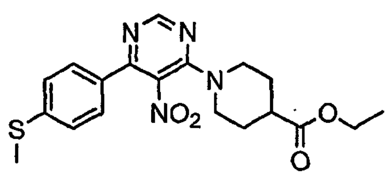 | 1-[6-(4-Methylsulfanyl-phenyl)-5-nitro-pyrimidin-4-yl]-piperidine-4-carboxylic acid ethyl ester |

(continued)

| Cmpd# | Structure | Chemical Name |
|---|---|---|
| C12 | 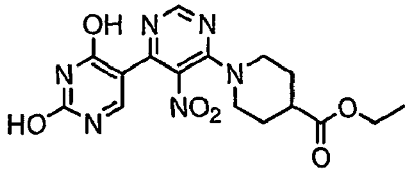 | 1-(2',4'-Dihydroxy-5-nitro-[4,5']bipyrimidinyl-6-yl)-piperidine-4-carboxylic acid ethyl ester |
| C13 | 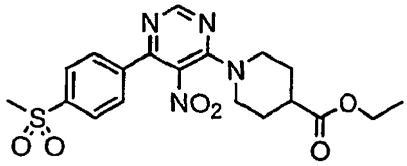 | 1-[6-(4-Methanesulfonyl-phenyl)-5-nitro-pyrimidin-4-yl] piperidine-4-carboxylic acid ethyl ester |
| C14 | 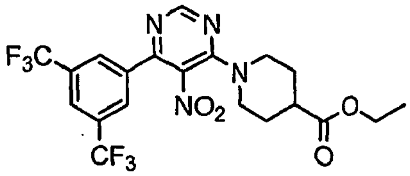 | 1-[6-(3,5-Bis-trifluoromethyl-phenyl)-5-nitro-pyrimidin-4-yl]-piperidine-4-carboxylic acid ethyl ester |
| C15 | 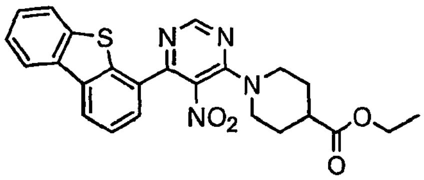 | 1-(6-Dibenzothiophen-4-yl-5-nitro-pyrimidin-4-yl)-piperidine-4-carboxylic acid ethyl ester |
| C16 | 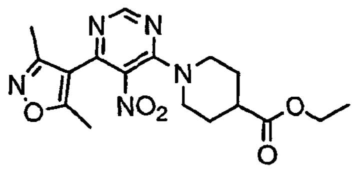 | 1-[6-(3,5-Dimethyl-isoxazol-4-yl)-5-nitro-pyfimidin-4-yl]-piperidine-4-carboxylic acid ethyl ester |
| C17 | 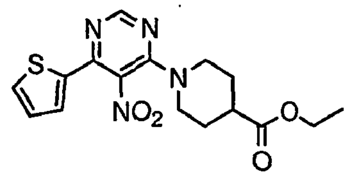 | 1-(5-Nitro-6-thiophen-2-yl-pyrimidin-4-yl)-piperidine-4-carboxylic acid ethyl ester |
| C18 | 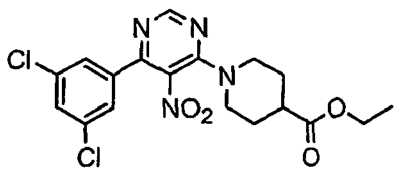 | 1-[6-(3,5-Dichloro-phenyl)-5-nitro-pyrimidin-4-yl]-piperidine-4-carboxylic acid ethyl ester |
| C19 | 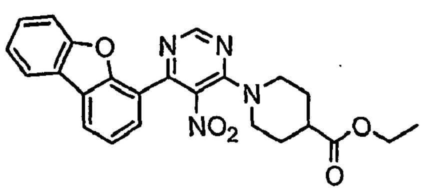 | 1-(6-Dibenzofuran-4-yl-5-nitro-pyrimidin-4-yl)-piperidine-4-carboxylic acid ethyl ester |

(continued)

| Cmpd# | Structure | Chemical Name |
|---|---|---|
| C20 | 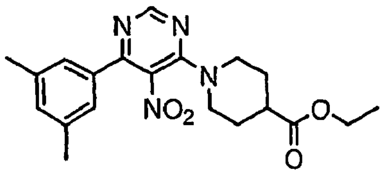 | 1-[6-(3,5-Dimethyl-phenyl)-5-nitro-pyrimidin-4-yl]-piperidine-4-carboxylic acid ethyl ester |
| C21 | 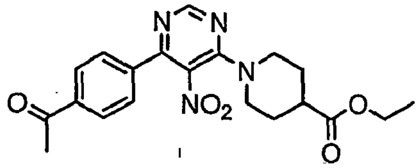 | 1-[6-(4-Acetyl-phenyl)-5-nitro-pyrimidin-4-yl]-piperidine-4-carboxylic acid ethyl ester |
| C22 | 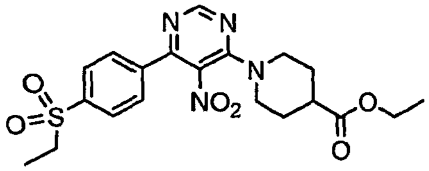 | 1-[6-(4-Ethanesulfonyl-phenyl)-5-nitro-pyrimidin-4-yl] piperidine-4-carboxylic acid ethyl ester |
| C23 | 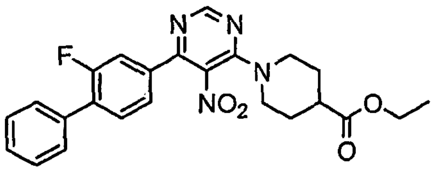 | 1-[6-(2-Fluoro-biphenyl-4-yl)-5-nitro-pyrimidin-4-yl]-piperidine-4-carboxylic acid ethyl ester |
| C24 | 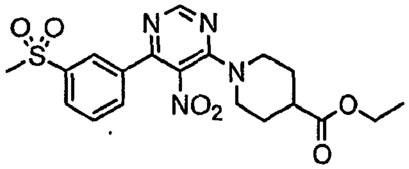 | 1-[6-(3-Methanesulfonyl-phenyl)-5-nitro-pyrimidin-4-yl]-piperidine-4-carboxylic acid ethyl ester |
| C25 | 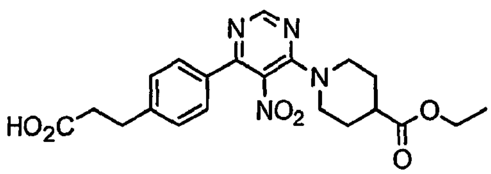 | 1-{6-[4-(2-Carboxy-ethyl)-phenyl]-5-nitro-pyrimidin-4-yl}-piperidine-4-carboxylic acid ethyl ester |
| C26 | 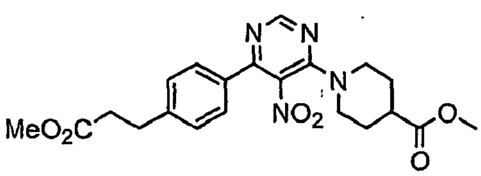 | 1-{6-[4-(2-Methoxycarbonyl-ethyl)-phenyl]-5-nitro-pyrimidin-4-yl}-piperidine-4-carboxylic acid methyl ester |
| C27 | 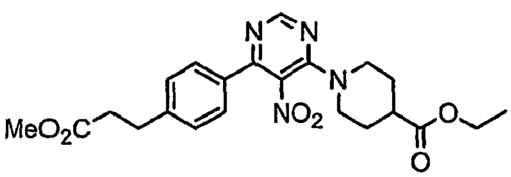 | 1-{6-[4-(2-Methoxycarbonyl-ethyl)phenyl]-5-nitro-pyrimidin-4-yl}-piperidine-4-carboxylic acid ethyl ester |

**TABLE D**

| Cmpd# | Structure | Chemical Name |
|---|---|---|
| D1 | 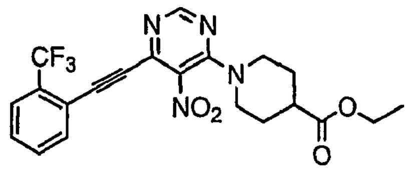 | 1-[5-Nitro-6-(2-trifluoromethyl-phenylethynyl)-pyrimidin-4-yl]-piperidine-4-carboxylic acid ethyl ester |
| D2 | 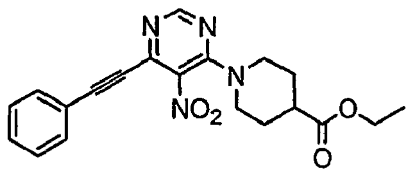 | 1-(5-Nitro-6-phenylethynyl-pyrimidin-4-yl)-piperidine-4-carboxylic acid ethyl ester |
| D3 | 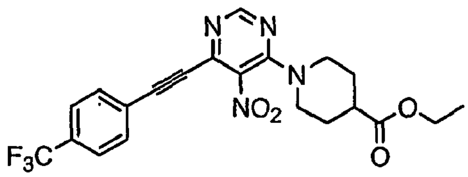 | 1-[5-Nitro-6-(4-trifluoromethyl-phenylethynyl)-pyrimidin-4-yl]-piperidine-4-carboxylic acid ethyl ester |
| D4 | 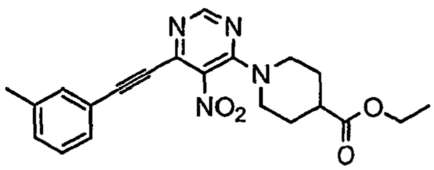 | 1-(5-Nitro-6-m-tolylethynyl-pyrimidin-4-yl)-piperidine-4-carboxylic acid ethyl ester |
| D5 | 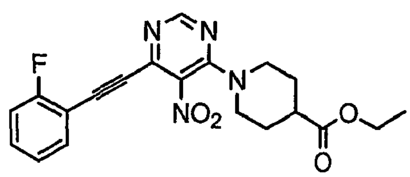 | 1-[6-(2-Fluoro-phenylethynyl)-5-nitro-pyrimidin-4-yl]-piperidine-4-carboxylic acid ethyl ester |
| D6 | 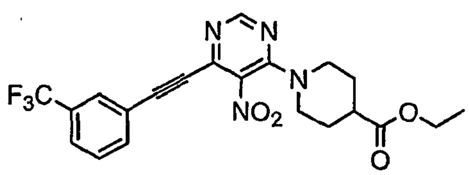 | 1-[5-Nitro-6-(3-trifluoromethyl-phenylethynyl)-pyrimidin-4.-yl]-piperidine-4-carboxylic acid ethyl ester |

**TABLE E**

| Cmpd# | Structure | Chemical Name |
|---|---|---|
| EI | 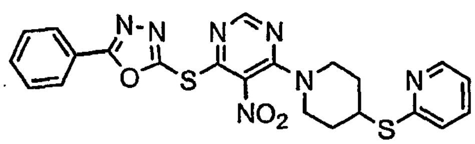 | 5-Nitro-4-(5-phenyl-[1,3,4]oxadiazol-2-ylsulfanyl)-6-[4-(pyridin-2-ylsulfanyl)-piperidin-1-yl]-pyrimidine |

Some embodiments of the present invention include a pharmaceutical composition comprising at least one compound according to any of the compound embodiments disclosed herein and a pharmaceutically acceptable carrier.

Additionally, compounds of Formula **(Ia)** encompass all pharmaceutically acceptable solvates, particularly

hydrates, thereof. The present invention also encompasses diastereomers as well as optical isomers, e.g. mixtures of enantiomers including racemic mixtures, as well as individual enantiomers and diastereomers, which arise as a consequence of structural asymmetry in certain compounds of Formula **(Ia).** Separation of the individual isomers or selective synthesis of the individual isomers is accomplished by application of various methods which are well known to practitioners in the art.

## INDICATIONS

In addition to the foregoing beneficial uses for compounds of the present invention disclosed herein, compounds of the invention are useful in the prophylaxis or treatment of additional diseases. Without limitation, these include the following.

The most significant pathologies in Type II diabetes are impaired insulin signaling at its target tissues ("insulin resistance") and failure of the insulin-producing cells of the pancreas to secrete an appropriate degree of insulin in response to a hyperglycemic signal. Current therapies to treat the latter include inhibitors of the β-cell ATP-sensitive potassium: channel to trigger the release of endogenous insulin stores, or administration of exogenous insulin. Neither of these achieves accurate normalization of blood glucose levels and both carry the risk of inducing hypoglycemia. For these reasons, there has been intense interest in the development of pharmaceuticals that function in a glucose-dependent action, i.e. potentiators of glucose signaling. Physiological signaling systems which function in this manner are well-characterized and include the gut peptides GLP1, GIP and PACAP. These hormones act via their cognate G-protein coupled receptor to stimulate the production of cAMP in pancreatic β-cells. The increased cAMP does not appear to result in stimulation of insulin release during the fasting or preprandial state. However, a series of biochemical targets of cAMP signaling, including the ATP-sensitive potassium channel, voltage-sensitive potassium channels and the exocytotic machinery, are modified in such a way that the insulin secretory response to a postprandial glucose stimulus is markedly enhanced. Accordingly, agonists of novel, similarly functioning, β-cell GPCRs, including **RUP3,** would also stimulate the release of endogenous insulin and consequently promote normoglycemia in Type II diabetes.

It is also established that increased cAMP, for example as a result of GLP1 stimulation, promotes β-cell proliferation, inhibits β-cell death and thus improves islet mass. This positive effect on β-cell mass is expected to be beneficial in both Type II diabetes, where insufficient insulin is produced, and Type I diabetes, where β-cells are destroyed by an inappropriate autoimmune response.

It is also well-established that metabolic diseases exert a negative influence on other physiological systems. Thus, there is often the codevelopment of multiple disease states (e.g. type I diabetes, type II diabetes, inadequate glucose tolerance, insulin resistance, hyperglycemia, hyperlipidemia, hypertriglyceridemia, hypercholesterolemia, dyslipidemia, obesity or cardiovascular disease in "Syndrome X") or secondary diseases which clearly occur secondary to diabetes (e.g. kidney disease, peripheral neuropathy). Thus, it is expected that effective treatment of the diabetic condition will in turn be of benefit to such interconnected disease states.

Some embodiments of the present invention include a method for prophylaxis or treatment of a metabolic disorder or complications thereof in an individual comprising administering to the individual a therapeutically effective amount of a compound of the present invention or a pharmaceutical composition thereof. In some embodiments, the metabolic disorder or complications thereof is type I, type II diabetes, inadequate glucose tolerance, insulin resistance, hyperglycemia, hyperlipidemia, hypertriglyceridemia, hypercholesterolemia, dyslipidemia syndrome X, or metabolic syndrome. In some embodiments, the metabolic disorder is type II diabetes. In some embodiments, the metabolic disorder is hyperglycemia. In some embodiments, the metabolic disorder is hyperlipidemia. In some embodiments, the metabolic disorder is hypertriglyceridemia. In some embodiments, the metabolic disorder is type I diabetes. In some embodiments, the metabolic disorder is dyslipidemia. In some embodiments, the metabolic disorder is syndrome X. In some embodiments, the individual is a mammal. In some embodiments, the mammal is a human.

Some embodiments of the present invention include a method of controlling or decreasing weight gain of an individual comprising administering to the individual a therapeutically effective amount of a compound of the present invention or pharmaceutical composition thereof. In some embodiments, the individual is a mammal. In some embodiments, the mammal is a human. In some embodiments, the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

One aspect of the present invention pertains to a compound of Formula **(Ia),** as described herein, for use in a method of treatment of the human or animal body by therapy.

Some embodiments of the present invention include a method of modulating a **RUP3** receptor comprising contacting the receptor with a compound of the present invention.

Some embodiments of the present invention include a method of modulating a **RUP3** receptor in an individual comprising contacting the receptor with a compound of the present invention. In some embodiments, the compound is

an agonist. In some embodiments, the compound is an inverse agonist.

Some embodiments of the present invention include a method of modulating a **RUP3** receptor in an individual comprising contacting the receptor with a compound of the present invention wherein the modulation of the **RUP3** receptor is prophylaxis or treatment of a metabolic disorder and complications thereof. In some embodiments, the metabolic disorder is type I, type II diabetes, inadequate glucose tolerance, insulin resistance, hyperglycemia, hyperlipidemia, hypertriglyceridemia, hypercholesterolemia, dyslipidemia syndrome X, or metabolic syndrome. In some embodiments, the metabolic disorder is type II diabetes. In some embodiments, the metabolic disorder is hyperglycemia. In some embodiments, the metabolic disorder is hyperlipidemia. In some embodiments, the metabolic disorder is hypertriglyceridemia. In some embodiments, the metabolic disorder is type I diabetes. In some embodiments, the metabolic disorder is dyslipidemia. In some embodiments, the metabolic disorder is syndrome X. In some embodiments, the individual is a mammal. In some embodiments, the mammal is a human.

Some embodiments of the present invention include a method of modulating a **RUP3** receptor in an individual comprising contacting the receptor with a compound of the present invention wherein the modulation of the **RUP3** receptor controls or reduces weight gain of the individual. In some embodiments, the individual is a mammal. In some embodiments, the mammal is a human. In some embodiments, the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

Some embodiments of the present invention include the use of a compound of the present invention for production of a medicament for use in prophylaxis or treatment of a metabolic disorder. In some embodiments, the metabolic disorder is type II diabetes, inadequate glucose tolerance, insulin resistance, hyperglycemia, hyperlipidemia, hypertriglyceridemia, hypercholesterolemia, dyslipidemia syndrome X, or metabolic syndrome.

Some embodiments of the present invention include the use of a compound of the present invention for production of a medicament for use in controlling or decreasing weight gain in an individual. In some embodiments, the individual is a mammal. In some embodiments, the mammal is a human. In some embodiments, the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

Compounds of the present invention are identified as an agonist or an inverse agonist using methods known to those skilled in art, such as an assay as described in Example 1. Accordingly, representative examples of compounds of the present invention that are agonists include the following:

[6-(4-Benzenesulfonyl-piperidin-1-yl)-5-nitro-pyrimidin-4-yl]-(4-methanesulfonyl-phenyl)-amine; {4-[6-(4-Methanesulfonyl-phenylamino)-5-nitro-pyrimidin-4-yl]-piperazin-1-yl}-acetic acid ethyl ester; (2-Fluoro-phenyl)-{5-nitro-6-[4-(pyridin-2-ylsulfanyl)-piperidin-1-yl]-pyrimidin-4-yl}-amine; 1-[6-(4-Imidazol-1-yl-phenoxy)-5-nitro-pyrimidin-4-yl]-piperidine-4-carboxylic acid ethyl ester; 1-[5-Nitro-6-(4-[1,2,4]triazol-1-yl-phenoxy)-pyrimidin-4-yl]-piperidine-4-carboxylic acid ethyl ester; {6-[4-(4-Fluoro-phenoxy)-piperidin-1-yl]-5-nitro-pyrimidin-4-yl}-(4-methanesulfonyl-phenyl)-amine; {6-[4-(3-Isopropyl-[1,2,4]oxadiazol-5-yl)-piperidin-1-yl]-5-nitro-pyrimidin-4-yl}-(4-methanesulfonyl-phenyl)-amine; {6-[4-(3-Cyclopropyl-[1,2,4]oxadiazol-5-yl)-piperidin-1-yl]-5-nitro-pyrimidin-4-yl}-(4-methanesulfonyl-phenyl)-amine; (4-Methanesulfonyl-phenyl)-(5-nitro-6-{4-(3-(3-trifluoromethyl-phenyl)-[1,2,4]oxadiazol-5-yl]-piperidin-1-yl}-pyrimidin-4-yl)-amine; {6-[4-(3-Ethyl-[1,2,4]oxadiazol-5-yl)-piperidin-1-yl]-5-nitro-pyrimidin-4-yl}-(2-fluoro-phenyl)-amine; (2-Fluoro-4-methanesulfonyl-phenyl)-{6-[4-(3-isopropyl-[1,2,4]oxadiazol-5-yl)-piperidin-1-yl]-5-nitro-pyrimidin-4-yl}-amine; {6-[4-(3-Ethyl-[1,2,4]oxadiazol-5-yl)-piperidin-1-yl]-5-nitro-pyrimidin-4-yl}-(2-fluoro-4-methanesulfonyl-phenyl)-amine; (4-Methanesulfonyl-phenyl)-{5-nitro-6-[4-(3-propyl-[1,2,4]oxadiazol-5-yl)-piperidin-1-yl]-pyrimidin-4-yl}-amine; {6-[4-(3-Cyclopropylmethyl-[1,2,4]oxadiazol-5-yl)-piperidin-1-yl]-5-nitro-pyrimidin-4-yl}-(4-methanesulfonyl-phenyl)-amine; (4-Methanesulfonyl-phenyl)-{5-nitro-6-[4-(pyridin-4-yloxy)-piperidin-1-yl]-pyrimidin-4-yl}-amine; (4-Methanesulfonylphenyl)-15-nitro-6-[4-(pyrimidin-2-yloxy)-piperidin-1-yl]-pyrimidin-4-yl}-amine; 1-[6-(4-Carbamoylmethyl-phenoxy)-5-nitro-pyrimidin-4-yl]-piperidine-4-carboxylic acid ethyl ester; 1-{6-[4-(1,3-Dioxo-1,3-dihydro-isoindol-2-yl)-phenoxy]-5-nitro-pynimdin-4-yl}-pipename-4-carboxylic acid ethyl ester; 4'-[4-(2-Methoxycarbonyl-acetyl)-phenoxy]-3-nitro-3,4,5,6-tetrahydro-2H-[1,2']bipyridinyl-4-carboxylic acid ethyl ester; {6-[4-(2-Methoxy-phenylsulfanyl)-piperidin-1-yl]-5-nitro-pyrimidin-4-yl}-(4-[1,2,4]triazol-1-yl-phenyl)-amine; 4'-(2-Amino-4-ethanesulfonyl-phenoxy)-3'-nitro-3,4,5,6-tetrahydro-2H-[1,2']bipyridinyl-4-carboxylic acid ethyl ester; 4'-(4-Imidazol-1-yl-phenoxy)-3'-nitro-3,4,5,6-tetrahydro-2H-[1,2']bipyridinyl-4-carboxylic acid ethyl ester; (4-Methoxy-2-{5-nitro-6-[4-(pyridin-2-ylsulfanyl)-piperidin-1-yl]-pyrimidin-4-yloxy}-phenyl)-phenyl-methanone; 4-{4-[6-(4-Cyclopropylmcthoxyznethyl-piperidin-1-yl)-5-nitro-pyrimidin-4-yloxy]-phenyl}-butan-2-one; 4-{4-[5-Nitro-6-(4-propoxymethyl-piperidin-1-yl)-pyrimidin-4-yloxy]-phenyl}-butan-2-one; 4-{4-[6-(4-Butoxymethyl-piperidin-1-yl)-5-nitro-pyrimidin-4-yloxy]-phenyl}-butan-2-one; 4-{4-[6-(4-Isobutoxymethyl-piperidin-l-yl)-5-nitro-pynmidin-4-yloxy]-phenyl}-butan-2-one; {1-[6-(Benzo[1,3]dioxol-5-ylamino)-5-nitro-pyrimidin-4-yl]-piperidin-4-yl}-(4-fluorophenyl)-methanone;

(2,3.-Difluoro-phenyl)-{5-nitro-6-[4-(pyridin-2-ylsulfanyl)-piperidin-1-yl]-pyrimidin-4-yl}-amine; (2,4-Difluoro-phenyl)-{5-nitro-6-[4-(pyridin-2-ylsulfanyl)-piperidin-1-yl]-pyrimidin-4-yl}-amine; 1-{2-Nitro-3-[4-(3-oxo-butyl)-phenoxy]-phenyl}- piperidine-4-carboxylic acid ethyl ester; 1-[6-(4-Acetyl-phenoxy)-5-nitro-pyrimidin-4-yl]-piperidine-4-carboxylic acid ethyl ester; 3'-Nitro-2'-[4-(3-oxo-butyl)-phenoxy]-3,4,5,6-tetrahydro-2H-[1,4']bipyridinyl-4-carboxylic acid ethyl ester; 4-(4-{5-Nitro-6-[4-(pyridin-2-ylsulfanyl)-piperidm-l-yl]-pyrimidin-4-yloxy}-phenyl)-butan-2-one; 4-(4-{5-Nitro-6-[4-(2-trifluoromethyl-phenoxy)-piperidin-1-yl]-pyrimidin-4-yloxy}-phenyl)-butan-2-one; 4-(4-{6-[4-(3-Methyl-[1,2,4]oxadiazol-5-yl)-piperidin-1-yl]-5-nito-pyrimidin-4-yloxy}-phenyl)-butan-2-one; 4-(2,4-Difluoro-phenoxy)-5-nitro-6-[4-(pyridin-2-ylsulfanyl)-piperidin-1-yl]-pyrimidine; 4-(4-{6-[4-(4-Fluoro-benzoyl)-piperidin-1-yl]-5-nitro-pyrimidin-4-yloxy}- phenyl)-butan-2-one; 4-(4-Methanesulfonyl-phenoxy)-5-nitro-6-[4-(pyridin-2-ylsulfanyl)-cyclohexyl]-pyrimidine; 4- (4-Methanesulfonyl-phenoxy)-5-nitro-6-[4-(pyridin-4-ylsulfanyl)-cyclohexyl]-pyrimidine; 4-(4-Methanesulfonyl-phenoxy)-5-nitro-6-(4-phenylsulfanyl-cyclohexyl)-pyrimidine; 1-{6-[(Benzo[1,3]dioxol-5-ylmethyl)-amino]-5-nitro-pyrimidin-4-yl}-piperidine-4-carboxylic acid ethyl ester; 1-{6-[4-(1,1-Dioxo-1$\lambda^6$-thiomorpholin-4-ylmethyl)-phenylamino]-5-nitro-pyrimidin-4-yl}-piperidine-4-carboxylic acid ethyl ester; 1-[6-(4-Methanesulfonyl-phenylamino)-5-nitro-pyrimidin-4-yl]-piperidine-4-carboxylic acid ethyl ester; 1-[6-(4-Dimethylsulfamoyl-phenylamino)-5-nitro-pyrimidin-4-yl]-piperidine-4-carboxylic acid ethyl ester; 1-[6-(3-Methoxy-phenylamino)-5-nitro-pyrimidin-4-yl]-piperidine-4-carboxylic acid ethyl ester; 1-[6-(2-Methoxy-phenylamino)-5-nitro-pyrimidin-4-yl]-piperidine-4-carboxylic acid ethyl ester; 1-[6-(4-Methanesulfonyl-phenoxy)-5-nitro-pyrimidin-4-yl]-piperidine-4-carboxylic acid ethyl ester; 1-{6-[4-(2-Methoxycarbonyl-acetyl)-phenoxy]-5-nitro-pynmidm-4-yl}-piperidine-4-carboxylic acid ethyl ester; 1-[6-(2-Amino-4-ethanesulfonyl-phenoxy)-5-nitro-pyrimidin-4-yl]-piperidine-4-carboxylic acid ethyl ester; 1-[6-(2,5-Dimethoxy-phenylamino)-5-nitro-pyrimidin-4-yl]-piperidine-4-carboxylic acid ethyl ester; (4-{5-Nitro-6-[4-(pyridin-2-ylsulfanyl)-piperidin-1-yl]-pyrimidin-4-ylamino}-phenyl)-phenyl-methanone; 1-[6-(4-Cyclohexyl-phenylamino)-5-nitro-pyrimidin-4-yl]-piperidine-4-carboxylic acid ethyl ester; 1-[5-Nitro-6-(4-[1,2,4]triazol-1-yl-phenylamino)-pyrimidin-4-yl]-piperidine-4-carboxylic acid ethyl ester; 1-[5-Nitro-6-(4-trifluoromethanesulfonyl-phenylamino)-pyriTnidin-4-yl]-piperidine4-carboxylic acid ethyl ester; 1-[5-Nitro-6-(4-[1,2,3]thiadiazol-4-yl-phenylainino)-pyrimidin-4-yl]-piperidine-4-carboxylic acid ethyl ester; [6-(4-Ethoxymethyl-piperidin-1-yl)-5-nitro-pyrimidin-4-yl]-(4-methanesulfonyl-phenyl)-amine; [5-Nito-6-(4-propyl-piperidin-1-yl)-pyrimidin-4-yl]-(4-[1,2,4]tiazol-1-yl-phenyl)-amine; {5-Nitro-6-[4-(pyndin-2-ylsulfanyl)-pipendin-1-yl]-pyrimidin-4-yl}-(4-[1,2,4]triazol-1-yl-phenyl)-amine; (2-Fluoro-phenyl)-{6-[4-(3-methyl-[1,2,4]oxadiazol-5-yl)-piperidin-1-yl]-5-nitro-pyrimidin-4-yl}-amine; (4-Methanesulfonyl-phenyl)-{6-[4-(3-methyl-[1,2,4]oxadiazol-5-yl)-piperidin-1-yl]-5-nitro-pyrimidin-4-yl}-amine; {6-[4-(3-Methyl-[1,2,4]oxadiazol-5-yl)-piperidin-1-yl]-5-nitro-pyrimidin-4-yl}-(4-[1,2,4]triazol-1-yl-phenyl)-amine; (4-Methanesulfonyl-phenyl)-{5-nitro-6-[4-(pyfidin-2-yisulfanyl)- piperidin-1-yl]-pyrimidin-4-yl}-amine; (3-Methoxy-phenyl)-{5-nitro-6-[4-(pyridin-2-ylsulfanyl)-piperidin-1-yl]-pyrimidin-4-yl}-amine; 1-[6-(Benzo[1,3]dioxol-5-ylamino)-5-nitro-pyrimidin-4-yl]-piperidine-4-carboxylic acid ethyl ester; 1-[6-(2-Fluoro-phenylamino)-5-nitro-pyrimidin-4-yl]-piperidine-4-carboxylic acid ethyl ester; 1-[6-(3-Fluoro-phenylamino)-5-nitro-pyrimidin-4-yl]-piperidine-4-carboxylic acid ethyl ester; 1-[6-(3,4-Dihydro-2H-benzo[b][1,4]dioxepin-7-ylamino)-5-nitro-pyrimidin-4-yl]-piperidine-4-carboxylic acid ethyl ester; 1-{6-[4-(Morpholine-4-sulfonyl)-phenylamino]-5-nitro-pyrimidin-4-yl}-piperidine-4-carboxylic acid ethyl ester; Benzo[1,3]dioxol-5-yl-[5-nitro-6-(4-propyl-piperidin-1-yl)-pyrimidin-4-yl]-amine; (4-Fluoro-phenyl)-{1-[5-nitro-6-(4-[1,2,4]triazol-1-yl-phenylamino)-pyrimidin-4-yl]-piperidin-4-yl}-methanone; [5-Nitro-6-(4-phenylsulfanyl-piperidin-1-yl)-pyrimidin-4-yl]-(4-[1,2,4]triazol-1-yl-phenyl)-amine; (4-Fluoro-phenyl)-{ 1-[6-(2-fluoro-phenylamino)-5-nitro-pyrimidin-4-yl]-piperidin-4-yl}-methanone; (4-Methanesulfonyl-phenyl)-[5-nitro-6-(4-phenylsulfanyl-piperidin-1-yl)-pyrimidin-4-yl]-amine; (4-Methanesulfonyl-phenyl)-{5-nitro-6-[4-(pyridin-2-yloxy)-piperidin-1-yl]-pyrimidin-4-yl}-amine; (4-Methanesulfonyl-phenyl)-{5-nitro-6- [4-(pyridin-4-ylsulfanyl)-piperidin-1-yl]-pyrimidin-4-yl}-amine; (4-Methanesulfonyl-phenyl)- {6-[4-(4-methoxy-phenylsulianyl)-piperidm-1-yl]-5-nitro-pyrimidin-4-yl}-amine; 2-Methoxy-phenyl)-{5-nitro-6-[4-(pyridin-2-ylsulfanyl)-piperidin-1-yl]-pyrimidin-4-yl}-amine; (4-Methanesulfonyl-phenyl)-(5-nitro-6-{4-[3-(3-trifluoromethylphenyl)-[1,2,4]oxadiazol-5-yl]-piperidin-1-yl}-pyrimidin-4-yl)-amine; {6-[4-(3-Ethyl-[1,2,4]oxadiazol-5-yl)-piperidin-1-yl]-5-nitro-pyrimidin-4-yl}-(4-methanesulfonyl-phenyl)-amine; (6-{4-[5-(4-Fluoro-phenyl)-[1,3,4]oxadiazol-2-yl]-piperidin-1-yl}-5-nitro-pyrimidin-4-yl)-(4-methanesulfonyl-phenyl)-amine; (4-Methanesulfonyl-phenyl)-[5-nitro-6-(4-pyridin-2-ylmethyl-piperidin-1-yl)-pyrimidin-4-yl]-amine; 1-{6-[4-(2,5-Dioxo-imidazolidin-4-yl)-phenoxy]-5-nitro-pyrimidin-4-yl}-piperidine-4-carboxylic acid ethyl ester; 1-[5-Nitro-6-(4-propionyl-phenoxy)-pyrimidin-4-yl]-piperidine-4-carboxylic acid ethyl ester; 1-[5-Nitro-6-(4-[1,2,3]thiadiazol-4-yl-phenoxy)-pyrimidin-4-yl]-piperidine-4-carboxylic acid ethyl ester; 1-[6-[4-(3-Oxo-butyl)-phenoxy]-5-(2,2,2-trifluoro-acetylamino)-pyrimidin-4-yl] piperidine-4-carboxylic acid ethyl ester; 1-[6-(2-Benzoyl-5-methoxy-phenoxy)-5-nitro-pyrimidin-4-yl]-piperidine-4-carboxylic acid ethyl ester; 3'-Nitro-4'-[4-(3-oxo-butyl)-phenoxy]-3,4,5,6-tetrahydro-2H-[1,2']bipyridinyl-4-carboxylic acid ethyl ester; 1-[6-(4-Dimethyl sulfamoyl-phenylamino)-5-nitro-pyrimidin-4-yl]-piperidine4-carboxylic acid ethyl ester; 1-{6-[4-(4,5-Dichloro-imidazol-1-yl)-phenylamino]-5-nitro-pyrimidin-4-yl}-piperidine-4-carboxylic acid ethyl ester; Benzo[1,3]dioxol-5-yl-{5-nitro-6-[4-(pyridin-2-ylsulfanyl)-piperidin-1-yl]-pyrimidin-4-yl}-amine; (4-Fluoro-phenyl)-{1-[6-(2-fluoro-phenylamino)-5-nitro-pyrimidin-4-yl]-piperidin-4-yl}-methanone; (2,5-Difluoro-phenyl)-{5-nitro-6-[4-(pyridin-2-ylsulfanyl)-Piperidin-1-yl]-pyriniidin-4-yl}-amine; 1-{5-Nitro-6-[4-(3-oxo-butyl)-phenoxy]-pyrimidin-4-yl}-piperidine-4-carboxylic acid ethyl ester; 4-[4-(3-Isopropyl-[1,2,4]oxadiazol-5-yl)-piperidin-1-yl]-6-(4-methanesulfonyl-phenoxy)-pyrimidine-5-carbonitrile; 5-[1,3]Dioxolan-2-yl-4-[4-(3-isopropyl-[1,2,4]oxadiazol-5-yl)-piperidin-1-yl]-6-(4-methanesulfonyl-phenoxy)- pyrimidine; 4-[4-(3-Isopropyl-[1,2,4]oxadiazol-5-yl)-piperidin-l-yll-6-(4-methanesulfonyl-phenoxy)-pyrinfdine-5-carbaldehyde; 5-[1,3]Dioxolan-2-yl-4-[4-(3-isopropyl-[1,2,4]oxadiazol-5-yl)-piperidin-l-yl]-6-(4-[1,2,3]thiadiazol-4-yl-phenoxy)-pyrimidine; 4-[4-(3-Isopropyl-[1,2,4]oxadiazol-5-yl)-piperidin-1-yl]-6-(4-[1,2,3]thiadiazol-4-yl-phenoxy)- pyrimidine-5-carbaldehyde; 4-[4-(3-Isopropyl-[1,2,4]oxadiazol-5-yl)-piperidin-1-yl]-6-(4-[1,2,3]thiadiazol-4-yl-phenoxy)-pyrimidine-5-carboxylic acid; [4-[4-(3-Isopropyl-[1,2,4]oxadiazol-5-yl)-piperidin-1-yl]-6-(4-[1,2,3]thiadiazol-4-yl-phenoxy)-pyrimidin-5-yl]-methanol; [4-[4-(3-Isopropyl-[1,2,4]oxadiazol-5-yl)-piperidin-1-yl]-6-(4-[1,2,3]thiadiazol-4-yl-phenoxy)-pyrimidin-5-ylmethyl]-dimethyl-amine; 4-[4-(3-Isopropyl-[1,2,4]oxadiazol-5-yl)-piperidin-1-yl]-6-(4-methylsulfanyl-phenylamino)-pyrimidine-5-carbonitrile;4-[4-(3-Isopropyl-[1,2,4]oxadiazol-5-yl)-piperidin-1-yl]-6-(4-methanesulfinyl-phenylamino)-pyrimidine-5-carbonitrile; (4-Methanesuifonyl-phenyl)-{5-nitro-6-[4-(4-trifluoromethoxy-phenoxy)-piperidin-1-yl]-pyrimidin-4-yl}-amine;4-[4-(3-Isopropyl-[1,2,4]oxadiazol-5-yl)-piperidin-1-yl]-6-(4-methanesulfonyl-phenylamino)-pyrimidine-5-carbonitrile; 1-{1-[6-(2-Fluoro-4-methanesulfonyl-phenylamino)-5-nitro- pyrimidin-4-yl]-piperidin-4-yl}-hexan-1-one; 1-{1-[6-(4-Methanesulfonyl-phenylamino)-5-nitro-pyrimidin-4-yl]-piperidin-4-yl}-hexan-1-one; {6-[4-(3-tert-Butyl-[1,2,4]oxadiazol-5-yl)-piperidin-1-yl]-5-nitro-pyrimidin-4-yl}-(2-fluoro-4-methanesulfonyl-phenyl)-amine; {6-[4-(3-tert-Butyl-[1,2,4]oxadiazol-5-yl)-piperidin-1-yl]-5-nitro-pyrimidin-4-yl}-(4-methanesulfonyl-phenyl)-amine; [6-(4-Benzofuran-2-yl-piperidin-1-yl)-5-nitro-pyrimidin-4-yl]-(4-methanesulfonyl-phenyl)-amine and 5-Nitro-4-(5-phenyl-[1,3,4]oxadiazol-2-ylsulfanyl)-6-[4-(pyridin-2-ylsulfanyl)-piperidin-1-yl]-pyrimidine.

Representative examples of compounds of the present invention that are inverse agonists include the following:

1-{6-[4-(2-Carboxy-ethyl)-phenyl]-5-nitro-pyrimidin-4-yl}-piperidine-4-carboxylic acid ethyl ester; 1-{6-[4-(2-Methoxycarbonyl-ethyl)-phenyl]-5-nitro-pyrimidin-4-yl}-piperidine-4-carboxylic acid methyl ester; 1-{6-[4-(2-Methoxycarbonyl-ethyl)-phenyl]-5-nitro-pyrimidin-4-yl}-piperidine-4-carboxylic acid ethyl ester; 1-[5-Nitro-6-(2-trifluoromethyl-phenylethynyl)-pyrimidin-4-yl]-piperidine-4-carboxylic acid ethyl ester; 1-(5-Nitro-6-phenylethynyl-pyrimidin-4-yl)-piperidine-4-carboxylic acid ethyl ester; 1-[5-Nitro-6-(4-trifluoromethyl-phenylethynyl)-pyrimidin-4-yl]-piperidine-4-carboxylic acid ethyl ester; 1-(5-Nitro-6-m-tolylethynyl-pyrimidin-4-yl)-piperidine-4-carboxylic acid ethyl ester; 1-[6-(2-Methyl-5-trifluoromethyl-2H-pyrazol-3-yloxy)-5-nitro-pyrimidin-4-yl]-piperidine-4-carboxylic acid ethyl ester; 1-[6-(Benzo[1,2,5]oxadiazol-5-yloxy)-5-nitro-pyrimidin-4-yl]-piperidine-4-carboxylic acid ethyl ester; 1-[6-(2-Methyl-5-trifluoromethyl-2H-pyrazol-3-yloxy)-5-nitro-pyrimidin-4-yl]piperidine-4-carboxylic acid methyl ester; 2,6-Dimethyl-4-[6-(2-methyl-5-trifluoromethyl-2H-pyrazol-3-yloxy)-5-nitro-pyrimidin-4-yl]-morpholine; 1-{6-[4-(5-Hydroxy-pyrimidin-2-yl)-phenoxy]-5-nitro-pyrimidin4-yl}-pipetidine-4-carboxylic acid ethyl ester; 1-[5-Nitro-6-(4-sulfo-phenoxy)-pyrimidin-4-yl]-piperidine-4-carboxylic acid ethyl ester; 1-[6-(4'-Methoxy-biphenyl-4-yloxy)-5-nitro-pyrinudm-4-yl]-piperidme-4-carboxylic acid ethyl ester; 4-(4,4-Difluoro-piperidin-1-yl)-6-(2-methyl-5-trifluoromethyl-2H-pyrazol-3-yloxy)-5-nitro-pyrimidine; 1-{5-Nitro-6-[4-(4-oxo-cyclohexyl)-phenoxy]-pyrimidin-4-yl}-piperidine-4-carboxylic acid ethyl ester; 1-{6-[4-(2-Hydroxy-ethyl)-phenoxy]-5-nitro-pyrimidin-4-yl}-piperidine-4-carboxylic acid ethyl ester; 1-[6-(2-Methyl-5-trifluoromethyl-2H-pyrazol-3-yloxy)-5-nitro-pyrimidin-4-yl]-piperidine-3-carboxylic acid ethyl ester; 1-[6-(2-Methyl-5-trifluoromethyl-2H-pyrazol-3-yloxy)-5-nitro-pyrimidin-4-yl]-piperidine-4-carboxylic acid ethylamide; 1-[6-(2-Methyl-5-phenyl-2H-pyrazol-3-yloxy)-5-nitro-pyrimidin-4-yl]-piperidine-4-carboxylic acid ethyl ester; 4-(2-Methyl-5-trifluoromethyl-2H-pyrazol-3-yloxy)-5-nitro-6-piperidin-1-yl-pyrimidine; 1-[5-Acetylamino-6-(2-methyl-5-trifluoromethyl-2H-pyrazol-3-yloxy)-pyrimidin-4-yl]-piperidine-4-carboxylic acid ethyl ester; 1-[5-Diacetylamino-6-(2-methyl-5-trifluoromethyl-2H-pyrazol-3-yloxy)-pyrimidin-4-yl]-piperidine-4-carboxylic acid ethyl ester; 1-[6-(2-Methyl-5-trifluoromethyl-2H-pyrazol-3-yloxy)-5-nitro-pyrimidin-4-yl]-piperidine-4-carboxylic acid; 1-{5-Nitro-6-[2-(2-trifluoromethyl-phenyl)-ethoxy]-pyrimidin-4-yl}-piperidine-4-carboxylic acid ethyl ester; 1-{5-Nitro-6-[2-(3-trifluoromethyl-phenyl)-ethoxy]-pyrimidin-4-yl}-piperidine-4-carboxylic acid ethyl ester; 1-[5-Di-(methanesulfonyl)amino-6-(2-methyl-5-trifluoromethyl-2H-pyrazol-3-yloxy)-pyrimidin-4-yl]-piperidine-4-carboxylic acid ethyl ester; 1-[5-Nitro-6-(3-trifluoromethyl-phenoxy)-pyrimidin-4-yl]-piperidine-4-carboxylic acid ethyl ester; 1-[5-Methyl-6-(2-methyl-5-trifluoromethyl-2H-pyrazol-3-yloxy)-pyrimidin-4-yl]-piperidine-4-carboxylic acid ethyl ester; 1-[5 Nitro-6-(2-trifluoromethyl-phenoxy)-pyrimidin-4-yl]-piperidine-4-carboxylic acid ethyl ester; 1-[5-Nitro-6-(4-trifluoromethyl-phenoxy) pyrimidin-4-yl]-piperidine-4-carboxylic acid ethyl ester; 1-[6-(4-Fluoro-phenoxy)-5-nitro-pyrimidin-4-yl]-piperidine-4-carboxylic acid ethyl ester; 1-[6-(2,5-Dimethyl-2H-pyrazol-3-yloxy)-5-nitro-pyrimidin-4-yl]-piperidine-4-carboxylic acid ethyl ester; 1-[5-Nitro-6-(2-oxo-benzo[1,3]oxathiol-6-yloxy)-pyrimidin-4-yl]-piperidine-4-carboxylic acid ethyl ester; 1-[6-(9H-Carbazol-2-yloxy)-5-nitro-pyrimidin-4-yl]-piperidine-4-carboxylic acid ethyl ester; 1-[5-Nitro-6-(9-oxo-9H-fluoren-2-yloxy)-pyrimidin-4-yl]-piperidine-4-carboxylic acid ethyl ester; 1- {5-Amino-6-[4-(3-oxo-butyl)-phenoxy]-pyrimidin-4-yl}-piperidine-4-carboxylic acid ethyl ester; 1-{5-Amino-6-[4-(hydroxy-phenyl-methyl)-phenoxy]-pyrimidin-4-yl}-piperidine-4-carboxylic acid ethyl ester; 1-[6-(6-Chloro-pyridin-3-yloxy)-5-nitro-pyrimidin-4-yl]-piperidine-4-carboxylic acid ethyl ester; 1-[6-(Benzo[1,3]dioxol-5-yloxy)-5-nitro-pyrimidin-4-yl]-piperidine-4-carboxylic acid ethyl ester; 1-[6-(4-Benzyloxy-phenoxy)-5-nitro-pyrimidin-4-yl]-piperidine-4-carboxylic acid ethyl ester; 1-[6-(4-Bromo-phenoxy)-5-nitro-pyrimidin-4-yl]-piperidine-4-carboxylic acid ethyl ester; 1-[6-(4-Chloro-phenoxy)-5-nitro-pyrimidin-4-yl]-piperidine-4-carboxylic acid ethyl ester; 1-[6-(4-Carbamoyl-phenoxy)-5-nitro-pyrimidin-4-yl]-piperidine-4-carboxylic acid ethyl ester; 1-{6-[4-(2-Methoxy-ethyl)-phenoxy]-5-nitro-pyrimidin-4-yl}-piperidine-4-carboxylic acid ethyl ester; 1-[6-(4-Benzoyl-phenoxy)-5-nitro-pyrimidin-4-yl]-piperidine-4-carboxylic acid ethyl ester; 1-{6-[4-(4-Hydroxy-benzenesulfonyl)-phenoxy]-5-nitro-pyrimidin-4-yl}-piperidine-4-carboxylic acid ethyl ester; 1-[5-Amino-6-(2-methyl-5-triflupromethyl-2H-pyrazol-3-yloxy)-pyrimidin-4-yl]-piperidine-4-carboxylic acid ethyl ester; 1-[6-(2-Methyl-5-trifluoromethyl-2H-pyrazol-3-yloxy)-5-(2,2,2-trifluoro-acetylamino)-pyrimidin-4-yl]-piperidine-4-carboxylic acid ethyl ester; Propionic acid 1-[2-amino-5-formyl-6-(2-methyl-5-trifluoromethyl-2H-pyrazol-3-yloxy)-pyrimidin-4-yl]-piperidin-4-yl ester; 4-[6-(2-Methyl-5-trifluoromethyl-2H-pyrazol-3-yloxy)-5-nitro-pyrimidin-4-yl]-piperazine-1-carboxylic acid ethyl ester; 1-[6-(4'-Cyanobiphenyl-4-yloxy)-5-nitro-pyrimidin-4-yl]-piperidine-4-carboxylic acid ethyl ester; {4-[6-(4,4-Difluoro-piperidin-1-yl)-5-nitro-pyrimidin-4-yloxy]-phenyl}-phenyl-methanone; 3-{4-[6-(4,4-Difluoro-piperidin-1-yl)-5-nitro-pyrimidin-4-yloxy]-phenyl}-3-oxo-propionic acid methyl ester; 2-[6-(4,4-Difluoro-piperidin-1-yl)-5-nitro-pyrimidin-4-yloxy]-5-ethanesulfonyl-phenylamine; 4-(4-Cyclopentyl-phenoxy)-6-(4,4-difluoro-piperidin-1-yl)-5-nitro-pyrimidine; 1-[6-(2,6-Dichloro-4-methanesulfonyl-phenoxy)-5-nitro-pyrimidin-4-yl]-piperidine-4-carboxylic acid ethyl ester; 1-{6-[4-(4-Chloro-benzoyl)-phenoxy]-5-nitro-pyrimidin-4-yl}-piperidine-4-carboxylic acid ethyl ester; 1-{6-[4-(4-Hydroxy-benzoyl)-phenoxy]-5-nitro-pyrimidin-4-yl}-piperidine-4-carboxylic acid ethyl ester; 1-[6-(4-Cyanomethyl-phenoxy)-5-nitro-pyrimidin-4-yl]-piperidine-4-carboxylic acid ethyl ester; (4-{6-[4-(2-Methanesulfonyl-ethyl)-piperazin-1-yl]-5-nitro-pyrimidin-4-yloxy}-phenyl)-phenyl-methanone; 4-(4-{6-[4-(2-Methanesulfonyl-ethyl)- piperazin-1-yl]-5-nitro-pyrimidin-4-yloxy}-phenyl)-butan-2-one; 3-(4-{6-[4-(2-Methanesulfonyl-ethyl)-piperazin-1-yl]-5-nitro-pyrimidin-4-yloxy}-phenyl)-3-oxo-propionic acid methyl ester; 4-(4-Methyl-piperidin-1-yl)-6-(2-methyl-5-trifluoromethyl-2H-pyrazol-3-yloxy)-5-nito-pyrimidine; 4-(4-Bromo-piperidin-1-yl)-6-(2-methyl-5-trifluoromethyl-2H-pyrazol-3-yloxy)-5-nitro-pyrimidine; 4-(2-Methyl-5-trifluoromethyl-2H-pyrazol-3-yloxy)-5-nitro-6-(4-propyl-piperidin-1-yl)-pyrimidine; 1-[5-Nitro-6-(2-trifluoromethyl-benzyloxy)-pyrimidin-4-yl]-piperidine-4-carboxylic acid ethyl ester; 1-[5-Nitro-6-(3-trifluoromethyl-benzyloxy)-pyrimidin-4-yl]-piperidine-4-carboxylic acid ethyl ester; 1-[5-Nitro-6-(4-trifluoromethyl-benzyloxy)-pyrimidin-4-yl]-piperidine-4-carboxylic acid ethyl ester; 1-[5-Bromo-6-(2-methyl-5-trifluoromethyl-2H-pyrazol-3-yloxy)-pyrimidin-4-yl]-piperidine-4-carboxylic acid ethyl ester; 1-[6-(2-Methyl-5-trifluoromethyl-2H-pyrazol-3-yloxy)-5-nitro-pyrimidin-4-yl]-piperidine-4-carboxylic acid amide; 1-[5-Nitro-6-(2-oxo-2H-chromen-6-yloxy)-pyrimidin-4-yl]-piperidine-4-carboxylic acid ethyl ester; 1-[6-(3-Morpholin-4-yl-phenoxy)-5-nitro-pyrimidin-4-yl]-piperidine-4-carboxylie acid ethyl ester; 1-[5-Nitro-6-(4-trifluoromethylsulfanyl-phenoxy)-pyrimidin-4-yl]-piperidine-4-carboxylic acid ethyl ester; 1-[5-Nitro-6-(4-trifluoromethoxy-phenoxy)-pyrimidin-4-yl]-piperidine-4-carboxylic acid ethyl ester; 1-[6-(4-Benzoyl-phenoxy)-5-(2,2,2-trifluoro-acetylamino)-pyrimidin-4-yl]-piperidine-4-carboxylic acid ethyl ester; {4-[5-Nitro-6-(4-propyl-piperidin-1-yl)-pyrimidin-4-yloxy]-phenyl}-phenyl-methanone; {4-Methoxy-2-[5-nitro-6-(4-propyl-piperidin-1-yl)-pyrimidin-4-yloxy]-pheny1}-phenyl-methanone; 4-{4-[5-Nitro-6-(4-propyl-piperidin-1-yl)-pyrimidin-4-yloxy]-phenyl}-butan-2-one; 5-Nito-4-(4-propyl-piperidin-1-yl)-6-(4-[1,2,3]thiadiazol-4-yl-phenoxy)-pyrimidine; 3-{4-[5-Nitro-6-(4-propyl-piperidin-1-yl)-pyrimidin-4-yloxy]-phenyl}-3-oxo-propionic acid methyl ester; 5-Ethanesulfonyl-2-[5-nitro-6-(4-propyl-piperidin-1-yl)-pyrimidin-4-yloxy]-phenylamine; Benzo[1,3]dioxol-5-yl-[5-nitro-6-(4-propyl-piperidin-1-yl)-pyrimidin-4-yl]-amine; 1-[6-(4-Difluoromethoxy-benzyloxy)-5-nitro-pyrimidin-4-yl]-pipenidine-4-carboxylic acid ethyl ester; 1-[6-(3-Ethynyl-phenoxy)-5-nitro-pyrimidin-4-yl]-piperidine-4-carboxylic acid ethyl ester; 1-[6-(4-Chloro-2-fluoro-phenoxy)-5-nitro-pyrimidin-4-yl]-piperidine-4-carboxylic acid ethyl ester; 1-[6-(2,4-Difluoro-phenoxy)-5-nitro-pyrimidin-4-yl]-piperidine-4-carboxylic acid ethyl ester; 1-[6-(4-Bromo-2-fluoro-phenoxy)-5-nitro-pyrimidin-4-yl]-piperidine-4-carboxylic acid ethyl ester; 4-(3-Ethynyl-phenoxy)-5-nitro-6-(4-propyl-piperidin-1-yl)-pyrimidine; 4-(4-Chloro-2-fluoro-phenoxy)-5-nitro-6-(4-propyl-piperidin-1-yl)-pyrimidine; 4-(2,4-Difluoro-phenoxy)-5-nitro-6-(4-propyl-piperidin-1-yl)-pyrimidine; 1-[6-(3-Difluoromethoxy-benzyloxy)-5-nitro-pyrimidin-4-yl]-piperidine-4-carboicylic acid ethyl ester; 4-[4-(4-Methyl-benzyl)-piperidin-1-yl]-6-(2-methyl-5-trifluoromethyl-2H-pyrazol-3-yloxy)-5-nitro-pyrimidine; 4-(3-Methanesulfonyl-pyrrolidin-1-yl)-6-(2-methyl-5-trifluoromethyl-2H-pyrazol-3-yloxy)-5-nitro-pyrimidine; 4-(2-Methyl-5-trifluoromethyl-2H-pyrazol-3-yloxy)-5-nitro-6-[4-(2-trifluoromethyl-phenoxy)-piperidin-1-yl]-pyrimidine; 4-(2-Methyl-5-trifluoromethyl-2H-pyrazol-3-yloxy)-5-nitro-6-[4-(pyridin-2-ylsulfanyl)-piperidin-1-yl]-pyrimidine; 1-[6-(4-Cyclopentyl-phenoxy)-5-nitro-pyrimidin-4-yl]-piperidine-4-carboxylic acid ethyl ester; 1-[5-Nitro-6-(4-pyrrol-1-yl-phenoxy)-pyrimidin-4-yl]-piperidine-4-carboxylic acid ethyl ester; 4'-(4-Benzoyl-phenoxy)-3'-nitro-3,4,5,6-tetrahydro-2H-[1,2']bipyridinyl-4-carboxylic acid ethyl ester; 4-(2-Methyl-5-trifluoromethyl-2H-pyrazol-3-yloxy)-5-nitro-6-(4-trifluoromethyl-piperidin-1-yl)-pyrimidine; 4-(2-Methyl-5-trifluoromethyl-2H-pyrazol-3-yloxy)-5-nitro-6-(4-phenylsulfanyl-piperidin-1-yl)-pyrimidine; 4-(4-Bromo-2-fluoro-phenoxy)-5-nitro-6-(4-propyl-piperidin-1-yl)-pyrimidine; 4-[4-(3'-Nitro-4-propyl-3,4,5,6-tetrahydro-2H-[1,4']bipyridinyl-2'-yloxy)-phenyl]-butan-2-one; 2'-(4-Benzoyl-phenoxy)-3'-nitro-3,4,5,6-tetrahydro-2H-[1,4']bipyridinyl-4-carboxylic acid ethyl ester; (5-Nitro-6-piperidin-1-yl-pyriinidin-4-yl)-(3-trifluoromethyl-benzyl)-amine; 1-[5-Nitro-6-(2-trifluoromethylbenzylamino)-pyrimidin-4-yl]-piperidine-4-carboxylic acid ethyl ester; 1-[5-Nitro-6-(4-tifluoromethyl-benzylamino)-pyrimidin-4-yl]-piperidine-4-carboxylic acid ethyl ester; 1-[5-Nitro-6-(3-trifluoromethyl-benzylamino) pyrimidin-4-yl]-piperidine-4-carboxylic acid ethyl ester; (5-Nitro-6-piperidin-1-yl-pyrimidin-4-yl)-(2-trifluoromethyl-benzyl)-amine; [4-(3'-Nitro-4-propyl-3,4,5,6-tetrahydro-2H-[1,4']bipyridinyl-2'-yloxy)-phenyl]-phenyl-methanone; (4-{6-[4-(3-Methyl-[1,2,4]oxadiazol-5-yl)-piperidin-1-yl]-5-nitro-pyrimidin-4-yloxy}-phenyl)-phenyl-methanone; 1-{6-[4-(4-Fluoro-benzoyl)-phenoxy]-5-nitro-pyrimidin-4-yl}-piperidine-4-carboxylic acid ethyl ester; (4-Fluoro-phenyl)-{4-[5-nito-6-(4-propyl-piperidin-1-yl)-pyrimidin-4-yloxy]-phenyl}-methanone; 4-[4-(3-Methyl-[1,2,4]oxadiazol-5-yl)-piperidin-1-yl]-6-(2-methyl-5-trifluoromethyl-2H-pyrazol-3-yloxy)-5-nitro-pyrimidine; 4-(4-Methoxymethyl-piperidin-1-yl)-6-(2-methyl-5-trifluoromethyl-2H-pyrazol-3-yloxy)-5-nitro-pyrimidine; 4-{4-[6-(4-Methoxymethyl-piperidin-1-yl)-5-nitro-pyrimidin-4-yloxy]-phenyl}-butan-2-one; 1-{5-Nitro-6-[3-(1,1,2,2-tetrailuoro-ethoxy)-phenylammo]-pyrimidin-4-yl}-piperidine-4-carboxylic acid ethyl ester; 1-(5 Nitro-6-phenyl-pyrimidin-4-yl)-piperidine-4-carboxylic acid ethyl ester; 1-(6-Naphthalen-2-yl-5-nitro-pyrimidin-4-yl)-piperidine-4-carboxylic acid ethyl ester; 4-[4-(2-Methoxy-ethyl)-piperidin-1-yi]-6-(2-methyl-5-trifluoromethyl-2H-pyrazol-3-yloxy)-5-nitro-pyrimidine; 4-{4-[6-(4-Ethoxymethyl-piperidin-1-yl)-5-nito-pyrimidin-4-yloxy]-phenyl}-butan-2-one; 4-[4-(3'-Nitro-4-propyl-3,4,5,6-tetrahydro-2H-[1,2']bipyridinyl-4'-yloxy)-phenyl]-butan-2-one; 3'-Nitro-4-propyl-(4-[1,2,4]triazol-1-yl-phenoxy)-3,4,5,6-tetrahydro-2H-[1,2']bipyridinyl; 1-[3-(4-Benzoyl-phenoxy)-2-nitro-phenyl]-piperidine-4-carboxylic acid ethyl ester; {4-[6-(4-Ethoxy-piperidin-1-yl)-5-nitro-pyrimidin-4-yloxy]-phenyl}-(4-fluoro-phenyl)-methanone; 1-[6-(2-Methyl-5-trifluoromethyl-2H-pyrazol-3-yloxy)-5-nitro-pyrimidin-4-yl]-piperidin-4-ol; 2-{1-[6-(2-Methyl-5-trifluoromethyl-2H-pyrazol-3-yloxy)-5-nitro-pyrimidin-4-yl]piperidin-4-yl}-ethanol; 3-{1-[6-(2-Methyl-5-trifluoromethyl-2H-pyrazol-3-yloxy)-5-nitro-pyrimidin-4-yl]-piperidin-4-yl}-propionic acid; (1-{6-[4-(4-Fluorobenzoyl)-phenoxy]-5-nitro-pyrimidin-4-yl}-piperidin-4-yl)-(4-fluoro-phenyl)-methanone; 1-[5-Nitro-6-(4-trifluoromethylsulfanyl-phenoxy)-pyrimidin-4-yl]-piperidine-4-carboxylic acid ethyl ester; 1-[5-Nitro-6-(3,4,5-trimethoxy-benzylamino)-pyrimidin-4-yl]-piperidine-4-carboxylic acid ethyl ester; (5-Nitro-6-piperidin-1-yl-pyrimidin-4-yl)-(4-trifluoromethylbenzyl)-amine; 1-[5-Amino-6-(3-trifluoromethyl-benzylamino)-pyrimidin-4-yl]-piperidine-4-carboxylic acid ethyl ester; 1-[5-Amino-6-(4-trifluoromethyl-benzylamino)-pyrimidin-4-yl]-piperidine-4-carboxylic acid ethyl ester; 1-[6-(4-Bromo-phenylamino)-5-nitro-pyrimidin-4-yl]-piperidine-4-carboxylic acid ethyl ester; 1-[5-Nitro-6-(4-trifluoromethyl-phenylamino)-pyrimidin-4-yl]-piperidine-4-carboxylic acid ethyl ester; 1-[6-(Methyl-phenyl-amino)-5-nitro-pyrimidin-4-yl]-piperidine-4-carboxylic acid ethyl ester; 1-[5-Nitro-6-(4-trifluoromethoxy-phenylamino)-pyrimidin-4-yl]-piperidine-4-carboxylic acid ethyl ester; 1-[6-(4-Fluoro-phenylamino)-5-nito-pyrimidin-4-yl]-piperidine-4-carboxylic acid ethyl ester; 1-{6-[(2,3-Dihydro-benzo[1,4]dioxin-6-ylmethyl)-amino]-5-nitro-pyrimidin-4-yl}-piperidine-4-carboxylic acid ethyl ester; 1-{6-[(2,3-Dihydro-benzo[1,4]dioxin-2-ylmethyl)-amino]-5-nitro-pyrimidin-4-yl}-piperidine-4-carboxylic acid ethyl ester; 1-{6-[(2,3-Dihydro-benzofuran-5-ylmethyl)-amino]-5-nitro-pyrimidin-4-yl}-piperidine-4-carboxylic acid ethyl ester; 1-{6-[(6-Fluoro-4H-benzo[1,3]dioxin-8-ylmethyl)-amino]-5-nitro-pyrimidin-4-yl}-piperidine-4-carboxylic acid ethyl ester; 1-[6-(2,2-Difluoro-benzo[1,3]dioxol-4-ylamino)-5-nitro-pyrimidin-4-yl] piperidine-4-carboxylic acid ethyl ester; 1-[6-(2,2-Difluoro-benzo[1,3]dioxol-5-ylamino)-5-nitro pyrimidin-4-yl]-piperidine-4-carboxylic acid ethyl ester; 1-[6-(1,1-Dioxo-1H-1$\lambda^6$-benzo[b]thiophen-6-ylamino)-5-nitro-pyrimidin-4-yl]-piperidine-4-carboxylic acid ethyl ester; 1-{6-[(Furan-3-ylmethyl)-amino]-5-nitro-pyrimidin-4-yl}-piperidine-4-carboxylic acid ethyl ester; 1-{6-[2-(4-Methoxy-phenoxy)-ethylamino]-5-nitro-pyrimidin-4-yl}-piperidine-4-carboxylic acid ethyl ester; 1-{6-[2-(5-Methoxy-1H-indol-3-yl)-ethylamino]-5-nitro-pyrimidin-4-yl}-piperidine-4-carboxylic acid ethyl ester; (3,4-Dihydro-2H-benzo[b][1,4]dioxepin-7-yl)-[5-nitro-6-(4-propyl-piperidin-1-yl)-pyrimidin-4-yl]-amine; (3-Fluoro-phenyl)-[5-nitro-6-(4-propyl-piperidin-1-yl)-pyrimidin-4-yl]-amine; (3-Methoxyphenyl)-[5-nitro-6-(4-propyl-piperidin-1-yl)-pyrimidin-4-yl]-amine; 1-{6-[(3-Fluoro-phenyl)-methyl-amino]-5-nitro-pyrimidin-4-yl}-piperidine-4-carboxylic acid ethyl ester; 1-[6-(4-Benzoyl-phenylamino)-5-nitro-pyrimidin-4-yl]-piperidine-4-carboxylic acid ethyl ester; 1-[6-(3,5-Bis-trifluoromethyl-phenylamino)-5-nitro-pyrimidin-4-yl]-piperidine-4-carboxylic acid ethyl ester; 1-[6-(3,5-Dimethoxy-benzylamino)-5-nitro-pyrimidin-4-yl]-piperidine-4-carboxylic acid ethyl ester; 1-[6-(4-Heptyl-phenylamino)-5-nitro-pyrimidin-4-yl]-piperidine-4-carboxylic acid ethyl ester; 2'-(4-Benzoyl-phenylamino)-3'-nitro-3,4,5,6-tetrahydro-2H-[1,4']bipyridinyl-4-carboxylic acid ethyl ester; 1-[5-Nitro-6-(3,4,5-trimethoxy-phenylamino)-pyrimidin-4-yl]-piperidine-4-carboxylic acid ethyl ester; 1-[5-Nitro-6-(4-pentyl-phenylamino)-pyrin-jidin-4-yl]-piperidine-4-carboxylic acid ethyl ester; 1-{6-[4-(3-Carboxypropyl)-phenylamino]-5-nitro-pyrimidin-4-yl}-piperidine-4-carboxylic acid ethyl ester; [5-Nitro-6-(4-propyl piperidin-1-yl)-pyrimidin-4-yl]-(3,4,5-trimethoxy-benzyl)-amine; (3,5-Dimethoxy-benzyl)-[5-nitro-6-(4-propyl-piperidin-1-yl)-pyrimidin-4-yl]-amine; (4-{5-Nitro-6-[4-(2-trifluoromethyl-phenoxy)-piperidin-1-yl]-pyrimidin-4-ylamino}-phenyl)-phenyl-methanone; 1-[6-(3,5-Difluoro-phenylamino)-5-nitro-pyrimidm-4-yl]-pipendine-4-carboxylic acid ethyl ester; 1-[6-(3,5-Dichloro phenylamino)-5-nitro-pyrimidin-4-yl]-piperidine-4-carboxylic acid ethyl ester; 1-[6-(2-Bromo-4-trifluoromethoxy phenylamino)-5-nitro-pyrimidin-4-yl]-piperidine-4-carboxylic acid ethyl ester; 1-{6-[(2-Fluoro-phenyl)-methylamino]-5-nitro-pyrimidin-4-yl}-piperidine-4-carboxylic acid ethyl ester; 1-[6-(Ethyl-phenyl-amino)-5-nitro-pyrimidin-4-yl]-piperidine-4-carboxylic acid ethyl ester; 1-{6-[(4-Chlorophenyl)-methyl-amino]-5-nito-pyrimidin-4-yl}-piperidine-4-carboxylic acid ethyl ester; 1-[6-(4-Difluoromethyl-benzylamino)-5-nitro-pyrirnidin-4-yl]-piperidine-4-carboxylic acid ethyl ester; 1-[6-(4-Cyano-phenylamino)-5-nitro-pyrimidin-4-yl]-piperidine-4-carboxylic acid ethyl ester; 1-[6-(3,5-Dimethoxy-phenylamino)-5-nitro-pyrimidin-4-yl]-piperidine-4-carboxylic acid ethyl ester; 1-[6-(4-sec-Butyl-phenylamino)-5-nitro-pyrimidin-4-yl]-piperidine-4-carboxylic acid ethyl ester; 1-{6-[4-(Cyano-phenyl-methyl)-phenylamino]-5-nitro-pyrimidin-4-yl}-piperidine-4-carboxylic acid ethyl ester; 1-{5-Nitro-6-[4-(4-trifluoromethyl-phenoxy)-phenylamino]-pyrimidin-4-yl}-piperidine-4-carboxylic acid ethyl ester; 1-[6-(2-Methyl-5-phenyl-2H-pyrazol-3-ylamino)-5-nitro-pyrimidin-4-yl]-piperidine-4-carboxylic acid ethyl ester; 1-[6-(4-Benzenesulfonyl-phenylamino)-5-nitro-pyrimidin-4-yl]-piperidine-4-carboxylic acid ethyl ester; 1-[5-Nitro-6-(2-trifluoromethyl-3H-benzoimidazol-5-ylamino)-pyrimidin-4-yl]-piperidine-4-carboxylic acid ethyl ester, 1-[6-(4-Methanesulfonyl-phenyl)-5-nitro-pyrimidin-4-yl]-piperidine-4-carboxylic acid ethyl ester; 1-(6-Benzofuran-5-yl-5-nitro-pyrimidin-4-yl)-piperidine-4-carboxylic acid ethyl ester; 1-[5-Nitro-6-(3-trifluoromethylphenyl)-pyrimidin-4-yl]-piperidine-4-carboxylic acid ethyl ester; 1-[6-(4-Methoxy-phenyl)-5-nitro-pyrimidin-4-yl]-piperidine-4-carboxylic acid ethyl ester; 4-(4-Butyl-piperidin-1-yl)-6-furan-3-yl-5-nitro-pyrimidine; 1-[6-(3-Chloro-phenyl)-5-nitro-pyrimidin-4-yl]-piperidine-4-carboxylic acid ethyl ester; 1-[6-(2,6-Dimethoxy-phenyl)-5-nitro-pyrimidin-4-yl]-piperidine-4-carboxylic acid ethyl ester; 1-(6-Naphthalen-1-yl-5-nitro-pyrimidin-4-yl)-piperidine-4-carboxylic acid ethyl ester; 1-[6-(4-Methylsulfanyl-phenyl)-5-nitro-pyrimidin-4-yl]-piperidine-4-carboxylic acid ethyl ester; 1-(2',4'-Dihydroxy-5-nitro-[4,5']bipyrimidinyl-6-yl)-piperidine-4-carboxylic acid ethyl ester; 1-[6-(4-Methanesulfonyl-phenyl)-5-nitro-pyrimidin-4-yl]-piperidine-4-carboxylic acid ethyl ester; 1-[6-(3,5-Bis-trifluoromethyl-phenyl)-5-nitro-pyrimidin-4-yl]-piperidine-4-carboxylic acid ethyl ester; 1-(6-Dibenzothiophen-4-yl-5-nitro-pyrimidin-4-yl)-piperidine-4-carboxylic acid ethyl ester; 4-(2,4-Difluoro-phenoxy)-6-(4-ethoxymethyl-piperidin-1-yl)-5-nitro-pyrimidine; 1-{4-[6-(4-Methoxymethyl-piperidin-1-yl)-5-nitro-pyrimidin-4-yloxy]-phenyl}-ethanone; 4-{4-[2-Nitro-3-(4-propyl-piperidin-1-yl)-phenoxy]-phenyl}-butan-2-one; 1-{4-[2-Nitro-3-(4-propyl-piperidin-1-yl)-phenoxy]-phenyl}-ethanone; {4-[2-Nitro-3-(4-propyl-piperidin-1-yl)-phenoxy)-phenyl}-phenyl-methanone; 3-{4-[2-Nitro-3-(4-propyl-piperidin-1-yl)-phenoxy]-phenyl}-3-oxo-propionic acid methyl ester; (4-Fluoro-phenyl)-[4-(3'-nitro-4-propyl-3,4,5,6-tetrahydro-2H-[1,2]bipyridinyl-4'-yloxy)-phenyl]-methanone; 1-[6-(3,5-Dimethyl-isoxazol-4-yl)-5-nitro-pyrimidin-4-yl]-piperidine-4-carboxylic acid ethyl ester; 1-(5-Nitro-6-thiophen-2-yl-pyrimidin-4-yl)-piperidine-4-carboxylic acid ethyl ester; 1-[6-(3,5-Dichloro-phenyl)-5-nitro-pyrimidin-4-yl]-piperidine-4-carboxylic acid ethyl ester; 1-(6-Dibenzofuran-4-yl-5-nitro-pyrimidin-4-yl)-piperidine-4-carboxylic acid ethyl ester; 1-[6-(3,5-Dimethyl-phenyl)-5-nitro-pyrimidin-4-yl]-piperidine-4-carboxylic acid ethyl ester; 1-[6-(4-Acetyl-phenyl)-5-nitro-pyrimidin-4-yl]-piperidine-4-carboxylic acid ethyl ester; 1-[6-(4-Ethanesulfonyl-phenyl)-5 nitro-pyrimidin-4-yl]-piperidine-4-carboxylic acid ethyl ester; 1-[6-(2-Fluoro-biphenyl-4-yl)-5-nitro-pyrimidin-4-yl]-piperidine-4-carboxylic acid ethyl ester; 1-[6-(3-Methanesulfonyl-phenyl)-5-nitro-pyrimidin-4-yl]-piperidine-4-carboxylic acid ethyl ester; 1-[6-(2-Fluoro-phenylethynyl)-5-nitro-pyrimidin-4-yl]-piperidine-4-carboxylic acid ethyl ester and 1-[5-Nitro-6-(3-trifluoromethyl-phenylethynyl)-pyrimidin-4-yl]-piperidine-4-carboxylic acid ethyl ester.

## Pharmaceutical compositions

Some embodiments of the present invention include a method of producing a pharmaceutical composition comprising admixing at least one compound according to any of the compound embodiments disclosed herein and a pharmaceutically acceptable carrier.

A compound of the present invention can be formulated into pharmaceutical compositions using techniques well known to those in the art. Suitable pharmaceutically-acceptable carriers, outside those mentioned herein, are available to those in the art; for example, see Remington's Pharmaceutical Sciences, 16th Edition, 1980, Mack Publishing Co., (Oslo et al., eds.) and the most current version.

While it is possible that, for use in the prophylaxis or treatment, a compound of the invention may in an alternative use be administered as a raw or pure chemical, it is preferable however to present the compound or active ingredient as a pharmaceutical formulation or composition further comprising a pharmaceutically acceptable carrier.

The invention thus further provides pharmaceutical formulations comprising a compound of the invention or a pharmaceutically acceptable salt or derivative thereof together with one or more pharmaceutically acceptable carriers therefor and/or prophylactic ingredients. The carrier(s) must be "acceptable" in the sense of being compatible with the other ingredients of the formulation and not overly deleterious to the recipient thereof.

Pharmaceutical formulations include those suitable for oral, rectal, nasal, topical (including buccal and sub-lingual), vaginal or parenteral (including intramuscular, subcutaneous and intravenous) administration or in a form suitable for administration by inhalation or insufflation.

The compounds of the invention, together with a conventional adjuvant, carrier, or diluent, may thus be placed into the form of pharmaceutical formulations and unit dosages thereof, and in such form may be employed as solids, such as tablets or filled capsules, or liquids such as solutions, suspensions, emulsions, elixirs, gels or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

For oral administration, the pharmaceutical composition may be in the form of, for example, a tablet, capsule, suspension or liquid. The pharmaceutical composition is preferably made in the form of a dosage unit containing a

particular amount of the active ingredient. Examples of such dosage units are capsules, tablets, powders, granules or a suspension, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethylcellulose; and with lubricants such as talc or magnesium stearate. The active ingredient may also be administered by injection as a composition wherein, for example, saline, dextrose or water may be used as a suitable pharmaceutically acceptable carrier.

The dose when using the compounds of Formula **(Ia)** can vary within wide limits, and as is customary and is known to the physician, it is to be tailored to the individual conditions in each individual case. It depends, for example, on the nature and severity of the illness to be treated, on the condition of the patient, on the compound employed or on whether an acute or chronic disease state is treated or prophylaxis is conducted or on whether further active compounds are administered in addition to the compounds of the Formula **(Ia).** Representative doses of the present invention include, about 0.01 mg to about 1000 mg, about 0.01 to about 750 mg, about 0.01 to about 500 mg, 0.01 to about 250 mg, 0.01 mg to about 200 mg, about 0.01 mg to 150 mg, about 0.01 mg to about 100 mg, and about 0.01 mg to about 75 mg. Multiple doses may be administered during the day, especially when relatively large amounts are deemed to be needed, for example 2, 3 or 4, doses. If appropriate, depending on individual behavior and as appropriate from the patients physician or care-giver it may be necessary to deviate upward or downward from the daily dose.

The amount of active ingredient, or an active salt or derivative thereof, required for use in treatment will vary not only with the particular salt selected but also with the route of administration, the nature of the condition being treated and the age and condition of the patient and will ultimately be at the discretion of the attendant physician or clinician. In general, one skilled in the art understands how to extrapolate in vivo data obtained in a model system, typically an animal model, to another, such as a human. Typically, animal models include, but are not limited to, the rodents diabetes models as described in Example 6, *infra* (other animal models have been reported by Reed and Scribner in Diabetes, Obesity and Metabolism, 1, 1999, 75-86). In some circumstances, these extrapolations may merely be based on the weight of the animal model in comparison to another, such as a mammal, preferably a human, however, more often, these extrapolations are not simply based on weights, but rather incorporate a variety of factors. Representative factors include the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized, on whether an acute or chronic disease state is being treated or prophylaxis is conducted or on whether further active compounds are administered in addition to the compounds of the Formula **(Ia)** and as part of a drug combination. The dosage regimen for treating a disease condition with the compounds and/or compositions of this invention is selected in accordance with a variety factors as cited above. Thus, the actual dosage regimen employed may vary widely and therefore may deviate from a preferred dosage regimen and one skilled in the art will recognize that dosage and dosage regimen outside these typical ranges can be tested and, where appropriate, may be used in the methods of this invention.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations. The daily dose can be divided, especially when relatively large amounts are administered as deemed appropriate, into several, for example 2, 3 or 4, part administrations. If appropriate, depending on individual behavior, it may be necessary to deviate upward or downward from the daily dose indicated.

The compounds of the present invention can be administrated in a wide variety of oral and parenteral dosage forms. It will be obvious to those skilled in the art that the following dosage forms may comprise, as the active component, either a compound of the invention or a pharmaceutically acceptable salt of a compound of the invention.

For preparing pharmaceutical compositions from the compounds of the present invention, the selection of a suitable pharmaceutically acceptable carrier can be either solid, liquid or a mixture of both. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding capacity in suitable proportions and compacted to the desire shape and size.

The powders and tablets may contain varying percentage amounts of the active compound. A representative amount in a powder or tablet may contain from 0.5 to about 90 percent of the active compound; however, an artisan would know when amounts outside of this range are necessary. Suitable carriers for powders and tablets are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and

lozenges are included. Tablets, powders, capsules, pills, cachets, and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

For topical administration to the epidermis the compounds according to the invention may be formulated as ointments, creams or lotions, or as a transdermal patch.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multi-dose form. In the latter case of a dropper or pipette, this may be achieved by the patient administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided in a pressurized pack with a suitable propellant. If the compounds of the Formula **(Ia)** or pharmaceutical compositions comprising them are administered as aerosols, for example as nasal aerosols or by inhalation, this can be carried out, for example, using a spray, a nebulizer, a pump nebulizer, an inhalation apparatus, a metered inhaler or a dry powder inhaler. Pharmaceutical forms for administration of the compounds of the Formula **(Ia)** as an aerosol can be prepared by processes well-known to the person skilled in the art. For their preparation, for example, solutions or dispersions of the compounds of the Formula **(Ia)** in water, water/alcohol mixtures or suitable saline solutions can be employed using customary additives, for example benzyl alcohol or other suitable preservatives, absorption enhancers for increasing the bioavailability, solubilizers, dispersants and others, and, if appropriate, customary propellants, for example include carbon dioxide, CFC's, such as, dichlorodifluoromethane, trichlorofluoromethane, or dichlorotetrafluoroethane; and the like. The aerosol may conveniently also contain a surfactant such as lecithin. The dose of drug may be controlled by provision of a metered valve.

In formulations intended for administration to the respiratory tract, including intranasal formulations, the compound will generally have a small particle size for example of the order of 10 microns or less. Such a particle size may be obtained by means known in the art, for example by micronization. When desired, formulations adapted to give sustained release of the active ingredient may be employed.

Alternatively the active ingredients may be provided in the form of a dry powder, for example, a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

The term "prodrug" refers to compounds that are rapidly transformed in vivo to yield the parent compound of the above formulae, for example, by hydrolysis in blood. A thorough discussion is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987, both of which are hereby incorporated by reference.

**Combination Therapy/Prophylaxis**

While the compounds of the invention can be administered as the sole active pharmaceutical agent as described herein above, they can also be used in combination with one or more agents belonging to the class of drugs known as α-glucosidase inhibitors, aldose reductase inhibitors, biguanides, HMG-CoA reductase inhibitors, squalene synthesis inhibitors, fibrate compounds, LDL catabolism enhancers and angiotensin converting enzyme (ACE) inhibitors.

α-Glucosidase inhibitors belong to the class of drugs which competitively inhibit digestive enzymes such as α-amylase, maltase, α-dextrinase, sucrase, etc. in the pancreas and or small intesting. The reversible inhibition by α-glucosidase inhibitors retard, diminish or otherwise reduce blood glucose levels by delaying the digestion of starch and sugars. Some representative examples of α-glucosidase inhibitors include acarbose, N-(1,3-dihydroxy-2-propyl)valiolamine (generic name; voglibose), miglitol, and α-glucosidase inhibitors known in the art.

The class of aldose reductase inhibitors are drugs which inhibit the first-stage rate-limiting enzyme in the polyol pathway that prevent or arrest diabetic complications. In the hyperglycemic state of diabetes, the utilization of glucose in the polyol pathway is increased and the excess sorbitol accumulated intracellularly as a consequence acts as a tissue toxin and hence evokes the onset of complications such as diabetic neuropathy, retinopathy, and nephropathy. Examples of the aldose reductase inhibitors include tolurestat; epalrestat; 3,4-dihydro-2,8-diisopropyl-3-thioxo-2*H*-1,4-benzoxazine-4-acetic acid; 2,7-difluorospiro(9*H*-fluorene-9,4'-imidazolidine)-2',5'-dione (generic name: imirestat); 3-[(4-bromo-2-flurophenyl)methy]-7-chloro-3,4-dihydro-2,4-dioxo-1(2*H*)-quinazoline acetic acid (generic name: zenarestat); 6-fluoro-2,3-dihydro-2',5'-dioxo-spiro[4*H*-1-benzopyran-4,4'-imidazolidine]-2-carboxamide (SNK-860); zopolrestat; sorbinil; and 1-[(3-bromo-2-benzofuranyl)sulfonyl]-2,4-imidazolidinedione (M-16209), and aldose reductase inhibitors known in the art.

The biguanides are a class of drugs that stimulate anaerobic glycolysis, increase the sensitivity to insulin in the peripheral tissues, inhibit glucose absorption from the intestine, suppress of hepatic gluconeogenesis, and inhibit fatty acid oxidation. Examples of biguanides include phenformin, metformin, buformin, and biguanides known in the art.

Statin compounds belong to a class of drugs that lower blood cholesterol levels by inhibiting hydroxymethylglutalyl CoA (HMG-CoA) reductase. HMG-CoA reductase is the rate-limiting enzyme in cholesterol biosynthesis. A statin that inhibits this reductase lowers serum LDL concentrations by upregulating the activity of LDL receptors and responsible for clearing LDL from the blood. Examples of the statin compounds include rosuvastatin, pravastatin and its sodium salt, simvastatin, lovastatin, atorvastatin, fluvastatin, cerivastatin, and HMG-CoA reductase inhibitors known in the art.

Squalene synthesis inhibitors belong to a class of drugs that lower blood cholesterol levels by inhibiting synthesis of squalene. Examples of the squalene synthesis inhibitors include (S)-α-[Bis[2,2-dimethyl-1-oxopropoxy)methoxy] phosphinyl]-3-phenoxybenzenebutanesulfonic acid, mono potassium salt (BMS-188494) and squalene synthesis inhibitors known in the art.

Fibrate compounds belong to a class of drugs that lower blood cholesterol levels by inhibiting synthesis and secretion of triglycerides in the liver and activating a lipoprotein lipase. Fibrates have been known to activate peroxisome proliferators-activated receptors and induce lipoprotein lipase expression. Examples of fibrate compounds include bezafibrate, beclobrate, binifibrate, ciplofibrate, clinofibrate, clofibrate, clofibric acid, etofibrate, fenofibrate, gemfibrozil,

nicofibrate, pirifibrate, ronifibrate, simfibrate, theofibrate, and fibrates known in the art.

LDL (low-density lipoprotein) catabolism enhancers belong to a class of drugs that lower blood cholesterol levels by increasing the number of LDL (low-density lipoprotein) receptors, examples include LDL catabolism enhancers known in the art.

Angiotensin converting enzyme (ACE) inhibitors belong to the class of drugs that partially lower blood glucose levels as well as lowering blood pressure by inhibiting angiotensin converting enzymes. Examples of the angiotensin converting enzyme inhibitors include captopril, enalapril, alacepril, delapril; ramipril, lisinopril, imidapril, benazepril, ceronapril, cilazapril, enalaprilat, fosinopril, moveltopril, perindopril, quinapril, spirapril, temocapril, trandolapril, and angiotensin converting enzyme inhibitors known in the art.

Insulin secretion enhancers belong to the class of drugs having the property to promote secretion of insulin from pancreatic β cells. Examples of the insulin secretion enhancers include sulfonylureas (SU). The sulfonylureas (SU) are drugs which promote secretion of insulin from pancreatic β cells by transmitting signals of insulin secretion via SU receptors in the cell membranes. Examples of the sulfonylureas include tolbutamide; chlorpropamide; tolazamide; acetohexamide; 4-chloro-N-[(1-pyrolidinylamino) carbonyl]-benzenesulfonamide (generic name: glycopyramide) or its ammonium salt; glibenclamide (glyburide); gliclazide; 1-butyl-3-metanilylurea; carbutamide; glibonuride; glipizide; gliquidone; glisoxepid; glybuthiazole; glibuzole; glyhexamide; glymidine; glypinamide; phenbutamide; tolcyclamide, glimepiride, and other insulin secretion enhancers known in the art. Other insulin secretion enhancers include N-[[4-(1-methylethyl)cyclohexyl)carbonyl]-D-phenylalanine (Nateglinide); calcium (2S)-2-benzyl-3-(cis-hexahydro-2-isoindolinylcarbonyl)propionate dihydrate (Mitiglinide, KAD-1229); and other insulin secretion enhancers known in the art.

Thiazolidinediones belong to the class of drugs more commoningly known as TZDs. Examples of thiazolidinediones include rosiglitazone, pioglitazone, and thiazolidinediones known in the art.

Some embodiments of the invention include, a pharmaceutical composition comprising a compound of Formula **(Ia)** or a pharmaceutically acceptable salt thereof in combination with at least one member selected from the group consisting of an α-glucosidase inhibitor, an aldose reductase inhibitor, a biguanide, a HMG-CoA reductase inhibitor, a squalene synthesis inhibitor, a fibrate compound, a LDL catabolism enhancer and an angiotensin converting enzyme inhibitor. In another embodiment, the pharmaceutical composition is a compound of Formula **(Ia)** or a pharmaceutically acceptable salt thereof in combination with a HMG-CoA reductase inhibitor. In still another embodiment, the HMG-CoA reductase inhibitor is selected from the group consisting of prevastatin, simvastatin, lovastatin, atorvastatin, fluvastatin and lipitor.

In accordance with the present invention, the combination can be used by mixing the respective active components either all together or independently with a physiologically acceptable carrier, excipient, binder, diluent, etc., as described herein above, and administering the mixture or mixtures either orally or non-orally as a pharmaceutical composition. When a compound or a mixture of compounds of Formula **(Ia)** are administered as a combination therapy or prophylaxis with another active compound the therapeutic agents can be formulated as a separate pharmaceutical compositions given at the same time or at different times, or the therapeutic agents can be given as a single composition.

***Other Utility***

Another object of the present invention relates to radiolabelled compounds of Formula **(Ia)** that would be useful not only in radio-imaging but also in assays, both in vitro and in vivo, for localizing and quantitating **RUP3** in tissue samples, including human, and for identifying **RUP3** ligands by inhibition binding of a radiolabelled compound. It is a further object of this invention to develop novel **RUP3** assays of which comprise such radiolabelled compounds.

Suitable radionuclides that may be incorporated in compounds of the present invention include but are not limited to $^{3}H$ (also written as T), $^{11}C$, $^{14}C$, $^{18}F$, $^{125}I$, $^{82}Br$, $^{123}I$, $^{124}I$, $^{125}I$, $^{131}I$, $^{75}Br$, $^{76}Br$, $^{15}O$, $^{13}N$, $^{35}S$ and $^{77}Br$. The radionuclide that is incorporated in the instant radiolabelled compounds will depend on the specific application of that radiolabelled compound. Thus, for in vitro RUP3 labeling and competition assays, compounds that incorporate $^{3}H$, $^{14}C$, $^{125}I$, $^{131}I$, $^{35}S$ or $^{82}Br$ will generally be most useful. For radio-imaging applications $^{11}C$, $^{18}F$, $^{125}I$, $^{123}I$, $^{124}I$ $^{131}I$ $^{75}Br$ , $^{76}Br$ or $^{77}Br$ will generally be most useful.

It is understood that a "radio-labelled " or "labelled compound" is a compound of Formula **(Ia)** that has incorporated at least one radionuclide; In some embodiments, the radionuclide is selected from the group consisting of $^{3}H$, $^{14}C$, $^{125}I$ , $^{35}S$ and $^{82}Br$; In some embodiments, the radionuclide $^{3}H$ or $^{14}C$. Moreover, it should be understood that all of the atoms represented in the compounds of the invention can be either the most commonly occurring isotope of such atoms or the more scarce radio-isotope or nonradio-active isotope.

Synthetic methods for incorporating radio-isotopes into organic compounds including those applicable to those compounds of the invention are well known in the art and include incorporating activity levels of tritium into target molecules include: **A.** Catalytic Reduction with Tritium Gas - This procedure normally yields high specific activity products and requires halogenated or unsaturated precursors. **B**. Reduction with Sodium Borohydride [$^{3}H$] - This procedure is rather inexpensive and requires precursors containing reducible functional groups such as aldehydes, ketones, lactones,

esters, and the like. **C**. Reduction with Lithium Aluminum Hydride [$^3$H ] - This procedure offers products at almost theoretical specific activities. It also requires precursors containing reducible functional groups such as aldehydes, ketones, lactones, esters, and the like. **D**. Tritium Gas Exposure Labeling - This procedure involves exposing precursors containing exchangeable protons to tritium gas in the presence of a suitable catalyst. **E**. N-Methylation using Methyl Iodide [$^3$H]- This procedure is usually employed to prepare O-methyl or N-methyl ($^3$H) products by treating appropriate precursors with high specific activity methyl iodide ($^3$H). This method in general allows for high specific activity, such as about 80-87 Ci/mmol.

Synthetic methods for incorporating activity levels of $^{125}$I into target molecules include: **A.** Sandmeyer and like reactions - This procedure transforms an aryl or heteroaryl amine into a diazonium salt, such as a tetrafluoroborate salt, and subsequently to $^{125}$I labelled compound using Na$^{125}$I. A represented procedure was reported by Zhu, D.-G. and co-workers in J. Org. Chem. 2002, 67, 943-948. **B.** Ortho $^{125}$Iodination of phenols - This procedure allows for the incorporation of $^{125}$I at the ortho position of a phenol as reported by Collier, T. L. and co-workers in J. Labelled Compd Radiopharm. 1999, 42, S264-S266. **C.** Aryl and heteroaryl bromide exchange with $^{125}$I- This method is generally a two step process. The first step is the conversion of the aryl or heteroaryl bromide to the corresponding tri-alkyltin intermediate using for example, a Pd catalyzed reaction [i.e. $Pd(Ph_3P)_4$] or through an aryl or heteroaryl lithium, in the presence of a tri-alkyltinhalide or hexaalkylditin [e.g., $(CH_3)_3SnSn(CH_3)_3$]. A represented procedure was reported by Bas, M.-D. and co-workers in J. Labelled Compd Radiopharm. 2001, 44, S280-S282.

A radiolabelled **RUP3** compound as described herein can be used in a screening assay to identify/evaluate compounds. In general terms, a newly synthesized or identified compound (i.e., test compound) can be evaluated for its ability to reduce binding of the "radiolabelled compound of Formula **(Ia)"** to the **RUP3** receptor. Accordingly, the ability of a test compound to compete with the "radio-labelled compound of Formula **(Ia)"** for the binding to the **RUP3** receptor directly correlates to its binding affinity.

The labelled compounds of the present invention bind to the RUP3 receptor. In one embodiment the labelled compound has an $IC_{50}$ less than about 500 μM, in another embodiment the labelled compound has an $IC_{50}$ less than about 100 μM, in yet another embodiment the labelled compound has an $IC_{50}$ less than about 10 μM, in yet another embodiment the labelled compound has an $IC_{50}$ less than about 1 μM, and in still yet another embodiment the labelled inhibitor has an $IC_{50}$ less than about 0.1 μM.

Other uses of the disclosed receptors and methods will become apparent to those in the art based upon, inter alia, a review of this patent document.

This application claims priority benefit of U.S. Provisional Application Number 60/440,394, filed January 14, 2003; U.S. Provisional Patent Application Number 60/449,829, filed February 24, 2003, U.S. Provisional Patent Application Number 60/453,390, filed March 6, 2003, and U.S. Non-Provisional Patent Application No. 60/470,875, filed May 14, 2003; wherein each are hereby incorporated by reference in their entirety.

The following examples are given to illustrate the invention and are not intended to be inclusive in any manner:

## EXAMPLES

The compounds of the present invention and their syntheses are further illustrated by the following examples. The examples are provided to further define the invention without, however, limiting the invention to the specifics of these examples.

### Example 1

### 96-well Cyclic AMP membrane assay for RUP3

Materials:

1) Adenlyl cyclase Activation Flashplate Assay kit from Perkin Elmer -- 96 wells (SMP004B) and $^{125}$I tracer (NEX130) which comes with the kit. Keep in refrigerator, in a box, and do not expose the Flashplates to light.
2) Phosphocreatine-Sigma P-7936
3) Creatine Phosphokinase --- Sigma C-3755
4) GTP-Sigma G-8877
5) ATP- Sigma A-2383
6) IBMX-Sigma 1-7018
7) Hepes-1M solution in distilled water- Gibco #15630080
8) MgC12-Sigma M-1028-1M Solution

9) NaCl - Sigma - S6546 - 5M Solution
10) Bradford Protein Assay Kit - Biorad # 5000001
11) Proclin 300- Sigma #4-8126

Binding Buffer - filter through 45- micron Nalgene filter and keep in refrigerator. All buffers and membranes should be kept cold (in ice bucket) while performing assay.
20 mM Hepes, pH7.4
1 mM MgCl2
100 mM NaCl

2X Regeneration Buffer (make in binding buffer):
20 mM Phosphocreatine (1.02 gm/200 ml binding buffer)
20 units Creatine phosphokinase (4 mg/200 ml)
20 uM GTP (make up 10.46 mg/ml in binding buffer and add 200 ul /200 ml)
0.2 mM ATP (22.04 mg/200 ml)
100 mM IBMX (44.4 mg IBMX dissolved in 1 ml 100% DMSO first and then add the entire amount to 200 ml of buffer).

Regeneration buffer can be aliquotted into 40-45 ml portions (in 50 ml sterile tubes) and kept frozen for up to 2 months. Simply put the tube in a beaker with room temperature water to thaw out the regeneration buffer on the day of the assay.

**A. Assay procedure**

1) Pipet 50 ul regeneration buffer in all 96 wells using Matrix 1250 8-channel pipettor.

2) Pipet 5 ul DMSO in columns 1 and columns 11 and 12.

3) Pipet 50 ul cAMP standards in columns 11 and 12 in this format: 50 pmole/well for row A, 25 pmole/well for row B, 12.5 pmol/well for row C, 5 picomol/well for row D, 2.5 pmole/well for row E, 1.25 pmole/well for row F, 0.5 pmole/well for row G, and 0 pmole/well (buffer only) for row H.

4) Pipet 5 ul compounds from each well of a compound dilution plate, for IC50s, using the following dilution scheme:

Well H: 400 uM compound (final concentration of compound in reaction mix = 5/100 x 400 uM = 20 uM
Well G: 1:l0 dilution of Well H (i.e. 5ul compound from well H + 45 ul 100% DMSO) (final concentration = 2 uM)
Well F: 1:10 dilution of well G (final concentration = 0.2 uM)
Well E: 1:10 dilution of well F (final concentration = 0.02 uM)
Well D: 1:10 dilution of well E (final concentration = 0.002 uM)
Well C: 1:10 dilution of well D (final concentration = 0.0002 uM
Well B: 1:10 dilution of well C (final concentration = 0.00002 uM)
Well A: 1:10 dilution of well B (final concentration = 0.000002 uM)

$IC_{50}$s or $EC_{50}$s are done in triplicate. One Flashplate can therefore be set up to handle 3 compounds. (i.e., columns 2, 3, and 4 are for compound #1, columns 5, 6, and 7 are for compound #2, and columns 8, 9, and 10 are for compound #3.)

5) Add 50 ul of **RUP3** membranes to all wells in Columns 2 to 10. (Prior to the start of the assay, the frozen membrane pellets for both **RUP3** and CMV (cells transfected with an expression plasmid containing no **RUP3** sequences), are suspended in binding buffer, usually 1 ml binding buffer for 1 plate of membranes: The membranes are kept in ice all the time, and a polytron (Brinkmann polytron, model # PT-3100) is used (setting 6-7, for 15-20 seconds) to obtain a homogeneous membrane suspension.) Protein concentration is determined by Bradford protein assay kit using instructions given in the kit, using the standard supplied with the kit as a reference. The protein concentration of the membranes is adjusted with binding buffer, so that 50 ul membranes = 15 ug protein (i.e. 0.3 mg/ml protein).

6) In column 1, Wells A, B, C, and D, add 50 ul **RUP3** membranes. To wells E, F, G, and H, add 50 ul CMV membranes, (CMV membranes being of the same protein concentration as the **RUP3** membranes).

7) Incubate 1 hour at room temperature with agitation on a rotating platform shaker. Cover with foil while shaking.

8) After 1 hour, add (to all 96 wells), 100 ul of the $^{125}I$ tracer in detection buffer supplied with the Flashplate kit plus proclin, made up in the following manner:
Pipet per 10 ml per Flashplate: 100 ml of detection buffer + 1 ml $^{125}I$ + 0.2 ml of Proclin ( the proclin helps to stop the production of cAMP). Make a smaller quantity of detection buffer mix if you have fewer plates.

9) Shake the plates on a rotating platform shaker for 2 hours, covering the plates with lead sheeting.

10) Seal the plates with the plastic film sealers provided with the Flashplate kit.

11) Count the plates using a TRILUX 1450 Microbeta Counter. See the door of the counter to determine which counting protocol to use.

12) Data is analyzed on the Arena Database according to the **RUP3** non-fusion, $IC_{50}$ $EC_{50}$ for 96-well cAMP membrane assay, and the compound numbers and the concentrations of compounds must be entered by the user.

**B. Membrane Cyclase Criteria**

1) Signal to Noise:

An acceptable signal-to-noise ratio for **RUP3** can vary from 4 to 6. The raw cpms are approximately 1800 to 2500 for **RUP3** and 3500-4500 for CMV. The cpm (or ultimately pmoles of cAMP/well) cannot be outside the standard curve, and should not approach well A of the standard curve (50 pmole/well) and well H (no cAMP). Generally, the pmoles of cAMP produced by **RUP3** receptor are around 11 to 13 pmole/well (for 15 ug/well protein), and for CMV are between 2 to 3 pmole/well (for 15 ug protein /well).

2) Standard curve:

The slope should be linear and the error bars for duplicates should be very small. The receptor and CMV controls cannot be off scale of the standard curve, as described above. If the receptor controls are off the high end of the standard curve,i.e. 50 pmole/well or higher, one must repeat the experiment using less protein. However, such a case has not been observed with transiently transfected **RUP3** membranes (10 ug DNA/15 cm plate, using 60 ul Lipofectamine, and preparing membranes after 24 hour of transfection.)

3) The $IC_{50}$ or $EC_{50}$ curve should be at 100% (+ or - 20%) of control **RUP3** membranes at the top, and should go down to 0 (or up to 20%) at the bottom. The standard error of the triplicate determinations should be + or -10%.

**C. Stimulation of cAMP in HIT-T15 cells**

HTT-T15 (ATCC CRL#1777) is an immortalized hamster insulin-producing cell line. These cells express **RUP3** and therefore can be used to assess the ability of **RUP3** ligands to stimulate or inhibit cAMP accumulation via its endogenously expressed receptor. In this assay, cells are grown to 80% confluence and then distributed into a 96-well Flashplate (50,000 cells/ well) for detection of cAMP via a "cAMP Flashplate Assay" (NEN, Cat # SMP004). Briefly, cells are placed into anti-cAMP antibody-coated wells that contain either vehicle, the test ligand(s) at a concentration of interest, or 1 uM forskolin. The latter is a direct activator of adenylyl cyclase and serves as a positive control for stimulation of cAMP in HIT-T15 cells. All conditions are tested in triplicate. After a 1 hour incubation to allow for stimulation of cAMP, a Detection Mix containing $^{125}I$-cAMP is added to each well and the plate is allowed to incubate for another 1 hour. The wells are then aspirated to remove unbound $^{125}I$-cAMP. Bound $^{125}I$-cAMP is detected using a Wallac Microbeta Counter. The amount of cAMP in each sample is determined by comparison to a standard curve, obtained by placing known concentrations of cAMP in some wells on the plate.

**D. Stimulation of insulin secretion in HIT-T15 cells**

It is known that stimulation of cAMP in HIT-T15 cells causes an increase in insulin secretion when the glucose concentration in the culture media is changed from 3mM to 15 mM. Thus, **RUP3** ligands can also be tested for their ability to stimulate glucose-dependent insulin secretion (GSIS) in HIT-T15 cells. In this assay, 30,000 cells/well in a 12-well plate are incubated in culture media containing 3 mM glucose and no serum for 2 hours. The media is then changed;

wells receive media containing either 3 mM or 15 mM glucose, and in both cases the media contains either vehicle (DMSO) or **RUP3** ligand at a concentration of interest. Some wells receive media containing 1 uM forskolin as a positive control. All conditions are tested in triplicate. Cells are incubated for 30 minutes, and the amount of insulin secreted into the media is determined by ELISA, using a kit from either Peninsula Laboratories (Cat # ELIS-7536) or Crystal Chem Inc. (Cat # 90060).

**E. Stimulation of insulin secretion in isolated rat islets**

As with HIT-T15 cells, it is known that stimulation of cAMP in isolated rat islets causes an increase in insulin secretion when the glucose concentration in the culture media is changed from 60 mg/dl to 300 mg/dl. **RUP3** is an endogenously expressed GPCR in the insulin-producing cells of rat islets. Thus, **RUP3** ligands can also be tested for their ability to stimulate GSIS in rat islet cultures. This assay is performed as follows:

**A.** Select 75-150 islet equivalents (IEQ) for each assay condition using a dissecting microscope. Incubate overnight in low-glucose culture medium. (Optional.)

**B.** Divide the islets evenly into triplicate samples of 25-40 islet equivalents per sample. Transfer to 40 μm mesh sterile cell strainers in wells of a 6-well plate with 5 ml of low (60 mg/dl) glucose Krebs-Ringers Buffer (KRB) assay medium.

**C.** Incubate 30 minutes (1 hour if overnight step skipped) at 37˚C and 5% $CO_2$. Save the supernatants if a positive control for the RIA is desired.

**D.** Move strainers with islets to new wells with 5ml/well low glucose KRB. This is the second pre-incubation and serves to remove residual or carryover insulin from the culture medium. Incubate 30 minutes.

**E.** Move strainers to next wells (Low 1) with 4 or 5 ml low glucose KRB. Incubate @ 37˚ C for 30 minutes. Collect supernatants into low-binding polypropylene tubes pre-labelled for identification and keep cold.

**F.** Move strainers to high glucose wells (300mg/dl, which is equivalent to 16.7mM). Incubate and collect supernatants as before. Rinse islets in their strainers in low-glucose to remove residual insulin. If the rinse if to be collected for analysis, use one rinse well for each condition (i.e. set of triplicates.)

**G.** Move strainers to final wells with low-glucose assay medium (Low 2). Incubate and collect supernatants as before.

**H.** Keeping cold, centrifuge supernatants at 1800rpm for 5 minutes @ 4-8˚C to remove small islets/islet pieces that escape the 40mm mesh. Remove all but lower 0.5- 1 ml and distribute in duplicate to pre-labelled low-binding tubes. Freeze and store at <-20˚ C until insulin concentrations can be determined.

**I.** Insulin determinations are done as above, or by Linco Labs as a custom service, using their rat insulin RIA (Cat. # RI-13K).

**Example 2**

**A. RT-PCR analysis of RUP3 expression in human tissues (Figure 1A).**

RT-PCR was applied to determine the tissue distribution of RUP3. Oligonucleotides used for PCR had the following sequences:

ZC47: 5'-CATTGCCGGGCTGTGGTTAGTGTC-3' (forward primer), (SEQ ID NO:3);
ZC48: 5'-GGCATAGATGAGTGGGTTGAGCAG-3' (reverse primer), (SEQ ID NO:4);

and the human multiple tissue cDNA panels (MTC, Clontech) were used as templates (1 ng cDNA per PCR amplification). Twenty-two (22) human tissues were analyzed. PCR was performed using Platinum PCR SuperMix (Life Technologies, Inc.; manufacture instructions were followed) in a 50 μl reaction by the following sequences: step 1, 95˚C for 4 min; step 2, 95˚C for 1 min; step 3,60˚C for 30 sec; step 4, 72˚C for 1 min; and step 5, 72˚C for 7 min. Steps 2 through 4 were repeated 35 times.

The resulting PCRreactions (15 μl) were loaded on a 1.5% agarose gel to analyze the RT-PCR products, and

a specific 466 base-pair DNA fragment representing **RUP3** was specifically amplified from cDNA of pancreas origin. Low expression was also evident in subregions of brain.

**B. cDNA Dot-Blot analysis of RUP3 expression in human tissues (Figure 1B).**

Results from RT-PCR analysis were further confirmed in cDNA dot-blot analysis. In this assay, a dot-blot membrane containing cDNA from 50 human tissues (Clontech) was hybridized with a $^{32}$P-radiolabelled DNA probe having sequences derived from human **RUP3.** Hybridyzation signals were seen in pancreas and fetal liver, suggesting these tissues express **RUP3.** No significant expression was detected in other tissues analyzed.

**C. Analysis of RUP3 by RT-PCR with isolated human pancreatic islets of Langerhans (Figure 1C).**

Further analysis of **RUP3** by RT-PCR with isolated human pancreatic islets of Langerhans showed robust expression **of RUP3** in islet cells but not in control samples.

**D. Analysis of RUP3 expression with cDNAs of rat origin by RT-PCR (Figure 1D).**

**RUP3** expression was further analyzed with cDNAs of rat origin by RT-PCR technique. Tissue cDNAs used for this assay were obtained from Clontech except those for hypothalamus and islets, which were prepared in house. Concentrations of each cDNA sample were normalized via a control RT-PCR analysis of the house-keeping gene GAPDH before assaying for **RUP3** expression. Oligonucleotides used for PCR had the following sequences:

rat **RUP3 ("rRUP3")** forward: 5'-CATGGGCCCTGCACCTTCTTTG-3' (SEQ ID NO:5);
**rRUP3** reverse: 5'-GCTCCGGATGGCTGATGATAGTGA-3' (SEQ ID NO:6).

PCR was performed using Platinum PCR SuperMix (Life Technologies, Inc.; manufacture instructions were followed) in a 50 μl reaction by the following sequences: step 1, 95°C for 4 min; step 2, 95°C for 1 min; step 3, 60°C for 30 sec; step 4, 72°C for 1 min; and step 5, 72°C for 7 min. Steps 2 through 4 were repeated 35 times.

The resulting PCR reactions (15 μl) were loaded on a 1.5% agarose gel to analyze the RT-PCR products, and a specific 547 base-pair DNA fragment representing rat **RUP3** was specifically amplified from cDNA of pancreas origin, revealing a similar expression profile with human. Of particular note, robust expression was seen in isolated islets and hypothalamus.

**Example 3**

**RUP3 protein expression is restricted to β cell lineage of pancreatic islets (Figure 2).**

**A. A polyclonal anti-RUP3 antibody was prepared in rabbits (Figure 2A).**

Rabbits were immunized with an antigenic peptide with sequence derived from rat **RUP3 ("rRUP3").** The peptide sequence was RGPERTRESAYHIVTISHPELDG and shared 100% identity with mouse RUP3 in the corresponding region. A cysteine residue was incorporated at the N-terminal end of this antigenic peptide to facilitate KLH crosslinking before injecting into rabbits. The resulting antisera ("anti**-rRUP3**") and the corresponding preimmune sera ("pre-r**RUP3**") were tested for immune reactivity to mouse **RUP3** in immunobloting assays (lanes 1 thought 4). In this assay, the GST-**RUP3** fusion protein was readily recognized by the anti-**rRUP3** antisera (lane 4), but not by the preimmune sera (lane 2). The immunoreactive signal could be efficiently eliminated when the immunobloting assay was performed in the presence of excess antigenic peptide (lane 6).

**B. RUP3 expression in insulin-producing β cells of pancreatic islets (Figure 2B).**

Rat pancreas was perfused with 4% paraformaldehyde (PFA) in PBS and embedded in OCT embedding medium. Ten micron sections were prepared, fixed on glass slides, and immunostained with either pre**-rRUP3** (Figure **2B,** panel *a*) or with anti**-rRUP3** antisera (Figure **2B,** panels *c* and *e*) followed by secondary staining with donkey anti-rabbit IgG conjugated to the fluorochrome Cy-3. Each section was also co-immunostained with a monoclonal anti-insulin antibody (Santa Cruz, Figure **2B,** panels b and d) in primary staining followed by a secondary staining with donkey anti-mouse IgG conjugated with FITC, or with a goat anti-glucagon antibody (Santa Cruz, Figure **2B,** panel f) and donkey anti-goat IgG coupled to FITC. Immunofluorescent signals were examined under a fluorescent microscope. **RUP3** was found expressed in insulin producing cells (panels c and d), but not in glucagons producing cells (panels e and f). These

data demonstrated that **RUP3** is expressed in β cells but not in α cells of the rat pancreatic islets. Analogous results were obtained when mouse pancreatic sections were investigated for **RUP3** expression.

**Example 4**

**Functional Activities of RUP3 *In Vitro* (Figure 3).**

It was established that **RUP3** stimulates the production of cAMP by cotransfection of 293 cells with: (1) a CRE-Luciferase reporter, wherein the ability to stimulate the production of firefly luciferase depends on increased cAMP in cells, and (2) an expression plasmid encoding the human form of **RUP3** (Figure **3A**). Note that cells co-transfected with an expression plasmid containing no **RUP3** sequences ("CMV" in Figure **3A)** produce very little luciferase activity, whereas cells transfected with an expression plasmid encoding **RUP3 ("RUP3"** in Figure **3A)** have at least a 10-fold increase in luciferase activity. This indicates that **RUP3** stimulates the production of cAMP when introduced into 293 cells. This property **of RUP3** is conserved across species, because hamster **RUP3** stimulates luciferase activity when introduced into 293 cells in a manner analogous to that described for human **RUP3** (Figure **3B).**

It is established that, when cAMP is increased in insulin-producing cells of the pancreas, these cells exhibit an enhanced ability to secrete insulin when glucose concentrations rise. To test whether **RUP3** might impart enhanced glucose-dependent insulin release, retrovirus containing human **RUP3** was used to generate Tu6 cells that express high levels **of RUP3.** Tu6 cells produce insulin, but do not express appreciable levels **of RUP3** and do not normally exhibit an increase in insulin release when increased glucose is present in the culture media. As shown in Figure **3C**, Tu6 cells transduced with a control virus that contains no receptor are still able to produce insulin, but do not show an increase in insulin secretion when the concentration of glucose in the culture media is shifted from 1 mM to 16 mM. By contrast, Tu6 cells transduced with **RUP3**-containing retrovirus display significant glucose-dependent insulin secretion (Figure **3C**).

**Example 5**

**Functional Activities of RUP3 Agonists *In Vitro* (Figure 4).**

To demonstrate that **RUP3** agonists stimulate endogenously expressed **RUP3** in insulin-producing cells, two *in vitro* models can be used. In the first of these, **RUP3** agonists are used to stimulate HIT-T15 cells, which express **RUP3** at significant levels, as indicated in the Northern blot shown in Figure **4A.** Moreover, these cells are known to exhibit enhanced glucose-dependent insulin release when intracellular cAMP concentrations are elevated. In the example shown in Figure **4B,** the **RUP3** agonist Compound B84 stimulates cAMP production in HIT cells, at a level comparable to that seen with the adenyl cyclase activator forskolin. This indicates that Compound B84 is a very robust stimulator of cAMP in HIT-T15 cells. In the example shown in Figure **4C**, Compound B84 also stimulates insulin secretion in HIT cells exposed to 15 mM glucose, once again at a level comparable to that seen with the adenyl cyclase activator forskolin. This indicates that Compound B84 is a very robust potentiator of insulin secretion in HTT-T15 cells.

Isolated rat islets are the other *in vitro* model used to demonstrate the efficacy of **RUP3** agonists. In this model, agents that induce cAMP are not expected to stimulate insulin secretion when glucose concentrations are low (e.g. 60 mg/dl). However, when glucose concentrations are increased (e.g. to 300 mg/dl), these agents are expected to enhance insulin secretion to levels above those seen with glucose alone. In the example shown in Figure **4D,** both **RUP3** agonists (Compounds 48 and 51 at 10 uM concentration) enhanced glucose-dependent insulin release. Moreover, the level of enhancement was comparable to that seen with 25 nM GLP-1, a gut hormone known to act on islets in this manner.

**Example 6**

***In vivo* effects of RUP3 agonists on glucose homeostasis in mice.**

**A. Oral Glucose tolerance test (oGTT) (Figure 5A).**

Male C57b1/6N mice at age of 8 weeks were fasted for 18 hours and randomly grouped (n=11) to receive a **RUP3** agonist (Compound B70) at indicated doses, or with control extendin-4 (ex-4, 1 μg/kg), a GLP-1 peptide analog known to stimulate glucose-dependent insulin secretion. Compound B70 was delivered orally via a gavage needle (p.o. volume at 100 μl). Control Ex-4 was delivered intraperitoneally. Thirty minutes after administration of test compound and control ex-4, mice were administered orally with dextrose at 5 g/kg dose. Levels of blood glucose were determined at the indicated time points using Glucometer Elite XL (Bayer). Figure 5A shows the mean glucose concentration averaged from eleven animals in each treatment group. These results demonstrated that the **RUP3** agonist, Compound B70, lowered blood glucose in a dose-dependent manner in mice after challenged with glucose.

**B. Acute response of db mice to RUP3 agonist (Figure 5B).**

Male db mice (C57BL/KsOlahsd-Leprdb, diabetic, Harlan) at age of 10 weeks were randomly grouped (n=6) to receive vehicle (oral gavage), Compound B70 (60 mg/kg, oral gavage), or Ex-4 (1 μg/kg, intraperitoneally). After compound administration, food was removed and blood glucose levels were determined at indicated times as shown in Figure **5B.** Reduction in blood glucose at each time point was expressed as percentage of original glucose levels, averaged from six animals for each group. These animals had blood glucose levels (fed state) of 300-400 mg/dl, significantly higher than non-diabetic wild type animals. Treatment with Compound B70 or Ex-4 significantly reduced glucose levels compared to vehicle control as shown in Figure **5B.** These data demonstrated that the **RUP3** agonist had efficacy in improving glucose homeostasis in diabetic animals.

**Example 7**

**CRE-Luciferase Assay in 293 Cells**

293 cells were plated in 96-well tissue culture plates at a concentration of 20,000 cells per well. The following day, the cells are transfected with a mixture of pCRE-Luc (Stratagene, Cat. # 219076), the indicated expression plasmid, and pEGFP-Nl (Clontech, Cat. # 6085-1) at a ratio of 5:1:0.25 using Lipofectamine Reagent (Invitrogen, Cat. #18324-020) according to the manufacturer's directions. pEGFP-N1 encodes a "green fluorescent protein" and was used as a control to determine that most cells were successfully transfected. After 24-48 hr, the cells were lysed *in situ* with 100 ul/ well reconstituted Luclite buffer (Luclite Reporter Gene Assay Kit, Packard, Cat. # 6016911), according to the manufacturer's directions. After a 10 minute incubation in the dark, luminescence was measured using a TRILUX 1450 Microbeta Counter (Wallac).

**Example 8**

**Generation of Tu6/ RUP3 Stable Lines**

To produce Tu6 cells that express **RUP3** at high levels, a retrovirus bearing an expression cassette for **RUP3** was generated. Briefly, **RUP3** coding sequence was cloned into the retroviral vector pLNCX2 (Clontech, Cat # 6102-1). The amphotropic packaging cell line PT-67 (Clontech, K1060-D) was then transfected with either the parental vector pLNCX2 or pLNCX2/**RUP3** using Lipofectamine and stable lines were established using guidelines provided by the PT-67 vendor. Retrovirus-containing supernatant was obtained by collecting media from the resultant stables according to the manufacturer's directions. Tu6 cells, in a 10 cm dish, were then infected with retrovirus by incubating in a solution of 1 ml viral supernatant/ 9 ml culture media containing 40 ug/ml polybrene for 24 hours. The medium was then changed to culture media containing 300 ug/ml G418. G418-resistant clones were ultimately created by virtue of the neomycin-resistance gene cassette present in the pLNCX2 vector, thus indicating the successful integration of retrovirus into the Tu6 genome. The expression **of RUP3** in the Tu6/**RUP3** G418-resistant colonies was confirmed by Northern blot.

**Example 9**

**Insulin secretion, Tu6 Stables**

To measure insulin secretion from rodent insulin-producing cell lines, cells were first cultured overnight in serum-free, glucose-deficient media. The following morning, the cells were then placed in the same media supplemented with either 1 mM or 16 mM glucose. After an incubation of 4 hours, the media was collected and analyzed for insulin content using a Rat Insulin Enzyme-Immunoassay (EIA) System (Amersham Pharmacia Biotech, Cat. # RPN 2567). Typically, the assay was performed using multiple dilutions of sample media in order to ensure that the sample measurements fell within the boundaries of the standard curve (generated using known amounts of insulin), as recommended by the manufacturer.

**Example 10**

**RUP3 RNA Blot**

To determine the expression of **RUP3** in insulin-producing or non islet cells, the following cell lines were obtained and cultured according to guidelines provided by American Type Culture Collection or the indicated provider.

| Cell Line | Provider | Cat. # |
|---|---|---|
| HIT-T15 | American Type Culture Collection | CRL-1777 |
| NIT-1 | American Type Culture Collection | . CRL-2055 |
| RIN-5F | American Type Culture Collection | CRL-2058 |
| Tu-6 | Ole Madsen, Hagedorn Res. Lab. | N/A |
| αTC-9 | American Type Culture Collection | CRL-2350 |
| RIN-14B | American Type Culture Collection | CRL-2059 |
| ARIP | American Type Culture Collection | CRL-1674 |
| AR42J | American Type Culture Collection | CRL-1492 |
| Panc-1 | American Type Culture Collection | CRL-1469 |
| BxPc-3 | American Type Culture Collection | CRL-1687 |
| 293 | Q-Biogene | AES0503 |
| NIH-3T3 | American Type Culture Collection | CRL-1658 |

Total RNA was isolated from each of these cell lines using TRIZOL (Invitrogen, Cat # 15596-018), subjected to electrophoresis through an agarose/formaldehyde gel and an RNA blot was prepared using standard molecular biological techniques. A radiolabelled **RUP3** probe, corresponding to the full-length coding sequence of **RUP3,** was prepared using a Prime-It II Random Primer Labeling Kit (Stratagene, Cat # 300385). The denatured probe, 10 ml ExpressHyb solution (Clontech, Cat # 8015-2) and the RNA blot were incubated in a hybridization oven, washed and exposed to film using standard molecular biology practices.

**Example 11**

**Receptor Binding Assay**

In addition to the methods described herein, another means for evaluating a test compound is by determining binding affinities to the **RUP3** receptor. This type of assay generally requires a radiolabelled ligand to the **RUP3** receptor. Absent the use of known ligands for the **RUP3** receptor and radiolabels thereof, compounds of Formula (**Ia**) can be labelled with a radioisotope and used in an assay for evaluating the affinity of a test compound to the **RUP3** receptor.

A radiolabelled **RUP3** compound as described herein can be used in a screening assay to identify/evaluate compounds. In general terms, a newly synthesized or identified compound (i.e., test compound) can be evaluated for its ability to reduce binding of the "radiolabelled compound of Formula (**Ia**)" to the **RUP3** receptor. Accordingly, the ability to compete with the "radio-labelled compound of Formula (**Ia**)" or **Radiolabelled RUP3 Ligand** for the binding to the **RUP3** receptor directly correlates to its binding affinity of the test compound to the **RUP3** receptor.

**ASSAY PROTOCOL FOR DETERMINING RECEPTOR BINDING FOR RUP3:**

**A. RUP3 RECEPTOR PREPARATION**

293 cells (human kidney, ATCC), transiently transfected with 10 ug human **RUP3** receptor and 60 ul Lipofectamine (per 15-cm dish), were grown in the dish for 24 hours (75% confluency) with a media change and removed with 10 ml/dish of Hepes-EDTA buffer ( 20mM Hepes + 10 mM EDTA, pH 7.4). The cells were then centrifuged in a Beckman Coulter centrifuge for 20 minutes, 17,000 rpm (JA-25.50 rotor). Subsequently, the pellet was resuspended in 20 mM Hepes + 1 mM EDTA, pH 7.4 and homogenized with a 50- ml Dounce homogenizer and again centrifuged. After removing the supernatant, the pellets were stored at -80˚C, until used in binding assay. When used in the assay, membranes were thawed on ice for 20 minutes and then 10 mL of incubation buffer (20 mM Hepes, 1 mM $MgCl_2$,100 mM NaCl, pH 7.4) added. The membranes were then vortexed to resuspend the crude membrane pellet and homogenized with a Brinkmann PT-3100 Polytron homogenizer for 15 seconds at setting 6. The concentration of membrane protein was determined using the BRL Bradford protein assay.

### B. BINDING ASSAY

For total binding, a total volume of 50ul of appropriately diluted membranes (diluted in assay buffer containing 50mM Tris HCl (pH 7.4), 10mM $MgCl_2$, and 1mM EDTA; 5-50ug protein) is added to 96-well polyproylene microtiter plates followed by addition of 100ul of assay buffer and 50ul of **Radiolabelled RUP3 Ligand.** For nonspecific binding, 50 ul of assay buffer is added instead of 100ul and an additional 50ul of 10uM cold **RUP3** is added before 50ul of **Radiolabelled RUP3 Ligand** is added. Plates are then incubated at room temperature for 60-120 minutes. The binding reaction is terminated by filtering assay plates through a Microplate Devices GF/C Unifilter filtration plate with a Brandell 96-well plate harvestor followed by washing with cold 50 mM Tris HCl, pH 7.4 containing 0.9% NaCl. Then, the bottom of the filtration plate are sealed, 50ul of Optiphase Supermix is added to each well, the top of the plates are sealed, and plates are counted in a Trilux MicroBeta scintillation counter. For compound competition studies, instead of adding 100u1 of assay buffer, 100u1 of appropriately diluted test compound is added to appropriate wells followed by addition of 50ul of **Radiolabelled RUP3 Ligand.**

### C. CALCULATIONS.

The test compounds are initially assayed at 1 and 0.1 μM and then at a range of concentrations chosen such that the middle dose would cause about 50% inhibition of a **Radio-RUP3 Ligand** binding (i.e., $IC_{50}$). Specific binding in the absence of test compound ($B_o$) is the difference of total binding ($B_T$) minus non-specific binding (NSB) and similarly specific binding (in the presence of test compound) (B) is the difference of displacement binding ($B_D$) minus non-specific binding (NSB). $IC_{50}$ is determined from an inhibition response curve, logit-log plot of % $B/B_o$ vs concentration of test compound.

$K_i$ is calculated by the Cheng and Prustoff transformation:

$$K_i = IC_{50} / (1 + [L]/K_D)$$

where [L] is the concentration of a **Radio-RUP3 Ligand** used in the assay and $K_D$ is the dissociation constant of a **Radio-RUP3 Ligand** determined independently under the same binding conditions.

### CHEMISTRY

### SYNTHESES OF COMPOUNDS OF THE PRESENT INVENTION

### EXAMPLE 12

Illustrated syntheses for compounds of Formula **(Ia)** are shown in **Figure 6** where the symbols have the same definitions as used throughout this disclosure. The preparation of N-oxides is well know in the art, which include, but not limited to pyridines, pyrimidines and the like. For example, N-oxides can be prepared at normal or elevated pressure, in the presence of an oxidizing agent, such as, hydrogen peroxide, peracetic acid, perbenzoic, m-chloroperbenzoic acid (mCPBA), ozone, oxygen and the like, in the presence or absence of solvent, such as chloroform, dichlormethane, acetic acid, trifluoroacetic acid, and the like or mixtures thereof.

**Chemistry:** Proton nuclear magnetic resonance ($^1H$ NMR) spectra were recorded on a Varian Mercury Vx-400 equipped with a 4 nucleus auto switchable probe and z-gradient or a Bruker Avance-400 equipped with a QNP (Quad Nucleus Probe) or a BBI (Broad Band Inverse) and z-gradient. Chemical shifts are given in parts per million (ppm) with the residual solvent signal used as reference. NMR abbreviations are used as follows: s = singlet, d = doublet, t = triplet, q = quartet, m = multiplet, br = broad. Microwave irradiations were carried out using the Smith Synthesizer (Personal Chemistry). Thin-layer chromatography (TLC) was performed on silica gel 60 $F_{254}$ (Merck), preparatory thin-layer chromatography (prep TLC) was preformed on PK6F silica gel 60 A 1 mm plates (Whatman), and column chromatography was carried out on a silica gel column using Kieselgel 60, 0.063-0.200 mm (Merck). Evaporation was done in vacuo on a Buchi rotary evaporator. Celite 545 ® was used during palladium filtrations.
LCMS specs: 1) PC: HPLC-pumps: LC-10AD *VP,* Shimadzu Inc.; HPLC system controller: SCL-10A *VP,* Shimadzu Inc; UV-Detector: SPD-10A *VP,* Shimadzu Inc; Autosampler: CTC HTS, PAL, Leap Scientific; Mass spectrometer: API 150EX with Turbo Ion Spray source, AB/MDS Sciex; Software: Analyst 1.2. 2) Mac: HPLC-pumps: LC-8A *VP,* Shimadzu Inc; HPLC system controller: SCL-10A *VP,* Shimadzu Inc.
UV-Detector: SPD-10A *VP,* Shimadzu Inc; Autosampler: 215 Liquid Handler, Gilson Inc;
Mass spectrometer: API 150EX with Turbo Ion Spray source, AB/MDS Sciex

Software: Masschrom 1.5.2.

**Compound A1**

**1-[6-(4-Imidazol-1-yl-phenoxy)-5-nitro-pyrimidin-4-yl]-piperidine-4-carboxylic acid ethyl ester: General Method 1.**

A mixture of 1-(6-chloro-5-nitro-pyrimidin-4-yl)-piperidine-4-carboxylic acid ethyl ester (63 mg, 0.2 mmol), 4-imidazol-1-yl-phenol (42 mg, 0.26 mmol) and potassium carbonate (36 mg, 0.26 mmol, 1 equivalent) in anhydrous DMF (1 ml) was heated to 90 ˚C overnight. The resulting residue was purified by Flash Chromatography (hexanes:ethyl acetate = 1:1) to give 1-[6-(4-Imidazol-1-yl-phenoxy)-5-nitro-pyrimidin-4-yl]-piperidine-4-carboxylic acid ethyl ester **[A1]** as a yellow solid (80 mg, 92 %). $^1$H NMR ($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.96-4.03 (m, 2H), 4.15 (q, 2H), 7.38-7.42 (m, 2H), 7.46 (t, 1H), 7.55-7.59 (m, 3H), 8.17 (s, 1H), 8.90-8.92 (m, 1H). Exact mass calculated for $C_{21}H_{22}N_6O_5$ 438.17, found 439.2 ($MH^+$).

The intermediate 1-(6-chloro-5-nitro-pyrimidin-4-yl)-piperidine-4-carboxylic acid ethyl ester was prepared by following general method 2.

**General Method 2: Addition of Substituted piperidines to dichloro-5-nitropyrimidine.**

Compound **2,6-dichloro-5-nitropyrimidine** (500 mg, 2.57 mmol) was dissolved in dichloromethane (40 ml) and cooled to 0 ˚C. To this was added diisopropylethyl amine (0.54 ml, 3.08 mmol) followed by a solution of piperidine-4-ethyl-ester (3.69 mmol) in dichloromethane (5 ml) dropwise. The mixture was stirred at 0 ˚C for 1 hour and then concentrated in *vacuo.* Flash chromatography (hexanes:ethyl acetate =1:1) provided Compound Ala, 1-(6-chloro-5-nitro-pyrimidin-4-yl)-piperidine-4-carboxylic acid ethyl ester as a yellow solid (466 mg, 60 %). $^1$HNMR (DMSO, 400 MHz) δ 1.57-1.63 (m,2H), 1.91-1.95 (m, 2H), 2.72-2.74 (m, 1H), 3.17(t, 2H), 3.60 (s, 3H), 3.84-3.89 (m, 2H), 8.46 (s, 1H). Exact mass calculated for $C_{11}H_{13}ClN_4O_4$ 300.06, found 301.2 ($MH^+$).
Compounds **A2-A155** were prepared in an analogous manner as described for Compound **A1,** *supra.*

**Compound A2**

**1-[6-(2-Methyl-5-trifluoromethyl-2H-pyrazol-3-yloxy)-5-nitro-pyrimidin-4-yl]-piperidine-4-carboxylic acid ethyl ester. General Method 3:**

4,6-dichloro-5-nitro pyrimidine (5.00 g, 25.7 mmol) and diisopropylethylamine (7.66 ml, 43.9 mmol) were dissolved in dichloromethane (17.0 ml). The mixture was stirred in an ice bath and 4-ethyl ester piperidine (3.33 ml, 43.9 mmol) dissolved in dichloromethane (17.0 ml) was added dropwise. The mixture was warmed to room temperature and after 30 min. the desired product was observed by LCMS m/z 315 ($M+H^+$). The solvent was removed under reduced pressure, the crude dissolved in dioxane (20 ml), sequentially, diisopropylethylamine (6.31 ml, 36.22 mmol) and 2-Methyl-5-trifluoromethyl-2H-3-hydroxypyrazole(3.60 g, 21.73 mmol) were added and the mixture was heated at 90 ˚C for 18 h. Work up yielded a crude red oil. Purification by Flash Chromatography (0-35% diethyl ether/ hexane). Yield 58.47 % yellow solid. $^1$H NMR 400MHz DMSO δ (ppm): 8.34 (s, 1H); 6.71 (s, 1H); 4.07 (m, 2H); 3.88 (d, 2H); 3.70 (s, 3H); 3.27 (m, 2H); 2.72 (m, 1H); 1.94 (m, 2H); 1.62 (m, 2H); 1.18 (t, 3H). LCMS (ESI) m/z 444.3 ($M+H^+$, 100%)

**Compound A3**

**1-[6-(4-Methanesulfonyl-phenoxy)-5-nitro-pyrimidin-4-yl]-piperidine-4-carboxylic acid ethyl ester**

Following the general procedure **[method 1]** compound **A3** was obtained as a yellow solid (52%). $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.35-4.05 (m, 16H), 4.15 (q, 2H), 7.55-7.58 (m, 2H), 7.62-7.66 (m, 1H), 7.88-7.92 (m, 2H), 7.95 (s, 1H). Exact mass calculated for $C_{24}H_{32}N_6O_6S$ 532.21, found 533.3

**Compound A4**

**1-[6-(Benzo [1,2,5] oxadiazol-5-yloxy)-5-nitro-pyrimidin-4-yl]-piperidine-4-carboxylic acid ethyl ester**

Following the general procedure, **[method 1]** compound **A4** was obtained as a brown solid (65 %). $^1$HNMR ($CDCl_3$, 400 MHz) δ 1.28 (t, 3H), 1.80-1.96 (m, 2H), 2.00-2.08 (m, 2H), 2.65-2.75 (m, 1H), 4.02-4.05(m, 2H), 4.14 (q,

2H), 7.22 (d, 1H), 7.60 (s, 1H), 7.82 (d, 1H), 8.16 (s, 1H). Exact mass calculated for $C_{18}H_{18}N_6O_6$ 414.13, found 415.3 ($MH^+$).

**Compound A5**

**1-{6-[4-(2-Methoxycarbonyl-acetyl)-phenoxy]-5-nitro-pyrimidin-4-yl}-piperidine-4-carboxylic acid ethyl ester: [method 1]**

Flash chromatography (hexanes:ethyl acetate = 2:1) provided compound **A5** as a yellow solid (250 mg, 65 %). $^1$HNMR ($CDCl_3$, 400 MHz) δ 1.28 (t, 3H), 1.78-1.88 (m, 2H), 1.95-2.05 (m, 2H), 2.62-2.70 (m, 1H), 3.22 (t, 2H), 3.76 (s, 2H), 4.00 (s, 3H), 4.02-4.08 (m, 2H), 4.18 (q, 2H), 7.25 (d, 2H), 8.00 (d, 2H), 8.22 (s, 1H). Exact mass calculated for $C_{22}H_{24}N_4O_8$ 472.16, found 473.4 ($MH^+$).

**Compound A6**

**1-[5-Amino-6-(2-methyl-5-tritluoromethyl-2H-pyrazol-3-yloxy)-pyrimidin-4-yl]-piperidine-4-carboxylic acid ethyl ester: General Method 4:**

**A2** (100 mg, 0.225 mmol) was dissolved in ethanol (8.0 ml), 5% Pd/C (0.1 mmol) was added to the reaction vessel with stirring. The mixture was purged with hydrogen and after 15 min the desired product **A6** was observed by LCMS. Filtration followed by solvent removal under reduced pressure yielded a solid that was purified by filtration through a silica plug using 50% ethyl acetate/hexane as solvent. Yield 32.13%. $^1$H NMR 400 MHz DMSO δ (ppm): 8.34 (s, 1H); 6.71 (s, 1H); 4.07 (m, 2H); 3.88 (d, 2H); 3.70 (s, 2H); 3.30 (s, 3H); 3.23 (m, 2H); 2.72 (m, 1H); 1.95 (m, 2H); 1.62 (m, 2H); 1.17 (t, 3H). LCMS (ESI) m/z 415.3 ($M^+H^+$, 100%)

**Compound A7**

**1-[6-(2-Methyl-5-trifluoromethyl-2H-pyrazol-3-yloxy)-5-(2,2,2-trifluoro-acetylamino)-pyrimidin-4-yl]-piperidine-4-carboxylic acid ethyl ester;**

**General Method 5:**

**A6** (50 mg, 0.12 mmol) and diisopropylamine (0.83 ml, 0.476 mmol) were dissolved in anhydrous dichloromethane (2.0 ml), and excess $(CF_3CO)_2O$ was added dropwise at 0 ˚C. Stirring at room temp. was maintained for 12 hours. Purification by RP-HPLC. Yield 46 % white solid.$^1$H NMR 400MHz DMSO δ (ppm): 8.36 (s, 1H); 6.73 (s, 1H); 4.06 (m, 2H); 3.89 (d, 2H); 3.72 (s, 3H); 3.23 (m, 2H); 2.73 (m, 1H); 1.97 (m, 2H); 1.62 (m, 2H); 1.17 (t, 3H).LCMS (ESI) m/z 511.3 ($M^+H^+$, 100%)

**Compound A8**

**Propionic acid 1-[2-amino-5-formyl-6-(2-methyl-5-trifluoromethyl-2H-pyrazol-3-yloxy)-pyrimidin-4-yl]-piperidin-4-yl ester;**

**General Method 6:**

4,6-dichloro-2-amino-5-formylpyrimidine (1.90 g, 9.89 mmol) and diisopropylethylamine (3.30 ml, 18.95 mmol) were dissolved in anhydrous 1,4-dioxane (25.0 ml). The mixture was stirred on an ice bath and 4-ethyl ester piperidine (1.46 ml, 9.47 mmol) dissolved in dioxane (25.0 ml) was added dropwise. The mixture attained room temperature and after 30 min. the desired product was observed by LCMS m/z 313 ($M+H^+$). The solvent was removed under reduced pressure, and the crude residue dissolved in dioxane (20 ml), to it were added diisopropylethylamine (6,31 ml, 36.22 mmol) and 2-Methyl-5-trifluoromethyl-2H-pyrazol-3-ol (3.95 g, 23.77 mmol). The mixture heated at 90 ˚C for 18 h. Aqueous work up yielded a pale orange solid. Recrystalization from ether and hexanes, followed by filtration of the solid yielded white crystals. Yield 50.28%, $^1$H NMR 400MHz DMSO δ (ppm): 9.90 (s, 1H); 7.42 (d, 2H); 6.76 (s, 1H); 4.09 (m, 2H); 3.95 (d, 2H); 3.76 (s, 3H); 3.09 (m, 2H); 2.63 (m, 1H); 1.90 (m, 2H); 1.66 (m, 2H); 1.19 (t, 3H). LCMS (ESI) m/z 443 ($M+H^+$, 100 %)

**Compound A9**

**4-[6-(2-Methyl-5-trifluoromethyl-2H-pyrazol-3-yloxy)-5-nitro-pyrimidin-4-yl]-piperazine-1-carboxylic acid ethyl ester;**

**[method 3].** Purification by Flash Chromatography (5-30% ethyl acetate / hexane). Yield 33 % yellow oil. $^1$H NMR 400MHz DMSO δ (ppm): 8.38 (s, 1H); 6.71 (s, 1H); 4.04 (m, 2H); 3.88 (d, 2H); 3.70 (s, 3H); 3.54 (m, 2H); 3.42 (m, 2H); 3.29 (m, 2H) 1.18 (t, 3H). LCMS (ESI) m/z 446,2 ($M^+H^+$, 100%)

**Compound A10**

**1-[6-(2-Methyl-5-tritlnoromethyl-2H-pyrazol-3-yloxy)-5-nitro-pyrimidin-4-yl]-piperidine-4-carboxylic acid methyl ester;**

**[method 3].** Flash chromatography (hexanes:ethyl acetate = 2:1) provided compound **A10** as a yellow solid (173 mg, 30 %). $^1$H NMR ($CDCl_3$, 400 MHz) δ 1.80-1.88 (m, 2H), 2.02-2.12, (m, 2H), 2.72-2.74 (m, 1H), 3.72 (s, 3H), 3.78 (s, 3H). 3.94-4.06 (m, 2H), 6.49 (s, 1H), 8.25 (s, 1H). Exact mass calculated for $C_{16}H_{17}F_3N_6O_5$ 430.12, found 431.4 ($MH^+$).

**Compound A11**

**2,6-Dimethyl-4-[6-(2-methyl-5-trifluoromethyl-2H-pyrazol-3-yloxy)-5-nitro-pyrimidin-4-yl]-morpholine;**

**[method 3]**. Purification by Flash Chromatography (0-30% ethyl acetate / hexane). Yield 57 % yellow oil. $^1$H NMR 400MHz DMSO δ (ppm): 8.37 (s, 1H); 6.71 (s, 1H); 3.83 (m, 2H); 3.72 (s, 3H); 3.62 (m, 2H); 2.81 (m, 2H); 1.12 (t, 6H). LCMS (ESI) m/z 403 ($M^+H^+$, 100%)

**Compound A12**

**1-[6-(2-Methyl-5-trifluoromethyl-2H-pyrazol-3-yloxy)-5-nitro-pyrimidin-4-yl]-piperidine-3-carboxylic acid ethyl ester;**

**[method 3].** Purification by Flash Chromatography (0-50% ethyl acetate / hexane). Yield 50 % yellow oil. $^1$H NMR 400 MHz DMSO δ (ppm): 8.36 (s, 1H); 6.72 (s, 1H); 4.08 (m, 2H); 3.98 (m, 2H); 3.72 (s, 3H); 3.58 (m, 1H); 3.45 (m, 1H); 2.69 (m, 1H); 1.99 (m, 1H); 1.76 (m, 2H); 1.57 (m, 1H); 1.17 (t, 3H). LCMS (ESI) m/z 445 ($M^+H^+$, 100%)

**Compound A13**

**1-[6-(2-Methyl-5-trifluoromethyl-2H-pyrazol-3-yloxy)-5-nitxo-pyrimidin-4-yl]-piperidine-4-carboxylic acid ethylamide;**

**[method 1] .** Purification by HPLC. Yield 16 %. $^1$H NMR 400 MHz $CDCl_3$ δ (ppm): 8.17 (s, 1H); 6.43 (s, 1H); 4.06 (m, 2H); 3.72 (s, 3H); 3.12 (m, 2H); 2.58 (m, 3H); 1.88 (m, 4H). LCMS (ESI) m/z 416.1 ($M+H^+$, 100 %)

**Compound A14**

**1-[6-(2-Methyl-5-phenyl-2H-pyrazol-3-yloxy)-5-nitro-pyrimidin-4-yl]-piperidine-4-carboxylic acid ethyl ester;**

**[method 1].** The product was purified by column chromatography on silica (Biotage) using hexane/ ethyl acetate (7:3). $^1$H NMR 400MHz $CDCl_3$ δ (ppm): 8.23 (s, 1H, pyrimidine); 7.78 (d, 2H), 7.40-7.35 (dd, 2H), 6.50 (s, 1H), 4.20 (q, 2H), 4.10-4.00 (m, 2H), 3.78 (s, 3H), 3.30-3.20 (m, 2H), 2.70-2.60 (m, 1H), 2.10-2.00 (m, 2H), 2.95-2.80 (m, 2H), 1.30 (t, 3H). LCMS (ESI) for $C_{22}H_{24}N_6O_5$: m/z 453.2 ($M + H^+$, 100 %)

**Compound A15**

**4-(2-Methyl-5-trifluoromethyl-2H-pyrazol-3-yloxy)-5-nitro-6-piperidin-1-yl-pyrimidine;**

**[method 3].** The product was purified by Preparatory TLC using hexane/ ethyl acetate (7:3). $^1$H NMR 400MHz

$CDCl_3$ δ (ppm): 8.20 (s,1H, pyrimidine); 6.45 (s, 1H); 3.80 (s, 3H, $CH_3$), 3.60-3.50 (m, 4H); 1.75-1.60 (m, 5H). LCMS (ESI) for $C_{14}H_{15}F_3N_6O_3$: m/z 373 (M + $H^+$, 100 %)

**Compound A16**

**1-[5-Nitro-6-(2-trifluoromethyl-benzyloxy)-pyrimidin-4-yl]-piperidine-4-carboxylic acid ethyl ester; General Method 7:**

1-(6-chloro-5-nitropyrimidine-4-yl)-piperidine-4-carboxylic acid ethyl ester 1 (0.22 g, 0.63 mmol) and 2-(trifluoromethanol) benzyl alcohol (0.11 g, 1.26 mmol) were dissolved in DMF at room temperature. Sodium hydride (30 mg, 1.26 mmol) was added and the mixture was stirred at 40 ˚C for 30 minutes. Diluted the mixture with 10 % aq. HCl, and extracted twice with EtOAc. The combined organic extracts were washed with water and brine, dried over anhydrous $Na_2SO_4$, and concentrated in vacuo. The residue was purified by preparatory LCMS. $^1H$ NMR, 400MHz, $CDCl_3$, δ (ppm): 8.20 (s, 1H); 7.63 (t, 2H); 7.55 (t, 1H); 7.39 (t, 1H); 5.63 (s, 2H); 4.13 (q, 2H); 3.95 (d, 2H); 3.14 (t, 2H); 2.54 (m, 1H); 1.98 (m, 2H); 1.79 (m, 2H); 1.24 (t, 3H). LCMS (ESI) for $C_{20}H_{21}F_3N_4O_5$: m/z 454 (M+$H^+$, 100 %)

**Compound A17**

**1-[5-Nitro-6-(3-trifluoromethyl-benzyloxy)-pyrimidin-4-yl]-piperidine-4-carboxylic acid ethyl ester;**

**[method 7].** The residue was purified by preparatory TLC using hexane/ethyl acetate (3/1, v/v). $^1H$ NMR, 400MHz, $CDCl_3$, δ (ppm): 8.22 (s, 1H); 7.66 (s, 1H); 7.58 (t, 2H); 7.49 (t, 1H); 5.52 (s, 2H); 4.15 (q, 2H); 3.62 (m, 2H); 3.15 (t, 2H); 2.60 (m, 1H); 2.00 (m, 2H); 1.80 (m, 2H); 1.27 (t, 3H). LCMS (ESI) for $C_{21}H_{23}F_3N_4O_5$: m/z 469 (M+$H^+$, 100 %)

**Compound A18**

**1-[5-Nitro-6-(4-trifluoromethyl-benzyloxy)-pyrimidin-4-yl]-piperidine-4-carboxylic acid ethyl ester;**

**[method 7.].** The residue was purified by preparatory TLC using hexane/ethyl acetate(3/1, v/v). $^1H$ NMR, 400MHz, $CDCl_3$, δ (ppm): 8.20(s, 1H); 7.62(d, 2H); 7.52(d, 2H); 5.52(s, 2H); 4.11(q, 2H); 3.96(m, 2H); 3.15(t, 2H); 2.58 (m, 1H); 2.01(m, 2H); 1.81(m, 2H); 1.25(t, 3H). LCMS (ESI) for $C_{20}H_{21}F_3N_4O_5$: m/z 454 (M+$H^+$, 100 %)

**Compound A19**

**1-[5-Bromo-6-(2-methyl-5-trifluoromethyl-2H-pyrazol-3-yloxy)-pyrhnidin-4-yl]-piperidine-4-carboxylic acid ethyl ester; General Method 8.**

A mixture of 1-[6-(2-methyl-5-trifluoromethyl-2H-pyrazol-3-yloxy)-pyrimidin-4-yl]-piperidine-4-carboxylic acid ethyl ester (150 mg, 0.37 mmol) and NBS (65 mg, 0.37 mmol) in DMF (3 ml) was stirred at 60 ˚C for 2 days. The mixture was quenched with sodium thiosulfate, and extracted with ethyl acetate. The combined organic layer was dried with anhydrous sodium sulfate and concentrated in *vacuo.* Flash chromatography (hexanes:ethyl acetate = 3:1) provided compound **A19** as a white solid (100 mg, 57 %). $^1$HNMR ($CDCl_3$, 400 MHz) d 1.28 (t, 3H), 1.78-1.88 (m, 2H), 1.95-2.05 (m, 2H), 2.57-2.62 (m, 1H), 3.17 (t, 2H), 3.81 (s, 3H), 4.18 (q, 2H), 4.22-4.33 (m, 2H), 6.41 (s, 1H), 8.22 (s, 1H). Exact mass calculated for $C_{17}H_{19}BrF_3N_5O_3$ 477.06, found 478.0 ($MH^+$).

**Compound A20**

**1-[5-Acetylamino-6-(2-methyl-5-trifluoromethyl-2H-pyrazol-3-yloxy)-pyrimidin-4-yl]-piperidine-4-carboxylic acid ethyl ester**

**and**

**Compound A21 1-[5-Diacetylamino-6-(2-methyl-5-trifluoromethyl-2H-pyrazol-3-yloxy)-pyrimidin-4-yl]-piperidine-4-carboxylic acid ethyl ester**

**General Method 9:**

A mixture of compound **A6** (100 mg, 0.24 mmol) and acetic anhydride (0.1 ml, 1 mmol) in pyridine (5 ml) was heated to 60˚C for three days. The mixture was concentrated in *vacuo.* Flash chromatography (hexanes:ethyl acetate =1:1) provided compound **A20a** (7 mg, 7 %) and **A20b** (40 mg, 34 %) both as a yellow oil. Compound **A20a** $^1$HNMR ($CDCl_3$, 400 MHz) δ 1.33 (t, 3H), 1.77-1.85 (m,2H), 1.95-2.05 (m, 2H), 2.22 (s, 3H), 2.55-2.62 (m, 1H), 3.16 (t, 2H), 3.76 (s, 3H), 4.06-4.22 (m, 4H), 6.24 (s, 1H), 6.50 (s, 1H), 8.22 (s, 1H). Exact mass calculated for $C_{19}H_{24}F_3N_6O_4$ 456:17, found 457.2 ($MH^+$). Compound **A20b** $^1$H NMR ($CDCl_3$, 400 MHz) δ 1.25 (t, 3H), 1.77-1.85 (m, 2H), 1.95-2.05 (m, 2H), 2.37 (s, 6H), 2.55-2.62 (m, 1H), 3.18 (t, 2H), 3.66 (s, 3H), 4.06-4.22 (m, 4H), 6.24 (s, 1H), 6.31 (s, 1H), 8.25 (s, 1H). Exact mass calculated for $C_{21}H_{25}F_3N_6O_5$ 498.18, found 499.4 ($M+H^+$).

**Compound A22**

**1-[6-(2-Methyl-5-tritluoromethyl-2H-pyrazol-3-yloxy)-5-nitro-pyrimidin-4-yl]-piperidine-4-carboacylic acid;**

**[method 3]**. Purification of part of the crude by HPLC. Yield 8.58 % yellow solid. $^1$H NMR 400MHz DMSO δ (ppm): 8.33 (s, 1H); 6.71 (s, 1H); 3.88 (m, 2H); 3.70 (s, 3H); 3.22 (m, 2H); 2.63 (m, 1H); 1.93 (m, 2H); 1.59 (m, 2H). LCMS (ESI) m/z 417 ($M+H^+$, 100 %)

**Compound A23**

**1-{5-Nitro-6-[2-(2-trifluoromethyl-phenyl)-ethoxy]-pyrimidin-4-yl}-piperidine-4-carboxylic acid ethyl ester;**

**[method 7]**. The residue was purified by preparatory TLC using hexane/ethyl acetate (9/1,v/v). $^1$H NMR, 400MHz, $CDCl_3$, δ (ppm): 8.63 (s,1H); 7.45 (m, 4H); 4.16 (q,2H); 4.03 (m, 2H); 3.89 (t, 2H); 3.08 (t, 2H); 3.20 (t, 2H); 2.55 (m, 1H); 2.03 (m, 2H); 1.80 (m, 2H); 1.28 (t, 3H). LCMS (ESI) for $C_{21}H_{23}F_3N_4O_5$: m/z 468 ($M+H^+$, 100 %)

**Compound A24**

**1-{5-Nitro-6-[2-(3-trifluoromethyl-phenyl)-ethoxy]-pyrimidin-4-yl}-piperidine-4-carboxylic acid ethyl ester;**

**[method 7].** The residue was purified by preparatory TLC using hexane/ethyl acetate (9/1, v/v). $^1$H NMR, 400MHz, $CDCl_3$, δ (ppm): 8.63 (s,1H); 7.45 (m, 4H); 4.16 (q,2H); 4.03 (m, 2H); 3.89 (t, 2H); 3.08 (t, 2H); 3.20 (t, 2H); 2.55 (m, 1H); 2.03 (m, 2H); 1.80 (m, 2H); 1.28 (t, 3H). LCMS (ESI) for $C_{21}H_{23}F_3N_4O_5$: m/z 468 ($M+H^+$, 100 %)

**Compound A25**

**1-[5-Di-(methanesulfonyl)amino-6-(2-methyl-5-tritluoromethyl-2H-pyrazol-3-yloxy)-pyrimidin-4-yl]-piperidine-4-carbozylic acid ethyl ester; General Method 10:**

Compound **A6** (150 mg, 0.36 mmol) was dissolved in dichloromethane (5 ml). To this was added DIEA (0.125 ml) and methane sulfonic anhydride (94 mg, 0.54 mmol). The mixture was stirred at room temperature for 24 hours, quenched with water and extracted with dichloromethane. The combined organic layer was dried in *vacuo* and purified by HPLC to give compound **A25** as a yellow solid (24 mg, 12 %). $^1$HNMR ($CDCl_3$, 400 MHz) δ 1.28 (t, 3H), 1.80-1.95 (m, 2H), 2.05-2.15 (m, 2H), 2.60-2.68 (m, 1H), 3.38 (t, 2H), 3.50 (s, 6H), 3.79 (s, 3H), 4.17(q, 2H), 4.44-4.55 (m, 2H), 6.24 (s, 1H), 8.22 (s, 1H). Exact mass calculated for $C_{19}H_{25}F_3N_6O_5S_2$ 570.12, found 571.3 ($MH^+$).

**Compound A26**

**1-[5-Nitro-6-(3-trifluoromethyl-phenoxy)-pyrimidin-4-yl]-piperidine-4-carboxylic acid ethyl ester;**

**[method 1].** The residue was purified by column chromatography (Biotage) using 10 % hexane/ethyl acetate. $^1H$ NMR, 400MHz, $CDCl_3$, δ (ppm): 8.16 (s, 1H); 7.53 (m, 2H); 7.41 (s, 1H); 7.02 (d, 1H); 4.16 (q, 2H); 4.01 (m, 1H); 3.21 (t, 2H); 2.97 (s, 9H); 2.90 (s, 9H); 2.63 (m, 1H); 2.02 (m, 2H); 1.85 (m, 4H); 1.27 (t, 3H). LCMS (ESI) for $C_{19}H_{19}F_3N_4O_5$: m/z 440 ($M+H^+$, 100 %)

**Compound A27**

**1-[5-Methyl-6-(2-methyl-5-trifluoromethyl-2H-pyrazol-3-yloxy)-pyrimidin-4-yl]-piperidine-4-carboxylic acid ethyl ester; General Method 11.**

A mixture of **A19** (100 mg, 0.21mmo1), methyl boronic acid (25 mg, 0.42 mmol), dichlorobis(triphenylphosphine) palladium (II) (15 mg, 0.021 mmol) and potassium carbonate (87 mg, 0.62 mmol) in dry DMF (5 ml) was heated to 100 ˚C under nitrogen overnight. The mixture was cooled to room temperature, quenched with water and extracted with ethyl acetate. The combined organic layer was dried with anhydrous sodium sulfate and concentrated in *vacuo.* Flash chromatography (hexanes:ethyl acetate = 3:1) provided compound **A27** as an oil (20 mg, 23 %). $^1H$NMR ($CDCl_3$, 400 MHz) δ 1.28 (t, 3H), 1.82-1.94 (m, 2H), 1.97-2.05 (m, 2H), 2.23 (s, 3H), 2.57-2.62 (m, 1H), 3.02 (t, 2H), 3.77 (s, 3H), 3.82-3.88 (m, 2H), 4.18 (q, 2H), 6.34 (s, 1H), 8.22 (s, 1H). Exact mass calculated for $C_{18}H_{22}F_3N_5O_3$ 413.17, found 414.4 ($MH^+$).

**Compound A28**

**1-[5-Nitro-6-(2-trifluoromethyl-phenoxy)-pyrimidin-4-yl]-piperidine-4-carboxylic acid ethyl ester;**

**[method 1].** The residue was purified by column chromatography (Biotage) using 10 % hexane/ethyl acetate. Yield 74 %. $^1H$ NMR, 400MHz, $CDCl_3$, δ (ppm): 8.14 (s, 1H); 7.70 (d, 1H); 7.62 (t, 1H); 7.38 (t, 1H); 7.26 (d, 1H); 4.17 (q, 2H); 4.03 (m, 2H); 3.23 (t, 2H); 2.63 (m, 1H); 2.04 (m, 2H); 1.85 (m,2H); 1.28 (t, 3H). LCMS (ESI) for $C_{19}H_{19}F_3N_4O_5$: m/z 440 ($M+H^+$, 100 %)

**Compound A29**

**1-{1-[5-Nitro-6-(4-tritluoromethyl-phenoxy)-pyrimidin-4-yl]-piperidin-4-carboxylic acid ethyl ester**

**[method 1].** The residue was purified by column chromatography (Biotage) using 10 % hexane/ethyl acetate. Yield 58 %.$^1H$ NMR, 400 MHz, $CDCl_3$, δ (ppm): 8.01 (s, 1H); 7.47 (d, 2H); 6.93 (d, 2H); 4.16 (q, 2H); 4.05 (m, 2H); 3.23 (t, 2H); 2.66 (m, 1H); 2.09 (m, 2H); 1.93 (m ,2H); 1.28 (t, 3H). LCMS (ESI) for $C_{19}H_{191}F_3N_4O_5$: m/z 440 ($M+H^+$, 100 %)

**Compound A30**

**1-[6-(4-Fluoro-phenoxy)-5-nitro-pyrimidin-4-yl]-piperidine-4-carboxylic acid ethyl ester;**

**[method 1].** Yield 0.016 g, 16 %. Yellow solid. $^1H$ NMR 400MHz $CDCl_3$ δ (ppm): 8.17 (s, 1H); 7.10 (m, 4H); 4.18 (q, 2H); 4.03 (m, 2H); 3.22 (m, 2H); 2.63 (m 1H); 2.03 (m, 2H); 1.84 (m, 2H); 1.29 (m, 3H). LCMS (ESI) m/z 391 ($M+H^+$,100%)

**Compound A31**

**1-[6-(2,5-Dimethyl-2H-pyrazol-3-yloxy)-5-nitro-pyrimidin-4-yl]-piperidine-4-carboxylic acid ethyl ester;**

**[method 3].** The product was purified by column chromatography on silica (Biotage) using hexane/ ethyl acetate (7:3). $^1H$NMR 400MHz $CDCl_3$ δ (ppm): 8.23 (s,1H, pyrimidine); 5.97 (s, 1H), 4.20 (q, 2H), 4.10-4.00 (m,2H), 3.50 (s,3H), 3.30-3.20 (m, 2H), 2.70-2.60 (m, 1H), 2.10 (s, 3H), 2.00 (m,2H), 1.95-1.90 (m, 2H), 1.30 (t, 3H). LCMS (ESI) for $C_{17}H_{22}N_6O_5$: m/z 391.3 ($M+H^+$, 100 %)

**Compound A32**

**1-[6-(4-Bromo-phenoxy)-5-nitro-pyrimidin-4-yl]-piperidine-4-carboxylic acid ethyl ester;**

**[method 1].** The solvent was removed in vacuo and the residue purified by preparatory thin layer chromatography [$SiO_2$; EtOAc/hexane; 50:50]. Yield 0.010 g, 9 %. Yellow solid.$^1$H NMR 400MHz $CDCl_3$ δ (ppm): 8.17 (s, 1H); 7.52 (d, 2H); 7.03 (d, 2H); 4.17 (q, 2H); 4.02 (m, 2H); 3.22 (m, 2H); 2.63 (m, 1H); 2.03 (m, 2H); 1.84 (m, 2H); 1.29 (m, 3H) LCMS (ESI) m/z 452, 453 ($M+H^+$,100%)

**Compound A33**

**1-[6-(4-Chloro-phenoxy)-5-nitro-pyrimidin-4-yl]-piperidine-4-carboxylic** acid ethyl **ester;**

[method 1]. Yield 0.009g, 9%. Yellow solid. $^1$H NMR 400MHz $CDCl_3$ δ (ppm): 8.17 (s, 1H); 7.37 (d, 2H); 7.08 (d, 2H); 4.17 (m, 2H); 4.03 (m, 2H); 3.22 (m, 2H); 2.64 (m, 1H); 2.03 (m, 2H); 1.84 (m, 2H); 1.29 (m, 3H). LCMS (ESI) m/z 407 ($M+H^+$,100%)

**Compound A34**

**1-[6-(4-Carbamoyl-phenoxy)-5-nitro-pyrimidin-4-yl]-piperidine-4-carboxylic acid ethyl ester;**

**General Method 1a: A** mixture of compound 7 (63 mg, 0.2 mmol), 4-hydroxybenzene carboxamide (35 mg, 0.26 mmol) and potassium carbonate (36 mg, 0.26 mmol) in DMF (1 ml) was heated in microwave for 2 minutes at 80˚C. Following the general procedure, compound **A34** was obtained as a yellow solid (65 %). $^1$HNMR ($CDCl_3$, 400 MHz) δ 1.19 (t, 3H), 1.70-1.80 (m, 2H), 1.98-2.03 (m, 2H), 2.65-2.80 (m, 1H), 3.22 (t, 2H), 3.82-3.96 (m, 2H), 4.07 (q, 2H), 7.25 (d, 2H), 7.40 (s, 1H), 7.92 (d, 2H), 8.00 (s, 1H), 8.20 (s, 1H). Exact mass calculated for $C_{19}H_{21}N_5O_6$ 415.15, found 416.2 ($MH^+$).

**Compound A35**

**1-{6-[4-(2-Methoxy-ethyl)-phenoxy]-5-nitro-pyrimidin-4-yl}-piperidine-4-carboxylic acid ethyl ester;**

**[method 1a].** Following the general procedure, compound **A35** was obtained as a yellow solid (71 %). $^1$HNMR ($CDCl_3$, 400 MHz) δ 1.28 (t, 3H), 1.70-1.80 (m, 2H), 1.98-2.03 (m, 2H), 2.65-2.75 (m, 1H), 2.94 (t, 2H), 3.22 (t, 2H), 3.37 (s, 3H), 3.62 (t, 2H), 3.96-4.03 (m, 2H), 4.15 (q, 2H), 7.05 (d, 2H), 7.25 (d, 2H), 8.17 (s, 1H). Exact mass calculated for $C_{21}H_{26}N_4O_6$ 430.19, found 431.4 ($MH^+$).

**Compound A36**

**1-[6-(4-Cyclopentyl-phenoxy)-5-nitro-pyrimidin-4-yl]-piperidine-4-carboxylic acid ethyl ester;**

**[method 1a]**. Following the general procedure, compound **A36** was obtained as a yellow solid (58%). $^1$HNMR ($CDCl_3$, 400 MHz) δ 1.28 (t, 3H), 1.58-1.92 (m, 8H), 1.98-2.15 (m, 4H), 2.65-2.75 (m, 1H), 3.00 (quintet, 1H), 3.22 (t, 2H), 3.96-4.03 (m, 2H), 4.15 (q, 2H), 7.04 (d, 2H), 7.25 (d, 2H), 8.18 (s, 1H). Exact mass calculated for $C_{23}H_{28}N_4O_5$ 440.21, found 441.2 ($MH^+$).

**Compound A37**

**1-[5-Nitro-6-(4-pyrrol-1-yl-phenoxy)-pyrimidin-4-yl]-piperidine-4-carboxylic acid ethyl ester**;

**[method 1a]**. Following the general procedure, compound **A37** was obtained as a yellow solid (77 %). $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.96-4.03 (m, 2H), 4.15 (q, 2H), 6.36 (d, 2H), 7.04 (d, 2H), 7.19 (d, 2H), 7.40 (d, 2H), 8.18 (s, 1H). Exact mass calculated for $C_{22}H_{23}N_5O_5$ 437.17, found 438.2 ($MH^+$).

**Compound A38**

**1-[6-(4-Benzoyl-phenoxy)-5-nitro-pyrimidin-4-yl]-piperidine-4-carboxylic acid ethyl ester;**

**[method 1a].** Following the general procedure, compound **A38** was obtained as a yellow solid (70 %). $^1$HNMR ($CDCl_3$,400 MHz) δ 1.28 (t, 3H), 1.80-1.96 (m, 2H), 2.00-2.08 (m, 2H), 2.65-2.75 (m, 1H), 3.22 (t, 2H), 3.96-4.03 (m, 2H), 4.15 (q, 2H), 7.24-7.28 (m, 2H), 7.46-7.51 (m, 2H), 7.58-7.62 (m, 1H), 7.78-7.82 (m, 2H), 7.87-7.90 (m, 2H), 8.21 (s, 1H). Exact mass calculated for $C_{25}H_{24}N_4O_6$ 476.17, found 477.2 ($MH^+$).

**Compound A39**

**1-{6-[4-(4-Hydroxy-benzenesulfonyl)-phenoxy]-5-nitro-pyrimidin-4-yl}-piperidine-4-carboxylic acid ethyl ester;**

**[method 1a]**. Following the general procedure, compound **A39** was obtained as a yellow solid (51 %). $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.96-4.03 (m, 2H), 4.15 (q, 2H), 6.82 (d, 2H), 7.25 (d, 2H), 7.78 (d, 2H), 7.96 (d, 2H), 8.13 (s, 1H). Exact mass calculated for $C_{24}H_{24}N_4O_8S$ 528.13, found 529.2 ($MH^+$).

**Compound A40**

**1-[6-(4'-Cyano-biphenyl-4-yloxy)-5-nitro-pyrimidin-4-yl]-piperidine-4-carboxylic acid ethyl ester;**

**[method 1a].** Following the general procedure, compound **A40** was obtained as a yellow solid. $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.96-4.03 (m, 2H), 4.15 (q, 2H), 7.24-7.26 (m, 2H), 7.61-7.78 (m, 6H), 8.19 (s, 1H). Exact mass calculated for $C_{25}H_{23}N_5O_5$ 473.17, found 473.3 ($MH^+$).

**Compound A41**

**1-[6-(2-Amino-4-ethanesulfonyl-phenoxy)-5-nitro-pyrimidin-4-yl]-piperidine-4-carboxylic acid ethyl ester;**

**[method 1a].** Following the general procedure, compound **A41** was obtained as a yellow solid. $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.14 (q, 2H), 3.40 (t, 2H), 3.96-4.03 (m, 2H), 4.18 (q, 2H), 7.18 (d, 2H), 7.68 (d, 1H), 7.72 (d, 1H), 8.06 (s, 1H), 10.35 (s, 2H). Exact mass calculated for $C_{20}H_{25}N_5O_7S$ 479.15, found 480.0 ($MH^+$).

**Compound A42**

**1-{6-[4-(5-Hydroxy-pyrimidin-2-yl)-phenoxy]-5-nitro-pyrimidin-4-yl}-piperidine-4-carboxylic acid ethyl ester;**

**[method 1a]**. Following the general procedure, compound **A42** was obtained as a yellow solid. $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.14 (q, 2H), 3.40 (t, 2H), 3.96-4.03 (m, 2H), 4.18 (q, 2H), 7.36 (d, 2H), 8.20 (s, 1H), 8.38 (d, 2H), 8.77 (s, 2H). Exact mass calculated for $C_{22}H_{22}N_6O_6$ 466.16, found 467.2 ($MH^+$).

**Compound A43**

**1-[5-Nitro-6-(4-sulfo-phenoxy)-pyrimidin-4-yl]-piperidine-4-carboxylic acid ethyl ester;**

**[method 1a].** Following the general procedure, compound **A43** was obtained as a yellow solid. $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.14 (q, 2H), 3.40 (t, 2H), 3.70-3.82 (m, 2H), 4.05 (q, 2H), 7.12 (d, 2H), 7.60 (d, 2H), 8.20 (s, 1H). Exact mass calculated for $C_{18}H_{19}N_4O_8SNa$ 474.08, found 475 ($M+H^+$).

**Compound A44**

**1-[5-Nitro-6-(4-[1,2,4]triazol-1-yl-phenoxy)-pyrimidin-4-yl]-piperidine-4-carboxylic acid ethyl ester;**

**[method 1a]**. Following the general procedure, compound **A44** was obtained as a yellow solid. $^1H$ NMR ($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.96-4.03 (m, 2H), 4.15 (q, 2H), 7.33 (d, 2H), 7.73 (d, 2H), 8.17 (s, 2H), 8.69 (s, 1H). Exact mass calculated for $C_{20}H_{21}N_7O_5$ 439.16, found 440.4 ($MH^+$).

**Compound A45**

**1-[6-(4-Carbamoylmethyl-phenoxy)-5-nitro-pyrimidin-4-yl]-piperidine-4-carboaylic** acid **ethyl ester**;

**[method 1a]**. Following the general procedure, compound **A45** was obtained as a yellow solid. $^1H$ NMR ($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.60 (s, 2H), 3.96-4.03 (m, 2H), 4.15 (q, 2H), 7.17(d, 2H), 7.38 (d, 2H), 8.17 (s, 1H). Exact mass calculated for $C_{20}H_{23}N_5O_6$ 429.16, found 430.3 ($MH^+$).

**Compound A46**

**1-{6-[4-(1,3-Dioxo-1,3-dihydro-isoindol-2-yl)-phenoxy]-5-nitro-pyrimidin-4-yl}-piperidine-4-carboxylic acid ethyl ester;**

**[method 1a].** Following the general procedure, compound **a46** was obtained as a yellow solid. $^1H$NMR ($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.60 (s, 2H), 3.96-4.03 (m, 2H), 4.15 (q, 2H), 7.26-7.29 (m, 2H), 7.52-7.55 (m, 2H), 7.82 (dd, 2H), 7.94 (dd,2H), 8.19 (s, 1H). Exact mass calculated for $C_{26}H_{23}N_5O_7$ 517.16, found 518.3 ($MH^+$).

**Compound A47**

**1-[6-(4'-Methoxy-biphenyl-4-yloxy)-5-nitro-pyrimidin-4-yl]-piperidine-4-carboxylic** acid **ethyl ester;**

**[method 1a].** Following the general procedure, compound **A47** was obtained as a yellow solid.$^1H$ NMR($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.60 (s, 2H), 3.85 (s, 3H), 3.96-4.03 (m, 2H), 4.15 (q, 2H), 6.96 (d, 2H), 7.17(d, 2H), 7.54 (d, 2H), 7.60 (d, 2H), 8.17 (s, 1H). Exact mass calculated for $C_{25}H_{26}N_4O_6$ 478.19, found 479.2 ($MH^+$).

**Compound A48**

**1-{6-[4-(2,5-Dioxo-imidazolidin-4-yl)-phenoxy]-5-nitro-pyrimidin-4-yl}-piperidine-4-carboxylic acid ethyl ester;**

**[method 1a].** Following the general procedure, compound **A48** was obtained as a yellow solid. $^1H$ NMR ($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.60 (s, 2H), 3.96-4.03 (m, 2H), 4.15 (q, 2H), 5.16 (s, 1H), 5.30 (s, 1H), 5.60 (s, 1H), 7.26 (d, 2H), 7.43 (d, 2H), 8.16 (s, 1H). Exact mass calculated for $C_{21}H_{22}N_6O_7$ 470.15, found 471.1 ($MH^+$).

**Compound A49**

**4-(4,4-Difluoro-piperidin-1-yl)-6-(2-methyl-5-trifluoromethyl-2H-pyrazol-3-yloxy)-5-nitro-pyrimidine;**

**[method 2 followed by method 3]**. Purification by HPLC yielded a yellow solid. Yield 54.71%. 1H NMR 400 MHz $CDCl_3$ δ (ppm): 8.29 (s, 1H); 6.52 (s, 1H); 3.80 (s, 3H); 3.71 (m, 2H); 2.11 (m, 2H); 2.01 (m, 4H). LCMS (ESI) m/z 409.2 ($M^+H^+$, 100%)

**Compound A50**

**1-{5-Nitro-6-[4-(4-oxo-cyclohexyl)-phenoxy]-pyrimidin-4-yl}-piperidine-4-carboxylic acid ethyl ester;**

**[method 1a].** Following the general procedure, compound **A50** was obtained as a yellow solid (45 %). $^1$HNMR ($CDCl_3$, 400 MHz) δ 1.28 (t, 3H), 1.80-2.05 (m, 6H), 2.21-2.24 (m, 2H), 2.52-2.56 (m, 2H), 2.65-2.75 (m, 1H), 3.22 (t, 2H), 3.60 (s, 2H), 3.96-4.03 (m, 2H), 4.15 (q, 2H), 7.10 (d, 2H), 7.26 (d, 2H), 8.17 (s, 1H). Exact mass calculated for $C_{24}H_{283}N_4O_6$ 468.2, found 469.2 ($MH^+$).

**Compound A51**

**1-{5-Nitro-6-[4-(3-oxo-butyl)-phenoxyl-pyrimidin-4-yl}-piperidine-4-carboxylic acid ethyl ester;**

**[method 1a].** Following the general procedure, compound **A51** was obtained as a yellow solid (61 %). $^1$HNMR ($CDCl_3$, 400 MHz) δ 1.28 (t, 3H), 1.80-1.96 (m, 2H), 2.00-2.08 (m, 2H), 2.16 (s, 3H), 2.65-2.75 (m, 1H), 2.72-2.78 (m, 2H), 2.82-2.90 (m, 2H), 3.22 (t, 2H), 3.60 (s, 2H), 3.96-4.03 (m, 2H), 4.15 (q, 2H), 7.02 (d, 2H), 7.22 (d, 2H), 8.17 (s, 1H). Exact mass calculated for $C_{22}H_{26}N_4O_6$ 442.19, found 443.0 ($MH^+$).

**Compound A52**

**1-[5-Nitro-6-(4-propionyl-phenoxy)-pyrimidin-4-yl]-piperidine-4-carboxylic acid ethyl ester;**

**[method 1a].** Following the general procedure, compound **A52** was obtained as a yellow solid (70 %). $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.01 (q, 2H), 3.22 (t, 2H), 3.96-4.03 (m, 2H), 4.15 (q, 2H), 7.21(d, 2H), 8.02 (d, 2H), 8.17 (s, 2H). Exact mass calculated for $C_{21}H_{24}N_4O_6$ 428.17, found 429.3 ($MH^+$).

**Compound A53**

**1-[5-Nitro-6-(4-[1,2,3]thiadiazol-4-yl-phenoxy)-pyrimidin-4-yl]-piperidine-4-carboxylic acid ethyl ester;**

**[method 1a].** Following the general procedure, compound **A53** was obtained as a yellow solid (57 %). $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.22 (t, 2H), 3.96-4.03 (m, 2H), 4.15 (q, 2H), 7.28 (d, 2H), 8.10 (d, 2H), 8.19 (s, 1H), 8.64 (s, 1H). Exact mass calculated for $C_{20}H_{20}N_6O_5S$ 456.12, found 457.2 ($MH^+$).

**Compound A54**

**1-{6-[4-(2-Hydroxy-ethyl)-phenoxy]-5-nitro-pyrimidin-4-yl}-piperidine-4-carboxylic acid ethyl ester;**

**[method 1a].** Following the general procedure, compound **A54** was obtained as a yellow solid. $^1$HNMR ($CDCl_3$, 400 MHz) δ 1.28 (t, 3H), 1.80-1.96 (m, 2H), 2.00-2.08 (m, 2H), 2.65-2.75 (m, 1H), 2.88 (t, 2H), 3.05 (t, 0.5H), 3.22 (t, 2H), 3.87 (t, 2H), 3.96-4.03 (m, 2H), 4.15 (q, 2H), 4.55 (t, 0.5H), 7.07(d, 2H), 7.22 (d, 2H), 8.15 (s, 1H). Exact mass calculated for $C_{20}H_{24}N_4O_6$ 416.17, found 417.3 ($MH^+$).

**Compound A55**

**{4-[6-(4,4-Difluoro-piperidin-1-yl)-5-nitro-pyrimidin-4-yloxy]-phenyl}-phenyl-methanone;**

**General Procedure 2 followed by method 1a.** Flash column chromatography [Hexane:Ethyl Acetate=1:1] provided the intermediate as a yellow oil (444 mg, 84 %). $^1$H NMR 400MHz $CDCl_3$ δ(ppm): 8.40(s, 1H); 3.70(sb, 4H); 2.1(t, 4H). Exact mass calculated for $C_9H_9ClF_2N_4O_2$ 278.04, LCMS (ESI) m/z 279.3($M+H^+$, 100%).
**[method 1a].** HPLC provided compound **A55** as yellow solid (26 mg, 59 %). $^1$H NMR 400MHz $CDCl_3$ δ(ppm): 8.25(s, 1H); 7.90(dd,2H);7.80(dd, 2H); 7.61(td, 1H); 7.50( td, 2H); 7.26(d,2H); 3.75 (sb, 4H);2.15(qu,4H). Exact mass calculated for $C_{22}H_{18}F_2N_4O_4$ 440.13, LCMS (ESI) m/z 441.3($M+H^+$, 100 %).

**Compound A56**

**3-{4-[6-(4,4-Difluoro-piperidin-1-yl)-5-nitro-pyrimidin-4-yloxy]-phenyl}-3-oxo-propionic acid methyl ester;**

**[method 1a].** HPLC provided compound **A56** as yellow solid (10 mg, 24 %). $^1$H NMR 400MHz $CDCl_3$ δ(ppm): 8.21(s,1H); 8.04(d,2H);7.24(d, 2H); 4.01(s, 2H); 3.75 (sb, 4H);2.15(q,4H); 2.00(sb, 3H). Exact mass calculated for $C_{19}H_{18}F_2N_4O_6$ 436.12, LCMS (ESI) m/z 437.3 ($M+H^+$, 100 %).

**Compound A57**

**2-[6-(4,4-Difluoro-piperidin-1-yl)-5-nitro-pyrimidin-4-yloxy]-5-ethanesulfonyl-phenylamine;**

**[method 1a].** HPLC provided compound **A57** as yellow oil (31mg, 70 %). $^1$H NMR 400MHz $CDCl_3$ δ (ppm): 10.35 (s,1H); 8.18 (s,1H); 7.85 (s,1H); 7.63 (d, 1H); 7.19 ( d, 1H); 3.78 (s, 4H); 3.16 (q, 2H); 2.20 (q,4H); 1.21 (sb,3H). Exact mass calculated for $C_{17}H_{19}F_2N_5O_5S$ 443.11, LCMS (ESI) m/z 444.3 ($M+H^+$, 100 %).

**Compound A58**

**4-(4-Cyclopentyl-phenoxy)-6-(4,4-difluoro-pipendin-l-yl)-5-nitro-pyrimidine;**

**[method 1a].** HPLC provided compound **A58** as yellow solid (20 mg, 50 %). $^1$H NMR 400 MHz $CDCl_3$ δ(ppm): 8.21 (s,1H); 7.25 (d,2H); 7.06 (d, 2H); 3.70 (s, 4H); 3.03 (q,1H); 2.10 (sb, 4h); 1.80 (d,2H); 1.71 (t,2H); 1.60 (sb, 4H). Exact mass calculated for $C_{20}H_{22}F_2N_4O_3$ 404.17, LCMS (ESI) m/z 405.2 ($M+_H^+$, 100%).

**Compound A59**

**1-[6-(2,6-Dichloro-4-methanesulfonyl-phenoxy)-5-nitro-pyrimidin-4-yl]-piperidine-4-carboxylic acid ethyl ester;**

**[method 1a].** Following the general procedure, compound **A59** was obtained as a yellow solid (52 %).$^1$H NMR ($CDCl_3$, 400 MHz) δ 1.28 (t, 3H), 1.80-1.96 (m, 2H), 2.00-2.08 (m, 2H), 2.65-2.75 (m, 1H), 3.35-4.05 (m, 16H), 4.15 (q, 2H), 7.55-7.58 (m, 2H), 7.62-7.66 (m, 1H), 7.88-7.92 (m, 2H), 7.95 (s, 1H). Exact mass calculated for $C_{24}H_{32}N_6O_6S$ 532.21, found 533.3 ($MH^+$).

**Compound A60**

**1-{6-[4-(4-Chloro-benzoyl)-phenoxy]-5-nitro-pyrimidin-4-yl}-piperidine-4-carboxylic acid ethyl ester;**

**[method 1a].** Following the general procedure, compound **A60** was obtained as a yellow solid (52 %). $^1$ENMR ($CDCl_3$, 400 MHz) δ 1.28 (t, 3H), 1.80-1.96 (m, 2H), 2.00-2.08 (m, 2H), 2.65-2.75 (m, 1H), 3.35-4.05 (m, 16H), 4.15 (q, 2H), 7.55-7.58 (m, 2H), 7.62-7.66 (m, 1H), 7.88-7.92 (m, 2H), 7.95 (s, 1H). Exact mass calculated for $C_{24}H_{32}N_6O_6S$ 532.21, found 533.3 ($MH^+$).

**Compound A61**

**1-{6-[4-(4-Hydroxy-benzoyl)-phenoxy)-5-nitro-pyrimidin-4-yl}-piperidine-4-carboxylic acid ethyl ester;**

**[method 1a].** Following the general procedure, compound **A61** was obtained as a yellow solid (52 %). $^1$H NMR ($CDCl_3$, 400 MHz) δ 1.28 (t, 3H), 1.80-1.96 (m, 2H), 2.00-2.08 (m, 2H), 2.65-2.75 (m, 1H), 3.35-4.05 (m, 16H), 4.15 (q, 2H), 7.55-7.58 (m, 2H), 7.62-7.66 (m, 1H), 7.88-7.92 (m, 2H), 7.95 (s, 1H). Exact mass calculated for $C_{24}H_{32}N_6O_6S$ 532.21, found 533.3 ($MH^+$).

**Compound A62**

**1-[6-(4-Cyanomethyl-phenoxy)-5-nitro-pyrimidin-4-yl]-piperidine-4-carboxylic acid ethyl ester;**

**[method 1a].** Following the general procedure, compound **A62** was obtained as a yellow solid (52 %). $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.35-4.05 (m, 16H), 4.15 (q,

2H), 7.55-7.58 (m, 2H), 7.62-7.66 (m, 1H), 7.88-7.92 (m, 2H), 7.95 (s, 1H). Exact mass calculated for $C_{24}H_{32}N_6O_6S$ 532.21, found 533.3 ($MH^+$).

**Compound A63**

**(4-{6-[4-(2-Methanesulfonyl-ethyl)-piperazin-1-yl]-5-nitro-pyrimidin-4-yloxy}-phenyl)-phenyl-methanone;**

**General Procedure 2 followed by method 1a.** Flash column chromatography [Methanol: Dichloromethane=1: 9] provided the intermediate monochloro compound as red oil (124 mg, 35 %). Exact mass calculated for $C_{11}H_{16}ClN_5O_4S$ 349.06, LCMS (ESI) m/z 350.1($M+H^+$, 100 %). [method 1a]. Flash column chromatography [Hexane: Ethyl Acetate=2: 1 then Methanol: Dichloromethane=1:9] provided compound **A63** as yellow oil (7 mg, 14 %).$^1$H NMR 400MHz $CDCl_3$ $\delta$ (ppm): 8.13 (d,2H); 7.91 (d,2H); 7.80 (d, 2H); 7.60 (d, 1H); 7.50 (d, 2H); 7.21 (t, 1H); 4.03 (m, 2H); 3.71 (db, 6H); 3.31 (sb, 2H); 3.08 (s,3H); 3.00 (s,2H). Exact mass calculated for $C_{24}H_{25}N_5O_6S$ 511.15, LCMS (ESI) m/z 512.1 ($M+H^+$, 100%).

**Compound A64**

**4-(4-{6-[4-(2-Methanesulfonyl-ethyl)-piperazin-1-yl]-5-nitro-pyrimidin-4-yloxy}-phenyl)-butan-2-one;**

**[method 1a].** Flash column chromatography [Hexane: Ethyl Acetate=2:1 then Methanol: Dichloromethane=1: 9] provided compound **A64** as yellow oil (6 mg, 13 %).$^1$H NMR 400MHz $CDCl_3$ $\delta$(ppm): 8.20 (s,1H); 7.21 (d,2H); 7.05 (d, 2H); 3.63 (s, 4H); 3.20 (d, 2H); 3.03 (s,3H); 2.90 (m, 4H); 2.80 (t, 2H); 2.61 (sb, 4H); 2.18 (s, 3H). Exact mass calculated for $C_{21}H_{27}N_5O_6S$ 477.17, LCMS (ESI) m/z 478.1 ($M+H^+$, 100%).

**Compound A65**

**3-(4-{6-[4-(2-Methanesulfonyl-ethyl)-piperazin-1-yl]-5-nitro-pyrimidin-4-yloxy}-phenyl)-3-oxo-propionic acid methyl ester;**

**[method 1a].** Flash column chromatography [Hexane: Ethyl Acetate=2:1 then Methanol: Dichloromethane=1: 9] provided compound **A65** as yellow oil (7 mg, 14 %). $^1$H NMR 400MHz $CDCl_3$ $\delta$(ppm): 8.20 (s,1H); 8.02 (d, 2H); 7.26 (d,2H); 4.01 (d, 2H); 3.73 (s, 7H); 3.10 (sb, 5H). Exact mass calculated for $C_{21}H_{25}N_5O_8S$ 507.14, LCMS (ESI) m/z 508.2 ($M+H^+$, 100%).

**Compound A66**

**4-(4-Methyl-piperidin-1-yl)-6-(2-methyl-5-trifluoromethyl-2H-pyrazol-3-yloxy)-5-nitro-pyrimidine;**

**[method 2 followed by general method 3]**. Purification by HPLC yielded **A66** as a yellow solid. Yield: 18 %. $^1$H NMR 400MHz $CDCl_3$ $\delta$ (ppm): 8.10 (s, 1H); 6.36 (s, 1H); 3.95 (d, 2H); 3.65 (s, 3H); 2.93 (m, 2H); 1.62 (m, 3H); 1.14 (m, 2H); 0.85 (m, 3H). LCMS (ESI) m/z 387 ($M^+H^+$, 100%)

**Compound A67**

**4-(4-Bromo-piperidin-1-yl)-6-(2-methyl-5-trifluoromethyl-2H-pyrazol-3-yloxy)-5-nitro-pyrimidine;**

**[method 2 followed by general method 3]**. Final purification by HPLC yielded an orange solid. Yield 22 %. $^1$H NMR 400MHz $CDCl_3$ $\delta$ (ppm): 8.31 (s, 1H); 6.51 (s, 1H); 4.41 (m, 1H); 3.84 (m, 2H); 3.81 (s, 3H); 3.66 (m, 2H); 2.17 (m, 2H); 1.98 (m, 2H). LCMS (ESI) m/z 453 ($M^+H^+$, 100%)

**Compound A68**

**4-(2-Methyl-5-trifluoromethyl-2H-pyrazol-3-yloxy)-5-nitro-6-(4-propyl-piperidin-1-yl)-pyrimidine;**

**[method 1a].** provided compound **A68** as yellow solid (501 mg, 55 % yield). $^1$H NMR 400MHz $CDCl_3$ $\delta$ (ppm): 8.25 (s, 1H); 6.51 (s, 1H); 4.13 (dt, 2H); 3.80 (s, 3H); 3.07 (td, 2H); 1.82 (d, 2H); 1.63-1.56 (m, 1H); 1.40-1.22 (m, 6H); 0.93(t, 3H). Exact mass calculated for $C_{17}H_{21}F_3N_6O_3$ 414.16, LCMS (ESI) m/z 415.3 ($M+H^+$, 100%).

**Compound A69**

**1-[6-(2-Methyl-5-trifluoromethyl-2H-pyrazol-3-yloxy)-5-nitro-pyrimidin-4-yl]-piperidine-4-carboxylic acid amide;**

**[method 3].** Purification by HPLC yielded a yellow solid. Yield 16 %. $^1$H NMR 400 MHz $CDCl_3$ δ (ppm): 8.17 (s, 1H); 6.43 (s, 1H); 4.06 (m, 2H); 3.72 (s, 3H); 3.12 (m, 2H); 2.58 (m, 3H); 1.88 (m, 4H). LCMS (ESI) m/z 416.1 (M+H$^+$, 100 %)

**Compound A70**

**1-[5-Nitro-6-(2-oxo-2H-chromen-6-yloxy)-pyrimidin-4-yl]-piperidine-4-carboxylic acid ethyl ester;**

**[method 1a].** Following the general procedure, compound **A70** was obtained as a yellow solid (43 %). $^1$HNMR ($CDCl_3$, 400 MHz) δ 1.28 (t, 3H), 1.80-1.96 (m, 2H), 2.00-2.08 (m, 2H), 2.65-2.75 (m, 1H), 4.02-4.05(m, 2H), 4.14 (q, 2H), 6.38 (d, 1H), 7.02 (d, 1H), 7.10 (s, 1H), 7.42 (d, 1H), 7.63 (d, 1H), 8.13 (s, 1H). Exact mass calculated for $C_{21}H_{20}N_4O_7$ 440.13, found 441.3 (MH$^+$).

**Compound A71**

**1-[5-Nitro-6-(2-oxo-benzo[1,3]oxathiol-6-yloxy)-pyrimidin-4-yl]-piperidine-4-carboxylic acid ethyl ester;**

**[method 1a].** Following the general procedure, compound **A71** was obtained as a yellow solid (34 %).$^1$H NMR ($CDCl_3$, 400 MHz) 81.28 (t, 3H), 1.80-1.96 (m, 2H), 2.00-2.08 (m, 2H), 2.65-2.75 (m, 1H), 4.02-4.05(m, 2H), 4.14 (q, 2H), 7.02 (d, 1H), 7.15 (s, 1H), 7.40 (d, 1H), 8.14 (s, 1H). Exact mass calculated for $C_{19}H_{18}N_4O_7S$ 446.09, found 447.0 (MH$^+$).

**Compound A72**

**1-[6-(9H-Carbazol-2-yloxy)-5-nitro-pyrimidin-4-yl]-piperidine-4-carboxylic acid ethyl ester;**

**[method 1a].** Following the general procedure, compound **A72** was obtained as a yellow solid (89 %). $^1$H NMR ($CDCl_3$, 400 MHz) δ 1.28 (t, 3H), 1.80-1.96 (m, 2H), 2.00-2.08 (m, 2H), 2.65-2.75 (m, 1H), 4.02-4.05(m, 2H), 4.14 (q, 2H), 6.90 (d, 1H), 7.00 (d, 1H), 7.03-7.08 (m, 1H), 7.30-7.36 (m, 2H), 7.78-7.82 (m, 2H), 8.12 (s, 1H), 8.26 (s, 1H). Exact mass calculated for $C_{24}H_{23}N_5O_5$ 461.17, found 462.3 (MH$^+$).

**Compound A73**

**1-[5-Nitro-6-(9-oxo-9H-fluoren-2-yloxy)-pyrimidin-4-yl]-piperidine-4-carboxylic acid ethyl ester**;

**[method 1a].** Following the general procedure, compound **A73** was obtained as a yellow solid (84 %). $^1$HNMR ($CDCl_3$, 400 MHz) d 1.28 (t, 3H), 1.80-1.96 (m, 2H), 2.00-2.08 (m, 2H), 2.65-2.75 (m, 1H), 4.02-4.05(m, 2H), 4.14 (q, 2H), 7.20-7.25 (m, 3H), 7.40-7.60 (m, 4H), 8.16 (s, 1H). Exact mass calculated for $C_{25}H_{22}N_4O_6$ 474.15, found 475.1 (MH$^+$).

**Compound A74**

**1-{5-Amino-6-[4-(3-oxo-butyl)-phenoxy]-pyrimidin-4-yl}-piperidine-4-carboxylic acid ethyl ester**

**General method 12:**

**A51** (348 mg, 0.786 mmol) was dissolved in ethyl acetate (10 mL), palladium on activated carbon (10wt - degussa type) was added and the mixture purged with $H_2$. The reaction was monitored by LCMS and after completion the crude was filtered through celite and activated carbon. Purification by HPLC afforded Compound **A74** as a light yellow oil. Yield: 26 %. $^1$H NMR 400MHz $CDCl_3$ δ (ppm): 8.14(s, 1H); 7.26 (d, 2H); 7:05 (d, 2H); 6.38 (s, 2H); 4.19 (m, 2H); 3.88 (d, 2H); 3.17 (m, 2H); 2.94 (m, 2H); 2.81 (m, 2H); 2,63 (m, 1H); 2.18 (s, 3H); 2.14 (d, 2H); 1.91 (m, 2H); 1.30 (t, 3H). LCMS (ESI) m/z 413.4 (M$^+$H$^+$, 100 %).

**Compound A75**

**1-[6-[4-(3-Oxo-butyl)-phenoxy]-5-(2,2,2-trifluoro-acetylamino)-pyrimidin-4-yl]-piperidine-4-carboxylic acid ethyl ester;**

**General method 13:**

**A74** (61 mg, 0,148 mmol) and diisopropylethylamine (84 ml, 0.484 mmol) were dissolved in dichloromethane and trifluoroacetic anhydride (0.51 ml, 0.363 mmol) was added. The mixture was stirred at room temperature for 3 hours and LCMS indicated the desired product. Purification by HPLC yielded light yellow oil. Yield 72.97%. $^1$H NMR 400MHz $CDCl_3$ $\delta$ (ppm): 8.21 (s, 1H); 8.14 (s, 1H); 7.20 (d, 2H); 7.00 (d, 2H); 4.15 (m, 4H); 3.13 (m, 2H); 2.89 (m, 2H); 2.77 (m, 2H); 2.57 (m, 2H); 2,15 (s, 3H); 1.99 (m, 2H); 1.77 (m, 2H); 1.26 (t, 3H). LCMS (ESI) m/z 509.2 (M+H$^+$, 100%)

**Compound A76**

**1-{5-Amino-6-[4-(hydroxy-phenyl-methyl)-phenoxy]-pyrimidin-4-yl}-piperidine-4-carboxylic acid ethyl ester;**

**[method 12].** Purification by HPLC yielded oil. Yield 8 %. $^1$H NMR 400MHz $CDCl_3$ $\delta$ (ppm): 8.05 (s, 1H); 7.38 (m, 8H); 7.12 (d, 2H); 5.86 (s, 1H); 4.18 (m, 2H); 3.99 (s, 2H); 3.80 (d, 2H); 2.94 (m, 2H); 2.55 (m, 1H); 2.09 (m, 2H); 1.88 (m,2H); 1.29 (t, 3H). LCMS (ESI) m/z 449.3 (M$^+$H$^+$, 100%)

**Compound A77**

**1-[6-(2-Benzoyl-5-methoxy-phenoxy)-5-nitro-pyrimidin-4-yl]-piperidine-4-carboxylic acid ethyl ester;**

**[method 1a].** Following the general procedure, compound **A77** was obtained as a yellow solid (52 %). $^1$HNMR ($CDCl_3$, 400 MHz) $\delta$ 1.28 (t, 3H), 1.80-1.96 (m, 2H), 2.00-2.08 (m, 2H), 2.65-2.75 (m, 1H), 3.35-4.05 (m, 16H), 4.15 (q, 2H), 7.55-7.58 (m, 2H), 7.62-7.66 (m, 1H), 7.88-7.92 (m, 2H), 7.95 (s, 1H). Exact mass calculated for $C_{24}H_{32}N_6O_6S$ 532.21, found 533.3 (MH$^+$).

**Compound A78**

**1-[6-(6-Chloro-pyridin-3-yloxy)-5-nitro-pyrimidin-4-yl]-piperidine-4-carboxylic acid ethyl ester;**

**[method 1a].** HPLC provided compound **A78** as yellow solid (63 mg, 61 %).$^1$H NMR 400MHz $CDCl_3$ $\delta$ (ppm): 8.20 (d,1H); 8.08 (s, 1H); 7.43 (dd,1H); 7.30 (d, 1H); 4.09 (q, 2H); 3.95 (dt,2H); 3.15 (td, 2H); 2.55 (m, 1H); 1.95 (dt, 2H); 1.77 (td, 2H); 1.19 (t, 3H). Exact mass calculated for $C_{17}H_{18}ClN_5O_5$407.10, LCMS (ESI) m/z 408.3 (M+H$^+$, 100 %).

**Compound A79**

**1-[6-(Benzo[1,3]dioxol-5-yloxy)-5-nitro-pyrimidin4-yl]-piperidine-4-carboxylic acid ethyl ester;**

**[method 1a].** Following the general procedure, compound **A79** was obtained as an oil (46 %). $^1$HNMR ($CDCl_3$, 400 MHz) $\delta$ 1.28 (t, 3H), 1.80-1.96 (m, 2H), 2.00-2.08 (m, 2H), 2.65-2.75 (m, 1H), 4.02-4.05(m, 2H), 4.14 (q, 2H), 6.00 (s, 2H), 6.57 (d, 1H), 6.60 (s, 1H), 6.80 (d, 1H), 8.16 (s, 1H). Exact mass calculated for $C_{19}H_{20}N_4O_7$ 416.13, found 417.0 (MH$^+$).

**Compound A80**

**1-[6-(4-Benzyloxy-phenoxy)-5-nitro-pyrimidin-4-yl]-piperidine-4-carboxylic acid ethyl ester;**

**[method 1a].** Following the general procedure, compound **A80** was obtained as a yellow solid (25 %).$^1$HNMR ($CDCl_3$, 400 MHz) d 1.28 (t, 3H), 1.80-1.96 (m, 2H), 2.00-2.08 (m, 2H), 2.65-2.75 (m, 1H), 4.02-4.05(m, 2H), 4.14 (q, 2H), 5.04 (s, 2H), 6.94-7.05 (m, 4H), 7.27-7.41 (m, 5H), 8.19 (s, 1H). Exact mass calculated for $C_{25}H_{26}N_4O_6$ 478.19, found 479.1 (MH$^+$).

**Compound A81**

**1-[6-(3-Morpholin-4-yl-phenoxy)-5-nitro-pyrimidin-4-yl]-piperidine-4-carboxylic acid ethyl ester;**

**[method 1a].** Following the general procedure, compound **A81** was obtained as an oil (84 %). $^1$HNMR ($CDCl_3$, 400 MHz) δ 1.28 (t, 3H), 1.80-1.96 (m, 2H), 2.00-2.08 (m, 2H), 2.65-2.75 (m, 1H), 3.20-3.35 (m, 6H), 3.82-3.84 (m, 2H), 3.98-4.05(m, 2H), 4.14 (q, 2H), 6.75-6.80 (m, 2H), 6.95-6.99 (m, 1H), 7.32-7.38 (m, 1H), 8.17 (s, 1H). Exact mass calculated for $C_{22}H_{27}N_5O_6$ 457.20, found 458.3 ($MH^+$).

**Compound A82**

**1-[5-Nitro-6-(4-trifluoromethylsulfanyl-phenoxy)-pyrimidin-4-yl]-piperidine-4-carboxylic acid ethyl ester;**

**[method 1a].** Following the general procedure, compound **A82** was obtained as a yellow solid (32 %). $^1$HNMR ($CDCl_3$, 400 MHz) δ 1.28 (t, 3H), 1.80-1.96 (m, 2H), 2.00-2.08 (m, 2H), 2.65-2.75 (m, 1H), 4.02-4.05(m, 2H), 4.14 (q, 2H), 7.26 (d, 2H), 7.68 (d, 2H), 8.17 (s, 1H). Exact mass calculated for $C_{19}H_{19}F_3N_4O_5S$ 472.1, found 473.1 ($MH^+$).

**Compound A83**

**1-[5-Nitro-6-(4-trifluoromethoxy-phenoxy)-pyrimidin-4-yl]-piperidine-4-carboxylic acid ethyl ester;**

**[method 1a].** Following the general procedure, compound **A83** was obtained as a yellow solid (79 %). $^1$H NMR ($CDCl_3$, 400 MHz) δ 1.28 (t, 3H), 1.80-1.96 (m, 2H), 2.00-2.08 (m, 2H), 2.65-2.75 (m, 1H), 4.02-4.05(m, 2H), 4.14 (q, 2H), 7.18 (d, 2H), 7.26 (d, 2H), 8.17 (s, 1H). Exact mass calculated for $C_{19}H_{19}F_3N_4O_6$ 456.13, found 457.1 ($MH^+$).

**Compound A84**

**1-[6-(4-Benzoyl-phenoxy)-5-(2,2,2-trifluoro-acetylamino)-pyrimidin-4-yl]-piperidine-4-carboxylic acid ethyl ester;**

**[method 13].** Purification by HPLC. $^1$H NMR 400 MHz $CDCl_3$ δ (ppm): 8.20 (s, 1H); 7.97 (s, 1H); 7.79 (d, 2H); 7.72 (d, 2H); 7.52 (m, 1H); 7.41 (m, 2H); 7.13 (d, 2H); 4.09 (m, 4H); 3.11 (m, 2H); 2.53 (m, 1H); 1.94 (m, 2H); 1.72 (m, 2H); 1.19 (t, 3H). LCMS (ESI) m/z 543.5 ($M+H^+$, 100 %)

**Compound A85**

**{4-[5-Nitro-6-(4-propyl-piperidin-1-yl)-pyrimidin-4-yloxy]-phenyl}-phenyl-methanone; General Procedure 2 followed by method 3.**

Intermediate: $^1$H NMR 400MHz $CDCl_3$ δ(ppm): 8.26(s, 1H); 4.035(d, 2H); 2.94(td, 2H); 1.73(dt, 2H); 1.50 (m, 1H); 1.27 (m, 2H); 1.18 (m, 2H); 1.12 (dd, 2H); 0.84(t, 3H). Exact mass calculated for $C_{12}H_{17}ClN_4O_2$284.10, LCMS (ESI) m/z 285.0($M+H^+$, 100%).

**[method 1].** HPLC provided compound **A85** as yellow oil (77 mg, 69 % yield). $^1$H NMR 400MHz $CDCl_3$ δ (ppm): 8.01(s, 1H); 7.71 (dd,2H); 7.62 (dd,2H); 7.41 (tt,1H); 7.30 (dt,2H); 7.07 (dt,3H); 3.92 (d, 2H); 2.86 (td,2H); 1.62 (dd, 2H); 1.39 (m, 1H); 1.14 (dt, 2H); 1.06 (t,2H); 1.06 (q, 2H); 0.72 (t,3H). Exact mass calculated for $C_{25}H_{26}N_4O_4$ 446.20, LCMS (ESI) m/z 447.2 ($M+H^+$, 100%).

**Compound A86**

**{4-Methoxy-2-[5-nitro-6-(4-propyl-piperidin-1-yl)-pyrimidin-4-yloxy]-phenyl}-phenyl-methanone;**

**[method 1].** HPLC provided compound **A86** as orange oil (67 mg, 57 % yield). $^1$H NMR 400MHz $CDCl_3$ δ (ppm): 7.88 (s, 1H); 7.50 (dd,2H); 7.40 (s,1H); 7.37 (tt,1H); 7.30 (t,1H); 7.22 (t,2H); 6.71 (dd,1H); 6.625 (d, 1H); 3.81 (d, 2H); 3.71 (s,3H); 2.81 (td,2H); 1.58 (dd, 2H); 1.36 (m, 1H); 1.16 (dt, 2H); 1.06 (td,2H); 1.01 (dd, 2H); 0.73 (t,3H). Exact mass calculated for $C_{26}H_{28}N_4O_5$ 476.21, LCMS (ESI) m/z 476.9($M+H^+$, 100 %).

**Compound A87**

**4-{4-[5-Nitro-6-(4-propyl-piperidin-1-yl)-pyrimidin-4-yloxy]-phenyl}-butan-2-one;**

**[method 1].** HPLC provided compound **A87** as yellow solid (62 mg, 59 % yield). $^1H$ NMR 400MHz $CDCl_3$ $\delta$ (ppm): 8.08 (s, 1H); 7.13 (d,2H); 6.95 (d,2H); 4.00 (d, 2H); 2.96 (td,2H); 2.82 (t,2H); 2.69 (t, 2H); 2.07 (s, 3H); 1.71 (dd, 2H); 1.48 (m, 1H); 1.27 (m, 2H); 1.17 (m, 4H); 0.83 (t,3H). Exact mass calculated for $C_{22}H_{28}N_4O_4$412.21, LCMS (ESI) m/z 413.4 ($M+H^+$, 100 %).

**Compound A88**

**5-Nitro-4-(4-propyl-piperidin-1-yl)-6-(4-[1,2,3]thiadiazol-4-yl-phenoxy)-pyrimldine;**

**[method 1].** HPLC provided compound **A88** as yellow solid (61 mg, 56 % yield). $^1H$ NMR 400 MHz $CDCl_3$ $\delta$ (ppm): 8.56 (s, 1H); 8.11 (s, 1H); 8.02 (d, 2H); 7.22 (d,2H); 4.03 (d, 2H); 2.96 (td,2H); 1.73 (dd, 2H); 1.48 (m, 1H); 1.27 (m, 2H); 1.2 (m, 4H); 0.84 (t,3H). Exact mass calculated for $C_{20}H_{22}N_6O_3$ S 426.15, LCMS (ESI) m/z 427.1 ($M+H^+$, 100 %).

**Compound A89**

**3-{4-[5-Nitro-6-(4-propyl-piperidin-1-yl)-pyrimidin-4-yloxy]-phenyl}-3-oxo-propionic acid methyl ester;**

**[method 1]**. HPLC provided compound **A89 and A89a [enol]** as yellow solid (12 mg, 4 mg, 18 % yield).$^1H$ NMR 400 MHz $CDCl_3$ $\delta$ (ppm): **A89**: 8.08 (s, 1H); 7.94 (d, 2H); 7.18 (d, 2H); 4.02 (d, 2H); 3.92 (s,2H); 3.68 (s,3H); 2.96 (td,2H); 1.72 (dd, 2H); 1.50 (m, 1H); 1.26 (m, 2H); 1.17 (m, 4H); 0.83 (t,3H). **A89a:** 8.08(s, 1H); 7.94(d, 2H); 7.75(dd, 2H); 7.12 (dd,2H); 5.57(s, 1H); 4.02 (d, 2H); 3.73 (s,3H); 2.96 (t,2H); 1.72 (d, 2H); 1.51(m, 1H); 1.26 (m, 2H); 1.18 (m, 4H); 0.83 (t,3H). Exact mass calculated for $C_{22}H_{26}N_4O_6$ 442.19, LCMS (ESI) m/z 443.3($M+H^+$, 100%).

**Compound A90**

**5-Ethanesulfonyl-2-[5-nitro-6-(4-propyl-piperidin-1-yl)-pyrimidin4-yloxy]-phenylamine;**

[method 1]. HPLC provided compound **A92** as yellow solid (60 mg, 53 % yield).$^1H$ NMR 400MHz $CDCl_3$ $\delta$ (ppm):10.2 (s, 1H); 7.97 (s, 1H); 7.63 (d, 1H); 7.61 (s, 1H); 7.11 (d, 1H); 3.13 (td, 2H); 3.04 (q, 2H); 1.81 (d, 2H); 1.59 (m, 1H); 1.28 (m, 2H); 1.2 (m, 4H); 0.84 (t, 3H). Exact mass calculated for $C_{20}H_{27}N_5O_5$ S 449.17, LCMS (ESI) m/z 450.3 ($M+H^+$, 100 %).

**Compound A91**

**4-[4-(3-Isopropyl-[1,2,4]oxadiazol-5-yl)-piperidin-1-yl]-6-(4-methanesulfonyl-phenoxy)-pyrinnidine-5-carbonitrile;**

4,6-Dichloro-pyrimidine-5-carbonitrile (254 mg, 1.47 mmol) was dissolved in DMF (3 mL). $K_2CO_3$ (203 mg, 1.47 mmol) and phenoxy methyl sulfone (253 mg, 1.47 mmol) were added to the solution at 0 ˚C. The reaction was stirred for 30 min. The completion of the reaction was judged with TLC (EtOAc: Hex =1:1, $R_f$= 0.82). After the completion of the reaction, were added the oxodiimidazol (340 mg, 1.47 mmol) and $K_2CO_3$ (406 mg, 2.94 mmol) to the reaction at 0 ˚C. The reaction was warmed to rt and stirred for 30 min. The reaction was heated to 40 ˚C and maintained for 1 h. The reaction was cooled to rt, poured in to $H_2O$ (50 mL) and extracted with EtOAc (50 mL, two times). The EtOAc was dried over $MgSO_2$ and concentrated under vacuum. The crude product was purified over $SiO_2$ (EtOAc: Hex =1:1, $R_f$= 0.39) to afford the desired compound ( 523 mg; 76.1%).$^1H$-NMR(DMSO-$d_6$): 8.32 (1H, s), 8.02 (2H, J = 4.3 Hz, d), 8.00 (2H, J = 4.3 Hz, d), 4.68 (2H, m), 3.50 (2H, m), 3.32 (1H, m), 3.44 (3H, s), 3.05 (1H, m), 2.22 (2H, m), 1.83 (2H, J = 17Hz, d), 1.25 (6H, J = 7 Hz, d) ppm. LCMS: 469.4, 384.9, 357.2.

The starting material, 4,6-Dichloro-pyrimidine-5-carbonitrile, used in the preparation of Compound **A91** was prepared in the following manner: To a solution of 5-fomyl-4,6-dichloropyrimidine (3.6 g, 20.3 mmol) in EtOAc (50 ml), was added a solution of $NH_2OH{\cdot}HCl$ (1.41 g, 20.3 mmol) in $H_2O$ (30 ml) followed by AcONa (1.67 g, 20.3 mmol) at rt. After stirring for 2h, the reaction was washed with $H_2O$ (50 ml, two times) and dried over $MgSO_4$. The EtOAc was concentrated under vacuum to afford the crude iminohydroxy compound (3.51 g, 90.2%). The crude compound was used for next step without further purification. The iminohydroxy compound (3.51 g, 18.3 mmol) was dissolved in $SOCl_2$ (20 ml) at 0 ˚C and stirred for 30 min. The reaction was warmed to rt and maintained for 3h The reaction was poured

into $H_2O$ (100 g) portionwise and stirred for 30 min. The precipitate was filtered, washed with $H_2O$ (100 mL) and dried under vacuum to afford 4,6-Dichloro-pyrimidine-5-carbonitrile (2.99g, 91%). $^1$H-NMR (DMSO-$d_6$): 8.53 ppm; LCMS: not detectable.

**Compound A92**

**1-[6-(4-Difluoromethoxy-benzyloxy)-5-nitro-pyrimidin-4-yl]-piperidine-4-carboxylic acid ethyl ester;**

**[Method 7].** The residue was purified by preparatory LCMS. $^1$H NMR, 400 MHz, $CDCl_3$, δ (ppm): 8.19 (s, 1H); 7.38 (d, 2H); 7.08 (d, 2H); 6.47 (m, 1H); 5.43 (s, 2H); 4.12 (q, 2H); 3.93 (m, 2H); 3.12 (m, 2H); 2.56 (m, 1H); 1.96 (m, 2H); 1.76 (m 2H); 1.23 (t, 3H). LCMS (ESI) for $C_{20}H_{22}F_2N_4O_6$: m/z 452 (M+H$^+$, 100 %).

**Compound A93**

**1-[6-(3-Difluoromethoxy-benzyloxy)-5-nitro-pyrimidin-4-yl]-piperidine-4-carboxylic acid ethyl ester;**

**[Method 7]:** The residue was purified by preparatory LCMS. $^1$H NMR, 400MHz, $CDCl_3$, δ (ppm): 8.16 (s, 1H); 7.29 (t, 1H); 7.17 (d, 1H); 7.11 (s, 1H); 7.00 (d, 1H); 6.45 (m, 1H); 5.40 (s, 2H); 4.08 (q, 2H); 3.89 (m, 2H); 3.08 (m, 2H); 2.52 (m, 1H); 1.92 (m, 2H); 1.74 (m 2H); 1.19 (t, 3H). LCMS (ESI) for $C_{20}H_{22}F_2N_4O_6$: m/z 452 (M+H$^+$, 100 %).

**Compound A94**

**2-{1-[6-(2-Methyl-5-trifluoromethyl-2H pyrazol-3-yloxy)-5-nitro-pyrimidin-4-yl]-piperidin-4-yl}-ethanol;**

**[methods 2 followed by 3].** Purification by HPLC yielded brownish oil. Yield 21 %.$^1$H NMR 400MHz $CDCl_3$ δ (ppm): 8.50 (s, 1H); 6.76 (s, 1H); 4.37 (d, 2H); 4.04 (s, 3H); 3.99 (m, 2H); 3.33 (t, 2H); 1.82 (m, 8H). LCMS (ESI) m/z 417 (M+H$^+$, 100 %)

**Compound A95**

**3-{1-[6-(2-Methyl-5-trifluoromethyl-2H-pyrazol-3-yloxy)-5-nitro-pyrimidin-4-yl]-piperidin-4-yl}-propionic acid;**

**[method 2 followed by 3].** Purification by HPLC yielded brownish solid. Yield 21 %. $^1$H NMR 400 MHz $CDCl_3$ δ (ppm): 8.20 (s, 1H); 6.45 (s, 1H); 4.07 (d, 2H); 3.74 (s, 3H); 3.01 (m, 2H); 2.33 (m, 2H); 2.19 (m, 2H); 1.58 (m, 4H); 1.28 (m, 4H). LCMS (ESI) m/z 459 (M+H$^+$, 100 %)

**Compound A96**

**4-[4-(4-Methyl-benzyl)-piperidin-1-yl]-6-(2-methyl-5-trifluoromethyl-2H-pyrazol-3-yloxy)-5-nitro-pyrimidine;**

**[method 2 followed by 3].** Purification by Flash Chromatography yielded an yellow oil. Yield 29 %. $^1$H NMR 400MHz $CDCl_3$ δ (ppm): 8.25 (s, 1H); 7.11 (m, 2H); 7.04 (m, 2H); 6.51 (s, 1H); 4.12 (m, 2H); 3.79 (s, 3H); 3.03 (t, 2H); 2.57 (d, 2H); 2.34 (s, 3H); 1.83 (m, 3H); 1.33 (m, 2H). LCMS (ESI) m/z 477 (M+H$^+$, 100 %)

**Compound A97**

**4-(3-Methanesulfonyl-pyrrolidin-1-yl)-6-(2-methyl-5-trifluoromethyl-2H-pyrazol-3-yloxy)-5-nitro-pyrimidine;**

**[methods 2 and 3].** Purification by HPLC yielded brownish oil. Yield 30 %. LCMS (ESI) m/z 438 (M+H$^+$, 100%)

**Compound A98**

**4-(2-Methyl-5-trifluoromethyl-2H-pyrazol-3-yloxy)-5-nitro-6-[4-(2-trifluoromethyl-phenoxy)-piperidin-1-yl]-pyrimidine;**

**[methods 2 and 3].** Purification by HPLC yielded orange solid. Yield 77 %. $^1$H NMR 400 MHz $CDCl_3$ δ (ppm): 8.29 (s, 1H); 7.1 (d, 1H); 7.51 (t, 1H); 7.02 (m, 2H); 6.54 (s, 1H); 3.82 (m, 7H); 2.09 (m, 5H). LCMS (ESI) m/z 533.1 (M+H$^+$, 100 %)

**Compound A99**

**4-(2-Methyl-5-trifluoromethyl-2H-pyrazol-3-yloxy)-5-nitro-6-[4-(pyridin-2-ylsulfanyl)-piperidin-1-yl]-pyrimidine;**

**[methods 2 and 3].** Purification by HPLC yielded **A99** as a brown oil. Yield 12 %. $^1$H NMR 400MHz $CDCl_3$ δ (ppm): 8.31 (s, 1H); 6.54 (s, 1H); 4.22 (d, 2H); 3.82 (s, 3H); 3.12 (m, 2H); 2.40 (m, 1H); 2.03 (m, 2H); 1.72 (m, 2H). LCMS (ESI) m/z 482.1 (M+H$^+$, 100 %)

**Compound A100**

**4'-(4-Benzoyl-phenoxy)-3'-nitro-3,4,5,6-tetrahydro-2H-[1,2']bipyridinyl-4-carboxylic acid ethyl ester;**

**[method 1a].** Purification by HPLC provided compound **A100** as yellow solid (43 mg, 41 %). $^1$H NMR 400MHz $CDCl_3$ δ(ppm): 7.84 (d,1H); 7.81 (s, 2H); 7.73 (d, 2H); 7.52 (t,1H); 7.41 (q,2H); 7.17 (d, 2H); 6.57 (d, 1H); 4.10 (q, 2H); 3.48 (dt,2H); 3.03 (td, 2H); 2.50 (m, 5H); 1.96 (dd, 2H); 1.81 (td, 2H); 1.21 (t, 3H). Exact mass calculated for $C_{26}H_{25}N_3O_6$475.17 LCMS (ESI) m/z 476.0 (M+H$^+$, 100%).

**Compound A101**

**3'-Nitro-4'-[4-(3-oxo-butyl)-phenoxyJ-3,4,5,6-tetrahydro-2H-[1,2']bipyridinyl-4-carboxylic acid ethyl ester;**

**[method 1].** Purification by HPLC provided compound **A101** as yellow oil (49 mg, 49 %). $^1$H NMR 400MHz $CDCl_3$ δ(ppm): 7.89 (d,1H); 7.21 (d, 2H);7.05 ( d, 2H); 6.60 (d,1H); 4.19 (q,2H); 3.56 (dt, 2H); 3.12 (td, 2H); 2.91 (t, 2H); 2.78 (t,2H); 2.59 (t, 1H); 2.17 (s, 3H); 2.04 (dd, 2H); 1.89 (m, 2H); 1.30 (t, 3H). Exact mass calculated for $C_{23}H_{27}N_3O_6$441.19 LCMS (ESI) m/z 442.5 (M+H$^+$, 100%).

**Compound A102**

**4'-[4-(2-Methoxycarbonyl-acetyl)-phenoxy]-3'-nitro-3,4,5,6-tetrahydro-2H-[1,2']bipyridinyl-4-carboxylic acid ethyl ester;**

**[method 1a].** HPLC provided compound **A102** as yellow solid (28 mg, 27 %). $^1$H NMR 400MHz $CDCl_3$ δ (ppm): 8.03 (d, 2H); 7.92 (d, 1H); 7.24 (d, 2H); 6.65 (d, 1H); 4.19 (q, 2H); 3.57 (dd, 2H); 3.12 (td, 2H); 2.62 (s, 3H); 2.59 (m, 1H); 2.05 (dd, 2H); 1.90 (m, 2H); 1.30(t, 3H). Exact mass calculated for $C_{23}H_{25}N_3O_8$ 471.16 LCMS (ESI) m/z 472.4 (M+H$^+$, 100 %).

**Compound A103**

**4'-(2-Amino-4-ethanesulfonyl-phenoxy)-3'-nitro-3,4,5,6-tetrahydro-2H-[1,2']bipyridinyl-4-carboxylic acid ethyl ester;**

**[method 1a].** HPLC provided compound **A103** as brown solid (61 mg, 57 %).$^1$H NMR 400MHz $CDCl_3$ δ(ppm): 9.83 (s,1H); 7.97 (d, 1H); 7.45 (dd, 1H); 7.10 (d,1H); 6.76 (d, 1H); 4.10 (q,2H); 3.45 (d, 2H); 3.16 (m, 4H); 2.69 (m, 1H); 1.93 (d,2H); 1.69 (t, 2H); 1.18 (t,3H); 1.12 (t, 3H). Exact mass calculated for $C_{21}H_{26}N_4O_7S$ 478.15 LCMS (ESI) m/z 479.2 (M+H$^+$, 100 %).

**Compound A104**

**4'-(4-tmidazol-1-yl-phenoxy)-3'-nitro-3,4,5,6-tetrahydro-2H-[1,2']bipyridinyt-4-carboxylic acid ethyl ester;**

**[method 1a].** HPLC provided compound **A104** as brown oil (64 mg, 65 %). $^1$H NMR 400MHz $CDCl_3$ δ (ppm): 8.85 (s, 1H); 7.90 (d, 1H); 7.57 (d, 1H); 7.53 (dd,2H); 7.45 (d, 1H); 7.41 (dd, 2H); 4.20 (q, 2H); 3.56 (d, 2H); 3.13 (td, 2H); 2.60 (m, 1H); 2.06 (dd, 2H); 1.90 (td, 2H); 1.30 (t, 3H). Exact mass calculated for $C_{22}H_{23}N_5O_5$437.17 LCMS (ESI) m/z 438.3(M+H$^+$, 100%).

**Compound A105**

**4-(2-Methyl-5-trifluoromethyl-2H-pyrazol-3-yloxy)-5-nitro-6-(4-trifluoromethyl-piperidin-1-yl)-pyrimidine;**

**[method 2 followed by 3].** Purification by HPLC yielded orange oil.. Yield 41 %. $^1$H NMR 400MHz $CDCl_3$ δ (ppm): 8.31 (s, 1H); 6.54 (s, 1H); 4.22 (d, 2H); 3.82 (s, 3H); 3.12 (m, 2H); 2.40 (m, 1H); 2.03 (m, 2H); 1.72 (m, 2H). LCMS (ESI) m/z 481.1 ($M^+H^+$, 100%)

**Compound A106**

**4-(2-Methyl-5-trifluoromethyl-2H-pyrazol-3-yloxy)-5-nitro-6-(4-phenylsulfanyl-piperidin-1-yl)-pyrimidine;**

**[method 3].** Purification by HPLC yielded **A106** as an orange solid. Yield 55 %. $^1$H NMR 400MHz $CDCl_3$ δ (ppm): 8.28 (s, 1H);7.47 (m, 2H); 7.34 (m, 2H); 6.53 (s, 1H); 4.03 (d, 2H); 3.82 (s, 3H); 3.42 (m, 1H); 3.33 (m, 2H); 2.09 (m, 3H); 1.74 (m, 2H). LCMS (ESI) m/z 481.1 ($M+H^{+\prime}$, 100 %)

**Compound A107**

**1-[6-(3-Ethynyl-phenoxy)-5-nitro-pyrimidin-4-yl]-piperidine-4-carboxylic acid ethyl ester;**

**[method 1a].** Purification by by semi preparatory HPLC afforded the pure product in 50 %. $^1$H NMR, 400MHz, $CDCl_3$, δ (ppm): 8.12 (s, 1H); 7.30 (m, 2H); 7.19 (s, 1H); 7.06 (m, 1H); 4.10 (q, 2H); 3.95 (m, 2H); 3.14 (m, 2H); 3.03 (s, 1H); 2.56 (m, 1H); 1.95 (m, 2H); 1.76 (m, 2H); 1.20 (t, 3H). LCMS (ESI) for $C_{20}H_{20}N_4O_5$: m/z 396 ($M+H^+$, 100 %).

**Compound A108**

**1-[6-(4-Chloro-2-fluoro-phenoxy)-5-nitro-pyrimidin-4-yl]-piperidine-4-carboxylic acid ethyl ester;**

**[method 1a].** Purified by semi preparatory HPLC afforded the pure product in 42 %. $^1$H NMR, 400MHz, $CDCl_3$, δ (ppm): 8.00 (s, 1H); 7.06 (m, 3H); 4.02 (q, 2H); 3.86 (m, 2H); 3.07 (m, 2H); 2.48 (m, 1H); 1.87 (m, 2H); 1.69 (m, 2H); 1.12 (t, 3H). LCMS (ESI) for $C_{18}H_{18}ClFN_4O_5$: m/z 424 ($M+H^+$, 100 %).

**Compound A109**

**1-[6-(2,4-Difluoro-phenoxy)-5-nitro-pyrimidin-4-yl]-piperidine-4-carboxylic acid ethyl ester;**

**[method1a].** purified by semi preparatory HPLC afforded the pure product in 34 %. $^1$H NMR, 400MHz, $CDCl_3$, δ (ppm): 8.14 (s, 1H); 7.15 (m, 1H); 6.89 (m, 2H); 4.14 (q, 2H); 3.98 (m, 2H); 3.19 (m, 2H); 2.60 (m, 1H); 2.00 (m, 2H); 1.82 (m, 2H); 1.24 (t, 3H). LCMS (ESI) for $C_{18}H_{18}F_2N_4O_5$: m/z 408 ($M+H^+$, 100 %).

**Compound A110**

**1-[6-(4-Bromo-2-fluoro-phenoxy)-5-nitro-pyrituidin-4-yl]-piperidine-4-carboxylic acid ethyl ester;**

**[method 1a].** purified by semi preparatory HPLC afforded the pure product in 41 %. $^1$H NMR, 400MHz, $CDCl_3$, δ (ppm): 8.07 (s, 1H); 7.26(m, 2H); 7.02 (t, 1H); 4.09(q, 2H); 3.94 (m, 2H); 3.14 (m, 2H); 2.55 (m, 1H); 1.95 (m, 2H); 1.76 (m, 2H); 1.19 (t, 3H). LCMS (ESI) for $C_{18}H_{18}BrFN_4O_5$: m/z 468 ($M+H^+$, 100 %).

**Compound A111**

**4-(3-Ethynyl-phenoxy)-5-nitro-6-(4-propyl-piperidin-1-yl)-pyrimidine;**

**[method 1a].** purified by semi preparatory HPLC afforded the pure product in 28 %. $^1$H NMR, 400MHz, $CDCl_3$, δ (ppm): 8.07 (s, 1H); 7.28 (m, 2H); 7.19 (m, 1H); 7.05 (m, 1H); 4.00 (m, 2H); 3.01 (s, 1H); 2.93 (m, 2H); 1.71 (m, 2H); 1.48 (m, 1H); 1.28 (m, 2H); 1.16 (m, 4H); 0.83 (t, 3H). LCMS (FSI) for $C_{20}H_{22}N_4O_3$: m/z 366 ($M+H^+$, 100 %).

**Compound A112**

**4-(4-Chloro-2-fluoro-phenoxy)-5-nitro-6-(4-propyl-piperidin-1-yl)-pyrimidine;**

**[method 1a].** Purified by semi preparatory HPLC afforded the pure product in 39 %. $^1H$ NMR, 400MHz, $CDCl_3$, δ (ppm): 8.04 (s, 1H); 7.13 (m, 3H); 4.01 (m, 2H); 2.94 (m, 2H); 1.71 (m, 2H); 1.49 (m, 1H); 1.28 (m, 2H); 1.16 (m, 4H); 0.82 (t, 3H). LCMS (ESI) for $C_{18}H_{20}ClFN_4O_3$: m/z 394 ($M+H^+$, 100 %).

**Compound A113**

**4-(2,4-Difluoro-phenoxy)-5-nitro-6-(4-propyl-piperidin-1-yl)-pyrimidine;**

**[method 1a].** purified by semi preparatory HPLC afforded the pure product in 54 %.$^1H$ NMR, 400MHz, $CDCl_3$, δ (ppm): 8.04 (s, 1H); 7.10 (m, 1H); 6.85 (m, 2H); 4.00 (m, 2H); 2.94 (m, 2H); 1.71 (m, 2H); 1.49 (m, 1H); 1.28 (m, 2H); 1.16 (m, 4H); 0.82 (t, 3H).LCMS (ESI) for $C_{18}H_{20}F_2N_4O_3$: m/z 378($M+H^+$, 100 %).

**Compound A114**

**4-(4-Bromo-2-fluoro-phenoxy)-5-nitro-6-(4-propyl-piperldin-1-yl)-pyrimidine;**

**[method 1a].** purified by semi preparatory HPLC afforded the pure product in 62 %.$^1H$ NMR, 400MHz, $CDCl_3$, δ (ppm): 8.15 (s, 1H); 7.35 (m, 2H); 7.12 (t, 1H); 4.10 (m, 2H); 3.10 (m, 2H); 1.81 (m, 2H); 1.59 (m, 1H); 1.36 (m, 2H); 1.26 (m, 4H); 0.93, (t, 3H). LCMS (ESI) for $C_{18}H_{20}BrFN_4O_3$: m/z 438 ($M+H^+$, 100 %).

**Compound A115**

**3'-Nitro-2'-[4-(3-oxo-butyl)-phenoxy]-3,4,5,6-tetrahydro-2H-[1,4']bipyridinyl-4-** , **carboxylic acid ethyl ester;**

**General Procedure 2:**

Intermediate monochloro as a yellow oil (128 mg, 80 % yield).Exact mass calculated for $C_{15}H_{13}ClN_2O_4$ 320.06, LCMS (ESI) m/z 320.8($M+H^+$, 100%). **[method 1a].** HPLC provided compound **A119** as yellow oil (44 mg, 50 %). $^1H$ NMR 400MHz $CDCl_3$ δ(ppm): 8.30 (d,1H); 7.42 (d, 2H); 7.18 ( d, 2H); 6.40 (d, 1H); 4.34 (q, 2H); 3.95 (dt, 2H); 3.36 (td, 2H); 3.10(t, 2H); 2.96 (t, 2H); 2.74 (m, 1H); 2.34 (s, 3H); 2.20 (dt, 2H); 2.05 (td, 2H); 1.44 (t, 3H). Exact mass calculated for $C_{23}H_{27}N_3O_6$441.19 LCMS (ESI) m/z 442.3 ($M+H^+$, 100%).

**Compound A116**

**4-[4-(3'-Nitro-4-propyl-3,4,5,6-tetrahydro-2H-[1,4']bipyridinyl-2'-yloxy)-phenyl]-butan-2-one;**

**[method 1a].** HPLC provided compound **A116** as yellow oil (34 mg, 32 %).$^1H$ NMR 400MHz $CDCl_3$ δ(ppm): 8.09 (d,1H); 7.24 (d, 2H); 7.00 (d, 2H); 6.15 (d,1H); 3.80 (d, 2H); 3.06 (td, 2H); 2.91 (t,2H); 2.77 (t, 2H); 1.77 (d, 2H); 1.51 (m, 1H); 1.36-1.22 (m, 6H); 0.8 (t,3H). Exact mass calculated for $C_{23}H_{29}N_3O_4$411.22 LCMS (ESI) m/z 412.4 ($M+H^+$, 100 %).

**Compound A117**

**2'-(4-Benzoyl-phenoxy)-3'-nitro-3,4,5,6-tetrahydro-2H-[1,4']bipyridinyl-4-carboxylic acid ethyl ester;**

**[method 1a].** HPLC provided compound **A117** as yellow oil (37 mg, 39 %). $^1H$ NMR 400MHz $CDCl_3$ δ(ppm): 8.24 (d,1H); 7.97 (d, 2H); 7.86 (d, 2H); 7.68 (t,1H);7.57(t, 2H); 7.27 (d, 2H); 6.36 (d,1H); 4.23 (q, 2H); 3.87 (dt, 2H); 3.23 (td, 2H); 2.66-2.60 (m, 1H); 2.08 (dt, 2H); 1.92 (td, 2H); 1.33 (t,3H). Exact mass calculated for $C_{26}H_{25}N_3O_6$475.17 LCMS (ESI) m/z 476.2($M+H^+$, 100%).

**Compound A118**

**4-(4-{5-Nitro-6-[4-(pyridin-2-ylsulfanyl)-piperidin-1-yl]-pyrimidin-4-yloxy}-phenyl)-butan-2-one;**

**[method 1a].** The crude was dissolved in dichloromethane and purified by preparative TLC. [$SiO_2$; 20/80 EtOAc/hexanes]. Yield 37 mg, 48 %. Yellow solid. $^1$H-NMR (400 MHz, $CDCl_3$) δ (ppm) 8.36 (d, 1H); 8.11 (s, 1H); 7.42 (ddd, 1H); 7.11 (m, 3H); 6.95 (m, 3H); 4.12 (heptet, 1H); 3.94 (tt, 2H); 3.33 (m, 2H); 2.84 (m, 2H); 2.70 (m, 2H); 2.15 (m, 2H); 2.08 (s, 3H); 1.75 (m, 2H). LCMS (ESI), m/z 480 ($M+H^+$, 100%)

**Compound A119**

**[4-(3'-Nitro-4-propyl-3,4,5,6-tetrahydro-2H-[1,4']bipyridinyl-2'-yloxy)-phenyl]-phenyl-methanone;**

**General Procedure 2**: Intermediate monochloro as a yellow oil (142 mg, 80 % yield). Exact mass calculated for $C_{18}H_{11}$ $ClN_2O_4$354.04, LCMS (ESI) m/z 355.2($M+H^+$, 100%). **[method 1a].** HPLC provided compound **A119** as yellow solid (26 mg, 29 %). $^1$H NMR 400MHz $CDCl_3$ δ(ppm): 8.33 (d,1H); 8.07 (d, 2H); 7.94 (d, 2H); 7.77 (m,1H); 7.65 (t, 2H); 7.37 (d, 2H); 6.44 (d,1H); 3.96 (d, 2H); 3.28 (td, 2H); 1.95 (d, 2H); 1.71-1.65 (m, 1H); 1.51-1.38 (m, 6H); 1.1 (t, 3H). Exact mass calculated for $C_{26}H_{27}N_3O_4$ 445.20 LCMS (ESI) m/z 446.0($M+H^+$, 100 %).

**Compound A120**

**4-(4-{5-Nitro-6-[4-(2-trifluoromethyl-phenoxy)-piperidin-1-yl]-pyrimidin-4-yloxy}-phenyl)-butan-2-one;**

**[method 2 followed by method 1].** Yield 0.173 g, 83 %. Yellow solid. $^1$H NMR 400MHz $CDCl_3$ d (ppm): 8.30 (s, 1H); 7.53 (m, 1H); 7.42 (m, 1H);6.93 (m, 2H); 4.75 (m, 1H); 3.77 (m, 2H); 3.56 (m, 2H); 1.95 (m, 4H). LCMS (ESI) m/z 403 ($M+H^+$,100%)

**[method 1a].** Compound **A120** purified through a silica plug [$SiO_2$; EtOAclhexane; 20:80]. Yield 0.067 g, 85 %. Yellow oil. $^1$H NMR 400MHz $CDCl_3$ δ (ppm): 8.12 (s, 1H); 7.53 (m, 1H); 7.42 (m, 1H); 7.15 (m, 2H); 6.97 (m, 4H); 3.72 (m, 2H); 3.60 (m, 2H); 2.82 (m, 2H); 2.70 (m, 3H); 2.08 (m, 4H); 1.97 (m, 4H). LCMS (ESI) m/z 531 ($M+H^+$,100 %)

**Compound A121**

**4-(4-{6-[4-(3-Methyl-[1,2,4] oxadiazol-5-yl)-piperidin-1-yl]-5-nitro-pyrimidin-4-yloxy}-phenyl)-butan-2-one;**

**[method 1].** Purification by HPLC. Yield 52 %. Yellow solid. $^1$H NMR 400MHz $CDCl_3$ δ (ppm): 8.11 (s, 1H);); 7.17 (d, 2H); 6.98 (d, 2H); 4.06 (d, 2H); 3.23 (t, 2H); 3.12 (d, 2H); 3.00 (m, 2H); 2,85 (t, 2H); 2.72 (t, 2H); 2.10 (s, 3H); 1.81 (m, 4H); 1,28 (m, 2H); 0.84 (t, 6H). LCMS (ESI) m/z 453 ($M+H^+$, 100 %)

**[method 1a].** Purification by HPLC yielded a yellow solid. Yield 62 %.$^1$H NMR 400MHz $CDCl_3$ δ (ppm): 8.13 (s, 1H); 7.15 (d, 2H); 6.98 (d, 2H); 4.02 (d, 2H); 3.22 (m, 3H); 2.83 (t, 2H); 2,70 (t, 2H); 2.33 (s, 3H); 2.10 (m, 5H); 1.04 (m, 2H). LCMS (ESI) m/z 453.2 ($M+H^+$, 100 %)

**Compound A122**

**(4-{6-[4-(3-Methyl-[1,2,4]oxadiazol-5-yl)-piperidin-1-yl]-5-nitro-pyrimidin-4-yloxy}-phenyl)-phenyl-methanone;**

Purification by HPLC yielded yellow solid. Yield 68 %. $^1$H NMR 400MHz $CDCl_3$ δ (ppm): 8.04 (s, 1H); 7.71 (d, 2H); 7.61 (d, 2H); 7.41 (m, 1H); 7.30 (m,2H); 7.08 (m, 2H); 3.92 (d, 2H); 3.13 (m, 3H); 2.21 (s, 3H); 2,02 (m, 2H); 1.83 (m, 2H). LCMS (ESI) m/z 487.1 ($M+H^+$, 100 %)

**Compound A123**

**1-{6-[4-(4-Fluoro-benzoyl)-phenoxy]-5-nitro-pyrimidin-4-yl}-piperidine-4-carboxylic acid ethyl ester;**

**[method 1].** HPLC provided compound **A123** as yellow solid (85 mg, 86 % yield). $^1$H NMR 400MHz $CDCl_3$ δ (ppm): 8.13 (s,1H); 7.79 (d, 2H); 7.76 (d, 2H); 7.20 (d, 2H); 7.10 (d,2H); 4.09 (q, 2H); 3.96 (dt,2H); 3.15 (td, 2H); 2.59-2.52 (m, 1H); 1.96 (dt, 2H); 1.77 (td, 2H); 1.19 (t,3H). Exact mass calculated for $C_{25}H_{23}$ $FN_4O_6$ 494.16, LCMS (ESI) m/z 495.1 ($M+H^+$, 100 %).

**Compound A124**

**(4-Fluoro-phenyl)-{4-[5-nitro-6-(4-propyl-piperidin-1-yl)-pyrimidin-4-yloxy]-phenyl}-methanone;**

**[method 1].** HPLC provided compound **A124** as yellow solid (69 mg, 84 % yield). $^1$H NMR 400MHz $CDCl_3$ $\delta$ (ppm): 8.10 (s, 1H); 7.78 (d, 2H); 7.76 (d, 2H); 7.19 (d, 2H); 7.09 (tt, 2H); 4.03 ( d, 2H); 2.96 (td, 2H); 1.73 (dd, 2H); 1.51 (m, 1H); 1.37 ( m, 2H); 1.21 (m, 2H); 1.15( m, 2H); 0.83 (t, 3H). Exact mass calculated for $C_{25}H_{25}FN_4O_4$ 464.19, LCMS (ESI) m/z 465.2 (M+H$^+$, 100%).

**Compound A125**

**4-[4-(3-Methyl-[1,2,4]oxadiazol-5-yl)-piperidin-1-yl]-6-(2-methyl-5-trifluoromethyl-2H-pyrazol-3-yloxy)-5-nitro-pyrimidine;**

**[method 3].** Purification by HPLC yielded yellow oil. Yield 38 %. $^1$H NMR 400MHz $CDCl_3$ $\delta$ (ppm): 8.16 (s, 1H); 6.38 (s, 1H); 3.98 (d, 2H); 3.65 (s, 3H); 3.21 (m, 3H); 2.26 (s, 3H); 2.08 (m, 2H); 1.89 (m, 2H). LCMS (ESI) m/z 417 (M+H$^+$, 100 %)

**Compound A126**

**4-(4-Methoxymethyl-piperldin-1-yl)-6-(2-methyl-5-trifluoromethyl-2H-pyrazol-3-yloxy)-5-nitro-pyrimidine;**

**[method 3].** Purification by HPLC yielded **A126** as a yellow oil. Yield 21 %. $^1$H NMR 400MHz $CDCl_3$ $\delta$ (ppm): 8.05 (s, 1H); 6.30 (s, 1H); 3.93 (d, 2H); 3.59 (s, 3H); 3.15 (s, 3H); 3.07 (m, 2H); 2,89 (m, 2H); 1.69 (m, 3H); 1.15 (m, 2H). LCMS (ESI) m/z 417 (M+H$^+$, 100%)

**Compound A127**

**4-{4-[6-(4-Methoxymethyl-piperidin-1-yl)-5-nitro-pyrimidin-4-yloxy]-phenyl}-butan-2-one;**

**[method 1a].** Purification by HPLC yielded **A127** as a yellow solid. Yield 19 %. $^1$H NMR 400 MHz $CDCl_3$ $\delta$ (ppm): 8.04 (s, 1H); 7.07 (d, 2H); 6.91 (d, 2H); 3.98 (d, 2H); 3.22 (s, 3H); 3.14 (d, 2H); 2.92 (m, 2H); 2,76 (m, 2H); 2.64 (m, 2H); 2.02 (s, 3H); 1.74 (m, 3H); 1,20 (m, 2H). LCMS (ESI) m/z 414.45 (M+H$^+$, 100 %)

**Compound A128**

**4-[4-(2-Methoxy-ethyl)-piperidin-1-yl]-6-(2-methyl-5-trifluoromethyl-2H-pyrazol-3-yloxy)-5-nitro-pyrimidine;**

**[method 2 followed by 1a].** Purification by HPLC yielded orange solid. Yield 41 %. $^1$H NMR 400 MHz $CDCl_3$ $\delta$ (ppm): 8.11 (s, 1H); 6.36 (s, 1H); 3.97 (d, 2H); 3.65 (s, 3H); 3.33 (m, 2H); 3.22 (s, 3H); 2,93 (m, 2H); 1.69 (m, 3H); 1.43 (m, 2H); 1.16 (m, 2H). LCMS (ESI) m/z 431.1 (M+H$^+$, 100 %)

**Compound A129**

**4-{4-[6-(4-Ethoxymethyl-piperidin-1-yl)-5-nitro-pyrimidin-4-yloxy]-phenyl}-butan-2-one;**

**[method 1a] A129** was purified by HPLC. $^1$H NMR 400MHz $CDCl_3$ $\delta$ (ppm): 8.00 (s, 1H), 7.10 (d, 2H), 6.80 (d, 2H), 3.90 (m, 2H), 3.30 (q, 2H), 3.15 (d, 2H), 2.90 (m, 2H),2.70 (t, 2H), 2.60 (t, 2H), 2.00 (s, 3H) 1.70 (m, 3H), 1.20 (m, 2H), 1.00 (t, 2H). LCMS (ESI) for $C_{22}H_{28}N_4O_5$: m/z 429.0 (M + H$^+$, 100 %)

**Compound A130**

**4-(2,4-Difluoro-phenoxy)-5-nitro-6-[4-(pyridin-2-ylsnlfanyl)-piperidin-1-yl]-pyrimidine; [method 1a] A130** was purified by semi preparatory HPLC afforded the pure product in 73

%.$^1$H NMR, 400MHz, $CDCl_3$, $\delta$ (ppm): 8.67 (d, 1H); 8.10(s, 1H); 7.80 (t, 1H); 7.42 (d, 1H); 7.31(t, 1H); 7.11 (m, 1H); 6.88 (m, 2H); 4.12 (m, 1H); 3.99 (m, 2H); 3.29 (m, 2H); 2.14 (m, 2H); 1.76 (m, 2H). LCMS (BSI) for $C_{20}H_{17}F_2N_5O_3S$: m/z 445 (M+H$^+$, 100 %).

**Compound A131**

**(4-Metboxy-2-{5-nitro-6-[4-(pyridin-2-ylsulfanyl)-piperidin-1-yl]-pyrimidin-4-yloxy}-phenyl)-phenyl-methanone;**

**[method 1a]** Purified by semi preparatory LCMS afforded the pure product in 38 %. $^1$H NMR, 400 MHz, $CDCl_3$, δ (ppm): 8.55 (d, 1H); 7.98 (s, 1H); 7.62 (m, 3H); 7.46 (m, 2H); 7.31 (m, 3H); 7.15 (m, 1H); 6.80 (m, 1H); 6.72 (d, 1H); 4.05 (m, 1H); 3.86 (m, 2H); 3.82 (s, 3H, $-OCH_3$); 3.22 (m, 2H); 2.09 (m, 2H); 1.70 (m, 2H). LCMS (ESI) for $C_{28}H_{25}N_sO_sS$: m/z 543 ($M+H^+$, 100 %).

**Compound A132**

**4-(2,4-Difluoro-phenoxy)-6-(4-ethoxymethyl-piperidin-1-yl)-5-nftro-pyrimidine;**

**[method 1].** crude product was purified by HPLC to afford yellow oil (35.4 mg, 27 %). $^1$H NMR 400MHz $CDCl_3$ δ (ppm): 8.20 (s, 1H), 7.20 (m, 1H), 6.80 (d, 2H),7.00-6.90 (m, 2H),4.10 (m, 2H), 3.50 (d, 2H), 3.10 (m, 2H), 2.00 (m, 1H), 1.90 (m, 2H), 1.40 (m, 2H), 1.20 (t, 3H). LCMS (ESI) for $C_{18}H_{20}FN_4O_4$: m/z 395.1 ($M + H^+$, 100 %)

**Compound A133**

**4-{4-[6-(4-Cyclopropylmethoxymethyl-piperidin-1-yl)-5-nitro-pyrimidin-4-yloxy]-phenyl}-butan-2-one;**

**[method 2 followed by method 1a].** Purification by HPLC yielded yellow solid. Yield 53 %. $^1$H NMR 400 MHz $CDCl_3$ δ (ppm): 7.97 (s, 1H);); 7.02 (d, 2H); 6.84 (d, 2H); 3.92 (d, 2H); 3.13 (m, 2H); 3.07 (m, 2H); 2.86 (m, 2H); 2,71 (m, 2H); 2.57 (m, 2H); 1.56 (s, 3H); 1.70 (m, 3H); 1.13 (m, 2H); 0.85 (m, 1H); 0.34 (m, 2H); 0.01 (m, 2H). LCMS (ESI) m/z 455.2 ($M+H^+$, 100 %)

**Compound A134**

**4-{4-[5-Nitro-6-(4-propoxymethyl-piperidin-1-yl)-pyrimidin-4-yloxy]-phenyl}-butan-2-one;**

**[method 2 followed by 1a].** Purification by HPLC yielded yellow solid. Yield 22 %.$^1$H NMR 400MHz $CDCl_3$ δ (ppm): 8.03 (s, 1H);); 7.08 (d, 2H); 6.90 (d, 2H); 3.98 (d, 2H); 3.24 (t, 2H); 3.16 (d, 2H); 2.92 (m, 2H); 2,76 (m, 2H); 2.63 (m, 2H); 2.01 (s, 3H); 1.74 (m, 3H); 1.45 (m, 2H); 1.19 (m, 2H); 0.78 (t, 3H). LCMS (ESI) m/z 443.3 ($M+H^+$, 100 %)

**Compound A135**

**1-{4-[6-(4-Methoxymethyl-piperidin-1-yl)-5-nitro-pyrimidin-4-yloxyl-phenyl}-ethanone;**

**[method 1a].** Yield 12 %.$^1$H NMR 400MHz $CDCl_3$ δ (ppm): 8.03 (s, 1H);); 7.89 (d, 2H); 7.10 (d, 2H); 3.99 (d, 2H); 3.21 (t, 3H); 3.13 (m, 2H); 2.93 (m, 2H); 2,47 (s, 3H); 1.75 (m, 3H); 1.19 (m, 2H). LCMS (ESI) m/z 387 ($M^+H^+$, 100%)

**Compound A136**

**4-{4-[2-Nitro-3-(4-propyl-piperidin-1-yl)-phenoxyl-phenyl)-butan-2-one;**

Following general procedure 1, compound **A136** was obtained as a yellow oil (70 %). $^1$HNMR ($CDCl_3$, 400 MHz) δ 0.70 (t, 3H), 1.04-1.17 (m, 7H), 1.53(d, 2H), 1.97 (s, 3H), 2.54-2.70 (m, 6H), 3.10(d, 2H), 6.36 (d, 2H), 6.69 (d, 1H), 6.77 (d, 2H), 6.96 (d, 2H ), 7.04(t,1H). Exact mass calculated for $C_{24}H_{30}N_2O_4$ 410.22, found 411.2 ($MH^+$).

**Compound A137**

**1-{4-[2-Nitro-3-(4-propyl-piperidin-1-yl)-phenoxy]-phenyl}-ethanone;**

Following the general procedure 1, compound **A137** was obtained as a yellow solid (11 %) $^1$HNMR ($CDCl_3$, 400 MHz) δ 0.82 (t, 3H), 1.17-1.28 (m, 7H), 1.64 (d, 2H), 2.50 (s, 3H), 2.67 (t, 2H), 3.20 (d, 2H), 6.58 (d, 1H), 6.89 (d, 1H), 6.98 (d, 2H), 7.24 (t, 1H), 7.88 (d, 2H). Exact mass calculated for $C_{22}H_{26}N_2O_4$ 382.19, found 383.3 ($MH^+$).

**Compound A138**

**{4-[2-Nitro-3-(4-propyl-piperidin-1-yl)-phenoxy]-phenyl}-phenyl-methanone;**

The intermediate monofluoro was made by method 2. It was obtained as a yellow solid (88 % yield). $^1$HNMR ($CDCl_3$, 400 MHz) δ 0.88 (t, 3H), 1.24-1.33 (m, 7H), 1.71 (m, 2H), 2.74 (t, 2H), 3.23 (d, 2H), 6.77(t, 1H), 6.87 (d, 1H), 7.30 (d, 1H). Exact mass calculated for $C_{14}H_{19}FN_2O_2$ 266.14, found 297.0 ($MH^+$).

Following the general procedure 2, compound **A138** was obtained as a yellow oil (72 %). $^1$HNMR ($CDCl_3$, 400 MHz) δ 0.75 (t, 3H), 1.10-1.24 (m, 7H), 1.59-1.62 (m, 2H), 2.67 (t, 2H), 3.20 (d, 2H), 6.59 (d, 1H), 6.88 (d, 1H), 6.95 (d, 2H), 7.21 (t, 1H), 7.34 (t, 2H), 7.44 (t,1H), 7.63 (d, 2H), 7.68 (d, 2H). Exact mass calculated for $C_{27}H_{28}N_2O_4$ 444.20, found 445.1($MH^+$).

**Compound A139**

**3-{4-[2-Nitro-3-(4-propyl-piperidin-1-yl)-phenoxy]-phenyl}-3-oxo-propionic acid methyl ester;**

**[method 1].** Following the general procedure, compound **A139** was obtained as a yellow solid (6 %). $^1$BNNM ($CDCl_3$, 400 MHz) δ 0.71 (t, 3H), 0.99-1.16 (m, 7H), 1.54 (m, 2H), 2.33-2.35 (m, 2H), 2.62 (m, 2H), 3.68 (s, 3H), 6.74 (d, 1H), 6.96 (d, 2H), 7.06 (d, 1H), 7.37 (t, 1H), 7.83(d, 2H). Exact mass calculated for $C_{24}H_{28}N_2O_6$ 440.19, found 399.2 ($MH^+$).

**Compound A140**

**4-{4-[6-(4-Butozymethyl-piperidin-1-yl)-5-nitro-pyrimidin-4-yloxy]-phenyl}-butan-2-one;**

**[method 1a].** Purification by HPLC yielded yellow oil. Yield 41 %. $^1$H NMR 400MHz $CDCl_3$ δ (ppm): 8.03 (s, 1H);); 7.08 (d, 2H); 6.89 (d, 2H); 3.97 (d, 2H); 3.27 (t, 2H); 3.16 (d, 2H); 2.90 (m, 2H); 2,76 (t, 2H); 2.63 (t, 2H); 2.02 (s, 3H); 1.74 (m, 1H); 1,70 (d, 2H); 1,41 (m, 2H); 1,22 (m, 4H); 0.78 (t, 3H). LCMS (ESI) m/z 457 ($M^+H^+$, 100%)

**Compound A141**

**4-{4-[6-(4-Isobutoxymethyl-piperidin-1-yl)-5-nitro-pyrimidin-4-yloxy]-phenyl}-butan-2-one;**

**[method 1a].** Purification by HPLC yielded yellow oil. Yield 45 %. $^1$H NMR 400MHz $CDCl_3$ δ (ppm): 8.11 (s, 1H);); 7.17 (d, 2H); 6.98 (d, 2H); 4.06 (d, 2H); 3.23 (t, 2H); 3.12 (d, 2H); 3.00 (m, 2H); 2,85 (t, 2H); 2.72 (t, 2H); 2.10 (s, 3H); 1.81 (m, 4H); 1,28 (m, 2H); 0.84 (t, 6H). LCMS (ESI) m/z 457 ($M^+H^+$, 100 %)

**Compound A142**

**(4-Fluoro-phenyl)-[4-(3'-nitro-4-propyl-3,4,5,6-tetrahydro-2H-[1,2']bipyridinyl-4'-yloxy)-phenyl]-methanone;**

General Procedure 2 provided the monochloro intermediate as yellow crystal (484 mg, 66 % yield).$^1$H NMR 400MHz $CDCl_3$ δ (ppm): 8.21(d, 1H); 6.91(d, 1H); 3.62(d, 2H); 3.09 (td, 2H); 1.89 (d,2H); 1.61 (m, 1H); 1.50-1.34 (m, 6H); 1.03 (t, 3H). Exact mass calculated for $C_{13}H_{18}ClN_3O_2$283.11, LCMS (ESI) m/z 284.3(M+H$^+$, 100%).

**[method 1a].** HPLC provided compound **A142** as tan solid (34 mg, 37 % yield). $^1$H NMR 400MHz $CDCl_3$ δ (ppm): 7.83 (d, 1H); 7.78 (d, 2H); 7.75 (d, 2H); 7.15 (d, 2H); 7.11 (d, 2H); 6.58 ( d, 1H); 3.51 (d, 2H); 2.97 (td, 2H); 1.73 (dd, 2H); 1.49-1.42 (m, 1H); 1.31-1.17 (m, 6H); 0.84 (t, 3H). Exact mass calculated for $C_{26}H_{26}FN_3O_4$ 463.19, LCMS (ESI) m/z 464.0(M+H$^+$ 100%).

**Compound A143**

**4-[4-(3'-Nitro-4-propyl-3,4,5,6-tetrahydro-2H-[1,2']bipyridinyl-4'-yloxy)-phenyl]-butan-2-one;**

**[method 1a].** HPLC provided compound **A143** as yellow solid (57 mg, 70 % yield).$^1$H NMR 400MHz $CDCl_3$ δ (ppm): 7.75 (d, 1H); 7.11 (d, 2H); 6.95 (d, 2H); 6.46 (d, 2H); 3.46 (d, 2H); 2.90 (td, 2H); 2.81 (t, 2H); 2.68(t, 2H); 2.07 (s, 3H); 1.69 (m, 2H); 1.43-1.38 (m, 1H); 1.29-1.16 ( m, 6H); 0.83 (t,3H). Exact mass calculated for $C_{23}H_{29}N_3O_4$ 411.22, LCMS (ESI) m/z 412.0 (M+H$^+$, 100%).

**Compound A144**

**3'-Nitro-4-propyl-4'-(4-[1,2,4]triazol-1-yl-phenoxy)-3,4,5,6-tetrahydro-2H-[1,2']bipyridinyl;**

**[method 1a].** HPLC provided compound **A144** as yellow solid (46 mg, 56 % yield).$^1$H NMR 400MHz $CDCl_3$ δ (ppm): 8.62(s, 1H); 8.08 (s, 1H); 7.72(d, 1H); 7.58 (d, 2H); 7.18 (d, 2H); 6.49 (d, 1H); 3.43(d, 2H); 2.89 (td, 2H); 1.66 (d, 2H); 1.42-1.35 (m, 1H); 1.25-1.11 (m, 6H); 0.79 (t,3H). Exact mass calculated for $C_{21}H_{24}N_6O_3$ 408.19, LCMS (ESI) m/z 409.0 (M+H$^+$, 100%).

**Compound A145**

**1-{2-Nitro-3-[4-(3-oxo-butyl)-phenoxy]-phenyl}-piperidine-4-carboxylic acid ethyl ester;**

The intermediate monofluoro was made by method 2. It was obtained as a yellow solid (90 % yield). $^1$HNMR ($CDCl_3$, 400 MHz) δ 1.20 (t, 3H), 1.77-1.93 (m, 4H), 2.35 (m, 1H), 2.73-2.79 (t, 2H), 3.18-3.22 (m, 2H), 4.08 (q, 2H), 6.77-6.86 (m, 2H), 7.25-7.31 (m, 1H). Exact mass calculated for $C_{14}H_{17}FN_2O_4$ 296.12, found 297.2. Following general **procedure 1**, compound A145 was obtained as a yellow oil (61 %). $^1$HNMR ($CDCl_3$, 400 MHz) δ 1.19 (t, 3H), 1.77-1.83 (m, 2H), 1.88-1.92(m, 2H), 2.07(s, 3H), 2.30-2.33 (m, 1H), 2.66-2.82(m, 6H), 3.18-3.22(m, 2H), 4.07 (q, 2H), 6.50 (d, 1H), 6.79 (d, 1H ), 6.88(d, 2H), 7.08(d, 2H), 7.16(t,1H). Exact mass calculated for $C_{24}H_{28}N_2O_6$ 440.19, found 441.1 (MH$^+$).

**Compound A146**

**1-[3-(4-Benzoyl-phenoxy)-2-nitro-phenyl]-piperidine-4-carboxylic acid ethyl ester;**

**[method 2].** Intermediate was obtained as a yellow solid (90 % yield). $^1$HNMR ($CDCl_3$, 400 MHz) δ 1.20 (t, 3H), 1.77-1.93 (m, 4H), 2.35 (m, 1H), 2.73-2.79 (t, 2H), 3.18-3.22 (m, 2H), 4.08 (q, 2H), 6.77-6.86 (m, 2H), 7.25-7.31 (m, 1H). Exact mass calculated for $C_{14}H_{17}FN_2O_4$ 296.12, found 297.2 (MH$^+$). Following the general procedure 1, compound **A146** was obtained as a yellow solid (44 %). $^1$HNMR ($CDCl_3$, 400 MHz) δ 1.20 (t, 3H), 1.80-1.91 (m, 4H), 2.37 (m, 1H), 2.74-2.81(m, 2H), 3.22-3.25 (m, 2H), 4.10 (q, 2H), 6.68(d, 1H), 6.93 (d, 1H), 7.02 (d, 2H), 7.29 (t, 1H ), 7.42(t, 1H), 7.52(d, 1H), 7.70(d, 2H), 7.76(d, 2H). Exact mass calculated for $C_{27}H_{26}N_2O_6$ 474.18, found 475.2 (MH$^+$).

**Compound A147**

**{4-[6-(4-Ethoxy-piperidin-1-yl)-5-nitro-pyrimidin-4-yloxy]-phenyl}-(4-fluoro-phenyl)-methanone;**

**[method 1a].** Purification by HPLC. Yield 24 %. $^1$H NMR 400MHz $CDCl_3$ δ (ppm): 8.03 (s, 1H);); 7.66 (m, 4H); 7.07 (m, 2H);6.98 (m, 2H); 3.65 (m, 2H); 3.48 (m, 1H); 3.39 (q, 2H); 3.25 (m, 2H); 1.77 (m, 2H); 1.56 (m, 2H); 1.06 (t, 3H). LCMS (ESI) m/z 467 (M$^+$H$^+$, 100%)

**Compound A148**

**1-[6-(2-Methyl-5-trifluoromethyl-2H-pyrazol-3-yloxy)-5-nitro-pyrimidin-4-yl]-piperidin-4-ol**

**[method 3].** Purification by HPLC. Yield 35 % yellow oil. $^1$H NMR 400MHz DMSO δ (ppm): 8.26 (s, 1H); 6.67 (s, 1H); 4.82 (s, 1H); 3.75 (m, 2H); 3.68 (s, 3H); 3.31 (m, 2H); 1.76 (m, 2H); 1.41 (m, 2H). LCMS (ESI) m/z 389 (M$^+$H$^+$, 100%)

**Compound A149**

**1-[6-(4-Acetyl-phenoxy)-5-nitro-pyrimidin-4-yl]-piperidine-4-carboxylic** acid ethyl ester.

**[method 1a].** The mixture was purified by HPLC to give compound **A149** as a yellow solid (57 mg, 70 %). $^1$HNMR ($CDCl_3$, 400 MHz) δ 1.28 (t, 3H), 1.80-1.90 (m, 2H), 1.98-2.03 (m, 2H), 2.62 (s, 3H), 3.22 (t, 2H), 3.97-4.03 (m, 2H), 4.17 (q, 2H), 7.25 (d, 2H), 8.02 (d, 2H), 8.17 (s, 1H). Exact mass calculated for $C_{20}H_{22}N_4O_6$ 414.15, found 415.2 (MH$^+$).

**Compound A150**

**(1-{6-[4-(4-Fluoro-benzoyl)-phenoxy]-5-nitro-pyrimidin-4-yl}-piperidin-4-yl)-(4-fluorophenyl)-methanone**

**[method 1a]**. Purification by HPLC. Yield 40 % of a yellow solid as TFA salt. $^1H$ NMR 400MHz $CDCl_3$ δ (ppm): 10.0 (s, 1H); 8.19 (s, 1H); 8.01 (m, 2H); 7.91 (m, 2H); 7.76 (m, 4H); 7.19 (m, 2H); 7.09 (m, 4H); 4.0.6 (d, 2H); 3.52 (m, 1H); 3.25 (m, 2H); 1.88 (m, 4H). LCMS (ESI) m/z 545.4 ($M^+H^+$, 100 %)

**Compound A151**

**4-(4-{6-[4-(4-Fluoro-benzoyl)-piperidin-1-yl]-5-nitro-pyrimidin-4-yloxy}-phenyl)-butan-2-one**

**[method 1a].** Purification by HPLC yielded yellow solid. Yield 62 %. $^1H$ NMR 400MHz $CDCl_3$ δ (ppm): 8.25 (s, 1H); 8.04 (m, 2H); 7.26 (m, 4H); 7.10 (d, 2H); 4.19 (m, 2H); 3.61 (m, 1H);3.35 (m, 2H); 2.96(t; 2H); 2.83 (t, 2H); 2.21 (s, 3H); 2.00 (m, 4H). LCMS (ESI) m/z 493.4 ($M^+H^+$, 100%)

**Compound A152**

**4-(4-Methanesulfonyl-phenoxy)-5-nitro-6-[4-(pyridin-2-ylsulfanyl)-cyclohexyl]-pyrimidine**

**[method 1a]**. The crude was dissolved in DMF and purified by HPLC.Yellow solid. Yield 66 mg, 72 %. $^1H$-NMR (400 MHz, $CDCl_3$) δ(ppm): 8.43 (m, 1H); 8.12 (s, 1H); 7.94 (tt, 2H); 7.49 (ddd, 1H); 7.28 (tt, 2H), 7.15 (m, 1H); 7.01 (m, 1H); 4.10 (heptet, 1H); 3.96 (tt, 2H); 3.34 (m, 2H); 3.00 (s, 3H); 2.15 (m, 2H); 1.75 (m, 2H). LCMS (ESI), m/z 488 (M+H+, 100%)

**Compound A153**

**4-(4-Methanesulfonyl-phenoxy)-5-nitro-6-[4-(pyridin-4-ylsulfanyl)-cyclohexyl]-pyrimidine**

**[method 1a].** The crude was dissolved in dichloromethane and purified by HQLC. Yellow solid. Yield 85 mg, 87 %.$^1H$-NMR (400 MHz, $CDCl_3$) δ(ppm): 8.41 (m, 2H); 8.10(s, 1H); 7.94 (m, 2H); 7.79 (m, 2H); 7.33 (m, 2H); 4.02 (m, 3H); 3.37 (m, 2H); 3.06 (s, 3H); 2.20 (m, 2H); 1.78 (m, 2H). LCMS (ESI), m/z 488 ($M+H^+$, 100 %)

**Compound A154**

**4-(4-Methanesulfonyl-phenoxy)-5-nitro-6-(4-phenylsulfanyl-cyclohexyl)-pyrimidine**

**[method 1a].** The crude was dissolved in dichloromethane and purified by HPLC. Yellow solid. Yield 80 mg, 83 %.$^1H$-NMR (400 MHz, $CDCl_3$) δ(ppm): 7.97 (s, 1H); 7.78 (m, 2H); 7.21 (m, 2H); 7.15 (m, 3H); 7.08 (m, 2H); 3.80 (m, 2H); 3.16 (heptet, 1H); 3.06 (m, 2H); 2.86 (s, 3H); 1.87 (m, 2H); 1.50 (m, 2H). LCMS (ESI), m/z 487 ($M+H^+$, 100%)

**Compound A155**

**1-[5-Nitro-6-(4-trifluoromethylsulfanyl-phenoxy)-pyrimidin-4-yl]-piperidine-4-carboxylic acid ethyl ester**

**[method 1a].** yellow solid (92 %). $^1H$ NMR ($CDCl_3$, 400 MHz) δ 1.28 (t, 3H), 1.79 (m, 2H), 1.98 (m, 2H), 2.58 (m, 1H), 3.18 (t, 2H), 3.85 (m, 2H), 4.10 (q, 2H), 7.58 (d, 2H), 7.67 (d, 1H), 8.09 (s, 1H), 10.13 (s, 1H). Exact mass calculated for $C_{19}H_{20}F_3N_5O_4S$ 471.45, found 472.1 ($MH^+$).

**Compound A156**

**5-[1,3]Dioxolan-2-yl-4-[4-(3-isopropyl-[1,2,4]oxadiazol-5-yl)-piperidin-1-yl]-6-(4-methanesulfonyl-phenoxy)-pyrimidine**

Using the method described herein Compound **A156** was prepared. The crude mixture was purified by flash chromatography, eluted with 50% ethyl acetate/hexane to afford Compound A156 (316.5mg, 67.8%). $^1H$ NMR 400MHz $CDCl_3$ δ (ppm): 8.24 (s, 1H), 7.93 (d, 2H), 7.26(d, 2H), 5.95(s, 1H), 4.10 (m, 2H), 3.96 (m, 2H), 3.17 (q, 1H), 3.02 (s, 3H), 2.14 (m, 2H), 1.95 (m, 2H), 1.28 (d, 6H). LCMS (ESI) for $C_{24}H_{29}N_5O_6S$: m/z 516.3 ($M + H^+$, 100%)

**Compound A157**

**4-[4-(3-Isopropyl-[1,2,4]oxadiazol-5-yl)-piperidin-1-yl]-6-(4-methanesulfonyl-phenoxy)-pyrimidine-5-carbaldehyde**

Compound **A156** was hydrolyzed using HCl aq in a mixture of acetone/acetonitrile to give Compound **A157** (30.0mg, 93.72%); $^1$H NMR 400MHz $CDCl_3$ δ (ppm):10.3(s,1H), 8.17 (s, 1H), 7.96 (d, 2H), 7.32(d, 2H), 4.12 (m, 2H), 3.30 (m, 2H),3.22 (m,1H), 3.00(s, 3H), 3.00 (q,1H), 2.06 (m, 2H), 2.00 (m, 2H), 1.27 (d, 6H). LCMS (ESI) for $C_{22}H_{25}N_5O_5S$: m/z 472.2 (M + $H^+$, 100%).

**Compound A158**

**5-[1,3]Dioxolan-2-yl-4-[4-(3-isopropyl-[1,2,4]oxadiazol-5-yl)-piperidin-1-yl]-6-(4-[1,2,3]thiadiazol-4-yl-phenoxy)-pyrimidine**

White solid, $^1$H NMR 400MHz $CDCl_3$ δ (ppm): 8.62 (s, 1H), 8.31(s,1H), 8.08(d, 2H), 7.28(d, 2H), 6.06(s, 1H), 4.24 (m, 2H), 4.13 (m, 2H), 4.04 (m, 2H), 3.23 (m, 2H), 3.07(q, 1H), 2.20 (m, 2H), 2.02 (m, 2H), 1.33 (d, 6H); LCMS (ESZ) for $C_{25}H_{27}N_7O_4S$: m/z 522.3 (M + $H^+$, 100%).

The intermediate 4-chloro-5-[1,3]dioxolan-2-yl-6-(4-[1,2,3]thiadiazol-4-yl-phenoxy)-pyrimidine was prepared using the following method: Phosphorus Oxychoride (200 mL, 2184.8mmo1) was added drop wise (additional funnel) in DMF at 0 ˚C, and stirred for 1 hour, treated with 4,6 dihydroxypyridimidine (50.0g, 446.1 mmol) and stirred for half hour at room temperature. The heterogeneous mixture was refluxed for 3 hours. The volatiles were removed at reduce pressure, and the residue was poured in ice water and extract with chloroform and diethylether, wash with sodium bicarbonate and concentrate under high vacuum. The resulting mixture was purified on silica ($CH_2Cl_2$) to afford 4,6-dichloro-pyrimidine-5-carbaldehyde as a yellow solid (54.0 g). $^1$H NMR 400MHz $CDCl_3$ δ (ppm): 10.3 (s, 1H, aldehyde), 8.7 (s,1H, pyrimidine).

Compound 4,6-Dichloro-pyrimidine-5-carbaldehyde (8.6g, 0.049mmol), anhydrous ethylene glycol (8.2ml) andp-Toluene-sulfonic acid (150mg) were mixture in benzene (200 mL) and heated under reflux for 3 hours. Concentrate under high vacuum, worked up with chloroform, water, sodium bicarbonate and sodium chloride, concentrate. The reaction mixture was purified on silica ($CH_2Cl_2$) to give 4,6-Dichloro-5-[1,3]dioxolan-2-yl-pyrimidine (8.86g, 82.5%). $^1$H NMR 400MHz $CDCl_3$ δ (ppm): 8.8 (s, 1H), 6.3 (s,1H), 4.3 (m, 2H,), 4.1(m, 2H).

To a mixture of 4,6-dichloro-5-[1,3]dioxolan-2-yl-pyrimidine (100.0 mg, 0.45mmol) and added Potassium Carbonate (80.62mg, 0.45mmo1) in DMF (5mL) cooled to 0˚ C was added a solution of 4-[1,2,3]-thiadiazol-4-yl-phenol (DMF) drop wise. The resulting mixture was stirred at room temperature for 30 minutes to give 4-chloro-5-[1,3]dioxolan-2-yl-6-(4-[1,2,3]thiadiazol-4-yl-phenoxy)-pyrimidine. LCMS (ESI) for $C_{15}H_{11}ClN_4O_3S$: m/z 362.9 (M + $H^+$, 100%).

**Compound A159**

**4-[4-(3-Isopropyl-[1,2,4]oxadiazol-5-yl)-piperidin-1-yl]-6-(4-[1,2,3]thiadiazol-4-yl-phenoxy)-pyrimidine-5-carbaldehyde**

White solid (18.01 mg,25.9%); $^1$H NMR 400MHz $CDCl_3$ δ (ppm):10.7(s, 1H), 8.67 (s, 1H), 8.26(s,1H), 8.14(d, 2H), 7.34(d, 2H), 4.19 (m, 2H), 3.40 (m, 2H), 3.26 (m, 1H), 3.07(q, 1H), 2.20 (m, 2H), 2.02 (m, 2H), 1.33 (d, 6H). LCMS (ESI) for $C_{23}H_{23}N_7O_3S$: m/z 478.2 (M + $H^+$, 100%).

**Compound A160**

**4-[4-(3-Isopropyl-[1,2,4]oxadiazol-5-yl)-piperidin-1-yl]-6-(4-[1,2,3]thiadiazol-4-yl-**phenoxy)-pyrimidine-5-carboxylic acid

White solid (3.8 mg, 13.63%); $^1$H NMR 400 MHz MeOD δ (ppm): 9.23 (s, 1H), 8.13(d, 2H) 8.06(s,1H)" 7.29(d, 2H), 4.61 (m, 2H), 3.30 (m, 2H), 3.05(q, 1H), 2.15 (m, 2H), 1.97 (m, 2H), 1.32 (d, 6H); LCMS (ESI) for $C_{23}H_{23}N_7O_4S$: m/z 494.3 (M + $H^+$, 100%).

**Compound A161**

**[4-[4-(3-Isopropyl-[1,2,4]oxadiazol-5-yl)-piperidin-1-yl]-6-(4-[1,2,3]thiadiazol-4-yl-phenoxy)-pyrimidin-5-yl]-methanol**

Yellow solid (17.5mg, 85.03%); $^1$H NMR 400MHz $CDCl_3$ δ (ppm): 8.63 (s, 1H), 8.31(s,1H), 8.11(d, 2H), 7.27 (d, 2H),4.77 (s,2H), 4.23 (m, 2H), 3.28 (m, 2H), 3.28 (m, 1H), 3.07(q, 1H), 2.21 (m, 2H), 2.03 (m, 2H), 1.34 (d, 6H); LCMS (ESI) for $C_{23}H_{25}N_7O_3S$: m/z 480.3 (M + $H^+$, 100%)

**Compound A162**

**[4-[4-(3-Isopropyl-[1,2,4]oxadiazol-5-yl)-piperidin-1-yl]-6-(4-[1,2,3]thiadiazol-4-yl-phenoxy)-pyrimidin-5-ylmethyl]-dimethyl-amine**

White solid, (4.2mg, 15.83%), LCMS (ESI) for $C_{25}H_{30}N_8O_2S$: m/z 507.3 (M + $H^+$, 100%).

**Compound A163**

**4-[4-(3-tert-Butyl-[1,2,4]oxadiazol-5-yl)-piperidin-1-yl]-6-(6-methanesulfonyl-pyridin-3-yloxy)-5-nitro-pyrimidine**

Compound **A163** was prepared using the general procedure for the addition of amine to pyrimidine; yellow solid (82mg, 81%). $^1$H NMR 400MHz $CDCl_3$ δ(ppm): 8.60(s, 1H); 8.19(s, 1H); 8.18(d,1H); 7.79(d,1H); 4.12(db,2H); 3.39-3.29(m,3H); 3.26(s,3H); 2.22(db,2H); 2.06-2.02(m, 2H); 1.36(s,9H).Exact mass calculated for $C_{21}H_{25}$ $N_7O_6S$ 503.16, LCMS (ESI) m/z 504.2(M+$H^+$, 100%).

**Compound A164**

**4-[4-(3-Isopropyl-[1,2,4]oxadiazol-5-yl)-piperidin-1-yl]-6-(4-methanesulfonyl-phenoxy)-2-methyl-pyrimidine-5-carbonitrile**

To a solution of 4-chloro-6-(4-methanesulfonyl-phenoxy)-2-methyl-pyrimidine-5-carbonitrile (80.0 mg, 0.25 mmol) and 4-(3-isopropyl-[1,2,4]oxadiazol-5-yl)-piperidine (107.1 mg, 0.50 mmol) in DMF (1 mL) was added potassium carbonate (68.3 mg, 0.50 mmol) and the resulting mixture was left stirring for 2 hours at room temperature. Worked up with ethyl acetate, sodium bicarbonate, dried with magnesium sulfate and evaporated. The crude product was crystallized with ethyl acetate/hexane over night and filtered off to afford Compound **A164** as a yellow solid (30.6mg). LCMS (ESI) for $C_{23}H_{26}N_6O_4S$: m/z 483.3 (M + $H^+$, 100%), $^1$H NMR 400MHz $CDCl_3$ 6 (ppm): 8.00 (d, 2H), 7.38 ( d, 2H), 4.82 (m, 2H), 3.45 (m, 2H), 3.31 (m, 1H), 3.10 (s, 3H), 3.08 (m, 1H), 2.35 (s, 3H), 2.24(m, 2H), 2.03 (m, 2H), 1.34(d, 6H).

**Compound A165**

**1-[4-[4-(3-Isopropyl-[1,2,4]oxadiazol-5-yl)-piperidin-1-yl]-6-(4-methanesulfonyl-phenoxy)-pyrimidin-5-yl]-ethanone**

To a solution of 1-[4-chloro-6-(4-methanesulfonyl-phenoxy)-pyrimidin-5-yl]-ethanone (0.21 mmol, 70 mg) and 4-(3-isopropyl-[1,2,4]oxadiazol-5-yl)-piperidine (0.21 mmol, 49 mg) in N,N-dimethyl formamide (500 uL) was added potassium carbonate (.21 mmol, 29 mg). The mixture was microwaved at 100 ˚C for 150 seconds. Its progress was monitored by thin layer chromatography and LCMS. The reaction was treated with water and the desired compound was extracted in ethyl acetate. Organic layer was evaporated in vacuo. Purification by HPLC provided Compound **A165** as a white solid (20 mg, 20%). $^1$H-NMR (400 MHz, $CDCl_3$) δ(ppm): 8.24 (s, 1H); 8.01 (d, 2H); 7.32 (d, 2H); 4.02 (m, 2H); 3.22 (m, 3H); 3.10 (m, 1H); 3.08 (s, 3H); 2.69 (s, 3H), 2.18 (m, 2H), 2.02 (m, 2H); 1.35 (d, 6H). LCMS (ESI), m/z 486.3 (M+H+, 100%).

**EXAMPLE 13**

**SYNTHESES OF COMPOUNDS OF THE PRESENT INVENTION**

**Compound B1**

**1-{6-[(Benzo[1,3]dioxol-5-ylmethyl)-amino]-5-nitro-pyrimidin-4-yl}-piperidine-4-carboxylic acid ethyl ester; General Method 14.**

Starting material, {6-chloro-5-nitro-pyrimidin-4-yl}-piperidine-4-carboxylic acid ethyl ester was prepared by general method 2. A mixture of 6-chloro-5-nitro-pyrimidin-4-yl}-piperidine-4-carboxylic acid ethyl ester (63 mg,0.2 mmol), amine (1.1 eqv, 33 mg, 0.22 mmol) and potassium carbonate (1.1 eqv, 31 mg,0.22 mmol) in DMF (1 ml) was stirred at 100 ˚C for 3 minutes in Smith microwave Synthesiser. HPLC purification afforded compound **B1** as yellow oil (59 mg, 54 %). $^1$H NMR 400MHz $CDCl_3$ δ (ppm): 8.58 (s, 1H); 8.01 (s, 1H); 6.63 (d, 2H); 6.61 (s, 1H); 5.79 (s, 2H); 4.51 (d, 2H); 3.99 (q, 2H) 3.70 (dt, 2H); 3.10 (td, 2H); 2.48 (m, 1H); 1.88 (dt, 2H); 1.70 (td, 2H); 1.09 (t, 3H). Exact mass calculated for $C_{20}H_{23}$ $N_5O_6$ 429.16, LCMS (ESI) m/z 430.0 (M+H$^+$, 100%).

**Compound B2**

**1-[5-Nitro-6-(3,4,5-trimethoxy-benzylamino)-pyrimidin-4-yl]-piperidine-4-carboxylic acid ethyl ester;**

**[method 14].** Filtered the reaction mixture and the filtrate was purified by semi preparatory HPLC afforded the pure product in 28 % yield. $^1$H NMR, 400MHz, $CDCl_3$, δ (ppm): 8.71 (m, 1H); 8.07 (s, 1H); 7.10 (s, 2H); 6.42 (s, 2H); 4.56 (d, 2H); 4.02 (q, 2H); 3.71 (s, 6H); 3.69 (s, 3H); 3.16 (m, 2H); 2.52 (m, 1H); 1.91 (m, 2H); 1.74 (m, 2H); 1.12 (t, 3H). LCMS (ESI) for $C_{22}H_{29}N_5O_7$: m/z 475(M+H$^+$, 100 %).

**Compound B3**

**(5-Nitro-6-piperidin-1-yl-pyrimidin-4-yl)-(3-trifluoromethyl-benzyl)-amine;**

**[method 14].** The product was purified by Preparatory TLC using hexane/ ethyl acetate (9:1). $^1$H NMR 400MHz $CDCl_3$ δ (ppm): 8.65 (s); 8.05 (s, 1H, pyrimidine); 7.65 (d, 1H); 7.50 (m, 2H); 7.40 (d, 1H); 5.00(s, 2H); 3.50 (m, 4H), 1.80 (s, NH); 1.75-1.60 (m, 5H)

**Compound B4**

**1-[5-Nitro-6-(2-tritluoromethyl-benzylamino)-pyrimidin-4-yl]-piperidine-4-carboxylic acid ethyl ester;**

**[method 14].** The residue was filtered through a silica plug [$SiO_2$; EtOAc/hexane; 50:50] and solvent removed in vacuo. Yield 0.143 g, 50 %. Yellow oil. $^1$H NMR 400MHz $CDCl_3$ δ(ppm): 8.63 (m, 1H); 8.07 (s, 1H); 7.68 (m, 1H); 7.53 (m, 2H); 7.40 (m, 1H); 5.01 (m, 2H); 4.17 (m, 2H); 3.89 (m, 2H); 3.21 (m, 2H); 2.63 (m, 1H); 2.03 (m, 2H); 1.85 (m, 2H); 1.28 (m, 3H). LCMS (ESI) m/z 390 (M+H$^+$, 100 %)

**Compound B5**

**1-[5-Nitro-6-(4-trifluoromethyl-benzylamino)-pyrimidin-40-yl]-piperidine-4-carboxylic acid ethyl ester;**

**[method 14].** Solvent was removed in vacuo and residue was purified by preparatory TLC. [$SiO_2$; EtOAc/ hexane; 10:90]. Yield 0.227 g, 73 %. Yellow oil. $^1$H NMR 400MHz $CDCl_3$ δ (ppm): 8.69 (m, 1H); 8.06 (s, 1H); 7.60 (d, 2H); 7.45 (d, 2H); 4.87 (m, 2H); 4.17 (m, 2H); 3.90 (m, 2H); 3.22 (m, 2H); 2.64 (m, 1H); 2.03 (m, 2H); 1.85 (m, 2H); 1.28 (m, 3H)

**Compound B6**

**1-[5-Nitro-6-(3-trifluoromethyl-benzylamino)-pyrimidin-4-yl]-piperidine-4-carboxylic acid ethyl ester;**

**[method 14].** Solvent was removed in vacuo and residue was purified by preparatory TLC. [$SiO_2$; EtOAc/ hexane; 10:90. Yield 0.177g, 65 %. Yellow solid. $^1$H NMR 400MHz $CDCl_3$ δ(ppm): 8.69 (m, 1H); 8.06 (s, 1H); 7.61 (m,

1H); 7.54 (m, 2H); 7.48 (m, 1H); 4.87 (m, 2H); 4.17 (m, 2H); 3.90 (m, 2H); 3.22 (m, 2H); 2.64 (m, 1H); 2.03 (m, 2H); 1.85 (m, 2H); 1.28 (m, 3H)

**Compound B7**

**(5-Nitro-6-piperidin-1-yl-pyrimidin-4-yl)-(2-trifluoromethyl-benzyl)-amine;**

**[method 14]**. The product was purified by Preparatory TLC using hexane/ ethyl acetate (9:1). $^1H$ NMR 400MHz $CDCl_3$ δ (ppm): 8.65 (s); 8.05 (s, 1H, pyrimidine); 7.65 (d, 1H); 7.50 (m, 2H); 7.40 (d, 1H); 5.00(s, 2H); 3.50 (m, 4H,), 1.80 (s, NH),1.75-1.60 (m, 5H)

**Compound B8**

**(5-Nitro-6-piperidin-1-yl-pyrimidin-4-yl)-(4-trifluoromethyl-benzyl)-amine;**

The product was purified by Preparatory TLC using hexane/ ethyl acetate/dichloromethane (8:1:1). $^1H$NMR 400MHz $CDCl_3$ δ (ppm): 9.05 (s); 8.05 (s,1H, pyrimidine); 7.70 (d, 2H); 7.50 (d, 2H); 4.90 (s, 2H); 3.40 (m,4H,), 1.75-1.60 (.m, 6H).

**Compound B9**

**1-[5-Amino-6-(3-trifluoromethyl-benzylamino)-pyrimidin-4-yl]-piperidine-4-carboxylic acid ethyl ester; General Method 15.**

Di-substituted-5-nitropyrimidine (180 mg, 0.4 mmol) was dissolved in ethyl acetate (5 mL) and flushed with $N_2$ gas. Palladium catalyst [5 %, Pd/C] was added and hydrogen gas was introduced into the mixture. The reaction mixture was stirred for 4 hours at room temperature. The reaction mixture was filtered through Celite and the solvent was removed under vacuo. Yield 0.158 g, 94 %. White solid. $^1H$ NMR 400MHz $CDCl_3$ δ(ppm): 8.69 (m, 1H); 8.06 (s, 1H); 7.61 (m, 1H); 7.54 (m, 2H); 7.48 (m, 1H); 4.87 (m, 2H); 4.17 (m, 2H); 3.90 (m, 2H); 3.22 (m, 2H); 2.64 (m, 1H); 2.03 (m, 2H); 1.85 (m, 2H); 1.28 (m, 3H). LCMS (ESI) m/z 424 ($M+H^+$,100%)

**Compound B10**

**1-[5-Amino-6-(4-trifluoromethyl-benzylamino)-pyrimidin-4-yl]-piperidine-4-carboxylic acid ethyl ester;**

**[method 15].** Yield 0.121 g, 72 %. White solid. $^1H$ NMR 400MHz $CDCl_3$ δ(ppm): 8.13 (s, 1H); 7.58 (d, 2H); 7.46 (d, 2H); 4.75 (m, 2H); 4.17 (m, 2H); 3.43 (m, 2H); 2.86 (m, 2H); 2.49 (m, 1H); 2.05 (m, 2H); 1.86 (m, 2H); 1.29 (m, 3H). LCMS (BSI) m/z 424 ($M+H^+$, 100%)

**Compound B11**

**1-[6-(4-Bromo-phenylamino)-5-nitro-pyrimidin-4-yl]-piperidine-4-carboxylic acid ethyl ester; General Method 16.**

[6-chloro-5-nitro-pyrimidin-4-yl]-piperidine-4-carboxylic acid ethyl ester (415 mg, 1.32 mmol) and 4-bromoaniline (309 mg, 1.80 mmol) were dissolved in anhydrous 1,4-dioxane (0.5-1 ml) and irradiated in a sealed microwave reaction tube at 250 ˚C for 300 seconds. The reaction mixture was passed through a silica plug [$SiO_2$; EtOAc/hexane; 20:80]. Yield 0.070 g, 12 %. Yellow solid. $^1H$ NMR 400MHz $CDCl_3$ δ (ppm): 9.98 (s, 1H); 8.03 (s, 1H); 7.41 (m, 2H); 7.17 (m, 2H); 4.09 (m, 2H); 3.83 (m, 2H); 3.16 (m, 2H); 2.77 (m, 1H); 1.97 (m, 2H); 1.78 (m, 2H); 1.20 (m, 3H). LCMS (ESI) m/z 451, 452 ($M+H^+$, 100 %)

**Compound B12**

**1-[5-Nitro-6-(4-trifluoromethyl-phenylamino)-pyrimidin-4-yl]-piperidine-4-carboxylic acid ethyl ester;**

**[Method 16].** Yield 0.010g, 14%. Yellow solid. $^1H$ NMR 400MHz $CDCl_3$ δ (ppm): 10.11 (s, 1H); 8.07 (s, 1H); 7.69 (d, 2H); 7.54 (d, 2H); 4.09 (m, 2H); 3.84 (m, 2H); 3.17 (m, 2H); 2.58 (m, 1H); 1.97 (m, 2H); 1.79 (m, 2H); 1.20 (m, 3H). LCMS (ESI) m/z 440 ($M+H^+$, 100 %)

**Compound B13**

**1-[6-(Methyl-phenyl-amino)-5-nitro-pyrimidin-4-yl]-piperidine-4-carboxylic acid ethyl ester;**

**[method 16]** Yield 0.062 g, 93 %. Yellow solid. $^1$H NMR 400MHz $CDCl_3$ δ(ppm): 8.09 (s, 1H); 7.33 (m, 2H); 7.17 (m, 3H); 4.14 (m, 2H); 3.92 (m, 2H); 3.54 (s, 3H); 3.19 (m, 2H); 2.58 (m, 1H); 1.99 (m, 2H); 1.83 (m, 2H); 1.26 (m, 3H). LCMS (ESI) m/z 386 ($M+H^+$, 100 %)

**Compound B14**

**1-[5-Nitro-6-(4-trifluoromethoxy-phenylamino)-pyrimidin-4-yl]-piperidine-4-carboxylic acid ethyl ester;**

**[Method 16].** Yield 0.066 g, 92 %. Yellow solid. $^1$H NMR 400MHz $CDCl_3$ δ(ppm): 10.09 (s, 1H); 8.11 (s, 1H); 7.63 (d, 2H); 7.22 (d, 2H); 4.17 (m, 2H); 3.91 (m, 2H); 3.25 (m, 2H); 2.66 (m, 1H); 2.05 (m, 2H); 1.86 (m, 2H); 1.28 (m, 3H). LCMS (ESI) m/z 456 ($M+H^+$,100%)

**Compound B15**

**1-[6-(4-Fluoro-phenylamino)-5-nitro-pyrimidin-4-yl]-piperidine-4-carboxylic acid ethyl ester;**

**[Method 16].** Yield 0.071 g, 100 %. Yellow solid. $^1$H NMR 400MHz $CDCl_3$ δ(ppm): 10.01 (s, 1H); 8.08 (s, 1H); 7.50 (m, 2H); 7.06 (m, 2H); 4.16 (m, 2H); 3.90 (m, 2H); 3.23 (m, 2H); 2.65 (m, 1H); 2.03 (m, 2H); 1.86 (m, 2H); 1.27 (m, 3H). LCMS (ESI) m/z 390 ($M+H^+$,100%)

**Compound B16**

**1-[6-(3,5-Difluoro-phenylamino)-5-nitro-pyrimidin-4-yl]-piperidine-4-carboxylic acid ethyl ester;**

**[Method 16].** Yield 0.066 g, 89 %. Yellow solid. $^1$H NMR 400MHz $CDCl_3$ δ(ppm): 10.13 (s, 1H); 8.15 (s, 1H); 7.29 (m, 2H); 6.60 (m, 1H); 4.16 (m, 2H); 3.90 (m, 2H); 3.22 (m, 2H); 2.64 (m, 1H); 2.03 (m, 2H); 1.84 (m, 2H); 1.27 (m, 3H). LCMS (ESI) m/z 408 ($M+H^+$,100%)

**Compound B17**

**1-[6-(3,5-Dichloro-phenylamino)-5-nitro-pyrimidin-4-yl]-piperidine-4-carboxylic acid ethyl ester;**

**[Method 16].** Yield 0.023 g, 33 %. Yellow solid. $^1$H NMR 400MHz $CDCl_3$ δ(ppm): 10.08 (s, 1H); 8.16 (s, 1H); 7.62 (m, 2H); 7.15 (m, 1H); 4.17 (m, 2H); 3.91 (m, 2H); 3.23 (m, 2H); 2.66 (m, 1H); 2.05 (m, 2H); 1.85 (m, 2H); 1.28 (m, 3H). LCMS (ESI) m/z 440 ($M+H^+$, 100%)

**Compound B18**

**1-[6-(Benzo[1,3]dioxol-5-ylamino)-5-nitro-pyrimidin-4-yl]-piperidine-4-carboxylic acid ethyl ester;**

**[Method 16].** The solvent was removed in vacuo and the residue purified by preparatory TLC [SiO2; EtOAc/hexane; 20:80]. Yield 0.063 g, 70 %. Orange oil. $^1$H NMR 400MHz $CDCl_3$ δ(ppm): 9.96 (s, 1H); 8.09 (s, 1H); 7.17 (s, 1H); 6.81 (m, 2H); 5.98 (s, 2H); 4.16 (q, 2H); 3.91 (m, 2H); 3.24 (m, 2H); 2.64 (m, 1H); 2.04 (m, 2H); 1.86 (m, 2H); 1.27 (m, 3H). LCMS (ESI) m/z 416 ($M+H^+$, 100 %)

**Compound B19**

**1-[6-(2-Bromo-4-trifluoromethoxy-phenylamino)-5-nitro-pyrimidin-4-yl]-piperidine-4-carboxylic acid ethyl ester;**

**[Method 16].** The reaction mixture was passed through a silica plug [SiO2; EtOAc/hexane; 10:90]. Yield 0.020 g, 24 %. Yellow solid. $^1$H NMR 400MHz $CDCl_3$ δ(ppm): 10.21 (s, 1H); 8.28 (m, 1H); 8.06 (s, 1H); 7.42 (m, 1H); 7.17 (m, 1H); 4.09 (m, 2H); 3.85 (m, 3.85); 3.18 (m, 2H); 2.55 (m, 1H); 1.98 (m, 2H); 1.79 (m, 2H); 1.20 (m, 3H). LCMS (ESI) m/z 535, 536 ($M+H^+$, 100 %)

**Compound B20**

**1-[6-(2-Fluoro-phenylamino)-5-nitro-pyrimidin-4-yl]-piperidine-4-carboxylic acid ethyl ester;**

**[Method 16].** The solvent was removed in vacuo and the residue was purified by preparatory TLC [$SiO_2$; EtOAc/hexane; 20:80]. Yield 0.016 g, 22 %. Yellow oil. $^1H$ NMR 400MHz $CDCl_3$ δ(ppm): 10.11 (s, 1H); 8.16 (m, 2H); 7.15 (m, 3H); 4.16 (m, 2H); 3.92 (m, 2H); 3.25 (m, 2H); 2.65 (m, 1H); 2.03 (m, 2H); 1.86 (m, 2H); 1.28 (m, 3H). LCMS (ESI) m/z 390 ($M+H^+$,100%)

**Compound B21**

**1-[6-(3-Fluoro-phenylamino)-5-nitro-pyrimidin-4-yl]-piperidine-4-carboxylic acid ethyl ester;**

**[Method 16].** The reaction mixture was passed through a silica plug [SiO2; EtOAc/hexane; 20:80]. Yield 0.034 g, 43 %. Yellow solid. $^1H$ NMR 400MHz $CDCl_3$ δ(ppm): 10.12 (s, 1H); 8.14 (s, 1H); 7.63 (m, 1H); 7.31 (m, 1H); 7.23 (m, 1H); 6.87 (m, 1H); 4.17 (m, 2H); 3.91 (m, 2H); 3.24 (m, 2H); 2.65 (m, 1H); 2.05 (m, 2H); 1.86 (m, 2H); 1.28 (m, 3H). LCMS (ESI) m/z 390 ($M+H^+$, 100%)

**Compound B22**

**1-{6-[(2-Fluoro-phenyl)-methyl-amino]-5-nitro-pyrimidin-4-yl}-piperidine-4-carboxylic acid ethyl ester;**

**[Method 16].** purified by preparatory TLC [SiO2; EtOAc/hexane; 20:80]. Yield 0.018 g, 23 %. Yellow oil. $^1H$ NMR 400MHz $CDCl_3$ δ(ppm): 8.09 (s, 1H); 7.14 (m, 2H); 7.03 (m, 2H); 4.15 (m, 2H); 3.93 (m, 2H); 3.51 (s, 3H); 3.20 (m, 2H); 2.60 (m, 1H); 2.00 (m, 2H); 1.83 (m, 2H); 1.27 (m, 3H). LCMS (ESI) m/z 404 ($M+H^+$,100%)

**Compound B23**

**1-[6-(Ethyl-phenyl-amino)-5-nitro-pyrimidin-4-yl]-piperidine-4-carboxylic acid ethyl ester;**

**[Method 16].** The solvent was removed in vacuo and the residue purified by preparatory TLC [$SiO_2$; EtOAc/hexane; 20:80]. Yield 0.008 g, 8 %. Yellow oil. $^1H$ NMR 400MHz $CDCl_3$ δ(ppm): 8.13 (s, 1H); 7.32 (m, 2H); 7.20 (m, 2H); 7.12 (m, 1H); 4.14 (m, 4H); 3.86 (m, 2H); 3.14 (m, 5H); 2.57 (m, 1H); 1.98 (m,2H); 1.83 (m, 2H); 1.24 (m, 4H). LCMS (ESI) m/z 400 ($M+H^+$,100%)

**Compound B24**

**1-{6-[(4-Chloro-phenyl)-methyl-amino]-5-nitro-pyrimidin-4-yl}-piperidine-4-carboxylic acid ethyl ester;**

**[Method 16].** The solvent was removed in vacuo and the residue purified by preparatory TLC [$SiO_2$; EtOAc/hexane; 20:80]. Yield 0.008 g, 8 %. Yellow oil. $^1H$ NMR 400MHz $CDCl_3$ δ(ppm): 8.10 (s, 1H); 7.29 (d, 2H); 7.10 (d, 2H); 4.15 (m, 2H); 3.93 (m, 2H); 3.52 (s, 3H); 3.21 (m, 2H); 2.56 (m, 1H); 2.01 (m, 2H); 1.85 (m, 2H); 1.26 (m, 3H) . LCMS (ESI) m/z 420 ($M+H^+$,100%)

**Compound B25**

**1-[6-(4-Difluoromethyl-benzylamino)-5-nitro-pyrimidin-4-yl]-piperidine-4-carboxylic acid ethyl ester;**

**[method 14].** HPLC provided compound **B25** as yellow oil (58 mg, 64 %). $^1H$ NMR 400 MHz $CDCl_3$ δ (ppm): 8.48 (s, 1H); 8.13 (s, 1H); 7.27 (d, 2H); 7.05 (d, 2H); 4.72 (d, 2H); 4.10 (q, 2H); 3.80 (dt, 2H); 3.25 (td, 2H); 2.60 (m, 1H); 2.00 (dt, 2H); 1.84 (td, 2H); 1.19 (t, 3H). Exact mass calculated for $C_{20}H_{23}F_2N_5O_5$ 451.17, LCMS (ESI) m/z 452.1 ($M+H^+$, 100%).

**Compound B26**

**1-{6-[(2,3-Dihydro-benzo[1,4]dioxin-6-ylmethyl)-amino]-5-nitro-pyrimidin-4-yl}-piperidine-4-carboxylic acid ethyl ester;**

**[method 14].** HPLC provided compound **B26** as yellow solid (62 mg, 56 %). $^1$H NMR 400MHz $CDCl_3$ $\delta$ (ppm): 8.86 (s,1H); 8.28 (s, 1H); 6.95 (d,1H); 6.93 (s, 1H); 6.89 (d, 1H); 4.75 (d,2H); 4.25 (q, 2H); 3.96 (dt, 4H); 3.87 (dt, 2H); 3.38 (td, 2H); 2.75 (m,1H); 2.15 (dt,2H); 1.98 (td, 2H); 1.35 (t, 3H). Exact mass calculated for $C_{21}H_{25}N_5O_6$ 443.18, LCMS (ESI) m/z 444.6(M+H$^+$, 100%).

**Compound B27**

**1-{6-[(2,3-Dihydro-benzo[1,4]dioxin-2-ylmethyl)-amino]-5-nitro-pyrimidin-4-yl}-piperidine-4-carboxylic acid ethyl ester;**

**[method 14].** HPLC provided compound **B27** as yellow solid (62 mg, 56 %). $^1$H NMR 400MHz $CDCl_3$ $\delta$(ppm): 8.86 (s, 1H); 8.28 (s, 1H); 6.95 (d, 1H); 6.93 (s, 1H); 6.89 (d, 1H); 4.75 (d, 2H); 4.25 (q, 2H); 3.96 (dt, 4H); 3.87 (dt, 2H); 3.38 (td, 2H); 2.75 (m, 1H); 2.15 (dt, 2H); 1.98 (td, 2H); 1.35 (t, 3H). Exact mass calculated for $C_{21}H_{25}N_5O_6$ 443.18, LCMS (ESI) m/z 444.6(M+H$^+$, 100%).

**Compound B28**

**1-{6-[(2,3-Dihydro-benzofuran-5-ylmethyl)-amino]-5-nitro-pyrimidin-4-yl}-piperidine-4-carboxylic acid ethyl ester;**

**[method 14].** HPLC provided compound **B28** as yellow solid (62 mg, 57 %). $^1$H NMR 400MHz $CDCl_3$ $\delta$(ppm): 8.47(s,1H); 8.02(s, 1H); 7.12(s, 1H); 7.02(d, 1H); 6.68(d,1H); 4.62(d, 2H); 4.50(t, 2H); 4.09(q, 2H); 3.81(dt, 2H); 3.15(d, 2H); 3.12(t, 2H); 2.55(m, 1H); 1.95(dt, 2H); 1.77(td, 2H); 1.19(t, 3H). Exact mass calculated for $C_{21}H_{25}N_5O_5$427.19, LCMS (ESI) m/z 428.1 (M+H$^+$, 100%).

**Compound B29**

**1-{6-[(6-Fluoro-4H-benzo[1,3]dioxin-8-ylmethyl)-amino]-5-nitro-pyrimidin-4-yl}-piperidine-4-carboxylic acid ethyl ester;**

**[method 14]**. HPLC provided compound **B29** as yellow solid (77 mg, 67 %). $^1$H NMR 400MHz $CDCl_3$ $\delta$(ppm): 8.92 (s,1H); 8.09 (s, 1H); 6.88 (dd, 1H); 6.58 (dd,1H); 5.22 (s,2H); 4.80 (s, 2H); 4.68 (d, 2H); 4.09 (q, 2H); 3.80 (d, 2H); 3.19 (td, 2H); 2.57 (m, 1H); 1.96 (dt, 2H); 1.79 (td, 2H); 1.19 (t, 3H). Exact mass calculated for $C_{21}H_{24}FN_5O_6$461.17, LCMS (ESI) m/z 462.3 (M+H$^+$, 100 %).

**Compound B30**

**1-[6-(3,4-Dihydro-2H-benzo [b] [1,4] dioxepin-7-ylamino)-5-nitro-pyrimidin-4-yl]-piperidine-4-carboxylic acid ethyl ester;**

**[Method 16].** The solvent was removed in vacuo and the residue purified by preparatory TLC [$SiO_2$; EtOAc/hexane; 20:80]. Yield 0.069 g, 71 %. Yellow oil. $^1$H NMR 400 MHz $CDCl_3$ $\delta$ (ppm): 9.96 (s, 1H); 8.09 (s, 1H); 7.24 (m, 1H); 7.04 (m, 1H); 6.95 (m, 1H); 4.22 (m, 6H); 3.90 (m, 2H); 3.22 (m, 2H); 2.63 (m, 1H); 2.19 (m, 2H); 2.03 (m, 2H); 1.85 (m, 2H); 1.26 (m, 3H). LCMS (ESI) m/z 444 (M+H$^+$, 100 %)

**Compound B31**

**1-{6-[4-(Morpholine-4-sulfonyl)-phenylamino]-5-nitro-pyrimidin-4-yl}-piperidine-4-carboxylic acid ethyl ester;**

**[Method 16].** The solvent was removed in vacuo and the residue purified by preparatory TLC [$SiO_2$; EtOAc/hexane; 20:80]. Yield 0.030 g, 29 %. Yellow oil. $^1$H NMR 400MHz $CDCl_3$ $\delta$ (ppm): 10.29 (s, 1H); 8.19 (s, 1H); 7.89 (d, 2H); 7.74 (d, 2H); 4.17 (m, 2H); 3.91 (m, 2H); 3.75 (m, 3.75); 3.27 (m, 2H); 3.02 (m, 6H); 2.66 (m, 1H); 2.07 (m, 2H); 1.87 (m, 2H); 1.28 (m, 4H). LCMS (ESI) m/z 521 (M+H$^+$, 100%)

**Compound B32**

**1-[6-(2,2-Difluoro-benzo[1,3]dioxol-4-ylamino)-5-nitro-pyrimidin-4-yl]-piperidine-4-carboxylic acid ethyl ester;**

**[Method 16].** purified by preparatory TLC [$SiO_2$; EtOAc/hexane; 20:80]. Yield 0.069 g, 74 %. Yellow solid. $^1H$ NMR 400MHz $CDCl_3$ δ (ppm): 10.06 (s, 1H); 8.10 (s, 1H); 7.61 (s, 1H); 7.25 (s, 1H); 7.04 (m, 2H); 4.17 (m, 2H); 3.91 (m, 2H); 3.25 (m, 2H); 2.65 (m, 1H); 2.05 (m, 2H); 1.87 (m. 2H); 1.28 (m, 3H). LCMS (ESI) m/z 452 ($M+H^+$, 100 %)

**Compound B33**

**1-[6-(2,2-Difluoro-benzo[1,3]dioxol-5-ylamino)-5-nitro-pyrimidin-4-yl]-piperidine-4-carboxylic acid ethyl ester;**

**[Method 16].** Preparatory TLC [SiO2; EtOAc/hexane; 20:80]. Yield 0.048g, 50%. Yellow solid. $^1H$ NMR 400MHz $CDCl_3$ δ(ppm): 9.87 (s, 1H); 8.13 (s, 1H); 7.67 (m, 1H); 7.25 (s, 1H); 7.09 (m, 1H); 6.92 (m, 1H); 4.17 (m, 2H); 3.92 (m, 2H); 3.26 (m, 2H); 2.66 (m, 1H); 2.06 (m, 2H); 1.88 (m, 2H); 1.58 (m, 2H); 1.28 (m, 3H). LCMS (ESI) m/z 452 ($M+H^+$, 100 %)

**Compound B34**

**1-[6-(1,1-Dioxo-1H-1λ$^6$-benzo[b]thiophen-6-ylamino)-5-nitro-pyrimidin-4-yl]-piperidine-4-carboxylic acid ethyl ester;**

**[Method 16].** preparatory TLC [SiO2; EtOAc/hexane; 20:80]. Yield 0.021 g, 22 %. Yellow solid. $^1H$ NMR 400MHz $CDCl_3$ δ (ppm): 10.23 (s, 1H); 8.35 (m, 1H); 8.07 (s, 1H); 7.51 (m, 1H); 7.21 (m, 1H); 7.08 (m, 1H); 6.57 (m, 1H); 4.05 (q, 2H); 3.79 (m, 2H); 3.13 (m, 2H); 2.53 (m, 1H); 1.93 (m, 2H); 1.74 (m, 2H); 1.15 (m, 3H). LCMS (ESI) m/z 460 ($M+H^+$,100%)

**Compound B35**

**1-{6-[(Furan-3-ylmethyl)-amino]-5-nitro-pyrimidin-4-yl}-piperidine-4-carboxylic acid ethyl ester;**

[method 14]. HPLC provided compound **B35** as yellow solid (46 mg, 61 %). $^1H$ NMR 400MHz $CDCl_3$ δ(ppm); 8.71(s,1H); 8.16(s, 1H); 7.44(s,1H); 7.37(d, 1H); 6.35(d, 1H); 4.59(d,2H); 4.11(q,2H); 3.82(dt, 2H); 3.26(td, 2H); 2.61 (m, 1H); 2.00(dt, 2H); 1.84(td, 2H); 1.20(t, 3H). Exact mass calculated for $C_{17}H_{21}$ $N_5O_5$ 375.15, LCMS (ESI) m/z 376.1 ($M+H^+$, 100%).

**Compound B36**

**1-{6-[2-(4-Methoxy-phenoxy)-ethylamino]-5-nitro-pyrimidin-4-yl}-piperidine-4-carboxylic acid ethyl ester;**

**[method 14].** HPLC provided compound **B36** as yellow solid (77 mg, 69 %). $^1H$ NMR 400MHz $CDCl_3$ δ (ppm): 9.22 (s,1H); 8.26 (s, 1H); 6.90 (d,2H); 6.87 (d, 2H); 4.20 (t,2H); 4.19 (t,2H); 4.04 (q, 2H); 3.93 (dt,2H); 3.79 (s,3H); 3.39 (td, 2H); 2.72 (m, 1H); 2.11 (dt, 2H); 1.94 (td, 2H); 1.30 (t, 3H). Exact mass calculated for $C_{21}H_{27}N_5O_6$ 445.20, LCMS (ESI) m/z 446.2 ($M+H^+$, 100 %).

**Compound B37**

**1-{6-[2-(5-Methoxy-1H-indol-3-yl)-ethylamino]-5-nitro-pyrimidin-4-yl}-piperidine-4-carboxylic acid ethyl ester;**

**[method 14].** HPLC provided compound **B37** as yellow solid (63 mg, 54 %). $^1H$ NMR 400MHz $CDCl_3$ δ (ppm): 8.47 (s,1H); 8.04 (s, 1H); 7.91 (s, 1H); 7.19 (d,1H); 7.01 (dd,2H); 6.80 (dd, 1H); 4.08 (q, 2H); 3.84 (dt,2H); 3.78 (s, 3H); 3.13 (td, 2H); 3.03 (t,2H); 2.54 (m, 5H); 1.94 (dt, 2H); 1.76 (td, 2H); 1.18 (t, 3H). Exact mass calculated for $C_{23}H_{28}N_6O_5$468.21 (ESI) m/z 469.2, 100 %).

**Compound B38**

**(3,4-Dihydro-2H-benzo[b][1,4]dioxepin-7-yl)-[5-nitro-6-(4-propyl-piperidin-l-yl)-pyrimidin-4-yl]-amine;**

**[Method 16].** preparatory TLC [$SiO_2$; EtOAc/hexane; 20:80]. Yield 0.003 g, 3%. Yellow oil. $^1H$ NMR 400MHz

$CDCl_3$ δ (ppm): 9.78 (s, 1H); 7.87 (s, 1H); 6.85 (m, 1H); 6.74 (m, 1H); 4.00 (m, 4H); 3.71 (m, 2H); 2.86 (m, 2H); 1.97 (m, 2H); 1.58 (m, 2H); 1.12 (m, 2H); 1.03 (m, 6H); 0.69 (m, 3H). LCMS (ESI) m/z 414 ($M+H^+$, 100 %)

**Compound B39**

**(3-Flnoro-phenyl)-[5-nitro-6-(4-propyl-piperidin-1-yl)-pyrimidin-4-yl]-amine;**

**[Method 16].** The solvent was removed in vacuo and the residue purified by preparatory TLC [$SiO_2$; EtOAc/hexane; 20:80]. Yield 0.007 g, 9 %. Yellow oil. $^1H$ NMR 400MHz $CDCl_3$ δ(ppm): 10.11 (s, 1H); 8.08 (s, 1H); 7.59 (m, 1H); 7.26 (m, 1H); 7.20 (m, 1H); 6.82 (m, 1H); 3.90 (m, 2H); 3.04 (m, 2H); 1.76 (m, 2H); 1.56 (m, 1H); 1.29 (m, 2H); 1.20 (m, 4H); 0.86 (m, 3H). LCMS (ESI) m/z 360 ($M+H^+$, 100 %)

**Compound B40**

**(3-Methoxy-phenyl)-[5-nitro-6-(4-propyl-piperidin-1-yl)-pyrimidin-4-yl]-amine;**

**[Method 16]**. The solvent was removed in vacuo and the residue purified by preparatory TLC [$SiO_2$ EtOAc/hexane; 20:80]. Yield 0.002 g, 2 %. Yellow oil. $^1H$ NMR 400MHz $CDCl_3$ δ(ppm): 10.04 (s, 1H); 8.04 (s, 1H); 7.21 (m, 2H); 7.04 (m, 1H); 6.67 (m, 1H); 3.87 (m, 2H); 3.75 (s, 3H); 3.02 (m, 2H); 1.74 (m, 2H); 1.49 (m, 1H); 1.27 (m, 2H); 1.19 (m, 4H); 0.84 (m, 3H). LCMS (ESI) m/z 404 ($M+H^+$,100%)

**Compound B41**

**1-{6-[(3-Fluoro-phenyl)-methyl-amino]-5-nitro-pyrimidin-4-yl}-piperidine-4-carboxylic acid ethyl ester;**

**[Method 16]**. The solvent was removed in vacuo and the residue purified by preparatory TLC [$SiO_2$; EtOAc/hexane; 20:80]. Yield 0.023 g, 30%. Light brown oil. $^1H$ NMR 400MHz $CDCl_3$ δ(ppm): 8.13 (s, 1H); 7.26 (m, 1H); 6.88 (m, 3H); 4.13 (q, 2H); 3.91 (m, 2H); 3.53 (s, 3H); 3.19 (m, 2H); 2.58 (m, 1H); 1.99 (m, 2H); 1.81 (m, 2H); 1.24 (m, 3H). LCMS (ESI) m/z 404 ($M+H^+$,100%)

**Compound B42**

**1-[6-(4-Benzoyl-phenylamino)-5-nitro-pyrimidin-4-yl]-piperidine-4-carbozylic acid ethyl ester;**

**[Method 16].** The solvent was removed in vacuo and the residue purified by preparatory TLC [$SiO_2$; EtOAc/hexane; 20:80]. Yield 0.059 g, 65 %. Light yellow solid. $^1H$ NMR 400MHz $CDCl_3$ δ (ppm): 10.31 (s, 1H); 8.21 (s, 1H); 7.89 (m, 2H); 7.82(m, 4H); 7.61 (m, 1H); 7.50 (m, 2H); 4.19 (q, 2H); 3.94 (m, 2H); 3.27 (m, 2H); 2.67 (m, 1H); 2.08 (m, 2H); 1.89 (m, 2H); 1.29 (m, 3H). LCMS (ESI) m/z 476 ($M+H^+$,100%)

**Compound B43**

**1-{6-[4-(1,1-Dioxo-1λ$^6$-thiomorpholin-4-ylmethyl)-phenylamino]-5-nitro-pyrimidin-4-yl}-piperidine-4-carboxylic acid ethyl ester;**

**[Method 16]**. The solvent was removed in vacuo and the residue purified by preparatory TLC [$SiO_2$; EtOAc/hexane; 50:50]. Yield 0.055 g, 56 %. Yellow oil. $^1H$ NMR 400MHz $CDCl_3$ δ(ppm): 10.09 (s, 1H); 8.11 (s, 1H); 7.55 (d, 2H); 7.31 (d, 2H); 5.29 (s, 1H); 4.15 (m, 2H); 3.89 (m, 2H); 3.63 (m, 2H); 3.22 (m, 2H); 3.01 (m, 6H); 2.64 (m, 1H); 2.03 (m, 2H); 1.84 (m, 2H); 1.25 (m, 4H). LCMS (ESI) m/z 519 ($M+H^+$,100%)

**Compound B44**

**1-[6-(4-Methanesulfonyl-phenylamino)-5-nitro-pyrimidin-4-yl]-piperidine-4-carboxylic acid ethyl ester;**

**[Method 16].** The solvent was removed in vacuo and the residue purified by preparatory TLC [$SiO_2$; EtOAc/hexane; 50:50]. Yield 0.032 g, 37 %. Yellow solid.$^1H$ NMR 400MHz $CDCl_3$ δ (ppm): 10.21 (s, 1H); 8.11 (s, 1H); 7.83 (m, 4H); 4.09 (m, 2H); 3.84 (m, 2H); 3.18 (m, 2H); 2.99 (s, 3H); 2:59 (m, 1H); 1.98 (m, 2H); 1.79 (m, 2H); 1.20 (m, 3H). LCMS (ESI) m/z 450 ($M+H^+$,100%)

**Compound B45**

**1-[6-(4-Dimethylsulfamoyl-phenylamino)-5-nitro-pyrimidin-4-yl]-piperidine-4-carboxylic acid ethyl ester;**

**[Method 16].** The solvent was removed in vacuo and the residue purified by preparatory TLC [$SiO_2$; EtOAc/hexane; 40:60]. Yield 0.060 g, 57 %. Yellow solid. $^1H$ NMR 400MHz $CDCl_3$ δ(ppm): 10.20 (s, 1H); 8.10 (s, 1H); 7.79 (d, 2H); 7.68 (d, 2H); 4.09 (q, 2H); 3.84 (m, 2H); 3.18 (m, 2H); 2.64 (s, 6H); 2.57 (m, 1H); 1.98 (m, 2H); 1.79 (m, 2H); 1.20 (m, 3H). LCMS (ESI) m/z 479 (M+$H^+$,100%)

**Compound B46**

**1-[6-(3-Methoxy-phenylamino)-5-nitro-pyrimidin4-yll-piperidine-4-carboxylic acid ethyl ester;**

**[Method 16]** purified by preparatory TLC. [$SiO_2$; 2:3 EtOAc/hexanes]. Yield 75 mg, 84 %. Yellow solid. $^1H$-NMR (400 MHz, $CDCl_3$) δ(ppm): 10.15 (s, 1H); 8.16 (s, 1H); 7.24 (m, 2H); 7.11 (m, 1H); 6.88 (m, 1H); 4.10 (q, 2H); 3.92 (m, 2H); 3.82 (s, 1H); 3.17 (m, 2H); 2.62 (heptet, 1H); 2.09 (m, 2H); 1.95 (m, 2H); 1.25 (t, 3H). LCMS (ESI), m/z 401 (M+$H^+$, 100%)

**Compound B47**

**1-[6-(2-Methoxy-phenylamino)-5-nitro-pyrimidin-4-yl]-piperidine-4-carboxylic acid ethyl ester;**

**[Method 16].** purified by preparatory TLC. [$SiO_2$; 15/85 EtOAc/hexanes]. Yield 56 mg, 63 %. Yellow solid. $^1H$-NMR (400 MHz, $CDCl_3$) δ(ppm): 10.57 (s, 1H); 8.41 (m, 1H); 8.18 (s, 1H); 7.15 (m, 1H); 7.0 (m, 1H); 4.21 (q, 2H); 3.92 (m, 5H); 3.25 (m, 2H); 2.63 (m, 2H); 2.08 (m, 2H); 1.88 (m,2H); 1.24 (m, 3H). LCMS (ESI), m/z 401 (M+$H^+$, 100%)

**Compound B48**

**1-[6-(3,5-Bis-trifluoromethyl-phenylamino)-5-nitro-pyrimidin-4-yl]-piperidine-4-carboxylic acid ethyl ester;**

**[Method 16].** Purified by flash chromatography. [Silica Gel 60; 20/80 EtOAclhexanes]. Yield 89 mg, 80 %. Yellow solid. $^1H$-14MR (400 MHz, $CDCl_3$) δ (ppm): 10.23 (s, 1H); 8.20 (m, 3H); 7.65 (s, 1H); 4.20 (m, 2H); 3.92 (m, 2H); 3.15 (m, 2H); 2.68 (heptet, 1H); 2.10 (m, 2H); 1.94 (m, 2H); 1.30 (t, 3H). LCMS (ESI), m/z 507 (M+$H^+$, 100%)

**Compound B49**

**1-[6-(2,5-Dimethoxy-phenylamino)-5-nitro-pyrimidin-4-yl]-piperidine-4-carboxylic acid ethyl ester;**

**[Method 16].** purified by preparatory TLC. [$SiO_2$; 20/80 EtOAc/hexanes]. Yield 61 mg, 64 %. Orange solid. $^1H$-NMR (400 MHz, $CDCl_3$) δ (ppm): 10.61 (s, 1H); 8.24 (s, 1H); 8.17 (s, 1H); 6.84 (d, 1H); 6.62 (dd, 1H); 4.17(q, 2H); 3.92 (m, 5H); 3.80 (s, 3H); 3.23 (m, 2H); 2.63 (heptet, 1H); 2.10 (m, 2H); 1.84 (m, 2H); 1.25 (t, 3H). LCMS (ESI), m/z 431 (M+$H^+$, 100 %)

**Compound B50**

**1-[6-(3,5-Dimethoxy-benzylamino)-5-nitro-pyrimidin-4-yl]-piperidine-4-carboxylic acid ethyl ester;**

**[method 14]**. Semi preparatory HPLC afforded the pure product in 28 %.$^1H$ NMR, 400MHz, $CDCl_3$, δ (ppm): 8.85 (m, 1H); 8.32 (s, 1H); 7.50 (s, 1H); 6.73 (d, 2H); 6.63 (t, 1H); 4.96 (d, 2H); 4.40 (q, 2H); 4.13 (m, 2H); 4.03 (s, 6H); 3.45 (m, 2H); 2.86 (m, 1H); 2.26 (m, 2H); 2.08 (m, 2H); 1.50 (t, 3H). LCMS (ESI) for $C_{22}H_{29}N_5O_7$: m/z 475(M+$H^+$, 100 %).

**Compound B51**

**[5-Nitro-6-(4-propyl-piperidin-1-yl)-pyrimidin-4-yl]-(3,4,5-trimethoxy-benzyl)-amine;**

**[method 14].** semi preparative HPLC afforded the pure product in 16 %. $^1H$ NMR, 400MHz, $CDCl_3$, δ (ppm): 8.81(m, 1H); 8.05(s, 1H); 7.07 (s, 1H); 6.39 (s, 2H); 4.53 (d, 2H); 3.76 (m, 1H); 3.67 (s, 6H); 3.66 (s, 3H); 3.01 (m, 2H); 1.68 (d, 2H); 1.12 (m, 6H); 0.72 (t, 3H). LCMS (ESI) for $C_{22}H_{31}N_5O_5$: m/z 445 (M+$H^+$, 100 %).

**Compound B52**

**(3,5-Dimethoxy-benzyl)-[5-nitro-6-(4-propyl-piperidin-1-yl)-pyrimidin-4-yl]-amine;**

**[method 14].** Semi preparative HPLC afforded the pure product in 20 %. $^1H$ NMR, 400MHz, $CDCl_3$, δ (ppm): 8.66 (m, 1H); 8.11 (s, 1H); 7.30 (s, 1H); 6.54 (d, 2H); 6.43 (t, 1H); 4.76 (d, 2H); 3.96 (m, 1H); 3.83 (s, 6H); 3.12 (m, 2H); 1.84 (m, 2H); 1.38 (m 2H); 1.30 (m 4H); 0.95 (t, 3H). LCMS (ESI) for $C_{21}H_{29}N_5O_4$: m/z 415 ($M+H^+$, 100 %).

**Compound B53**

**(4-{5-Nitro-6-[4-(pyridin-2-ylsulfanyl)-piperidin-1-yl]-pyrimidin-4-ylamino}-phenyl)-phenyl-methanone;**

**[Method 16].** purified by preparative TLC. [$SiO_2$; 30/70 EtOAc/hexanes]. Yield 42 mg, 51 %. Yellow solid. $^1H$-NMR (400 MHz, $CDCl_3$) δ(ppm): 10.3 (s, 1H); 8.44 (d, 1H); 8.20 (s, 1H); 7.87 (m, 2H); 7.81 (m, 4H); 7.59 (m, 1H); 7.50 (m, 3H); 7.18 (d, 1H); 7.01 (d, 1H); 4.22 (heptet, 1H); 3.92 (m, 2H); 3.45 (dt, 2H); 2.26 (m, 2H); 1.86 (m, 2H). LCMS (ESI), m/z 513 (M+H+, 100%)

**Compound B54**

**(4-{5-Nitro-6-[4-(2-trifluoromethyl-phenoxy)-piperidin-1-yl]-pyrimidin-4-ylamino}-phenyl)-phenyl-methanone;**

**[Method 16].** residue purified by preparatory TLC [$SiO_2$; EtOAc/hexane; 20:80]. Yield 0.057 g, 68 %. Yellow solid. $^1H$ NMR 400 MHz $CDCl_3$ δ (ppm): 10.24 (s, 1H); 8.13 (s, 1H); 7.80 (m, 2H); 7.74 (m, 4H); 7.52 (m, 2H); 7.42 (m, 4H); 6.95 (m, 2H); 4.78 (m, 1H); 3.64 (m, 4H); 2.02 (m, 4H). LCMS (ESI) m/z 564 ($M+H^+$, 100 %)

**Compound B55**

**1-[6-(4-Cyano-phenylamino)-5-nitro-pyrimidin-4-yl]-piperidine-4-carboxylic acid ethyl ester;**

**[Method 16].** residue purified by preparatory TLC [$SiO_2$; EtOAc/hexane; 20:80]. Yield 0.035 g, 40 %. Yellow solid. $^1H$ NMR 400MHz $CDCl_3$ δ(ppm): 10.19 (s, 1H); 8.10 (s, 1H); 7.76 (d, 2H); 7.58 (d, 2H); 4.09 (q, 2H); 3.83 (m, 2H); 3.17 (m, 2H); 2.58 (m, 1H); 1.97 (m, 2H); 1.78 (m, 2H); 1.19 (m, 3H) LCMS (ESI) m/z 397 ($M+H^+$,100%)

**Compound B56**

**1-[6-(3,5-Dimethozy-phenylamino)-5-nitro-pyrimidin-4-yl]-piperidine-4-carboxylic acid ethyl ester;**

**[Method 16].** residue purified by preparatory TLC [$SiO_2$; EtOAc/hexane; 20:80]. Yield 0.070 g, 73 %. Orange solid. $^1H$ NMR 400MHz $CDCl_3$ δ (ppm): 9.84 (s, 1H); 7.91 (s, 1H); 6.60 (d, 2H); 6.10 (t, 1H); 3.954 (q, 2H); 3.69 (m, 2H); 3.58 (s, 6H); 3.01 (m, 2H); 2.42 (m, 1H); 1.82 (m, 2H); 1.63 (m, 2H); 1.05 (m, 3H). LCMS (ESI) m/z 432 ($M+H^+$,100%)

**Compound B57**

**1-[6-(4-sec-Butyl-phenylamino)-5-nitro-pyrimidin-4-yl]-piperidine-4-carboxylic acid ethyl ester;**

**[Method 16].** residue purified by preparatory TLC [$SiO_2$; EtOAc/hexane; 20:80]. Yield 0.088 g, 93 %. Orange oil. $^1H$ NMR 400MHz $CDCl_3$ δ (ppm): 10.10 (s, 1H); 8.14 (s, 1H); 7.50 (d, 2H); 7.21 (d, 2H); 4.19 (q, 2H); 3.94 (m, 2H); 3.26 (m, 2H); 2.63 (m, 2H); 2.06 (m, 2H); 1.87 (m, 2H); 1.61 (m, 2H); 1.27 (m, 6H); 0.86 (m, 3H). LCMS (ESI) m/z 428 ($M+H^+$, 100 %)

**Compound B58**

**1-[6-(4-Heptyl-phenylamino)-5-nitro-pyrimidin-4-yl]-piperidine-4-carboxylic acid ethyl ester;**

**[Method 16].** residue purified by preparatory TLC [$SiO_2$; EtOAc/hexane; 20:80]. Yield 0.092 g, 89 %. Orange oil. $^1H$ NMR 400MHz $CDCl_3$ δ(ppm): 10.05 (s, 1H); 8.09 (s, 1H); 7.43 (d, 2H); 7.18 (d, 2H); 4.16 (q, 2H); 3.90 (m, 2H); 3.22 (m, 2H), 2.60 (m, 3H); 2.02 (m, 2H); 1.84 (m, 2H); 1.27 (m, 13H); 0.87 (m, 3H). LCMS (ESI) m/z 470 ($M+H^+$, 100 %)

**Compound B59**

**2'-(4-Benzoyl-phenylamino)-3'-nitro-3,4,5,6-tetrahydro-2H-[1,4')bipyridinyl-4-carboxylic acid ethyl ester. General Method 17.**

A mixture of 2, 4-dichloro-3-nitro-pyridine (77 mg, 0.4 mmol), 4-benzoyl-aniline (1.0 eqv, 7 9mg, 0.4 mmol) and potassium carbonate (1.4 eqv, 78 mg, 0.56 mmol) in DMF (1 ml) was stirred in a sealed vessel at 150 ˚C for 30 mins under microwave irradiation. HPLC provided intermediate compound 2-[4-benzoyl-anilino]-4-dichloro-3-nitro pyridine as brown solid (58 mg, 41 % yield). Exact mass calculated for $C_{18}H_{12}ClN_3O_3$ 353.06, LCMS (ESI) m/z 353.6(M+H$^+$, 100%).

**[method 14]**. RP-HPLC provided compound **B59** as orange solid (26 mg, 27 % yield). $^1$H NMR 400MHz $CDCl_3$ δ (ppm): 9.55 (s, 1H); 7.89 (d, 3H); 7.74 (d, 2H); 7.57 (t, 1H); 7.46 (t, 2H); 7.32 ( d, 2H); 6.50 (d, 1H); 4.12 (q, 2H); 3.63 (d, 2H); 3.26 ( t, 2H); 2.63-2.58 (m, 1H); 2.04( d, 2H); 1.94 (td, 2H); 1.21 (t, 3H). Exact mass calculated for $C_{26}H_{26}N_4O_5$ 474.19, LCMS (ESI) m/z 475.3 (M+H$^+$, 100%).

**Compound B60**

**1-[5-Nitro-6-(3,4,5-trimethoxy-phenylamino)-pyrimidin-4-yl]-piperidine-4-carboxylic acid ethyl ester;**

**[Method 16].** purified by preparatory TLC. [$SiO_2$; 30/70 EtOAc/hexanes].Yield 42 mg, 41 %. Orange oil. $^1$H-NMR (400 MHz, $CDCl_3$) δ(ppm): 9.97 (s, 1H); 8.05 (s, 1H); 6.77 (s, 2H); 4.08 (q, 2H); 3.81 (m, 11H); 3.17 (t, 2H); 2.58 (heptet, 1H); 1.97 (t, 2H); 1.78 (q, 2H); 1.19 (t, 3H). LCMS (ESI), m/z 462 (M+H+, 100%)

**Compound B61**

**1-[5-Nitro-6-(4-pentyl-phenylamino)-pyrimidin-4-yl]-piperidine-4-carboxylic** acid ethyl **ester;**

**[Method 16].** purified by preparatory TLC. [$SiO_2$; 20/80 EtOAc/hexanes]. Yield 79 mg, 81 %. Yellow oil. $^1$H-NNM (400 MHz, $CDCl_3$) δ (ppm): 9.99 (s, 1H); 8.00 (s, 1H); 7.38 (d, 2H); 7.12 (d, 2H); 4.09 (q, 2H); 3.83 (m, 2H); 3.15 (m, 2H); 2.55 (m, 3H); 1.95 (m, 2H); 1.79 (m, 2H); 1.53 (m, 2H); 1.24 (m, 7H); 0.81 (t, 3H). LCMS (ESI),m/z 442 (M+H$^+$, 100 %)

**Compound B62**

**1-{6-[4-(3-Carboxy-propyl)-phenylamino]-5-nitro-pyrimidin-4-yl}-piperidine-4-carboxylic acid ethyl ester;**

**[Method 16].** purified by preparatory TLC. [$SiO_2$; 30/70 EtOAc/hexanes]. Yield 67 mg, 67%. Yellow solid. $^1$H-NMR (400 MHz, $CDCl_3$) δ (ppm): 10.08 (s, 1H); 8.13 (s, 1H); 7.49 (d, 2H); 7.22 (d, 2H); 4.18 (q, 2H); 3.92 (m, 2H); 3.25 (m, 2H); 2.68 (m, 3H); 2.40 (t, 2H); 2.06(m, 2H); 1.96 (m, 2H); 1.88 (m, 2H); 1.27 (t, 3H). LCMS (ESI), m/z 458 (M+H$^+$, 100 %)

**Compound B63**

**1-{6-[4-(Cyano-phenyl-methyl)-phenylamino]-5-nitro-pyrimidin-4-yl}-piperidine-4-carboxylic acid ethyl ester;**

**[Method 16].** Dry crude B63 was purified by Biotage Horizon 2 [12+M column; 30/70 EtOAc/hexanes]. Yield 93 mg, 87 %. Yellow film. $^1$H-NMR (400 MHz, $CDCl_3$) δ(ppm): 10.13 (s, 1H); 8.13 (s, 1H); 7.64 (tt, 2H); 7.36 (m, 7H); 5.31 (s, 1H); 4.17 (q, 2H); 3.92 (m, 2H); 3.25 (m, 2H); 2.66 (heptet, 1H); 2.06 (m, 2H); 1.88(m, 2H); 1.28 (t, 3H). LCMS (ESI), m/z 486 (M+H$^+$, 100 %)

**Compound B64**

**1-[6-(4-Cyclohexyl-phenylamino)-5-nitro-pyrimidin-4-yl]-piperidine-4-carboxylic acid ethyl ester;**

**[Method 16].** Crude **B64** was purified by preparatory TLC [SiO2; 20/80 EtOAc/hexanes]. Yield 55 mg, 55 %. Yellow solid. $^1$H-NMR (400 MHz, $CDCl_3$) δ(ppm): 9.99 (s, 1H); 8.04 (s, 1H); 7.38 (tt, 2H); 7.16 (m, 2H); 4.09 (m, 2H); 3.84 (m; 2H); 3.16 (m, 2H); 2.56 (heptet, 1H); 2.43(m, 1H); 1.95 (m, 2H); 1.79 (m, 8H); 1.31 (m, 2H), 1.20(t, 5H). LCMS (ESI), m/z 453 (M+H$^+$, 100%)

**Compound B65**

**1-[5-Nitro-6-(4-[1,2,4]triazol-1-yl-phenylamino)-pyrimidin-4-yl]-piperidine-4-carboxylic acid ethyl ester;**

**[Method 16]**. Crude **B65** was purified by flash chromatography [Silica Gel 60; 30/70 EtOAc/hexanes]. Yield 53 mg, 55 %. Yellow solid. $^1$H-NMR (400 MHz, $CDCl_3$) δ(ppm): 10.42 (s, 1H); 8.56 (s, 1H); 8.15 (s, 1H); 8.11 (s, 1H); 7.79 (tt, 2H); 7.71 (tt, 2H); 4.17 (q, 2H); 3.93 (m, 2H); 3.26 (m, 2H); 2.66(heptet, 1H); 2.05 (m, 2H); 1.87 (m, 2H); 1.27 (m, 3H). LCMS (ESI), m/z 438 ($M+H^+$, 100 %)

**Compound B66**

**1-[5-Nitro-6-(4-trifluoromethanesulfonyl-phenylamino)-pyrimidin-4-yl]-piperidine-4-carboxylic acid ethyl ester;**

**[Method 16].** Purified by flash chromatography [Silica Gel 60; 30/70 EtOAc/hexanes]. Yield 34 mg, 31 %. Yellow solid. $^1$H-NMR (400 MHz, $CDCl_3$) δ(PPM): 10.72 (s, 1H); 8.16 (s, 1H); 7.98 (m, 4H); 4.10 (q, 2H); 3.85 (m, 2H); 3.20 (m, 2H); 2.60 (heptet, 1H); 1.99 (m, 2H); 1.81(m, 2H); 1.19 (m, 3H). LCMS (ESI), m/z 503 ($M+H^+$, 100 %)

**Compound B67**

**1-[5-Nitro-6-(4-[1,2,3]thiadiazol-4-yl-phenylamino)-pyrimidin-4-yl]-piperidine-4-carboxylic acid ethyl ester;**

**[Method 16].** purified by flash chromatography [Silica Gel 60; 30/70 EtOAc/hexanes]. Yield 39 mg, 39 %. Yellow solid. $^1$H-NMR (400 MHz, $CDCl_3$) δ (ppm): 10.48 (s, 1H); 8.65 (s,1H); 8.18 (s, 1H); 8.08 (tt, 2H); 7.80 (tt, 2H); 4.16 (q, 2H); 3.94 (m, 2H); 3.26 (m, 2H); 2.66(heptet, 1H); 2.06 (m, 2H); 1.89 (m, 2H); 1.28 (t, 3H).

**Compound B68**

**[6-(4-Ethoxymethyl-piperidm-1-yl)-5-mtro-pyrimidin-4-yl]-(4-methanesuKbnyl-phenyl)-amine;**

**[Method 16].** purified by HPLC [Semi-Prep]. Yield 13 mg, 17 %. Yellow solid. $^1$H-NMR (400 MHz, $CDCl_3$) δ (ppm): 10.13 (s, 1H); 7.99 (s,1H); 7.73 (m, 4H); 3.79 (m, 2H); 3.3 (q, 2H); 3.11 (d, 2H); 2.95 (m, 2H); 2.86 (s, 3H); 1.77 (m, 3H); 1.18 (m, 2H); 1.02 (m, 3H). LCMS (FSI), m/z 436 ($M+H^+$, 100 %)

**Compound B69**

**[5-Nitro-6-(4-propyl-piperidin-1-yl)-pyrimidin-4-yl]-(4-[1,2,4]triazol-1-yl-phenyl)-amine;**

**[Method 16].** purified by preparatory TLC [$SiO_2$; 50/50 EtOAc/hexanes]. Yield 27 mg, 27 %. Yellow solid. $^1$H-NMR (400 MHz, $CDCl_3$) δ (ppm): 10.17 (s, 1H); 8.47 (s,1H); 8.07 (s, 1H); 8.04 (s, 1H); 7.73 (tt, 2H); 7.63 (tt, 2H); 3.89 (m, 2H); 3.05 (m, 2H); 1.76(m, 2H); 1.40 (m, 1H); 1.24 (m, 6H); 0.85 (t, 3H). LCMS (ESI), m/z 408 ($M+H^+$,100%)

**Compound B70**

**{5-Nitro-6-[4-(pyridin-2-ylsulfanyl)-piperidin-1-yl]-pyrimidin-4-yl}-(4-[1,2,4]triazol-1-yl-phenyl)-amine;**

**[Method 16].** The crude was purified by HPLC. Yield 52 mg, 55 %. Yellow solid. $^1$H-NMR (400 MHz, $CDCl_3$) δ (ppm): 10.18 (s, 1H); 8.47 (s, 1H); 8.37 (m, 1H); 8.09 (s, 1H); 8.04 (s, 1H); 7.72 (tt, 2H); 7.63 (tt, 2H); 7.43 (ddd, 1H); 7.12 (tt, 1H); 6.94 (m, 1H); 4.15 (heptet, 1H); 3.85 (m, 2H); 3.37 (m, 2H); 2.19 (m, 2H); 1.79 (m, 2H). LCMS (ESI), m/z 476 ($M+H^+$, 100%)

**Compound B71**

**(2-Fluoro-phenyl)-{6-[4-(3-methyl-[1,2,4]oxadiazol-5-yl)-piperidin-1-yl]-5-nitro-pyrimidin-4-yl}-amine;**

**[Method 16].** Purified by HPLC. Yield 33 mg, 38%. Yellow solid.$^1$H-NMR (400 MHz, $CDCl_3$) δ (ppm): ): 10.01 (s, 1H); 8.09 (s, 1H); 7.63 (t, 1H); 7.28 (m, 1H); 7.16 (m, 2H); 3.98 (m, 2H); 3.41 (m, 2H); 3.33 (heptet, 1H); 2.36 (s, 3H); 2.23 (m, 2H); 2.05 (m, 2H). LCMS (ESI), m/z 399 ($M+H^+$, 100%)

**Compound B72**

**(4-Methanesnlfonyl-phenyl)-{6-[4-(3-methyl-[1,2,4]oxadiazol-5-yl)-piperidin-1-yl]-5-nitro-pyrimidin-4-yl}-amine;**

**[Method 16].** yellow solid.Yield 15.2%. $^1$H NMR 400MHz $CDCl_3$ δ (ppm): 10.5 (s, 1H); 8.49 (s, 1H); 8.2 (dd, 4H); 4.26 (d, 2H); 3.64 (m, 2H); 3.60 (m, 1H); 3.33 (s, 3H); 2,67 (s, 3H); 2.5 (d, 2H); 2.33 (m, 2H). LCMS (ESI) m/z 460,2 ($M^+H^+$, 100%)

**Compound B73**

**{6-[4-(3-Methyl-[1,2,4] oxadiazol-5-yl)-piperidin-1-yl]-5-nitro-pyrimidin-4-yl}-(4-[1,2,4]triazol-1-yl-phenyl)-amine;**

**[Method 16].** yellow solid. Yield 11 %. $^1$H NMR 400MHz $CDCl_3$ δ (ppm): 10 (s, 1H); 8.41 (s, 1H);); 8.02 (s, 1H); 7.97 (s, 1H); 7.64 (d, 2H); 7.55 (d, 2H); 3.84 (d, 2H); 3.24 (m, 2H); 3.20 (m, 1H); 2.25 (s, 3H); 2,09 (d, 2H); 1.90 (m, 2H). LCMS (ESI) m/z 449,2 ($M^+H^+$, 100%)

**Compound B74**

**1-{5-Nitro-6-[4-(4-trifluoromethyl-phenoxy)-phenylamino]-pyrimidin-4-yl}-piperidine-4-carboxylic acid ethyl ester;**

**[Method 16].** residue purified by preparatory TLC [SiO2; EtOAc/hexane; 20:80] Yield 0.057g, 56 %. Yellow solid. $^1$H NMR 400MHz $CDCL_3$ δ (ppm): 10.23 (s, 1H); 8.25 (s, 1H); 7.72 (m, 4H); 7.20 (m, 4H); 4.30 (q, 2H); 4.05 (m, 2H); 3.37 (m, 2H); 2.78 (m, 1H); 2.18 (m, 2H); 2.00 (m, 2H); 1.40 (m, 3H). LCMS (ESI) m/z 532 ($M+H^+$,100%)

**Compound B75**

**{6-[4-(3-Ethyl-[1,2,4]oxadiazol-5-yl)-piperidin-1-yl]-5-nitro-pyrimidin-4-yl}-(2-fluorophenyl)-amine;**

**[Method 16].** The desired product was observed by LCMS m/z 414 ($M+H^+$). Purification by RP-HPLC.Yield 69 %.$^1$H NMR 400MHz $CDCl_3$ δ (ppm): 10.00 (s, 1H); 8.11 (s,1H); 8.09 (m, 1); 7.12 (m, 3H); 3.97 (d, 2H); 3.29 (m, 2H); 3.26 (m, 1H); 2.69 (m, 2H); 2.18 (m, 2H); 2.06 (m, 2H); 1.29 (t, 3H). LCMS (ESI) m/z 414 ($M+H^+$, 100%)

**Compound B76**

**{6-[4-(2-Methoxy-phenylsulfanyl)-piperidin-1-yl]-5-nitro-pyrimidin-4-yl}-(4-[1,2,4]triazol-1-yl-phenyl)-amine;**

**[Method 16].** Yield 43 %. Yellow solid. $^1$H NMR 400MHz $CDCl_3$ δ (ppm): 9.98 (s, 1H); 8.33 (s, 1H);); 7.91 (d, 2H); 7.57 (d, 2H); 7.47 (d, 2H); 7.20 (m, 1H); 7.09 (m, 2H); 6.70 (m, 2H); 3.69 (s, 3H); 3.33 (m, 1H); 3.10 (m, 2H); 1.85 (m, 2H); 1.51 (m, 4H). LCMS (ESI) m/z 505 ($M+H^+$, 100 %)

**Compound B77**

**(4-Methanesulfonyl-phenyl)-{5-nitro-6-[4-(pyridin-2-ylsulfanyl)-piperidin-1-yl]-pyrimidin-4-yl}-amine**

Crude **B77** was purified by flash chromatography [Silica gel 60; 50/50 EtOAc/hexanes]. Yield 49 mg, 50 %. Yellow solid. $^1$H-NMR (400 MHz, $CDCl_3$) δ(ppm): ): 10.25 (s, 1H); 8.13 (s, 1H); 7.85 (m, 4H); 7.64 (tt, 1H); 7.43 (m, 1H); 7.11 (d, 1H); 6.95 (m, 1H); 4.15 (m, 1H); 3.85(m, 2H); 3.38 (m, 2H); 2.99 (s, 3H); 2.19 (m, 2H); 1.79 (m, 2H). LCMS (ESI), m/z 487 ($M+H^+$, 100% )

**Compound B78**

**(3-Methoxy-phenyl)-{5-nitro-6-[4-(pyridin-2-ylsulfanyl)-piperidin-1-yl]-pyrimidin-4-yl}-amine**

**[Method 16].** The crude mixture was purified by preparatory TLC [$SiO_2$; 20/80 EtOAc/hexanes].

Yield 70 mg, 87 %. Yellow solid. $^1$H-NMR (400 MHz, $CDCl_3$) δ (ppm): 10.25 (s, 1H); 8.55 (m, 1H); 8.25 (s, 1H);

7.60 (ddd, 1H); 7.40 (m, 2H); 7.38 (s, 1H); 7.29 (d, 1H); 7.22 (d, 1H); 7.12 (m, 1H); 4.33 (heptet, 1H); 4.01 (m, 2H); 3.94 (s, 3H); 3.54 (m, 2H); 2.36 (m, 2H); 1.98 (m, 2H). LCMS (ESI), m/z 439 ($M+H^+$, 100% )

**Compound B79**

**Benzo[1,3]dioxol-5-yl-[5-nitro-6-(4-propyl-piperidin-1-yl)-pyrimidin-4-yl]-amine.**

**[Method 16].** Residue purified by preparatory TLC [$SiO_2$; EtOAc/hexane; 20:80]. Yield 0.005 g, 6 %. Yellow oil. $^1H$ NMR 400MHz $CDCl_3$ δ(ppm): 9.92 (s, 1H); 8.01 (s, 1H); 7.13 (m, 1H); 6.78 (m, 1H); 6.72 (m, 1H); 5.92 (s, 2H); 3.87 (m, 2H); 3.02 (m, 2H); 1.74 (m, 2H); 1.48 (m, 1H); 1.27 (m, 2H); 1.18 (m, 4H); 0.84 (m, 3H). LCMS (ESI) m/z 386 ($M+H^+$,100%)

**Compound B80**

**(4-Fluoro-phenyl)-{1-[5-nitro-6-(4-[1,2,4]triazol-1-yl-phenylamino)-pyrimidin-4-yl]-piperidin-4-yl}-methanone**

Purification by HPLC. Yield 56 %. yellow solid TFA salt. $^1H$ NMR 400MHz $CDCl_3$ δ (ppm): 11.61 (s, 3H); 10.3 (s, 1H); 9.18 (s,1H); 8.39 (s, 1H); 8.18 (s, 1H); 8.01 (m, 2H); 7.84 (d, 2H); 7.74 (d, 2H); 7.19 (t, 2H); 4.07 (d, 2H); 3.67 (m, 1H); 3.47 (m, 2H); 2.05 (m, 4H). LCMS (ESI) m/z 489.4 ($M^+H^+$, 100%)

**Compound B81**

**[5-Nitro-6-(4-phenylsulfanyl-piperidin-1-yl)-pyrimidin-4-yl]-(4-[1,2,4]triazol-1-yl-phenyl)-amine**

**[Method 16].** product precipitated from the crude as a yellow solid. It was filtered and washed with hexane. Yield 36 %. $^1H$ NMR 400MHz $CDCl_3$ δ (ppm): 10.17 (s, 1H); 8.57 (s, 1H); 8.10 (d, 2H); 7.76 (d, 2H); 7.66 (d, 2H); 7.42 (m, 2H); 7.29 (m, 3H); 3.89 (m, 2H); 3.39 (m, 1H);3.27 (m, 2H); 2.06(m; 2H); 1.71 (m, 2H). LCMS (ESI) m/z 475.3 ($M^+H^+$, 100%)

**Compound B82**

**(4-Fluoro-phenyl)-{1-[6-(2-fluoro-phenylamino)-5-nitro-pyrmudin-4-yl]-pipendin-4-yl}-methanone**

**[Method 16].** Purification by HPLC. Yield 17 %. red oil. $^1H$ NMR 400MHz $CDCl_3$ δ (ppm): 9.92 (s, 1H); 7.96 (s, 1H); 7.79 (m, 2H); 7.46 (m, 1H); 7.17 (m, 1H); 7.02 (m, 4H); 3.86 (d, 2H); 3.46 (m, 1H); 3.31 (m, 2H); 1.86 (m, 4H). LCMS (ESI) m/z 440.4 ($M^+H^+$, 100%)

**Compound B83**

**1-[6-(2-Methyl-5-phenyl-2H-pyrazol-3-ylamino)-5-nitro-pyrimidin-4-yl]-piperidine-4-carboxylic acid ethyl ester**

**[Method 16].** Purification by HPLC yielded orange solid.Yield 30 %. $^1H$ NMR 400MHz $CDCl_3$ δ (ppm):, 8.34 (s, 1H); 7.90 (m, 2H); 7.68 (d, 3H); 7.04 (s, 1H); 4.32 (m, 2H); 4.11 (s, 3H); 4.06 (m, 2H); 3.44 (m, 2H); 2.83 (m, 1H); 2.22 (m, 2H); 2.04 (m, 2H); 1.41 (t, 3H). LCMS (ESI) m/z 452 ($M^+H^+$, 100%)

**Compound B84**

**(4-Methanesulfonyl-phenyl)-[5-nitro-6-(4-phenylsulfanyl-piperidin-1-yl)-pyrimidin-4-yl]-amine**

**[Method 16].** Purification by HPLC yielded yellow solid. Yield 9 %. $^1H$ NMR 400MHz $CDCl_3$ δ (ppm): 10.0 (s, 1H); 8.00 (s, 1H); 7.75 (d, 2H); 7.68 (d, 2H); 7.26 (m, 2H); 7.14 (m, 3H); 3.73 (d, 2H); 3.22 (m, 1H); 3.13 (m, 2H); 2.87 (s, 3H); 1.91 (m, 2H); 1.55 (m,2H). LCMS (ESI) m/z 486 ($M^+H^+$, 100%)

**Compound B85**

**(4-Methanesulfonyl-phenyl)-{5-nitro-6-[4-(pyridin-2-yloxy)-piperidin-1-yl]-pyrimidin-4-yl}-amine**

**[Method 16].** as yellow solid (68 mg, 72 %). $^1H$ NMR 400MHz $CDCl_3$ δ(ppm): 10.2 (s, 1H); 8.13 (s, 1H); 8.12

(d, 1H); 7.87 (d, 2H); 7.83 (d, 2H); 7.60 (t, 1H); 6.87 (t,1H); 6.73 (d,1H); 5.29 (m,1H); 3.76-3.70 (m,2H); 3.56-3.51 (m, 2H); 2.99 (s,3H); 2.10-2.05 (m, 2H); 1.95-1.90 (m,2H). Exact mass calculated for $C_{21}H_{22}N_6O_5S$ 470.14, LCMS (ESI) m/z 471.4(M+H$^+$, 100%).

**Compound B86**

**{6-[4-(4-Fluoro-phenoxy)-piperidin-1-yl]-5-nitro-pyrimidin-4-yl}-(4-methanesulfonylphenyl)-amine**

**[method 16]** afforded compound B86 as yellow solid (58 mg, 60 %). $^1H$ NMR 400MHz $CDCl_3$ δ(ppm): 10.2 (s, 1H); 8.13 (s, 1H); 7.87 (d, 2H); 7.81 (d, 2H); 6.92(m, 2H); 6.82 (m,2H); 4.50 (m,1H); 3.71-3.65 (m,2H); 3.54-3.51 (m, 2H); 2.99 (s,3H); 1.99-1.91 (m 2H). Exact mass calculated for $C_{22}H_{22}$ $FN_5O_5S$ 487.13, LCMS (ESI) m/z 488.3(M+H$^+$, 100%).

**Compound B87**

**(4-Methanesulfonyl-phenyl)-{5-nitro-6-[4-(pyridin-4-yloxy)-piperidin-1-yl]-pyrimidin-4-yl}-amine**

Method 16 afforded compound **B87** as yellow solid (56 mg, 60 %). $^1H$ NMR 400MHz $CDCl_3$ δ(ppm): 10.2(s, 1H); 8.68 (d, 2H); 8.17(s,1H); 7.88 (d, 2H); 7.83(d, 2H); 7.26 (d, 2H); 4.96 (m,1H); 3.76-3.69 (m,2H); 3.62-3.57 (m,2H); 3.00 (s,3H); 2.21-2.15 (m 2H); 2.03-1.99 (m,2H). Exact mass calculated for $C_{21}H_{22}$ $N_6O_5S$ 470.14, LCMS (ESI) m/z 471.2(M+H$^+$, 100%).

**Compound B88**

**(4-Methanesuifonyl-phenyl)-{5-nitro-6-[4-(pyrimidin-2-yloxy)-piperidin-1-yl]-pyrimidin-4-yl}-amine**

Method 16 afforded compound **B88** as a yellow solid (69 mg, 73 %). $^1H$ NMR 400MHz $CDCl_3$ δ(ppm): 10.2 (s, 1H); 8.52 (d, 2H); 8.14(s,1H); 7.88 (d, 2H); 7.83 (d, 2H); 6.96(t, 1H); 5.34 (m,1H); 3.79-3.72 (m,2H); 3.58-3.52 (m,2H); 2.99 (s,3H); 2.14-2.08 (m 2H); 2.02-1.93 (m,2H). Exact mass calculated for $C_{20}H_{21}$ $N_7O_5S$ 471.13, LCMS (ESI) m/z 472.0(M+H$^+$, 100%).

**Compound B89**

**(4-Methanesulfonyl-phenyl)-{5-nitro-6-[4-(pyridin-4-ylsulfanyl)-piperidin-1-yl]-pyrimidin-4-yl}-amine**

Method 16 afforded compound **B89** as a yellow solid (52 mg, 54 %). $^1H$ NMR 400MHz $CDCl_3$ δ(ppm): 10.1(s, 1H); 8.56(d, 2H); 8.17(s,1H); 7.89 (d, 2H); 7.83(d, 2H); 7.53(d, 2H); 3.92-3.89(m,2H); 3.84(m,1H); 3.44-3.38(m,2H); 3.00(s,3H); 2.26-2.22(m 2H); 1.94-1.88(m,2H). Exact mass calculated for $C_{21}H_{22}$ $N_6O_4S_2$ 486.11, LCMS (ESI) m/z 487.2 (M+H$^+$, 100%).

**Compound B90**

**(4-Methanesulfonyl-phenyl)-{6-[4-(4-methoxy-phenylsulfanyl)-piperidin-1-yl]-5-nitro-pyrimidin-4-yl}-amine**

Method 16 afforded compound **B90** as a as yellow solid (50 mg, 49 %). $^1H$ NMR 400MHz $CDCl_3$ δ(ppm): 10.2 (s, 1H); 8.11(s,1H); 7.87 (d, 2H); 7.80(d, 2H); 7.34(d, 2H); 6.79(d,2H); 3.86-3.83(m,2H); 3.74(s,3H); 3.22-3.12(m,3H); 2.99(s,3H); 1.99-1.95(m 2H); 1.66-1.57(m,2H). Exact mass calculated for $C_{23}H_{25}N_5O_5S_2$ 515.13, LCMS (ESI) m/z 516.1 (M+H$^+$, 100%).

**Compound B91**

**[6-(4-Benzenesulfonyl-piperidin-1-yl)-5-nitro-pyrimidin-4-yl]-(4-methanesulfonylphenyl)-amine**

**Method 16** afforded compound **B91** a as yellow solid (51 mg, 50 %). $^1H$ NMR 400MHz $CDCl_3$ δ(ppm): 10.2 (s, 1H); 8.12(s,1H); 7.87 (d, 2H); 7.81(d,2H); 7.79(d, 2H); 7.64(t, 1H); 7.53(t,1H); 4.01(m,2H); 3.17(m,1H); 3.08-3.04(m, 2H); 2.99(s,3H); 2.08-2.04(m 2H); 1.82-1.78(m,2H). Exact mass calculated for $C_{22}H_{23}N_5O_6S_2$ 517.11, LCMS (ESI) m/z 518.3(M+H$^+$, 100%).

**Compound B92**

**{4-[6-(4-Methanesulfonyl-phenylamino)-5-nitro-pyrimidin-4-yl]-piperazin-1-yl}-acetic acid ethyl ester**

**[Method 16].** afforded compound **B92** as a yellow solid (45 mg, 48 %). $^1$H NMR 400MHz $CDCl_3$ δ (ppm): 10.2 (s, 1H); 8.18(s,1H); 7.88 (d, 2H); 7.81 (d, 2H); 4.21(q,2H); 3.86-3.83 (m,6H); 3.46-3.43 (m, 4H); 3.00 (s,3H); 1.23 (t, 3H). Exact mass calculated for $C_{19}H_{24}N_6O_6S$ 464.15, LCMS (ESI) m/z 465.3 (M+H$^+$, 100%).

**Compound B93**

**(2-Fluoro-phenyl)-{5-nitro-6-[4-(pyridin-2-ylsulfanyl)-cyclohexyl]-pyrimidin-4-yl}-amine**

**[Method 16].** The crude was dissolved in dichloromethane and purified by preparative TLC. [$SiO_2$; 15/85 EtOAc/hexanes].Yellow solid. Yield 7 mg, 10 %. $^1$H-NMR (400 MHz, $CDCl_3$) δ (ppm): 10.07 (s, 1H); 8.37 (d, 1H); 8.13 (m, 1H); 8.08 (s, 1H); 7.43 (ddd, 1H); 7.07 (m, 4H); 6.94 (m, 1H); 4.15 (heptet, 1H); 3.85 (m, 2H); 3.36 (m, 2H); 2.18 (m, 2H); 1.79 (m, 2H). LCMS (ESI), m/z 427 (M+H$^+$, 100 %)

**Compound B94**

**(2-Methoxy-phenyl)-{5-nitro-6-[4-(pyridin-2-ylsulfanyl)-cyclohexyl]-pyrimudiu-4-yl}-amine**

**[Method 16].** purified by preparative TLC. [SiO2; 15/85 EtOAc/hexanes]. Yellow solid. Yield 42 mg, 56 %. $^1$H-NMR (400 MHz, $CDCl_3$) δ (ppm): 10.51 (s, 1H); 8.36 (d, 2H); 8.10(s, 1H); 7.42 (ddd, 1H); 7.10 (d, 1H); 7.03 (ddd, 1H); 6.87 (d, 1H); 4.14 (heptet, 1H); 3.85 (m, 5H); 3.35 (m, 2H); 2.17 (m, 2H); 1.78 (m, 2H). LCMS (ESI), m/z 438 (M+H+, 100%)

**Compound B95**

**(4-Methanesulfonyl-phenyl)-(5-nitro-6-{4-[3-(3-trifluoromethyl-phenyl)-[1,2,4]oxadiazol-5-yl]-piperidin-1-yl}-pyrimidin-4-yl)-amine**

Following the general procedure 16, compound **B95** was obtained as a yellow solid (61 %). $^1$H NMR ($CDCl_3$, 400 MHz) δ 2.06-2.10 (m, 2H), 2.23-2.27 (m, 2H), 2.99 (s, 3H), 3.31-3.38 (m, 3H), 3.96-3.99 (m, 2H), 7.55 (t, 1H), 7.70 (d, 1H), 7.86 (dd, 4H), 8.16 (s, 1H), 8.20 (d, 1H), 8.28 (s, 1H), 10.2 (s, 1H). Exact mass calculated for $C_{25}H_{22}F_3N_7O_5S$ 589.1, found 590.4 (MH$^+$).

**Compound B96**

**{6-[4-(3-Ethyl-[1,2,4]oxadiazol-5-yl)-piperidin-1-yl]-5-nitro-pyrimidin-4-yl}-(4-methanesulfonyl-phenyl)-amine**

Following the general procedure 16, compound B96 was obtained as a yellow solid (31 %). $^1$H NMR ($CDCl_3$, 400 MHz) δ 1.25 (t, 3H), 2.06-2.10 (m, 2H), 2.23-2.27 (m, 2H), 2.70 (q, 2H), 2.99 (s, 3H), 3.31-3.38 (m, 3H), 3.96-3.99 (m, 2H), 7.84 (dd, 4H), 8.14 (s, 1H), 10.2 (s, 1H). Exact mass calculated for $C_{20}H_{23}N_7O_5S$ 473.1, found 474.2 (MH$^+$).

**Compound B97**

**(6-{4-[5-(4-Fluoro-phenyl)-[1,3,4]oxadiazol-2-yl]-piperidin-1-yl}-5-nitro-pyrimidin-4-yl)-(4-methanesuffonyl-phenyl)-amine**

Following the general procedure 16, compound **B97** was obtained as a yellow solid (93 %). $^1$H NMR ($CDCl_3$, 400 MHz) δ 2.06-2.10 (m, 2H), 2.23-2.27 (m, 2H), 2.99 (s, 3H), 3.31-3.38 (m, 3H), 3.96-3.99 (m, 2H), 7.24 (dd, 2H), 7.96 (dd, 4H), 8.04-8.08 (m, 2H), 8.25 (s, 1H), 10.3 (s, 1H). Exact mass calculated for $C_{24}H_{22}FN_7O_5S$ 539.14, found 540.3 (MH$^+$).

**Compound B98**

**(4-Methanesulfonyl-phenyl)-[5-nitro-6-(4-pyridin-2-ylmethyl-piperidin-1-yl)-pyrimidin-4-yl]-amine**

Following the general procedure 16, Compound **B98** was obtained as a yellow solid (95 %). $^1$HNMR ($CDCl_3$,

400 MHz) δ 1.36-1.47 (m, 2H), 1.69-1.72 (m, 2H), 2.12-2.18 (m, 1H), 2.99 (s, 3H), 3.00-3.05 (m, 2H), 3.91-3.94 (m, 2H), 7.52 (d, 1H), 7.65 (dt, 1H), 7.85 (dd, 4H), 8.11 (s, 1H), 8.17 (dt, 1H), 8.82 (d, 1H), 10.2 (s, 1H). Exact mass calculated for $C_{22}H_{24}N_6O_4S$ 468.1, found 469.4 ($MH^+$).

**Compound B99**

**4-[4-(3-Isopropyl-[1,2,4]oxadiazol-5-yl)-piperldin-1-yl]-6-(4-methylsulfanyl-phenylamino)-pyrimidine-5-carbonitrile**

Following the general procedure, Compound **B99** was prepared. $^1$H-NMR (DMSO-$d_6$): 9.38 (1H, s), 8.21 (1H, s), 7.47 (2H, J = 4.3 Hz, d), 7.23 (2H, J = 4.3 Hz, d), 4.50 (2H, m), 3.35 (2H, m), 3.02 (1H, m), 2.51 (3H, s), 2.18 (2H, m), 1.79 (2H, m) 1.83 (6H, J = 7Hz, d) ppm. LCMS: 436.3, 351.9, 324.4, 270.2.

**Compound B100**

**1-{6-[4-(4,5-Dichloro-imidazol-1-yl)-phenylamino]-5-nitro-pyrimidin-4-yl}-piperidine-4-carboxylic acid ethyl ester**

**[Method 16].** yellow solid (40 mg, 49 %). $^1$HNMR ($CDCl_3$, 400 MHz) δ 1.28 (t, 3H), 1.87 (m, 2H), 2.06 (m, 2H), 2.66 (m, 1H), 3.26 (t, 2H), 3.93 (m, 2H), 4.17 (q, 2H), 7.37 (m, 2H), 7.54 (s, 1H), 7.82 (m, 2H), 8.17 (s, 1H), 10.23 (s, 1H). Exact mass calculated for $C_{21}H_{21}Cl_2N_7O_4$ 506.34, found 506.2 ($MH^+$).

**Compound B101**

**Benzo[1,3]dioxol-5-yl-{5-nitro-6-[4-(pyridin-2-yisulfanyl)-piperidin-1-yl]-pyrimidin-4-yl}-amine**

**[Method 16].** orange solid (9 mg, 14 %). $^1$HNMR ($CDCl_3$, 400 MHz) δ 1.78 (m, 2H), 2.17 (m, 2H), 3.35 (m, 2H), 3.83 (m, 2H), 4.14 (m, 1H), 5.92 (m, 2H), 6.75 (m, 2H), 6.94 (m, 1H), 7.11 (m, 1H), 7.19 (m, 1H), 7.42 (m, 1H), 8.02 (s, 1H), 8.36 (m, 1H), 9.91 (s, 1H). Exact mass calculated for $C_{24}H_{24}N_4O_8S$ 452.49, found 453.2 ($MH^+$).

**Compound B102**

**(4-Fluoro-phenyl)-{1-[6-(2-fluoro-phenylamino)-5-nitro-pyrimidin-4-yl]-pipendin-4-yl}-methanone**

**[Method 16].** yellow solid (16mg, 44%). $^1$HNMR ($CDCl_3$, 400 MHz) δ 1.89 (m, 4H), 3.26 (m, 2H), 3.51 (m, 1H), 3.96 (m, 2H), 7.10 (m, 5H), 7.92 (m, 2H), 8.08 (s, 1H), 8.13 (m, 1H), 10.06 (s, 1H). Exact mass calculated for $C_{24}13_{24}N_4O_8S$ 439.41, found 440.3 ($MH^+$).

**Compound B103**

**{1-[6-(Benzo[1,3]dioxol-5-ylamino)-5-nitro-pyrimidin-4-yl]-piperidin-4-yl}-(4-fluorophenyl)-methanone**

**[Method 16].** orange solid (20 mg, 53 %). $^1$HNMR ($CDCl_3$, 400 MHz) δ 1.89 (m, 4H), 3.26 (m, 2H), 3.51 (m, 1H), 3.96 (m, 2H), 5.92 (s, 2H), 6.76 (m, 2H), 7.10 (m, 3H), 7.92 (m, 2H), 8.03 (s, 1H), 9.91 (s, 1H). Exact mass calculated for $C_{23}H_{20}FN_5O_5$ 465.43, found 466.3 ($MH^+$).

**Compound B104**

**(2,3-Difluoro-phenyl)-{5-nitro-6-[4-(pyridin-2-ylsulfanyl)-piperidin-1-yl]-pyrimidin-4-yl}-amine**

**[Method 16].** yellow solid (5 mg, 8 %). $^1$HNMR ($CDCl_3$, 400 MHz) δ 1.78 (m, 2H), 2.17 (m, 2H), 3.35 (m, 2H), 3.83 (m, 2H), 4.14 (m, 1H), 6.94 (m, 2H), 7.03 (m, 1H), 7.10 (m, 1H), 7.42 (m, 1H), 7.89 (m, 1H), 8.08 (s, 1H), 8.37 (m, 1H), 10.05 (s, 1H). Exact mass calculated for $C_{20}H_{18}F_2N_6O_2S$ 444.46, found 444.9 ($M+H^+$).

**Compound B105**

**(2,4-Difluoro-phenyl)-{5-nitro-6-[4-(pyridin-2-ylsulfanyl)-piperldin-1-yl]-pyrimidin-4-yl}-amine**

**[Method 16].** yellow solid (12 mg, 19 %). $^1$HNMR ($CDCl_3$, 400 MHz) δ 1.78 (m, 2H), 2.17 (m, 2H), 3.35 (m, 2H), 3.83 (m, 2H), 4.14 (m, 1H), 6.85 (m, 2H), 6.93 (m, 1H), 7.10 (m, 1H), 7.42 (m, 1H), 7.89 (m, 1H), 8.05 (s, 1H), 8.37 (m, 1H), 9.91 (s, 1H). Exact mass calculated for $C_{20}H_{18}F_2N_6O_2S$ 444.46, found 445.4 ($M+H^+$).

**Compound B106**

**(2,5-Difluoro-phenyl)-{5-nitro-6[4-(pyridin-2-ylsulfanyl)-piperidin-l-yl]-pyrimidin-4-yl}-amine**

**[Method 16].** yellow solid (3 mg, 5 %). $^1$HNMR ($CDCl_3$, 400 MHz) δ 1.78 (m, 2H), 2.17 (m, 2H), 3.35 (m, 2H), 3.83 (m, 2H), 4.14 (m, 1H), 6.73 (m, 1H), 6.93 (m, 1H), 7.03 (m, 1H), 7.11 (m, 1H), 7.42 (m, 1H), 8.13 (s, 1H), 8.25 (m, 1H), 8.37 (m, 1H), 10.25 (s, 1H). Exact mass calculated for $C_{20}H_{18}F_2N_6O_2S$ 444.46, found 445.3 ($M+H^+$).

**Compound B107**

**1-[6-(4-Benzenesulfonyl-phenylamino)-5-nitro-pyrimidin-4-yl]-pipendine-4-carboxylic acid ethyl ester**

**[Method 16].** yellow solid (32 mg, 39 %). $^1$HNMR ($CDCl_3$,400 MHz) δ 1.22 (t, 3H), 1.80 (m, 2H), 2.00 (m, 2H), 2.60 (m, 1H), 3.20 (t, 2H), 3.85 (m, 2H), 4.12 (q, 2H), 7.48 (m, 3H), 7.78 (d, 2H), 7.90 (m, 4H), 8.11 (s, 1H), 10.19 (s, 1H). Exact mass calculated for $C_{24}H_{25}N_5O_6S$ 511.55, found 512.3 ($MH^+$).

**Compound B108**

**1-[5-Nitro-6-(2-trifluoromethyl-3H-benzoimidazol-5-ylamino)-pyrimidin-4-yl]-piperidine-4-carboxylic acid ethyl ester**

**[Method 16].** yellow solid (11mg, 14%). $^1$HNMR ($CDCl_3$, 400 MHz) δ 1.40 (t, 3H), 2.00 (m, 2H), 2.18 (m, 2H), 2.79 (m, 1H), 3.39 (t, 2H), 4.06 (m, 2H), 4.30 (q, 2H), 7.43 (m, 1H), 7.65 (m, 1H), 7.95 (m, 1H), 8.26 (m, 2H), 10.40 (s, 1H). Exact mass calculated for $C_{20}H_{20}F_3N_7O_4$ 479.41, found 480.3 ($MH^+$).

**Compound B109**

**1-{5-Nitro-6-[3-(1,1,2,2-tetrafluoro-ethoxy)-phenylamino]-pyrimdin-4-yl}-pipendine-4-carboxylic acid ethyl ester**

**[Method 16].** yellow solid (65 mg, 84 %). $^1$HNNR ($CDCl_3$, 400 MHz) δ 1.36 (t, 3H), 1.96 (m, 2H), 2.14 (m, 2H), 2.74 (m, 1H), 3.34 (m, 2H), 4.01 (m, 2H), 4.26 (q, 2H), 6.02 (m, 1H), 7.14 (m, 1H), 7.47 (m, 1H), 7.56 (m, 1H), 7.77 (m, 1H), 8.24 (s, 1H), 10.23 (s, 1H). Exact mass calculated for $C_{20}H_{21}F_4N_5O_5$ 487.40, found 488.2 ($MH^+$).

**Compound B110**

**{6-[4-(4-lodo-phenoxy)-piperidin-1-yl]-5-nitro-pyrimidin-4-yl}-(4-methanesulfonylphenyl)-amine**

Yellow solid; yield 82.6%. $^1$H NMR 400MHz $CDCl_3$ δ (ppm): 10.2 (s, 1H); 8.13 (s, 1H); 7.86 (m, 4H); 7.50 (m, 2H); 6.64 (m, 2H); 4.55 (m, 1H); 3.65 (m, 2H); 3.55 (m, 2H); 2.98 (s, 3H); 1.96 (m, 4H). LCMS (ESI) m/z 596 ($M^+H^+$, 100%).

**Compound B111**

**(2-Fluoro-4-methanesulfonyl-phenyl)-16-[4-(3-isopropyl-[1,2,4]oxadiazol-5-yl)-piperidin-1-yl]-5-nitro-pyrimidin-4-yl}-amine**

Following the general procedure, Compound **B111** was obtained as a yellow solid (38%). $^1$H NMR 400MHz $CDCl_3$ δ 10.3(s, 1H); 8.74(t,1H); 8.17(s, 1H); 7.70 (d, 1H); 7.67(d, 1H); 3.95-3.92(m,2H); 3.33-3.27(m,2H); 3.29-3.23(m, 1H); 3.00(s,3H); 3.03-2.96(m, 1H); 2.19-2.11 (m, 2H); 2.03-1.96(m, 2H); 1.26(d, 6H). Exact mass calculated for $C_{21}H_{24}FN_7O_5S$ 505.15, LCMS (ESI) m/z 506.2($M+H^+$, 100%).

**Compound B112**

**{6-[4-(3-Ethyl-[1,2,4]oxadiazol-5-yl)-piperidin-1-yl]-5-nitro-pyrimidin-4-yl}-(2-fluoro-4-methanesulfonyl-phenyl)-amine**

Following the general procedure, Compound **B112** was obtained as a yellow solid (31%). $^1$HNMR ($CDCl_3$,400 MHz) δ 1.25 (t, 3H), 2.06-2.10 (m, 2H), 2.23-2.27 (m, 2H), 2.70 (q, 2H), 2.99 (s, 3H), 3.31-3.38 (m, 3H), 3.96-3.99 (m, 2H), 7.84 (dd, 4H), 8.14 (s, 1H), 10.2 (s, 1H). Exact mass calculated for $C_{20}H_{23}N_7O_5S$ 473.1, found 474.2 ($MH^+$).

**Compound B113**

**(4-Methanesulfonyl-phenyl)-{5-nitro-6-[4-(3-propyl-[1,2,4] oxadiazol-5-yl)-piperidin-1-yl]-pyrimidin-4-yl}-amine**

Following the general procedure, Compound **B113** was obtained as a yellow solid (44%). $^1$H NMR 400MHz $CDCl_3$ δ 10.2(s, 1H); 8.13(s, 1H); 7.87 (d, 2H); 7.83(d, 2H); 3.92(m,2H); 3.32-3.29(m,2H); 3.26-3.23(m,1H); 2.99(s,3H); 2.62(t, 2H); 2.19-2.15(m, 2H); 2.01-1.95(m, 2H); 1.69(se, 2H); 0.91 (t, 3H). Exact mass calculated for $C_{21}H_{25}$ $N_7O_5S$ 487.16, LCMS (ESI) m/z 488.2(M+H$^+$, 100%).

**Compound B114**

**{6-[4-(3-Cyclopropylmethyl-[1,2,4]oxadiazol-5-yl)-piperidin-1-yl]-5-nitro-pyrimidin-4-yl}-(4-methanesnlfonyl-phenyl)-amine**

Following the general procedure, Compound **B114** was obtained as a yellow solid (45%). $^1$H NMR 400MHz $CDCl_3$ δ 10.2(s, 1H); 8.15(s, 1H); 7.87 (d, 2H); 7.78(d, 2H); 3.95(m,2H); 3.34-3.30(m,2H); 3.30-3.27(m,1H); 3.00(s,3H); 2.57(d, 2H); 2.21-2.17(m, 2H); 2.04-1.96(m, 2H); 1.06-1.02(m, 1H); 0.53-0.48(m, 2H); 0.25-0.16(m, 2H). Exact mass calculated for $C_{22}H_{25}$ $N_7O_5S$ 499.16, LCMS (ESI) m/z 500.5(M+H$^+$, 100%).

**Compound B115**

**{6-[4-(3-Isopropyl-[1,2,4]oxadiazol-5-yl)-piperidin-1-yl]-5-nitro-pyrimidin-4-yl}-(4-methanesulfonyl-phenyl)-amine**

Following the general procedure, Compound **B115** was obtained as a yellow solid (76%). $^1$H NMR 400MHz $CDCl_3$ δ 10.2 (s, 1H); 8.14 (s, 1H); 7.85 (dd, 4H); 3.92 (d, 2H); 3.27 (m, 3H); 3.0 (s, 3H); 2,14 (m, 2H); 1.99 (m, 2H); 1.25 (d, 6H). LCMS (ESI) m/z 488 ($M^+H^+$, 100%)

**Compound B116**

**{6-[4-(3-Cyclopropyl-[1,2,4]oxadiazol-5-yl)-piperidin-1-yl]-5-nitro-pyrimidin-4-yl}-(4-methanesulfonyl-phenyl)-amine**

Following the general procedure, Compound **B116** was obtained as a yellow solid (83.6%). $^1$H NMR 400MHz $CDCl_3$ δ 10.2 (s, 1H); 8.14 (s, 1H); 7.86 (dd, 4H); 3.90 (d, 2H); 3.30 (m, 2H); 3.27 (m, 1H); 3.00 (s, 3H); 2.13 (m, 2H); 1.98 (m, 3H); 0.97 (m, 4H). LCMS (ESI) m/z 486 ($M^+H^+$, 100%)

**Compound B117**

**(4-Methanesulfonyl-phenyl)-(5-nitro-6-{4-[3-(3-trifluoromethyl-phenyl)-[1,2,4]oxadiazol-5-yl]-piperidin-1-yl}-pyrimidin-4-yl)-amine**

Following the general procedure, Compound **B117** was obtained as a yellow solid (61%). $^1$HNMR ($CDCl_3$,400 MHz) δ 2.06-2.10 (m, 2H), 2.23-2.27 (m, 2H), 2.99 (s, 3H), 3.31-3.38 (m, 3H), 3.96-3.99 (m, 2H), 7.55 (t, 1H), 7.70 (d, 1H), 7.86 (dd, 4H), 8.16 (s, 1H), 8.20 (d, 1H), 8.28 (s, 1H), 10.2 (s, 1H). Exact mass calculated for $C_{25}H_{22}F_3N_7O_5S$ 589.1, found 590.4 ($MH^+$).

**Compound B118**

**4-[4-(3-Isopropyl-[1,2,4]oxadiazol-5-yl)-piperidin-1-yl]-6-(4-methanesulfinylphenylamino)-pyrimidine-5-carbonitrile**

Compound B99 was selectively oxidized using mCPBA to give Compound **B118** as the sulfoxide. $^1$H-NMR (DMSO-$d_6$): 9.65 (1H, s), 8.26 (1H, s), 7.75 (2H, m), 7.63 (2H, m), 4.52 (2H, m), 3.45 (1H, m), 3.32 (2H, m), 3.04 (1H, m), 2.73 (3H, s), 2.18 (2H, m), 1.79 (2H, m) 1.18 (6H, J = 7Hz, d) ppm. LCMS: 468.4, 384.1, 356.2, 302.1.

**Compound B119**

**(4-Methanesulfonyl-phenyl)-{5-nitro-6-[4-(4-tritluoromethoxy-phenoxy)-piperidin-1-yl]-pyrimidin-4-yl}-amine**

A mixture of compound (6-chloro-5-nitro-pyrimidin-4-yl)-(4-methanesulfonyl-phenyl)-amine (400 mg, 1.22 mmol), 4-(4-trifluoromethoxy-phenoxy)-piperidine (399 mg, 1.34 mmol) and potassium carbonate (336 mg, 2.44 mmol) in DMF (8 ml) was heated in an oil bath at 60˚C for 2 hours. The crude mixture was cooled to 0˚C and quenched with water. The solid was filtered off, rinsed with water and dried in vacuum oven to give product **B119** as a yellow solid (604 mg, 90%). $^1$HNUR ($CDCl_3$, 400 MHz) d 2.01-2.08 (m, 4H), 3.06 (s, 3H), 3.64-3.66 (m, 2H), 3.73-3.75 (m, 2H), 4.62-4.66 (m, 1H), 6.93 (d, 2H), 7.17 (d, 2H), 7.93 (dd, 4H), 8.21 (s, 1H), 10.2 (s, 1H). Exact mass calculated for $C_{23}H_{22}F_3N_5O_6S$ 553.1, found 554.3 ($MH^+$).

**Compound B120**

**4-[4-(3-Isopropyl-[1,2,4]oxadiazol-5-yl)-piperidin-1-yl]-6-(4-methanesulfonylphenylamino)-pyrimidine-5-carbonitrile**

Compound B99 was oxidized using mCPBA to give Compound **B120** as the sulfone. $^1$H NMR (DMSO-$d_6$): 9.86 (1H, s), 8.34 (1H, s), 7.93 ~ 7.84 (4H, m), 4.54 (2H, m), 3.50 ~ 3.39 (3H, m), 3.21 (3H, s) 3.05 (1H, m), 2.21 (2H, m), 1.83 (2H, m) 1.27 (6H, J = 7Hz, d) ppm. LCMS: 452.1, 437.2,368.1,340.0.

**Compound B121**

**1-{1-[6-(2-Fluoro-4-methanesulfonyl-phenylamino)-5-nitro-pyrimidin-4-yl]-piperidin-4-yl}-hexan-1-one**

The general procedure for the addition of Amine to pyrimidine afforded Compound **121;** $^1$H NMR, 400MHz, $CDCl_3$, $\delta$ (ppm): 10.48(s, NH); 8.84(s, 1H); 8.23(s, 1H); 7.75(m, 2H); 3.98(m, 2H); 3.22(m, 2H); 3.06 (s, $CH_3$); 2.69(m, 1H); 2.48(m, 2H); 1.99(m, 2H); 1.77(m, 2H), 1.59(m, 2H), 1.29(m, 4H), 0.89(t, 3H); LCMS (ESI) for $C_{22}H_{28}FN_5O_5S$: m/z 493($M+H^+$, 100%).

**Compound B122**

**1-{1-[6-(4-Methanesulfonyl-phenylamino)-5-nitro-pyrimidin-4-yl]-piperidin-4-yl}-hexan-1-one**

The general procedure for the addition of Amine to pyrimidine afforded Compound **122;** $^1$H NMR, 400MHz, $CDCl_3$, $\delta$ (ppm): 10.2(s, NH); 8.20(s, 1H); 7.92(m, 4H); 3.98(m, 2H); 3.22(m, 2H); 3.06 (s, $CH_3$); 2.69(m, 1H); 2.48(m, 2H); 1.99(m, 2H); 1.77(m, 2H), 1.59(m, 2H), 1.29(m, 4H), 0.89(t, 3H); LCMS (ESI) for $C_{22}H_{29}N_5O_5S$: m/z 476($M+H^+$, 100 %).

**Compound B123**

**{6-[4-(3-tert-Butyl-[1,2,4]oxadiazol-5-yl)-piperidin-1-yl]-5-nitro-pyrimidin-4-yl}-(2-fluoro-4-methanesulfonyl-phenyl)-amine**

The general procedure for the addition of Amine to pyrimidine afforded Compound **B123** as yellow oil (40mg, 51%). $^1$H NMR 400MHz $CDCl_3$ d(ppm): 10.4(s,NH); 8.83(t, 1H); 8.25(s, 1H); 7.76 (t, 2H); 4.01(d,2H); 3.41-3.34(m, 2H); 3.32-3.28(m, 1H); 3.08(s,3H); 2.27-2.22(m, 2H); 2.11-2.04(m, 2H); 3.36(s, 9H). Exact mass calculated for: $C_{23}H_{27}FN_6O_5S$ 518.17, LCMS (ESI) m/z 520.4($M+H^+$, 100%).

**Compound B124**

**{6-[4-(3-tert-Butyl-[1,2,4]oxadiazol-5-yl)-piperidin-1-yl]-5-nitro-pyrimidin-4-yl}-(4-methanesulfonyl-phdnyl)-amine**

The general procedure for the addition of amine to pyrimidine afforded Compound **124** as a yellow solid, yield 90%; $^1H$ NMR 400MHz $CDCl_3$ δ (ppm): 10.2 (s, 1H); 8.23 (s, 1H); 7.97 (d, 2H); 7.76 (d, 2H); 4.02 (d, 2H); 3.44 (m, 3H); 3.2 (s, 3H); 2,27 (m, 2H); 2.03 (m, 2H); 1.37 (s, 9H); LCMS (ESI) m/z 502 ($M^+H^+$, 100%)

**Compound B125**

**[6-(4-Benzofuran-2-yl-piperidin-1-yl)-5-nitro-pyrimidin-4-yl]-(4-methanesulfonylphenyl)-amine**

The general procedure for the addition of amine to pyrimidine afforded Compound **B125** as a yellow solid (82mg, 91%). $^1H$ NMR ($CDCl_3$, 400 MHz) δ 1.00 (s, 1H), 1.54 (m, 2H), 1.93 (m, 2H), 2.97 (s, 3H), 3.09 (m, 2H), 3.74 (m, 2H), 6.42 (s, 1H), 6.98 (m, 3H), 7.27 (d, 1H), 7.32 (d, 1H), 7.66 (m, 4H), 7.97 (s, 1H). Exact mass calculated for $C_{20}H_{21}F_2N_7O_3$ 493.53, found 494.4 ($M+H^+$).

**Compound B126**

**4-(3-Fluoro-4-methanesulfonyl-phenylamino)-6-[4-(3-isopropyl-[1,2,4] oxadiazol-5-yl)-piperidin-1-yl]-pyrimidine-5-carbonitrile**

Compound **B126** was obtained as a solid (90%). $^1H$NMR ($CDCl_3$, 400 MHz) δ 1.34 (d, 6H), 2.01-2.08 (m, 2H), 2.23-2.27 (m, 2H), 3.06 (heptet, 1H), 3.22 (s, 3H), 3.30-3.34 (m, 1H), 3.46-3.50 (m, 2H), 4.70-4.77 (m, 2H), 7.33 (dd, 1H), 7.40 (s, 1H), 7.90 (t, 1H), 8.08 (dd, 1H), 8.38 (s, 1H). Exact mass calculated for $C_{22}H_{24}FN_7O_3S$ 485.2, found 486.3 ($MH^+$).

**Compound B127**

**{6-[4-(3-Isopropyl-[1,2,4]oxadiazol-5-yl)-piperidin-1-yl]-5-nitro-pyrimidin-4-yl}-(5-methanesulfonyl-pyridin-2-yl)-amine**

Compound **B127** was prepared by the general procedure for the addition of pyridinylsulfone to pyrimidine to give a solid (4 mg, 4%). $^1H$ NMR 400MHz $CDCl_3$ δ(ppm): 10.5(s, NH); 8.86(s,1H); 8.74(d, 1H); 8.29(s,1H); 8.20(d, 1H); 4.02-3.99(m,2H); 3.40-3.28(m, 3H); 3.11(s,3H); 3.11-3.06(m, 1H); 2.26-2.22(m, 2H); 2.09-2.03(m, 2H); 1.33(d, 6H). Exact mass calculated for $C_{20}H_{24}N_8O_5S$ 488.16, LCMS (ESI) m/z 489.3($M+H^+$, 100%).

**Compound B128**

**(3-Fluoro-4-methanesulfonyl-phenyl)-{6-[4-(3-isopropyl-[1,2,4]oxadiazol-5-yl)-piperidin-1-yl]-5-nitro-pyrimidin-4-yl}-amine**

Compound **B128** was prepared using the general procedure for the oxidation of a sulfide to sulfone; yellow solid (9 mg, 36%). $^1H$ NMR 400MHz $CDCl_3$ δ(ppm): 10.3(s,NH); 8.25(s, 1H); 8.13(d, 1H); 7.92(t,1H); 7.40(d,1H); 4.00 (db,2H); 3.41-3.29(m,3H); 3.23(s,3H);3.10-3.07(m, 1H); 2.26-2.24(m,2H); 2.10-2.02(m,2H); 1.34(d,6H). Exact mass calculated for $C_{21}H_{24}FN_7O_5S$ 505.15, LCMS (ESI) m/z 506.3($M+H^+$, 100%).

**Compound B129**

**{6-[4-(3-Isopropyl-[1,2,4]oxadiazol-5-yl)-piperidin-1-yl]-5-nitro-pyrimidin-4-yl}-(6-methanesulfonyl-pyridin-3-yl)-amine**

Compound **B129** was prepared using the general procedure for the oxidation of a sulfide to sulfone; yellow solid (6 mg, 67%). $^1H$ NMR 400MHz $CDCl_3$ δ(ppm): 10.3(s,NH); 8.93(s, 1H); 8.52(d, 1H); 8.22(s,1H); 8.10(d,1H); 4.00 (db,2H); 3.41-3.31(m,3H); 3.23(s,3H); 3.08(qu, 1H); 2.27-2.23(m,2H); 2.10-2.04(m,2H); 1.33(d,6H).Exact mass calculated for $C_{20}H_{24}N_8O_5S$ 488.16, LCMS (ESI)m/z489.2($M+H^+$, 100%).

**Compound B130**

**4-(2,3-Difluoro-phenylamino)-6-[4-(3-isopropyl-[1,2,4]oxadiazol-5-yl)-piperidin-l-yl]-pyrimidine-5-carbonitrile**

To a solution of 4,6-dichloro pyrimidine-5-carbonitrile (254 mg, 1.47 mmol) and 2,3-difluoroaniline (190 mg, 1.47 mmol) in DMF (3 mL) at 0 ˚C was added $K_2CO_3$ (203 mg, 1.47 mmol). The completion of the reaction was monitored by TLC (EtOAc:Hex 1:1, $R_f$= 0.90). After the completion of the reaction, 4-(3-isopropyl-[1,2,4]oxadiazol-5-yl)-piperidine (340 mg, 1.47 mmol) and $K_2CO_3$ (406 mg, 2.94 mmol) were added at 0˚C. The reaction was warmed to rt and stirred for 30 min. The reaction was heated to 40˚C and maintained for 1 h. The reaction was cooled to rt, poured in to $H_2O$ (50 mL) and extracted with EtOAc (50 mL, two times). The EtOAc was dried over $MgSO_2$ and concentrated under vacuum. The crude product was purified over $SiO_2$ (EtOAc:Hex =1:1, $R_f$= 0.49) to afford Compound **B130** (501 mg; 76.7 %). $^1$H-NMR (DMSO-$d_6$): 9.60 (1H, s), 8.28 (1H, s), 7.45~7.29 (3H, m), 4.64 (2H, m), 3.59~3.52 (4H, m), 3.41~3.49 (2H, m), 2.29~2.25 (2H, b), 1.96~1.86 (2H, m), 1.39 (6H, d) ppm. LCMS: 426.43.

**Compound B131**

**4-(2,5-Difluoro-phenylamino)-6-[4-(3-isopropyl-[1,2,4]oxadiazol-5-yl)-piperidin-1-yl]-pyrimidine-5-carbonitrile**

To a solution of 4,6-Dichloro-pyrimidine-5-carbonitrile (254 mg, 1.47 mmol) and 2,5-difluoroaniline (190 mg, 1.47 mmol) in DMF (3 mL) at 0˚C was added $K_2CO_3$ (203 mg, 1.47 mmol). The completion of the reaction was monitored by TLC (EtOAc:Hex =1:1, $R_f$= 0.90). After the reaction was complete, 4-(3-isopropyl-[1,2,4]oxadiazol-5-yl)-piperidine (340 mg, 1.47 mmol) and $K_2CO_3$ (406 mg, 2.94 mmol) were added at 0˚C. The reaction was warmed to rt and stirred for 30 min. The reaction was heated to 40˚C and maintained for 1 h. The reaction was cooled to rt, poured in to $H_2O$ (50 mL) and extracted with EtOAc (50 mL, two times). The EtOAc was dried over $MgSO_2$ and concentrated under vacuum. The crude product was purified over $SiO_2$ (EtOAc:Hex =1:1, $R_f$= 0.44) to afford the desired Compound **B131** (465 mg; 71.1 %). $^1$H-NMR (DMSO-$d_6$): 9.32 (1H, s), 8.19 (1H, s), 7.40~7.08 (3H, m), 4.54 (2H, m), 3.48~3.32 (4H, m), 3.07~3.01 (2H, m), 2.18~2.14 (2H, b),1.96~1.86 (2H, m), 1.27 (6H, d) ppm. LCMS: 426.43.

**Compound B132**

**4-[4-(3-Isopropyl-[1,2,4]oxadiazol-5-yl)-piperidin-1-yl]-6-(4-methylsulfanyl-phenylamino)-pyrimidine-5-carbonitrile**

Compound **B132** was prepared by the general procedure for the addition of amine to pyrimidine as described herein using 4-chloro-6-(4-methylsulfanyl-phenylamino)-pyrimidine-5-carbonitrile and 4-(3-isopropyl-[1,2,4]oxadiazol-5-yl)-piperidine. $^1$H-NMR (DMSO-d6): 9.38 (1H, s), 8.21 (1H, s), 7.47 (2H, J=4.3 Hz, d), 7.23 (2H, J = 4.3 Hz, d), 4.50 (2H, m), 3.35 (2H, m), 3.02 (1H, m), 2.51 (3H, s), 2.18 (2H, m), 1.79 (2H, m) 1.83 (6H, J = 7Hz, d) ppm. LCMS: 436.3, 351.9, 324.4, 270.2.

Intermediate 4-chloro-6-(4-methylsulfanyl-phenylamino)-pyrimidine-5-carbonitrile was prepared in a similar manner as described herein using 4,6-dichloro-pyrimidine-5-carbonitrile and 4-methylsulfanyl-phenylamine. $^1$H-NMR (DMSO-d6): 10.22 (1H, s), 8.53 (1H, s) 7.43 (2H, m), 7.40 (2H, m), 2.49 (3H, s) ppm. LCMS: 277.0, 234.0, 149.0.

**Compound B133**

**4-[4-(3-Isopropyl-[1,2,4]oxadiazol-5-yl)-piperidin-1-yl]-6-(4-methanesulfonyl-phenylamino)-pyrimidine-5-carbonitrile**

To a solution of 4-[4-(3-isopropyl-[1,2,4]oxadiazol-5-yl)-piperidin-1-yl]-6-(4-methylsulfanyl-phenylamino)-pyrimidine-5-carbonitrile (200mg, 0.46 mmol) in dichloromethane (5 mL) at 0˚C was added 216 mg of *m*-CPBA (0.94 mmol). The reaction was stirred for 10 min and warmed to room temperature. The reaction was maintained for 2h at the same temperature and the completion of the reaction was judged by TLC. The reaction was concentrated under vacuum and purified over $SiO_2$ (ethyl acetate/ Hex =1/1m $R_f$= 0.69) to afford Compound **B133** (167 mg, 80%). $^1$H-NMR (DMSO-d6): 9.86 (1H, s), 8.34 (1H, s), 7.93 ~ 7.84 (4H, m), 4.54 (2H, m), 3.50 ~ 3.39 (3H, m), 3.21 (3H, s) 3.05 (1H, m), 2.21 (2H, m), 1.83 (2H, m) 1.27 (6H, J= 7Hz, d) ppm. LCMS: 452.1, 437.2, 368.1, 340.0.

**Compound B134**

**4-(4-Hexanoyl-piperidin-1-yl)-6-(6-methylsulfanyl-pyridin-3-ylamino)-pyrimidine-5-carbonitrile**

4-Chloro-6-(4-methylsulfanyl-phenylamino)-pyrimidine-5-carbonitrile (150.00mg, 0.54mmol) in DMF (2ml) was mixture with Potassium Carbonate (82.1mg, 0.59mmol), added 1-piperidin-4-yl-hexan-1-one hydrochloride (237.4mg, 1.08 mmol) in DMF (1ml) and left stirring at room temperature for 1 hour. Reaction was worked up with ethyl acetate, sodium bicarbonate, dried with magnesium sulfate, filtered and concentrate under high vacuum to afford a lithe yellow solid as product. Compound was recrystalized using hexane in ethyl acetate to afford Compound **B134** (157mg). LCMS (ESI) for $C_{22}H_{28}N_6OS$: m/z 425.4 ($M + H^+$, 100%). $^1$H NMR 400MHz $CDCl_3$ $\delta$ (ppm): 8.72 (d, 1H), 8.40 (s, 1H),7.97 (q, 1H), 7.21 (s,1H,NH), 4.92 (m, 2H), 3.42 (m, 2H), 2.86 (m, 1H), 2.75 (s, 3H), 2.65 (t, 2H), 2.15 (m, 2H), 1.77 (m, 2H), 1.50(m, 2H), 1.50 (m, 2H), 1.07 (t, 3H).

**Compound B135**

**4-(4-Hexanoyl-piperidin-1-yl)-6-(6-methanesulfonyl-pyridin-3-ylamino)-pyrimidine-5-carbonitrile**

4-(4-Hexanoyl-piperidin-1-yl)-6-(6-methylsulfanyl-pyridin-3-ylamino)-pyrimidine-5-carbonitrile (100 mg, 0.236mmol) in chloroform was mixture with *m*CPBA (122.0mg, 0.71mmol) at 0 C under stirring, left reaction warmed up to room temperature and reacted for an additional 12 hours. Worked up with water (pH=10 using ammonium hydroxide as base), chloroform, sodium bicarbonate, dried with magnesium sulfate, concentrate under high vacuum and crystallized using hexane and ethyl acetate to afford Compound **B135** as a solid (90mg,84%). LCMS (ESI) for $C_{22}H_{28}N_6O_3S$: m/z 457.2 ($M + H^+$, 100%). $^1$H NMR 400MHz $CDCl_3$ $\delta$ (ppm): 8.87 (d, 1H), 8.46 (q, 1H), 8.32 (s,1H), 8.07(d,1H), 7.42 (s,1H, NH), 4.74 (m, 2H), 3.48 (m, 2H),3.21(s, 3H), 2.70 (m, 1H), 2.48 (t, 2H), 1.98 (m, 2H), 1.58 (m, 2H), 1.27(m, 2H), 1.27 (m, 2H), 0.89 (t, 3H).

**Compound B136**

**4-[4-(3-Isopropyl-[1,2,4]oxadiazol-5-yl)-piperidin-1-yl]-6-(6-methylsulfanyl-pyridin-3-ylamino)-pyrimidine-5-carbonitrile**

4-Chloro-6-(6-methylsulfanyl-pyridin-3 ylamino)-pyrimidine-5-carbonitrile (80.00mg, 0.29mmol) in DMF (2m1) was mixture with Potassium Carbonate (79.62mg, 0.58mmol), added 4-(3-isopropyl-[1,2,4]oxadiazol-5-yl)-piperidine hydrochloride (237.4 mg, 1.08 or 1.02 mmol) in DMF (1ml) and left stirring at room temperature for 1 hour. Reaction was worked up with ethyl acetate, sodium bicarbonate, dried with magnesium sulfate, filtered and concentrate under high vacuum. Compound was recrystallized using hexane in ethyl acetate to afford Compound **B136.** $^1$H NMR 400MHz DMSO $\delta$ (ppm): 9.48 (s,1H), 8.55 (d, 1H), 8.35 (s, 1H), 8.19 (s, 1H), 7.77 (d, 1H), 7.26 (d,1H), 4.52(m, 2H), 3.48 (m, 1H), 3.37 (m, 2H), 3.32 (s, 3H), 3.04 (m, 1H), 2.14 (m, 2H), 1.75 (m, 2H), 1.33 (d, 6H).

**Compound B137**

**4-[4-(3-Isopropyl-[1,2,4]oxadiazol-5-yl)-piperidin-1-yl]-6-(6-methanesulfonyl-pyridin-3-ylamino)-pyrimidine-5-carbonitrile**

4-[4-(3-Isopropyl-[1,2,4]oxadiazol-5-yl)-piperidin-1-yl]-6-(6-methylsulfanyl-pyridin-3-ylamino)-pyrimidine-5-carbonitrile (26.0mg, 0.06mmo1) in chloroform was mixture with *m*CPBA (41.1mg, 0.24mmol) at 0 C under stirring, left reaction warmed up to room temperature and reacted for an additional 12 hours. Worked up with water (pH=10 using ammonium hydroxide as base), chloroform, sodium bicarbonate, dried with magnesium sulfate, concentrate under high vacuum and crystallized using hexane and ethyl acetate to afford Compound **B137** as a solid (7.5 mg). $^1$H NMR 400MHz DMSO $\delta$ (ppm): 8.88 (d, 1H), 8.55 (d, 1H), 8.35 (s, 1H), 8.08 (d, 1H), 7.44 (s, 1H), 4.72 (m, 2H), 3.48 (m, 1H), 3.30 (m, 1H), 3.22 (s, 3H), 3.08 (m, 1H), 2.23 (m, 2H), 2.03 (m, 2H), 1.33 (d, 6H).

**Compound B138**

**1-[4-[4-(3-Isopropyl-[1,2,4]oxadiazol-5-yl)-piperidin-1-yl]-6-(4-methanesulfonyl-phenylamino)-pyrimidin-5-yl]-ethanone**

To a solution of 1-[4-Chloro-6-(4-methanesulfonyl-phenylamino)-pyrimidin-5-yl]-ethanone (0.21 mmol, 70 mg)

and 4-(3-isopropyl-[1,2,4]oxadiazol-5-yl)-piperidine hydrochloride (0.21 mmol, 49 mg) in N,N-dimethyl formamide (500 uL) was added potassium carbonate (0.21 mmol, 29 mg). The resulting mixture was microwaved at 100 °C for 150 seconds. Its progress was monitored by thin layer chromatography and LCMS. The reaction was treated with water and the desired compound was extracted in ethyl acetate. Organic layer was evaporated in vacuo. Purification by HPLC provided Compound **B138** as a white solid (15 mg, 15%). $^1$H-NMR (400 MHz, $CDCl_3$) δ(ppm): 10.85 (s, 1H); 8.32 (s, 1H); 7.92 (s, 4H); 4.07 (m, 2H); 3.40 (m, 2H); 3.29 (h, 1H); 3.09 (m, 1H); 3.07 (s, 3H); 2.47 (s, 3H); 2.18 (m, 2H); 2.03 (m, 2H), 1.33 (d, 6H). LCMS (ESI), m/z 485.3 (M+H+, 100%).

**Compound B139**

**1-[4-[4-(3-Isopropyl-[1,2,4]oxadiazol-5-yl)-piperidin-1-yl]-6-(6-methanesulfonyl-pyridin-3-ylamino)-pyrimidin-5-yl]-ethanone**

To a solution of 1-[4-chloro-6-(6-methanesulfonyl-pyridin-3-ylamino)-pyrimidin-5-yl]-ethanone (0.21 mmol, 70 mg) and 4-(3-isopropyl-[1,2,4]oxadiazol-5-yl)-piperidine hydrochloride (0.21 mmol, 49 mg) in N,N-dimethyl formamide (500 uL) was added potassium carbonate (.21 mmol, 29 mg). The mixture was microwaved at 100 °C for 150 seconds. Its progress was monitored by thin layer chromatography and LCMS. The reaction was treated with water and the desired compound was extracted in ethyl acetate. Organic layer was evaporated in vacuo. Purification by HPLC provided Compound **B139** as a white solid (31 mg, 31%). $^1$H-NMR (400 MHz, $CDCl_3$) δ(ppm): 10.9 (s, 1H); 8.90 (d, 1H); 8.58 (d, 1H); 8.32 (s, 1H); 8.05 (d, 1H); 4.09 (m, 2H); 3.41 (m, 2H); 3.29 (h, 1H); 3.23(s, 3H); 3.09 (h, 1H); 2.48 (s, 3H), 2.18 (m, 2H), 2.02 (m, 2H), 1.35 (d, 6H)LCMS (BSI), m/z 486.3 (M+H+, 100%).

**EXAMPLE 14**

**SYNTHESES OF COMPOUNDS OF THE PRESENT INVENTION**

**Compound C1**

**1-(5-Nitro-6-phenyl-pyrimidin-4-yl)-piperidine-4-carboxylic acid ethyl ester**

**General Method 17.** A mixture of 1-(6-chloro-5-nitropyrimidine-4-yl)-piperidine-4-carboxylic acid ethyl ester (0.157 g, 0.49 mmol), phenyl boronic acid (62.1 mg, 0.50 mmol), tetrakistriphenylphosphinepalladium(0) (11.6 mg), 2M $Na_2CO_3$ (375 μL), DME/$H_2O$/ethanol (7/3/2) (1239 μL) was heated in a microwave tube at 140 °C for 120 s in a microwave reactor. The reaction mixture was cooled and filtered and the filtrate was partitioned between ethyl acetate and water. The organic layer was washed with 1N NaOH (2x) and brine, dried ($Na_2CO_3$) and evaporated. Flash column chromatography (Biotage, silica, 20 % EtOAc/hexane) afforded the desired product **C1** in 47 % yield. $^1$H NMR, 400MHz, $CDCl_3$, δ (ppm): 8.63(s, 1H); 7.45(m, 5H); 4.16(q, 2H); 4.07(m, 2H); 3.20(t, 2H); 2.62(m, 1H); 2.03(m, 2H); 1.84(m, 2H); 1.28(t, 3H). LCMS (ESI) for $C_{18}H_{20}N_4O_4$: m/z 356 ($M+H^+$, 100 %)

**Compound C2**

**1-(6-Naphthalen-2-yl-5-nitro-pyrimidin-4-yl)-piperidine-4-carboxylic acid ethyl ester**

**[method 17].** Purification of the crude product by HPLC afforded the pure product.$^1$H NMR, 400MHz, $CDCl_3$, δ (ppm): 8.62(s, 1H); 7.93(s, 1H); 7.80 (m, 3H); 7.50(m, 3H); 4.10(q, 2H); 3.99(m, 2H); 3.16 (t, 2H); 2.58(m, 1H); 1.98 (m, 2H); 1.21(t, 3H). LCMS (ESI) for $C_{22}H_{22}N_4O_4$: m/z 406 ($M+H^+$, 100 %)

**Compound C3**

**1-[6-(4-Methanesulfonyl-phenyl)-5-nitro-pyrirnidin-4-yl]-piperidine-4-carboxylic acid ethyl ester;**

**[method 17]**. Purification of the crude product by preparatory LCMS afforded the pure product in 7 %.$^1$H NMR, 400 MHz, $CDCl_3$, δ (ppm): 8.66(s, 1H); 8.02(d, 1H); 7.66(d, 1H); 4.18(q, 2H); 4.05(m, 2H); 3.27(t, 2H); 3.09(s, 3H); 2.66 (m, 1H); 2.06(m, 2H); 1.88(m, 2H); 1.28(t, 3H). LCMS (ESI) for $C_{19}H_{22}N_4O_6S$: m/z 434 ($M+H^+$, 100 %)

**Compound C4**

**1-(6-Benzofuran-5-yl-5-nitro-pyrimidin-4-yl)-piperidine-4-carboxylic acid ethyl ester;**

**[method 17].** Flash column chromatography (Biotage, silica, 20 % EtOAc/hexane) afforded the product in 61 % yield. $^1H$ NMR, 400MHz, $CDCl_3$, δ (ppm): 8.63(s, 1H); 7.66(d, 1H); 7.57(s, 1H); 7.54(d, 1H); 7.39(t, 1H); 7.28(t, 1H); 4.15(m, 4H); 3.22(t, 2H); 2.63(m, 1H); 2.04(m,2H); 1.85(m, 2H); 1.28(t, 3H). LCMS (ESI) for $C_{22}H_{20}N_4O_5$: m/z 396 ($M+H^+$, 100 %)

**Compound C5**

**1-[5-Nitro-6-(3-trifluoromethyl-phenyl)-pyrimidin-4-yl]-piperidine-4-carboxylic acid ethyl ester;**

**[method 17].** Purification of the crude product by HPLC afforded the pure product in 11 %. $^1H$ NMR, 400MHz, $CDCl_3$, δ (ppm): 8.65 (s, 1H); 7.46 (q, 1H); 4.18 (q, 2H); 4.05 (m, 2H); 3.24 (m, 3H); 2.65 (m, 1H); 2.05 (m, 2H); 1.86 (m, 2H); 1.27 (t, 3H). LCMS (BSI) for $C_{19}H_{22}N_4O_4$: m/z 370 ($M+H^+$, 100 %)

**Compound C6**

**1-[6-(4-Methoxy-phenyl)-5-nitro-pyrimidin-4-yl]-piperidine-4-carboxylic acid ethyl ester;**

**[method 17].** Flash column chromatography (Biotage, silica, 20 % EtOAc/hexane) afforded the product in 24 % yield. $^1H$ NMR, 400MHz, $CDCl_3$, δ (ppm): 8.60(s, 1H); 7.47(d, 1H); 6.94(, 2H); 4.15(q, 2H); 4.03(m, 2H); 3.84(s, 2H); 3.17(m, 2H); 2.61(m, 1H); 1.96(m, 2H); 1.83(m, 2H); 1.27(t, 3H). LCMS (ESI) for $C_{19}H_{22}N_4O_5$: m/z 386 ($M+H^+$, 100 %)

**Compound C7**

**4-(4-Butyl-piperidin-1-yl)-6-furan-3-yl-5-nitro-pyrimidine;**

**[method 17].** Flash column chromatography (Biotage, silica, 20 % EtOAc/hexane) afforded the product in 35 % yield.$^1H$ NMR, 400MHz, $CDCl_3$, δ (ppm): 8.55(s, 1H); 7.97(s, 1H); 7.46(s, 1H); 6.73(s, 2H); 4.16(q, 2H); 3.18 (m, 2H); 2.61(m, 1H); 2.01(m, 2H); 1.83(m, 2H); 1.27(t, 3H). LCMS (ESI) for $C_{16}H_{18}N_4O_5$: m/z 346 ($M+H^+$, 100 %)

**Compound C8**

**1-[6-(3-Chloro-phenyl)-5-nitro-pyrimidin-4-yl]-piperidine-4-carboxylic acid ethyl ester**

**[method 17]**. Purification of the crude product by HPLC afforded the pure product in 14 %. $^1H$ NMR, 400MHz, $CDCl_3$, δ (ppm): 8.67(s, 1H); 7.47(m, 1H); 7.38(t, 1H); 7.32(m, 1H); 4.18(q, 2H); 4.05 (m, 2H); 3.28 (t, 2H); 2.67(m, 1H); 2.07(m, 2H); 1.88(m, 2H); 1.28(t, 3H). LCMS (ESI) for $C_{18}H_{19}ClN_4O_4$: m/z 390 ($M+H^+$, 100 %)

**Compound C9**

**1-[6-(2,6-Dimethoxy-phenyl)-5-nitro-pyrimidin-4-yl]-piperidine-4-carboxylic acid ethyl ester;**

**[method 17].** Purification of the crude product by HPLC afforded the pure product in 28 %. $^1H$ NMR, 400MHz, $CDCl_3$, δ (ppm): 8.79(s, 1H); 7.95(s, 3H); 7.40(t, 1H); 6.59(d, 2H); 4.18(q, 2H); 4.07 (m, 2H); 3.74 (s, 6H); 3.38 (t,2H); 2.70(m, 1H); 2.10(m, 2H); 1.95(m, 2H); 1.28(t, 3H). LCMS (ESI) for $C_{18}H_{19}ClN_4O_4$: m/z 390 ($M+H^+$, 100 %)

**Compound C10**

**1-(6-Naphthalen-1-yl-5-nitro-pyrimidin-4-yl)-piperidine-4-carboxylic acid ethyl ester;**

**[method 17].** The reaction mixture was filtered through Celite and activated carbon and purified by preparatory LCMS. $^1H$ NMR 400MHz $CDCl_3$ δ(ppm): 8.75 (s, 1H); 7.96 (m, 1H); 7.90 (m, 2H); 7.51 (m, 3H); 7.40 (m, 1H); 4.19 (m, 2H); 4.11 (m, 2H); 3.31 (m, 2H); 2.68 (m, 1H); 2.08 (m, 2H); 1.91 (m,2H); 1.29 (m, 3H). LCMS (ESI) m/z 407 ($M+H^+$,100%)

**Compound C11**

**1-[6-(4-Methylsulfanyl-phenyl)-5-nitro-pyrimidin-4-yl]-piperidine-4-carboxylic acid ethyl ester;**

**[method 17].** The reaction mixture was filtered through Celite and activated carbon and purified by preparatory LCMS. $^1$H NMR 400MHz $CDCl_3$ δ(ppm): 8.68 (s, 1H); 7.41 (m, 2H); 7.28 (m, 2H); 4.18 (m, 2H); 4.07 (m, 2H); 3.27 (m, 2H); 2.66 (m, 1H); 2.52 (s, 3H); 2.03 (m, 2H); 1.85 (m, 2H); 1.29 (m, 3H). LCMS (ESI) m/z 403 (M+H$^+$,100%)

**Compound C12**

**1-(2',4'-Dihydroxy-5-nitro-[4,5']bipyrimidinyl-6-yl)-piperidine-4-carboxylic acid ethyl ester;**

**[method 17]**. The reaction mixture was filtered through Celite and activated carbon and purified by HPLC w/TFA. Yield 0.015 g, 11 %. Yellow solid. $^1$H NMR 400MHz $CDCl_3$ □(ppm): 8.48, 7.33 (s, 1H); 8.02 (s, 1H); 4.07 (m, 2H); 3.86 (m, 2H); 3.13 (m, 2H); 2.65 (s, 2H); 2.55 (m, 1H.); 1.93 (m, 2H); 1.74 (m, 2H); 1.18 (m, 3H). LCMS (ESI) m/z 391 (M+H$^+$,100%)

**Compound C13**

**1-[6-(4-Methanesulfonyl-phenyl)-5-nitro-pyrimidin-4-yl]-piperidine-4-carboxylic acid ethyl ester;**

[method 17] Yield: 6 mg (4 %). $^1$H NMR, 400MHz, $CDCl_3$, δ (ppm): 8.66(s, 1H); 8.02(d, 1H); 7.66(d, 1H); 4.18 (q, 2H); 4.05(m, 2H); 3.27(t, 2H); 3.09(s, 3H); 2.66(m, 1H); 2.06(m, 2H); 1.88(m, 2H); 1.28(t, 3H). LCMS (ESI) for $C_{19}H_{22}N_4O_6S$: m/z 434 (M+H$^+$, 100 %)

**Compound C14**

**1-[6-(3,5-Bis-trifluoromethyl-phenyl)-5-nitro-pyrimidin-4-yl]-piperidine-4-carboxylic acid ethyl ester**;

**[method 17].** Purification of the crude product by preparatory LCMS afforded the pure product in 23 %. $^1$H NMR, 400MHz, $CDCl_3$, δ (ppm): 8.64(s, 1H); 7.96(s, 3H); 4.18(q, 2H); 4.05(m, 2H); 3.25(t, 2H); 2.65(m, 1H); 2.05(m, 2H); 1.85(m, 4H); 1.28(t, 3H). LCMS (ESI) for $C_{20}H_{18}F_6N_4O_4$: m/z 492 (M+H$^+$, 100 %)

**Compound C15**

**1-(6-Dibenzothiophen-4-yl-5-nitro-pyrimidin-4-yl)-piperidine-4-carboxylic acid ethyl ester;**

**[method 17].** Purification of the crude product by preparatory LCMS afforded the pure product in 24 %. $^1$H NMR, 400MHz, $CDCl_3$, δ (ppm): 8.72(s, 1H); 8.20(d, 1H); 8.12 (m, 1H); 7.76(m, 1H); 7.45(m, 4H); 7.37(m, 1H); 4.14(q, 2H); 4.05(m, 2H); 3.27(t, 2H); 2.64(m, 1H); 2.03(m, 2H); 1.86(m, 2H); 1.25(t, 3H). LCMS (ESI) for $C_{24}H_{22}N_4O_4S$: m/z 462 (M+H$^+$, 100 %)

**Compound C16**

**1-[6-(3,5-Dimethyl-isoxazol-4-yl)-5-nitro-pyrimidin-4-yl]-piperidine-4-carboxylic acid ethyl ester;**

[method 17]. Purification of the crude product by preparatory LCMS afforded the pure product in 12 %. $^1$H NMR, 400MHz, $CDCl_3$, δ (ppm): 8.64(s, 1H); 4.18(q, 2H); 4.05(m, 2H); 3.25(t, 2H); 2.66(m, 1H); 2.35(s, 3H); 2.21(s, 3H); 2.05(m, 2H); 1.86(m, 2H); 1.28(t, 3H). LCMS (ESI) for $C_{17}H_{21}N_5O_5$: m/z 375 (M+H$^+$, 100 %)

**Compound C17**

**1-(5-Nitro-6-thiophen-2-yl-pyrimidin-4-yl)-piperidine-4-carboxylic acid ethyl ester;**

**[method 17].** Purification of the crude product by preparatory LCMS afforded the pure product in 23%. $^1$H NMR, 400MHz, $CDCl_3$, δ (ppm): 8.56(s, 1H); 7.58(d, 1H); 7.47(d, 1H); 7.10(t, 1H); 4.17(q, 2H); 4,09(d, 2H); 3.21(t, 2H); 2.62(m, 1H); 2.03(m, 2H); 1.85(m, 2H); 1.28(t, 3H). LCMS (ESI) for $C_{16}H_{18}N_4O_4S$: m/z 362 (M+H$^+$, 100 %)

**Compound C18**

**1-[6-(3,5-Dichloro-phenyl)-5-nitro-pyrimidin-4-yl]-piperidine-4-carboxylic acid ethyl ester;**

**[method 17].** Purification of the crude product by preparatory LCMS afforded the pure product in 7 %.$^1$H NMR, 400MHz, $CDCl_3$, δ (ppm): 8.59(s, 1H); 7.44(s, 1H); 7.30(s, 2H); 7.23(s, 1H); 4.14(q, 2H); 4.00(m, 2H); 3.21(m, 2H); 2.61 (m, 1H); 2.02(m, 2H); 1.81(m, 2H); 1.25(t, 3H). LCMS (ESI) for $C_{16}H_{18}N_4O_4S$: m/z 362 ($M+H^+$, 100 %)

**Compound C19**

**1-(6-Dibenzofuran-4-yl-5-nitro-pyrimidin-4-yl)-piperidine-4-carboxylic acid ethyl ester;**

**[method 17].** Purification of the crude product by preparatory LCMS afforded the pure product in 17 %. $^1$H NMR, 400MHz, $CDCl_3$, δ (ppm): 8.77(s, 1H); 8.07(d, 1H); 7.96(d, 1H); 7.45(m, 4H); 7.36(t, 1H); 4.18(q, 2H); 4.08(m, 2H); 3.31(t, 2H); 2.69(m, 1H); 2.07(m, 2H); 1.92(m, 2H); 1.29(t, 3H). LCMS (ESI) for $C_{24}H_{22}N_4O_5$: m/z 446 ($M+H^+$, 100 %)

**Compound C20**

**1-[6-(3,5-Dimethyl-phenyl)-5-nitro-pyrimidin-4-yl]-piperidine-4-carboxylic acid ethyl ester;**

**[method 17].** Purification of the crude product by preparatory LCMS afforded the pure product in 23 %. $^1$H NMR, 400MHz, $CDCl_3$, δ(ppm):. 8.46(s, 1H); 6.93(s, 3H); 4.02(q, 2H); 3.89(m, 2H); 3.05(m, 2H); 2.47(m, 1H); 2.20(s, 6H); 1.88(m, 2H); 1.70(m, 4H); 1.18(t, 3H). LCMS (ESI) for $C_{18}H_{18}ClN_4O_4$: m/z 384 ($M+H^+$, 100 %)

**Compound C21**

**1-[6-(4-Acetyl-phenyl)-5-nitro-pyrimidin-4-yl]-piperidine-4-carboxylic acid ethyl ester;**

Filtered the reaction mixture and the filtrate was purified by semi preparatory HPLC afforded the pure product in 21 %. $^1$H NMR, 400MHz, $CDCl_3$, δ (ppm): 8.63(s, 1H); 8.00(d, 2H); 7.57(d, 2H); 4.16(q, 2H); 4.03(m, 2H); 3.22(m, 2H); 2.64(s, 3H); 2.03(m, 2H); 1.85(m, 4H); 1.27(t, 3H). LCMS (BSI) for $C_{20}H_{22}N_4O_5$: m/z 398 ($M+H^+$, 100 %)

**Compound C22**

**1-[6-(4-Ethanesulfonyl-phenyl)-5-nitro-pyrimidin-4-yl]-piperidine-4-carboxylic acid ethyl ester;**

**[method 17].** Purification of the crude product by preparatory TLC using 20 % EtOAc/hexane afforded the pure product in 21 %. $^1$H NMR, 400MHz, $CDCl_3$, δ (ppm): 8.63(s, 1H); 7.97(d, 2H); 7.67(d, 2H); 4.18(q, 2H); 3.22(m, 2H); 3.12(m, 3H); 2.65(m, 1H); 2.04(m, 2H); 1.85(m, 2H); 1.28(t, 3H). LCMS (ESI) for $C_{20}H_{24}N_4O_6S$: m/z 448 ($M+H^+$, 100 %)

**Compound C23**

**1-[6-(2-Fluoro-biphenyl-4-yl)-5-nitro-pyrimidin-4-yl]-piperidine-4-carboxylic acid ethyl ester;**

**[method 17].** purified by semi preparatory HPLC afforded the pure product in 14 %. $^1$H NMR, 400MHz, $CDCl_3$, δ (ppm): 8.54(s, 1H); 7.35(m, 8H); 4.07(q, 2H); 3.96(m, 2H); 3.13(t, 2H); 2.53(m, 1H); 1.95(m, 2H); 1.77(m, 2H); 1.19(t, 3H). LCMS (ESI) for $C_{20}H_{22}N_4O_5$: m/z 398 ($M+H^+$, 100 %)

**Compound C24**

**1-[6-(3-Methanesulfonyl-phenyl)-5-nitro-pyrimidin-4-yl]-piperidine-4-carboxylic acid ethyl ester;**

**[method 17].** Purification of the crude product by flash column chromatography (Biotage,silica, 20 % EtOAc/hexane) afforded the pure product in 5 %.$^1$H NMR, 400MHz, $CDCl_3$, δ (ppm): 8.67(s, 1H); 8.07(m, 2H); 7.69(m, 2H); 4.18(q, 2H); 4.09(m, 2H); 3.29(t, 2H); 3.08(s, 3H); 2.71(m, 1H); 2.06(m, 2H); 1.87(m, 2H); 1.27(t, 3H). LCMS (ESI) for $C_{19}H_{22}N_4O_6S$: m/z 434($M+H^+$, 100 %)

**Compound C25**

**1-{6-[4-(2-Carboxy-ethyl)-phenyl]-5-nitro-pyrimidin-4-yl}-piperidine-4-carboxylic acid ethyl ester;**

**[method 17]. C25** was purified by preparatory HPLC afforded the pure product in 24 %. $^1$H NMR, 400MHz, $CDCl_3$, δ (ppm): 8.52(s, 1H); 7.33(d, 2H); 7.17(d, 2H); 4.07(m, 2H); 3.94(m, 2H); 3.10(t, 2H); 2.94(t, 2H); 2.64(t, 2H); 2.52(m, 1H); 1.40(t, 3H). LCMS (ESI) for $C_{21}H_{24}N_4O_6$: m/z 428(M+H$^+$, 100 %)

**Compound C26**

**1-{6-[4-(2-Methoxycarbonyl-ethyl)-phenyl]-5-nitro-pyrimidin-4-yl}-piperidine-4-carboxylic acid methyl ester**

**[method 17].** Purification of the residue by column chromatography (silica, 20 % EtOAc/hexane) afforded the pure product in 14 %. $^1$H NMR, 400MHz, $CDCl_3$, δ (ppm): 8.55(s, 1H); 7.36(d, 2H); 7.19(d, 2H); 3.95(m, 2H); 3.64(s, 3H); 3.60(s, 3H); 3.11(m, 2H); 2.92(t, 2H); 2.57(t, 2H); 2.54(m, 1H); 1.95(m, 2H); 1.76(m, 2H). LCMS (ESI) for $C_{21}H_{24}N_4O_6$: m/z 428 (M+H$^+$, 100 %).

**Compound C27**

**1-{6-[4-(2-Methoxycarbonyl-ethyl)-phenyl]-5-nitro-pyrimidin-4-yl}-piperidine-4-carboxylic acid ethyl ester.**

**[method 17].** Purification of the residue by preparatory TLC (silica, 20 % EtOAc/hexane) afforded the pure product in 16 %. $^1$H NMR, 400MHz, $CDCl_3$, δ (ppm): 8.55(s, 1H); 7.35(d, 2H); 7.19(d, 2H); 4.09(q, 2H); 3.96(m, 2H); 3.60(s, 3H); 3.11(m, 2H); 2.92(t, 2H); 2.57(t, 2H); 1.94(s, 3H); 1.77(m, 2H); 1.19(t, 3H). LCMS (ESI) for $C_{22}H_{26}N_4O_6$: m/z 442 (M+H$^+$, 100 %).

**EXAMPLE 15**

**SYNTHESES OF COMPOUNDS OF THE PRESENT INVENTION**

**Compound D1**

**1-[5-Nitro-6-(2-tnfluoromethyl-phenytethynyl)-pyrimidin-4-yl]-piperidine-4-carboxylic acid ethyl ester. General Method 18.**

1-{6-chloro-5-nitro-pyrimidin-4-yl}-piperidine-4-carboxylic acid ethyl ester (100 mg, 0.317 mmol), $Pd(PPh_3)_2Cl_2$ (12 mg, 0.017 mmol) and copper(I)iodide (5 mg, 0.026 mmol) were dissolved in diisopropylethylamine and stirred for 2 min. o-ethynyl-trifluoromethylbenzene (100 ul, 0.072 mmol) was added and the mixture stirred under $N_2$ at 70 ˚C for 18 hours. The yellow suspension turned black. After cooling to room temperature, the reaction mixture was diluted with chloroform (12 ml), passed through a celite plug and concentrated under *vacuo.* Purification by Flash Chromatography (0-30% Ethyl acetate / Hexanes) gave Compound **D1.** Yield 35 %.$^1$H NMR 400MHz $CDCl_3$ δ (ppm): 8.58 (s, 1H); 7.78 (d, 1H); 7.71 (d, 1H); 7,56 (m, 2H); 4.16 (m, 2H); 4.01 (d, 2H); 3.21 (m, 2H); 2.63 (m, 1H); 2.03 (m, 2H); 1.84 (m, 2H); 1.26 (t, 3H). LCMS (ESI) m/z 449 (M$^+$H$^+$, 100 %)

**Compound D2**

**1-(5-Nitro-6-phenylethynyl-pyrimidin-4-yl)-piperidine-4-carboxylic acid ethyl ester**

**[method 18].** Purification by HPLC yielded red oil. Yield 9 %. $^1$H NMR 400MHz $CDCl_3$ δ (ppm): 8.49 (s, 1H); 7.54 (d, 2H); 7.32 (d, 3H); 4.11 (m, 2H); 3.95 (d, 2H); 3.20 (m, 2H); 2.58 (m, 1H); 2.01 (m, 2H); 1.80 (m, 2H); 1.21 (t, 3H). LCMS (ESI) m/z 381 (M+H$^+$, 100 %)

**Compound D3**

**1-[5-Nitro-6-(4-triffuoromethyl-phenylethynyl)-pyrimidin-4-yl]-piperidine-4-carboxylic acid ethyl ester;**

**[method 18].** Purification by HPLC. Yield 30 % brown solid. $^1$H NMR 400MHz $CDCl_3$ δ (ppm): 8.55 (s, 1H); 7.7 (d, 2H); 7.65 (d, 2H); 4.17 (m, 2H); 4.00 (d, 2H); 3.24 (m, 2H); 2.64 (m, 1H); 1.96 (m, 4H); 1.28 (t, 3H). LCMS (ESI)

m/z 449 ($M+H^+$, 100%)

**Compound D4**

**1-(5-Nitro-6-m-tolylethynyl-pyrimidin-4-yl)-piperidine-4-carboxylic acid ethyl ester;**

Purification by HPLC. Yield 60 % brown solid. $^1H$ NMR 400MHz $CDCl_3$ $\delta$ (ppm): 8.55 (s, 1H); 7.44 (d, 2H); 7.27 (d, 2H); 4.18 (m, 2H); 4.00 (d, 2H); 3.24 (m, 2H); 2.64 (m, 1H); 2.37 (s, 3H); 2.04 (m, 2H); 1.87 (m, 2H); 1.28 (t, 3H). LCMS (EST) m/z 395 ($M+H^+$, 100 %)

**Compound D5**

**1-[6-(2-Fluoro-phenylethynyl)-5-nitro-pyrimidin-4-yl]-piperidine-4-carboxylic acid ethyl ester;**

$^1H$ NMR 400MHz $CDCl_3$ $\delta$ (ppm): 8.58 (s, 1H); 7.78 (d, 1H); 7.71 (d, 1H); 7,56 (m, 2H); 4.16 (m, 2H); 4.01 (d, 2H); 3.21 (m, 2H); 2.63 (m, 1H); 2.03 (m, 2H); 1.84 (m, 2H); 1.26 (t, 3H). LCMS (ESI) m/z 449 ($M+H^+$, 100 %)

**Compound D6**

**1-[5-Nitro-6-(3-triauoromethyl-phenylethynyl)-pyrimidm-4-yl]-pipendine-4-carboxylic acid ethyl ester;**

**[method 18]**. Purification by Flash Chromatography (20-30 % Ethyl acetate/ Hexanes). Yield 42 %. $^1H$ NMR 400MHz $CDCl_3$ $\delta$ (ppm): 8.55 (s, 1H); 7.87 (s, 1M; 7.78 (d, 1H); 7.68 (d, 1H); 7.55 (m, 1H); 4.19 (m, 2H); 4.00 (d, 2H); 3.23 (m, 2H); 2.65 (m, 1H); 2.04 (m, 2H); 1.86 (m, 2H); 1.27 (t, 3H). LCMS (ESI) m/z 449 ($M^+H^+$, 100%)

**EXAMPLE 16**

**SYNTHESES OF COMPOUNDS OF THE PRESENT INVENTION**

**5-Nitro-4-(5-phenyl-[1,3,4]oxadiazol-2-ylsulfanyl)-6-[4-(pyridin-2-ylsulfanyl)-piperidin-1-yl]-pyrimidine**

**Compound E1**

**[Method 16].** yellow solid (56 mg, 80 %). $^1$HNMR ($CDCl_3$, 400 MHz) $\delta$ 1.61 (m, 2H), 2.02 (m, 2H), 3.22 (m, 2H), 3.69 (m, 2H), 3.98 (m, 2H), 6.87 (m, 1H), 6.94 (m, 1H), 7.30 (m, 4H), 7.72 (m, 2H), 7.89 (m, 1H), 8.20 (m, 1H). Exact mass calculated for $C_{22}H_{19}N_7O_3S_2$ 493.56, found 494.5 ($MH^+$).

The compounds in the above examples were screened in the Membrane Cyclase Assay. Representative compounds are shown in the table below:

| Compound | RUP3 ($IC_{50}$) Membrane Cyclase ($\mu$M) |
|---|---|
| A124 | 0.241 |
| B70 | 0.129 |
| B84 | 0.050 |

The other compounds in the Examples showed $IC_{50}$ activities in the membrane cyclase assay less than about 500 $\mu$M.

Those skilled in the art will recognize that various modifications, additions, substitutions, and variations to the illustrative examples set forth herein can be made without departing from the spirit of the invention and are, therefore, considered within the scope of the invention. All documents referenced above, including, but not limited to, printed publications, and provisional and regular patent applications, are incorporated herein by reference in their entirety.

## Claims

1. A compound of Formula **(Ia):**

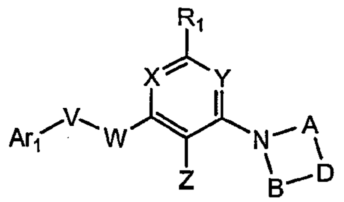

**(Ia)**

or a pharmaceutically acceptable salt, hydrate or solvate thereof,
wherein:

A and B are independently $C_{1-3}$ alkylene optionally substituted with 1 to 4 methyl groups;
D is O, S, S(O), $S(O)_2$, $CR_2R_3$ or N-$R_2$;
V is selected from the group consisting of $C_{1-3}$ alkylene, ethynylene and $C_{1-2}$ heteroalkylene wherein each are optionally substituted with 1 to 4 substituents selected from the group consisting of $C_{1-3}$ alkyl, $C_{1-4}$ alkoxy, carboxy, cyano, $C_{1-3}$ haloalkyl and halogen; or
V is absent;
W is $NR_4$, O, S, S(O) or $S(O)_2$; or
W is absent;
X is N or $CR_5$;
Y is N or $CR_6$;
Z is selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, $C_{1-2}$ alkylamino, $C_{2-4}$ dialkylamino, carbo-$C_{1-6}$-alkoxy, carboxamide, carboxy, cyano, $C_{4-8}$ diacylamino, $C_{2-6}$ dialkylcarboxamide, $C_{1-4}$ dialkylthiocarboxamide, $C_{2-6}$ dialkylsulfonamide, $C_{1-4}$ dialkylsulfonylamino, formyl, $C_{1-4}$ haloalkoxy, $C_{1-4}$ haloalkyl, $C_{1-4}$ haloalkylcarboxamide, $C_{1-4}$ haloalkylsulfinyl, $C_{1-4}$ haloalkylsulfonyl, $C_{1-4}$ haloalkylthio, halogen, aryl, heterocyclic, heteroaryl, hydroxyl, hydroxylamino, nitro and tetrazolyl, wherein $C_{1-8}$ alkyl and $C_{1-5}$ acyl are each optionally substituted with 1, 2, 3 or 4 groups selected from the group consisting of $C_{1-5}$ acyl, $C_{1-5}$ acyloxy, $C_{1-4}$ alkoxy, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, $C_{1-2}$ alkylamino, $C_{2-4}$ dialkylamino,

carbo-$C_{1-6}$-alkoxy, carboxamide, carboxy, cyano, formyl, $C_{1-4}$ haloalkoxy, $C_{1-4}$ haloalkylsulfinyl, $C_{1-4}$ haloalkylsulfonyl, $C_{1-4}$ haloalkylthio, halogen, hydroxyl, hydroxylamino and nitro; or
Z is a group of Formula (A):

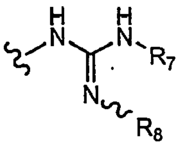

(A)

wherein:

$R_7$ is H, $C_{1-8}$ alkyl or $C_{3-6}$ cycloalkyl; and
$R_8$ is H, nitro or nitrile;

$Ar_1$ is aryl or heteroaryl wherein each are optionally substituted with $R_9$-$R_{13}$;
$R_1$ is selected from the group consisting of H, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, $C_{1-4}$ alkylamino, $C_{2-8}$ dialkylamino, carboxamide, cyano, $C_{3-6}$ cycloalkyl, $C_{2-6}$ dialkylcarboxamide, $C_{2-6}$ dialkylsulfonamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio and hydroxyl;
$R_2$ is selected from the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, heteroaryl, hydroxyl and phenyl; and wherein $C_{1-8}$ alkyl, heteroaryl and phenyl are each optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl, $C_{3-6}$-cycloalkyl-$C_{1-3}$-alkylene, $C_{3-6}$-cycloalkyl-$C_{1-3}$-heteroalkylene, $C_{2-8}$ dialkylamino, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heterocyclic, hydroxyl, hydroxylamino and nitro; or
$R_2$ is -$Ar_2$-$Ar_3$ wherein $Ar_2$ and $Ar_3$ are independently aryl or heteroaryl each optionally substituted with 1 to 5 substituents selected from the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, hydroxyl and nitro; or
$R_2$ is a group of Formula (B):

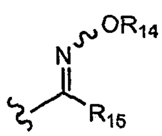

(B)

wherein:

$R_{14}$ is $C_{1-8}$ alkyl or $C_{3-6}$ cycloalkyl; and $R_{15}$ is F, Cl, Br or CN; or

$R_2$ is a group of Formula (C):

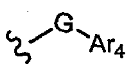

**(C)**

wherein:

G is C=O, $CR_{16}R_{17}$, O, S, S(O), $S(O)_2$; where $R_{16}$ and $R_{17}$ are independently H or $C_{1-8}$ alkyl; and $Ar_4$ is phenyl or heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkyl, $C_{1-4}$ haloalkylthio, halogen, heteroaryl, hydroxyl, hydroxylamino and nitro;

$R_3$ is H, $C_{1-8}$ alkyl, $C_{1-4}$ alkoxy, halogen or hydroxyl;
$R_4$ is H or $C_{1-8}$ alkyl;
$R_5$ and $R_6$ are independently H, $C_{1-8}$ alkyl or halogen;
$R_9$ is selected from the group consisting of $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylamino, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, arylsulfonyl, carbo-$C_{1-6}$-alkoxy, carboxamide, carboxy, cyano, $C_{3-6}$ cycloalkyl, $C_{2-6}$ dialkylamino, $C_{2-6}$ dialkylcarboxamide, $C_{2-6}$ dialkylsulfonamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, heterocyclic, heterocyclicsulfonyl, heteroaryl, hydroxyl, nitro, $C_{4-7}$ oxo-cycloalkyl, phenoxy, phenyl, sulfonamide and sulfonic acid, and wherein $C_{1-5}$ acyl, $C_{1-4}$ alkoxy, $C_{1-8}$ alkyl, $C_{1-4}$ alkylsulfonamide, alkylsulfonyl, arylsulfonyl, heteroaryl, phenoxy and phenyl are each optionally substituted with 1 to 5 substituents selected independently from the group consisting of $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, heteroaryl, heterocyclic, hydroxyl, nitro and phenyl; or
$R_9$ is a group of Formula (**D**):

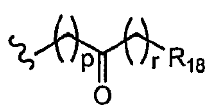

**(D)**

wherein:

"p" and "r" are independently 0, 1, 2 or 3; and
$R_{18}$ is H, $C_{1-5}$ acyl, $C_{2-6}$ alkenyl, $C_{1-8}$ alkyl, $C_{1-4}$ alkylcarboxamide, $C_{2-6}$ alkynyl, $C_{1-4}$ alkylsulfonamide, carbo-$C_{1-6}$-alkoxy, carboxamide, carboxy, cyano, $C_{3-6}$ cycloalkyl, $C_{2-6}$ dialkylcarboxamide, halogen, heteroaryl or phenyl, and wherein the heteroaryl and phenyl are each optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl; and

$R_{10}$-$R_{13}$ are independently selected form the group consisting of $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, hydroxyl and nitro; or two adjacent $R_{10}$-$R_{11}$ groups together with $Ar_1$ form a 5, 6 or 7 membered cycloalkyl, cycloalkenyl or heterocyclic group wherein the 5, 6 or 7 membered group is optionally substituted with halogen.

2. The compound according to claim 1 wherein W is $NR_4$.

3. The compound according to claim 2 wherein $R_4$ is H.

4. The compound according to claim 2 wherein $R_4$ is $CH_3$ or $CH_2CH_3$.

5. The compound according to claim 1 wherein W is O.

6. The compound according to claim 1 wherein W is S.

7. The compound according to claim 1 wherein W is absent.

8. The compound according to claim 1 wherein W is absent and V is ethynylene.

9. The compound according to any one of claims 1 to 7 wherein V is $-CH_2-$ or - $CH_2CH_2-$,

10. The compound according to any one of claims 1 to 7 wherein V is $-OCH_2CH_2-$.

11. The compound according to any one of claims 1 to 7 wherein V is absent.

12. The compound according to any one of claims 1 to 11 wherein A is ethylene and B is methylene.

13. The compound according to any one of claims 1 to 11 wherein A is propylene and B is methylene.

14. The compound according to any one of claims 1 to 11 wherein A and B are both ethylene wherein A and B are optionally substituted with 1 to 4 methyl groups.

15. The compound according to any one of claims 1 to 14 wherein D is O, S, S(O) or $S(O)_2$.

16. The compound according to any one of claims 1 to 14 wherein D is $CR_2R_3$.

17. The compound according to claim 16 wherein $R_2$ is selected from the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxyl, $C_{3-6}$ cycloalkyl, $C_{1-4}$ haloalkoxy, $C_{1-4}$ haloalkyl, halogen and hydroxyl.

18. The compound according to claim 17 wherein $R_2$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $OC(O)CH_3$, $OC(O)CH_2CH_3$, $OC(O)CH_2CH_2CH_3$, $OCH_3$, $OCH_2CH_3$, $OCH_2CH_2CH_3$, $OCH(CH_3)_2$, $OCH_2(CH_2)_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, $CH_2(CH_2)_3CH_3$, $C(O)NH_2$, $CO_2CH_3$, $CO_2CH_2CH_3$, $CO_2CH_2CH_2CH_3$, $CO_2CH(CH_3)_2$, $CO_2CH_2(CH_2)_2CH_3$, and $CO_2H$.

19. The compound according to claim 17 wherein $R_2$ is selected from the group consisting of $S(O)_2CH_3$, $S(O)_2CH_2CH_3$, $S(O)_2CH_2CH_2CH_3$, $S(O)_2CH(CH_3)_2$, $S(O)_2CH_2(CH_2)_2CH_3$, cyclopropyl, cyclobutyl, cyclopentyl, cyclohexyl, hydroxyl, and F.

20. The compound according to claim 16 wherein $R_2$ is $C_{1-8}$ alkyl, or heteroaryl each optionally substituted with 1 to 5 substituents selected from the group consisting of $C_{1-5}$ acyloxy, $C_{1-4}$ alkoxy, $C_{1-8}$ alkyl, $C_{1-4}$ alkylsulfonyl, carbo-$C_{1-6}$-alkoxy, carboxamide, carboxy, $C_{3-6}$-cycloalkyl, $C_{3-6}$-cycloalkyl-$C_{1-3}$-alkylene, $C_{3-6}$-cycloalkyl-$C_{1-3}$-heteroalkylene, and hydroxyl.

21. The compound according to claim 20 wherein $R_2$ is selected from the group consisting of $CH_2OCH_3$, $CH_2CH_2OCH_3$, $CH_2OCH_2CH_3$, $CH_2OCH_2CH_2CH_3$, $CH_2CH_2OCH_2CH_3$, $CH_2CH_2OCH_2CH_2CH_3$, $CH_2OCH(CH_3)_2$, $CH_2OCH_2CH(CH_3)_2$, $CH_2CO_2H$, $CH_2CH_2CO_2H$, $CH_2OH$, $CH_2CH_2OH$ and $CH_2CH_2CH_2OH$.

22. The compound according to claim 20 wherein $R_2$ is selected from the group consisting of $CH_2S(O)_2CH_3$, $CH_2S(O)_2CH_2CH_3$, $CH_2S(O)_2CH_2CH_2CH_3$, $CH_2S(O)_2CH(CH_3)_2$, $CH_2S(O)_2CH_2(CH_2)_2CH_3$, $CH_2CH_2S(O)_2CH_3$, $CH_2CH_2S(O)_2CH_2CH_3$, $CH_2CH_2S(O)_2CH_2CH_2CH_3$, $CH_2CH_2S(O)_2CH(CH_3)_2$, $CH_2CH_2S(O)_2CH_2(CH_2)_2CH_3$,

$CH_2OCH_2$-cyclopropyl, $CH_2OCH_2$-cylobutyl, $CH_2OCH_2$-cyclopentyl, and $CH_2OCH_2$-cyclohexyl.

**23.** The compound according to claim 20 wherein $R_2$ is selected from the group consisting of 1,2,4-oxadiazol-3-yl, 1,2,4-oxadiazol-5-yl, 1,3,4-oxadiazol-2-yl, 3-methyl-1,2,4-oxadiazol-5-yl, 3-ethyl-1,2,4-oxadiazol-5-yl, 3-isopropyl-1,2,4-oxadiazol-5-yl, 3-propyl-1,2,4-oxadiazol-5-yl, 3-t-butyl-1,2,4-oxadiazol-5-yl, and 3-cyclopropyl-1,2,4-oxadiazol-5-yl.

**24.** The compound according to claim 16 wherein $R_2$ is $-Ar_2-Ar_3$ wherein $Ar_2$ and $Ar_3$ are independently aryl or heteroaryl each optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, hydroxyl and nitro.

**25.** The compound according to claim 24 wherein $Ar_2$ is a heteroaryl and $Ar_3$ is phenyl.

**26.** The compound according to claim 16 wherein $R_2$ is Formula **(C):**

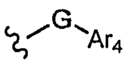

(C)

wherein:

G is C=O, $CR_{16}R_{17}$, O, S, S(O), $S(O)_2$; wherein $R_{16}$ and $R_{17}$ are independently H or $C_{1-2}$ alkyl; and

$Ar_4$ is phenyl or heteroaryl optionally substituted with 1 to 5 substituents selected from the group consisting of $C_{1-4}$alkoxy, $C_{1-8}$ alkyl, $C_{1-4}$ haloalkoxy, $C_{1-4}$ haloalkyl, and halogen.

**27.** The compound according to claim 26 wherein G is C=O, $CH_2$ or O.

**28.** The compound according to claim 26 wherein G is S, S(O) or $S(O)_2$.

**29.** The compound according to any one of claims 26 to 28 wherein $Ar_4$ is selected from the group consisting of pyridinyl, pyridazinyl, pyrimidinyl and pyrazinyl.

**30.** The compound according to any one of claims 26 to 29 wherein $Ar_4$ is 2-pyridyl.

**31.** The compound according to any one of claims 26 to 30 wherein $R_{16}$ and $R_{17}$ are both H.

**32.** The compound according to any one of claims 16 to 31 wherein $R_3$ is H.

**33.** The compound according to any one of claims 1 to 14 wherein D is $N-R_2$.

**34.** The compound according to claim 33 wherein $R_2$ is H, or carbo-$C_{1-6}$-alkoxy.

**35.** The compound according to claim 34 wherein $R_2$ is selected from the group consisting of $CO_2CH_3$, $CO_2CH_2CH_3$, $CO_2CH_2CH_2CH_3$, $CO_2CH(CH_3)_2$ and $CO_2CH_2(CH_2)_2CH_3$.

**36.** The compound according to claim 33 wherein $R_2$ is $C_{1-8}$ alkyl optionally substituted with 1 to 5 substituents selected from the group consisting of $C_{1-4}$ alkylsulfonyl, carbo-$C_{1-6}$-alkoxy, and carboxy.

**37.** The compound according to claim 36 wherein $R_2$ is $CH_2CO_2Et$, or $CH_2CH_2CO_2H$.

**38.** The compound according to claim 36 wherein $R_2$ is selected from the group consisting of $CH_2CH_2S(O)_2CH_3$, $CH_2CH_2S(O)_2CH_2CH_3$, $CH_2CH_2S(O)_2CH_2CH_2CH_3$, $CH_2CH_2S(O)_2CH(CH_3)_2$ and $CH_2CH_2S(O)_2CH_2(CH_2)_2CH_3$.

**39.** The compound according to any one of claims 1 to 38 wherein Z is selected from the group consisting of $C_{1-5}$ acyl, $C_{1-8}$ alkyl, $C_{1-4}$ alkylcarboxamide, amino, cyano, $C_{4-8}$ diacylamino, $C_{2-6}$ dialkylsulfonamide, formyl, halogen, heterocyclic, and nitro wherein $C_{1-8}$ alkyl and $C_{1-5}$ acyl are each optionally substituted with 1, or 2 groups selected from the group consisting of $C_{2-4}$ dialkylmino, hydroxy, and halogen.

**40.** The compound according to claim 39 wherein Z is selected from the group consisting of nitro, amino, formyl, $NHC(O)CF_3$, Br, $NHC(O)CH_3$, $N(C(O)CH_3)_2$, $N(S(O)_2CH_3)_2$, $CH_3$, [1,3]dioxolan-2-yl, $CH_2OH$, $CH_2N(CH_3)_2$, and $C(O)CH_3$.

**41.** The compound according to any one of claims 1 to 40 wherein $R_1$ is selected from the group consisting of H, $C_{1-8}$ alkyl, and amino.

**42.** The compound according to any one of claims 1 to 41 wherein $Ar_1$ is phenyl optionally substituted with $R_9$-$R_{13}$.

**43.** The compound according to claim 42 wherein $R_9$ is selected from the group consisting of $C_{1-5}$ acyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{2-6}$ dialkylsulfonamide, $C_{1-4}$ alkylsulfinyl, $C_{1-4}$ alkylsulfonyl, $C_{1-4}$ alkylthio, amino, arylsulfonyl, $C_{2-6}$ dialkylamino, $C_{2-6}$ dialkylsulfonamide, and carboxamide.

**44.** The compound according to claim 43 wherein $R_9$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $OCH_3$, $OCH_2CH_3$, $OCH_2CH_2CH_3$, $OCH(CH_3)_2$, $OCH_2CH_2CH_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, $CH_2(CH_2)_3CH_3$, $CH_2(CH_2)_4CH_3$, $CH_2(CH_2)_5CH_3$, $C(O)NHCH_3$, $C(O)NHCH_2CH_3$, $C(O)NHCH_2CH_2CH_3$, $C(O)NHCH(CH_3)_2$, C=CH, $S(O)_2NHCH_3$, $S(O)_2NHCH_2CH_3$, $S(O)_2NHCH_2CH_2CH_3$, $S(O)_2NHCH(CH_3)_2$, $S(O)_2NHCH_2(CH_2)_2CH_3$, $S(O)_2NHCH(CH_3)CH_2CH_3$, $S(O)_2N(CH_3)_2$, $S(O)_2N(Et)(CH_3)$, $S(O)_2CH_3$, $S(O)_2CH_2CH_3$, $S(O)_2CH_2CH_2CH_3$, $S(O)_2CH(CH_3)_2$, $S(O)_2CH_2(CH_2)_2CH_3$, $S(O)_2CH(CH_3)CH_2CH_3$, $SCH_3$, $SCH_2CH_3$, $SCH_2CH_2CH_3$, $SCH(CH_3)_2$, $SCH_2(CH_2)_2CH_3$, amino, $S(O)_2Ph$, $N(CH_3)_2$, $N(CH_3)(Et)$, $N(Et)_2$ and $C(O)NH_2$.

**45.** The compound according to claim 42 wherein $R_9$ is selected from the group consisting of cyano, $C_{3-6}$ cycloalkyl, halogen, $C_{1-4}$ haloalkoxy, $C_{1-4}$ haloalkyl, $C_{1-4}$ haloalkylsulfonyl, and $C_{1-4}$ haloalkylthio.

**46.** The compound according to claim 45 wherein $R_9$ is selected from the group consisting of cyclopropyl, cyclobutyl, cyclopentyl, cyclohexyl, Cl, F, Br, $OCF_3$, $OCHF_2$, $OCH_2CF_3$, $CF_3$, $CHF_2$, $CH_2CF_3$, $SCF_3$, $SCHF_2$ and $SCH_2CF_3$.

**47.** The compound according to claim 42 wherein $R_9$ is selected from the group consisting of heterocyclic, heterocyclicsulfonyl, heteroaryl, hydroxy, $C_{4-7}$ oxo-cycloalkyl, phenoxy and phenyl.

**48.** The compound according to claim 47 wherein $R_9$ is selected from the group consisting of morpholin-4-yl, thiomorpholin-4-yl, 1-oxo-1$\lambda^4$-thiomorpholin-4-yl, 1,1-Dioxo-1$\lambda^6$-thiomorpholin-4-yl, piperazin-1-yl, 4-methyl-piperazin-1-yl, 4-ethyl-piperazin-1-yl, 4-propyl-piperazin-1-yl, piperidin-1-yl, pyrrolidin-1-yl, 2,5-dioxo-imidazolidin-4-yl, 2,4-dioxo-thiazolidin-5-yl, 4-oxo-2-thioxo-thiazolidin-5-yl, 3-methyl-2,5-dioxo-imidazolidin-4-yl, 3-methyl-2,4-dioxo-thiazolidin-5-yl, 3-methyl-4-oxo-2-thioxo-thiazolidin-5-yl, 3-ethyl-2,5-dioxo-imidazolidin-4-yl, 3-ethyl-2,4-dioxo-thiazolidin-5-yl, and 3-ethyl-4-oxo-2-thioxo-thiazolidin-5-yl.

**49.** The compound according to claim 47 wherein $R_9$ is selected from the group consisting of 1H-imidazol-4-yl, [1,2,4]triazol-1-yl, [1,2,3]triazol-1-yl, [1,2,4]triazol-4-yl, pyrrol-1-yl, pyrazol-1-yl, 1H-pyrazol-3-yl, imidazol-1-yl, oxazol-5-yl, oxazol-2-yl, [1,3,4]oxadiazol-2-yl, [1,3,4]thiadiazol-2-yl, [1,2,4]oxadiazol-3-yl, [1,2,4]thiadiazol-3-yl, tetrazol-1-yl, pyrimidin-5-yl, pyrimidin-2-yl, pyrimidin-4-yl, pyridazin-3-yl, pyridazin-4-yl, pyrazin-2-yl, 1,3-dioxo-1,3-dihydro-isoindol-2-yl and [1,2,3]thiadiazol-4-yl.

**50.** The compound according to claim 42 wherein $R_9$ is $C_{1-8}$ alkyl or $C_{1-4}$ alkoxy optionally substituted with 1 to 5 substituents selected independently from the group consisting of $C_{1-5}$ acyl, $C_{1-4}$ alkoxy, $C_{1-4}$ alkylcarboxamide, $C_{1-4}$ alkylsulfonyl, carbo-$C_{1-6}$-alkoxy, carboxamide, carboxy, cyano, and hydroxyl.

**51.** The compound according to claim 50 wherein $R_9$ is selected from the group consisting of $CH_2OCH_3$, $CH_2OCH_2CH_3$, $CH_2OCH_2CH_2CH_3$, $CH_2OCH(CH_3)_2$, $CH_2OCH_2(CH_2)_2CH_3$, $CH_2CH_2OCH_3$, $CH_2CH_2OCH_2CH_3$, $CH_2CH_2OCH_2CH_2CH_3$, $CH_2CH_2OCH(CH_3)_2$ and $CH_2CH_2OCH_2(CH_2)_2CH_3$.

**52.** The compound according to claim 42 wherein $R_9$ is of Formula (**D**):

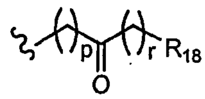

(D)

wherein:

"p" and "r" are independently 0, or 1; and
$R_{18}$ is H, carbo-$C_{1-6}$-alkoxy, heteroaryl or phenyl, and

wherein the heteroaryl and phenyl are each optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl.

**53.** The compound according to claim 52 wherein p = 0 and r = 0.

**54.** The compound according to claim 53 wherein $R_{18}$ is phenyl optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl.

**55.** The compound according to claim 52 wherein p = 0 and r =1.

**56.** The compound according to claim 55 wherein $R_{18}$ is carbo-$C_{1-6}$-alkoxy or carboxy.

**57.** The compound according to any one of claims 43 to 56 wherein $R_9$ is substituted at the para position on the phenyl.

**58.** The compound according to any one of claims 42 to 57 wherein $R_{10}$-$R_{13}$ are independently selected from the group consisting of $C_{1-5}$ acyl, $C_{1-4}$ alkoxy, $C_{1-8}$ alkyl, $C_{1-4}$ alkylcarboxamide, $C_{1-4}$ alkylureyl, carbo-$C_{1-6}$-alkoxy, carboxamide, carboxy, cyano, $C_{3-6}$ cycloalkyl, halogen, $C_{1-4}$ haloalkoxy and $C_{1-4}$ haloalkyl.

**59.** The compound according to any one of claims 42 to 57 wherein one or two $R_{10}$-$R_{13}$ groups are independently halogen.

**60.** The compound according to any one of claims 42 to 59 wherein two adjacent $R_{10}$-$R_{11}$ groups together with the phenyl form a 5, 6 or 7 membered cycloalkyl, cycloalkenyl or heterocyclic group wherein the 5, 6 or 7 membered group is optionally substituted with halogen.

**61.** The compound according to claim 60 wherein the heterocyclic group together with the phenyl group is a 2,3-dihydro-benzofuran-5-yl, benzo[1,3]dioxol-5-yl group, 2,3-dihydro-benzo[1,4]dioxin-6-yl, 2,3-dihydro-benzo[1,4]dioxin-2-yl group, 3,4-dihydro-2H-benzo[b][1,4]dioxepin-7-yl group.

**62.** The compound according to any one of claims 1 to 41 wherein $Ar_1$ is heteroaryl optionally substituted with $R_9$-$R_{13}$.

**63.** The compound according to claim 62 wherein $R_9$ is selected from the group consisting of $C_{1-4}$ alkoxy, $C_{1-8}$ alkyl, $C_{1-4}$ alkylcarboxamide, $C_{1-4}$ alkylsulfonyl, $C_{1-4}$ haloalkyl, hydroxy, halogen, and phenyl.

**64.** The compound according to claim 63 wherein $R_9$ is selected from the group consisting $OCH_3$, $OCH_2CH_3$, $OCH_2CH_2CH_3$, $OCH(CH_3)_2$, $OCH_2CH_2CH_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, $CH_2(CH_2)_3CH_3$, $CH_2(CH_2)_4CH_3$, $CH_2(CH_2)_5CH_3$, $C(O)NHCH_3$, $C(O)NHCH_2CH_3$, $C(O)NHCH_2CH_2CH_3$, $C(O)NHCH(CH_3)_2$, $C(O)NHCH_2(CH_2)_2CH_3$, $S(O)_2CH_3$, $S(O)_2CH_2CH_3$, $S(O)_2CH_2CH_2CH_3$, $S(O)_2CH(CH_3)_2$, Cl, F, Br, $CF_3$, $CHF_2$, $CH_2CF_3$, and hydroxy.

**65.** The compound according to any one of claims 62 to 64 wherein $R_{10}$-$R_{13}$ are independently $C_{1-5}$ acyl, $C_{1-4}$ alkoxy, $C_{1-8}$ alkyl, $C_{1-4}$ alkylcarboxamide, $C_{1-4}$ alkylureyl, carbo-$C_{1-6}$-alkoxy, carboxamide, carboxy, cyano, $C_{3-6}$ cycloalkyl, halogen, $C_{1-4}$ haloalkoxy and $C_{1-4}$ haloalkyl.

**66.** The compound according to any one of claims 62 to 64 wherein one or two $R_{10}$-$R_{13}$ groups are independently halogen.

**67.** The compound according to any one of claims 1 to 66 wherein X is N and Y is CH.

**68.** The compound according to any one of claims 1 to 66 wherein X is N and Y is CF.

**69.** The compound according to any one of claims 1 to 66 wherein X is CH and Y is N.

**70.** The compound according to any one of claims 1 to 66 wherein X and Y are N.

**71.** The compound according to any one of claims 1 to 66 wherein X and Y are CH.

**72.** The compound according to any one of claims 1 to 66 wherein X is CH and Y are CF.

**73.** The compound according to claim 1 wherein said compound is selected from the group consisting of:

1-[6-(4-Imidazol-1-yl-phenoxy)-5-nitro-pyrimidin-4-yl]-piperidine-4-carboxylic acid ethyl ester;
1-[6-(2-Methyl-5-trifluoromethyl-2H-pyrazol-3-yloxy)-5-nitro-pyrimidin-4-yl]-piperidine-4-carboxylic acid ethyl ester;
1-[6-(4-Methanesulfonyl-phenoxy)-5-nitro-pyrimidin-4-yl]-piperidine-4-carboxylic acid ethyl ester;
1-[6-(Benzo[1,2,5]oxadiazol-5-yloxy)-5-nitro-pyrimidin-4-yl]-piperidine-4-carboxylic acid ethyl ester;
1-{6-[4-(2-Methoxycarbonyl-acetyl)-phenoxy]-5-nitro-pyrimidin-4-yl}-piperidine-4-carboxylic acid ethyl ester;
1-[5-Amino-6-(2-methyl-5-trifluoromethyl-2H-pyrazol-3-yloxy)-pyrimidin-4-yl]-piperidine-4-carboxylic acid ethyl ester;
1-[6-(2-Methyl-5-trifluoromethyl-2H-pyrazol-3-yloxy)-5-(2,2,2-trifluoro-acetylamino)-pyrimidin-4-yl]-piperidine-4-carboxylic acid ethyl ester;
Propionic acid 1-[2-amino-5-formyl-6-(2-methyl-5-trifluoromethyl-2H-pyrazol-3-yloxy)-pyrimidin-4-yl]-piperidin-4-yl ester;
4-[6-(2-Methyl-5-trafluoromethyl-2H-pyrazol-3-yloxy)-5-nitro-pyrimidin-4-yl]-piperazine-1-carboxylic acid ethyl ester;
1-[6-(2-Methyl-5-trifluoromethyl-2H-pyrazol-3-yloxy)-5-nitro-pyrimidin-4-yl]-piperidine-4-carboxylic acid methyl ester;
2,6-Dimethyl-4-[6-(2-methyl-5-trifluoromethyl-2H-pyrazol-3-yloxy)-5-nitro-pyrimidin-4-yl]-morpholine;
1-[6-(2-Methyl-5-trifluoromethyl-2H-pyrazol-3-yloxy)-5-nitro-pyrimidin-4-yl]-piperidine-3-carboxylic acid ethyl ester;
1-[6-(2-Methyl-5-trifluoromethyl-2H-pyrazol-3-yloxy)-5-nitro-pyrimidin-4-yl]-piperidine-4-carboxylic acid ethylamide;
1-[6-(2-Methyl-5-phenyl-2H-pyrazol-3-yloxy)-5-nitro-pyrimidin-4-yl]-piperidine-4-carboxylic acid ethyl ester;
4-(2-Methyl-5-trifluoromethyl-2H-pyrazol-3-yloxy)-5-nitro-6-piperidin-1-yl-pyrimidine;
1-[5-Nitro-6-(2-trifluoromethyl-benzyloxy)-pyrimidin-4-yl]-piperidine-4-carboxylic acid ethyl ester;
1-[5-Nitro-6-(3-trifluoromethyl-benzyloxy)-pyrimidin-4-yl]-piperidine-4-carboxylic acid ethyl ester;
1-[5-Nitro-6-(4-trifluoromethyl-benzyloxy)-pyrimidin-4-yl]-piperidine-4-carboxylic acid ethyl ester;
1-[5-Bromo-6-(2-methyl-5-trifluoromethyl-2H-pyrazol-3-yloxy)-pyrimidin-4-yl]-piperidine-4-carboxylic acid ethyl ester;
1-[5-Acetylamino-6-(2-methyl-5-trifluoromethyl-2H-pyrazol-3-yloxy)-pyrimidin-4-yl]-piperidine-4-carboxylic acid ethyl ester;
1-[5-Diacetylamino-6-(2-methyl-5-trifluoromethyl-2H-pyrazol-3-yloxy)-pyrimidin-4-yl]-piperidine-4-carboxylic acid ethyl ester;
1-[6-(2-Methyl-5-trifluoromethyl-2H-pyrazol-3-yloxy)-5-nitro-pyrimidin-4-yl]-piperidine-4-carboxylic acid;
1-{5-Nitro-6-[2-(2-trifluoromethyl-phenyl)-ethoxy]-pyrimidin-4-yl}-piperidine-4-carboxylic acid ethyl ester;
1-{5-Nitro-6-[2-(3-trifluoromethyl-phenyl)-ethoxy]-pyrimidin-4-yl}-piperidine-4-carboxylic acid ethyl ester;
1-[5-Di-(methanesulfonyl)amino-6-(2-methyl-5-trifluoromethyl-2H-pyrazol-3-yloxy)-pyrimidin-4-yl]-piperidine-4-carboxylic acid ethyl ester;
1-[5-Nitro-6-(3-trifluoromethyl-phenoxy)-pyrimidin-4-yl]-piperidine-4-carboxylic acid ethyl ester;
1-[5-Methyl-6-(2-methyl-5-trifluoromethyl-2H-pyrazol-3-yloxy)-pyrimidin-4-yl]-piperidine-4-carboxylic acid ethyl ester;
1-[5-Nitro-6-(2-trifluoromethyl-phenoxy)-pyrimidin-4-yl]-piperidme-4-carboxylic acid ethyl ester;
1-[5-Nitro-6-(4-trifluoromethyl-phenoxy)-pyrimidin-4-yl]-piperidine-4-carboxylic acid ethyl ester;

1-[6-(4-Fluoro-phenoxy)-5-nitro-pyrimidin4-yl]-piperidine4-carboxylic acid ethyl ester;
1-[6-(2,5-Dimethyl-2H-pyrazol-3-yloxy)-5-nitro-pyrimidin4-yl]-piperidine-4-carboxylic acid ethyl ester;
1-[6-(4-Bromo-phenoxy)-5-nitro-pyrimidin-4-yl]-piperidine-4-carboxylic acid ethyl ester;
1-[6-(4-Chloro-phenoxy)-5-nitro-pyrimidin-4-yl]-piperidine-4-carboxylic acid ethyl ester;
1-[6-(4-Carbamoyl-phenoxy)-5-nitro-pyrimidin-4-yl]-piperidine-4-carboxylic acid ethyl ester;
1-{6-[4-(2-Methoxy-ethyl)-phenoxy]-5-nitro-pyrimidin-4-yl}-piperidine-4-carboxylic acid ethyl ester;
1-[6-(4-Cyclopentyl-phenoxy)-5-nitro-pyrimidin-4-yl]-piperidine-4-carboxylic acid ethyl ester;
1-[5-Nitro-6-(4-pyrrol-1-yl-phenoxy)-pyrimidin-4-yl]-piperidine-4-carboxylic acid ethyl ester;
1-[6-(4-Benzoyl-phenoxy)-5-nitro-pyrimidin-4-yl]-piperidine-4-carboxylie acid ethyl ester;
1-{6-[4-(4-Hydroxy-benzenesulfonyl)-phenoxy]-5-nitro-pyrimidin-4-yl}-piperidine-4-carboxylic acid ethyl ester;
1-[6-(4'-Cyano-biphenyl-4-yloxy)-5-nitro-pyrimidin-4-yl]-piperidine-4-carboxylic acid ethyl ester;
1-[6-(2-Amino-4-ethanesulfonyl-phenoxy)-5-nitro-pyrimidin-4-yl]-piperidine-4-carboxylic acid ethyl ester;
1-{6-[4-(5-Hydroxy-pyrimidin-2-yl)-phenoxy]-5-nitro-pyrimidin-4-yl}-piperidine-4-carboxylic acid ethyl ester;
1-[5 Nitro-6-(4-sulfo-phenoxy)-pyrimidin-4-yl]-piperidine-4-carboxylic acid ethyl ester;
1-[5-Nitro-6-(4-[1,2,4]triazol-1-yl-phenoxy)-pyrimidin-4-yl]-piperidine-4-carboxylic acid ethyl ester;
1-[6-(4-Carbamoylmethyl-phenoxy)-5-nitro-pyrimidin-4-yl]-piperidine-4-carboxylic acid ethyl ester;
1-{6-[4-(1,3-Dioxo-1,3-dihydro-isoindol-2-yl)-phenoxy]-5-nitro-pyrimidin-4-yl}-piperidine-4-carboxylic acid ethyl ester;
1-[6-(4'-Methoxy-biphenyl-4-yloxy)-5-nitro-pyrimidin-4-yl]-piperidine-4-carboxylic acid ethyl ester;
1-{6-[4-(2,5-Dioxo-imidazolidin-4-yl)-phenoxy]-5-nitro-pyrimidin-4-yl}-piperidine-4-carboxylic acid ethyl ester;
4-(4,4-Difluoro-piperidin-1-yl)-6-(2-methyl-5-trifluoromethyl-2H-pyrazol-3-yloxy)-5-nitro-pyrimidine;
1-{5-Nitro-6-[4-(4-oxo-cyclohexyl)-phenoxy]-pyrimidin-4-yl}-piperidine-4-carboxylic acid ethyl ester;
1-{5-Nitro-6-[4-(3-oxo-butyl)-phenoxy]-pyrimidin-4-yl}-piperidine-4-carboxylic acid ethyl ester;
1-[5-Nitro-6-(4-propionyl-phenoxy)-pyrimidin-4-yl]-piperidine-4-carboxylic acid ethyl ester;
1-[5-Nitro-6-(4-[1,2,3]thiadiazol-4-yl-phenoxy)-pyrimidin-4-yl]-piperidine-4-carboxylic acid ethyl ester;
1-{6-[4-(2-Hydroxy-ethyl)-phenoxy]-5-nitro-pyrimidin-4-yl}-piperidine-4-carboxylic acid ethyl ester;
{4-[6-(4,4-Difluoro-piperidin-1-yl)-5-nitro-pyrimidin-4-yloxy]-phenyl}-phenyl-methanone;
3-{4-[6-(4,4-Difluoro-piperidin-l-yl)-5-nitro-pyrimidin-4-yloxy]-phenyl}-3-oxo-propionic acid methyl ester;
2-[6-(4,4-Difluoro-piperidin-l-yl)-5-nitro-pyrimidin-4-yloxy]-5-ethanesulfonyl-phenylamine;
4-(4-Cyclopentyl-phenoxy)-6-(4,4-difluoro-piperidin-1-yl)-5-nitro-pyrimidine;
1-[6-(2,6-Dichloro-4-methanesulfonyl-phenoxy)-5-nitro-pyrimidin-4-yl]-piperidine-4-carboxylic acid ethyl ester;
1-{6-[4-(4-Chloro-benzoyl)-phenoxy]-5-nitro-pyrimidin-4-yl}-piperidine-4-carboxylic acid ethyl ester;
1-{6-[4-(4-Hydroxy-benzoyl)-phenoxy]-5-nitro-pyrimidin-4-yl}-piperidine-4-carboxylic acid ethyl ester;
1-[6-(4-Cyanomethyl-phenoxy)-5-nitro-pyrimidin-4-yl]-piperidine-4-carboxylic acid ethyl ester;
(4-{6-[4-(2-Methanesulfonyl-ethyl)-piperazin-1-yl]-5-nitro-pyrimidin-4-yloxy} -phenyl)-phenyl-methanone;
4-(4-{6-[4-(2-Methanesulfonyl-ethyl)-piperazin-1-yl]-5-nitro-pyrimidin-4-yloxy}-phenyl)-butan-2-one;
3-(4- {6-[4-(2-Methanesulfonyl-ethyl)-piperazin-1-yl]-5-nitro-pyrimidin-4-yloxy}-phenyl)-3-oxo-propionic acid methyl ester;
4-(4-Methyl-piperidin-1-yl)-6-(2-methyl-5-trifluoromethyl-2H-pyrazol-3-yloxy)-5 -nitro-pyrimidine;
4-(4-Bromo-piperidin-1-yl)-6-(2-methyl-5-trifluoromethyl-2H-pyrazol-3-yloxy)-5-nitro-pyrimidine;
4-(2-Methyl-5-trifluoromethyl-2H-pyrazol-3-yloxy)-5-nitro-6-(4-propyl-piperidin-1-yl)-pyrimidine;
1-[6-(2-Methyl-5-trifluoromethyl-2H-pyrazol-3-yloxy)-5-nitro-pyrimidin-4-yl]-piperidine-4-carboxylic acid amide;
1-[5-Nitro-6-(2-oxo-2H-chromen-6-yloxy)-pyrimidin-4-yl]-piperidine-4-carboxylic acid ethyl ester;
1-[5-Nitro-6-(2-oxo-benzo[1,3]oxathiol-6-yloxy)-pyrimidin-4-yl]-piperidine-4-carboxylic acid ethyl ester;
1-[6-(9H-Carbazol-2-yloxy)-5-nitro-pyrimidin-4-yl]-piperidine-4-carboxylic acid ethyl ester;
1-[5-Nitro-6-(9-oxo-9H-fluoren-2-yloxy)-pyrimidin-4-yl]-piperidine-4-carboxylic acid ethyl ester;
1-{5-Amino-6-[4-(3-oxo-butyl)-phenoxy]-pyrimidin-4-yl}-piperidine-4-carboxylic acid ethyl ester;
1-[6-[4-(3-Oxo-butyl)-phenoxy]-5-(2,2,2-trifluoro-acetylamino)-pyrimidin-4-yl]-piperidine-4-carboxylic acid ethyl ester;
1- {5-Amino-6-[4-(hydroxy-phenyl-methyl)-phenoxy]-pyrimidin-4-yl}-piperidine-4-carboxylic acid ethyl ester;
1-[6-(2-Benzoyl-5-methoxy-phenoxy)-5-nitro-pyrinfidin-4-yl]-piperidine-4-carboxylic acid ethyl ester;
1-[6-(6-Chloro-pyridin-3-yloxy)-5-nitro-pyrimidin-4-yl]-piperidine-4-carboxylic acid ethyl ester;
1-[6-(Benzo[1,3]dioxol-5-yloxy)-5-nitro pyrimidin-4-yl]-piperidine-4-carboxylic acid ethyl ester;
1-[6-(4-Benzyloxy-phenoxy)-5-nitro-pyrimidin-4-yl]-piperidine-4-carboxylic acid ethyl ester;
1-[6-(3-Morpholin-4-yl-phenoxy)-5-nitro-pyrimidin-4-yl]-piperidine-4-carboxylic acid ethyl ester;
1-[5-Nitro-6-(4-trifluoromethylsulfanyl-phenoxy)-pyrimidin-4-yl]-piperidine-4-carboxylic acid ethyl ester;
1-[5-Nitro-6-(4-trifluoromethoxy-phenoxy)-pyrimidin-4-yl]-piperidine-4-carboxylic acid ethyl ester;
1-[6-(4-Benzoyl-phenoxy)-5-(2,2,2-trifluoro-acetylamino)-pyrimidin-4-yl]-piperidine-4-carboxylic acid ethyl ester;
{4-[5-Nitro-6-(4-propyl-piperidin-1-yl)-pyrimidin-4-yloxy]-phenyl}-phenyl-methanone;
{4-Methoxy-2-[5-nitro-6-(4-propyl-piperidin-1-yl)-pyrimidin-4-yloxy]-phenyl}-phenyl-methanone;
4-{4-[5-Nitro-6-(4-propyl-piperidin-1-yl)-pyrimidin-4-yloxy]-phenyl}-butan-2-one;
5-Nitro-4-(4-propyl-piperidin-1-yl)-6-(4-[1,2,3]thiadiazol-4-yl-phenoxy)-pyrimidine;
3-{4-[5-Nitro-6-(4-propyl-piperidin-1-yl)-pyrimidin-4-yloxy]-phenyl}-3-oxo-propionic acid methyl ester;
5-Ethanesulfonyl-2-[5-nitro-6-(4-propyl-piperidin-1-yl)-pyrimidin-4-yloxy]-phenylamine;
1-[6-(4-Difluoromethoxy-benzyloxy)-5-nitro-pyrimidin-4-yl]-piperidine-4-carboxylic acid ethyl ester;
1-[6-(3-Difluoromethoxy-benzyloxy)-5-nitro-pyrimidin-4-yl]-piperidine4-carboxylic acid ethyl ester;
2-{1-[6-(2-Methyl-5-trifluoromethyl-2H-pyrazol-3-yloxy)-5-nitro-pyrimidim-4-yl]-piperidin-4-yl}-ethanol;
3-{1-[6-(2-Methyl-5-trifluoromethyl-2H-pyrazol-3-yloxy)-5-nitro-pyrimidin-4-yl]-piperidin-4-yl}-propionic acid;
4-[4-(4-Methyl-benzyl)-piperidin-1-yl]-6-(2-methyl-5-trifluoromethyl-2H-pyrazol-3-yloxy)-5-nitro-pyrimidine;
4-(3-Methanesulfonyl-pyrrolidin-1-yl)-6-(2-methyl-5-trifluoromethyl-2H-pyrazol-3-yloxy)-5-nitro-pyrimidine;
4-(2-Methyl-5-trifluoromethyl-2H-pyrazol-3-yloxy)-5-nitro-6-[4-(2-trifluoromethyl-phenoxy)-piperidin-1-yl]-pyrimidine;
4-(2-Methyl-5-trifluoromethyl-2H-pyrazol-3-yloxy)-5-nitro-6-[4-(pyridin-2-ylsulfanyl)-piperidin-1-yl]-pyrimidine;
4'-(4-Benzoyl-phenoxy)-3'-nitro-3,4,5,6-tetrahydro-2H-[1,2']bipyridinyl-4-carboxylic acid ethyl ester;
3'-Nitro-4'-[4-(3-oxo-butyl)-phenoxy]-3,4,5,6-tetrahydro-2H-[1,2']bipyridinyl-4-carboxylic acid ethyl ester;
4'-[4-(2-Methoxycarbonyl-acetyl)-phenoxy]-3'-nitro-3,4,5,6-tetrahydro-2H-[1,2']bipyridinyl-4-carboxylic acid ethyl ester;
4'-(2-Amino-4-ethanesulfonyl-phenoxy)-3'-nitro-3,4,5,6-tetrahydro-2H-[1,2']bipyridinyl-4-carboxylic acid ethyl ester;
4'-(4-Imidazol-1-yl-phenoxy)-3'-nitro-3,4,5,6-tetrahydro-2H-[1,2']bipyridinyl-4-carboxylic acid ethyl ester;
4-(2-Methyl-5-trifluoromethyl-2H-pyrazol-3-yloxy)-5-nitro-6-(4-trifluoromethyl-piperidin-1-yl)-pyrimidine;
4-(2-Methyl-5-trifluoromethyl-2H-pyrazol-3-yloxy)-5-nitro-6-(4-phenylsulfanyl-piperidin-1-yl)-pyrimidine;
1-[6-(3-Ethynyl-phenoxy)-5-nitro-pyrimidin-4-yl]-piperidine-4-carboxylic acid ethyl ester;
1-[6-(4-Chloro-2-fluoro-phenoxy)-5-nitro-pyfimidin-4-yl]-piperidine-4-carboxylic acid ethyl ester;
1-[6-(2,4-Difluoro-phenoxy)-5-nitro-pyrirmdin-4-yl]-piperidine-4-carboxylic acid ethyl ester;
1-[6-(4-Bromo-2-fluoro-phenoxy)-5-nitro-pyrimidin-4-yl]-piperidine-4-carboxylic acid ethyl ester;
4-(3-Ethynyl-phenoxy)-5-nitro-6-(4-propyl-piperidin-1-yl)-pyrimidine;
4-(4-Chloro-2-fluoro-phenoxy)-5-nitro-6-(4-propyl-piperidin-1-yl)-pyrimidine;
4-(2,4-Difluoro-phenoxy)-5-nitro-6-(4-propyl-piperidin-1-yl)-pyrimidine;
4-(4-Bromo-2-fluoro-phenoxy)-5-nitro-6-(4-propyl-piperidin-1-yl)-pyrimidine;
3'-Nitro-2'-[4-(3-oxo-butyl)-phenoxy]-3,4,5,6-tetrahydro-2H-[1,4']bipyridinyl-4-carboxylic acid ethyl ester;
4-[4-(3'-Nitro-4-propyl-3,4,5,6-tetrahydro-2H-[1,4']bipyridinyl-2'-yloxy)-phenyl]-butan-2-one;
2'-(4-Benzoyl-phenoxy)-3'-nitro-3,4,5,6-tetrahydro-2H-[1,4']bipyridinyl-4-carboxylic acid ethyl ester;
4-(4-{5-Nitro-6-[4-(pyridin-2-ylsulfanyl)-piperidin-1-yl]-pyrimidin-4-yloxy}-phenyl)-butan-2-one;
[4-(3'-Nitro-4-propyl-3,4,5,6-tetrahydro-2H-[1,4']bipyridinyl-2'-yloxy)-phenyl]-phenyl-methanone;
4-(4-{5-Nitro-6-[4-(2-trifluoromethyl-phenoxy)-piperidin-1-yl]-pyrimidin-4-yloxy}-phenyl)-butan-2-one;
4-(4-{6-[4-(3-Methyl-[1,2,4]oxadiazol-5-yl)-piperidin-1-yl]-5-nitro-pyrimidin-4-yloxy}-phenyl)-butan-2-one;
(4-{6-[4-(3-Methyl-[1,2,4]oxadiazol-5-yl)-piperidin-1-yl]-5-nitro-pyrimidin-4-yloxy}-phenyl)-phenyl-methanone;
1-{6-[4-(4-Fluoro-benzoyl)-phenoxy]-5-nitro-pyrimidin-4-yl}-piperidine-4-carboxylic acid ethyl ester;
(4-Fluoro-phenyl)-{4-[5-nitro-6-(4-propyl-piperidin-1-yl)-pyrimidin-4-yloxy]-phenyl}-methanone;
4-[4-(3-Methyl-[1,2,4]oxadiazol-5-yl)-piperidin-1-yl]-6-(2-methyl-5-trifluoromethyl-2H-pyrazol-3-yloxy)-5-nitro-pyrimidine;
4-(4-Methoxymethyl-piperidin-1-yl)-6-(2-methyl-5-trifluoromethyl-2H-pyrazol-3-yloxy)-5-nitro-pyrimidine;
4-{4-[6-(4-Methoxymethyl-piperidin-1-yl)-5-nitro-pyrimidin-4-yloxy]-phenyl} -butan-2-one;
4-[4-(2-Methoxy-ethyl)-piperidin-1-yl]-6-(2-methyl-5-trifluoromethyl-2H-pyrazol-3-yloxy)-5-nitro-pyrimidine;
4-14-[6-(4-Ethoxymethyl-piperidin-1-yl)-5-nitro-pyrimidin-4-yloxy]-phenyl}-butan-2-one;
4-(2,4-Difluoro-phenoxy)-5-nitro-6-[4-(pyridin-2-ylsulfanyl)-piperidin-1-yl]-pyrimidine;
(4-Methoxy-2-{5-nitro-6-[4-(pyridin-2-ylsulfanyl)-piperidin-1-yl]-pyrimidin-4-yloxy}-phenyl)-phenyl-methanone;
4-(2,4-Difluoro-phenoxy)-6-(4-ethoxymethyl-piperidin-1-yl)-5-nitro-pyrimidine;
4-{4-[6-(4-Cyclopropylmethoxymethyl-piperidin-1-yl)-5-nitro-pyrimidin-4-yloxy]-phenyl}-butan-2-one;
4-{4-[5-Nitro-6-(4-propoxymethyl-piperidin-1-yl)-pyrimidin-4-yloxy]-phenyl}-butan-2-one;
1-{4-[6-(4-Methoxymethyl-piperidin-1-yl)-5-nitro-pyrimidin-4-yloxy]-phenyl}-ethanone;
4-{4-[2-Nitro-3-(4-propyl-piperidin-1-yl)-phenoxy]-phenyl}-butan-2-one;
1-{4-[2-Nitro-3-(4-propyl-piperidin-1-yl)-phenoxy]-phenyl}-ethanone;
{4-[2-Nitro-3-(4-propyl-piperidin-1-yl)-phenoxy]-phenyl}-phenyl-methanone;

3-{4-[2-Nitro-3-(4-propyl-piperidin-1-yl)-phenoxy]-phenyl}-3-oxo-propionic acid methyl ester;
4-(4-[6-(4-Butoxymethyl-piperidin-1-yl)-5-nitro-pyrimidin-4-yloxy]-phenyl}-butan-2-one;
4-{4-[6-(4-Isobutoxymethyl-piperidin-1-yl)-5-nitro-pyrimidin-4-yloxy]-phenyl}-butan-2-one;
(4-Fluoro-phenyl)-[4-(3'-nitro-4-propyl-3,4,5,6-tetrahydro-2H-[1,2']bipyridinyl-4'-yloxy)-phenyl]-methanone;
4-[4-(3'-Nitro-4-propyl-3,4,5,6-tetrahydro-2H-[1,2']bipyridinyl-4'-yloxy)-phenyl]-butan-2-one;
3'-Nitro-4-propyl-4'-(4-[1,2,4]triazol-1-yl-phenoxy)-3,4,5,6-tetrahydro-2H-[1,2']bipyridinyl;
1-{2-Nitro-3-[4-(3-oxo-butyl)-phenoxy]-phenyl}-piperidine-4-carboxylic acid ethyl ester;
1-[3-(4-Benzoyl-phenoxy)-2-nitro-phenyl]-piperidine-4-carboxylic acid ethyl ester;
{4-[6-(4-Ethoxy-piperidin-1-yl)-5-nitro-pyrimidin-4-yloxy]-phenyl}-(4-fluoro-phenyl)-methanone;
1-[6-(2-Methyl-5-trifluoromethyl-2H-pyrazol-3-yloxy)-5-nitro-pyrimidin-4-yl]-piperidin-4-ol;
1-[6-(4-Acetyl-phenoxy)-5-nitro-pyrimidin-4-yl]-piperidine-4-carboxylic acid ethyl ester;
(1-{6-[4-(4-Fluoro-benzoyl)-phenoxy]-5-nitro-pyrimidin-4-yl}-piperidin-4-yl)-(4-fluoro-phenyl)-methanone;
4-(4-{6-[4-(4-Fluoro-benzoyl)-piperidin-1-yl]-5-nitro-pyrimidin-4-yloxy}phenyl)-butan-2-one;
4-(4-Methanesulfonyl-phenoxy)-5-nitro-6-[4-(pyridin-2-ylsulfanyl)-cyclohexyl]-pyrimidine;
4-(4-Methanesulfonyl-phenoxy)-5-nitro-6-[4-(pyridin-4-ylsulfanyl)-cyclohexyl]-pyrimidine;
4-[4-(3-Isopropyl-[1,2,4]oxadiazol-5-yl)-piperidin-1-yl]-6-(4-methanesulfonyl-phenoxy)-pyrimidine-5-carbonitrile;
5-[1,3]Dioxolan-2-yl-4-[4-(3-isopropyl-[1,2,4]oxadiazol-5-yl)-piperidin-1-yl]-6-(4-methanesulfonyl-phenoxy)-pyrimidine;
4-[4-(3-Isopropyl-[1,2,4]oxadiazol-5-yl)-piperidin-1-yl]-6-(4-methanesulfonyl-phenoxy)-pyrimidine-5-carbaldehyde;
5-[1,3] Dioxolan-2-yl-4-[4-(3-isopropyl-[1,2,4] oxadiazol-5-yl)-piperidm-1-yl]-6-(4-[1,2,3] thiadiazol-4-yl-phenoxy)-pyrimidine;
4-[4-(3-Isopropyl-[1,2,4]oxadiazol-5-yl)-piperidin-1-yl]-6-(4-[1,2,3]thiadiazol-4-yl-phenoxy)-pyrimidine-5-carbaldehyde;
4-[4-(3-Isopropyl-[1,2,4]oxadiazol-5-yl)-piperidin-1-yl]-6-(4-[1,2,3]thiadiazol-4-yl-phenoxy)-pyrimidine-5-carboxylic acid;
[4-[4-(3-Isopropyl-[1,2,4]oxadiazol-5-yl)-piperidin-1-yl]-6-(4-[ 1,2,3]thiadiazol-4-yl-phenoxy)-pynmidm-5-yl]-methanol;
[4-[4-(3-Isopropyl-[1,2,4]oxadiazol-5-yl)-piperidin-1-yl]-6-(4-[1,2,3]thiadiazot-4-yl-phenoxy)-pyrimidin-5-ylmethyl]-dimethyl-amine;
4-(4-Methanesulfonyl-phenoxy)-5-nitro-6-(4-phenylsulfanyl-cyclohexyl)-pyrimidine;
4-[4-(3-tert-Butyl-[1,2,4]oxadiazol-5-yl)-piperidin-1-yl]-6-(6-methanesulfonyl-pyridin-3-yloxy)-5-nitro-pyrimidine;
4-[4-(3-Isopropyl-[1,2,4]oxadiazol-5-yl)-piperidin-1-yl]-6-(4-methanesulfonyl-phenoxy)-2-methyl-pyrimidine-S-carbonitrile;
and
1-[4-[4-(3-Isopropyl-[1,2,4]oxadiazol-5-yl)-piperidin-1-yl]-6-(4-methanesulfonyl-phenoxy)-pyrimidin-5-yl]-ethanone;

or a pharmaceutically acceptable salt, hydrate or solvate thereof.

**74.** The compound according to claim 1 wherein said compound is selected from the group consisting of:

1-{6-[(Benzo[1,3]dioxol-5-ylmethyl)-amino]-5-nitro-pyrimidin-4-yl}-piperidine-4-carboxylic acid ethyl ester;
1-[5-Nitro-6-(3,4,5-trimethoxy-benzylamino)-pyrimidin-4-yl]-piperidine-4-carboxylic acid ethyl ester;
(5-Nitro-6-piperidin-1-yl-pyritnidin-4-yl)-(3-trifluoromethyl-benzyl)-amine;
1-[5-Nitro-6-(2-trifluoromethyl-benzylamino)-pyrimidin-4-yl]-piperidine-4-carboxylic acid ethyl ester;
1-[5-Nitro-6-(4-trifluoromethyl-benzylamino)-pyrimidin-4-yl]-piperidine-4-carboxylic acid ethyl ester;
1-[5-Nitro-6-(3-trifluoromethyl-benzylamino)-pyrimidin-4-yl]-piperidine-4-carboxylic acid ethyl ester;
(5-Nitro-6-piperidin-1-yl-pyrimidin-4-yl)-(2-trifluoromethyl-benzyl)-amine;
(5-Nitro-6-piperidin-1-yl-pyrimidin-4-yl)-(4-trifluoromethyl-benzyl)-amine;
1-[5-Amino-6-(3-trifluoromethyl-benzylamino)-pyrimidin-4-yl]-piperidine-4-carboxylic acid ethyl ester;
1-[5-Amino-6-(4-trifluoromethyl-benzylamino)-pyrimidin-4-yl]-piperidine-4-carboxylic acid ethyl ester;
1-[6-(4-Bromo-phenylamino)-5-nitro-pyrimidin-4-yl]-piperidine-4-carboxylic acid ethyl ester;
1-[5-Nitro-6-(4-trifluoromethyl-phenylamino)-pyrimidin-4-yl]-piperidine-4-carboxylic acid ethyl ester;
1-[6-(Methyl-phenyl-amino)-5-nitro-pyrimidin-4-yl]-piperidine-4-carboxylic acid ethyl ester;
1-[5-Nitro-6-(4-trifluoromethoxy-phenylamino)-pyrimidin-4-yl]-piperidine-4-carboxylic acid ethyl ester;

1-[6-(4-Fluoro-phenylamino)-5-nitro-pyrimidin-4-yl]-piperidine-4-carboxylic acid ethyl ester;
1-[6-(3,5-Difluoro-phenylamino)-5-nitro-pyrimidin-4-yl]-piperidine-4-carboxylic acid ethyl ester;
1-[6-(3,5-Dichloro-phenylamino)-5-nitro pyrimidin-4-yl]-piperidine-4-carboxylic acid ethyl ester;
1-[6-(Benzo[1,3]dioxol-5-ylamino)-5-nitro-pyrimidin-4-yl]-piperidine-4-carboxylic acid ethyl ester;
1-[6-(2-Bromo-4-trifluoromethoxy-phenylamino)-5-nitro-pyrimidin-4-yl]-piperidine-4-carboxylic acid ethyl ester;
1-[6-(2-Fluoro-phenylamino)-5-nitro-pyrimidin-4-yl]-piperidine-4-carboxylic acid ethyl ester;
1-[6-(3-Fluoro-phenylamino)-5-nitro-pyrimidin-4-yl]-piperidine-4-carboxylic acid ethyl ester;
1-{6-[(2-Fluoro-phenyl)-methyl-amino]-5-nitro-pyrimidin-4-yl}-piperidine-4-carboxylic acid ethyl ester;
1-[6-(Ethyl-phenyl-amino)-5-nitro-pyrimidin-4-yl]-piperidine-4-carboxylic acid ethyl ester;
1-{6-[(4-Chloro-phenyl)-methyl-amino]-5-nitro-pyrimidin-4-yl}-piperidine-4-carboxylic acid ethyl ester;
1-[6-(4-Difluoromethyl-benzylamino)-5-nitro-pyrimidin-4-yl]-piperidine-4-carboxylic acid ethyl ester;
1-{6-[(2,3-Dihydro-benzo[1,4]dioxin-6-ylmethyl)-amino]-5-nitro-pyrimidin-4-yl}-piperidine-4-carboxylic acid ethyl ester;
1-{6-[(2,3-Dihydro-benzo[1,4]dioxin-2-ylmethyl)-amino]-5-nitro-pyrimidin-4-yl}-piperidine-4-carboxylic acid ethyl ester;
1-{6-[(2,3-Dihydro-benzofuran-5-ylmethyl)-amino]-5-nitro-pyrimidin-4-yl}-piperidine-4-carboxylic acid ethyl ester;
1-{6-[(6-Fluoro-4H-benzo[1,3]dioxin-8-ylmethyl)-amino]-5-nitro-pyrimidin-4-yl}-piperidine-4-carboxylic acid ethyl ester;
1-[6-(3,4-Dihydro-2H-benzo[b][1,4]dioxepin-7-ylamino)-5-nitro-pyrimidin-4-yl]-piperidine-4-carboxylic acid ethyl ester;
1-{6-[4-(Morpholine-4-sulfonyl)-phenylamino]-5-nitro-pyrimidin-4-yl}-piperidine-4-carboxylic acid ethyl ester;
1-[6-(2,2-Difluoro-benzo[1,3]dioxol-4-ylamino)-5-nitro-pyrimidin-4-yl]-piperidine-4-carboxylic acid ethyl ester;
1-[6-(2,2-Difluoro-benzo[1,3]dioxol-5-ylamino)-5-nitro-pyrimidin-4-yl]-piperidine-4-carboxylic acid ethyl ester;
1-[6-(1,1-Dioxo-1H-1$\lambda^6$-benzo[b]thiophen-6-ylammo)-5-nitro-pyrimidin-4-yl]-piperidine-4-carboxylic acid ethyl ester;
1-{6-[(Furan-3-ylmethyl)-amino]-5-nitro-pyrimidin-4-yl} piperidine-4-carboxylic acid ethyl ester;
1-{6-[2-(4-Methoxy-phenoxy)-ethylamino]-5-nitro-pyrimidin-4-yl}-piperidine-4-carboxylic acid ethyl ester;
1-{6-[2-(5-Methoxy-1H-indol-3-yl)-ethylamino]-5-nitro-pyrimidin-4-yl}-piperidine-4-carboxylic acid ethyl ester;
(3,4-Dihydro-2H-benzo[b] [1,4]dioxepin-7-yl)-[5-nitro-6-(4-propyl-piperidin-1-yl)-pyrimidin-4-yl]-amine;
(3-Fluoro-phenyl)-[5-nitro-6-(4-propyl-piperidin-1-yl)-pyrimidin-4-yl]-amine;
(3-Methoxy-phenyl)-[5-nitro-6-(4-propyl-piperidin-1-yl)-pyrimidin-4-yl]-amine;
1-{6-[(3-Fluoro-phenyl)-methyl-amino]-5-nitro-pyrimidin-4-yl}-piperidine-4-carboxylic acid ethyl ester;
1-[6-(4-Benzoyl-phenylamino)-5-nitro-pyrimidin-4-yl]-piperidine-4-carboxylic acid ethyl ester;
1-{6-[4-(1,1-Dioxo-1$\lambda^6$-thiomorpholin-4-ylmethyl)-phenylamino]-5-nitro-pyrimidin-4-yl}-piperidine-4-carboxylic acid ethyl ester;
1-[6-(4-Methanesulfonyl-phenylamino)-5-nitro-pyrimidin-4-yl]-piperidine-4-carboxylic acid ethyl ester;
1-[6-(4-Dimethylsulfamoyl-phenylamino)-5-nitro-pyrimidin-4-yl]-piperidine-4-carboxylic acid ethyl ester;
1-[6-(3-Methoxy-phenylamino)-5-nitro-pyrimidin-4-yl]-piperidine-4-carboxylic acid ethyl ester;
1-[6-(2-Methoxy-phenylamino)-5-nitro-pyrimidin-4-yl]-piperidine-4-carboxylic acid ethyl ester;
1-[6-(3,5-Bis-trifluoromethyl-phenylamino)-5-nitro-pyrimidin-4-yl]-piperidine-4-carboxylic acid ethyl ester;
1-[6-(2,5-Dimethoxy-phenylamino)-5-nitro-pyrimidin-4-yl]-piperidine-4-carboxylic acid ethyl ester;
1-[6-(3,5-Dimethoxy-benzylamino)-5-nitro-pyrimidin-4-yl]-piperidine-4-carboxylic acid ethyl ester;
[5-Nitro-6-(4-propyl-piperidin-1-yl)-pyrimidin-4-yl]-(3,4,5-trimethoxybenzyl)-amine;
(3,5-Dimethoxy-benzyl)-[5-nitro-6-(4-propyl-piperidin-1-yl)-pyrimidin-4-yl]-amine;
(4-{5-Nitro-6-[4-(pyridin-2-ylsulfanyl)-piperidin-1-yl]-pyrimidin-4-ylamino} -phenyl)-phenyl-methanone;
(4-{5-Nitro-6-[4-(2-trifluoromethyl-phenoxy)-piperidin-1-yl]-pyrimidin-4-ylamino} -phenyl)-phenyl-methanone;
1-[6-(4-Cyano-phenylamino)-5-nitro-pyrimidin-4-yl]-piperidine4-carboxylic acid ethyl ester;
1-[6-(3,5-Dimethoxy-phenylamino)-5-nitro-pyrimidin-4-yl]-piperidine-4-carboxylic acid ethyl ester;
1-[6-(4-sec-Butyl-phenylamino)-5-nitro-pyrimidin-4-yl]-piperidine-4-carboxylic acid ethyl ester;
1-[6-(4-Heptyl-phenylamino)-5-nitro-pyrimidin-4-yl]-piperidine-4-carboxylic acid ethyl ester;
2'-(4-Benzoyl-phenylamino)-3'-nitro-3,4,5,6-tetrahydro-2H-[1,4']bipyridinyl-4-carboxylic acid ethyl ester;
1-[5-Nitro-6-(3,4,5-trimethoxy-phenylamino)-pyrimidin-4-yl]-piperidine-4-carboxylic acid ethyl ester;
1-[5-Nitro-6-(4-pentyl-phenylamino)-pyrimidin-4-yl]-piperidine-4-carboxylic acid ethyl ester;
1-{6-[4-(3-Carboxy-propyl)-phenylamino]-5-nitro-pyrimidin-4-yl}-piperidine-4-carboxylic acid ethyl ester;
1-{6-[4-(Cyano-phenyl-methyl)-phenylamino]-5-nitro-pyrimidin-4-yl}-piperidine-4-carboxylic acid ethyl ester;
1-[6-(4-Cyclohexyl-phenylamino)-5-nitro-pyrimidin-4-yl]-piperidine-4-carboxylic acid ethyl ester;
1-[5-Nitro-6-(4-[1,2,4]triazol-1-yl-phenylamino)-pyrimidin-4-yl]-piperidine-4-carboxylic acid ethyl ester;

1-[5-Nitro-6-(4-trifluoromethanesulfonyl-phenylamino)-pyrimidin-4-yl]-piperidine-4-carboxylic acid ethyl ester;
1-[5-Nitro-6-(4-[1,2,3]thiadiazol-4-yl-phenylamino)-pyrimidin-4-yl]-piperidine-4-carboxylic acid ethyl ester;
[6-(4-Ethoxymethyl-piperidin-1-yl)-5-nitro-pyrimidin-4-yl]-(4-methanesulfonyl-phenyl)-amine;
[5-Nitro-6-(4-propyl-piperidin-1-yl)-pyrimidin-4-yl]-(4-[1,2,4]triazol-1-yl-phenyl)-amine;
{5-Nitro-6-[4-(pyridin-2-ylsulfanyl)-piperidin-1-yl]-pyrimidin-4-yl}-(4-[1,2,4]triazol-1-yl-phenyl)-amine;
(2-Fluoro-phenyl)-{6-[4-(3-methyl-[1,2,4]oxadiazol-5-yl)-piperidin-1-yl]-5-nitro-pyrimidin-4-yl}-amine;
(4-Methanesulfonyl-phenyl)-{6-[4-(3-methyl-[1,2,4]oxadiazol-5-yl)-piperidin-1-yl]-5-nitro-pyrimidin-4-yl}-amine;
{6-[4-(3- Methyl-[1,2,4] oxadiazol- 5- yl)-piperidin- 1- yl]- 5- nitro- pyrimidin- 4- yl}-(4-[1,2,4] triazol- 1- yl- phenyl)-amine;
1-{5-Nitro-6-[4-(4-trifluoromethyl-phenoxy)-phenylamino]-pyrimidin-4-yl)-piperidine-4-carboxylic acid ethyl ester;
{6-[4-(3-Ethyl-[1,2,4]oxadiazol-5-yl)-piperidin-1-yl]-5-nitro-pyrimidin-4-yl}-(2-fluoro-phenyl)-amine;
{6-[4-(2-Methoxy-phenylsulfanyl)-piperidin-1-yl]-5-nitro-pyrimidin-4-yl}-(4-[1,2,4]triazol-1-yl-phenyl)-amine;
(4-Methanesulfonyl-phenyl)-{5-nitro-6-[4-(pyridin-2-ylsulfanyl)-piperidin-1-yl]-pyrimidin-4-yl}-amine;
(3-Methoxy-phenyl)- {5-nitro-6-[4-(pyridin-2-ylsulfanyl)-piperidin-1-yl]-pyrimidin-4-yl}-amine;
Benzo[1,3]dioxol-5-yl-[5-nitro-6-(4-propyl-piperidin-1-yl)-pyrimidin-4-yl]-amine;
(4-Fluoro-phenyl)-{1-[5-nitro-6-(4-[1,2,4]triazol-1-yl-phenylamino)-pyrimidin-4-yl] -piperidin-4-yl}-methanone;
[5-Nitro-6-(4-phenylsulfanyl-piperidin-1-yl)-pyrimidin-4-yl]-(4-[1,2,4]triazol-1-yl-phenyl)-amine;
(4-Fluoro-phenyl)-{1-[6-(2-fluoro-phenylamino)-5-nitro-pyrimidin-4-yl]-piperidin-4-yl}-methanone;
1-[6-(2-Methyl-5-phenyl-2H-pyrazol-3-ylamino)-5-nitro-pyrimidin-4-yl]-piperidine-4-carboxylic acid ethyl ester;
(4-Methanesulfonyl-phenyl)-(5-nitro-6-(4-phenylsulfanyl-piperidin-1-yl)-pyrimidin-4-yl]-amine;
(4-Methanesulfonyl-phenyl)-{5-nitro-6-[4-(pyridin-2-yloxy)-piperidin-1-yl]-pyrimidin-4-yl}-amine;
{6-[4-(4-Fluoro-phenoxy)-piperidin-1-yl]-5-nitro-pyrimidin-4-yl}-(4-methanesulfonyl-phenyl)-amine;
(4-Methanesulfonyl-phenyl)-{5-nitro-6-[4-(pyridin-4-yloxy)-piperidin-1-yl]-pyrimidin-4-yl} -amine;
(4-Methanesulfonyl-phenyl)-{5-nitro-6-[4-(pyrimidin-2-yloxy)-piperidin-1-yl]-pyrimidin-4-yl} -amine;
(4-Methanesulfonyl-phenyl)-{5-nitro-6-[4-(pyridin-4-ylsulfanyl)-piperidin-1-yl]-pyrimidin-4-yl}-amine;
(4-Methanesulfonyl-phenyl)-{6-[4-(4-methoxy-phenylsulfanyl)-piperidin-1-yl]-5-nitro-pyrimidin-4-yl} -amine;
[6-(4-Benzenesulfonyl-piperidin-1-yl)-5-nitro-pyrimidin-4-yl]-(4-methanesulfonyl-phenyl)-amine;
{4-[6-(4-Methanesulfonyl-phenylamino)-5-nitro-pyrimidin-4-yl]-piperazin-1-yl}-acetic acid ethyl ester;
(2-Fluoro-phenyl)-{5-nitro-6-[4-(pyridin-2-ylsulfanyl)-piperidin-1-yl]-pyrimidin-4-yl}-amine;
2-Methoxy-phenyl)-{5-nitro-6-[4-(pyridin-2-ylsulfanyl)-piperidin-1-yl]-pyrimidin-4-yl}-amine;
(4-Methanesulfonyl-phenyl)-(5-nitro-6-{4-[3-(3-trifluoromethyl-phenyl)-[1,2,4]oxadiazol-5-yl]-piperidin-1-yl}-pyrimidin-4-yl)-amine;
{6-[4-(3 -Ethyl-[1,2,4]oxadiazol-5-yl)-piperidin-1-yl]-5-nitro-pyrimidin-4-yl}-(4-methanesulfonyl-phenyl)-amine;
(6-{4-[5-(4-riuoro-phenyl)-[1,3,4]oxadiazol-2-yl]-piperidin-l-yl}-5-nitro-pyrimidin-4-yl)-(4-methanesulfonyl-phenyl)-amine;
(4-Methanesulfonyl-phenyl)-[5-nitro-6-(4-pyridin-2-ylmethyl-piperidin-1-yl)-pyrimidin-4-yl]-amine;
1-{6-[4-(4,5-Dichloro-imidazol-1-yl)-phenylamino]-5-nitro-pyrimidin-4-yl}-piperidine-4-carboxylic acid ethyl ester;
Benzo[1,3]dioxol-S-yl-{5-nitro-6-[4-(pyridin-2-ylsulfanyl)-piperidin-1-yl]-pyrimidin-4-yl} -amine;
(4-Fluoro-phenyl)-{1-[6-(2-fluoro-phenylamino)-5-nitro-pyrimidin-4-yl]-piperidin-4-yl}-methanone;
{1-[6-(Benzo[1,3]dioxol-5-ylamino)-5-nitro-pyrimidin-4-yl]-piperidin-4-yl}-(4-fluoro-phenyl)-methanone;
(2,3-Difluoro-phenyl)-{5-nitro-6-[4-(pyridin-2-ylsulfanyl)-piperidin-1-yl]-pyrimidin-4-yl}-amine;
(2,4-Difluoro-phenyl)-{5-nitro-6-[4-(pyridin-2-ylsulfanyl)-piperidin-1-yl]-pyrimidin-4-yl}-amine;
(2,5-Difluoro-phenyl)-{5-nitro-6-[4-(pyridin-2-ylsulfanyl)-piperidin-1-yl]-pyrimidin-4-yl}-amine;
1-[6-(4-Benzenesulfonyl-phenylamino)-5-nitro-pyrimidin-4-yl]-piperidine-4-carboxylic acid ethyl ester;
1-[5-Nitro-6-(2-trifluoromethyl-3H-benzoimidazol-5-ylamino)-pyrimidin-4-yl]-piperidine-4-carboxylic acid ethyl ester;
1-{5-Nitro-6-[3-(1,1,2,2-tetrafluoro-ethoxy)-phenylamino]-pyrimidin-4-yl}-piperidine-4-carboxylic acid ethyl ester;
{6-[4-(4-Iodo-phenoxy)-piperidin-1-yl]-5-nitro-pyrimidin-4-yl}-(4-methanesulfonyl-phenyl)-amine;
(2-Fluoro-4-methanesulfonyl-phenyl)-{6-[4-(3-isopropyl-[1,2,4]oxadiazol-5-yl)-piperidin-1-yl]-5-nitro-pyrimidin-4-yl}-amine;
{6-[4-(3-Ethyl-[1,2,4] oxadiazol-5-yl)-piperidin-1-yl]-5-nitro-pyrimidin-4-yl}-(2-fluoro-4-methanesulfonyl-phenyl)-amine;
(4-Methanesulfonyl-phenyl)-{5-nitro-6-[4-(3-propyl-[1,2,4]oxadiazol-5-yl)-piperidin-1-yl]-pyrimidin-4-yl}-amine;
{6-[4-(3-Cyclopropylmethyl-[1,2,4] oxadiazol-5-yl)-piperidin-1-yl]-5-nitro-pyrimidin-4-yl}-(4-methanesulfonyl-phenyl)-amine;

{6-[4-(3- Isopropyl-[1,2,4] oxadiazol- 5- yl)-piperidin- 1- yl]- 5- nitro- pyrimidin- 4- yl}-(4- methanesulfonyl- phenyl)-amine;
{6-[4-(3- Cyclopropyl-[1,2,4] oxadiazol- 5- yl)-piperidin- 1- yl]- 5- nitro- pyrimidin- 4- yl}-(4- methanesulfonyl- phenyl)-amine;
4-[4-(3-Isopropyl-[1,2,4]oxadiazol-5-yl)-piperidin-1-yl]-6-(4-methylsulfanyl-phenylamino)-pyrimidine-5-carbonitrile;
4-[4-(3- Isopropyl-[1,2,4] oxadiazol- 5- yl)-piperidin- 1- yl]- 6-(4- methanesulfinyl- phenylamino)-pyrimidine- 5- carbonitrile;
(4-Methanesulfonyl-phenyl)-(5-nitro-6-[4-(4-trifluoromethoxy-phenoxy)-pipehdm-1-yl]-pyrinudm-4-yl}-amine;
4-[4-(3- Isopropyl-[1,2,4] oxadiazol- 5- yl)-piperidin- 1- yl]- 6-(4- methanesulfonyl- phenylamino)-pyrimidine- 5- carbonitrile;
1-{1-[6-(2-Fluoro-4-methanesulfonyl-phenylamino)-5-nitro-pyrimidin-4-yl]-piperidin-4-yl}-hexan-1-one;
1-{1-[6-(4-Methanesulfonyl-phenylamino)-5-nitro-pyrimidin-4-yl]-piperidin-4-yl}-hexan-1-one;
{6-[4-(3- tert- Butyl-[1,2,4] oxadiazol- 5- yl)-piperidin- 1- yl]- 5- nitro- pyrimidin- 4- yl}-(2- fluoro- 4- methanesulfonyl-phenyl)-amine;
{6-[4-(3- tert- Butyl-[1,2,4] oxadiazol- 5- yl)-piperidin- 1- yl]- 5- nitro- pyrimidin- 4- yl}-(4- methanesulfonyl- phenyl)-amine;
[6-(4-Benzofuran-2-yl-pipendin-1-yl)-5-nitro-pynmidin-4-yl]-(4-methanesulfonyl-phenyl)-amine;
4-(3- Fluoro- 4- methanesulfonyl- phenylamino)- 6-[4-(3- isopropyl-[1,2,4] oxadiazol- 5- yl)-piperidin- 1- yl]-pyrimidine-5-carbonitrile;
{6-[4-(3- Isopropyl-[1,2,4] oxadiazol- 5- yl)-piperidin- 1- yl]- 5- nitro- pyrimidin- 4- yl}-(5- methanesulfonyl- pyridin- 2-yl)-amine;
(3-Fluoro-4-methanesulfonyl-phenyl)-{6-[4-(3-isopropyl-[1,2,4]oxadiazol-5-yl)-piperidin-1-yl]-5-nitro-pyrimidin-4-yl}-aniine;
{6-[4-(3-Isopropyl-[1,2,4]oxadiazol-5-yl) piperidin-1-yl]-5-nitro-pyrimidin-4-yl}-(6-methanesulfonyl-pyridin-3-yl)-amine;
4-(2,3-Difluoro-phenylamino)-6-[4-(3-isopropyl-[1,2,4]oxadiazol-5-yl)-pipendin-1-yl]-pyrimidine-5-carbonitrile;
4-(2,5-Difluoro-phenylamino)-6-[4-(3-isopropyl-[1,2,4]oxadiazol-5-yl)-piperidin-1-yl]-pyrimidine-5-carbonitrile;
4-[4-(3 -Isopropyl-[1,2,4]oxadiazol-5-yl)-piperidin-1-yl]-6-(4-methylsulfanyl-phenylamino)-pyrimidine-5-carbonitrile;
4-[4-(3- Isopropyl-[1,2,4] oxadiazol- 5- yl)-piperidin- 1- yl]- 6-(4- methanesulfonyl- phenylamino)-pyrimidine- 5- carbonitrile;
4-(4-Hexanoyl-piperidin-1-yl)-6-(6-methylsulfanyl-pyridin-3-ylamino)-pyrimidine-5-carbonitrile;
4-(4-Hexanoyl-piperidin-1-yl)-6-(6-methanesulfonyl-pyridin-3-ylamino)-pyrimidine-5-carbonitrile;
4-[4-(3- Isopropyl-[1,2,4] oxadiazol- 5- yl)-piperidin- 1- yl]- 6-(6- methylsulfanyl- pyridin- 3- ylamino)-pyrimidine- 5-carbonitrile;
4-[4-(3-Isopropyl-[1,2,4]oxadiazol-5-yl)-piperidin-1-yl]-6-(6-methanesulfonyl-pyridin-3-ylamino)-pyrimidine-5-carbonitrile;
1-[4-[4-(3- Isopropyl-[1,2,4] oxadiazol- 5- yl)-piperidin- 1- yl]- 6-(4- methanesulfonyl- phenylamino)-pyrimidin- 5-yl]-ethanone;
and
1-[4-[4-(3-Isopropyl-[1,2,4]oxadiazol-5-yl)-piperidin-1-yl]-6-(6-methanesulfonyl-pyridin-3-ylamino)-pyrimidin-5-yl]-ethanone;

or a pharmaceutically acceptable salt, hydrate or solvate thereof.

**75.** The compound according to claim 1 wherein said compound is selected from the group consisting of:

1-(5-Nitro-6-phenyl-pyrimidin-4-yl)-piperidine-4-carboxylic acid ethyl ester;
1-(6-Naphfhalen-2-yl-5-nitro-pynmidin-4-yl)-pipehdine-4-carboxylic acid ethyl ester;
1-[6-(4-Methanesulfonyl-phenyl)-5-nitro-pyrimidin-4-yl]-piperidine-4-carboxylic acid ethyl ester;
1-(6-Benzofuran-5-yl-5-nitro-pyrimidin-4-yl)-piperidine-4-carboxylic acid ethyl ester;
1-[5-Nitro-6-(3-trifluoromethyl-phenyl)-pyrimidm-4-yl]-piperidine-4-carboxylic acid ethyl ester;
1-[6-(4-Methoxy-phenyl)-5-nitro-pyrimidin-4-yl]-piperidine-4-carboxylic acid ethyl ester;
4-(4-Butyl-piperidin-1-yl)-6-furan-3-yl-5-nitro-pyrimidine;
1-[6-(3-Chloro-phenyl)-5-nitro-pyrimidin-4-yl]-piperidine-4-carboxylic acid ethyl ester;
1-[6-(2,6-Dimethoxy-phenyl)-5-nitro-pyrimidin-4-yl]-piperidine-4-carboxylic acid ethyl ester;
1-(6-Naphthalen-1-yl-5-nitro-pyrimidin-4-yl)-piperidine-4-carboxylic acid ethyl ester;

1-[6-(4-Methylsulfanyl-phenyl)-5-nitro-pyrimidin-4-yl]-piperidine-4-carboxylic acid ethyl ester;
1-(2',4'-Dihydroxy-5-nitro-[4,5']bipyfimidinyl-6-yl)-piperidine-4-carboxylic acid ethyl ester;
1-[6-(4-Methanesulfonyl-phenyl)-5-nitro-pyrimidin-4-yl]-piperidine-4-carboxylic acid ethyl ester;
1-[6-(3,5-Bis-trifluoromethyl-phenyl)-5-nitro-pyrimidin-4-yl]-piperidine-4-carboxylic acid ethyl ester;
1-(6-Dibenzothiophen-4-yl-5-nitro-pyrimidin-4-yl)-piperidine-4-carboxylic acid ethyl ester;
1-[6-(3,5-Dimethyl-isoxazol-4-yl)-5-nitro-pyrimidin-4-yl]-piperidine-4-carboxylic acid ethyl ester;
1-(5-Nitro-6-thiophen-2-yl-pyrimidin-4-yl)-piperidine-4-carboxylic acid ethyl ester;
1-[6-(3,5-Dichloro-phenyl)-5-nitro-pyrimidin-4-yl]-piperidine-4-carboxylic acid ethyl ester;
1-(6-Dibenzofuran-4-yl-5-nitro-pyrimidin-4-yl)-piperidine-4-carboxylic acid ethyl ester;
1-[6-(3,5-Dimethyl-phenyl)-5-nitro-pyrimidin-4-yl]-piperidine-4-carboxylic acid ethyl ester;
1-[6-(4-Acetyl-phenyl)-5-nitro-pyrimidin-4-yl]-piperidine-4-carboxylic acid ethyl ester;
1-[6-(4-Ethanesulfonyl-phenyl)-5-nitro-pyrimidin-4-yl]-piperidine-4-carboxylic acid ethyl ester;
1-[6-(2-Fluoro-biphenyl-4-yl)-5-nitro-pyrimidin-4-yl]-piperidine-4-carboxylic acid ethyl ester;
1-[6-(3-Methanesulfonyl-phenyl)-5-nitro-pyrimidin-4-yl]-piperidine-4-carboxylic acid ethyl ester;
1-{6-[4-(2-Carboxy-ethyl)-phenyl]-5-nitro-pyrimidin-4-yl}-piperidine-4-carboxylic acid ethyl ester;
1-{6-[4-(2-Methoxycarbonyl-ethyl)-phenyl]-5-nitro-pyrimidin-4-yl}-piperidine-4-carboxylic acid methyl ester;
and
1-{6-[4-(2-Methoxycarbonyl-ethyl) phenyl]-5-nitro-pyrimidin-4-yl}-piperidine-4-carboxylic acid ethyl ester;

or a pharmaceutically acceptable salt, hydrate or solvate thereof.

**76.** The compound according to claim 1 wherein said compound is selected from the group consisting of:

1-[5-Nitro-6-(2-trifluoromethyl-phenylethynyl)-pyrimidin-4-yl]-piperidine-4-carboxylic acid ethyl ester;
1-(5-Nitro-6-phenylethynyl-pyrimidin-4-yl)-piperidine-4-carboxylic acid ethyl ester;
1-[5-Nitro-6-(4-trifluoromethyl-phenylethynyl)-pyrimidin-4-yl]-piperidine-4-carboxylic acid ethyl ester;
1-(5-Nitro-6-m-tolylethynyl-pyrimidin-4-yl)-piperidine-4-carboxylic acid ethyl ester;
1-[6-(2-Fluoro-phenylethynyl)-5-nitro-pyrimidin-4-yl]-piperidine-4-carboxylic acid ethyl ester;
1-[5-Nitro-6-(3-trifluoromethyl-phenylethynyl)-pyrimidin-4-yl]-piperidine-4-carboxylic acid ethyl ester;
and
1-[5-Amino-6-(3-trifluoromethyl-phenylethynyl)-pyrimidin-4-yl]-piperidine-4-carboxylic acid ethyl ester;

or a pharmaceutically acceptable salt, hydrate or solvate thereof.

**77.** The compound according to claim 1 wherein said compound is 5-Nitro-4-(5-phenyl-[1,3,4]oxadiazol-2-ylsulfanyl)-6-[4-(pyridin-2-ylsulfanyl)-piperidin-1-yl]-pyrimidine or a pharmaceutically acceptable salt, hydrate or solvate thereof.

**78.** A pharmaceutical composition comprising at least one compound according to any one of claims 1 to 77 and a pharmaceutically acceptable carrier.

**79.** A method for prophylaxis or treatment of a metabolic disorder in an individual comprising administering to the individual a therapeutically effective amount of a compound according to any one of claims 1 to 77 or a pharmaceutical composition of claim 78.

**80.** The method according to claim 79 wherein the metabolic disorder is type I, type II diabetes, inadequate glucose tolerance, insulin resistance, hyperglycemia, hyperlipidemia, hypertriglyceridemia, hypercholesterolemia, dyslipidemia, syndrome X or metabolic syndrome.

**81.** The method according to claim 79 wherein the metabolic disorder is type II diabetes.

**82.** A method for controlling or decreasing weight gain of an individual comprising administering to the individual a therapeutically effective amount of a compound according to any one of claims 1 to 77 or pharmaceutical composition of claim 78.

**83.** A method of modulating a **RUP3** receptor comprising contacting the receptor with a compound according to any one of claims 1 to 77.

**84.** A method of modulating a **RUP3** receptor in an individual comprising contacting the receptor with a compound

according to any one of claims 1 to 77.

**85.** The method of modulating the **RUP3** receptor according to claim 83 or 84 wherein the compound is an agonist.

**86.** The method of modulating the **RUP3** receptor according to claim 83 or 84 wherein the compound is an inverse agonist.

**87.** The method of modulating the **RUP3** receptor according to any one of claims 84 to 86 wherein the modulation of the **RUP3** receptor is prophylaxis or treatment of a metabolic disorder.

**88.** The method of modulating the **RUP3** receptor according to claim 87 wherein the metabolic disorder is type I, type II diabetes, inadequate glucose tolerance, insulin resistance, hyperglycemia, hyperlipidemia, hypertriglyceridemia, hypercholesterolemia, dyslipidemia, syndrome X or metabolic syndrome.

**89.** The method of modulating the **RUP3** receptor according to claim 87 wherein the metabolic disorder is type II diabetes.

**90.** The method of modulating the **RUP3** receptor according to any one of claims 84 to 86 wherein the modulation of the **RUP3** receptor controls or reduces weight gain of the individual.

**91.** The method according to any one of claims 84 to 90 wherein the individual is a mammal.

**92.** The method according to claim 91 wherein the mammal is a human.

**93.** Use of a compound according to any one of claims 1 to 77 for production of a medicament for use in prophylaxis or treatment of a metabolic disorder.

**94.** The use of a compound according to claim 93 wherein the metabolic disorder is type II diabetes, inadequate glucose tolerance, insulin resistance, hyperglycemia, hyperlipidemia, hypertriglyceridemia, hypercholesterolemia, dyslipidemia, syndrome X or metabolic syndrome.

**95.** Use of a compound according to any one of claims 1 to 77 for production of a medicament for use in controlling or decreasing weight gain in an individual.

**96.** The use according to claim 95 wherein the individual is a mammal.

**97.** The use according to claim 96 wherein the mammal is a human.

**98.** A compound according to any one of claims 1 to 77 or a pharmaceutical composition according to claim 78 for use in a method of treatment of the human or animal body by therapy.

**99.** A compound according to any one of claims 1 to 77 or a pharmaceutical composition according to claim 78 for use in a method of prophylaxis or treatment of a metabolic disorder of the human or animal body by therapy.

**100.** The method of producing a pharmaceutical composition comprising admixing at least one compound according to any one of claims 1 to 77 and a pharmaceutically acceptable carrier.

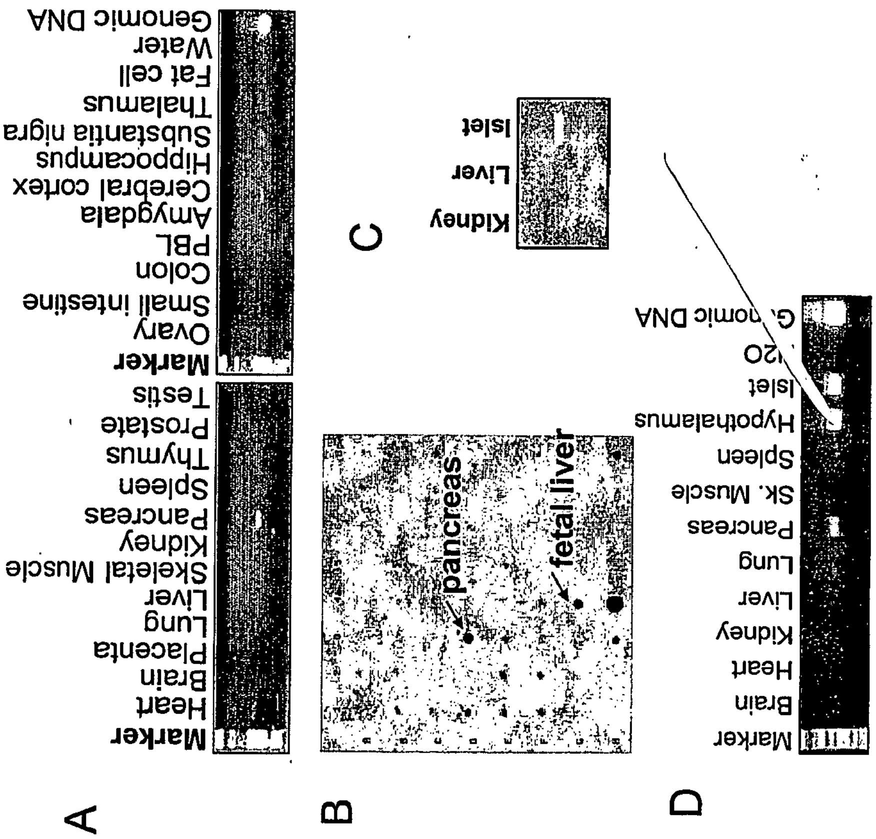
A
Marker
Heart
Brain
Placenta
Lung
Liver
Skeletal Muscle
Kidney
Pancreas
Spleen
Thymus
Prostate
Testis
Marker
Ovary
Small intestine
Colon
PBL
Amygdala
Cerebral cortex
Hippocampus
Substantia nigra
Thalamus
Fat cell
Water
Genomic DNA
B
pancreas
fetal liver
C
Kidney
Liver
Islet
D
Marker
Brain
Heart
Kidney
Liver
Lung
Pancreas
SK. Muscle
Spleen
Hypothalamus
Islet

FIGURE 1

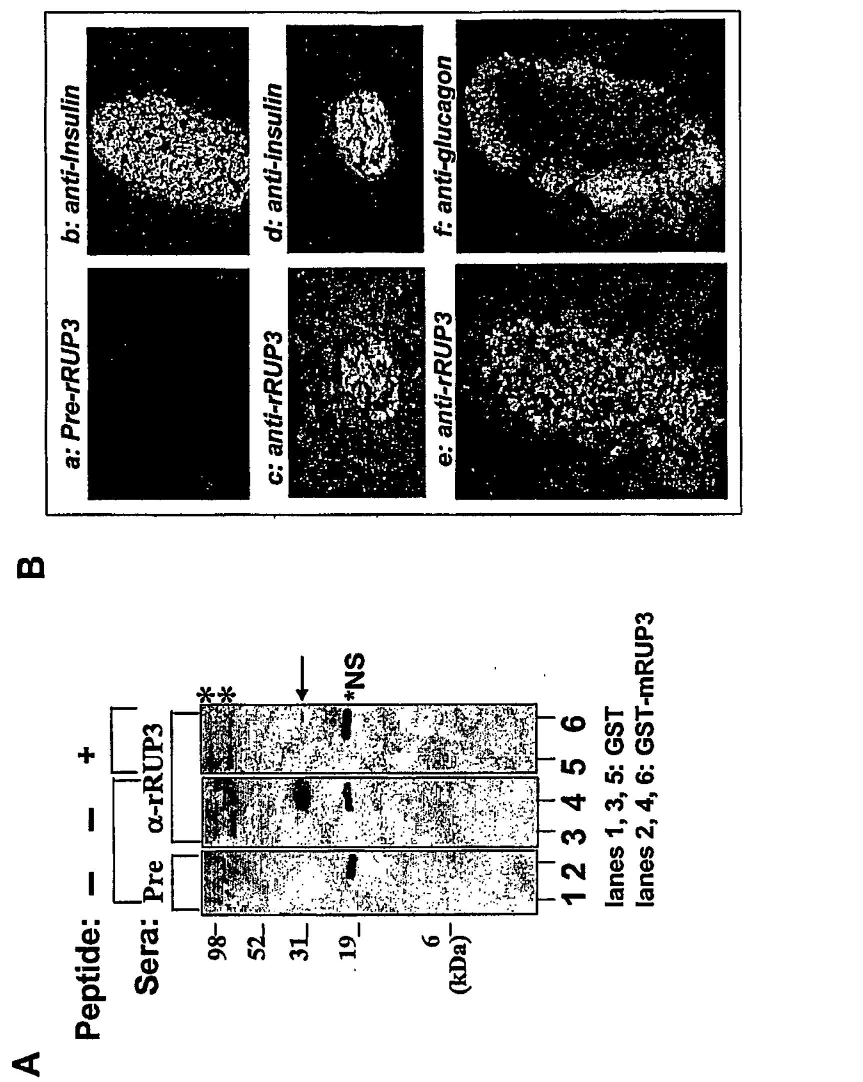
A
Peptide: – – +
Sera: Pre α-rRUP3
98
52
31
19
6
(kDa)
*NS
1 2 3 4 5 6
lanes 1, 3, 5: GST
lanes 2, 4, 6: GST-mRUP3
B
a: Pre-rRUP3
b: anti-Insulin
c: anti-rRUP3
d: anti-Insulin
e: anti-rRUP3
f: anti-glucagon

FIGURE 2

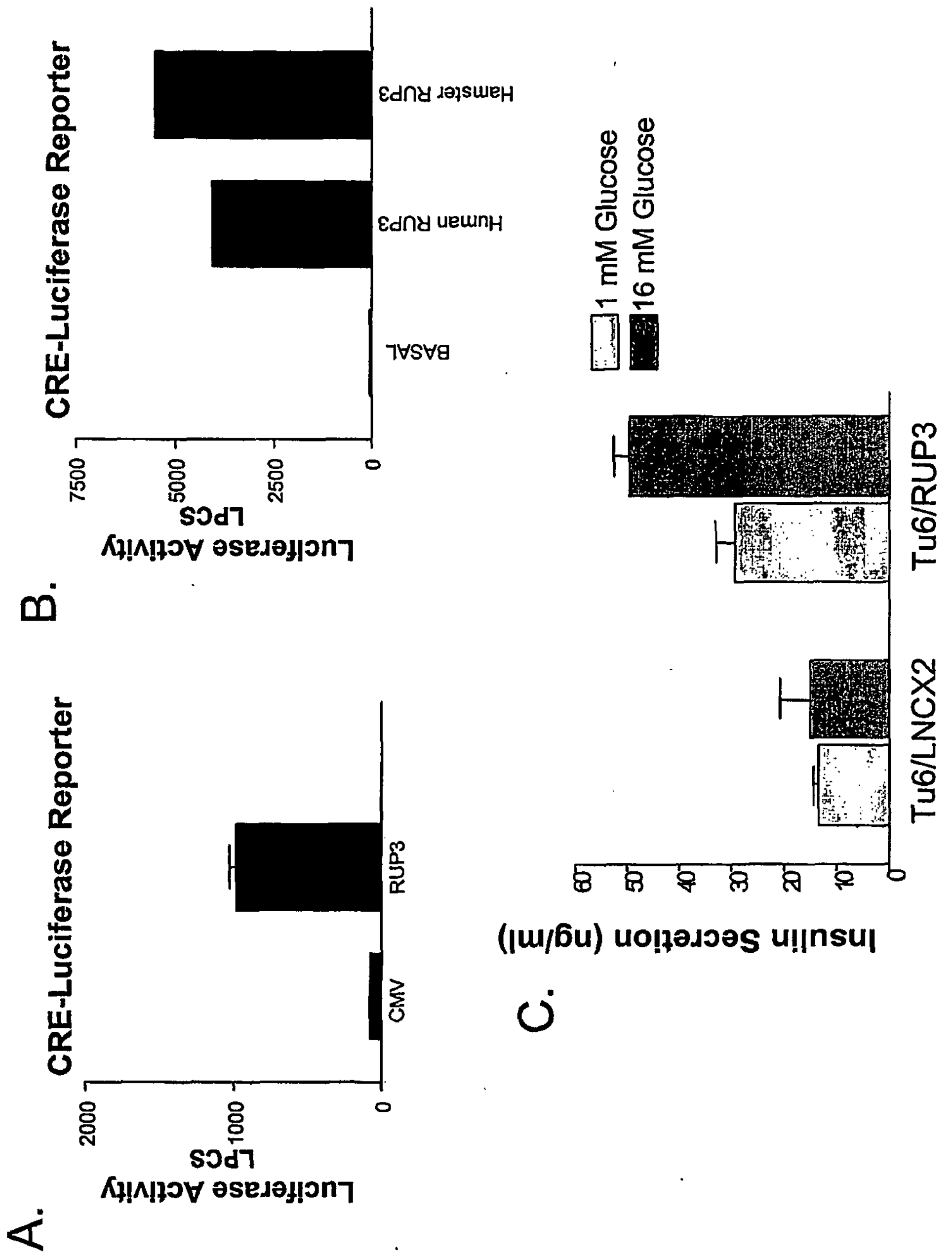
A.
CRE-Luciferase Reporter
Luciferase Activity LPCS
2000
1000
0
CMV
RUP3
B.
CRE-Luciferase Reporter
Luciferase Activity LPCS
7500
5000
2500
0
BASAL
Human RUP3
Hamster RUP3
C.
Insulin Secretion (ng/ml)
60
50
40
30
20
10
0
Tu6/LNCX2
Tu6/RUP3
1 mM Glucose
16 mM Glucose

FIGURE 3

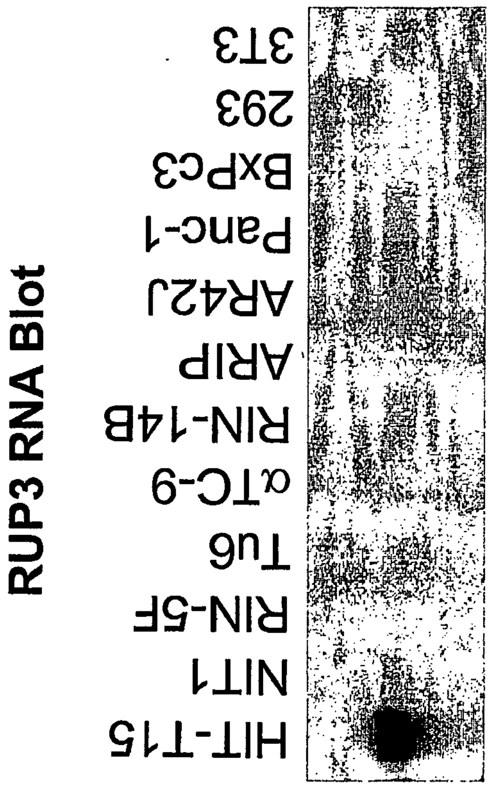
RUP3 RNA Blot
HIT-T15
NIT1
RIN-5F
Tu6
αTC-9
RIN-14B
ARIP
AR42J
Panc-1
BxPc3
293
3T3

FIGURE 4A

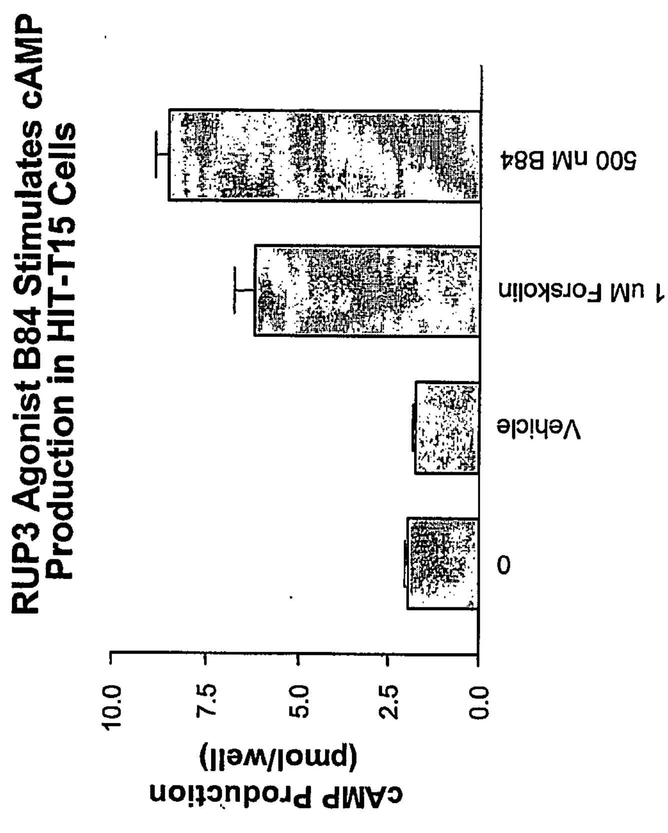
RUP3 Agonist B84 Stimulates cAMP Production in HIT-T15 Cells
cAMP Production (pmol/well)
10.0
7.5
5.0
2.5
0.0
0
Vehicle
1 uM Forskolin
500 nM B84

FIGURE 4B

## RUP3 Agonist Stimulates Insulin Secretion in HIT-T15 Cells

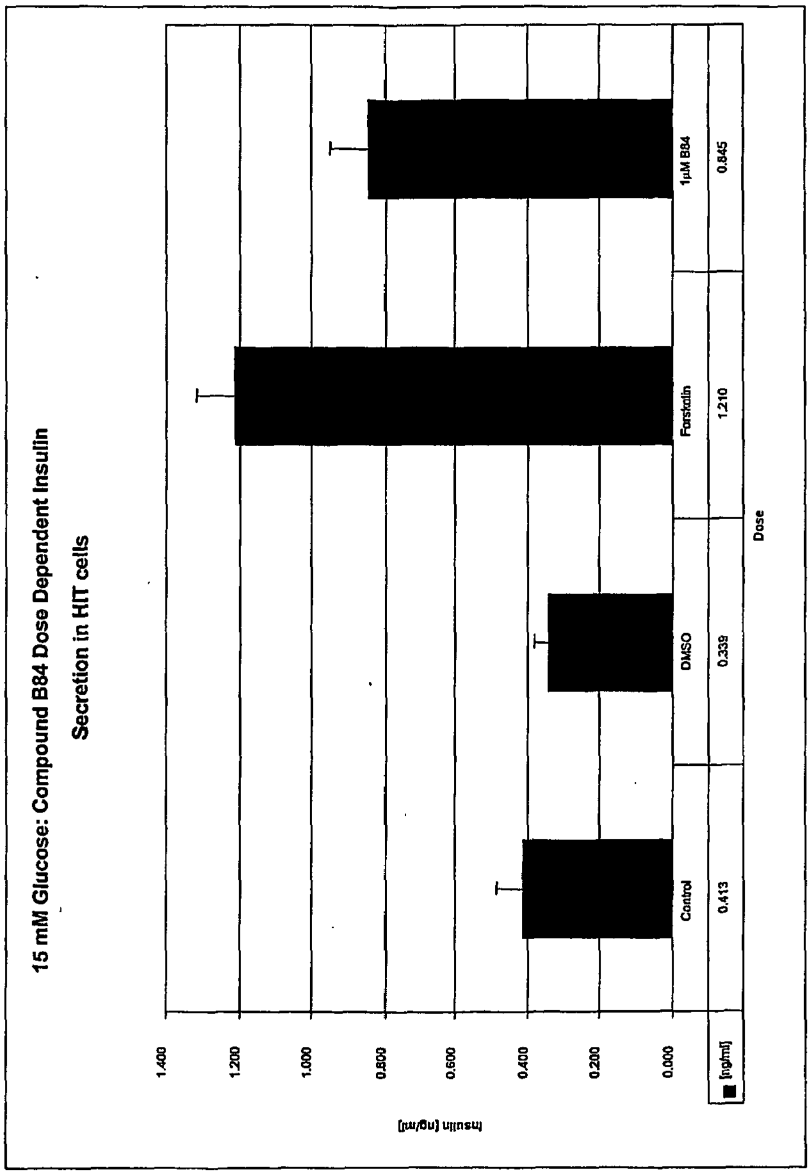


FIGURE 4C

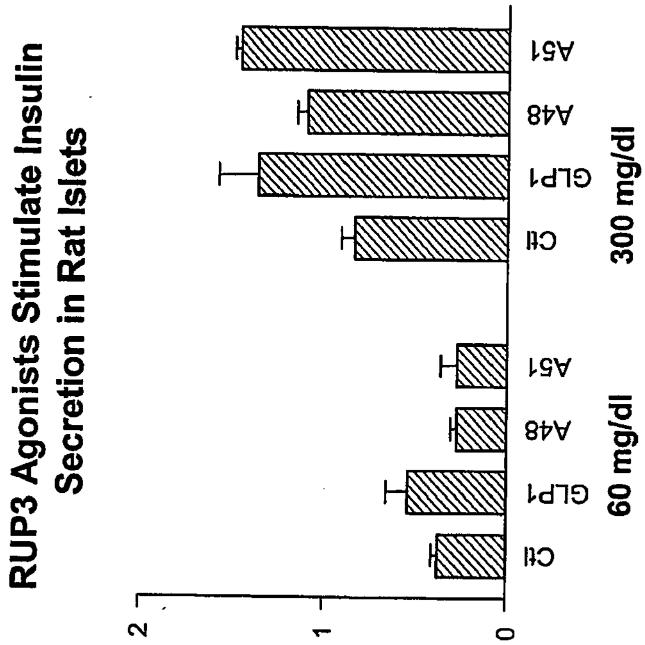
RUP3 Agonists Stimulate Insulin
Secretion in Rat Islets
2
1
0
Ctl
GLP1
A48
A51
Ctl
GLP1
A48
A51
60 mg/dl
300 mg/dl

FIGURE 4D

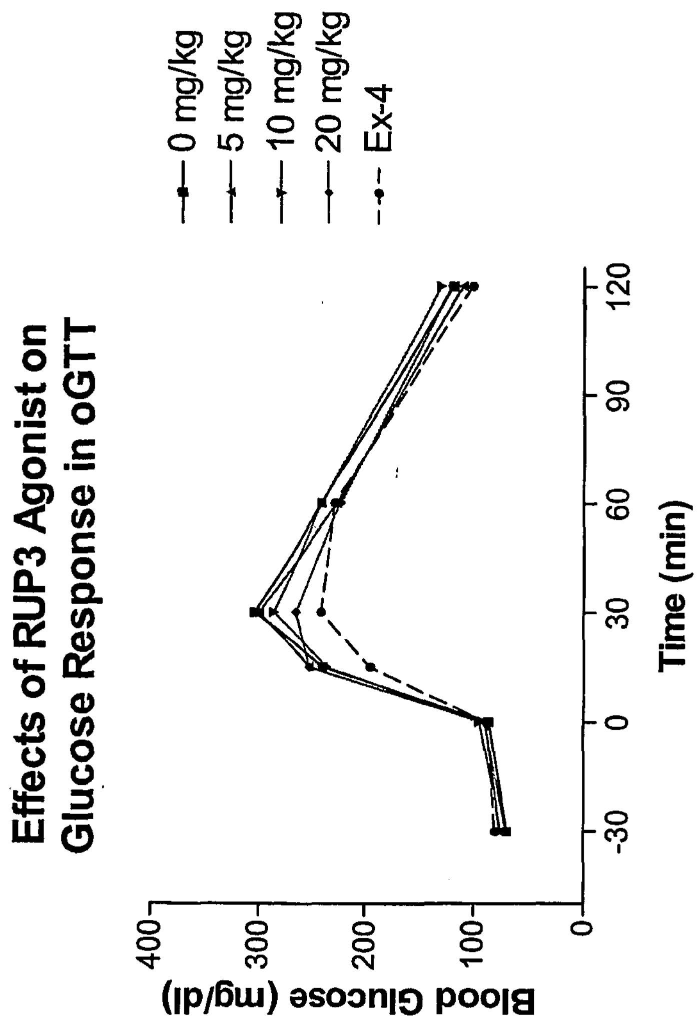
Effects of RUP3 Agonist on
Glucose Response in oGTT
Blood Glucose (mg/dl)
400
300
200
100
0
-30
0
30
60
90
120
Time (min)
0 mg/kg
5 mg/kg
10 mg/kg
20 mg/kg
Ex-4

FIGURE 5A

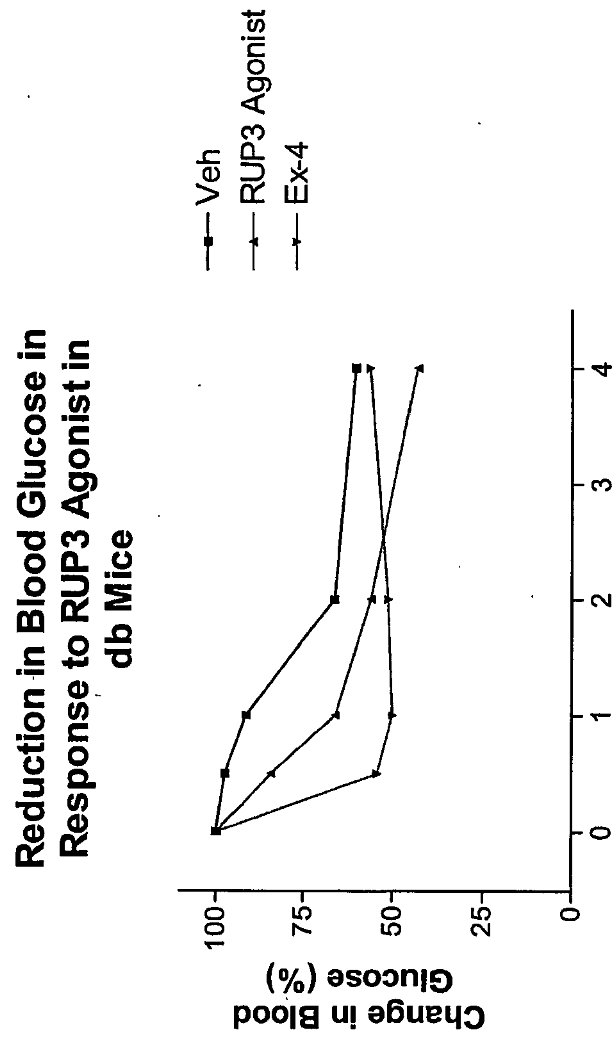
Reduction in Blood Glucose in Response to RUP3 Agonist in db Mice
Veh
RUP3 Agonist
Ex-4
Change in Blood Glucose (%)
100
75
50
25
0
0
1
2
3
4
Time (hr)

FIGURE 5B

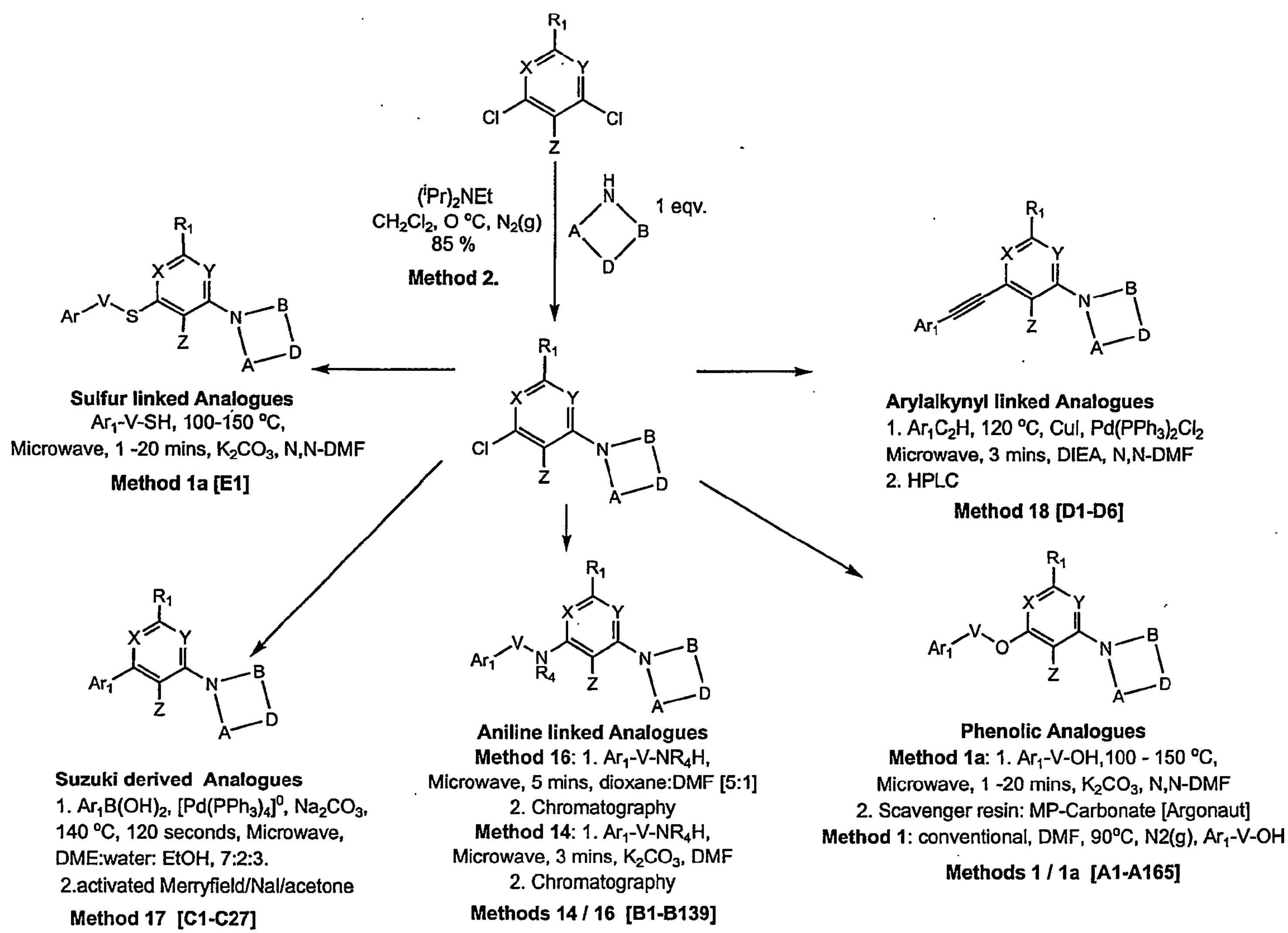

($^i$Pr)$_2$NEt
$CH_2Cl_2$, O °C, $N_2$(g)
85 %
Method 2.
1 eqv.
Sulfur linked Analogues
$Ar_1$-V-SH, 100-150 °C,
Microwave, 1 -20 mins, $K_2CO_3$, N,N-DMF
Method 1a [E1]
Arylalkynyl linked Analogues
1. $Ar_1C_2H$, 120 °C, CuI, $Pd(PPh_3)_2Cl_2$
Microwave, 3 mins, DIEA, N,N-DMF
2. HPLC
Method 18 [D1-D6]
Suzuki derived Analogues
1. $Ar_1B(OH)_2$, $[Pd(PPh_3)_4]^0$, $Na_2CO_3$,
140 °C, 120 seconds, Microwave,
DME:water: EtOH, 7:2:3.
2.activated Merryfield/NaI/acetone
Method 17 [C1-C27]
Aniline linked Analogues
Method 16: 1. $Ar_1$-V-$NR_4$H,
Microwave, 5 mins, dioxane:DMF [5:1]
2. Chromatography
Method 14: 1. $Ar_1$-V-$NR_4$H,
Microwave, 3 mins, $K_2CO_3$, DMF
2. Chromatography
Methods 14 / 16 [B1-B139]
Phenolic Analogues
Method 1a: 1. $Ar_1$-V-OH,100 - 150 °C,
Microwave, 1 -20 mins, $K_2CO_3$, N,N-DMF
2. Scavenger resin: MP-Carbonate [Argonaut]
Method 1: conventional, DMF, 90°C, N2(g), $Ar_1$-V-OH
Methods 1 / 1a [A1-A165]


Figure 6

European Patent Office

# PARTIAL EUROPEAN SEARCH REPORT

which under Rule 63 of the European Patent Convention shall be considered, for the purposes of subsequent proceedings, as the European search report

Application Number
EP 07 01 9362

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | WO 2004/000819 A (BAILEY ANDREW ; PATEL ANIL (GB); THOM STEPHEN (GB); PAIRAUDEAU GARRY () 31 December 2003 (2003-12-31) * claim 7 * ----- | 1 | INV. C07D401/14 C07D401/04 C07D413/14 C07D403/14 C07D405/14 C07D417/14 A61K31/506 A61K31/513 A61P3/10 |
| P,X | WO 2004/000843 A (BAILEY ANDREW ; PATEL ANIL (GB); THOM STEPHEN (GB); PAIRAUDEAU GARRY () 31 December 2003 (2003-12-31) * examples 9i-9iii,20i-20iii * ----- | 1 | |
| P,X | WO 03/077656 A (PREUSS ANDREA ; CIBA SC HOLDING AG (CH); HAAP WOLFGANG (DE); MEHLIN AN) 25 September 2003 (2003-09-25) * claim 26 * ----- | 1 | |
| E | WO 2004/029204 A (HAGMANN WILLIAM K ; LI BING (US); KOPKA IHOR E (US); MERCK & CO INC (U) 8 April 2004 (2004-04-08) * claims 1,2 * ----- | 1 | |
| | -/-- | | |

TECHNICAL FIELDS SEARCHED (IPC)

C07D

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

4

| Place of search | Date of completion of the search | Examiner |
|---|---|---|
| Munich | 10 April 2008 | Samsam Bakhtiary, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

European Patent Office

# PARTIAL EUROPEAN SEARCH REPORT

Application Number
EP 07 01 9362

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A<br><br><br><br><br><br><br><br><br>X | NORMAN M.H.: "Structure-activity relationships of a series of pyrrolo(3,2-d)pyrimidine derivatives and related compounds as neuropeptide Y5 receptor antagonists" JOURNAL OF MEDICINAL CHEMISTRY, vol. 43, no. 22, 2000, pages 4288-4312, XP002285195<br>* figure 1 *<br>* example 72 * | 1-100<br><br><br><br><br><br><br><br><br>1 | |
| A<br><br><br>X | US 6 187 777 B1 (NORMAN MARK HENRY ET AL) 13 February 2001 (2001-02-13)<br>* claim 1 *<br>* examples 185C,186A,187C * | 1-100<br><br><br>1 | TECHNICAL FIELDS SEARCHED (IPC) |
| A | WO 02/06274 A (AMERICAN HOME PROD) 24 January 2002 (2002-01-24)<br>* claims 1,14,21 * | 1-100 | |
| A | US 6 414 002 B1 (DEVASTHALE PRATIK ET AL) 2 July 2002 (2002-07-02)<br>* claim 1 * | 1-100 | |
| A | EP 0 940 387 A (TOKYO TANABE CO) 8 September 1999 (1999-09-08)<br>* claim 1 * | 1-100 | |
| A | EP 0 801 059 A (DAINIPPON PHARMACEUTICAL CO) 15 October 1997 (1997-10-15)<br>* claim 1 * | 1-100 | |
| X | WO 03/002544 A (SQUIBB BRISTOL MYERS CO ; WROBLESKI STEPHEN T (US); HENDERSON IAN (US)) 9 January 2003 (2003-01-09)<br>* claims 1,13,31 * | 1 | |

-/--

4

EPO FORM 1503 03.82 (P04C10)

European Patent Office

# PARTIAL EUROPEAN SEARCH REPORT

Application Number
EP 07 01 9362

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 23 41 925 A (THOMAE GMBH DR K) 6 March 1975 (1975-03-06) claim 1; RN 56035-91-7 | 1 | |
| X | US 5 849 759 A (ZHAO ZUCHUN ET AL) 15 December 1998 (1998-12-15) reaction scheme 3, compounds of formulae (17) and (18); RN 192696-51-8; RN 192696-52-9 | 1 | |
| X | WO 01/62233 A (HOFFMANN LA ROCHE) 30 August 2001 (2001-08-30) * claims 24-31 * | 1 | |
| X | WO 02/098864 A (HOFFMANN LA ROCHE) 12 December 2002 (2002-12-12) * claims 1,7 * | 1 | TECHNICAL FIELDS SEARCHED (IPC) |
| X | EP 0 556 889 A (DUPHAR INT RES) 25 August 1993 (1993-08-25) * claim 7 * | 1 | |
| X | WO 01/49677 A (BANG ANDERSEN BENNY ; ROTTLAENDER MARIO (DK); ANDERSEN KIM (DK); KEHLE) 12 July 2001 (2001-07-12) * claims 1,4,6,7 * | 1 | |
| X | DE 28 31 850 A (BASF AG) 7 February 1980 (1980-02-07) * claim 1; example 7 * | 1 | |
| X | EP 0 667 343 A (SANDOZ AG ; SANDOZ LTD (CH); SANDOZ AG (DE)) 16 August 1995 (1995-08-16) * example 1.19 * | 1 | |

-/--

4

EPO FORM 1503 03.82 (P04C10)

European Patent Office

# PARTIAL EUROPEAN SEARCH REPORT

Application Number
EP 07 01 9362

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | SU 938 559 A (VSESOYUZNYJ NI KHIM FARMATSEVT) 30 November 1993 (1993-11-30)<br>* claim 1 *<br>----- | 1 | |
| X | WO 01/27107 A (SQUIBB BRISTOL MYERS CO ; AHMAD SALEEM (US); ATWAL KARNAIL S (US); NEI) 19 April 2001 (2001-04-19)<br>* claims 1,12,22,24 *<br>----- | 1 | |
| X | WO 96/36613 A (NIPPON SODA CO ; UEDA AKIYOSHI (JP); MIYAZAWA YASUYUKI (JP); SATO DAIS) 21 November 1996 (1996-11-21)<br>* claim 1; tables 1-3 *<br>----- | 1 | |
| X | WO 96/33994 A (NIPPON SODA CO ; UEDA AKIYOSHI (JP); MIYAZAWA YASUYUKI (JP); SATO DAIS) 31 October 1996 (1996-10-31)<br>* claim 1; tables 1-9 *<br>----- | 1 | TECHNICAL FIELDS SEARCHED (IPC) |
| X | WO 97/26252 A (FMC CORP)<br>24 July 1997 (1997-07-24)<br>* claim 1; example 67 *<br>----- | 1 | |
| X | WO 02/02549 A (KUMAGAI TOSHIHITO ; KAMEO KAZUYA (JP); NAKAZATO ATSURO (JP); OKUBO TAK) 10 January 2002 (2002-01-10)<br>* claim 1 *<br>----- | 1 | |

4

EPO FORM 1503 03.82 (P04C10)

European Patent Office

INCOMPLETE SEARCH
SHEET C

Application Number
EP 07 01 9362

Although claims 79-92 are directed to a method of treatment of the human/animal body, the search has been carried out and based on the alleged effects of the compound/composition.

-----

## ANNEX TO THE EUROPEAN SEARCH REPORT ON EUROPEAN PATENT APPLICATION NO. EP 07 01 9362

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2008

| Patent document cited in search report | Publication date | Patent family member(s) | Publication date |
|---|---|---|---|
| WO 2004000819 A | 31-12-2003 | AT 353880 T | 15-03-2007 |
| | | AU 2003243095 A1 | 06-01-2004 |
| | | DE 60311820 T2 | 22-11-2007 |
| | | EP 1532121 A1 | 25-05-2005 |
| | | ES 2280794 T3 | 16-09-2007 |
| | | JP 2005533803 T | 10-11-2005 |
| | | US 2005222152 A1 | 06-10-2005 |
| WO 2004000843 A | 31-12-2003 | AU 2003243096 A1 | 06-01-2004 |
| | | DE 60311272 T2 | 15-11-2007 |
| | | EP 1532148 A1 | 25-05-2005 |
| | | ES 2279162 T3 | 16-08-2007 |
| | | JP 2005533804 T | 10-11-2005 |
| | | US 2005203107 A1 | 15-09-2005 |
| WO 03077656 A | 25-09-2003 | AT 366045 T | 15-07-2007 |
| | | AU 2003214110 A1 | 29-09-2003 |
| | | CN 1642422 A | 20-07-2005 |
| | | ES 2290436 T3 | 16-02-2008 |
| | | JP 2005520821 T | 14-07-2005 |
| | | US 2005143387 A1 | 30-06-2005 |
| WO 2004029204 A | 08-04-2004 | AU 2003275242 A1 | 19-04-2004 |
| | | CA 2499497 A1 | 08-04-2004 |
| | | EP 1546115 A2 | 29-06-2005 |
| | | JP 2006510597 T | 30-03-2006 |
| US 6187777 B1 | 13-02-2001 | AT 323088 T | 15-04-2006 |
| | | AU 747920 B2 | 30-05-2002 |
| | | CA 2319275 A1 | 12-08-1999 |
| | | DE 69930835 T2 | 19-10-2006 |
| | | DK 1054887 T3 | 08-05-2006 |
| | | EP 1054887 A1 | 29-11-2000 |
| | | JP 2003502272 T | 21-01-2003 |
| | | PT 1054887 T | 30-06-2006 |
| | | WO 9940091 A1 | 12-08-1999 |
| | | US 6583154 B1 | 24-06-2003 |
| WO 0206274 A | 24-01-2002 | AU 2293202 A | 30-01-2002 |
| US 6414002 B1 | 02-07-2002 | NONE | |
| EP 0940387 A | 08-09-1999 | AT 239694 T | 15-05-2003 |
| | | AU 715216 B2 | 20-01-2000 |
| | | AU 7335896 A | 15-05-1997 |
| | | CZ 9801265 A3 | 16-09-1998 |



For more details about this annex : see Official Journal of the European Patent Office, No. 12/82

## ANNEX TO THE EUROPEAN SEARCH REPORT ON EUROPEAN PATENT APPLICATION NO. EP 07 01 9362

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2008

| Patent document cited in search report | Publication date | Patent family member(s) | Publication date |
|---|---|---|---|
| EP 0940387 A | | DE 69628051 D1 | 12-06-2003 |
| | | DE 69628051 T2 | 26-02-2004 |
| | | ES 2193266 T3 | 01-11-2003 |
| | | HU 9802318 A2 | 30-08-1999 |
| | | WO 9715549 A1 | 01-05-1997 |
| | | JP 3926390 B2 | 06-06-2007 |
| | | NO 981858 A | 24-06-1998 |
| | | US 6069176 A | 30-05-2000 |
| EP 0801059 A | 15-10-1997 | AT 202559 T | 15-07-2001 |
| | | AU 688165 B2 | 05-03-1998 |
| | | AU 3936695 A | 19-06-1996 |
| | | BR 9509827 A | 30-09-1997 |
| | | CA 2206307 A1 | 06-06-1996 |
| | | CN 1174549 A | 25-02-1998 |
| | | CY 2247 A | 04-07-2003 |
| | | CZ 9701655 A3 | 12-11-1997 |
| | | DE 69521529 D1 | 02-08-2001 |
| | | DE 69521529 T2 | 11-10-2001 |
| | | DK 801059 T3 | 24-09-2001 |
| | | ES 2159648 T3 | 16-10-2001 |
| | | FI 972275 A | 29-05-1997 |
| | | GR 3036148 T3 | 28-09-2001 |
| | | HK 1003886 A1 | 22-02-2002 |
| | | HU 77308 A2 | 30-03-1998 |
| | | IL 116178 A | 26-07-2000 |
| | | WO 9616938 A1 | 06-06-1996 |
| | | JP 3100165 B2 | 16-10-2000 |
| | | NO 972427 A | 28-07-1997 |
| | | NZ 295749 A | 27-05-1998 |
| | | PL 320461 A1 | 29-09-1997 |
| | | PT 801059 T | 30-10-2001 |
| | | RO 118426 B1 | 30-05-2003 |
| | | RU 2137759 C1 | 20-09-1999 |
| | | SK 65697 A3 | 14-01-1998 |
| | | US 5817689 A | 06-10-1998 |
| | | ZA 9510130 A | 11-06-1996 |
| WO 03002544 A | 09-01-2003 | CA 2451128 A1 | 09-01-2003 |
| | | EP 1406875 A1 | 14-04-2004 |
| | | HU 0401711 A2 | 28-12-2004 |
| | | JP 2004535447 T | 25-11-2004 |
| DE 2341925 A | 06-03-1975 | BE 818990 A1 | 19-02-1975 |
| | | JP 50049288 A | 01-05-1975 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 07 01 9362

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2008

| Patent document cited in search report | Publication date | Patent family member(s) | Publication date |
|---|---|---|---|
| | | ZA 7405305 A | 28-04-1976 |
| US 5849759 A | 15-12-1998 | AT 215371 T | 15-04-2002 |
| | | AU 700894 B2 | 14-01-1999 |
| | | AU 1395697 A | 03-07-1997 |
| | | CN 1209062 A | 24-02-1999 |
| | | CZ 9801776 A3 | 16-09-1998 |
| | | DE 69620443 D1 | 08-05-2002 |
| | | DE 69620443 T2 | 28-11-2002 |
| | | DK 865281 T3 | 29-07-2002 |
| | | EP 0865281 A1 | 23-09-1998 |
| | | ES 2175179 T3 | 16-11-2002 |
| | | HK 1018208 A1 | 13-02-2004 |
| | | HU 9904066 A2 | 28-03-2000 |
| | | WO 9721437 A1 | 19-06-1997 |
| | | IL 124537 A | 21-04-2002 |
| | | JP 2000502082 T | 22-02-2000 |
| | | NO 982606 A | 10-08-1998 |
| | | NZ 325449 A | 28-01-2000 |
| | | PL 327169 A1 | 23-11-1998 |
| | | PT 865281 T | 30-09-2002 |
| | | SK 74698 A3 | 02-12-1998 |
| | | ZA 9610315 A | 17-06-1997 |
| WO 0162233 A | 30-08-2001 | AT 293962 T | 15-05-2005 |
| | | AU 780527 B2 | 24-03-2005 |
| | | AU 5464301 A | 03-09-2001 |
| | | BR 0108611 A | 06-05-2003 |
| | | CA 2398274 A1 | 30-08-2001 |
| | | CN 1438890 A | 27-08-2003 |
| | | CZ 20023199 A3 | 14-05-2003 |
| | | DE 60110391 D1 | 02-06-2005 |
| | | DE 60110391 T2 | 26-01-2006 |
| | | ES 2240449 T3 | 16-10-2005 |
| | | HR 20020673 A2 | 31-12-2004 |
| | | HU 0300029 A2 | 28-05-2003 |
| | | JP 2003523380 T | 05-08-2003 |
| | | MA 26878 A1 | 20-12-2004 |
| | | MX PA02008240 A | 29-11-2002 |
| | | NO 20024006 A | 22-08-2002 |
| | | NZ 520241 A | 28-05-2004 |
| | | PL 365140 A1 | 27-12-2004 |
| | | RU 2277911 C2 | 20-06-2006 |
| | | US 2001027196 A1 | 04-10-2001 |
| | | UY 26598 A1 | 27-08-2001 |
| | | ZA 200206077 A | 30-10-2003 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 07 01 9362

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2008

| Patent document cited in search report | Publication date | Patent family member(s) | Publication date |
|---|---|---|---|
| WO 02098864 A | 12-12-2002 | BR 0210102 A | 08-06-2004 |
| WO 02098864 A | | CA 2448602 A1 | 12-12-2002 |
| | | CN 1512988 A | 14-07-2004 |
| | | JP 4049742 B2 | 20-02-2008 |
| | | JP 2004536814 T | 09-12-2004 |
| | | MX PA03010822 A | 17-02-2004 |
| | | US 2003060466 A1 | 27-03-2003 |
| | | ZA 200308859 A | 14-02-2005 |
| EP 0556889 A | 25-08-1993 | CA 2089534 A1 | 19-08-1993 |
| | | JP 6025201 A | 01-02-1994 |
| | | US 5463050 A | 31-10-1995 |
| WO 0149677 A | 12-07-2001 | AR 029217 A1 | 18-06-2003 |
| | | AT 261958 T | 15-04-2004 |
| | | AU 2152001 A | 16-07-2001 |
| | | BG 106961 A | 30-04-2003 |
| | | BR 0016951 A | 25-02-2003 |
| | | CA 2395867 A1 | 12-07-2001 |
| | | CN 1437596 A | 20-08-2003 |
| | | DE 60009154 D1 | 22-04-2004 |
| | | EP 1246815 A1 | 09-10-2002 |
| | | HU 0203645 A2 | 28-02-2003 |
| | | IS 6431 A | 19-06-2002 |
| | | JP 2003519223 T | 17-06-2003 |
| | | MX PA02006499 A | 29-11-2002 |
| | | NO 20023149 A | 28-06-2002 |
| | | NZ 519692 A | 28-05-2004 |
| | | PL 355540 A1 | 04-05-2004 |
| | | SK 11062002 A3 | 03-12-2002 |
| | | TR 200201684 T2 | 21-10-2002 |
| | | US 2003083336 A1 | 01-05-2003 |
| | | ZA 200204970 A | 26-01-2004 |
| DE 2831850 A | 07-02-1980 | JP 55017382 A | 06-02-1980 |
| | | ZA 7903667 A | 30-07-1980 |
| EP 0667343 A | 16-08-1995 | BR 9500069 A | 17-10-1995 |
| | | CA 2139888 A1 | 19-07-1995 |
| | | CN 1108652 A | 20-09-1995 |
| | | CZ 9500064 A3 | 18-10-1995 |
| | | GB 2289275 A | 15-11-1995 |
| | | HU 69054 A2 | 28-08-1995 |
| | | JP 7224046 A | 22-08-1995 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 07 01 9362

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2008

| Patent document cited in search report | Publication date | Patent family member(s) | Publication date |
|---|---|---|---|
| | | PL 306730 A1 | 24-07-1995 |
| | | SG 64382 A1 | 27-04-1999 |
| | | SK 2995 A3 | 09-08-1995 |
| EP 0667343 A | | TR 28254 A | 20-03-1996 |
| | | US 5547919 A | 20-08-1996 |
| | | ZA 9500225 A | 12-07-1996 |
| SU 938559 A | 30-11-1993 | NONE | |
| WO 0127107 A | 19-04-2001 | AT 314364 T | 15-01-2006 |
| | | AU 7859600 A | 23-04-2001 |
| | | BR 0014725 A | 17-06-2003 |
| | | CA 2388813 A1 | 19-04-2001 |
| | | CN 1468232 A | 14-01-2004 |
| | | DE 60025245 T2 | 24-08-2006 |
| | | DK 1224183 T3 | 06-03-2006 |
| | | EP 1224183 A2 | 24-07-2002 |
| | | ES 2254236 T3 | 16-06-2006 |
| | | HU 0300195 A2 | 28-07-2003 |
| | | JP 2003527331 T | 16-09-2003 |
| | | MX PA02003626 A | 22-09-2003 |
| | | NO 20021717 A | 10-06-2002 |
| | | NZ 517668 A | 24-09-2004 |
| | | PL 366131 A1 | 24-01-2005 |
| | | RU 2002111867 A | 10-05-2004 |
| | | TR 200201014 T2 | 21-01-2003 |
| | | US 2005137216 A1 | 23-06-2005 |
| | | US 6887870 B1 | 03-05-2005 |
| | | ZA 200202479 A | 27-07-2004 |
| WO 9636613 A | 21-11-1996 | NONE | |
| WO 9633994 A | 31-10-1996 | NONE | |
| WO 9726252 A | 24-07-1997 | AU 1580997 A | 11-08-1997 |
| | | US H2007 H1 | 04-12-2001 |
| WO 0202549 A | 10-01-2002 | AR 028782 A1 | 21-05-2003 |
| | | AT 353885 T | 15-03-2007 |
| | | AU 6943701 A | 14-01-2002 |
| | | BG 107374 A | 30-09-2004 |
| | | BR 0112166 A | 02-09-2003 |
| | | CA 2412287 A1 | 10-01-2002 |
| | | CN 1439001 A | 27-08-2003 |
| | | CN 1535968 A | 13-10-2004 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 07 01 9362

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2008

| Patent document cited in search report | Publication date | Patent family member(s) | Publication date |
|---|---|---|---|
| | | CZ 20024229 A3 | 18-06-2003 |
| | | DE 60126616 T2 | 25-10-2007 |
| | | EA 5289 B1 | 30-12-2004 |
| | | EE 200300007 A | 16-08-2004 |
| WO 0202549 A | | EP 1299378 A1 | 09-04-2003 |
| | | HK 1057042 A1 | 13-10-2006 |
| | | HR 20030074 A2 | 28-02-2005 |
| | | HU 0301165 A2 | 28-08-2003 |
| | | JP 2004502685 T | 29-01-2004 |
| | | MX PA02012820 A | 14-05-2003 |
| | | NO 20026125 A | 04-02-2003 |
| | | PL 358411 A1 | 09-08-2004 |
| | | SK 132003 A3 | 05-08-2003 |
| | | TW 591022 B | 11-06-2004 |
| | | US 2004034061 A1 | 19-02-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 44039403 P
- US 44982903 P
- US 45339003 P
- US 47087503 P


### Non-patent literature cited in the description


- **ROTWEIN, R et al.** *N. Engl. J. Med.,* 1983, vol. 308, 65-71
- **RIMOIN, D. L.** Emery and Rimoin's Principles and Practice of Medical Genetics. 1996, vol. 1, 1401-1402
- **LE STUNFF et al.** *Diabetes,* 1989, vol. 43, 696-702
- **PEDERSON, P.** *Diab. Metab. Rev.,* 1989, vol. 5, 505-509
- **BRANCATI, F. L et al.** *Arch. Intern. Med.,* 1999, vol. 159, 957-963
- **HILL, J. O et al.** *Science,* 1998, vol. 280, 1371-1374
- **PERRY, I. J et al.** *BMJ,* 1995, vol. 310, 560-564
- Remington's Pharmaceutical Sciences. Mack Publishing Co, 1980
- **REED ; SCRIBNER.** *Diabetes, Obesity and Metabolism,* 1999, vol. 1, 75-86
- **T. HIGUCHI ; V. STELLA.** Pro-drugs as Novel Delivery Systems. American Pharmaceutical Association and Pergamon Press, 1987, vol. 14
- **ZHU, D.-G.** *J. Org. Chem.,* 2002, vol. 67, 943-948
- **COLLIER, T. L.** *J. Labelled Compd Radiopharm.,* 1999, vol. 42, S264-S266
- **BAS, M.-D.** *J. Labelled Compd Radiopharm.,* 2001, vol. 44, S280-S282

SEQUENCE LISTING

<110> Arena Pharmaceuticals, Inc.
Jones, Robert M.
Semple, Graeme
Fioravanti, Beatriz
Pereira, Guilherme
Calderon, Imelda
Uy, Jane
Choi, Jin Sun Karoline
Xiong, Yifeng
Dave, Vibha

<120> 1,2,3-TRISUBSTITUTED ARYL AND HETEROARYL DERIVATIVES AS MODULATORS OF METABOLISM AND THE PROPHYLAXIS AND TREATMENT OF DISORDERS RELATED THERETO

<130> 34.W01

<160> 6

<170> PatentIn version 3.2

<210> 1
<211> 1191
<212> DNA
<213> Homo sapien

<400> 1
atgtacccat acgacgtccc agactacgct ggaagcttgg aatcatcttt ctcatttgga 60

gtgatccttg ctgtcctggc ctccctcatc attgctacta acacactagt ggctgtggct 120

gtgctgctgt tgatccacaa gaatgatggt gtcagtctct gcttcacctt gaatctggct 180

gtggctgaca ccttgattgg tgtggccatc tctggcctac tcacagacca gctctccagc 240

ccttctcggc ccacacagaa gaccctgtgc agcctgcgga tggcatttgt cacttcctcc 300

gcagctgcct ctgtcctcac ggtcatgctg atcacctttg acaggtacct tgccatcaag 360

cagcccttcc gctacttgaa gatcatgagt gggttcgtgg ccggggcctg cattgccggg 420

ctgtggttag tgtcttacct cattggcttc ctcccactcg gaatccccat gttccagcag 480

actgcctaca aagggcagtg cagcttcttt gctgtatttc accctcactt cgtgctgacc 540

ctctcctgcg ttggcttctt cccagccatg ctcctctttg tcttcttcta ctgcgacatg 600

ctcaagattg cctccatgca cagccagcag attcgaaaga tggaacatgc aggagccatg 660

gctggaggtt atcgatcccc acggactccc agcgacttca aagctctccg tactgtgtct 720

gttctcattg ggagctttgc tctatcctgg acccccttcc ttatcactgg cattgtgcag 780

gtggcctgcc aggagtgtca cctctaccta gtgctggaac ggtacctgtg gctgctcggc 840

gtgggcaact ccctgctcaa cccactcatc tatgcctatt ggcagaagga ggtgcgactg 900

cagctctacc acatggccct aggagtgaag aaggtgctca cctcattcct cctctttctc 960

tcggccagga attgtggccc agagaggccc agggaaagtt cctgtcacat cgtcactatc 1020

tccagctcag agtttgatgg cgaattcgga tccaagggca attctgcaga tatccagcac 1080

agtggcggcc gctcgagtct agagggcccg cggttcgaag gtaagcctat ccctaaccct 1140

ctcctcggtc tcgattctac gcgtaccggt catcatcacc atcaccattg a 1191

<210> 2
<211> 396
<212> PRT
<213> Homo sapien

<400> 2

```
Met Tyr Pro Tyr Asp Val Pro Asp Tyr Ala Gly Ser Leu Glu Ser Ser
1               5                   10                  15

Phe Ser Phe Gly Val Ile Leu Ala Val Leu Ala Ser Leu Ile Ile Ala
            20                  25                  30

Thr Asn Thr Leu Val Ala Val Ala Val Leu Leu Leu Ile His Lys Asn
        35                  40                  45

Asp Gly Val Ser Leu Cys Phe Thr Leu Asn Leu Ala Val Ala Asp Thr
    50                  55                  60

Leu Ile Gly Val Ala Ile Ser Gly Leu Leu Thr Asp Gln Leu Ser Ser
65                  70                  75                  80

Pro Ser Arg Pro Thr Gln Lys Thr Leu Cys Ser Leu Arg Met Ala Phe
                85                  90                  95

Val Thr Ser Ser Ala Ala Ala Ser Val Leu Thr Val Met Leu Ile Thr
            100                 105                 110

Phe Asp Arg Tyr Leu Ala Ile Lys Gln Pro Phe Arg Tyr Leu Lys Ile
        115                 120                 125

Met Ser Gly Phe Val Ala Gly Ala Cys Ile Ala Gly Leu Trp Leu Val
    130                 135                 140

Ser Tyr Leu Ile Gly Phe Leu Pro Leu Gly Ile Pro Met Phe Gln Gln
145                 150                 155                 160

Thr Ala Tyr Lys Gly Gln Cys Ser Phe Phe Ala Val Phe His Pro His
                165                 170                 175

Phe Val Leu Thr Leu Ser Cys Val Gly Phe Phe Pro Ala Met Leu Leu
            180                 185                 190

Phe Val Phe Phe Tyr Cys Asp Met Leu Lys Ile Ala Ser Met His Ser
        195                 200                 205

Gln Gln Ile Arg Lys Met Glu His Ala Gly Ala Met Ala Gly Gly Tyr
    210                 215                 220

Arg Ser Pro Arg Thr Pro Ser Asp Phe Lys Ala Leu Arg Thr Val Ser
225                 230                 235                 240
```

Val Leu Ile Gly Ser Phe Ala Leu Ser Trp Thr Pro Phe Leu Ile Thr
245 250 255

Gly Ile Val Gln Val Ala Cys Gln Glu Cys His Leu Tyr Leu Val Leu
260 265 270

Glu Arg Tyr Leu Trp Leu Leu Gly Val Gly Asn Ser Leu Leu Asn Pro
275 280 285

Leu Ile Tyr Ala Tyr Trp Gln Lys Glu Val Arg Leu Gln Leu Tyr His
290 295 300

Met Ala Leu Gly Val Lys Lys Val Leu Thr Ser Phe Leu Leu Phe Leu
305 310 315 320

Ser Ala Arg Asn Cys Gly Pro Glu Arg Pro Arg Glu Ser Ser Cys His
325 330 335

Ile Val Thr Ile Ser Ser Ser Glu Phe Asp Gly Glu Phe Gly Ser Lys
340 345 350

Gly Asn Ser Ala Asp Ile Gln His Ser Gly Gly Arg Ser Ser Leu Glu
355 360 365

Gly Pro Arg Phe Glu Gly Lys Pro Ile Pro Asn Pro Leu Leu Gly Leu
370 375 380

Asp Ser Thr Arg Thr Gly His His His His His His
385 390 395

<210> 3
<211> 24
<212> DNA
<213> Artificial

<220>
<223> Homo sapien Primer

<400> 3
cattgccggg ctgtggttag tgtc 24

<210> 4
<211> 24
<212> DNA
<213> Artificial

<220>
<223> Homo sapien Primer

<400> 4
ggcatagatg agtgggttga gcag 24

<210> 5
<211> 22
<212> DNA

<213> Artificial

<220>
<223> Rat Primer

<400> 5
catgggccct gcaccttctt tg 22

<210> 6
<211> 24
<212> DNA
<213> Artificial

<220>
<223> Rat Primer

<400> 6
gctccggatg gctgatgata gtga 24